(19) Europäisches Patentamt
European Patent Office
Office européen des brevets

(11) EP 4 035 659 A1

(12) **EUROPEAN PATENT APPLICATION**

(43) Date of publication:
**03.08.2022 Bulletin 2022/31**

(21) Application number: **22151613.1**

(22) Date of filing: **29.11.2017**

(51) International Patent Classification (IPC):
***A61K 9/133*** (2006.01) ***A61K 9/127*** (2006.01)
***A61K 9/10*** (2006.01) ***A61K 9/00*** (2006.01)
***A61K 48/00*** (2006.01)

(52) Cooperative Patent Classification (CPC):
**A61K 9/1276; A61K 9/0053; A61K 31/713; A61P 3/00; A61P 9/00; A61P 25/00; A61P 29/00; A61P 31/04; A61P 31/12; A61P 35/00; A61P 37/02; A61P 37/08; C12N 15/87**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2016 US 201662427531 P**
**18.09.2017 US 201762559921 P**
**18.09.2017 US 201762559967 P**

(62) Document number(s) of the earlier application(s) in accordance with Art. 76 EPC:
**17875645.8 / 3 548 005**

(71) Applicant: **PureTech LYT, Inc.**
**Boston, Massachusetts 02210 (US)**

(72) Inventors:
- **BOLEN, Joseph**
  **Boston, 02116 (US)**
- **BONNER, Daniel Kenneth**
  **Sharon, 02067 (US)**
- **FERREIRA, Lisa V.**
  **Franklin, 02038 (US)**
- **KRUMOVA, Katerina**
  **Swampscott, 01907 (US)**
- **JANTZ, John**
  **Arlington, 02476 (US)**
- **MUTAMBA, James Tendai**
  **Newton, 02462 (US)**
- **SHYAM, Rishab R.**
  **Arlington, 02476 (US)**

(74) Representative: **Tostmann, Holger Carl**
**Wallinger Ricker Schlotter Tostmann**
**Patent- und Rechtsanwälte Partnerschaft mbB**
**Zweibrückenstrasse 5-7**
**80331 München (DE)**

Remarks:
•This application was filed on 14-01-2022 as a divisional application to the application mentioned under INID code 62.
•Claims filed after the date of filing of the application (Rule 68(4) EPC).

# (54) EXOSOMES FOR DELIVERY OF THERAPEUTIC AGENTS

(57) The present invention provides exosomes as drug delivery vehicles, compositions comprising a therapeutic agent encapsulated within such exosomes, methods of producing such exosomes and compositions thereof, as well as methods of delivering such exosomes and compositions to a specific patient tissue or organ. The present invention also provides methods of treating a disease, disorder, or condition such as cancer, an inflammatory disease, an infectious disease, an allergic disease, or an autoimmune disease, comprising administering to a patient in need thereof a provided therapeutic-loaded exosome or a pharmaceutical composition thereof.

FIG. 11

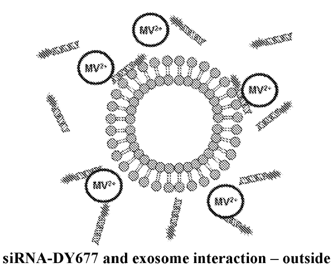


siRNA-DY677 and exosome interaction – outside

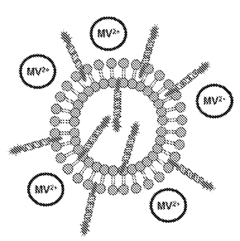


Ch-siRNA-DY677 and exosome interaction – surface + inside

EP 4 035 659 A1

## Description

### CROSS-REFERENCE TO RELATED APPLICATIONS

This application claims the benefit under 35 U.S.C. § 119(e) of U.S. Provisional Application nos. 62/427,531, filed November 29, 2016; 62/559,921, filed September 18, 2017; and 62/559,967, filed September 18, 2017; the contents of all of which are incorporated herein in their entireties by reference.

### TECHNICAL FIELD

The present invention relates, in part, to microvesicles, e.g. exosomes, capable of loading (e.g., encapsulating) therapeutic agents, for example biologics such as proteins, nucleic acids, or other agents, and, in some embodiments, improving their stability or other properties and/or delivering them to a tissue or organ in a patient. The present invention also relates to compositions and methods of using such microvesicles.

### BACKGROUND OF THE INVENTION

Recent years have seen tremendous development of biologics and related therapeutic agents to treat, diagnose, and monitor disease. However, the challenge of generating suitable vehicles to package, stabilize and deliver payloads to sites of interest remains unaddressed. Many therapeutics suffer from degradation due to their inherent instability and active clearance mechanisms *in vivo.* Poor *in vivo* stability is particularly problematic when delivering these payloads orally. The harsh conditions of the digestive tract, including acidic conditions in the stomach, peristaltic motions coupled with the action of proteases, lipases, amylases, and nucleases that break down ingested components in the gastrointestinal tract, make it particularly challenging to deliver many biologics orally. The scale of this challenge is evidenced by the number of biologics limited to delivery via non-oral means, including IV, transdermal, and subcutaneous administration. A general oral delivery vehicle for biologics and related therapeutic agents would profoundly impact healthcare.

Recent efforts have focused on the packaging of biologics into polymer-based, liposomal, or biodegradable and erodible matrices that result in biologic-encapsulated nanoparticles. Despite their advantageous encapsulation properties, such nanoparticles have not achieved widespread use due to toxicity or poor release properties. Additionally, current nanoparticle synthesis techniques are limited in their ability to scale for manufacturing purposes. The development of an effective, non-toxic, and scalable delivery platform thus remains an unmet need.

Exosomes (a class of microvesicles), which until fairly recently were thought of as cellular garbage containers, have emerged as entities known to play a key role in the communication of biological messages and the maintenance of physiological homeostasis. This means of biological communication seems to be conserved across many organisms, and includes the transport of various biomolecules including nucleic acids, proteins, and small molecules.

Milk, which is orally ingested and known to contain a variety of miRNAs important for immune development, protects and delivers these miRNAs in exosomes. Milk exosomes therefore represent a gastrointestinally-privileged (GI-privileged), evolutionarily conserved means of communicating important messages from mother to baby while maintaining the integrity of these complex biomolecules. Indeed, when compared with other types of exosomes, milk exosomes have been observed to have a favorable stability profile at acidic pH and other high-stress or degradative conditions *(See,* e.g., Int J Biol Sci. 2012;8(1):118-23. Epub 2011 Nov 29). Additionally, bovine miRNA levels in circulation have been observed to increase in a dose-dependent manner after consuming varying quantities of milk *(See,* e.g., PLoS One 2015; 10(3): e0121123).

Collectively, the available data suggest that humans have the ability to absorb intact nucleic acid contents of milk. Since milk exosomes are known to encapsulate miRNA species *(See,* e.g., J Nutr. 2014 Oct;144(10):1495-500) appropriate milk exosomes would enable oral delivery of a variety of therapeutic agents. Concordant with this hypothesis, poorly orally available small molecules have been packaged in milk exosomes and delivered orally in rodent models *(See,* e.g., Cancer Lett 2016 Feb 1;371(1):48-61).

The present invention harnesses milk-derived exosomes to meet the urgent need for suitable delivery vehicles for therapeutics that were previously not orally administrable or suffered from other delivery challenges such as poor bioavailability, storage instability, metabolism, off-target toxicity, or decomposition *in vivo.*

### SUMMARY OF THE INVENTION

In one aspect, the present invention provides microvesicles, such as milk-derived exosomes, as vehicles for therapeutic agents such as DNA, RNA, iRNA and antisense oligonucleotides and analogs of nucleic acids, antibodies, hormones, and other peptides and proteins. In some embodiments, the therapeutic agent is conjugated to a hydrophobic group such as a sterol, steroid, or lipid. In some embodiments, the hydrophobic group facilitates loading of the therapeutic

agent into the exosome and/or delivery of the therapeutic agent to a target tissue or organ. The microvesicles may be loaded with a therapeutic agent through a variety of different methods disclosed herein. In one aspect, the present invention provides a therapeutic agent-loaded exosome ("therapeutic-loaded exosome") and pharmaceutical compositions comprising the same. In certain embodiments, provided exosomes are useful for delivery of an effective amount of a therapeutic agent to a patient in need thereof for the diagnosis, prevention, treatment, prognosis, or monitoring of disease. Such therapeutic-loaded exosomes and methods of using the same are described in detail, herein.

## BRIEF DESCRIPTION OF THE FIGURES

**FIG. 1** shows a distribution curve of milk exosome diameters for exosomes isolated from colostrum and raw milk.

**FIG. 2** shows a Cryo-TEM image of a milk exosome.

**FIG. 3** shows results demonstrating that isolated milk exosomes contain CD81, a classical exosome tetraspanin.

**FIG. 4** shows the results of a 14-day stability study. Protein concentration was measured each day for a sample stored at 4 °C (upper graph). Protein concentrations were also measured at day 14 for samples stored at room temperature, 4 °C, -20 °C, and -80 °C, respectively (lower graph). The results show that milk exosomes from both raw milk ("PT Raw" data) and colostrum ("PT Colostrum" data) are stable for at least 14 days at all temperatures tested.

**FIG. 5** shows the results of a 14-day stability study. Particle size was measured each day for a sample stored at 4 °C (upper graph). Particle size was also measured at day 14 for samples stored at room temperature, 4 °C, -20 °C, and -80 °C, respectively (lower graph). The results show that milk exosomes from both raw milk ("PT Raw" data) and colostrum ("PT Colostrum" data) are stable for at least 14 days at all temperatures tested.

**FIG. 6** shows results of a shelf-life and gut stability study (14 days, 4 °C). Each of the two samples tested maintained their particle size during the study as shown in the upper bar graph. Results of a gut stability study (pH 2.5 SGF, simulated gastric fluid and pH 7 SIF, simulated intestinal fluid) are shown in the lower bar graph.

**FIG. 7** shows results of experiments to determine optimal siRNA to exosomes ratios for loading. The top portion of the figure shows a PAGE gel of RNA stained with SYBR Gold Nucleic Acid stain. The bottom portion of the figure shows PAGE of RNA fluorophore.

**FIG. 8** shows results of experiments to determine optimal siRNA to exosomes ratios for loading. The top portion of the figure shows a PAGE gel of RNA stained with SYBR Gold Nucleic Acid stain at ratios of 500:1, 400:1, 300:1, and 250:1. The bottom portion of the figure shows PAGE of RNA fluorophore. The amount of siRNA loaded in exosomes increased with the number of exosomes.

**FIG. 9** shows PAGE results of experiments to determine optimal siRNA to exosomes ratios for loading.

**FIG. 10** shows a pictorial representation of an experiment to determine if cholesterol-conjugated GFP siRNA are associated with the outer membrane of exosomes and if so whether they can be solubilized by MBCD (i.e. dissociated from the exosomes). The Figure includes PAGE results showing that MBCD indeed solubilizes chsiRNA (cholesterol siRNA).

**FIG. 11** shows cartoons of a dye quenching experiment to determine the degree of siRNA loading on the surface vs. inside exosomes. Exclusively surface-loaded siRNA would be fully quenched by the $MV^{2+}$ dye. siRNA on the interior would not be quenched, and so a fluorescence signal that does not quench upon sequential addition of more dye would result, i.e. a plateau effect.

**FIG. 12** shows encapsulation efficiency results for DY677 siRNA and cholesterol conjugated siRNA (Ch-siRNA). Ch-siRNA is encapsulated more efficiently than the siRNA.

**FIG. 13** shows results of Stern-Volmer quenching experiments on siRNA loaded on milk exosomes after loading via free-thaw cycles. Linear decrease in fluorescence was observed in samples of Colostrum/siRNA. However, the slope was lower compared to that of siRNA in PBS or in exosomes. The lack of plateau suggests that the siRNA is not encapsulated but is interacting with the colostrum and is less available for the quencher. ChsiRNA is fully quenched in PBS. Unquenchable fraction is noticed in samples of chsiRNA mixed with exosomes 500/1, chsiRNA-exosomes subjected to 12 freeze-thaw cycles, and chsiRNA mixed with colostrum and sonicated for 4×1 s cycles.

**FIG. 14** shows results of Stern-Volmer quenching experiments on siRNA loaded on milk exosomes after loading via free-thaw cycles. Unquenchable fraction was noted in samples of chsiRNA mixed with exosomes 500/1, chsiRNA-exosomes subjected to 12 freeze-thaw cycles, and chsiRNA mixed with colostrum and sonicated for 4×1 s cycles. The percentages of encapsulation of the siRNA in the exosomes was calculated and is shown in bar graph form.

**FIG. 15** shows PAGE results from exosomes loaded with siRNA or chsiRNA by mixing or freeze-thaw cycles.

**FIG. 16** shows PAGE results from exosomes loaded with siRNA or chsiRNA by mixing or freeze-thaw cycles.

**FIG. 17** shows shows results of Stern-Volmer quenching experiments on siRNA loaded on milk exosomes after loading via mixing or sonication at differing siRNA/exosome ratios.

**FIG. 18** shows shows results of Stern-Volmer quenching experiments on siRNA loaded on milk exosomes after

loading via mixing or sonication at differing siRNA/exosome ratios.

**FIG. 19** shows PAGE results from exosomes loaded with siRNA or chsiRNA by mixing or sonication at differing siRNA/exosome ratios.

**FIG. 20** shows PAGE results from exosomes loaded with siRNA or chsiRNA by mixing or sonication at differing siRNA/exosome ratios.

**FIG. 21** shows fluorescence measurements from cholesterol solubilization of ChsiRNA loaded in exosomes by 3.8 mM methyl beta cyclodextrin and 1% Triton X.

**FIG. 22** shows fluorescence measurements from cholesterol solubilization of ChsiRNA loaded in exosomes by 3.8 mM methyl beta cyclodextrin and 1% Triton X.

**FIG. 23** shows PAGE results from cholesterol solubilization of ChsiRNA loaded in exosomes by 3.8 mM methyl beta cyclodextrin and 1% Triton X.

**FIG. 24** shows PAGE results from cholesterol solubilization of ChsiRNA loaded in exosomes by 3.8 mM methyl beta cyclodextrin and 1% Triton X.

**FIG. 25** shows PAGE results comparing the efficiency of sonication vs. mixing on ChsiRNA loading into exosomes.

**FIG. 26** shows PAGE results comparing the efficiency of sonication vs. mixing on ChsiRNA loading into exosomes.

**FIG. 27A** shows relative fluorescence intensity of ChsiRNA-loaded exosome supernatant, pellet, and stock solution after ultracentrifugation. **FIG. 27B** shows Stern-Volmer quenching results and calculated ChsiRNA loading calculations.

**FIG. 28A** shows size exclusion chromatography purification of ChsiRNA. **FIG. 28B** shows size exclusion chromatography purification of ChsiRNA-loaded exosomes. Free chsiRNA comes at about 1.2 mL (each fraction is 200 uL), so chsiRNA/exo and free chsiRNA appear to co-elute under these conditions. Sephacryl-500HR may provide better separation.

## DETAILED DESCRIPTION OF THE INVENTION

### *1. __General Description of Certain Aspects of the Invention__*

*Therapeutic-Loaded Exosomes*

Microvesicles are naturally-occurring particles that are in the form of small assemblies of lipids about 30 to 1000 nm in size. They are not only produced by many types of cells in *in vitro* culture models and live cells, but are also found in bacteria, plants, and animals alike, and may be found in various fruits, vegetables, and bodily fluids, including blood, urine, and milk.

Microvesicles are formed by a variety of processes, including the release of apoptotic bodies, the budding of microvesicles directly from the cytoplasmic membranes of cells, and exocytosis from multivesicular bodies. For example, exosomes are typically secreted from the endosomal membrane compartments of cells after fusion of multivesicular bodies with the plasma membrane. Multivesicular bodies (MVBs) form by inward budding from an endosomal membrane and subsequent pinching off of small vesicles into the luminal space. The internal vesicles present in the MVBs are then released into the extracellular fluid as exosomes.

Microvesicles serve such purposes as eliminating unwanted molecules, proteins, and other materials from cells and mediating cell-cell communication. Cytosolic and plasma membrane proteins may also be incorporated into microvesicles during their formation, resulting in microvesicles carrying nucleic acids or proteins encapsulated within them as well as presented on the microvesicle surface. Microvesicles, and milk-derived exosomes in particular, have particle size distributions and lipid bilayer functional properties that allow the microvesicles to function as effective nanoparticle carriers of therapeutic agents. In some embodiments of the present invention, a provided microvesicle, such as a milk-derived exosome, includes a surface-bound, cytosolic, or transmembrane protein, nucleic acid, or glycoprotein. In some embodiments, such protein, nucleic acid, or glycoprotein provides advantageous properties to the milk-derived exosome such as enhanced *in vivo* stability or selective delivery to a target tissue or organ.

As used herein, the terms "microvesicle" and "exosome" are used interchangeably herein with the terms "microvesicle," "liposome," "exosome," "exosome-like particle," "exosome-like vesicle," "milk fat globule membrane," "nanovector," "archeosome," "lactosome," "extracellular vesicle," "argosome," "apoptotic body," "epididimosome," "exosome-like vesicle," "microparticle," "promininosome," "prostasome," "dexosome," "texosome," and "oncosome," and grammatical variations of each of the foregoing.

In some embodiments, an exosome is about 20 nm to about 200 nm in diameter. In some embodiments, an exosome is about 30 nm to about 190 nm or about 25 nm to about 180 nm in size. In some embodiments, an exosome is about 30 nm to about 170 nm in size. In some embodiments, an exosome is about 40 nm to about 160 nm in size. In some embodiments, an exosome is about 50 nm to about 150 or about 60 to about 140 nm, about 70 to about 130, about 80 to about 120, or about 90 to about 110 nm in diameter. In some embodiments, an exosome is about 20, 25,

30, 35, 50, 75, 100, 110, 125, or 150 nm in diameter. In some embodiments, an average exosome size in an exosomal composition or plurality of exosomes isolated or derived from milk is about 20, about 25, about 30, about 35, about 50, about 75, about 100, about 110, about 125, or about 150 nm; or about 20 to about 200, about 25 to about 250, about 30 to about 180, about 40 to about 170, about 50 to about 160, about 50 nm to about 150, about 60 to about 140 nm, about 70 to about 130, about 80 to about 120, or about 90 to about 110 nm in average diameter.

Milk, including colostrum, is not only a viable source of large quantities of microvesicles, but microvesicles derived from milk ("milk-derived exosomes" or "milk-derived microvesicles") are useful as an effective delivery vehicle for a number of therapeutic agents and can be used in a manner that retains the biological activity, including the bioavailability, of the therapeutic agents while stabilizing and protecting them. In some embodiments, milk-derived exosomes transport an encapsulated therapeutic agent, such as a biologic therapeutic agent, and release the therapeutic agent after passage through a patient's digestive tract. In some embodiments, a milk-derived exosome encapsulates and later releases the therapeutic agent in such a manner as to avoid first-pass metabolism, e.g. in the patient's liver.

The term "milk" as used herein refers to the opaque liquid containing proteins, fats, lactose, and vitamins and minerals that is produced by the mammary glands of mature female mammals including, but not limited to, after the mammals have given birth to provide nourishment for their young. In some embodiments, the term "milk" is further inclusive of colostrum, which is the liquid secreted by the mammary glands of mammals shortly after parturition that is rich in antibodies and minerals.

The term "milk-derived" or "colostrum-derived," when used in the context of a microvesicle derived from milk or colostrum, refers to a microvesicle that has been isolated from its native environment or otherwise manipulated and is therefore not a product of nature. In this regard, the terms "milk-derived exosomes" and "colostrum-derived exosomes" are used interchangeably herein with the phrases "milk exosomes" or "colostrum exosomes," respectively, in reference to exosomes that have been isolated from milk or colostrum. Additionally, in some embodiments, the term "milk-derived" is used interchangeably with the term "isolated from milk" to describe certain embodiments of the presently-disclosed subject matter.

Certain aspects of the present invention include exosomes, such as milk-derived exosomes, and compositions thereof that can be used to encapsulate a variety of therapeutic agents and are useful in the treatment of various diseases as described herein, *infra.* In some embodiments of the present invention, a microvesicle or composition thereof is provided that comprises one or more therapeutic agents encapsulated by the microvesicle. In some embodiments, the therapeutic agent encapsulated by a microvesicle is selected from a biologic therapeutic agent.

In some embodiments, the present invention provides a therapeutic agent-loaded exosome ("therapeutic-loaded exosome"). As used herein, the term "loaded" in reference to a "therapeutic-loaded exosome" refers to an exosome having one or more therapeutic agents that are encapsulated inside the exosome; associated with or partially embedded within the lipid membrane of the exosome (i.e. partly protruding inside the interior of the exosome); associated with or bound to the outer portion of the lipid membrane and associated components (i.e., partly protruding or fully outside the exosome); or entirely disposed within the lipid membrane of the exosome (i.e. entirely contained within the lipid membrane). Thus, in some embodiments, the therapeutic agent is encapsulated inside the exosome. In some embodiments, the therapeutic agent is associated with or partially embedded within the lipid membrane of the exosome (i.e. partly protruding inside the interior of the exosome). In some embodiments, the therapeutic agent is associated with or bound to the outer portion of the lipid membrane (i.e., partly protruding outside the exosome). In some embodiments, the therapeutic agent is entirely disposed within the lipid membrane of the exosome (i.e. entirely contained within the lipid membrane). In some embodiments, an exosome is loaded with a single therapeutic agent. In some embodiments, an exosome is loaded with two (or more) different therapeutic agents. In some embodiments, an exosome is loaded with two or more molecules or copies of a single therapeutic agent or two (or more) different therapeutic agents. In some embodiments, an exosome is loaded with three or more molecules or copies of a single therapeutic agent or two (or more) different therapeutic agents. In some embodiments, an exosome is loaded with 2-5 molecules or copies of a single therapeutic agent or two (or more) different therapeutic agents. In some embodiments, an exosome or pharmaceutical composition thereof is loaded with 1-4,000, 10-4,000, 50-3,500, 100-3,000, 200-2,500, 300-1,500, 500-1,200, 750-1,000, 1-2,000, 1-1,000, 1-500, 10-400, 50-300, 1-250, 1-100, 2-50, 2-25, 2-15, 2-10, 3-50, 3-25, 3-25, 3-10, 4-50, 4-25, 4-15, 4-10, 5-50, 5-25, 5-15, or 5-10 molecules or copies of a single therapeutic agent or two (or more) different therapeutic agents.

In some embodiments, an exosome is selected from a microvesicle, a liposome, an exosome, an exosome-like particle or vesicle, a milk fat globule membrane, a nano-vector, an archeosome, a lactosome, an extracellular vesicle, an argosome, an apoptotic body, an epididimosome, an exosome-like vesicle, a microparticle, a promininosome, a prostasome, a dexosome, a texosome, or an oncosome. In some embodiments, an exosome is a milk-derived exosome. In some embodiments, a milk-derived exosome is derived (e.g. isolated or manipulated) from milk or colostrum from a cow, human, buffalo, goat, sheep, camel, donkey, horse, reindeer, moose, or yak. In some embodiments, the milk is from a cow.

In some embodiments, the present invention provides a method of treating a disease, disorder, or condition in

a patient in need thereof, comprising administering to the patient a provided therapeutic-loaded exosome. In some embodiments, the disease, disorder, or condition is selected from those treated or treatable by administration of the therapeutic agent loaded therein. Such diseases, disorders, and conditions, and associated therapeutic agents, are described in detail, below.

As used herein, the term "biologic" is used interchangeably with the term "biologic therapeutic agent". One of ordinary skill in the art will recognize that such biologics include those described herein.

In one aspect, the present invention provides a therapeutic-loaded exosome, wherein the therapeutic is a biologic therapeutic agent.

In some embodiments, the biologic therapeutic agent is selected from an allergen, adjuvant, antigen, or immunogen.

In some embodiments, the biologic therapeutic agent is selected from an antibody, hormone, factor, cofactor, metabolic enzyme, immunoregulatory enzyme, interferon, interleukin, gastrointestinal enzyme, an enzyme or factor implicated in hemostasis, growth regulatory enzyme, vaccine, antithrombolytic, toxin, or an antitoxin.

In some embodiments, the biologic therapeutic agent is selected from an oligonucleotide therapeutic agent, such as a single-stranded or double-stranded oligonucleotide therapeutic agent.

In some embodiments, the oligonucleotide therapeutic agent is selected from a single-stranded or double-stranded DNA, iRNA, siRNA, mRNA, ncRNA, antisense RNA, miRNA, LNA, morpholino oligonucleotide, or analog or conjugate thereof.

In some embodiments, the biologic therapeutic agent is selected from a diagnostic or imaging biologic agent.

In some embodiments, the biologic therapeutic agent is an autoimmune antigen.

In some embodiments, the biologic therapeutic agent is a food allergen.

In some embodiments, the biologic therapeutic agent is selected from any of those set forth in Table 1, below.

In some embodiments, the biologic therapeutic agent is selected from any of those set forth in Table 2, below.

In some embodiments, the biologic therapeutic agent is an antigen selected from any of those set forth in Table 3, below.

In some embodiments, the biologic therapeutic agent is selected from any of those set forth in Table 4, below.

In some embodiments, the exosome is selected from a microvesicle, liposome, exosome, exosome-like particle, exosome-like vesicle, milk fat globule membrane, nano-vector, archeosome, lactosome, extracellular vesicle, argosome, apoptotic body, epididimosome, exosome-like vesicle, microparticle, promininosome, prostasome, dexosome, texosome, or oncosome.

In some embodiments, the exosome is a milk-derived exosome.

In some embodiments, the exosome is about 30 to about 220 nm in diameter, about 40 to about 175, about 50 to about 150, about 30 to about 150, or about 30 to about 120 nm in diameter.

In one aspect, the present invention provides a pharmaceutical composition comprising the therapeutic-loaded exosome as described herein, and a pharmaceutically acceptable adjuvant, vehicle, or carrier.

In one aspect, the present invention provides a method of treating a disease, disorder, or condition in a patient in need thereof, comprising administering to the patient a therapeutic-loaded exosome as described herein. In some embodiments, the exosome is selected from a microvesicle, liposome, exosome, exosome-like particle, exosome-like vesicle, milk fat globule membrane, nano-vector, archeosome, lactosome, extracellular vesicle, argosome, apoptotic body, epididimosome, exosome-like vesicle, microparticle, promininosome, prostasome, dexosome, texosome, or oncosome. In some embodiments, the exosome is a milk-derived exosome.

In some embodiments, the therapeutic is a biologic therapeutic agent selected from any of those set forth in Table 1, below.

In some embodiments, the therapeutic is a biologic therapeutic agent selected from any of those set forth in Table 2, 3, or 4, below.

In some embodiments, the biologic therapeutic agent modulates a target selected from any of those set forth in Table 5, below.

In some embodiments, the disease, disorder, or condition is selected from a hyperproliferative disorder, viral or microbial infection, autoimmune disease, allergic condition, inflammatory disease, disorder, or condition, cardiovascular disease, metabolic disease, or neurodegenerative disease.

In some embodiments, the disease, disorder, or condition is selected from those set forth in Table 1, 2, 3, 4, or 5, below.

In some embodiments, the therapeutic-loaded exosome is administered in combination with an additional therapeutic agent.

In some embodiments, the therapeutic-loaded exosome is administered by an oral, intravenous, subcutaneous, intranasal, inhalation, intramuscular, intraocular, intraperitoneal, intratracheal, transdermal, buccal, sublingual, rectal, topical, local injection, or surgical implantation route. In some embodiments, the therapeutic-loaded exosome is administered by an oral route.

*Therapeutic Agents, Hydrophobic Modifications, and Exemplary Associated Diseases*

In accordance with the present invention, a variety of therapeutic agents are loaded or encapsulated inside an exosome. In some embodiments, by using an exosome as a carrier, the present invention enhances desirable properties of the therapeutic agent such as improving oral bioavailability, for example by minimizing destruction of the agent in the gut or minimizing liver first-pass effect; or improving therapeutic agent delivery to a target tissue; or increasing the solubility and stability of the therapeutic agents, including the solubility and stability of the agents *in vivo.* In one aspect, the therapeutic agent comprises or is chemically modified to comprise a hydrophobic group. Suitable hydrophobic groups include sterols, steroids, lipids, phospholipids, or synthetic or natural hydrophobic polymers. Without wishing to be bound by theory, it is believed that hydrophobic modification, e.g. lipid, sterol, or steroid tagging, of a therapeutic agent facilitates its loading into or onto exosomes, such that higher loading efficiencies are enabled.

In one aspect, the present invention provides a therapeutic-loaded milk exosome, wherein the therapeutic is a biologic therapeutic agent and the therapeutic is not naturally-occurring in a milk exosome.

In some embodiments, the biologic therapeutic agent is selected from an antibody, a hormone, a factor, a cofactor, a metabolic enzyme, an immunoregulatory enzyme, an interferon, an interleukin, a gastrointestinal enzyme, an enzyme or factor implicated in hemostasis, a growth regulatory enzyme, a vaccine, an antithrombolytic, a toxin, or an antitoxin.

In some embodiments, the biologic therapeutic agent is a peptide.

In some embodiments, the biologic therapeutic agent is a protein.

In some embodiments, the biologic therapeutic agent is a nucleic acid.

In some embodiments, the nucleic acid is selected from a single-stranded or double-stranded DNA, an iRNA, a siRNA, a shRNA, a mRNA, a non-coding RNA (ncRNA), an antisense RNA, a LNA, a morpholino oligonucleotide, or an analog or conjugate thereof.

In some embodiments, the nucleic acid is a ncRNA of about 30 to about 200 nucleotides (nt) in length or a long non-coding RNA (IncRNA) of about 200 to about 800 nt in length.

In some embodiments, the IncRNA is a long intergenic non-coding RNA (lincRNA), pretranscript, pre-miRNA, pre-mRNA, competing endogenous RNA (ceRNA), small nuclear RNA (snRNA), small nucleolar RNA (snoRNA), pseudogene, rRNA, or tRNA.

In some embodiments, the ncRNA is selected from a piwi-interacting RNA (piRNA), primary miRNA (pri-miRNA), or premature miRNA (pre-miRNA).

In some embodiments, the biologic therapeutic agent is selected from any of those set forth in any of Table 1, Table 2, Table 3, or Table 4.

In some embodiments, the milk exosome is derived from cow, sheep, goat, camel, buffalo, yak, or human milk or colostrum.

In another aspect, the present invention provides a therapeutic-loaded exosome, wherein the therapeutic is a biologic therapeutic agent conjugated to a hydrophobic group.

In some embodiments, the biologic therapeutic agent is selected from an antibody, a hormone, a factor, a cofactor, a metabolic enzyme, an immunoregulatory enzyme, an interferon, an interleukin, a gastrointestinal enzyme, an enzyme or factor implicated in hemostasis, a growth regulatory enzyme, a vaccine, an antithrombolytic, a toxin, or an antitoxin.

In some embodiments, the biologic therapeutic agent is a peptide.

In some embodiments, the biologic therapeutic agent is a protein.

In some embodiments, the biologic therapeutic agent is a nucleic acid.

In some embodiments, the nucleic acid is selected from a single-stranded or double-stranded DNA, an iRNA, a siRNA, a shRNA, a mRNA, a ncRNA, an antisense RNA, a LNA, a morpholino oligonucleotide, or an analog or conjugate thereof.

In some embodiments, the nucleic acid is a non-coding RNA (ncRNA) of about 30 to about 200 nucleotides (nt) in length or a long non-coding RNA (IncRNA) of about 200 to about 800 nt in length.

In some embodiments, the IncRNA is a long intergenic non-coding RNA (lincRNA), pretranscript, pre-miRNA, pre-mRNA, competing endogenous RNA (ceRNA), small nuclear RNA (snRNA), small nucleolar RNA (snoRNA), pseudogene, rRNA, or tRNA.

In some embodiments, the ncRNA is selected from a piwi-interacting RNA (piRNA), primary miRNA (pri-miRNA), or premature miRNA (pre-miRNA).

In some embodiments, the biologic therapeutic agent is selected from any of those set forth in any of Table 1, Table 2, Table 3, or Table 4.

In some embodiments, the hydrophobic group is selected from a lipid, a sterol, a steroid, a terpene, cholic acid, adamantane acetic acid, 1-pyrene butyric acid, 1,3-bis-O(hexadecyl)glycerol, a geranyloxyhexyl group, hexadecylglycerol, borneol, 1,3-propanediol, heptadecyl group, O3-(oleoyl)lithocholic acid, O3-(oleoyl)cholenic acid, dimethoxytrityl,

or phenoxazine.

In some embodiments, the milk exosome is derived from cow, sheep, goat, camel, buffalo, yak, or human milk or colostrum.

In another aspect, the present invention provides a pharmaceutical composition comprising a disclosed therapeutic-loaded milk exosome, and a pharmaceutically acceptable adjuvant, vehicle, or carrier.

In another aspect, the present invention provides a method of treating a disease, disorder, or condition in a patient in need thereof, comprising administering to the patient a disclosed therapeutic-loaded milk exosome, or a pharmaceutically acceptable composition thereof.

In some embodiments, the disease, disorder, or condition is selected from a hyperproliferative disorder, viral or microbial infection, autoimmune disease, allergic condition, inflammatory disease, cardiovascular disease, metabolic disease, or neurodegenerative disease.

In some embodiments, the disease, disorder, or condition is selected from those set forth in Table 1, 2, 3, 4, or 5.

In some embodiments, the therapeutic-loaded milk exosome is administered orally.

In some embodiments, the method further comprises administering to the patient an additional therapeutic agent.

In some embodiments, the therapeutic agent comprising or conjugated to a hydrophobic group is selected from a iRNA, siRNA, mRNA, DNA, hormone, protein such as an antibody or others described herein, peptidomimetic, or small molecule. In some embodiments, the therapeutic agent is a siRNA modified to comprise a lipid or steroid or other hydrophobic group, such as those described in detail herein, *infra.* In some embodiments, the hydrophobic group is a fatty acid or a sterol or steroid such as cholesterol.

In some embodiments, the therapeutic agent comprises or is modified to comprise a hydrophobic group selected from a terpene such as nerolidol, farnesol, limonene, linalool, geraniol, carvone, fenchone, or menthol; a lipid such as palmitic acid or myristic acid; cholesterol; oleyl; retinyl; cholesteryl residues; cholic acid; adamantane acetic acid; 1-pyrene butyric acid; dihydrotestosterone; 1,3-Bis-O(hexadecyl)glycerol; geranyloxyhexyl group; hexadecylglycerol; borneol; 1,3-propanediol; heptadecyl group; O3-(oleoyl)lithocholic acid; O3-(oleoyl)cholenic acid; dimethoxytrityl; or phenoxazine. In some embodiments, the hydrophobic group is cholesterol. In some embodiments, the hydrophobic group is a fat-soluble vitamin. In some embodiments, the hydrophobic group is selected from folic acid; cholesterol; a carbohydrate; vitamin A; vitamin E; or vitamin K.

Other hydrophobic groups include, for example, steroids (e.g., uvaol, hecigenin, diosgenin), terpenes (e.g., triterpenes, e.g., sarsasapogenin, friedelin, epifriedelanol derivatized lithocholic acid), vitamins (e.g., folic acid, vitamin A, biotin, pyridoxal), carbohydrates, proteins, and protein binding agents, as well as lipophilic molecules, e.g, thio analogs of cholesterol, cholic acid, cholanic acid, lithocholic acid, adamantane acetic acid, 1-pyrene butyric acid, dihydrotestosterone, glycerol (e.g., esters (e.g., mono, bis, or tris fatty acid esters, e.g., C10, C11, C12, C13, C14, C15, C16, C17, C18, C19, or C20 fatty acids) and ethers thereof, e.g., C10, C11, C12, C13, C14, C15, C16, C17, C18, C19, or C20 alkyl; e.g., 1,3-bis-O(hexadecyl)glycerol, 1,3-bis-O(octaadecyl)glycerol), geranyloxyhexyl group, hexadecylglycerol, borneol, menthol, 1,3-propanediol, heptadecyl group, palmitic acid, stearic acid (e.g., gyceryl distearate), oleic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g. biotin), transport/absorption facilitators (e.g., aspirin, naproxen, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP.

In some embodiments, the hydrophobic group is a sterol, steroid, hopanoid, hydroxysteroid, secosteroid, or analog thereof with lipophilic properties.

In some embodiments, the hydrophobic group is a sterol, such as a phytosterol, mycosterol, or zoosterol. Exemplary zoosterols include cholesterol and 24S-hydroxycholesterol; exemplary phytosterols include ergosterol (mycosterol), campesterol, sitosterol, and stigmasterol. In some embodiments, the sterol is selected from ergosterol, 7-dehydrocholesterol, cholesterol, 24S-hydroxycholesterol, lanosterol, cycloartenol, fucosterol, saringosterol, campesterol, β-sitosterol, sitostanol, coprostanol, avenasterol, or stigmasterol. Sterols may be found either as free sterols, acylated (sterol esters), alkylated (steryl alkyl ethers), sulfated (sterol sulfate), or linked to a glycoside moiety (steryl glycosides), which can be itself acylated (acylated sterol gl ycosi des).

In some embodiments, the hydrophobic group is a steroid. In some embodiments, the steroid is selected from dihydrotestosterone, uvaol, hecigenin, diosgenin, progesterone, or cortisol.

The hydrophobic moiety may be conjugated to the therapeutic agent at any chemically feasible location, e.g. on a nucleic acid molecule at the 5' and/or 3' end (or one or both strands of the nucleic acid molecule, if it is a duplex). In a particular embodiment, the hydrophobic moiety is conjugated only to the 3' end, more particularly the 3' end of the sense strand in double stranded molecules. The hydrophobic moiety may be conjugated directly to the nucleic acid molecule or via a linker. The hydrophobic moiety may be selected from the group consisting of adamantane, cholesterol, a steroid, long chain fatty acid, lipid, phospholipid, glycolipid, or derivatives thereof.

For example, sterols may be conjugated to the therapeutic at the available -OH group. Exemplary sterols have

the general skeleton shown below:

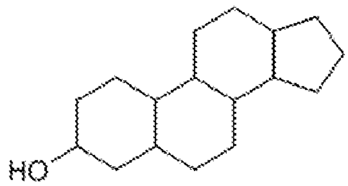

As a further example, ergosterol has the structure below:

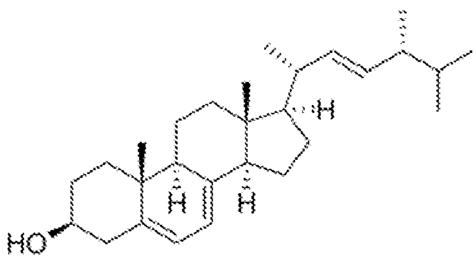

Cholesterol has the structure below:

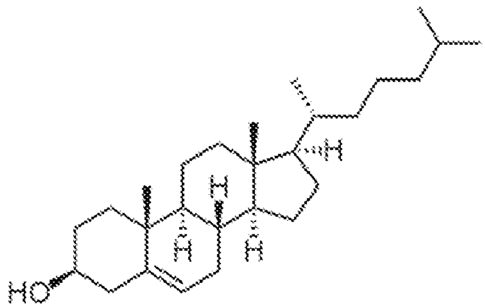

Accordingly, in some embodiments, the free -OH group of a sterol or steroid is used to conjugate the therapeutic to the sterol or steroid.

In some embodiments, the hydrophobic group is a lipid, such as a fatty acid, phosphatide, phospholipid, or analogue thereof (e.g. phophatidylcholine, lecithin, phosphatidylethanolamine, cephalin, or phosphatidylserine or analogue or portion thereof, such as a partially hydrolyzed portion thereof). In some embodiments, the fatty acid is a short-chain, medium-chain, or long-chain fatty acid. In some embodiments, the fatty acid is a saturated fatty acid. In some embodiments, the fatty acid is an unsaturated fatty acid. In some embodiments, the fatty acid is a monounsaturated fatty acid. In some embodiments, the fatty acid is a polyunsaturated fatty acid, such as an ω-3 (omega-3) or ω-6 (omega-6) fatty acid. In some embodiments, the lipid, e.g., fatty acid, has a $C_2$-$C_{60}$ chain. In some embodiments, the lipid, e.g., fatty acid, has a $C_2$-$C_{28}$ chain. In some embodiments, the lipid, e.g., fatty acid, has a $C_2$-$C_{40}$ chain. In some embodiments, the lipid, e.g., fatty acid, has a $C_2$-$C_{12}$ or $C_4$-$C_{12}$ chain. In some embodiments, the lipid, e.g., fatty acid, has a $C_4$-$C_{40}$ chain. In some embodiments, the lipid, e.g., fatty acid, has a $C_4$-$C_{40}$, $C_2$-$C_{38}$, $C_2$-$C_{36}$, $C_2$-$C_{34}$, $C_2$-$C_{32}$, $C_2$-$C_{30}$, $C_4$-$C_{30}$, $C_2$-$C_{28}$, $C_4$-$C_{28}$, $C_2$-$C_{26}$, $C_4$-$C_{26}$, $C_2$-$C_{24}$, $C_4$-$C_{24}$, $C_6$-$C_{24}$, $C_8$-$C_{24}$, $C_{10}$-$C_{24}$, $C_2$-$C_{22}$, $C_4$-$C_{22}$, $C_6$-$C_{22}$, $C_8$-$C_{22}$, $C_{10}$-$C_{22}$, $C_2$-$C_{20}$, $C_4$-$C_{20}$, $C_6$-$C_{20}$, $C_8$-$C_{20}$, $C_{10}$-$C_{20}$, $C_2$-$C_{18}$, $C_4$-$C_{18}$, $C_6$-$C_{18}$, $C_8$-$C_{18}$, $C_{10}$-$C_{18}$, $C_{12}$-$C_{18}$, $C_{14}$-$C_{18}$, $C_{16}$-$C_{18}$, $C_2$-$C_{16}$, $C_4$-$C_{16}$, $C_6$-$C_{16}$, $C_8$-$C_{16}$, $C_{10}$-$C_{16}$, $C_{12}$-$C_{16}$, $C_{14}$-$C_{16}$, $C_2$-$C_{15}$, $C_4$-$C_{15}$, $C_6$-$C_{15}$, $C_8$-$C_{15}$, $C_9$-$C_{15}$, $C_{10}$-$C_{15}$, $C_{11}$-$C_{15}$, $C_{12}$-$C_{15}$, $C_{13}$-$C_{15}$, $C_2$-$C_{14}$, $C_4$-$C_{14}$, $C_6$-$C_{14}$, $C_8$-$C_{14}$, $C_9$-$C_{14}$, $C_{10}$-$C_{14}$, $C_{11}$-$C_{14}$, $C_{12}$-$C_{14}$, $C_2$-$C_{13}$, $C_4$-$C_{13}$, $C_6$-$C_{13}$, $C_7$-$C_{13}$, $C_8$-$C_{13}$, $C_9$-$C_{13}$, $C_{10}$-$C_{13}$, $C_{10}$-$C_{13}$, $C_{11}$-$C_{13}$, $C_2$-$C_{12}$, $C_4$-$C_{12}$, $C_6$-$C_{12}$, $C_7$-$C_{12}$, $C_8$-$C_{12}$, $C_9$-$C_{12}$, $C_{10}$-$C_{12}$, $C_2$-$C_{11}$, $C_4$-$C_{11}$, $C_6$-$C_{11}$, $C_7$-$C_{11}$, $C_8$-$C_{11}$, $C_9$-$C_{11}$, $C_2$-$C_{10}$, $C_4$-$C_{10}$, $C_2$-$C_9$, $C_4$-$C_9$, $C_2$-$C_8$, $C_4$-$C_8$, $C_2$-$C_7$, $C_4$-$C_7$, $C_2$-$C_6$, or $C_4$-$C_6$, chain. In some embodiments, the lipid, e.g., fatty acid, has a $C_2$, $C_3$, $C_4$, $C_5$, $C_6$, $C_7$, $C_8$, $C_9$, $C_{10}$, $C_{11}$, $C_{12}$, $C_{13}$, $C_{14}$, $C_{15}$, $C_{16}$, $C_{17}$, $C_{18}$, $C_{19}$, $C_{20}$, $C_{21}$, $C_{22}$, $C_{23}$, $C_{24}$, $C_{25}$, $C_{26}$, $C_{27}$, $C_{28}$, $C_{29}$, $C_{30}$, $C_{31}$, $C_{32}$, $C_{33}$, $C_{34}$, $C_{35}$, $C_{36}$, $C_{37}$, $C_{38}$, $C_{39}$, $C_{40}$, $C_{41}$, $C_{42}$, $C_{43}$, $C_{44}$, $C_{45}$, $C_{46}$, $C_{47}$, $C_{48}$, $C_{49}$, $C_{50}$, $C_{51}$, $C_{52}$, $C_{53}$, $C_{54}$, $C_{55}$, $C_{56}$, $C_{57}$, $C_{58}$, $C_{59}$, or $C_{60}$ chain. In some embodiments, the therapeutic agent comprises two fatty acids, each of which is independently selected from a fatty acid having a chain with any one of the foregoing ranges or numbers of carbon atoms. In some embodiments, one of the fatty acids is independently a fatty acid with a $C_6$-$C_{21}$ chain and one is independently a fatty acid with a $C_{12}$-$C_{36}$ chain. In some

embodiments, each fatty acid independently has a chain of 11, 12, 13, 14, 15, 16, or 17 carbon atoms.

In some embodiments, the therapeutic agent comprises two lipids. In some embodiments, the two lipids, e.g. fatty acids, taken together have 6-80 carbon atoms (an equivalent carbon number (ECN) of 6-80). In some embodiments, the lipids, e.g., fatty acids, have an ECN of 6-80, 8-80, 10-80, 12-80, 14-80, 16-80, 18-80, 20-80, 22-80, 24-80, 26-80, 28-80, 30-80, 4-76, 6-76, 8-76, 10-76, 12-76, 14-76, 16-76, 18-76, 20-76, 22-76, 24-76, 26-76, 28-76, 30-76, 6-72, 8-72, 10-72, 12-72, 14-72, 16-72, 18-72, 20-72, 22-72, 24-72, 26-72, 28-72, 30-72, 6-68, 8-68, 10-68, 12-68, 14-68, 16-68, 18-68, 20-68, 22-68, 24-68, 26-68, 28-68, 30-68, 6-64, 8-64, 10-64, 12-64, 14-64, 16-64, 18-64, 20-64, 22-64, 24-64, 26-64, 28-64, 30-64, 6-60, 8-60, 10-60, 12-56, 14-56, 16-56, 18-56, 20-56, 22-56, 24-56, 26-56, 28-56, 30-56, 6-52, 8-52, 10-52, 12-52, 14-52, 16-52, 18-52, 20-52, 22-52, 24-52, 26-52, 28-52, 30-52, 6-48, 8-48, 10-48, 12-48, 14-48, 16-48, 18-48, 20-48, 22-48, 24-48, 26-48, 28-48, 30-48, 6-44, 8-44, 10-44, 12-44, 14-44, 16-44, 18-44, 20-44, 22-44, 24-44, 26-44, 28-44, 30-44, 6-40, 8-40, 10-40, 12-40, 14-40, 16-40, 18-40, 20-40, 22-40, 24-40, 26-40, 28-40, 30-40, 6-36, 8-36, 10-36, 12-36, 14-36, 16-36, 18-36, 20-36, 22-36, 24-36, 26-36, 28-36, 30-36, 6-32, 8-32, 10-32, 12-32, 14-32, 16-32, 18-32, 20-32, 22-32, 24-32, 26-32, 28-32, or 30-32.

Suitable fatty acids include saturated straight-chain fatty acids, saturated branched fatty acids, unsaturated fatty acids, hydroxy fatty acids, and polycarboxylic acids. In some embodiments, such fatty acids have up to 32 carbon atoms.

Examples of useful saturated straight-chain fatty acids include those having an even number of carbon atoms, such as butyric acid, caproic acid, caprylic acid, capric acid, lauric acid, myristic acid, palmitic acid, stearic acid, arachic acid, behenic acid, lignoceric acid, hexacosanoic acid, octacosanoic acid, triacontanoic acid and n-dotriacontanoic acid, and those having an odd number of carbon atoms, such as propionic acid, n-valeric acid, enanthic acid, pelargonic acid, hendecanoic acid, tridecanoic acid, pentadecanoic acid, heptadecanoic acid, nonadecanoic acid, heneicosanoic acid, tricosanoic acid, pentacosanoic acid, and heptacosanoic acid.

Examples of suitable saturated branched fatty acids include isobutyric acid, isocaproic acid, isocaprylic acid, isocapric acid, isolauric acid, 11-methyldodecanoic acid, isomyristic acid, 13-methyl-tetradecanoic acid, isopalmitic acid, 15-methyl-hexadecanoic acid, isostearic acid, 17-methyloctadecanoic acid, isoarachic acid, 19-methyl-eicosanoic acid, α-ethylhexanoic acid, α-hexyldecanoic acid, α-heptylundecanoic acid, 2-decyltetradecanoic acid, 2-undecyltetradecanoic acid, 2-decylpentadecanoic acid, 2-undecylpentadecanoic acid, and Fine oxocol 1800 acid (product of Nissan Chemical Industries, Ltd.). Suitable saturated odd-carbon branched fatty acids include anteiso fatty acids terminating with an isobutyl group, such as 6-methyl-octanoic acid, 8-methyl-decanoic acid, 10-methyl-dodecanoic acid, 12-methyl-tetradecanoic acid, 14-methyl-hexadecanoic acid, 16-methyl-octadecanoic acid, 18-methyl-eicosanoic acid, 20-methyl-docosanoic acid, 22-methyl-tetracosanoic acid, 24-methyl-hexacosanoic acid, and 26-methyloctacosanoic acid.

Examples of suitable unsaturated fatty acids include 4-decenoic acid, caproleic acid, 4-dodecenoic acid, 5-dodecenoic acid, lauroleic acid, 4-tetradecenoic acid, 5-tetradecenoic acid, 9-tetradecenoic acid, palmitoleic acid, 6-octadecenoic acid, oleic acid, 9-octadecenoic acid, 11-octadecenoic acid, 9-eicosenoic acid, *cis*-11-eicosenoic acid, cetoleic acid, 13-docosenoic acid, 15-tetracosenoic acid, 17-hexacosenoic acid, 6,9,12,15-hexadecatetraenoic acid, linoleic acid, linolenic acid, α-eleostearic acid, β-eleosteanc acid, punicic acid, 6,9,12,15-octadecatetraenoic acid, parinaric acid, 5,8,11,14-eicosatetraenoic acid, 5,8,11,14,17-eicosapentaenoic acid, 7,10,13,16,19-docosapentaenoic acid, 4,7,10,13,16,19-docosahexaenoic acid, and the like.

Examples of suitable hydroxy fatty acids include α-hydroxylauric acid, α-hydroxymyristic acid, α-hydroxypalmitic acid, α-hydroxystearic acid, ω-hydroxylauric acid, α-hydroxyarachic acid, 9-hydroxy-12-octadecenoic acid, ricinoleic acid, α-hydroxybehenic acid, 9-hydroxy-trans-10,12-octadecadienic acid, kamolenic acid, ipurolic acid, 9,10-dihydroxystearic acid, 12-hydroxystearic acid and the like.

Examples of suitable polycarboxylic acids include oxalic acid, malonic acid, succinic acid, glutaric acid, adipic acid, pimelic acid, suberic acid, azelaic acid, sebacic acid, D,L-malic acid, and the like.

In some embodiments, each fatty acid is independently selected from Propionic acid, Butyric acid, Valeric acid, Caproic acid, Enanthic acid, Caprylic acid, Pelargonic acid, Capric acid, Undecylic acid, Lauric acid, Tridecylic acid, Myristic acid, Pentadecylic acid, Palmitic acid, Margaric acid, Stearic acid, Nonadecylic acid, arachidic acid, Heneicosylic acid, Behenic acid, Tricosylic acid, Lignoceric acid, Pentacosylic acid, Cerotic acid, Heptacosylic acid, Montanic acid, Nonacosylic acid, Melissic acid, Henatriacontylic acid, Lacceroic acid, Psyllic acid, geddic acid, ceroplastic acid, hexatriacontylic acid, heptatriacontanoic acid, or octatriacontanoic acid.

In some embodiments, each fatty acid is independently selected from α-linolenic acid, stearidonic acid, eicosapentaenoic acid, docosahexaenoic acid, linoleic acid, gamma-linoleic acid, dihomo-gamma-linoleic acid, arachidonic acid, docosatetraenoic acid, palmitoleic acid, vaccenic acid, paullinic acid, oleic acid, elaidic acid, gondoic acid, eurcic acid, nervonic acid, mead acid, adrenic acid, bosseopentaenoic acid, ozubondo acid, sardine acid, herring acid, docosahexaenoic acid, or tetracosanolpentaenoic acid, or another monounsaturated or polyunsaturated fatty acid.

In some embodiments, one or both of the fatty acids is an essential fatty acid. In view of the beneficial health effects of certain essential fatty acids, the therapeutic benefits of disclosed therapeutic-loaded exosomes may be increased by including such fatty acids in the therapeutic agent. In some embodiments, the essential fatty acid is an n-6

or n-3 essential fatty acid selected from the group consisting of linolenic acid, gamma-linolenic acid, dihomo-gamma-linolenic acid, arachidonic acid, adrenic acid, docosapentaenoic n-6 acid, alpha-linolenic acid, stearidonic acid, the 20:4n-3 acid, eicosapentaenoic acid, docosapentaenoic n-3 acid, or docosahexaenoic acid.

In some embodiments, each fatty acid is independently selected from all-*cis*-7,10,13-hexadecatrienoic acid, $\alpha$-linolenic acid, stearidonic acid, eicosatrienoic acid, eicosatetraenoic acid, eicosapentaenoic acid (EPA), docosapentaenoic acid, docosahexaenoic acid (DHA), tetracosapentaenoic acid, tetracosahexaenoic acid, or lipoic acid. In other embodiments, the fatty acid is selected from eicosapentaenoic acid, docosahexaenoic acid, or lipoic acid. Other examples of fatty acids include all-*cis*-7,10,13-hexadecatrienoic acid, $\alpha$-linolenic acid (ALA or all-*cis*-9,12,15-octadecatrienoic acid), stearidonic acid (STD or all-*cis*-6,9,12,15-octadecatetraenoic acid), eicosatrienoic acid (ETE or all-*cis*-11,14,17-eicosatiienoic acid), eicosatetraenoic acid (ETA or all-*cis*-8,11,14,17-eicosatetraenoic acid), eicosapentaenoic acid (EPA), docosapentaenoic acid (DPA, clupanodonic acid or all-*cis*-7,10,13,16,19-docosapentaenoic acid), docosahexaenoic acid (DHA or all-*cis*-4,7,10,13,16,19-docosahexaenoic acid), tetracosapentaenoic acid (all-*cis*-9,12,15,18,21-docosahexaenoic acid), or tetracosahexaenoic acid (nisinic acid or all-*cis*-6,9,12,15,18,21-tetracosenoic acid). In some embodiments, the fatty acid is a medium-chain fatty acid such as lipoic acid.

Fatty acid chains differ greatly in the length of their chains and may be categorized aaccording to chain length, e.g. as short to very long.

Short-chain fatty acids (SCFA) are fatty acids with chains of about five or less carbons (e.g. butyric acid). In some embodiments, each of the fatty acids is independently a SCFA. In some embodiments, one of the fatty acids is independently a SCFA.

Medium-chain fatty acids (MCFA) include fatty acids with chains of about 6-12 carbons, which can form medium-chain triglycerides. In some embodiments, each of the fatty acids is independently a MCFA. In some embodiments, one of the fatty acids is independently a MCFA.

Long-chain fatty acids (LCFA) include fatty acids with chains of 13-21 carbons. In some embodiments, each of the fatty acids is independently a LCFA. In some embodiments, one of the fatty acids is independently a LCFA.

Very long chain fatty acids (VLCFA) include fatty acids with chains of 22 or more carbons, such as 22-60, 22-50, or 22-40 carbons. In some embodiments, each of the fatty acids is independently a VLCFA. In some embodiments, one of the fatty acids is independently a VLCFA.

In some embodiments, one of the fatty acids is independently a MCFA and one is independently a LCFA.

In certain embodiments, a provided exosome loaded with a therapeutic agent is useful for oral delivery of the therapeutic agent.

In other embodiments, the therapeutic agent can be used for diagnoses and prognosis of disease and measuring response to treatment. In another embodiment, following the administration of a therapeutic-loaded exosome (for example, a therapeutic-loaded milk-derived exosome), processing by or interaction with particular cell types yields markers that may be assessed through means known in the art to provide a diagnosis or prognosis or measure a response to treatment.

A variety of therapeutic agents are compatible with encapsulation in a microvesicle according to the present invention. In some embodiments, the therapeutic agent is a biologic. In some embodiments, the biologic is selected from an iRNA, siRNA, miRNA, mRNA, ncRNA, or other oligonucleotide therapeutic. In some embodiments, the biologic is selected from a hormone (for example, a growth hormone, parathyroid hormone, or insulin, or another substance, for example a peptide or steroid, produced by one tissue and conveyed by the bloodstream to another to effect physiological activity, such as growth or metabolism); an interferon (for example, a protein that is normally produced by cells in response to viral infection and other stimuli); an interleukin (such as a cytokine protein, e.g. such as are involved in directing other immune cells to divide and differentiate; a growth factor (for example, a substance such as a vitamin $B_{12}$ or an interleukin that promotes growth, for example cellular growth); a monoclonal antibody (mAb); a polypeptide, such as a peptide containing ten or more amino acids but less than 50; a protein, such as a protein containing 50 or more amino acids, or a protein having a mass from about 10 kD to about 30 kD, or about 30 kD to about 150 or to about 300 kD; a vaccine; a diagnostic; an antithrombolytic; a toxin; or an antitoxin.

In some embodiments, the biologic therapeutic agent is not naturally-occurring in the milk-derived microvesicle, i.e., the biologic is not among the endogenous proteins, nutrients, vitamins, other small molecules, or nucleic acids found in or associated with the milk-derived microvesicle in its natural environment. In some embodiments, the therapeutic agent is naturally-occurring in the milk-derived microvesicle and the milk-derived microvesicle is isolated, manipulated, or optimized for delivery of the therapeutic agent to a patient in need thereof, or the amount of the therapeutic agent is enriched relative to the amount that is naturally-occurring in a given sample of milk microvesicles. Examples of naturally-occurring proteins and other agents found naturally in milk-derived microvesicles include CD63, Transferrin receptor, sialic acid, mucins, Tsg101 (Tumor susceptibility gene 101), Alix, annexin II, EFla (Translation elongation factor la), CD82 (Cluster of Differentiation 82), ceramide, sphingomyelin, lipid raft markers, and PRNP (PRioN Protein).

A number of therapeutic agents are suitable for loading in microvesicles in accordance with the present invention.

More specifically, the present invention provides the following lipid-modified double-stranded RNA that may be

loaded in and delivered by the exosomes described herein. In some embodiments, the RNA is one of those described in CA 2581651 or US 8,138,161, each of which is hereby incorporated by reference in its entirety.

In some embodiments, the RNA is an siRNA molecule comprising a modified ribonucleotide, wherein said siRNA (a) comprises a two base deoxynucleotide "TT" sequence at its 3' end, (b) is resistant to RNase, and (c) is capable of inhibiting viral replication.

In some embodiments, the siRNA molecule is 2' modified. In some embodiments, the 2' modification is selected from the group consisting of fluoro-, methyl-, methoxyethyl- and propyl-modification. In some embodiments, the fluoro-modification is a 2'-fluoro-modification or a 2',2'-fluoro-modification.

In some embodiments, at least one pyrimidine of the siRNA is modified, and said pyrimidine is cytosine, a derivative of cytosine, uracil, or a derivative of uracil. In some embodiments, all of the pyrimidines in the siRNA are modified. In some embodiments, both strands of the siRNA contain at least one modified nucleotide. In some embodiments, the siRNA consists of about 10 to about 30 ribonucleotides. In some embodiments, the siRNA molecule is consists of about 19 to about 23 ribonucleotides.

In some embodiments, the siRNA molecule comprises a nucleotide sequence at least 80% identical to the nucleotide sequence of siRNA5, siRNAC1, siRNAC2, siRNA5B1, siRNA5B2 or siRNA5B4. In some embodiments, the siRNA molecule is linked to at least one receptor-binding ligand. In some embodiments, the receptor-binding ligand is attached to a 5'-end or 3'-end of the siRNA molecule. In some embodiments, the receptor binding ligand is attached to multiple ends of said siRNA molecule. In some embodiments, the receptor-binding ligand is selected from the group consisting of a cholesterol, an HBV surface antigen, and low-density lipoprotein. In some embodiments, the receptor-binding ligand is cholesterol.

In some embodiments, the siRNA molecule comprises a modification at the 2' position of at least one ribonucleotide, which modification at the 2' position of at least one ribonucleotide renders said siRNA resistant to degradation. In some embodiments, the modification at the 2' position of at least one ribonucleotide is a 2'-fluoro-modification or a 2',2'-fluoro-modification.

In an embodiment, the invention provides a double-stranded (dsRNA) molecule that mediates RNA interference in target cells wherein one or more of the pyrimidines in the dsRNA are modified to include a 2'-Fluorine.

In an embodiment, the invention provides a small interfering RNA (siRNA) that mediates RNA interference in target cells wherein one or more of the pyrimidines in the siRNA are modified to include a 2'-Fluorine.

In an embodiment, all of the pyrimidines in the dsRNA or siRNA molecules of the first and second embodiments are modified to include a 2'-Fluorine.

In an embodiment, the 2'-Fluorine dsRNA or siRNA of the third embodiment is further modified to include a two base deoxynucleotide "TT" sequence at the 3' end of the dsRNA or siRNA.

In an embodiment, there is provided a method of preparing an siRNA comprising the steps of:

(a) identifying a target nucleotide sequence in an HCV genome for designing a siRNA; and

(b) producing an siRNA that contains at least one pyrimidine in the siRNA which is modified to include a 2'-Fluorine.

In an embodiment, there is provided a method of preparing an siRNA comprising the steps of:

(a) identifying a target nucleotide sequence in an HCV genome for designing a siRNA; and
(b) producing an siRNA wherein all of the pyrimidines in the siRNA are modified to include a 2'-Fluorine.

In an embodiment, there is provided a method of preparing an siRNA comprising the steps of:

(a) identifying a target nucleotide sequence in an HCV genome for designing a siRNA; and
(b) producing an siRNA wherein all of the pyrimidines in the siRNA are modified to include a 2'-Fluorine and wherein the 2'-Fluorine siRNA is further modified to include a two base deoxynucleotide "TT" sequence at the 3' end of the dsRNA or siRNA.

In an embodiment, there is provided a dsRNA molecule of from about 10 to about 30 nucleotides that inhibits replication of HCV, wherein said dsRNA contains at least one pyrimidine in the siRNA which is modified to include a 2'-Fluorine.

In an embodiment, there is provided a dsRNA molecule of from about 10 to about 30 nucleotides that inhibits replication of HCV, wherein all of the pyrimidines in the dsRNA are modified to include a 2'-Fluorine.

In an embodiment, there is provided a dsRNA molecule of from about 10 to about 30 nucleotides that inhibits replication of HCV, wherein all of the pyrimidines in the dsRNA are modified to include a 2'-Fluorine and wherein the 2'-Fluorine dsRNA is further modified to include a two base deoxynucleotide "TT" sequence at the 3' end of the dsRNA.

In some embodiments, the siRNA molecule is about 10 to about 30 nucleotides long, and mediates RNA interference in target cells. In some embodiments, the siRNA molecules are chemically modified to confer increased stability against nuclease degradation, but retain the ability to bind to target nucleic acids.

A modified siRNA of the present invention comprises a modified ribonucleotide, and is resistant to enzymatic degradation, such as RNase degradation, yet retains the ability to inhibit viral replication in a cell containing the specific viral target RNA or DNA sequences. The siRNA may be modified at any position of the molecule so long as the modified siRNA binds to a target sequence and is resistant to enzymatic degradation. Modifications in the siRNA may be in the nucleotide base, i.e., the purine or the pyrimidine, the ribose or the phosphate. Preferably, the modification occurs at the 2' position of at least one ribose in an siRNA.

More specifically, the siRNA is modified in at least one pyrimidine, at least one purine or a combination thereof. However, generally all pyrimidines (cytosine or uracil), or all purines (adenosine or guanine) or a combination of all pyrimidines and all purines of the siRNA are modified. In some embodiments, the pyrimidines are modified, and these pyrimidines are cytosine, a derivative of cytosine, uracil, a derivative of uracil or a combination thereof. Ribonucleotides on either one or both strands of the siRNA may be modified.

Ribonucleotides containing pyrimidine bases found in RNA (cytidine and uridine) can be chemically modified by adding any molecule that inhibits RNA degradation or breakdown of the base, the ribose or the phosphates. As previously noted, the 2' position of ribose is a preferred site for modification. 2' modified siRNAs have a longer serum half-life and are resistant to degradation, relative to unmodified siRNAs or single-stranded RNAs, such as antisense or ribozyme. 2'-modified pyrimidine ribonucleotides can be formed by a number of different methods known in the art.

One particular chemical modification is the addition of a molecule from the halide chemical group to a ribonucleotide of siRNA. In some embodiments, the halide is fluorine. Besides fluorine, other chemical moieties such as methyl-, methoxyethyl- and propyl- may be added as modifications. The fluoro-modification includes in certain embodiments a 2'-fluoro-modification or a 2',2'-fluoro-modification.

Thus, in a preferred embodiment of the invention, siRNA is modified by the addition of a fluorine to the 2' carbon of a pyrimidine ribonucleotide. The siRNA may be fluorinated completely or partially. For example, only the cytosine ribonucleotides may be fluorinated. Alternatively, only the uracil ribonucleotides may be fluorinated. In some embodiments, both uracil and cytosine are fluorinated. Only one strand, either sense or antisense, of siRNA may be fluorinated. Even partial 2' fluorination of siRNA gives protection against nucleolytic degradation. Importantly, 2' fluorinated siRNA is not toxic to cells.

siRNA can be prepared in a number of ways, such as by chemical synthesis, T7 polymerase transcription, or by treating long double stranded RNA (dsRNA) prepared by one of the two previous methods with Dicer enzyme. Dicer enzyme creates mixed populations of dsRNA from about 21 to about 23 base pairs in length from dsRNA that is about 500 base pairs to about 1000 base pairs in size. Unexpectedly, Dicer can effectively cleave modified strands of dsRNA, such as 2' fluoro-modified dsRNA. Before development of this method, it was previously thought that Dicer would not be able to cleave modified siRNA. The Dicer method of preparing siRNAs can be performed using a Dicer siRNA Generation Kit available from Gene Therapy Systems (San Diego, Calif.).

The invention particularly includes a method of making a modified siRNA that targets a nucleic acid sequence in a virus, comprising (a) preparing a modified-double stranded RNA (dsRNA) fragment containing at least one modified ribonucleotide in at least one strand, and (b) cleaving the modified-dsRNA fragments with recombinant human Dicer, resulting in more than one modified siRNA. The method may further comprise (c) isolating the modified siRNAs.

In the methods for making siRNA, a dsRNA fragment can be prepared by chemical synthesis or in vitro translation. In one embodiment, the modified siRNA is a 2' modified siRNA in which the modification is at the 2' position of at least one ribonucleotide of said siRNA. The modification is selected from the group consisting of fluoro-, methyl-, methoxyethyl and propyl-modification. Preferably the fluoro-modification is a 2'-fluoro-modification or a 2',2'-fluoro-modification. The pyrimidines, the purines or a combination thereof of the siRNA are modified. In some embodiments, the pyrimidines are modified, such as cytosine, a derivative of cytosine, uracil, a derivative of uracil or a combination thereof. One or both strands of the siRNA may contain one or more modified ribonucleotides.

In some embodiments, the method of inactivating a virus utilizes an siRNA that is modified at the 2' position of at least one ribonucleotide of said siRNA. The siRNA may be modified with chemical groups selected from the group consisting of fluoro-, methyl-, methoxyethyl- and propyl-. Fluoro-modification includes a 2'-fluoro-modification or a 2',2'-fluoro-modification. The modification may be at a pyrimidine, a purine or a combination thereof of the siRNA. In some embodiments the pyrimidines are modified, such as cytosine, a derivative of cytosine, uracil, a derivative of uracil or a combination thereof. In one embodiment, one strand of the siRNA contains at least one modified ribonucleotide, while in another embodiment, both strands of the siRNA contain at least one modified ribonucleotide.

siRNAs useful in treatment methods may also be modified by the attachment of at least one, but preferably more than one, receptor-binding ligand(s) to the siRNA. Such ligands are useful to direct delivery of siRNA to a target virus in a body system, organ, tissue or cells of a patient, such as the liver, gastrointestinal tract, respiratory tract, the cervix or the skin.

In preferred embodiments, receptor-binding ligands are attached to either a 5'-end or a 3'-end of an siRNA molecule. Receptor-binding ligands may be attached to one or more siRNA ends, including any combination of 5'- and 3'-ends. Thus, when receptor binding ligands are attached only to the ends of an siRNA molecule, anywhere between 1 and 4 such ligands may be attached.

Selection of an appropriate ligand for targeting siRNAs to viruses in particular body systems, organs, tissues or cells may be made. For example, to target an siRNA to hepatocytes, cholesterol may be attached at one or more ends, including any combination of 5'- and 3'-ends, of an siRNA molecule. The resultant cholesterol-siRNA is delivered to hepatocytes in the liver, thereby providing a means to deliver siRNAs to this targeted location. Other ligands useful for targeting siRNAs to the liver include HBV surface antigen and low-density lipoprotein (LDL).

Modified siRNA can be prepared by chemical synthesis. In one embodiment, each C and U within a siRNA duplex, e.g. GL2, can be substituted with 2'-F-U and 2'-F-C. To produce siRNA with 3'-end overhangs comprising 2'-F-U and 2'F-C, a universal support can be used. By selectively cleaving the oligo from the support, a practitioner can ensure that residues of the overhangs comprise modified nucleotides. Alternatively, the nucleotides comprising the 3'-end overhang can be unmodified dTdT.

2'-F RNA oligonucleotides can be synthesized on an Applied Biosystems 8909 or 8905 DNA/RNA synthesizer using the standard 1 μmol beta-cyanoethyl phosphoramidite RNA chemistry protocol. The RNA phosphoramidite monomers and columns of Pac-A, 2'-F-Ac-C, iPr-Pac-G, 2'-F-U, and U-RNA CPG can be obtained from Glen Research (Sterling, Va.). (See catalog nos. 10-3000-05, 10-3415-02, 10-3021-05, 10-3430-02, and 20-3430-41E, respectively.) Glen Research's Sulfurizing Reagent (catalog no. 404036-10) can be used as an oxidant to obtain a single phosphorothioate backbone between the 3' CPG and a subsequent base. To attain the coupling, the oxidizing step of the standard RNA 1 μmol protocol can be replaced with the standard thioate 1 μmol protocol. Cholesteryl-TEG phosphoramidite (Glen Research, catalog no. 10-1975-90) and cholesteryl-TEG CPG (Glen Research, catalog no. 20-2975-41E) can be incorporated onto the 5' or 3' ends of one or more of the oliogoribonucleotides. After synthesis, the 2'-F RNA's are cleaved and deprotected with 1:1 ammonium hydroxide/methylamine, and the silyl groups are removed with triethylamine trihydrofluoride using standard protocols. See e.g. http://www.glenres.com/productfiles/technical/tb_rnadeprotection.pdf. The oligoribonucleotides are then desalted on Sephadex G25 columns (Pharmacia NAP 25, catalog no. 17-08252-02) with sterilized water and purified using standard gel electrophoresis protocols. Modified siRNAs also can be obtained from commercial vendors such as Dharmacon (Lafayette, Colo.).

Alternatively, modified siRNA can be prepared by transcription using the Durascribe T7 Transcription Kit purchased from Epicentre Technologies (Madison, Wis.).

Two exemplary modified siRNAs are provided below:

Chol-GL2 Chol-CGUACGCGGAAUACUUCGAUU
UUGCAUGCGCCUUAUGAAGCU

GL2 CGUACGCGGAAUACUUCGAUU
UUGCAUGCGCCUUAUGAAGCU

The present invention also provides the following lipid-modified double-stranded RNA that may be loaded into and delivered by the exosomes described herein. In some embodiments, the RNA is one of those described in EP 2264167 or US 9,040,492, the entirety of each of which is hereby incorporated by reference.

In some embodiments, the RNA is a double-stranded lipid-modified RNA comprising a sense strand having a nucleotide sequence complementary to a target sequence in a target gene, and an antisense strand having a nucleotide sequence complementary to the sense strand, the double-stranded RNA being capable of suppressing expression of the target gene, and the sense strand having a double-stranded lipid bound directly or via a linker to at least one of the first to sixth nucleotides from the 5' end.

In some embodiments, the RNA is blunt-ended on the 5'-end side of the sense strand, and is blunt-ended or has a dangling end on the 3'-end side of the sense strand.

In some embodiments, the RNA is a double-stranded lipid-modified RNA having dangling ends on both the 5'- and 3'-end sides of the sense strand. In some embodiments, the RNA has a sense strand consisting of 21 to 27 nucleotides. In some embodiments, the RNA is blunt-ended on both the 5'- and 3'-end sides of the sense strand, each of the sense and antisense strands consisting of 27 nucleotides. In some embodiments, the RNA is blunt-ended on both the 5'- and 3'-end sides of the sense strand, each of the sense and antisense strands consisting of 23 nucleotides. In some embodiments, the RNA is blunt-ended on the 5'-end side of the sense strand, the sense strand consisting of 25 nucleotides, and the antisense strand consisting of 23 nucleotides. In some embodiments, each of the sense and antisense strands consists of 21 nucleotides.

In some embodiments, two hydrophobic groups of the double-stranded lipid are the same or different, and each is a saturated or unsaturated fatty acid residue having 6 to 50 carbon atoms. In some embodiments, the double-stranded lipid is a glycerophospholipid, glyceroglycolipid, diacylglycerol, or ceramide. In some embodiments, the double-stranded lipid is glycerophospholipid. In some embodiments, the double-stranded lipid is phosphatidylethanolamine. In some embodiments, the double-stranded lipid is at least one of dimyristoylphosphatidylethanolamine, dipalmitoylphosphatidylethanolamine, 1-palmitoyl-2-oleyl-phosphatidylethanolamine, or dioleoylphosphatidylethanolamine.

In some embodiments, the lipid is bound to at least one of the first to sixth nucleotides from the 5' end of the sense strand via a linker represented by the formula (L-27)

[Chem. 1]

$$-CO-(CH2)n3-CO-NH-(CH2)n4- \qquad (L-27)$$

wherein n3 and n4 are the same or different and each represents an integer of 1 to 20.

The double-stranded lipid-modified RNA of the present invention is modified with a double-stranded lipid on the 5'-end side of the sense strand. Based on this structural feature, the double-stranded lipid-modified RNA has a significantly increased RNA interference effect. In particular, because the double-stranded lipid-modified RNA of the present invention has a double-stranded lipid bound to a specific site, a remarkably enhanced nuclease resistance and RNA interference effect are provided without impairing Dicer processing or the RNA's ability to form a complex with RISC, thus greatly contributing to its medicinal applications.

The double-stranded lipid-modified RNA of the invention comprises an antisense strand having a nucleotide sequence complementary to the sense strand.

When the double-stranded lipid-modified RNA of the invention has no dangling end on the antisense strand, the antisense strand consists of a nucleotide sequence complementary to a part or all of the "nucleotide sequence complementary to a target sequence" of the sense strand. When a dangling end is present at the 5' end and/or at the 3' end of the antisense strand, the antisense strand consists of a nucleotide sequence complementary to a part or all of the "nucleotide sequence complementary to a target sequence" of the sense strand, and a dangling end nucleotide sequence linked to the 5' end and/or the 3' end of the complementary nucleotide sequence of the sense strand.

Insofar as the RNA interference effect can be produced, the number of nucleotides that constitute the antisense strand in the double-stranded lipid-modified RNA of the invention is not particularly limited, and can be suitably selected according to the desired structure of the double-stranded RNA, etc. The number of the nucleotides is typically 21 to 27, preferably 21, 23, 25, or 27, and more preferably 21, 23, or 27. When no dangling end is present on the antisense strand, the number of nucleotides that constitute the antisense strand, as used herein, refers to the total number of nucleotides constituting the nucleotide sequence complementary to the nucleotide sequence of the target sequence. When a dangling end is present on the antisense strand, the number of nucleotides that constitute the antisense strand refers to the sum of the number of nucleotides constituting the dangling end, and the number of nucleotides constituting the nucleotide sequence complementary to the nucleotide sequence of the target sequence.

The nucleotides that constitute the sense strand and antisense strand of the double-stranded lipid-modified RNA of the invention are mainly ribonucleotides. To enhance resistance to enzymatic digestion, the RNA sequence may further include various chemically modified nucleotides, such as 2'-O-methyl-modified nucleotides, 2'-F-modified nucleotides, LNA (Locked Nucleic Acid) nucleotides, or deoxyribonucleotides. Particularly, when the double-stranded lipid-modified RNA of the invention has a dangling end, the dangling end of the sense strand and/or the antisense strand may be composed of deoxyribonucleotides. Examples of such chemically modified nucleotides include phosphate backbone-modified nucleotides such as phosphorothioate-modified DNA/RNA and boranophosphate-modified DNA/RNA; 2'-modified nucleotides such as 2"-OMe-modified RNA and 2'-F-modified RNA; modified nucleotides obtained by crosslinking the sugar molecule of a nucleotide, such as LNA (Locked Nucleic Acid) and ENA (2'-O,4'-C-ethylene-bridged nucleic acids); modified nucleotides having different backbones, such as PNA (Peptide Nucleic Acid) and morpholine-nucleotide; base-modified nucleotides such as 5-fluorouridine and 5-propyluridine; and the like.

The structure of the double-stranded lipid-modified RNA of the invention is not particularly limited, insofar as the sense and antisense strands are hybridized into a double strand. For examples, the following structures are preferable: structure 1.(A) in which the double-stranded RNA is blunt-ended (i.e., has a blunt end) on the 5'-end side of the sense strand, and is blunt-ended or has a dangling end (a single-stranded region or a projection) on the 3'-end side of the sense strand; and structure 2.(B) in which the double-stranded RNA has dangling ends on both the 5'- and 3'-end sides of the sense strand. Based on the above structure (A) or (B), the double-stranded lipid-modified RNA can maintain its RNA interference effect, although modified with a double-stranded lipid, and also has remarkably enhanced cellular uptake efficiency. The structure of "having a dangling end on the 3'-end side of the sense strand," as used herein, includes both of the following cases: the case in which the 3'-end region of the sense strand forms a dangling end; and the case in which the 5'-end region of the antisense strand forms a dangling end. The structure of "having a dangling end on the 5'-end side of the sense strand," as used herein, includes both of the following cases: the case in which the

5'-end region of the sense strand forms a dangling end; and the case in which the 3'-end region of the antisense strand forms a dangling end.

To provide a particularly excellent RNA interference effect, for example, the following structures of the double-stranded RNA of the double-stranded lipid-modified RNA of the invention are particularly preferable among the above structures (A) and (B): structure (A-1) in which the double-stranded RNA is blunt-ended on both the 5'- and 3'-end sides of the sense strand, and each of the sense and antisense strands consists of 27 nucleotides; structure (A-2) in which the double-stranded RNA is blunt-ended on both the 5'- and 3'-end sides of the sense strand, and each of the sense and antisense strands consists of 23 nucleotides; structure (A-3) in which the double-stranded RNA is blunt-ended on the 5'-end side of the sense strand, and the sense strand consists of 25 nucleotides, and the antisense strand consists of 23 nucleotides; and structure (B-1) in which the double-stranded RNA has two-nucleotide dangling ends at both 3' ends of the sense and antisense strands, and each of the sense and antisense strands consists of 21 nucleotides.

More specifically, in structures (A-1) and (A-2), the sense and antisense strands are hybridized without forming any dangling ends at the ends. In structure (A-3), the sense and antisense strands are hybridized in such a manner that the double-stranded RNA is blunt-ended on the 5'-end side of the sense strand, and the first and second nucleotides from the 3' end of the sense strand form a dangling end. In structure (B-1), the first to 19th nucleotides from the 5' end of the sense strand and the third to 21st nucleotides from the 3' end of the antisense strand are hybridized in such a manner that the first and second nucleotides from the 3' end of the sense strand form a dangling end, and the first and second nucleotides from 3' end of the antisense strand form a dangling end.

According to one embodiment of the double-stranded lipid-modified RNA provided by the present invention, a lipid is bound to at least one of the first to sixth nucleotides from the 5' end of the sense strand. In some embodiments, the double-stranded lipid-modified RNA of the invention has no substitutents bound to any position other than the 5'-end region of the sense strand. More specifically, in some embodiments, no portions of the sense strand other than the 5'-end region and the antisense strand have substituents, and these portions only consist of nucleotides. The binding of a lipid only to the 5'-end region of the sense strand enhances cellular uptake efficiency and can also remarkably increase the RNA interference effect. More specifically, in some embodiments of the double-stranded lipid-modified RNA of the present invention, a double-stranded RNA structure, the use of a double-stranded lipid to modify the double-stranded RNA, and the binding site of the double-stranded lipid are structural features that are inseparably related. Based on these structural features, the double-stranded lipid-modified RNA of the invention has excellent cellular uptake efficiency and nuclease resistance, and can produce a remarkably increased RNA interference effect.

In some embodiments of the double-stranded lipid-modified RNA of the invention, the double-stranded lipid bound to the sense strand is not particularly limited, insofar as the lipid has two hydrophobic groups. Examples of the double-stranded lipid include lipids having at least two hydrophobic groups selected from the group consisting of C6-50 saturated fatty acid residues and C6-50 unsaturated fatty acid residues. Each of the saturated fatty acid residue and the unsaturated fatty acid residue preferably has 8 to 30 carbon atoms, and more preferably 10 to 24 carbon atoms. More specifically, examples of hydrophobic groups of the lipid include fatty acid residues such as capric acid, lauric acid, myristic acid, palmitic acid, stearic acid, arachidic acid, behenic acid, lignoceric acid, myristoleic acid, palmitoleic acid, oleic acid, elaidic acid, vaccenic acid, erucic acid, gadoleic acid, linoleic acid, linolenic acid, and arachidonic acid. In some embodiments, at least one fatty acid residue selected from myristic acid, palmitic acid, stearic acid, and oleic acid may be used as the two hydrophobic groups of the double-stranded lipid in the present invention.

Examples of double-stranded lipids that can be used in the present invention include glycerophospholipid, glyceroglycolipid, diacylglycerol, ceramide, and the like. To further enhance the nuclease resistance, cellular uptake efficiency, and RNA interference effect, glycerophospholipid can be preferably used.

The glycerophospholipid that can be used in the present invention is not particularly limited. Examples of usable glycerophospholipid include phosphatidylethanolamine, phosphatidylglycerol, phosphatidylserine, phosphatidic acid, and phosphatidylinositol, etc.

Examples of phospholipids that can be used in the present invention include phosphatidylethanolamines, such as dilauroylphosphatidylethanolamine, dimyristoylphosphatidylethanolamine, dipalmitoylphosphatidylethanolamine, distearoylphosphatidylethanolamine, dioleoylphosphatidylethanolamine, 1-palmitoyl-2-oleylphosphatidylethanolamine, 1-oleyl-2-palmitoylphosphatidylethanolamine, and dierucoylphosphatidylethanolamine; phosphatidylglycerols, such as dilauroylphosphatidylglycerol, dimyristoylphosphatidylglycerol, dipalmitoylphosphatidylglycerol, distearoylphosphatidylglycerol, dioleoylphosphatidylglycerol, 1-palmitoyl-2-oleyl-phosphatidylglycerol, 1-oleyl-2-palmitoyl-phosphatidylglycerol, and dierucoylphosphatidylglycerol; phosphatidylserines, such as dilauroylphosphatidylserine, dimyristoylphosphatidylserine, dipalmitoylphosphatidylserine, distearoylphosphatidylserine, dioleoylphosphatidylserine, 1-palmitoyl-2-oleyl-phosphatidylserine, 1-oleyl-2-palmitoyl-phosphatidylserine, and dierucoylphosphatidylserine; phosphatidic acids, such as dilauroylphosphatidic acid, dimyristoylphosphatidic acid, dipalmitoylphosphatidic acid, distearoylphosphatidic acid, dioleoylphosphatidic acid, 1-palmitoyl-2-oleylphosphatidic acid, 1-oleyl-2-palmitoyl-phosphatidic acid, and dierucoylphosphatidic acid; and phosphatidylinositols, such as dilauroylphosphatidylinositol, dimyristoylphosphatidylinositol, dipalmitoylphosphatidylinositol, distearoylphosphatidylinositol, dioleoylphosphatidylinositol, 1-palmitoyl-2-oleyl-phosphatidylinositol, 1-oleyl-2-palmitoyl-phosphatidylinositol, and dierucoylphosphatidylinositol. To provide more remarkable nuclease resistance, cellular uptake efficiency, and a more remarkable RNA interference effect, phosphatidylethanolamines may be used. More preferably, dimyristoylphosphatidylethanolamine, dipalmitoylphosphatidylethanolamine, 1-palmitoyl-2-oleyl-phosphatidylethanolamine, and dioleoylphosphatidylethanolamine can be used.

The manner of binding the double-stranded lipid to the sense strand in the double-stranded lipid-modified RNA of the invention is not particularly limited. The lipid and the sense strand may be bound directly or via a linker (a linkage region). The linker used to bind the lipid to the sense strand does not comprise a nucleic acid.

The linker that can be used is not particularly limited insofar as the lipid and the sense strand are linked therethrough. Examples of usable linkers include those of the following structures:

[Chem. 2]

-O-CO-O- (L-1)

-NH-CO-O (L-2)

-NH-CO-NH- (L-3)

-NH-(CH$_2$)$_{n1}$- (L-4)

-S-(CH$_2$)$_{n1}$- (L-5)

-CO-(CH$_2$)$_{n1}$-CO- (L-6)

-CO-(CH$_2$)$_{n1}$-NH- (L-7)

-NH-(CH$_{2)n1}$-NH- (L-8)

-CO-NH-(CH$_2$)$_{n1}$-NH-CO- (L-9)

-C(=S)-NH-(CH$_2$)$_{n1}$-NH-CO- (L-10)

-C(=S)-NH-(CH$_{2)n1}$-NH-C-(=S)- (L-11)

-CO-O-NH-(CH$_{2)n1}$-O-CO- (L-12)

-C(=S)-O-(CH$_{2)n1}$-O-CO- (L-13)

-C(=S)-O-(CH$_{2)n1}$-O-C-(=S)- (L-14)

-CO-NH-(CH$_{2)n1}$-O-CO- (L-15)

-C(=S)-NH-(CH$_{2)n1}$-O-CO (L-16)

-C(=S)-NH-(CH$_{2)n1}$-O-C-(=S)- (L-17)

-C-NH-(CH$_{2)n1}$-O-CO- (L-18)

-C(=S)-NH-(CH$_{2)n1}$-CO- (L-19)

-C(=S)-O-(CH$_{2)n1}$-NH-CO- (L-20)

-C(=S)-NH-(CH$_{2)n1}$-O-C-(=S)- (L-21)

-NH-(CH$_2$CH$_2$O)$_{n2}$-CH-(CO$_2$OH)- (L-22)

-NH-(CH$_2$CH$_2$O$_{)n2}$-CH$_2$- (L-23)

-NH-(CH$_2$CH$_2$O)$_{n2}$-CH$_2$-CO (L-24)

$$-O-(CH_2)n_3-S-S-(CH_2)_{n4}-O-P(=O)_2- \quad (L-25)$$

$$-CO-(CH_2)_{n3}-O-CO-NH-(CH_2)_{n4}- \quad (L-26)$$

$$-CO-(CH_2)_{n3}-CO-NH-(CH_2)_{n4}- \quad (L-27)$$

In formulas (L-4) to (L-21), n1 is an integer of 1 to 40, preferably 2 to 20, and more preferably 2 to 12.

In formulas (L-22) to (L-24), n2 is an integer of 1 to 20, preferably 1 to 10, and more preferably 1 to 6.

In formulas (L-25) to (L-27), n3 and n4 may be the same or different, and are an integer of 1 to 20, preferably 1 to 10, and more preferably 1 to 6.

Single-stranded DNA may be bound to either the left or right side of the linkers of formulas (L-1) to (L-27). Preferably, a double-stranded lipid is bound to the left side of the linker, and the 5'-end region of the sense strand of a double-stranded RNA is bound to the right side thereof.

The binding site of the double-stranded lipid and the linker may be suitably selected according to the types of double-stranded lipid and linker. Any position other than hydrophobic groups of the double-stranded lipid may be linked to the linker by a chemical bond. For example, when using a phosphatidylethanolamine, the linkage may be made by forming an amide bond, etc. between the amino group of phosphatidylethanolamine and the linker. When using a phosphatidylglycerol, the linkage may be made by forming an ester bond, an ether bond, etc. between the hydroxyl group of the glycerol residue and the linker. When using a phosphatidylserine, the linkage may be made by forming an amide bond or an ester bond, etc. between the amino group or carboxyl group of the serin residue and the linker. When using a phosphatidic acid, the linkage may be made by forming a phosphoester bond, etc. between the phosphate residue and the linker. When using a phosphatidylinositol, the linkage may be made by forming an ester bond, an ether bond, etc. between the hydroxyl group of the inositol residue and the linker.

The linker can be suitably selected according to the type of lipid to be linked. For example, when the double-stranded lipid is an amino group-containing phospholipid (e.g., phosphatidylethanolamine or phosphatidylserine), or a hydroxyl-containing phospholipid (e.g., phosphatidylglycerol or phosphatidylinositol), linkers of formulas (L-6), (L-7), (L-9), (L-10), (L-18), (L-26), and (L-27) are preferably used.

In addition to the above examples of linkers, other linkers such as N-succinimidyl-3-(2-pyridyldithio)propionate, N-4-maleimide butyric acid, S-(2-pyridyldithio)cysteamine, iodoacetoxysuccinimide, N-(4-maleimidebutyryloxy)succinimide, N-[5-(3'-maleimide propylamide)-1-carboxypentyl]iminodiacetic acid, N-(5-aminopentyl)iminodiacetic acid, and like bifunctional linkers (linkers containing two functional groups) are also usable.

The nucleotide of the sense strand to which either the double-stranded lipid or the linker used to link the double-stranded lipid is bound is not particularly limited, insofar as it is at least one of the first to sixth nucleotides from the 5' end of the sense strand. At least one of the first to fourth nucleotides from the 5' end is preferable. The first and/or second nucleotide from the 5' end are further preferable. The nucleotide at the 5' end (the first nucleotide from the 5' end) is particularly preferable.

The binding site of the sense strand to which the double-stranded lipid or the linker used for linking the lipid is bound is not particularly limited. The double-stranded lipid or the linker used for linking the double-stranded lipid is preferably bound to the sense strand by substitution of the hydrogen atom of the hydroxyl group of the phosphate portion of a specific nucleotide on the sense strand with the lipid or linker.

The number of double-stranded lipids bound to a double-stranded lipid-modified RNA of the invention is not particularly limited. For example, one to three double-stranded lipids, preferably one or two double-stranded lipids, and more preferably one double-stranded lipid may be bound.

In some embodiments, a double-stranded lipid-modified RNA of the invention can be produced by synthesizing each of the above-mentioned sense strand having at least one double-stranded lipid bound thereto and the above-mentioned antisense strand, and hybridizing the sense and antisense strands according to a known method. A known method can also be used to produce the sense strand having a double-stranded lipid linked thereto.

Alternatively, the double-stranded lipid-modified RNA of the present invention can also be produced by synthesizing the above-mentioned sense and antisense strands according to known methods, hybridizing the sense and antisense strands into a double-stranded RNA, and then linking a double-stranded lipid to the 5' end of the sense strand of the double-stranded RNA by a known synthetic technique.

More specifically, in some embodiments, the present invention provides the following complexes, sequences, and modified RNAs that may be loaded into and delivered by the exosomes described herein. In some embodiments, the RNA comprises a complex or RNA sequence or modified RNA sequence disclosed in US 9,320,814, the entirety of which is hereby incorporated by reference.

In some embodiments, the complex comprises: a) a short nucleic acid molecule linked to a hydrophobic moiety, wherein said short nucleic acid molecule comprises less than about 50 nucleotides, wherein said short nucleic acid

molecule is an siRNA molecule, wherein said hydrophobic moiety is cholesterol; and b) a linear block copolymer consisting of at least one cationically charged polymeric segment and at least one hydrophilic polymeric segment, wherein said cationically charged polymeric segment consists of about 30 to about 50 lysines, wherein said hydrophilic polymeric segment comprises poly(ethylene oxide).

In some embodiments, the complex comprises a cationically charged polymeric segment consisting of about 30 lysines. In some embodiments, the complex comprises a hydrophobic moiety linked to the 3' end of the sense strand of the siRNA molecule. In some embodiments, the hydrophobic moiety is linked directly to the nucleic acid molecule or linked via a linker.

In some embodiments, the complex comprises at least one therapeutic agent or detectable agent.

In some embodiments, the complex comprises: a) a short nucleic acid molecule linked to a hydrophobic moiety, wherein said short nucleic acid molecule comprises less than about 50 nucleotides, wherein said short nucleic acid molecule is an siRNA molecule, wherein said hydrophobic moiety is cholesterol; and b) a linear block copolymer consisting of at least one cationically charged polymeric segment, at least one hydrophilic polymeric segment, and a targeting ligand, wherein said cationically charged polymeric segment consists of about 30 to about 50 lysines, wherein said hydrophilic polymeric segment comprises poly(ethylene oxide).

In some embodiments, the complex comprises comprises at least one short nucleic acid molecule linked (either directly or via a linker) to a hydrophobic moiety and at least one block copolymer comprising a cationically charged polymeric segment and a hydrophilic polymeric segment. The short nucleic acid molecule may be an inhibitory nucleic acid molecule such as an antisense molecule, siRNA, shRNA, DsiRNA, or miRNA. In a particular embodiment, the hydrophobic moiety is cholesterol. In a particular embodiment, the hydrophilic polymeric segment comprises poly(ethylene oxide) and the cationically charged polymeric segment comprises poly-lysine. The polyplexes of the instant invention may further comprise at least one other bioactive agent, such as a therapeutic agent.

I. Polyplexes

In some embodiments, the complex comprises at least one block copolymer and at least one nucleic acid molecule. The block copolymer comprises at least one cationically charged polymeric segment and at least one hydrophilic polymeric segment. In a particular embodiment, the block copolymer has the structure A-B or B-A. Typically, the block copolymer also comprises just the two blocks, but it may comprise more than 2 blocks. For example, the block copolymer may have the structure A-B-A, wherein B is a cationically charged polymeric segment. In a particular embodiment, the segments of the block copolymer comprise about 5 to about 500 repeating units, about 10 to about 300 repeating units, about 10 to about 250 repeating units, about 10 to about 200 repeating units, about 10 to about 150 repeating units, or about 10 to about 100 repeating units.

The cationically charged polymeric segment may comprise polymers and copolymers and their salts comprising units deriving from one or several monomers including, without limitation: primary, secondary and tertiary amines, each of which can be partially or completely quaternized forming quaternary ammonium salts. Examples of these monomers include, without limitation, cationic amino acids (e.g., lysine, arginine, histidine), alkyleneimines (e.g., ethyleneimine, propyleneimine, butyleneimine, pentyleneimine, hexyleneimine, and the like), spermine, vinyl monomers (e.g., vinylcaprolactam, vinylpyridine, and the like), acrylates and methacrylates (e.g., N,N-dimethylaminoethyl acrylate, N,N-dimethylaminoethyl methacrylate, N,N-diethylaminoethyl acrylate, N,N-diethylaminoethyl methacrylate, t-butylaminoethyl methacrylate, acryloxyethyltrimethyl ammonium halide, acryloxyethyl-dimethylbenzyl ammonium halide, methacrylamidopropyltrimethyl ammonium halide and the like), allyl monomers (e.g., dimethyl diallyl ammoniam chloride), and aliphatic, heterocyclic or aromatic ionenes. In a particular embodiment, the cationic polymeric segment comprises cationic amino acids, particularly poly-lysine. In a particular embodiment, the cationic polymeric segment of the block copolymer comprises about 5 to about 100 repeating units, about 10 to about 75 repeating units, about 10 to about 50 repeating units, about 20 to about 50 repeating units, about 20 to about 40 repeating units, or about 30 repeating units.

Examples of hydrophilic polymeric segments include, without limitation, polyetherglycols, poly(ethylene oxide), methoxy-poly(ethylene glycol), copolymers of ethylene oxide and propylene oxide, polysaccharides, polyvinyl alcohol, polyvinyl pyrrolidone, polyvinyltriazole, N-oxide of polyvinylpyridine, N-(2-hydroxypropyl)methacrylamide (HPMA), polyortho esters, polyglycerols, polyacrylamide, polyoxazolines, polyacroylmorpholine, and copolymers or derivatives thereof In a particular embodiment, the hydrophilic polymeric segment comprises poly(ethylene oxide).

The nucleic acid molecules of the polyplexes of the instant invention may be a short nucleic acid molecule such as a short inhibitory nucleic acid molecule (e.g., nucleic acid molecules which specifically hybridize (e.g., are complementary) with a target nucleic acid thereby inhibiting its expression; inhibitory nucleic acid molecules include antisense, siRNA, shRNA, DsiRNA (Dicer siRNA/Dicer-substrate RNA), miRNA (microRNA), etc.). The nucleic acid molecule may be single stranded or double stranded. The nucleic acid molecule may be DNA, RNA, or a mixture. In a particular embodiment, the nucleic acid molecule comprises less than about 100 nucleotides, particularly less than about 50 nucleotides or less than about 30 nucleotides. The nucleic acid molecule may be a probe. The nucleic acid molecules

may be conjugated (directly or via a linker) to one or more detectable labels (e.g., for diagnostic or detection methods). The nucleic acid molecules may also comprise at least one nucleotide analog. For example, the nucleotide analog may increase stability and/or resistance to nucleases. For example, the nucleic acid molecules may comprise, without limitation, Locked Nucleic Acid (LNA) bases, nucleotides with phosphate modifications (e.g., phosphorothioates, morpholinos, etc.), nucleotides with modified sugars (e.g., 2'-O-methylnucleotides), and nucleotide mimetics (e.g., peptide nucleic acids (PNA), etc.).

The nucleic acid molecules of the instant polyplexes are also conjugated to at least one hydrophobic moiety. The hydrophobic moiety may be conjugated to the nucleic acid molecule at the 5' and/or 3' end of either or both strands of the nucleic acid molecule. In a particular embodiment, the hydrophobic moiety is conjugated only to the 3' end, more particularly the 3' end of the sense strand in double stranded molecules. The hydrophobic moiety may be conjugated directly to the nucleic acid molecule or via a linker. The hydrophobic moiety may be selected from the group consisting of adamantane, cholesterol, steroid, long chain fatty acid, lipid, phospholipid, glycolipid, and derivatives thereof. The hydrophobic moiety may be a small molecule. In a particular embodiment, the nucleic acid molecules of the polyplex are conjugated to a cholesterol on the 3' end of the sense strand of the nucleic acid molecule.

Generally, a linker is a chemical moiety comprising a covalent bond or a chain of atoms that covalently attaches two compounds (e.g., the hydrophobic moiety to the nucleic acid molecule). The linker can be linked to any synthetically feasible position of the compounds. Exemplary linkers may comprise at least one optionally substituted; saturated or unsaturated; linear, branched or cyclic alkyl group or an optionally substituted aryl group. In a particular embodiment, the linker may contain from 0 (i.e., a bond) to about 500 atoms, about 1 to about 100 atoms, or about 1 to about 50 atoms. The linker may also be a polypeptide (e.g., from about 1 to about 5). The linker may be non-degradable and may be a covalent bond or any other chemical structure which cannot be substantially cleaved or cleaved at all under physiological environments or conditions.

The polyplexes of the instant invention may also be conjugated to a targeting ligand. A targeting ligand is a compound that will specifically bind to a specific type of tissue or cell type. In a particular embodiment, the targeting ligand is a ligand for a cell surface marker/receptor. The targeting ligand may be any molecule that selectively binds to a cell surface marker (e.g., protein of carbohydrate) preferentially or exclusively expressed on the targeted tissue or cell type (e.g., low molecular weight antagonist (e.g., less than 100 Da, particularly less than about 500 Da), an antibody or fragment thereof, aptamers, peptides, small molecules, etc. The targeting ligand may be linked directly to the polyplex or via a linker. In a particular embodiment, the targeting ligand is linked to the hydrophilic segment of the block copolymer (e.g., at the end).

The polyplexes of the instant invention may be synthesized by contacting at least one block copolymer with at least nucleic acid molecule. The opposite charges of the cationically charged segment of the block copolymer and the anionically charged nucleic acid molecule along with the presence of the hydrophilic segment of the block copolymer allow for self-assembly of the polyplexes in aqueous solutions. In a particular embodiment, the nucleic acid molecule and block copolymer are formed at molar N/P ratios that produce neutralized/electropositive polyplexes. In a particular embodiment, the N/P ratio is from about 1 to about 5. After complex formation, the polyplexes may be purified from non-complexed components by methods known in the art (e.g., size exclusion chromatography, centrifugal filtration, etc.). The resultant polyplexes typically have a diameter less than about 200 nm, particularly less than about 100 nm.

Polymers

BDNG, biodegradable nanogels (named "NG(PEGss)" in (Kohli et al. (2007) J. Control Rel., 121:19-27)) consisting of biodegradable PEI (28 kDa PEI formed from 2 kDa PEI via disulfide bonds) cross-linked with 8 kDa PEG through carbamate bonds, and PEI-PEG, polyethylenimine-g-poly(ethylene) glycol graft copolymer with a cationic block consisting of 2 kDa branched PEI (Sigma, St. Louis, Mo.) and a nonionic hydrophilic block consisting of 10 kDa PEG (Sigma, St. Louis, Mo.) (Vinogradov et al. (1998) Bioconjug. Chem., 9:805-12), may be employed in the foregoing embodiments. PLL10-PEG and PLL50-PEG, methoxy-poly(ethylene glycol)-b-poly(L-lysine hydrochloride) block copolymers with cationic blocks consisting of 10 (PLL10) or 50 (PLL50) poly-L-lysine groups and a nonionic hydrophilic block consisting of 5 kDa PEG may also be used. They may be purchased, for example, from Alamanda Polymers (Huntsville, Ala.).

More specifically, in some embodiments, the present invention provides the following lipid-modified double-stranded RNA that may be loaded into and delivered by the exosomes described herein. In some embodiments, the lipid-modified RNA is one of those disclosed in US 2010/0298411, the entirety of which is hereby incorporated by reference. In some embodiments, the RNA is a VEGF-targeting nucleic acid such as those described in US 2010/0298411, e.g. in Fig. 8 and Example 2 therein. In some embodiments, the RNA is selected from one of the following items.

Item 1. A lipid-modified double-stranded RNA comprising a sense strand having a nucleotide sequence complementary to a target sequence in a target gene, and an antisense strand having a nucleotide sequence complementary

to the sense strand, the double-stranded RNA being capable of inhibiting expression of the target gene, and the sense strand having a lipid linked to at least one of the first to sixth nucleotides from the 5' end directly or via a linker.
Item 2. A lipid-modified double-stranded RNA according to Item 1 which is blunt-ended on the 5' end side of the sense strand, and is blunt-ended or has a dangling end on the 3' end side of the sense strand.
Item 3. A lipid-modified double-stranded RNA according to Item 1 which has dangling ends on both the 5' and 3' end sides of the sense strand.
Item 4. A lipid-modified double-stranded RNA according to any one of Items 1 to 3 wherein the sense strand consists of 21 to 27 nucleotides.
Item 5. A lipid-modified double-stranded RNA according to Item 2 which is blunt-ended on both the 5' and 3' end sides of the sense strand, and in which each of the sense and antisense strands consists of 27 nucleotides.
Item 6. A lipid-modified double-stranded RNA according to Item 2 which is blunt-ended on both the 5' and 3' end sides of the sense strand, and in which each of the sense and antisense strands consists of 23 nucleotides.
Item 7. A lipid-modified double-stranded RNA according to Item 2 which is blunt-ended on the 5' end side of the sense strand, the sense strand consisting of 25 nucleotides, and the antisense strand consisting of 23 nucleotides.
Item 8. A lipid-modified double-stranded RNA according to Item 3, wherein each of the sense and antisense strands consists of 21 nucleotides.
Item 9. A lipid-modified double-stranded RNA according to any one of Items 1 to 8, wherein the lipid is a fatty acid having 6 to 50 carbon atoms.
Item 10. A lipid-modified double-stranded RNA according to any one of Items 1 to 9, wherein the lipid is lauric acid, stearic acid, myristic acid, or palmitic acid.
Item 11. A lipid-modified double-stranded RNA according to any one of Items 1 to 10, wherein the lipid is linked to at least one of the first to sixth nucleotides from the 5' end of the sense strand via a linker, the linker being represented by the structural formula $-NH-(CH_2)_{n1}-$ (L-4), wherein n1 is an integer of 1 to 40.

The nucleotides that constitute the sense strand and the antisense strand of the lipid-modified double-stranded RNA of the invention are basically ribonucleotides. To enhance the resistance to enzymatic digestion, the RNA sequence may contain various chemically modified nucleotides, such as 2'-O-methyl-modified nucleotides, 2'-F-modified nucleotides, LNA (Locked Nucleic Acid) nucleotides, deoxyribonucleotides, or the like. Particularly, when the lipid-modified double-stranded RNA of the invention has a dangling end, the dangling end of the sense strand and/or the antisense RNA may be composed of deoxyribonucleotides. Examples of such chemically modified nucleotides include phosphate backbone-modified nucleotides such as phosphorothioate-modified DNA/RNA and boranophosphate-modified DNA/RNA; 2'-modified nucleotides such as 2'-OMe-modified RNA and 2'-F-modified RNA; modified nucleotides obtained by crosslinking a sugar molecule of a nucleotide, such as LNA (Locked Nucleic Acid) and ENA (2'-O,4'-C-ethylene-bridged nucleic acids); modified nucleotides having different backbones, such as PNA (Peptide Nucleic Acid) and morpholine-nucleotide; base-modified nucleotides such as 5-fluorouridine and 5-propyluridine; and the like.
The lipid-modified double-stranded RNA of the invention is not particularly limited structurally, as long as the sense and antisense strands are hybridized into a double strand. For example, the lipid-modified double-stranded RNA preferably has the following structure: a structure (A) in which the double-stranded RNA is blunt-ended (i.e. has a blunt end) on the 5' end side of the sense strand, and is blunt-ended or has a dangling end (single-stranded region) on the 3' end side of the sense strand; a structure (B) in which the double-stranded RNA has dangling ends on the 5' and 3' end sides of the sense strand. The structure in which the double-stranded RNA has a dangling end on the 3' end side of the sense strand includes cases when the 3'-end region of the sense strand forms a dangling end, and cases when the 5'-end region of the antisense strand forms a dangling end. The structure in which the double-stranded RNA has a dangling end on the 5' end side of the sense strand includes the case in which the 5' end region of the sense strand forms a dangling end, and the case in which the 3' end region of the antisense strand forms a dangling end.
Among the double-stranded RNAs that can be used to form the lipid-modified double-stranded RNA of the invention, double-stranded RNAs having the structures (A-1) to (A-3) shown below are particularly preferable among those having the above structure (A), and double-stranded RNAs of the structure (B-1) shown below are particularly preferable among those having the above structure (B) to achieve a further enhanced RNA interference effect. The structure (A-1), in which the double-stranded RNA is blunt-ended on both the 5' and 3' end sides of the sense strand, and each of the sense and antisense strands consists of 27 nucleotides; the structure (A-2), in which the double-stranded RNA is blunt-ended on both the 5' and 3' end sides of the sense strand, and each of the sense and antisense strands consists of 23 nucleotides, respectively; the structure (A-3), in which the double-stranded RNA is blunt-ended on the 5' end side of the sense strand, and the sense strand consists of 25 nucleotides, and the antisense strand consists of 23 nucleotides; and the structure (B-1), in which the double-stranded RNA has dangling ends each consisting of two nucleotides on both the 3' end of the sense strand and the 3' end of the antisense strand, and each of the sense and antisense strands consists of 21 nucleotides.
More specifically, in the structures (A-1) and (A-2), sense and antisense strands are hybridized without any

dangling end formed on the ends. In the structure (A-3), sense and antisense strands are hybridized so that the double-stranded RNA is blunt-ended on the 5' end of the sense strand, and the first and second nucleotides from the 3' end of the sense strand form a dangling end. The structure (B-1) is that the first to 19th nucleotides from the 5' end of the sense strand and the third to 21st nucleotides from the 3' end of the antisense strand are hybridized so that the first and second nucleotides from the 3' end of the sense strand, and the first and second nucleotides from 3' end of the antisense strand form dangling ends, respectively.

In some embodiments, the lipid-modified double-stranded RNA of the invention has at least one lipid linked to at least one of the first to sixth nucleotides from the 5' end of the sense strand. In some embodiments, the lipid-modified double-stranded RNA of the invention has no substitutents at any other position than the 5' end region of the sense strand. More specifically, no substituents are present in any other area than the 5' end region of the sense strand and in the antisense strand, and these areas consist of nucleotides. Linking lipid(s) only to the 5' end region of the sense strand can enhance cellular uptake efficiency and provide an improved RNA interference effect.

The lipid linked to the sense strand of the lipid-modified double-stranded RNA of the invention is not particularly limited, and examples thereof include simple lipids (esters of fatty acids with various alcohols); complex lipids such as phospholipids and glycolipids; derived lipids such as fatty acids, higher alcohols, lipid soluble vitamins, steroids, and hydrocarbons. To enhance the cellular uptake efficiency and the RNA interference effect, the lipid used is in some embodiments a derived lipid, in some embodiments a fatty acid having 6 to 50 carbon atoms, in some embodiments a fatty acid having 10 to 22 carbon atoms, in some embodiments a fatty acid having 12 to 18 carbon atoms, in some embodiments lauric acid, stearic acid, myristic acid, or palmitic acid, and in other embodiments palmitic acid.

The manner of linking of the lipid to the sense strand to form the lipid-modified double-stranded RNA of the invention is not particularly limited. The lipid may be linked directly or via linker to the sense strand. In the present invention, the linker via which the lipid is linked to the sense strand is not the linker consisting of nucleic acid. The linker is not particularly limited as long as the lipid and the sense strand can be linked therethrough. For example, linkers having the following structures can be used as the linker:

-O-CO-O- (L-1)

-NH-CO-O- (L-2)

-NH-CO-NH- (L-3)

-NH-(CH2)n1- (L-4)

--S--(CH2)n1-- (L-5)

-CO-(CH2)n1-CO- (L-6)

-CO-(CH2)n1-NH- (L-7)

-NH-(CH2)n1-NH- (L-8)

-CO-NH-(CH2)n1-NH-CO- (L-9)

-C(=S)-NH-(CH2)n1-NH-CO- (L-10)

-C(=S)-NH-(CH2)n1-NH-C-(=S)- (L-11)

--CO--O-(CH2)n1--O--CO- (L-12)

-

C(=S)-O-(CH2)n1-O-CO-- (L-13)

-C(=S)-O-(CH2)n1-O-C-(=S)- (L-14)

-CO-NH-(CH2)n1-O-CO- (L-15)

--C(=S)-(CH2)n1--O--CO- (L-16)

-C(=S)-NH-(CH2)n 1 -O-C-(=S)- (L-17)

-

CO-(CH2)n1--O--CO- (L-18)

-C(=S)-NH-(CH2)n1-CO- (L-19)

-C(=S)-O-(CH2)n1-NH-CO- (L-20)

-C(=S)-NH-(CH2)n1--O--C--(=S)-- (L-21)

-NH-(CH2CH2O)n2-CH(CH2OH)- (L-22)

-NH-(CH2CH2O)n2-CH2- (L-23)

In the above Formulas (L-4) to (L-21), n1 is an integer of 1 to 40, in some embodiments an integer of 2 to 20, and in some embodiments an integer of 2 to 12.

In the above Formulas (L-22) and (L-23), n2 is an integer of 1 to 20, in some embodiments an integer of 1 to 10, and in some embodiments an integer of 1 to 6.

The linkers of Formulas (L-4) to (L-23) may link the sense strand on either the left or right side. In some embodiments, a specific site of the sense strand (or the nucleic acid of nucleic acid conjugate) is linked on the right side of the linkers of Formulas (L-4) to (L-23), and a lipid is linked on their left side.

The linking site of the lipid to the linker may be appropriately selected according to the types of lipid and linker used. For example, when a fatty acid is used as the lipid, it can be linked via an ester bond, an amide bond, or like bond formed between the carboxyl group of the fatty acid and the linker. More specifically, when a fatty acid is used as the lipid, the lipid is preferably linked by substitution of-OH of the carboxyl group of the fatty acid with the linker.

The linker is suitably selected according to the type of lipid to be linked. When a fatty acid is used as the lipid, the linkers represented by Formula (L-4) are preferably used.

In addition to the above-mentioned linkers, other linkers are also usable. Examples thereof include bifunctional linkers (linkers containing two functional groups), such as N-succinimidyl-3-(2-pyridyldithio)propionate, N-4-maleimide butyric acid, S-(2-pyridyldithio)cysteamine, iodoacetoxysuccinimide, N-(4-maleimidebutyloxy) succinimide, N-[5-(3'-maleimide propylamide)-1-carboxypentyl]iminodiacetic acid, N-(5-aminopentyl)-iminodiacetic acid, and the like. In the sense strand, the nucleotide linked to the lipid or to the linker used for linking the lipid is not particularly limited, as long as it is at least one of the first to sixth nucleotides from the 5' end of the sense strand, preferably at least one of the first to fourth nucleotides from the 5' end, more preferably the first and/or second nucleotide from the 5' end, and particularly preferably the nucleotide on the 5' end (the first nucleotide from the 5' end).

The linking site of the sense strand to the lipid or to the linker used for linking the lipid is not particularly limited. It is preferably linked by substitution of the hydrogen atom of the hydroxyl group of the phosphoric acid portion of a specific nucleotide of the sense strand.

The number of lipids linked to the lipid-modified double-stranded RNA of the invention is not particularly limited. For example, one to three lipids, preferably one or two lipids, and more preferably one lipid can be linked.

The lipid-modified double-stranded RNA of the invention can be produced by synthesizing a sense strand having at least one lipid linked thereto, and an antisense strand, respectively, and hybridizing the sense and antisense strands according to known methods. The sense strand having a lipid linked thereto can also be produced according to known synthetic methods.

More specifically, in one aspect the present invention provides a chemically-modified single- or double-stranded RNA that is loaded into and delivered by the exosomes described herein. In some embodiments, the chemically-modified RNA is one of those described in US 7,582,744, US 9,453,222, US 8,957,223, US 8,017,763, or US 8,404,862, the entirety of each of which is hereby incorporated by reference in its entirety.

In some embodiments, the RNA comprises a modified sugar, nucleoside monomer, or LCM (Ligand Conjugated Monomer) disclosed in US Patent No. 7,582,744, the entirety of which is hereby incorporated by reference.

In some embodiments, the present invention provides an isolated oligonucleotide agent comprising a nucleotide sequence consisting of from 12 to 23 nucleotides in length sufficiently complementary to a microRNA target sequence of about 12 to 23 nucleotides, wherein the nucleotide sequence of the oligonucleotide agent differs by no more than 1 or 2 nucleotides from full complementarity to the microRNA target sequence and wherein said oligonucleotide agent has the structure (I)

(5') QxQz1(Qy)nQz2Qz3Qz4Q-L (3') (I)

wherein

Q is a 2'-O-methyl modified nucleoside; x, z1, z2, z3, and z4 are all

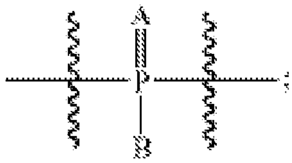

one of A and B is S while the other is O;

n=6-17;

L is

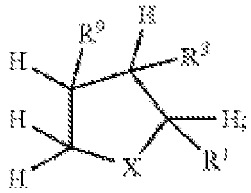

wherein:

X is N(CO)R7, or NR7;

each of R1, R3 and R9, is, independently, H, OH, or -CH2ORb provided that at least one of R1, R3, or R9 is OH and at least one of R1, R3 or R9 is -CH2ORb;

R7 is C1-C20 alkyl substituted with NRcRd or NHC(O)Rd;

Rc is H or C1-C6 alkyl;

Rd is a carbohydrate radical; or a sterol or steroid radical, which is optionally tethered to at least one carbohydrate radical; and

Rb is

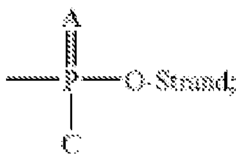

one of E and F is S while the other is O.

In some embodiments, Rd is cholesterol. In some embodiments, R1 is -CH2ORb. In some embodiments, R9 is OH. In some embodiments, R1 and R9 are trans. In some embodiments, R3 is OH. In some embodiments, R1 and R3 are trans. In some embodiments, R3 is -CH2ORb. In some embodiments, R1 is OH. In some embodiments, R1 and R3 are trans. In some embodiments, R9 is OH. In some embodiments, R3 and R9 are trans. In some embodiments, R9 is -CH2ORb. In some embodiments, R1 is OH. In some embodiments, R1 and R9 are trans. In some embodiments, X is NC(O)R7. In some embodiments, R7 is - CH2(CH2)3CH2NHC(O)Rd. In some embodiments, R1 is CH2ORb; R9 is OH; R1 and R9 are trans; X is NC(O)R7; R7 is CH2(CH2)3CH2NHC(O)Rd and Rd is a sterol or steroid radical.

In some embodiments, the nucleotide sequence of the oligonucleotide agent is SEQ ID NO:96 from US Patent No. 7,582,744. In some embodiments, the oligonucleotide agent consists of a sequence that differs at no more than 1 or 2 nucleotides from a sequence of 12 or more contiguous nucleotides of SEQ ID NO:96 from US Patent No. 7,582,744. In some embodiments, the nucleotide sequence of the oligonucleotide agent is SEQ ID NO:101 from US Patent No. 7,582,744. In some embodiments, the nucleotide sequence of the oligonucleotide agent is SEQ ID NO:102 from US Patent No. 7,582,744. In some embodiments, the nucleotide sequence of the oligonucleotide agent is SEQ ID NO: 103 from US Patent No. 7,582,744.

In one aspect, the invention features an oligonucleotide agent preferably comprising at least one subunit having the structure of formula (I):

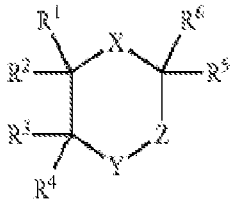

(I)

wherein:

X is N(CO)R7, NR7 or CH2;
Y is NR8, O, S, CR9R10, or absent;
Z is CR11R12 or absent;
Each of R1, R2, R3, R4, R9, and R10 is, independently, H, ORa, ORb, (CH2)nORa, or (CH2)nORb, provided that at least one of Rl, R2, R3, R4, R9, and R10 is ORa or ORb and that at least one of R1, R2, R3, R4, R9, and R10 is (CH2)nORa, or (CH2)nORb (when the SRMS is terminal, one of R1, R2, R3, R4, R9, and R10 will include Ra and one will include Rb; when the SRMSS is internal, two of R1, R2, R3, R4, R9, and R10 will each include an Rb); further provided that preferably ORa may only be present with (CH2)nORb and (CH2)nORa may only be present with ORb;
Each of R5, R6, R11, and R12 is, independently, H, C1-C6 alkyl optionally substituted with 1-3 R13, or C(O)NHR7; or R5 and R11 together are C3-C8 cycloalkyl optionally substituted with R14;
R7 can be a ligand, e.g., R7 can be Rd, or R7 can be a ligand tethered indirectly to the carrier, e.g., through a tethering moiety, e.g., C1-C20 alkyl substituted with NRcRd; or C1-C20 alkyl substituted with NHC(O)Rd;
R8 is C1-C6 alkyl;
R13 is hydroxy, C1-C4 alkoxy, or halo;
R14 is NRcR7;
Ra is:

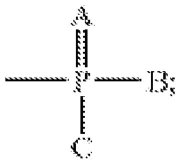

Rb is:

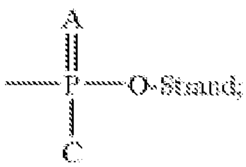

Each of A and C is, independently, O or S;
B is OH, O-, or

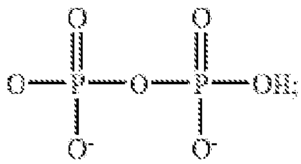

Rc is H or C1-C6 alkyl;
Rd is H or a ligand, e.g., a lipophilic ligand, e.g., cholesterol; and
n is 1-4.

Embodiments can include one or more of the following features: R1 can be CH2ORa and R3 can be ORb; or R1 can be CH2ORa and R9 can be ORb; or R1 can be CH2ORa and R2 can be ORb.

R1 can be CH2ORb and R3 can be ORb; or R1 can be CH2ORb and R9 can be ORb; or R1 can be CH2ORb and R2 can be ORb; or R1 can be CH2ORb and R3 can be ORa; or R1 can be CH2ORb and R9 can be ORa; or R1 can be CH2ORb and R2 can be ORa.

R1 can be ORa and R3 can be CH2ORb; or R1 can be ORa and R9 can be CH2ORb; or R1 can be ORa and R2 can be CH2ORb.

R1 can be ORb and R3 can be CH2ORb; or R1 can be ORb and R9 can be CH2ORb; or R1 can be ORb and R2 can be CH2ORb; or R1 can be ORb and R3 can be CH2ORa; or R1 can be ORb and R9 can be CH2ORa; or R1 can be ORb and R2 can be CH2ORa.

R3 can be CH2ORaand R9 can be ORb; or R3 can be CH2ORa and R4 can be ORb.

R3 can be CH2ORb and R9 can be ORb; or R3 can be CH2ORb and R4 can be ORb; or R3 can be CH2ORb and R9 can be ORa; or R3 can be CH2ORb and R4 can be ORa.

R3 can be ORb and R9 can be CH2ORa; or R3 can be ORb and R4 can be CH2ORa; or R3 can be ORb and R9 can be CH2ORb; or R3 can be ORb and R4 can be CH2ORb.

R3 can be ORa and R9 can be CH2ORb; or R3 can be ORa and R4 can be CH2ORb.

R9 can be CH2ORa and R10 can be ORb.

R9 can be CH2ORb and R10 can be ORb; or R9 can be CH2ORb and R10 can be ORa.

In a preferred embodiment the ribose is replaced with a pyrroline scaffold or with a 4-hydroxyproline-derived scaffold, and X is N(CO)R7 or NR7, Y is CR9R10, and Z is absent.

R1 and R3 can be cis or R1 and R3 can be trans.

n can be 1.

A can be O or S.

R1 can be (CH2)nORb and R3 can be ORb; or R1 can be (CH2)nORa and R3 can be ORb.

R7 can be (CH2)5NHRd or (CH2)5NHRd. Rd can be chosen from a folic acid radical; a cholesterol radical; a carbohydrate radical; a vitamin A radical; a vitamin E radical; a vitamin K radical. In some embodiments, Rd is a cholesterol radical.

R1 can be ORb and R3 can be (CH2)nORb; or R1 can be ORb and R3 can be (CH2)nORa; or R1 can be ORa and R3 can be (CH2)nORb; or R1 can be (CH2)nORb and R9 can be ORa.

R1 and R9 can be cis or R1 and R9 can be trans.

R1 can be ORa and R9 can be (CH2)nORb; or R1 can be (CH2)nORb and R9 can be ORb; or R1 can be (CH2)nORa and R9 can be ORb; or R1 can be ORb and R9 can be (CH2)nORb; or R1 can be ORb and R9 can be (CH2)nORa.

R3 can be (CH2)nORb and R9 can be ORa; or R3 can be (CH2)nORb and R9 can be ORb; or R3 can be (CH2)nORa and R9 can be ORb; or R3 can be ORa and R9 can be (CH2)nORb; R3 can be ORb and R9 can be (CH2)nORb; or R3 can be ORb and R9 can be (CH2)nORa.

R3 and R9 can be cis or R3 and R9 can be trans.

In other embodiments the ribose is replaced with a piperidine scaffold, and X is N(CO)R7 or NR7, Y is CR9R10, and Z is CR11R12.

R9 can be (CH2)nORb and R10 can be ORa.

n can be 1 or 2.

R9 can be (CH2)nORb and R10 can be ORb; or R9 can be (CH2)nORa and R10 can be ORb.

A can be O or S.

R7 can be (CH2)5NHRd or (CH2)5NHRd. Rd can be selected from a folic acid radical; a cholesterol radical; a carbohydrate radical; a vitamin A radical; a vitamin E radical; a vitamin K radical. In some embodiments, Rd is a cholesterol radical.

R3 can be (CH2)nORb and R4 can be ORa; or R3 can be (CH2)nORb and R4 can be ORb; or

R3 can be (CH2)nORa and R4 can be ORb.

R1 can be (CH2)nORb and R2 can be ORa; or R1 can be (CH2)nORb and R2 can be ORb; or R1 can be (CH2)nORa and R2 can be ORb.

R3 can be (CH2)nORb and R9 can be ORa.

R3 and R9 can be cis, or R3 and R9 can be trans.

R3 can be (CH2)nORb and R9 can be ORb; or R3 can be (CH2)nORb and R9 can be ORa; or R3 can be (CH2)nORa and R9 can be ORb.

R1 can be (CH2)nORb and R3 can be ORa.
R1 and R3 can be cis, or R1 and R3 can be trans.
R3 can be ORa and R9 can be (CH2)nORb.
R1 can be ORa and R3 can be (CH2)nORb.
In other preferred embodiments the ribose is replaced with a piperazine scaffold, and X is N(CO)R7 or NR7, Y is NR8, and Z is CR11R12.
R1 can be (CH2)nORb and R3 can be ORa.
R1 and R3 can be cis or R1 and R3 can be trans.
n can be 1.
R1 can be (CH2)nORb and R3 can be ORb; or R1 can be (CH2)nORa and R3 can be ORb.
A can be O or S.
R7 can be (CH2)5NHRd or (CH2)5NHRd. Rd can be chosen from the group of a folic acid radical; a cholesterol radical; a carbohydrate radical; a vitamin A radical; a vitamin E radical; a vitamin K radical. In some embodiments, Rd is a cholesterol radical.
R8 can be CH3.
R1 can be ORa and R3 can be (CH2)nORb.
In other embodiments the ribose is replaced with a morpholino scaffold, and X is N(CO)R7 or NR7, Y is O, and Z is CR11R12.
R1 can be (CH2)nORb and R3 can be ORa.
R1 and R3 can be cis, or R1 and R3 can be trans.
n can be 1.
R1 can be (CH2)nORb and R3 can be ORb; of R1 can be (CH2)nORa and R3 can be ORb.
A can be O or S.
R7 can be (CH2)5NHRd or (CH2)5NHRd. Rd can be chosen from the group of a folic acid radical; a cholesterol radical; a carbohydrate radical; a vitamin A radical; a vitamin E radical; a vitamin K radical. In some embodiments, Rd is a cholesterol radical.
R8 can be CH3.
R1 can be ORa and R3 can be (CH2)nORb.
In other embodiments the ribose is replaced with a decalin scaffold, and X is CH2; Y is CR9R10; and Z is CR11R12; and R5 and R11 together are C6 cycloalkyl.
R6 can be C(O)NHR7.
R12 can be hydrogen.
R6 and R12 can be trans.
R3 can be ORa and R9 can be (CH2)nORb.
R3 and R9 can be cis, or R3 and R9 can be trans.
n can be 1 or 2.
R3 can be ORb and R9 can be (CH2)nORb; or R3 can be ORb and R9 can be (CH2)nORa.
A can be O or S.
R7 can be (CH2)5NHRd or (CH2)5NHRd. Rd can be chosen from the group of a folic acid radical; a cholesterol radical; a carbohydrate radical; a vitamin A radical; a vitamin E radical; a vitamin K radical. In some embodiments, Rd is a cholesterol radical.
In other embodiments the ribose is replaced with a decalin/indane scaffold, e.g., X is CH2; Y is CR9R10; and Z is CR11R12; and R5 and R11 together are C5 cycloalkyl.
R6 can be CH3.
R12 can be hydrogen.
R6 and R12 can be trans.
R3 can be ORa and R9 can be (CH2)nORb.
R3 and R9 can be cis, or R3 and R9 can be trans.
n can be 1 or 2.
R3 can be ORb and R9 can be (CH2)nORa; or R3 can be ORb and R9 can be (CH2)nORa.
A can be O or S.
R14 can be N(CH3)R7. R7 can be (CH2)5NHRd or (CH2)nNHRd. Rd can be chosen from the group of a folic acid radical; a cholesterol radical; a carbohydrate radical; a vitamin A radical; a vitamin E radical; a vitamin K radical. Preferably, Rd is a cholesterol radical.
In another aspect, this invention features an oligonucleotide agent comprising at least one subunit having a structure of formula (II):

(II)

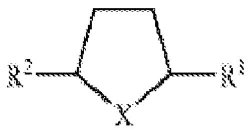

X is N(CO)R7 or NR7;

Each of R1 and R2 is, independently, ORa, ORb, (CH2)nORa, or (CH2)nORb, provided that one of R1 and R2 is ORa or ORb and the other is (CH2)nORa or (CH2)nORb (when the SRMS is terminal, one of R1 or R2 will include Ra and one will include Rb; when the SRMSS is internal, both R1 and R2 will each include an Rb); further provided that in some embodiments ORa may only be present with (CH2)nORb and (CH2)nORa may only be present with ORb;

R7 is C1-C20 alkyl substituted with NRcRd;

R8 is C1-C6 alkyl;

R13 is hydroxy, C1-C4 alkoxy, or halo;

R14 is NRcR7;

Ra is:

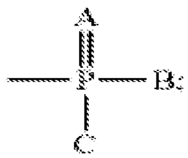

Rb is:

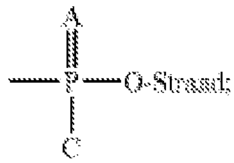

Each of A and C is, independently, O or S;

B is OH, O-, or

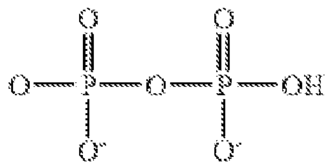

;

Rc is H or C1-C6 alkyl;

Rd is H or a ligand; and

n is 1-4.

The oligonucleotide agent of the conjugate is substantially single-stranded and comprises from about 12 to about 29 subunits, preferably about 15 to about 25 subunits. An oligonucleotide agent that is substantially single-stranded includes at least 60%, 70%, 80%, or 90% or more nucleotides that are not duplexed.

Embodiments can include one or more of the features described above.

In a further aspect, this invention features an oligonucleotide agent having at least one subunit comprising formula (I) or formula (II).

In one aspect, this invention features an oligonucleotide agent having at least two subunits comprising formula (I) and/or formula (II).

In another aspect, this invention provides a method of making an oligonucleotide agent described herein having at least one subunit comprising formula (I) and/or (II). In a further aspect, this invention provides a method of modulating expression of a target gene. The method includes administering an oligonucleotide agent described herein having at

least one subunit comprising formula (I) and/or (II) to a subject.

SRMSs or tethers described herein may be incorporated into any oligonucleotide agent described herein. An oligonucleotide agent may include one or more of the SRMSs described herein. An SRMS can be introduced at one or more points in an oligonucleotide agent. An SRMS can be placed at or near (within 1, 2, or 3 positions) the 3' or 5' end of the oligonucleotide. In some embodiments, it is preferred to not have an SRMS at or near (within 1, 2, or 3 positions of) the 5' end of the oligonucleotide. An SRMS can be internal, and will preferably be positioned in regions not critical for binding to the target.

In an embodiment, an oligonucleotide agent may have an SRMS at (or within 1, 2, or 3 positions of) the 3' end.

In another embodiment, an oligonucleotide agent may have an SRMS at an internal position. In other embodiments, an oligonucleotide agent may have an SRMS at the 3' end and an SRMS at an internal position.

Other modifications to sugars, bases, or backbones described herein can be incorporated into the oligonucleotide agents.

The oligonucleotide agents can take an architecture or structure described herein.

The oligonucleotide agent can be selected to target any of a broad spectrum of genes, including any of the genes described herein.

In a preferred embodiment the oligonucleotide agent has an architecture (architecture refers to one or more of the overall length) described herein. In addition to the SRMS-containing bases of the oligonucleotide agents described herein can include nuclease resistant monomers (NRMs).

In another aspect, the invention features an oligonucleotide agent to which is conjugated a lipophilic moiety, e.g., cholesterol, e.g., by conjugation to an SRMS of an oligonucleotide agent. In some embodiments, the lipophilic moiety enhances entry of the oligonucleotide agent into a cell. In some embodiments, the cell is part of an organism, tissue, or cell line, e.g., a primary cell line, immortalized cell line, or any type of cell line disclosed herein. Thus, the conjugated oligonucleotide agent can be used to inhibit expression of a target gene in an organism, e.g., a mammal, e.g., a human, or to inhibit expression of a target gene in a cell line or in cells which are outside an organism.

The lipophilic moiety (hydrophobic group) can be chosen, for example, from the group consisting of a lipid, cholesterol, oleyl, retinyl, cholesteryl residues, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine. In some embodiments, the lipophilic moiety is cholesterol. In some embodiments, the lipophilic moiety is selected from folic acid; cholesterol; a carbohydrate; vitamin A; vitamin E; or vitamin K.

The oligonucleotide agent can have at least one subunit having formula (I) or formula (II) incorporated into it. The oligonucleotide agent can have one or more of any of the features described herein. For example, when the subunit is of formula (I), Rd can be cholesterol; X can be N(CO)R7 or NR7, Y can be CR9R10, and Z can be absent, and R1 can be (CH2)nORb and R3 can be ORa; X can be N(CO)R7 or NR7, Y can be CR9R10, and Z can be CR11R12, and R9 can be (CH2)nORb and R10 can be ORa; X can be N(CO)R7 or NR7, Y can be NR8, and Z can be CR11R12, and R1 can be (CH2)nORb and R3 can be ORa; X can be CH2; Y can be CR9R10; and Z can be CR11R12, in which R6 can be C(O)NHR7; or X can be CH2; Y can be CR9R10; and Z can be CR11R12, in which R11 or R12 can be C(O)NHR7 or R5 and R11 together can be C5 or C6 cycloalkyl substituted with N(CH3)R7.

Exemplary single stranded oligonucleotide agents can target RNAs encoding the following polypeptides: vascular endothelial growth factor (VEGF); Apoliprotein B (ApoB); luciferase (luc); Androgen Receptor (AR); coagulation factor VII (FVII); hypoxia-inducible factor 1, alpha subunit (Hif-1$\alpha$); placenta growth factor (PLGF); Lamin A/C; and green fluorescent protein (GFP). Exemplary single stranded oligonucleotide agents are shown in **Table 1A** below. Additional suitable miRNA targets are described, e.g., in John et al., PLoS Biology 2:1862-1879, 2004 (correction in PLoS 3:1328, 2005), and The microRNA Registry (Griffiths-Jones S., NAR 32:D109-D111, 2004).

**Table 1A**

| Exemplary oligonucleotide agents | | |
|---|---|---|
| AL-SQ-NO: | Sequence (5'-3' unless otherwise indicated) | Target |
| 3186 | GCACAUAGGAGAGAUGAGCUUs-Chol | VEGF |
| 3191 | Naproxen-sGUCAUCACACUGAAUACCAAUs-Chol | ApoS |
| 3209 | CAUCACACUGAAUACCAAUdTdTs-Chol | Luc |
| 3230 | oUsoCsoAoCoGoCoGoAoGoCoCoGoAoAoCoGoAoAoCsoAsoAsoAs-Chol | Mir-375 |
| 3234 | oCoUGGGAAAGoUoCAAGoCoCoCAoUdTsdT-Chol | AR |
| 3235 | oCoUGoUGoCAAGoUGoCoCodTsdT-Chol | AR |
| 3253 | GGAfUfCAfUfCfUfCAAGfUfCfUfUAfCdTsdT-Chol | FVIT |

(continued)

| Exemplary oligonucleotide agents | | |
|---|---|---|
| 3256 | ACUGAGGUGAAGAAUUAdTsdTs-Chol | H1f-1α |
| 3257 | GCACAUAGGAGAGAUGAGCUsUs-Chol | VEGF |
| 3258 | GAACUGUGUGUGAGAGGUCCsUs-Chol | Luc |
| 3264 | CCAGGUUUUUUUCUUACUUTsTs-Chol | VEGF |
| 3265 | UUCUCAAAUCAAUUACCATsTs - Chol | VEGF |
| 3266 | GACACAGUGUGUUUGAUUUdTsdTs-Chol | Hif-1α |
| 3269 | UGCCAAGCCAGAUUCUUdTsdTs - Chol | PLGF |
| 3271 | CUCAGGAAUUCAGUSCCUUdTsdTs-Chol | PLGP |
| 3275 | CUGGACUCCAGAAGAACAdTdT-Chol | Lamin |
| 3150 | Chol-sGUCAUCACACUGAAUACCAAsU | ApoB |
| 5225 | GUCAUCACACUGAAUACCAAUs-Chol | ApoB |
| 4967 | GcACcAUCUUCUUcAAGGACGS-Chol | GFP |
| 5225 | GUCAUCACACAUGAAUACCAAUs-Chol | ApoB |
| 5221 | AGGUGUAUGGCUUCAACCCUGs-Chol | ApoB |
| AL-SQ-NO: | Sequence (5'-3' unless otherwise indicated) | Target |
| 5255 | GUGAUCAGACUCAAUACCAAUs-Chol | ApoB |
| 5474 | GGAAUCoUoUAoUAoUoUoUGAUCoCAAs-Chol | ApoB |
| 4750 | CCACAUGAAGCAGCACGACUUs-Chol | GFP |
| 3148 | GUCAUCACACUGAAUACCAAUs-Thiochol | ApoB |
| 3208 | ACUGGUAUUCAGUGUGAUGAoCsoAsCs-Thiochol | ApoB |
| 3233 | AGUGGUAUUCAGUGUGAUGAoCsoAsCs-Thiochol | ApoB |
| 2774 | CUUACGCUGAGUACUUCGAdTdT-Thiochol | Luc |
| 2775 | UCGAAGUACUCAGCGUAAGdTdT-Thiochol | Luc |
| 3149 | Thiocol-sGUCAUCACACUGAAUACCAAsU | ApoB |
| 3207 | AUUGGUAUUCAGUGUGAUGAoCsoAsCs-Cholanic acid | ApoB |
| 3231 | GUCAUCACACUGAAUACCAAUs-Lithocholic I | ApoB |
| 3189 | GUCAUCACACUGAAUACCAAUs-Distearylglyceride | ApoB |
| 2767 | CUUACGCUGAGUACUUCGAdTdT-Distearylglyceride | Luc |
| 2768 | 3' Distearylglyceride-dTdTGAAUGCGACUCAUGAAGCU 5' | Luc |
| | 3204 Distearylglyceride-sGUCAUCACACUGAAUACCAsU | ApoB |
| 2918 | Distearylglyceride-CUUACGCUGAGUACUUCGAdTdT | ApoB |
| 2919 | 3' dTdTGAAUGCGACUCAUGAAGCU-Distearylglyceride 5' | Luc |
| 3190 | GUCAUCACACUGAAUACCAAUs-Vitamin E | ApoB |
| 2920 | Vitamin E-CUUACGCUGAGUACUUCGA dTdT' | Luc |
| 2921 | 3' dTdTGAAUGCGACUCAUGAAGCU-Vitamin E 5' | ApoB |
| 3192 | Aminoalkyl-sGUCAUCACACUGAAUACCAAUs-Chol | ApoB |

"oN" (N = A, C, G or U) indicates 2' -O-Nethyl modified nucleotide;
"fN" (N = A, C, G or U) indicates 2' -deoxy-2' -fluoro modified nucleotide; "s" indicates phosphorothioate linkage;
"Chol" indicates cholesterol conjugate;
"Thiochol" indicates thiocholesterol conjugate;
"Cholanic Acid" indicates 5β acid conjugate;
"Naproxen" indicates Naproxen conjugate;
"Lithocholic I" indicates lithocholic acid derivative conjugate;
"Distearylglyceride" indicates distearylglyceride conjugate;
"Vitamin E" indicates vitamin E conjugate and "Aminoalkyl" indicates amino linker conjugate.

An oligonucleotide agent, e.g., a conjugated oligonucleotide agent, containing an exemplary, but nonlimiting ligand-conjugated monomer subunit is presented as formula (II) below and in the scheme in FIG. 1 of US 7,582,744, hereby incorporated by reference. The carrier (also referred to in some embodiments as a "linker") can be a cyclic or

acyclic moiety and includes two "backbone attachment points" (e.g., hydroxyl groups) and a ligand. The ligand can be directly attached (e.g., conjugated) to the carrier or indirectly attached (e.g., conjugated) to the carrier by an intervening tether (e.g., an acyclic chain of one or more atoms; or a nucleobase, e.g., a naturally occurring nucleobase optionally having one or more chemical modifications, e.g., an unusual base; or a universal base). The carrier therefore also includes a "ligand or tethering attachment point" for the ligand and tether/tethered ligand, respectively.

The ligand-conjugated monomer subunit may be the 5' or 3' terminal subunit of the RNA molecule, i.e., one of the two "W" groups may be a hydroxyl group, and the other "W" group may be a chain of two or more unmodified or modified ribonucleotides. Alternatively, the ligand-conjugated monomer subunit may occupy an internal position, and both "W" groups may be one or more unmodified or modified ribonucleotides. More than one ligand-conjugated monomer subunit may be present in a RNA molecule, e.g., an oligonucleotide agent. Exemplary positions for inclusion of a tethered ligand-conjugated monomer subunit, e.g., one in which a lipophilic moiety, e.g., cholesterol, is tethered to the carrier are at the 3' terminus, the 5' terminus, or at an internal position.

The modified RNA molecule of formula (II) can be obtained using oligonucleotide synthetic methods known in the art and, for example, described in US 7,582,744, hereby incorporated by reference. In some embodiments, the modified RNA molecule of formula (II) can be prepared by incorporating one or more of the corresponding monomer compounds (see, e.g., A, B, and C sections and in the scheme in FIG. 1 of US 7,582,744, hereby incorporated by reference) into a growing strand, utilizing, e.g., phosphoramidite or H-phosphonate coupling strategies.

The monomers, e.g., a ligand-conjugated monomers, generally include two differently functionalized hydroxyl groups (OFG1 and OFG2), which are linked to the carrier molecule (see A below and in FIG. 1 of US 7,582,744, hereby incorporated by reference), and a ligand/tethering attachment point. As used herein, the term "functionalized hydroxyl group" means that the hydroxyl proton has been replaced by another substituent. As shown in representative structures B and C below and in FIG. 1 of US 7,582,744, hereby incorporated by reference, one hydroxyl group (OFG1) on the carrier is functionalized with a protecting group (PG). The other hydroxyl group (OFG2) can be functionalized with either (1) a liquid or solid phase synthesis support reagent (solid circle) directly or indirectly through a linker, L, as in B, or (2) a phosphorus-containing moiety, e.g., a phosphoramidite as in C. The tethering attachment point may be connected to a hydrogen atom, a suitable protecting group, a tether, or a tethered ligand at the time that the monomer is incorporated into the growing strand (see variable "R" in A below). Thus, the tethered ligand can be, but need not be attached to the monomer at the time that the monomer is incorporated into the growing strand. In certain embodiments, the tether, the ligand or the tethered ligand may be linked to a "precursor" ligand-conjugated monomer subunit after a "precursor" ligand-conjugated monomer subunit has been incorporated into the strand. The wavy line used below (and elsewhere herein) refers to a connection, and can represent a direct bond between the moiety and the attachment point or a tethering molecule which is interposed between the moiety and the attachment point. Directly tethered means the moiety is bound directly to the attachment point. Indirectly tethered means that there is a tether molecule interposed between the attachment point and the moiety.

A:

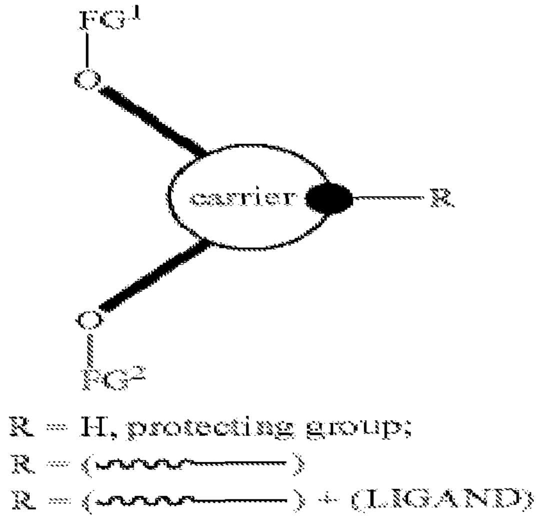


B:

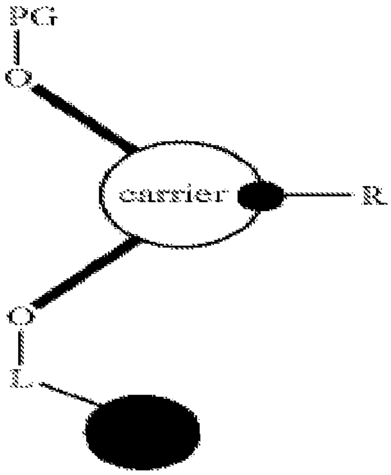

C:

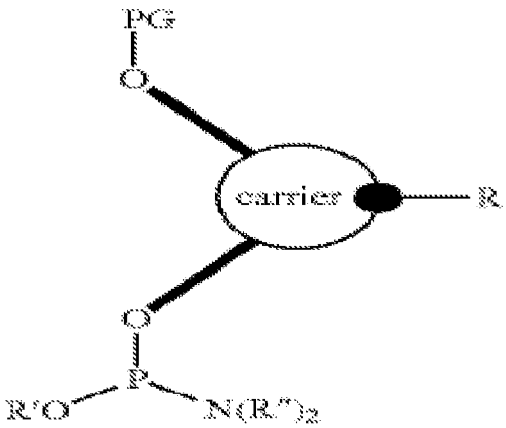

The (OFG1) protecting group may be selected as desired, e.g., from T. W. Greene and P. G. M. Wuts, Protective Groups in Organic Synthesis, 2d. Ed., John Wiley and Sons (1991). The protecting group is preferably stable under amidite synthesis conditions, storage conditions, and oligonucleotide synthesis conditions. Hydroxyl groups, -OH, are nucleophilic groups (i.e., Lewis bases), which react through the oxygen with electrophiles (i.e., Lewis acids). Hydroxyl groups in which the hydrogen has been replaced with a protecting group, e.g., a triarylmethyl group or a trialkylsilyl group, are essentially unreactive as nucleophiles in displacement reactions. Thus, the protected hydroxyl group is useful

in preventing e.g., homocoupling of compounds exemplified by structure C during oligonucleotide synthesis. In some embodiments, a preferred protecting group is the dimethoxytrityl group. In other embodiments, a preferred protecting group is a silicon-based protecting group having the formula below:

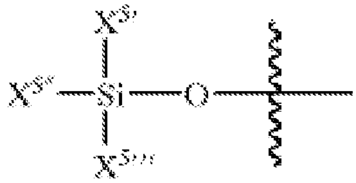

X5', X5", and X5‴ can be selected from substituted or unsubstituted alkyl, cycloalkyl, aryl, araklyl, heteroaryl, alkoxy, cycloalkoxy, aralkoxy, aryloxy, heteroaryloxy, or siloxy (i.e., R3SiO-, the three "R" groups can be any combination of the above listed groups). X5', X5", and X5‴ may all be the same or different; also contemplated is a combination in which two of X5', X5", and X5‴ are identical and the third is different. In certain embodiments X5', X5", and X5‴ include at least one alkoxy or siloxy groups and may be any one of the groups listed in FIG. 2A of US 7,582,744, hereby incorporated by reference, a preferred combination includes X5', X5" = trimethylsiloxy and X5‴ = 1,3-(triphenylmethoxy)-2-propoxy or cyclododecyloxy.

Other preferred combinations of X5', X5", and X5‴ include those that result in OFG1 groups that meet the deprotection and stability criteria delineated below. The group is preferably stable under amidite synthesis conditions, storage conditions, and oligonucleotide synthesis conditions. Rapid removal, i.e., less than one minute, of the silyl group from e.g., a support-bound oligonucleotide is desirable because it can reduce synthesis times and thereby reduce exposure time of the growing oligonucleotide chain to the reagents. Oligonucleotide synthesis can be improved if the silyl protecting group is visible during deprotection, e.g., from the addition of a chromophore silyl substituent.

Selection of silyl protecting groups can be complicated by the competing demands of the essential characteristics of stability and facile removal, and the need to balance these competitive goals. Most substituents that increase stability can also increase the reaction time required for removal of the silyl group, potentially increasing the level of difficulty in removal of the group.

The addition of alkoxy and siloxy substituents to OFG1 silicon-containing protecting groups increases the susceptibility of the protecting groups to fluoride cleavage of the silylether bonds. Increasing the steric bulk of the substituents preserves stability while not decreasing fluoride lability to an equal extent. An appropriate balance of substituents on the silyl group makes a silyl ether a viable nucleoside protecting group.

Candidate OFG1 silicon-containing protecting groups may be tested by exposing a tetrahydrofuran solution of a preferred carrier bearing the candidate OFG1 group to five molar equivalents of tetrahydrofuran at room temperature. The reaction time may be determined by monitoring the disappearance of the starting material by thin layer chromatography.

When the OFG2 in B includes a linker, e.g., a relatively long organic linker, connected to a soluble or insoluble support reagent, solution or solid phase synthesis techniques can be employed to build up a chain of natural and/or modified ribonucleotides once OFG1 is deprotected and free to act as a nucleophile with another nucleoside or monomer containing an electrophilic group (e.g., an amidite group). Alternatively, a natural or modified ribonucleotide or oligoribonucleotide chain can be coupled to monomer C via an amidite group or H-phosphonate group at OFG2. Subsequent to this operation, OFG1 can be deblocked, and the restored nucleophilic hydroxyl group can react with another nucleoside or monomer containing an electrophilic group. R' can be substituted or unsubstituted alkyl or alkenyl. In some embodiments, R' is methyl, allyl or 2-cyanoethyl. R" may a C1-C10 alkyl group, for example a branched group containing three or more carbons, e.g., isopropyl.

OFG2 in B can be hydroxyl functionalized with a linker, which in turn contains a liquid or solid phase synthesis support reagent at the other linker terminus. The support reagent can be any support medium that can support the monomers described herein. The monomer can be attached to an insoluble support via a linker, L, which allows the monomer (and the growing chain) to be solubilized in the solvent in which the support is placed. The solubilized, yet immobilized, monomer can react with reagents in the surrounding solvent; unreacted reagents and soluble by-products can be readily washed away from the solid support to which the monomer or monomer-derived products is attached. Alternatively, the monomer can be attached to a soluble support moiety, e.g., polyethylene glycol (PEG) and liquid phase synthesis techniques can be used to build up the chain. Linker and support medium selection is within skill of the art. Generally the linker may be -C(O)(CH2)qC(O)-, or -C(O)(CH2)qS-, in which q can be 0, 1, 2, 3, or 4; preferably, it is oxalyl, succinyl or thioglycolyl. Standard control pore glass solid phase synthesis supports can not be used in conjunction with fluoride labile 5' silyl protecting groups because the glass is degraded by fluoride with a significant reduction in the amount of full-length product. Fluoride-stable polystyrene based supports or PEG are preferred.

The ligand/tethering attachment point can be any divalent, trivalent, tetravalent, pentavalent or hexavalent

atom. In some embodiments, ligand/tethering attachment point can be a carbon, oxygen, nitrogen or sulfur atom. For example, a ligand/tethering attachment point precursor functional group can have a nucleophilic heteroatom, e.g., -SH, -NH2, secondary amino, ONH2, or NH2NH2. As another example, the ligand/tethering attachment point precursor functional group can be an olefin, e.g., -CH=CH2 or a Diels-Alder diene or dienophile and the precursor functional group can be attached to a ligand, a tether, or tethered ligand using, e.g., transition metal catalyzed carbon-carbon (for example olefin metathesis) processes or cycloadditions (e.g., Diels-Alder). As a further example, the ligand/tethering attachment point precursor functional group can be an electrophilic moiety, e.g., an aldehyde. When the carrier is a cyclic carrier, the ligand/tethering attachment point can be an endocyclic atom (i.e., a constituent atom in the cyclic moiety, e.g., a nitrogenatom) or an exocyclic atom (i.e., an atom or group of atoms attached to a constituent atom in the cyclic moiety).

The carrier can be any organic molecule containing attachment points for OFG1, OFG2, and the ligand. In certain embodiments, carrier is a cyclic molecule and may contain heteroatoms (e.g., O, N or S). E.g., carrier molecules may include aryl (e.g., benzene, biphenyl, etc.), cycloalkyl (e.g., cyclohexane, cis or trans decalin, etc.), or heterocyclyl (piperazine, pyrrolidine, etc.). In other embodiments, the carrier can be an acyclic moiety, e.g., based on serinol. Any of the above cyclic systems may include substituents in addition to OFG1, OFG2, and the ligand.

Sugar-Based Monomers

In some embodiments, the carrier molecule is an oxygen containing heterocycle. In some embodiments, the carrier is a ribose sugar as shown in structure LCM-I. In this embodiment, the ligand-conjugated monomer is a nucleoside.

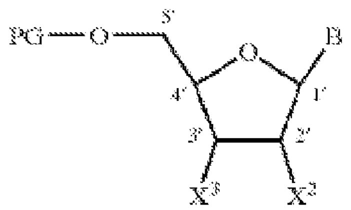

LCM-I

"B" represents a nucleobase, e.g., a naturally occurring nucleobase optionally having one or more chemical modifications, e.g., and unusual base; or a universal base.

As used herein, an "unusual" nucleobase can include any one of the following:

2-methyladeninyl, N6-methyladeninyl, 2-methylthio-N6-methyladeninyl, N6-isopentenyladeninyl, 2-methylthio-N6-isopentenyladeninyl, N6-(cis-hydroxyisopentenyl)adeninyl, 2-methylthio-N6-(cis-hydroxyisopentenyl)adeninyl, N6-glycinylcarbamoyladeninyl, N6-threonylcarbamoyladeninyl, 2-methylthio-N6-threonyl carbamoyladeninyl, N6-methyl-N6-threonylcarbamoyladeninyl, N6-hydroxynorvalylcarbamoyladeninyl, 2-methylthio-N6-hydroxynorvalyl carbamoyladeninyl,

N6,N6-dimethyladeninyl, 3-methylcytosinyl, 5-methylcytosinyl, 2-thiocytosinyl, 5-formylcytosinyl,

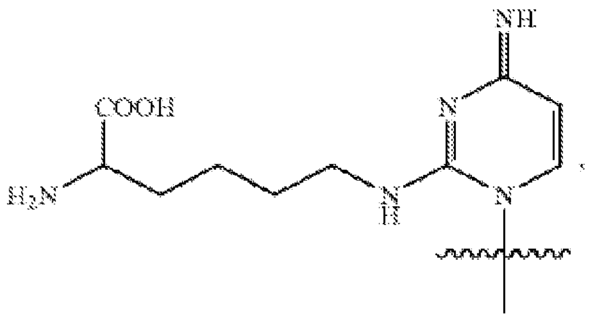

N4-methylcytosinyl, 5-hydroxymethylcytosinyl, 1-methylguaninyl, N2-methylguaninyl, 7-methylguaninyl, N2,N2-dimethylguaninyl, N2,7-dimethylguaninyl, N2,N2,7-trimethylguaninyl, 1-methylguaninyl, 7-cyano-7-deazaguaninyl, 7-aminomethyl-7-deazaguaninyl, pseudouracilyl, dihydrouracilyl, 5-methyluracilyl, 1-methylpseudouracilyl, 2-thiouracilyl, 4-thiouracilyl, 2-thiothyminyl, 5-methyl-2-thiouracilyl, 3-(3-amino-3-carboxypropyl)uracilyl, 5-hydroxyuracilyl, 5-methoxyuracilyl, uracilyl 5-oxyacetic acid, uracilyl 5-oxyacetic acid methyl ester, 5-(carboxyhydroxymethyl)uracilyl, 5-(carboxyhydroxymethyl)uracilyl methyl ester, 5-methoxycarbonylmethyluracilyl, 5-methoxycarbonylmethyl-2-thiouracilyl, 5-aminomethyl-2-thiouracilyl, 5-methylaminomethyluracilyl, 5-methylaminomethyl-2-thiouracilyl, 5-methylaminomethyl-2-selenouracilyl, 5-carbamoylmethyluracilyl, 5-carboxymethylaminomethyluracilyl, 5-carboxymethylaminomethyl-2-thiouracilyl, 3-methyluracilyl, 1-methyl-3-(3-amino-3-carboxypropyl)pseudouracilyl, 5-carboxymethyluracilyl, 5-methyldihydrouracilyl, 3-methylpseudouracilyl,

| | |
|---|---|
| 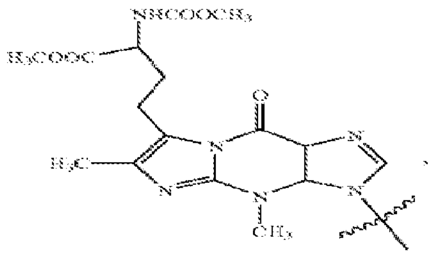 , 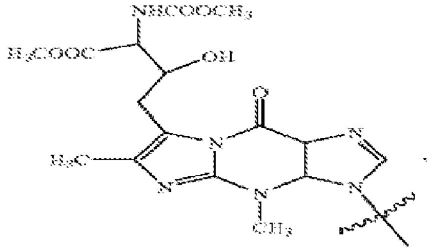 , 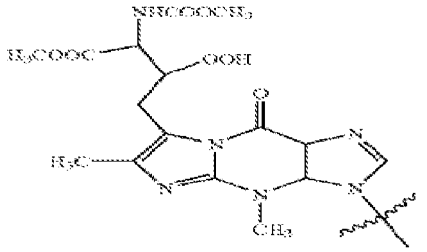 , 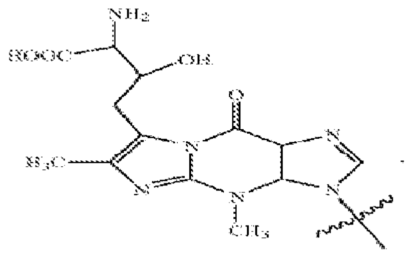 , 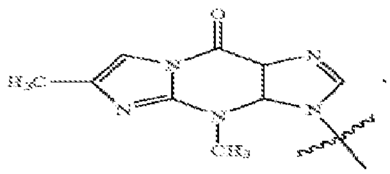 , 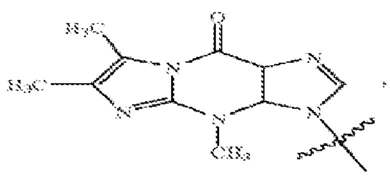 , 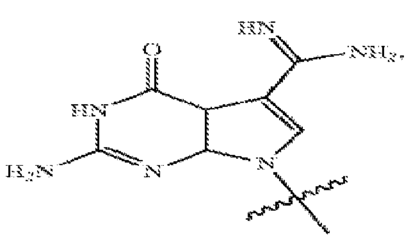 | 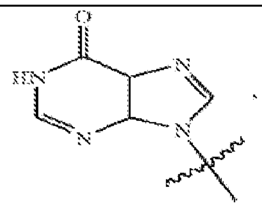 , 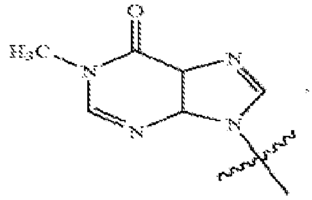 , 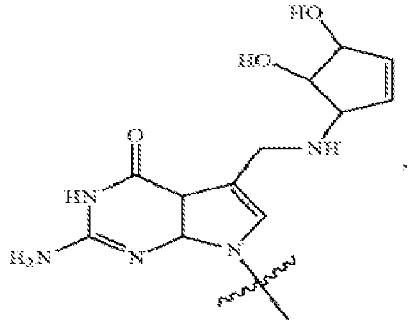 , 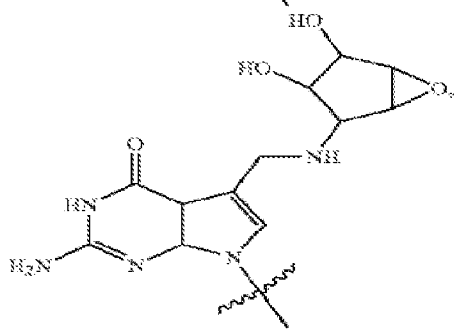 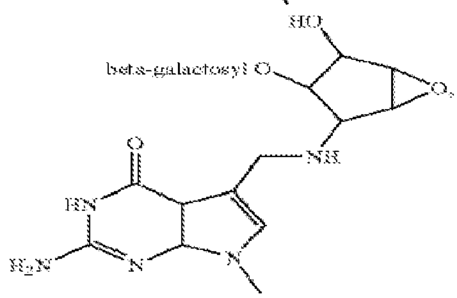 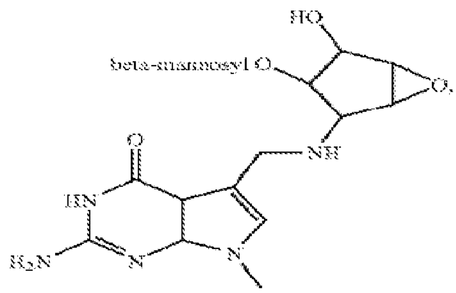 |

A universal base can form base pairs with each of the natural DNA/RNA bases, exhibiting relatively little discrimination between them. In general, the universal bases are non-hydrogen bonding, hydrophobic, aromatic moieties which can stabilize e.g., duplex RNA or RNA-like molecules, via stacking interactions. A universal base can also include hydrogen bonding substituents.

As used herein, a "universal base" can include anthracenes, pyrenes or any one of the following:

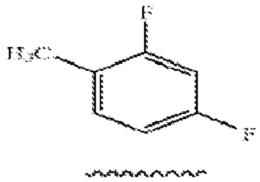 , 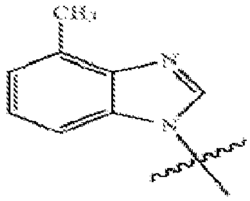 ,

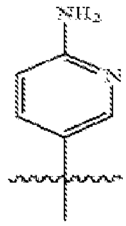 , 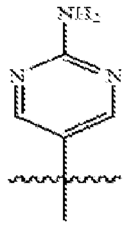 , 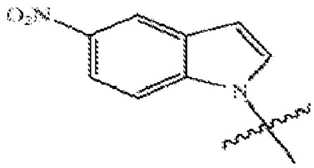 ,

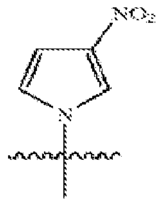 , 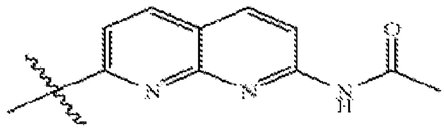 ,

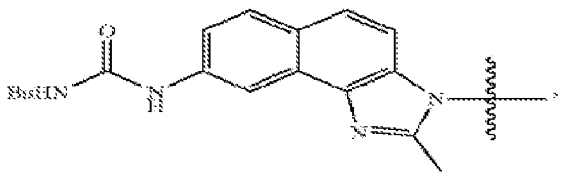 ,

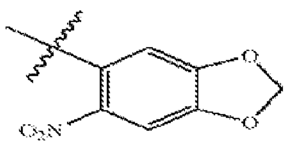 , 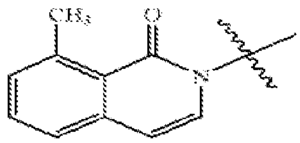 ,

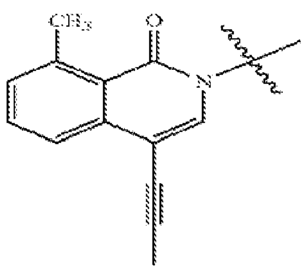 ,

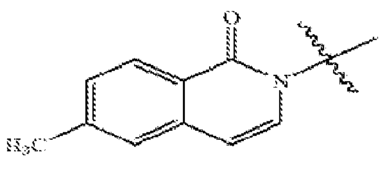 ,

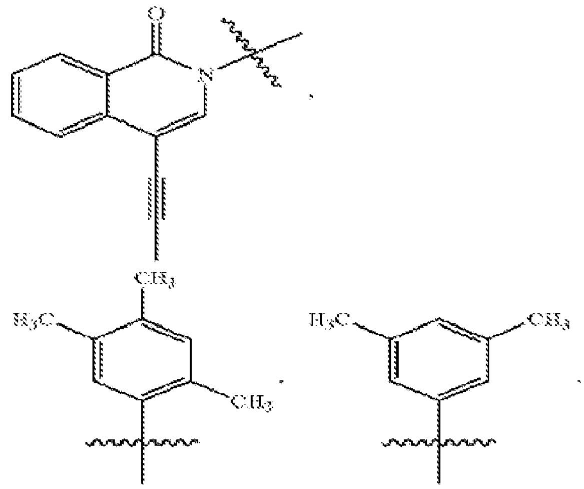

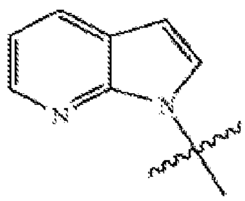,

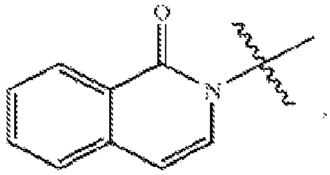,

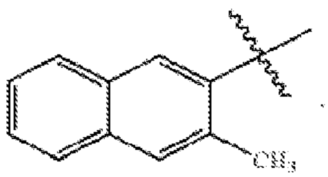,

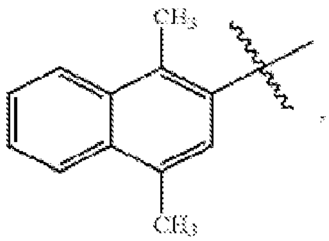,

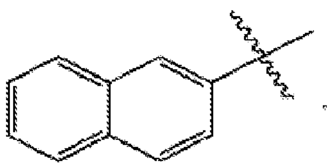,

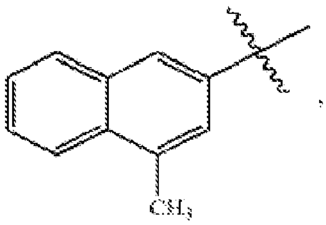, and

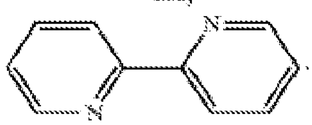.

In some embodiments, B can form part of a tether that connects a ligand to the carrier. For example, the tether can be B-CH=CH-C(O)NH-(CH2)5-NHC(O)-LIGAND. In a preferred embodiment, the double bond is trans, and the ligand is a substituted or unsubstituted cholesterolyl radical (e.g., attached through the D-ring side chain or the C-3

hydroxyl); an aralkyl moiety having at least one sterogenic center and at least one substituent on the aryl portion of the aralkyl group; or a nucleobase. In certain embodiments, B, in the tether described above, is uracilyl or a universal base, e.g., an aryl moiety, e.g., phenyl, optionally having additional substituents, e.g., one or more fluoro groups. B can be substituted at any atom with the remainder of the tether.

X2 can include "oxy" or "deoxy" substituents in place of the 2'-OH or be a ligand or a tethered ligand.

Examples of "oxy"-substituents include alkoxy or aryloxy (OR, e.g., R=H, alkyl, cycloalkyl, aryl, aralkyl, heteroaryl, sugar, or protecting group); polyethyleneglycols (PEG), $O(CH_2CH_2O)_nCH_2CH_2OR$; "locked" nucleic acids (LNA) in which the 2' hydroxyl is connected, e.g., by a methylene bridge, to the 4' carbon of the same ribose sugar; O-PROTECTED AMINE (AMINE = $NH_2$; alkylamino, dialkylamino, heterocyclyl, arylamino, diaryl amino, heteroaryl amino, or diheteroaryl amino, ethylene diamine, polyamino) and aminoalkoxy, $O(CH_2)_n$PROTECTED AMINE, (e.g., AMINE = $NH_2$; alkylamino, dialkylamino, heterocyclyl, arylamino, diaryl amino, heteroaryl amino, or diheteroaryl amino, ethylene diamine, polyamino), and orthoester. Amine protecting groups can include formyl, amido, benzyl, allyl, etc.

In some embodiments, the orthoester has the general formula J. The groups R31 and R32 may be the same or different and can be any combination of the groups listed in FIG. 2B of US 7,582,744, hereby incorporated by reference. An exemplary orthoester is the "ACE" group, shown below as structure K.

J

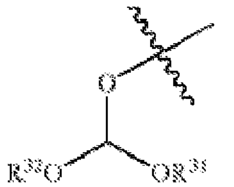

K

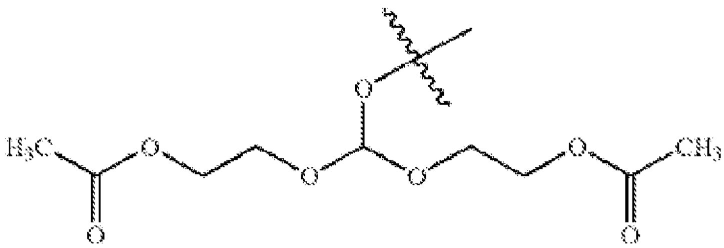

"Deoxy" substituents include hydrogen (i.e. deoxyribose sugars); halo (e.g., fluoro); protected amino (e.g. $NH_2$; alkylamino, dialkylamino, heterocyclyl, arylamino, diaryl amino, heteroaryl amino, diheteroaryl amino, or amino acid in which all amino are protected); fully protected polyamino (e.g., $NH(CH_2CH_2NH)_nCH_2CH_2$-AMINE, wherein AMINE = $NH_2$; alkylamino, dialkylamino, heterocyclyl, arylamino, diaryl amino, heteroaryl amino, or diheteroaryl amino and all amino groups are protected), -NHC(O)R (R=alkyl, cycloalkyl, aryl, aralkyl, heteroaryl or sugar), cyano; alkyl-thio-alkyl; thioalkoxy; and alkyl, cycloalkyl, aryl, alkenyl and alkynyl, which may be optionally substituted with e.g., a protected amino functionality. Preferred substitutents are 2'-methoxyethyl, 2'-$OCH_3$, 2'-O-allyl, 2'-C-allyl, and 2'-fluoro.

X3 is as described for OFG2 above.

PG can be a triarylmethyl group (e.g., a dimethoxytrityl group) or Si(X5')(X5")(X5''') in which (X5'), (X5"), and (X5''') are as described elsewhere.

**Sugar Replacement-Based Monomers**

Cyclic sugar replacement-based monomers, e.g., sugar replacement-based ligand-conjugated monomers, are also referred to herein as sugar replacement monomer subunit (SRMS) monomer compounds. Preferred carriers have the general formula (LCM-2) provided below. (In that structure preferred backbone attachment points can be chosen from R1 or R2; R3 or R4; or R9 and R10 if Y is CR9R10 (two positions are chosen to give two backbone attachment points, e.g., R1 and R4, or R4 and R9). Preferred tethering attachment points include R7; R5 or R6 when X is CH2. The carriers are described below as an entity, which can be incorporated into a strand. Thus, it is understood that the structures also encompass the situations wherein one (in the case of a terminal position) or two (in the case of an internal position) of the attachment points, e.g., R1 or R2; R3 or R4; or R9 or R10 (when Y is CR9R10), is connected to the phosphate, or modified phosphate, e.g., sulfur containing, backbone. E.g., one of the above-named R groups can be -CH2-, wherein

one bond is connected to the carrier and one to a backbone atom, e.g., a linking oxygen or a central phosphorus atom.

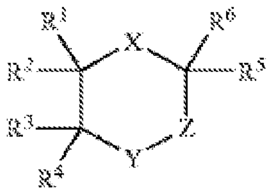

(LCM-2)

in which,

X is N(CO)R7, NR7 or CH2;
Y is NR8, O, S, CR9R10;
Z is CR11R12 or absent;
Each of R1, R2, R3, R4, R9, and R10 is, independently, H, ORa, or (CH2)nORb, provided that at least two of R1, R2, R3, R4, R9, and R10 are ORa and/or (CH2)nORb;
Each of R5, R6, R11, and R12 is, independently, a ligand, H, C1-C6 alkyl optionally substituted with 1-3 R13, or C(O)NHR7; or R5 and R11 together are C3-C8 cycloalkyl optionally substituted with R14;
R7 can be a ligand, e.g., R7 can be Rd, or R7 can be a ligand tethered indirectly to the carrier, e.g., through a tethering moiety, e.g., C1-C20 alkyl substituted with NRcRd; or C1-C20 alkyl substituted with NHC(O)Rd;
R8 is H or C1-C6 alkyl;
R13 is hydroxy, C1-C4 alkoxy, or halo;
R14 is NRcR7;
R15 is C1-C6 alkyl optionally substituted with cyano, or C2-C6 alkenyl;
R16 is C1-C10 alkyl;
R17 is a liquid or solid phase support reagent;
L is --C(O)(CH2)qC(O)--, or --C(O)(CH2)qS--;
Ra is a protecting group, e.g., CAr3; (e.g., a dimethoxytrityl group) or Si(X5')(X5")(X5‴) in which (X5'), (X5"), and (X5‴) are as described elsewhere.
Rb is P(O)(O-)H, P(OR15)N(R16)2 or L-R17;
Rc is H or C1-C6 alkyl;
Rd is H or a ligand;
Each Ar is, independently, C6-C10 aryl optionally substituted with C1-C4 alkoxy;
n is 1-4; and q is 0-4.

Exemplary carriers include those in which, e.g., X is N(CO)R7 or NR7, Y is CR9R10, and Z is absent; or X is N(CO)R7 or NR7, Y is CR9R10, and Z is CR11R12; or X is N(CO)R7 or NR7, Y is NR8, and Z is CR11R12; or X is N(CO)R7 or NR7, Y is O, and Z is CR11R12; or X is CH2; Y is CR9R10; Z is CR11R12, and R5 and R11 together form C6 cycloalkyl (H, z=2), or the indane ring system, e.g., X is CH2; Y is CR9R10; Z is CR11R12, and R5 and R11 together form C5 cycloalkyl (H, z=1).

In certain embodiments, the carrier may be based on the pyrroline ring system or the 4-hydroxyproline ring system, e.g., X is N(CO)R7 or NR7, Y is CR9R10, and Z is absent (D). OFG1 is preferably attached to a primary carbon, e.g., an exocyclic alkylene

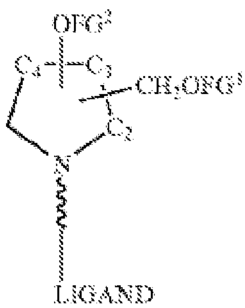

D

group, e.g., a methylene group, connected to one of the carbons in the five-membered ring (-CH2OFG1 in D). OFG2 is

preferably attached directly to one of the carbons in the five-membered ring (-OFG2 in D). For the pyrroline-based carriers, -CH2OFG1 may be attached to C-2 and OFG2 may be attached to C-3; or -CH2OFG1 may be attached to C-3 and OFG2 may be attached to C-4. In certain embodiments, CH2OFG1 and OFG2 may be geminally substituted to one of the above-referenced carbons. For the 3-hydroxyproline-based carriers, - CH2OFG1 may be attached to C-2 and OFG2 may be attached to C-4. The pyrroline- and 4-hydroxyproline-based monomers may therefore contain linkages (e.g., carbon-carbon bonds) wherein bond rotation is restricted about that particular linkage, e.g. restriction resulting from the presence of a ring. Thus, CH2OFG1 and OFG2 may be cis or trans with respect to one another in any of the pairings delineated above Accordingly, all cis/trans isomers are expressly included. The monomers may also contain one or more asymmetric centers and thus occur as racemates and racemic mixtures, single enantiomers, individual diastereomers and diastereomeric mixtures. All such isomeric forms of the monomers are expressly included (e.g., the centers bearing CH2OFG1 and OFG2 can both have the R configuration; or both have the S configuration; or one center can have the R configuration and the other center can have the S configuration and vice versa). The tethering attachment point is preferably nitrogen. Preferred examples of carrier D include the following:

| | |
|---|---|
| 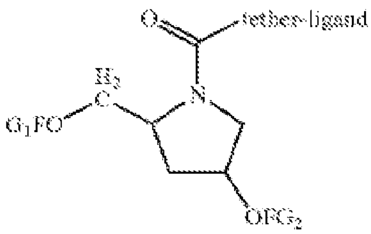 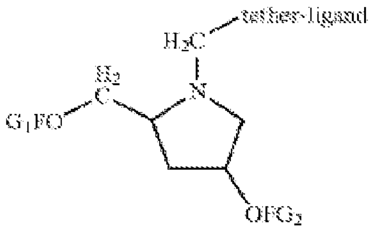 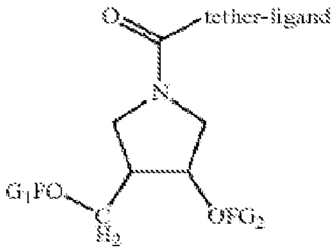 | 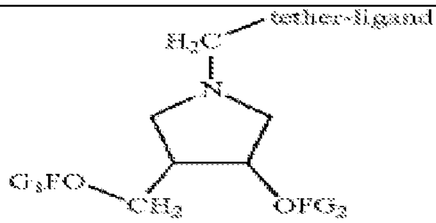 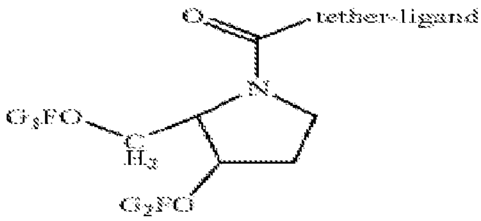 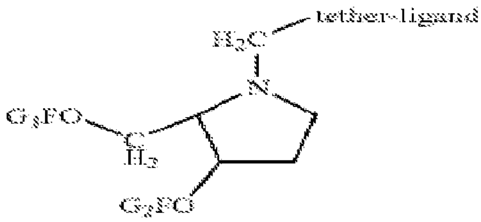 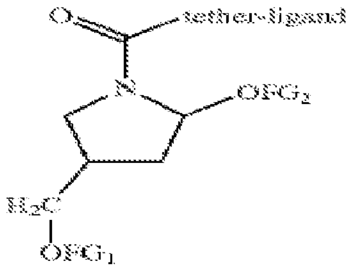 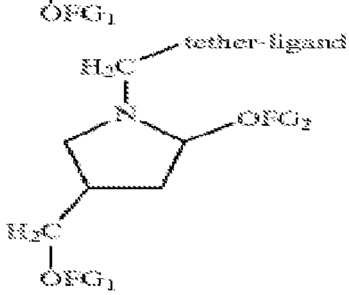 |

In certain embodiments, the carrier may be based on the piperidine ring system (E), e.g., X is N(CO)R7 or NR7, Y is CR9R10, and Z is CR11R12. OFG1 is preferably

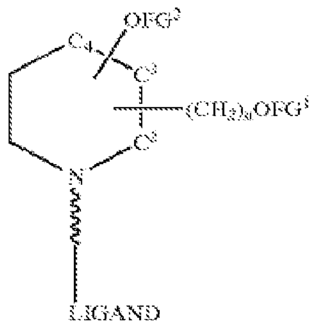

E

attached to a primary carbon, e.g., an exocyclic alkylene group, e.g., a methylene group (n=1) or ethylene group (n=2), connected to one of the carbons in the six-membered ring [-(CH2)nOFG1 in E]. OFG2 is preferably attached directly to one of the carbons in the six-membered ring (-OFG2 in E). -(CH2)nOFG1 and OFG2 may be disposed in a geminal manner on the ring, i.e., both groups may be attached to the same carbon, e.g., at C-2, C-3, or C-4. Alternatively, -(CH2)nOFG1 and OFG2 may be disposed in a vicinal manner on the ring, i.e., both groups may be attached to adjacent ring carbon atoms, e.g., -(CH2)nOFG1 may be attached to C-2 and OFG2 may be attached to C-3; -(CH2)nOFG1 may be attached to C-3 and OFG2 may be attached to C-2; -(CH2)nOFG1 may be attached to C-3 and OFG2 may be attached to C-4; or -(CH2)nOFG1 may be attached to C-4 and OFG2 may be attached to C-3. The piperidine-based monomers may therefore contain linkages (e.g., carbon-carbon bonds) wherein bond rotation is restricted about that particular linkage, e.g. restriction resulting from the presence of a ring. Thus, -(CH2)nOFG1 and OFG2 may be cis or trans with respect to one another in any of the pairings delineated above. Accordingly, all cis/trans isomers are expressly included. The monomers may also contain one or more asymmetric centers and thus occur as racemates and racemic mixtures, single enantiomers, individual diastereomers and diastereomeric mixtures. All such isomeric forms of the monomers are expressly included (e.g., the centers bearing CH2OFG1 and OFG2 can both have the R configuration; or both have the S configuration; or one center can have the R configuration and the other center can have the S configuration and vice versa). The tethering attachment point is preferably nitrogen.

In certain embodiments, the carrier may be based on the piperazine ring system (F), e.g., X is N(CO)R7 or NR7, Y is NR8, and Z is CR11R12, or the morpholine ring system (G), e.g., X is N(CO)R7 or NR7, Y is O, and Z is CR11R12. OFG1 is preferably

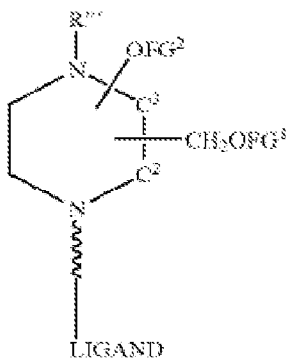

F

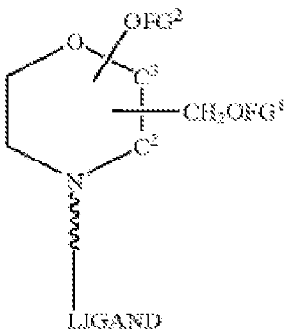

G

attached to a primary carbon, e.g., an exocyclic alkylene group, e.g., a methylene group, connected to one of the carbons in the six-membered ring (-CH2OFG1 in F or G). OFG2 is preferably attached directly to one of the carbons in the six-membered rings (-OFG2 in F or G). For both F and G, -CH2OFG1 may be attached to C-2 and OFG2 may be attached to C-3; or vice versa. In certain embodiments, CH2OFG1 and OFG2 may be geminally substituted to one of the above-referenced carbons. The piperazine- and morpholine-based monomers may therefore contain linkages (e.g., carbon-carbon bonds) wherein bond rotation is restricted about that particular linkage, e.g. restriction resulting from the presence of a ring. Thus, CH2OFG1 and OFG2 may be cis or trans with respect to one another in any of the pairings delineated above. Accordingly, all cis/trans isomers are expressly included. The monomers may also contain one or more asymmetric centers and thus occur as racemates and racemic mixtures, single enantiomers, individual diastereomers and diastereomeric mixtures. All such isomeric forms of the monomers are expressly included (e.g., the centers bearing CH2OFG1 and OFG2 can both have the R configuration; or both have the S configuration; or one center can have the R configuration and the other center can have the S configuration and vice versa). R''' can be, e.g., C1-C6 alkyl, preferably CH3. The tethering attachment point is preferably nitrogen in both F and G.

In certain embodiments, the carrier may be based on the decalin ring system, e.g., X is CH2; Y is CR9R10; Z is CR11R12, and R5 and R11 together form C6 cycloalkyl (H, z=2), or the indane ring system, e.g., X is CH2; Y is CR9R10; Z is CR11R12, and R5 and R11 together form C5 cycloalkyl (H, z=1). OFG1 is preferably attached to a primary carbon,

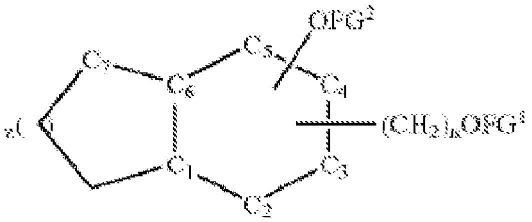

H

e.g., an exocyclic methylene group (n=1) or ethylene group (n=2) connected to one of C-2, C-3, C-4, or C-5 [-(CH2)nOFG1 in H]. OFG2 is preferably attached directly to one of C-2, C-3, C-4, or C-5 (-OFG2 in H). -(CH2)nOFG1 and OFG2 may be disposed in a geminal manner on the ring, i.e., both groups may be attached to the same carbon, e.g., at C-2, C-3, C-4, or C-5. Alternatively, -(CH2)nOFG1 and OFG2 may be disposed in a vicinal manner on the ring, i.e., both groups may be attached to adjacent ring carbon atoms, e.g., -(CH2)nOFG1 may be attached to C-2 and OFG2 may be attached to C-3; -(CH2)nOFG1 may be attached to C-3 and OFG2 may be attached to C-2; -(CH2)nOFG1 may be attached to C-3 and OFG2 may be attached to C-4; or -(CH2)nOFG1 may be attached to C-4 and OFG2 may be attached to C-3; -(CH2)nOFG1 may be attached to C-4 and OFG2 may be attached to C-5; or -(CH2)nOFG1 may be attached to C-5 and OFG2 may be attached to C-4. The decalin or indane-based monomers may therefore contain linkages (e.g., carbon-carbon bonds) wherein bond rotation is restricted about that particular linkage, e.g. restriction resulting from the presence of a ring. Thus, -(CH2)nOFG1 and OFG2 may be cis or trans with respect to one another in any of the pairings delineated above. Accordingly, all cis/trans isomers are expressly included. The monomers may also contain one or more asymmetric centers and thus occur as racemates and racemic mixtures, single enantiomers, individual diastereomers and diastereomeric mixtures. All such isomeric forms of the monomers are expressly included (e.g., the centers bearing CH2OFG1 and OFG2 can both have the R configuration; or both have the S configuration; or one center can have the R configuration and the other center can have the S configuration and vice versa). In a preferred embodiment, the substituents at C-1 and C-6 are trans with respect to one another. The tethering attachment point is preferably C-6 or C-7.

Other carriers may include those based on 3-hydroxyproline (J). Thus, - (CH2)nOFG1 and OFG2 may be cis

or trans with respect to one another. Accordingly, all cis/trans isomers are expressly included. The monomers may also contain one or more asymmetric centers

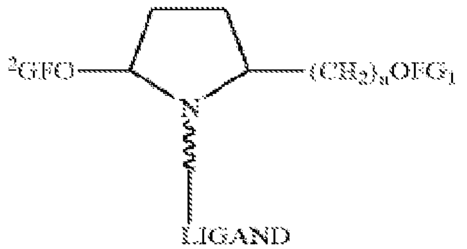

and thus occur as racemates and racemic mixtures, single enantiomers, individual diastereomers and diastereomeric mixtures. All such isomeric forms of the monomers are expressly included (e.g., the centers bearing CH2OFG1 and OFG2 can both have the R configuration; or both have the S configuration; or one center can have the R configuration and the other center can have the S configuration and vice versa). The tethering attachment point is preferably nitrogen.

Representative cyclic, sugar replacement-based carriers are shown in FIG. 3 of US 7,582,744, hereby incorporated by reference.

Sugar Replacement-Based Monomers (Acyclic)

Acyclic sugar replacement-based monomers, e.g., sugar replacement-based ligand-conjugated monomers, are also referred to herein as sugar replacement monomer subunit (SRMS) monomer compounds. Preferred acyclic carriers can have formula LCM-3 or LCM-4 below.

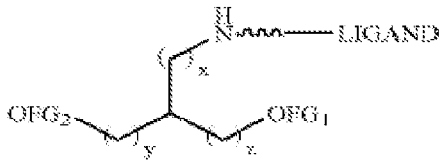

LCM-3

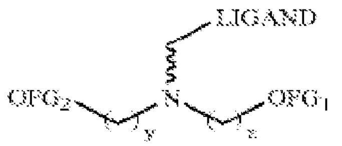

LCM-4

In some embodiments, each of x, y, and z can be, independently of one another, 0, 1, 2, or 3. In formula LCM-3, when y and z are different, then the tertiary carbon can have either the R or S configuration. In preferred embodiments, x is zero and y and z are each 1 in formula LCM-3 (e.g., based on serinol), and y and z are each 1 in formula LCM-3. Each of formula LCM-3 or LCM-4 below can optionally be substituted, e.g., with hydroxy, alkoxy, perhaloalkyl.

**Tethers**

In certain embodiments, a moiety, e.g., a ligand may be connected indirectly to the carrier via the intermediacy of an intervening tether. Tethers are connected to the carrier at a tethering attachment point (TAP) and may include any C1-C100 carbon-containing moiety, (e.g. C1-C75, C1-C50, C1-C20, C1-C10; C1, C2, C3, C4, C5, C6, C7, C8, C9, or C10), preferably having at least one nitrogen atom. In preferred embodiments, the nitrogen atom forms part of a terminal amino or amido (NHC(O)-) group on the tether, which may serve as a connection point for the ligand. Preferred tethers (underlined) include TAP-(CH2)nNH-; TAP-C(O)(CH2)nNH-; TAP-NR″″(CH2)nNH-, TAP-C(O)--(CH2)n--C(O)--; TAP-C(O)-(CH2)n-C(O)O-; TAP-C(O)-O-; TAP-C(O)--(CH2)n-NH-C(O)--; TAP-C(O)-(CH2)n-; TAP-C(O)-NH-; TAP-C(O)-; TAP-(CH2)n-C(O)--; TAP-(CH2)n--C(O)O--; TAP-(CH2)n-; or TAP-(CH2)n-NH-C(O)-; in which n is 1-20 (e.g., 1, 2, 3, 4, 5, 6, 7, 8, 9, 10, 11, 12, 13, 14, 15, 16, 17, 18, 19, or 20) and R"" is C1-C6 alkyl. Preferably, n is 5, 6, or 11. In other embodiments, the nitrogen may form part of a terminal oxyamino group, e.g., -ONH2, or hydrazino group, -NHNH2. The tether may optionally be substituted, e.g., with hydroxy, alkoxy, perhaloalkyl, and/or optionally inserted with one or more additional heteroatoms, e.g., N, O, or S. Preferred tethered ligands may include, e.g., TAP-(CH2)nNH(LIGAND); TAP-C(O)(CH2)nNH(LIGAND); TAP-NR″″(CH2)nNH(LIGAND); TAP-(CH2)nONH(LIGAND; TAP-C(O)(CH2)nONH(LIGAND); TAP-NR″″(CH2)nONH(LIGAND); TAP-(CH2)nNHNH2(LIGAND), TAP-C(O)(CH2)nNHNH2(LIGAND); TAP-NR″″(CH2)nNHNH2(LIGAND); TAP-C(O)-(CH2)n-C(O)(LIGAND); TAP-C(O)-(CH2)n-C(O)O(LIGAND); TAP-C(O)-O(LIGAND); TAP-C(O)-(CH2)n-NH-C(O)(LIGAND); TAP-C(O)-(CH2)n(LIGAND); TAP-C(O)-NH(LIGAND); TAP-C(O)(LIGAND); TAP-(CH2)n-C(O) (LIGAND); TAP-(CH2)n—C(O)O(LIGAND); TAP-(CH2)n(LIGAND); or TAP-(CH2)n-NH-C(O)(LIGAND). In some embodiments, amino terminated tethers (e.g., NH2, ONH2, NH2NH2) can form an imino bond (i.e., C=N) with the ligand. In some embodiments, amino terminated tethers (e.g., NH2, ONH2, NH2NH2) can acylated, e.g., with C(O)CF3.

In some embodiments, the tether can terminate with a mercapto group (i.e., SH) or an olefin (e.g., CH=CH2). For example, the tether can be TAP-(CH2)n-SH, TAP-C(O)(CH2)nSH, TAP-(CH2)n-(CH=CH2), or TAP-C(O)(CH2)n(CH=CH2), in which n can be as described elsewhere. In certain embodiments, the olefin can be a Diels-Alder diene or dienophile. The tether may optionally be substituted, e.g., with hydroxy, alkoxy, perhaloalkyl, and/or optionally inserted with one or more additional heteroatoms, e.g., N, O, or S. The double bond can be cis or trans or E or Z.

In other embodiments the tether may include an electrophilic moiety, preferably at the terminal position of the tether. Preferred electrophilic moieties include, e.g., an aldehyde, alkyl halide, mesylate, tosylate, nosylate, or brosylate, or an activated carboxylic acid ester, e.g. an NHS ester, or a pentafluorophenyl ester. Preferred tethers (underlined) include TAP-(CH2)nCHO; TAP-C(O)(CH2)nCHO; or TAP-NR″″(CH2)nCHO, in which n is 1-6 and R"" is C1-C6 alkyl; or TAP-(CH2)nC(O)ONHS; TAP-C(O)(CH2)nC(O)ONHS; or TAP-NR″″(CH2)nC(O)ONHS, in which n is 1-6 and R″″ is C1-C6 alkyl; TAP-(CH2)nC(O)OC6F5; TAP-C(O)(CH2)nC(O)OC6F5; or TAP-NR″″(CH2)nC(O)OC6F5, in which n is 1-11 and R″″ is C1-C6 alkyl; or -(CH2)nCH2LG; TAP-C(O)(CH2)nCH2LG; or TAP-NR""(CH2)nCH2LG, in which n can be as described elsewhere and R″″ is C1-C6 alkyl (LG can be a leaving group, e.g., halide, mesylate, tosylate, nosylate, brosylate). Tethering can be carried out by coupling a nucleophilic group of a ligand, e.g., a thiol or amino group with an electrophilic group on the tether.

In other embodiments, it can be desirable for the ligand-conjugated monomer or a ligand-conjugated monomer to include a phthalimido group (K) at the terminal position of the tether.

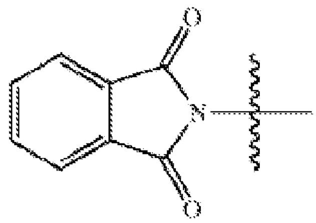

K

In other embodiments, other protected amino groups can be at the terminal position of the tether, e.g., alloc, monomethoxy trityl (MMT), trifluoroacetyl, Fmoc, or aryl sulfonyl (e.g., the aryl portion can be ortho-nitrophenyl or ortho, para-dinitrophenyl).

Any of the tethers described herein may further include one or more additional linking groups, e.g., -O-(CH2)n-, -(CH2)n-SS-, -(CH2)n-, or -(CH=CH)-.

**Tethered Ligands**

A wide variety of entities can be tethered to an oligonucleotide agent, e.g., to the carrier of a ligand-conjugated monomer. Examples are described below in the context of a ligand-conjugated monomer but that is only one preferred embodiment. Entities can be coupled at other points to an oligonucleotide agent.

A ligand tethered to an oligonucleotide agent (e.g., an oligonucleotide agent targeting an miRNA) can have a favorable effect on the agent. For example, the ligand can improve stability, hybridization thermodynamics with a target nucleic acid, targeting to a particular tissue or cell-type, or cell permeability, e.g., by an endocytosis-dependent or -independent mechanism. Ligands and associated modifications can also increase sequence specificity and consequently decrease off-site targeting.

A tethered ligand can include one or more modified bases or sugars that can function as intercalators. These are preferably located in an internal region, such as in a bulge of a miRNA/target duplex. The intercalator can be an aromatic, e.g., a polycyclic aromatic or heterocyclic aromatic compound. A polycyclic intercalator can have stacking capabilities, and can include systems with 2, 3, or 4 fused rings. The universal bases described herein can be included on a ligand.

In one embodiment, the ligand can include a cleaving group that contributes to target gene inhibition by cleavage of the target nucleic acid. The cleaving group can be, for example, a bleomycin (e.g., bleomycin-A5, bleomycin-A2, or

bleomycin-B2), pyrene, phenanthroline (e.g., O-phenanthroline), a polyamine, a tripeptide (e.g., lys-tyr-lys tripeptide), or metal ion chelating group. The metal ion chelating group can include, e.g., an Lu(III) or EU(III) macrocyclic complex, a Zn(II) 2,9-dimethylphenanthroline derivative, a Cu(II) terpyridine, or acridine, which can promote the selective cleavage of target RNA at the site of the bulge by free metal ions, such as Lu(III). In some embodiments, a peptide ligand can be tethered to a miRNA to promote cleavage of the target RNA, e.g., at the bulge region. For example, 1,8-dimethyl-1,3,6,8,10,13-hexaazacyclotetradecane (cyclam) can be conjugated to a peptide (e.g., by an amino acid derivative) to promote target RNA cleavage.

A tethered ligand can be an aminoglycoside ligand, which can cause an oligonucleotide agent to have improved hybridization properties or improved sequence specificity. Exemplary aminoglycosides include glycosylated polylysine, galactosylated polylysine, neomycin B, tobramycin, kanamycin A, and acridine conjugates of aminoglycosides, such as Neo-N-acridine, Neo-S-acridine, Neo-C-acridine, Tobra-N-acridine, and KanaA-N-acridine. Use of an acridine analog can increase sequence specificity. For example, neomycin B has a high affinity for RNA as compared to DNA, but low sequence-specificity. An acridine analog, neo-S-acridine has an increased affinity for the HIV Rev-response element (RRE). In some embodiments the guanidine analog (the guanidinoglycoside) of an aminoglycoside ligand is tethered to an oligonucleotide agent. In a guanidinoglycoside, the amine group on the amino acid is exchanged for a guanidine group. Attachment of a guanidine analog can enhance cell permeability of an oligonucleotide agent, e.g., an oligonucleotide agent targeting an miRNA or pre-miRNA.

A tethered ligand can be a poly-arginine peptide, peptoid or peptidomimetic, which can enhance the cellular uptake of an oligonucleotide agent.

Preferred moieties are ligands, which are coupled, preferably covalently, either directly or indirectly via an intervening tether, to the ligand-conjugated carrier. In preferred embodiments, the ligand is attached to the carrier via an intervening tether. As discussed above, the ligand or tethered ligand may be present on the monomer when the monomer is incorporated into the growing strand. In some embodiments, the ligand may be incorporated into a "precursor" a ligand-conjugated monomer subunit after a "precursor" a ligand-conjugated monomer has been incorporated into the growing strand. For example, a monomer having, e.g., an amino-terminated tether, e.g., TAP-(CH2)nNH2 may be incorporated into a growing oligonucleotide strand. In a subsequent operation, i.e., after incorporation of the precursor monomer into the strand, a ligand having an electrophilic group, e.g., a pentafluorophenyl ester or aldehyde group, can subsequently be attached to the precursor monomer subunit by coupling the electrophilic group of the ligand with the terminal nucleophilic group of the precursor monomer subunit tether.

In preferred embodiments, a ligand alters the distribution, targeting or lifetime of an oligonucleotide agent into which it is incorporated. In preferred embodiments a ligand provides an enhanced affinity for a selected target, e.g, molecule, cell or cell type, compartment, e.g., a cellular or organ compartment, tissue, organ or region of the body, as, e.g., compared to a species absent such a ligand.

Preferred ligands can improve transport, hybridization, and specificity properties and may also improve nuclease resistance of the resultant natural or modified oligoribonucleotide, or a polymeric molecule comprising any combination of monomers described herein and/or natural or modified ribonucleotides.

Ligands in general can include therapeutic modifiers, e.g., for enhancing uptake; diagnostic compounds or reporter groups e.g., for monitoring distribution; cross-linking agents; nuclease-resistance conferring moieties; and natural or unusual nucleobases. General examples include lipophiles, lipids, sterols, steroids (e.g., uvaol, hecigenin, diosgenin), terpenes (e.g., triterpenes, e.g., sarsasapogenin, Friedelin, epifriedelanol derivatized lithocholic acid), vitamins (e.g., folic acid, vitamin A, biotin, pyridoxal), carbohydrates, proteins, protein binding agents, integrin targeting molecules, polycationics, peptides, polyamines, and peptide mimics.

Ligands can include a naturally occurring substance, (e.g., human serum albumin (HSA), low-density lipoprotein (LDL), or globulin); carbohydrate (e.g., a dextran, pullulan, chitin, chitosan, inulin, cyclodextrin or hyaluronic acid); amino acid, or a lipid. The ligand may also be a recombinant or synthetic molecule, such as a synthetic polymer, e.g., a synthetic polyamino acid. Examples of polyamino acids include polyamino acid is a polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an alpha helical peptide.

Ligands can also include targeting groups, e.g., a cell or tissue targeting agent, e.g., a lectin, glycoprotein, lipid or protein, e.g., an antibody, that binds to a specified cell type such as a kidney cell. A targeting group can be a thyrotropin, melanotropin, lectin, glycoprotein, surfactant protein A, Mucin carbohydrate, multivalent lactose, multivalent galactose, N-acetyl-galactosamine, N-acetyl-glucosamine, multivalent mannose, multivalent fucose, glycosylated polyaminoacids, multivalent galactose, transferrin, bisphosphonate, polyglutamate, polyaspartate, a lipid, cholesterol, a sterol, a steroid, bile acid, folate, vitamin B12, biotin, or an RGD peptide or RGD peptide mimetic.

Other examples of ligands include dyes, intercalating agents (e.g. acridines and substituted acridines), cross-linkers (e.g. psoralene, mitomycin C), porphyrins (TPPC4, texaphyrin, Sapphyrin), polycyclic aromatic hydrocarbons (e.g., phenazine, dihydrophenazine, phenanthroline, pyrenes), lys-tyr-lys tripeptide, aminoglycosides, guanidium aminoglycodies, artificial endonucleases (e.g. EDTA), lipophilic molecules, e.g, cholesterol (and thio analogs thereof), cholic acid, cholanic acid, lithocholic acid, adamantane acetic acid, 1-pyrene butyric acid, dihydrotestosterone, glycerol (e.g., esters (e.g., mono, bis, or tris fatty acid esters, e.g., C10, C11, C12, C13, C14, C15, C16, C17, C18, C19, or C20 fatty acids) and ethers thereof, e.g., C10, C11, C12, C13, C14, C15, C16, C17, C18, C19, or C20 alkyl; e.g., 1,3-bis-O(hexadecyl)glycerol, 1,3-bis-O(octaadecyl)glycerol), geranyloxyhexyl group, hexadecylglycerol, borneol, menthol, 1,3-propanediol, heptadecyl group, palmitic acid, stearic acid (e.g., gyceryl distearate), oleic acid, myristic acid, O3-(oleoyl)lithocholic acid, O3-(oleoyl)cholenic acid, dimethoxytrityl, or phenoxazine)and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g. biotin), transport/absorption facilitators (e.g., aspirin, naproxen, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP.

Ligands can be proteins, e.g., glycoproteins, or peptides, e.g., molecules having a specific affinity for a co-ligand, or antibodies e.g., an antibody, that binds to a specified cell type such as a cancer cell, endothelial cell, or bone cell. Ligands may also include hormones and hormone receptors. They can also include non-peptidic species, such as lipids, lectins, carbohydrates, vitamins, cofactors, multivalent lactose, multivalent galactose, N-acetyl-galactosamine, N-acetyl-gulucosamine multivalent mannose, or multivalent fucose. The ligand can be, for example, a lipopolysaccharide, an activator of p38 MAP kinase, or an activator of NF-κB

The ligand can be a substance, e.g, a drug, which can increase the uptake of the oligonucleotide agent into the cell, for example, by disrupting the cell's cytoskeleton, e.g., by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin.

The ligand can increase the uptake of the oligonucleotide agent into the cell by activating an inflammatory response, for example. Exemplary ligands that would have such an effect include tumor necrosis factor alpha (TNFalpha), interleukin-1 beta, or gamma interferon.

In one aspect, the ligand is a lipid or lipid-based molecule. Such a lipid or lipid-based molecule preferably binds a serum protein, e.g., human serum albumin (HSA). An HSA binding ligand allows for distribution of the conjugate to a target tissue, e.g., a non-kidney target tissue of the body. For example, the target tissue can be the liver, including parenchymal cells of the liver. Other molecules that can bind HSA can also be used as ligands. For example, neproxin or aspirin can be used. A lipid or lipid-based ligand can (a) increase resistance to degradation of the conjugate, (b) increase targeting or transport into a target cell or cell membrane, and/or (c) can be used to adjust binding to a serum protein, e.g., HSA.

A lipid based ligand can be used to modulate, e.g., control the binding of the conjugate to a target tissue. For example, a lipid or lipid-based ligand that binds to HSA more strongly will be less likely to be targeted to the kidney and therefore less likely to be cleared from the body. A lipid or lipid-based ligand that binds to HSA less strongly can be used to target the conjugate to the kidney.

In an embodiment, the lipid based ligand binds HSA. A lipid-based ligand can bind HSA with a sufficient affinity such that the conjugate will be preferably distributed to a non-kidney tissue. However, it is preferred that the affinity not be so strong that the HSA-ligand binding cannot be reversed.

In another preferred embodiment, the lipid based ligand binds HSA weakly or not at all, such that the conjugate will be distributed to the kidney. Other moieties that target to kidney cells can also be used in place of or in addition to the lipid based ligand.

In another aspect, the ligand is a moiety, e.g., a vitamin, which is taken up by a target cell, e.g., a proliferating cell. These are particularly useful for treating disorders characterized by unwanted cell proliferation, e.g., of the malignant or non-malignant type, e.g., cancer cells. Exemplary vitamins include vitamin A, E, and K. Other exemplary vitamins include are B vitamin, e.g., folic acid, B12, riboflavin, biotin, pyridoxal or other vitamins or nutrients taken up by cancer cells. Also included are HSA and low density lipoprotein (LDL).

In another aspect, the ligand is a cell-permeation agent, preferably a helical cell-permeation agent. Preferably, the agent is amphipathic. An exemplary agent is a peptide such as tat or antennopedia. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

Peptides that target markers enriched in proliferating cells can be used. E.g., RGD containing peptides and peptidomimetics can target cancer cells, in particular cells that exhibit an $\alpha v\beta 3$ integrin. Thus, one could use RGD peptides, cyclic peptides containing RGD, RGD peptides that include D-amino acids, as well as synthetic RGD mimics. In addition to RGD, one can use other moieties that target the $\alpha v$-$\beta 3$ integrin ligand. Generally, such ligands can be used to control proliferating cells and angiogeneis. Preferred conjugates of this type include an oligonucleotide agent that

targets PECAM-1, VEGF, or other cancer gene, e.g., a cancer gene described herein.

The oligonucleotide agents of the invention are particularly useful when targeted to the liver. For example, a single stranded oligonucleotide agent featured in the invention can target an miRNA enriched in the liver, and the oligonucleotide agent can include a ligand for enhanced delivery to the liver. An oligonucleotide agent can be targeted to the liver by incorporation of a monomer derivatized with a ligand which targets to the liver. For example, a liver-targeting agent can be a lipophilic moiety. Preferred lipophilic moieties include lipid, cholesterols, oleyl, retinyl, or cholesteryl residues. Other lipophilic moieties that can function as liver-targeting agents include cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine.

An oligonucleotide agent can also be targeted to the liver by association with a low-density lipoprotein (LDL), such as lactosylated LDL. Polymeric carriers complexed with sugar residues can also function to target oligonucleotide agents to the liver.

A targeting agent that incorporates a sugar, e.g., galactose and/or analogues thereof, is particularly useful. These agents target, in particular, the parenchymal cells of the liver. For example, a targeting moiety can include more than one or preferably two or three galactose moieties, spaced about 15 angstroms from each other. The targeting moiety can alternatively be lactose (e.g., three lactose moieties), which is glucose coupled to a galactose. The targeting moiety can also be N-Acetyl-Galactosamine, N-Ac-Glucosamine. A mannose or mannose-6-phosphate targeting moiety can be used for macrophage targeting.

The ligand can be a peptide or peptidomimetic. A peptidomimetic (also referred to herein as an oligopeptidomimetic) is a molecule capable of folding into a defined three-dimensional structure similar to a natural peptide. The attachment of peptide and peptidomimetics to oligonucleotide agents can affect pharmacokinetic distribution of the iRNA, such as by enhancing cellular recognition and absorption. The peptide or peptidomimetic moiety can be about 5-50 amino acids long, e.g., about 5, 10, 15, 20, 25, 30, 35, 40, 45, or 50 amino acids long (see Table A below, for example). The SEQ. ID numbers below are taken from US 7,582,744, which is hereby incorporated by reference.

## Table A

Exemplary Cell Permeation Peptides

| Cell Permeation Peptide | Amino acid Sequence | Reference |
|---|---|---|
| Penetratin | RQIKIWFQNRRMKWKK (SEQ ID NO:1) | Derossi et al., J. Biol. Chem. 269:10444, 1994 |
| Tat fragment (48-60) | GRKKRRQRRRPPQC (SEQ ID NO:2) | Vives et al., J. Biol. Chem., 272:16010, 1997 |
| Signal Sequence-based peptide | GALFLGWLGAAGSTMGAWSQPKKKRKV (SEQ ID NO:3) | Chaloin et al., Biochem. Biophys. Res. Commun., 243:601, 1998 |
| PVEC | LLIILRRRIRKQAHAHSK (SEQ ID NO:4) | Elmquist et al., Exp. Cell Res., 269:237, 2001 |
| Transportan | GWTLNSAGYLLKINLKALAALAKKIL (SEQ ID NO:5) | Pooga et al., FASEB J., 12:67, 1998 |
| Amphiphilic model peptide | KLALKLALKALKAALKLA (SEQ ID NO:6) | Oehlke et al., Mol. Ther., 2:339, 2000 |
| $Arg_9$ | RRRRRRRRR (SEQ ID NO:7) | Mitchell et al., J. Pept. Res., 56:318, 2000 |
| Bacterial cell wall permeating | KFFKFFKFFK (SEQ ID NO:8) | |
| LL-37 | LLGDFFRKSKEKIGKEFKRIVQRIKDFLRNLVPRTES (SEQ ID NO:9) | |
| Cecropin P1 | SWLSKTAKKLENSAKKRISEGIAIAIQGGPR (SEQ ID NO:10) | |
| α-defensin | ACYCRIPACIAGERRYGTCIYQGRLWAFCC (SEQ ID NO:11) | |

Exemplary cell Permeation Peptides

| Cell Permeation Peptide | Amino acid Sequence | Reference |
|---|---|---|
| b-defensin | DHYNCVSSGGQCLYSACPIFTKIQGTCYR GKAKCCK (SEQ ID NO:12) | |
| Bactenecin | RKCRIVVIRVCR (SEQ ID NO:13) | |
| PR-39 | RRRPRPPYLPRPRPPPFFPPRLPPRIPPGFPP RFPPRFPGKR-NH2 (SEQ ID NO:14) | |
| Indolicidin | ILPWKWPWWPWRR-NH2 (SEQ ID NO:15) | |

In some embodiments, an oligonucleotide agent (referred to as "NA" in formula OT-I through OT-IV below, e.g., RNA, DNA, chimeric RNA-DNA, DNA-RNA, RNA-DNA-RNA, or DNA-RNA-DNA) can be chemically modified by conjugating a moiety that includes a ligand having one or more chemical linkages for attachment of the ligand (L) to the oligonucleotide or nucleic acid. The ligand of an oligonucleotide agent can be coupled by one or both of a tether and linker. In the diagram below, exemplary chemical linkages are represented as X, Y, and Z. These can be part of the tether or linker.

(OT-I)

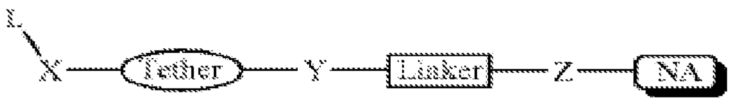


(OT-II)

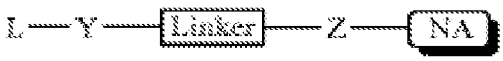


(OT-III)

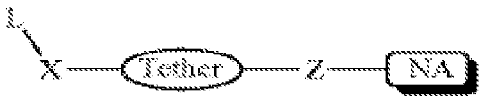


(OT-IV)

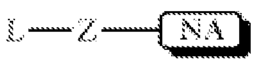


Ligands can be attached at one or both of the 3' end, the 5' end, and internal positions. In certain embodiments, the oligonucleotide agent can be chemically modified by conjugating one or more moieties having formula OT-I. Table B, below, shows a variety of conjugates.

## Table B

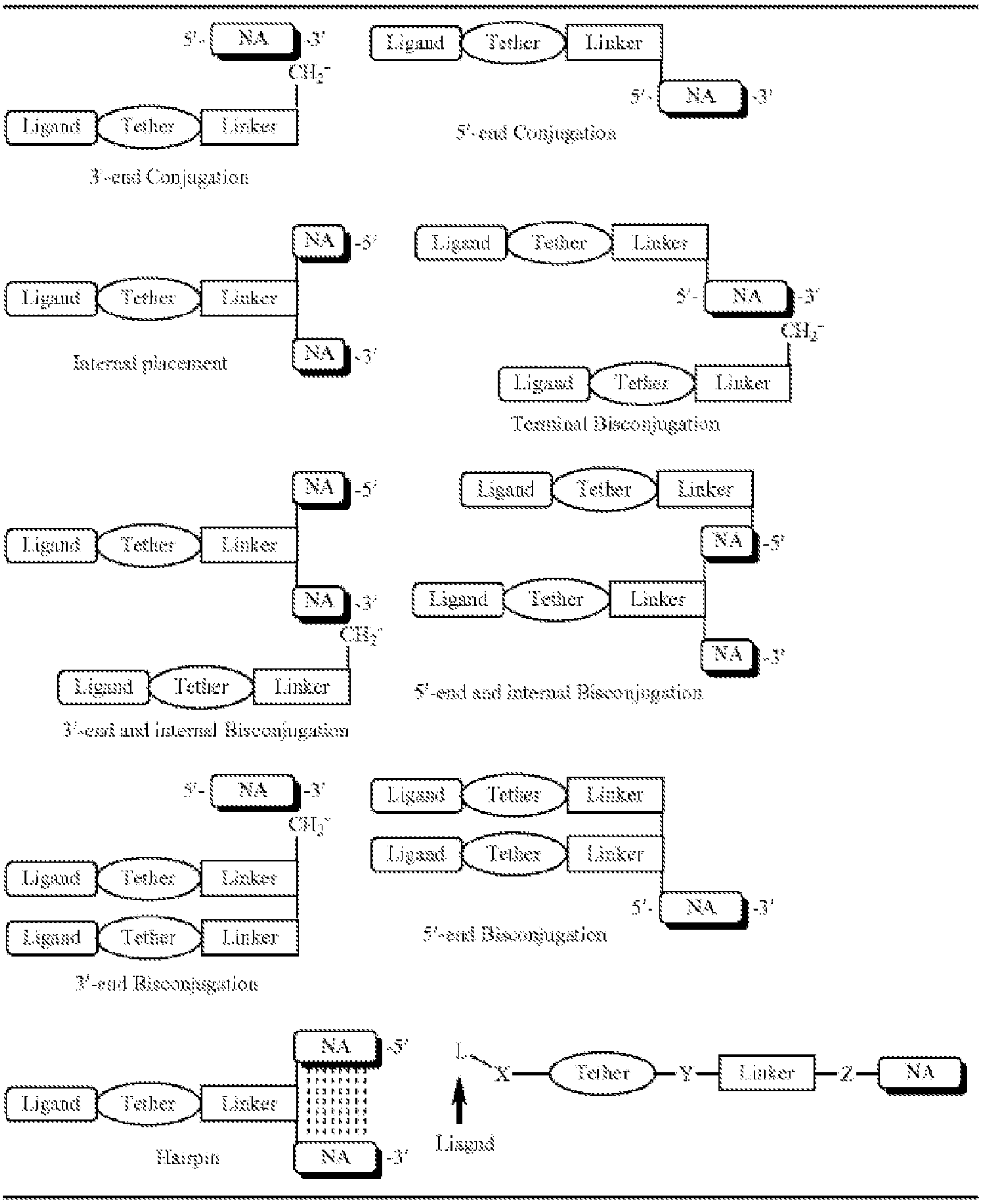


Exemplary ligands are listed in Table C and are discussed elsewhere herein. The exemplary ligands (L) shown in Table C are suitable for use in certain embodiments.

## TABLE C

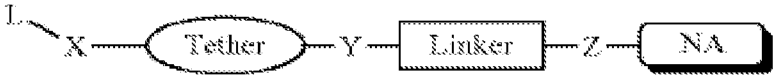


L =
Cholesterol
Thiocholesterol
5β-Cholanic Acid
Cholic acid
Lithocholic acid
Biotin
Vitamin E
Naproxen
Ibuprofen
Amines (mono, di, tri, tetraalkyl or aryl)
Folate
Sugar (N-Acetylgalactosamine, galactosamine, galactose, Mannose)
$—(CH_2)_nNQ_1Q_2$, where n = 0-40, $Q_1$, $Q_2$ = H, Me or Et; $Q_1$ = H, $Q_2$ = H, Me, Et or aryl
$—CH_2)_pCH═CH(CH_2)_qNQ_1Q_2$, where p and/or q = 0-40, $Q_1$, $Q_2$ = H, Me or Et; $Q_1$ = H, $Q_2$ = H, Me, Et or aryl with E and/or Z configuration
$—(CH_2)_pCH═CH(CH_2)_qNQ_1Q_2$, where p and/or q = 0-40, $Q_1$, $Q_2$ = H, Me or Et; $Q_1$ = H, $Q_2$ = H, Me, Et or aryl
$—(CH_2)_pCH═CH(CH_2)_qCH═CH(CH_2)_rNQ_1Q_2$, where p, q and/or r = 0-40, $Q_1$, $Q_2$ = H, Me or Et; $Q_1$ = H, $Q_2$ = H, Me, Et or aryl with E and/or Z configuration
$—O(CH_2)_m(OCH_2CH_2)_n—OR$, where m, n = 0-40 and R = H, Me, $NQ_1Q_2$, —C(O)NR'R"—C(S)NR'R"
$—NH(CH_2)_m(OCH_2CH_2)_n—OR$, where m, n = 0-40 and R = H, Me, $NQ_1Q_2$, —C(O)NR'R"—C(S)NR'R"
$—O(CH_2)_m(NHCH_2CH_2)_n—R$, where m, n = 0-40 and R = H, OH, Me, $NQ_1Q_2$, —C(O)NR'R"—C(S)NR'R"
$—NH(CH_2)_m(NHCH_2CH_2)_n—R$, where m, n = 0-40 and R = H, OH, Me, $NQ_1Q_2$, —C(O)NR'R"—C(S)NR'R"
Dialkylglycerol (sn3, sn1, sn2 and racemic) with number of methylene varies from 0-40
Diacylglycerol (sn3, sn1, sn2 and racemic) with number of methylene varies from 0-40
Dialkylglycerol (sn3, sn1, sn2 and racemic) with number of methylene varies from 0-40

and the alkyl chian contains one or more double bonds with E and/or Z isomers

Diacylglycerol (sn3, sn1, sn2 and racemic) with number of methylene varies from 0-40

and the alkyl chian contains one or more double bonds with E and/or

Exemplary X, Y, and Z moieties are shown in Table D. The X, Y, and Z moieties can be selected independently of one another.

## Table D

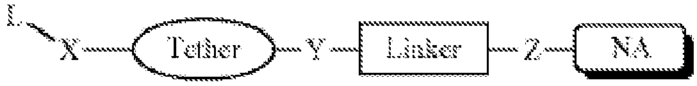


| | | |
|---|---|---|
| X = —NHC(O)— | Y = —NHC(O)— | Z = —NHC(O) |
| —C(O)NH— | —C(O)NH— | —C(O)NH— |
| —OC(O)NH— | —OC(O)NH— | —OC(O)NH— |
| —NHC(O)O— | —NHC(O)O— | —NHC(O)O— |
| —O— | —O— | —O— |
| —SS— | —SS— | —SS— |
| —S(O)— | —S(O)— | —S(O)— |
| —S($O_2$)— | —S($O_2$)— | —S($O_2$)— |
| —NHC(O)NH— | —NHC(O)NH— | —NHC(O)NH— |
| —NHC(S)NH— | —NHC(S)NH— | —NHC(S)NH— |
| —C(O)O— | —C(O)O— | —C(O)O— |
| —OC(O)— | —OC(O)— | —OC(O)— |
| —NHC(S)— | —NHC(S)— | —NHC(S)— |
| —NHC(S)O— | —NHC(S)O— | —NHC(S)O— |
| —C(S)NH— | —C(S)NH— | —C(S)NH— |
| —OC(S)NH— | —OC(S)NH— | —OC(S)NH— |
| —NHC(S)O— | —NHC(S)O— | —NHC(S)O— |
| —$CH_2$— | —$CH_2$— | —$CH_2$— |
| —$CH_2$CH=CH— | —$CH_2$CH=CH— | —$CH_2$CH=CH— |
| —C(O)CH=CH— | —C(O)CH=CH— | —C(O)CH=CH— |
| —NH—$CH_2$CH=CH— | —NH—$CH_2$CH=CH— | —NH—$CH_2$CH=CH— |
| —O—P(O)(OH)—O— | —O—P(O)(OH)—O— | —O—P(O)(OH)—O— |
| —O—P(S)(OH)—O— | —O—P(S)(OH)—O— | —O—P(S)(OH)—O— |
| —O—P(S)(SH)—O— | —O—P(S)(SH)—O— | —O—P(S)(SH)—O— |
| —S—P(O)(OH)—O— | —S—P(O)(OH)—O— | —S—P(O)(OH)—O— |
| —O—P(O)(OH)—S— | —O—P(O)(OH)—S— | —O—P(O)(OH)—S— |
| —S—P(O)(OH)—S— | —S—P(O)(OH)—S— | —S—P(O)(OH)—S— |
| —O—P(S)(OH)—S— | —O—P(S)(OH)—S— | —O—P(S)(OH)—S— |
| —S—P(S)(OH)—O— | —S—P(S)(OH)—O— | —S—P(S)(OH)—O— |
| —O—P(O)(R)—O— | —O—P(O)(R)—O— | —O—P(O)(R)—O— |
| —O—P(S)(R)—O— | —O—P(S)(R)—O— | —O—P(S)(R)—O— |
| —S—P(O)(R)—O— | —S—P(O)(R)—O— | —S—P(O)(R)—O— |
| —S—P(S)(R)—O— | —S—P(S)(R)—O— | —S—P(S)(R)—O— |
| —S—P(O)(R)—S— | —S—P(O)(R)—S— | —S—P(O)(R)—S— |
| —O—P(S)(R)—S— | —O—P(S)(R)—S— | —O—P(S)(R)—S— |

R = Alkyl, fluroalkyl, aryl or aralkyl

Exemplary tethers are shown in Table E.

# Table E

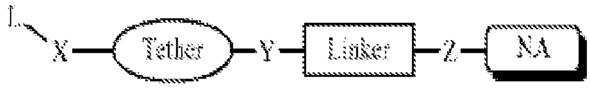


Tether:

$-(CH_2)_n-$, where n = 1-40
$-(CH_2-CH_2O)_n-$, where n = 1-20
$-O(CH_2-CH_2O)_n-$, where n = 1-20
$-(CH_2-CH_2NH)_n-$, where n = 1-20
$-NH(CH_2-CH_2NH)_n-$, where n = 1-20
$-(CH_2)_l[(CH=CH)_m(CH_2)_n]_p(CH=CH)_q(CH_2)_r-$,
where l, m, n, p, q and/or r = 0-20
$-(CH_2)_l[(C\equiv C)_m(CH_2)_n]_p(C\equiv C)_q(CH_2)_r-$,
where l, m, n, p, q and/or r = 0-20

-continued

| Linker = | | |
|---|---|---|
| 3'-end | 5'-end | interior |
| 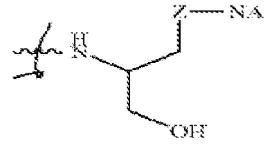 | 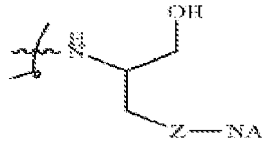 | 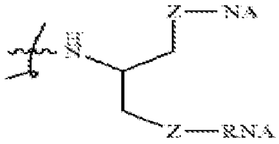 |
| 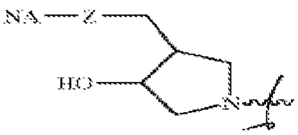 | 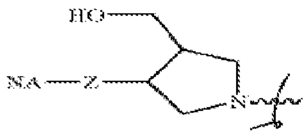 | 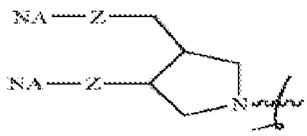 |
| 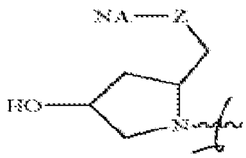 | 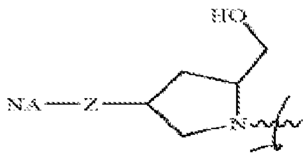 | 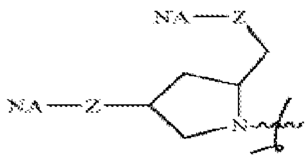 |
| 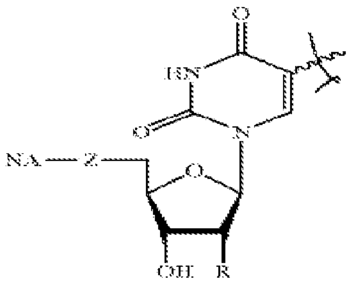 | 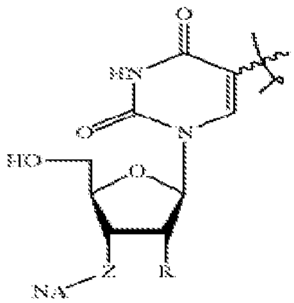 | 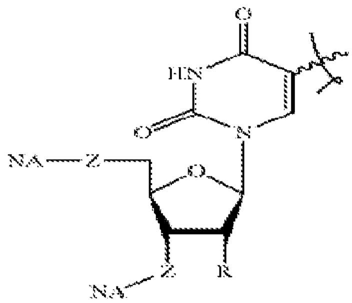 |
| 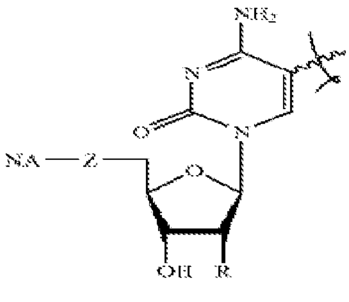 | 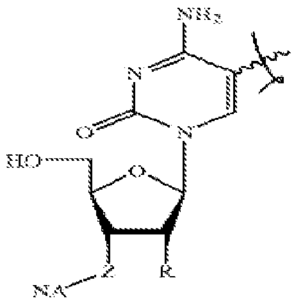 | 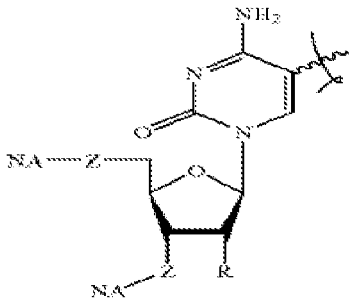 |
| | 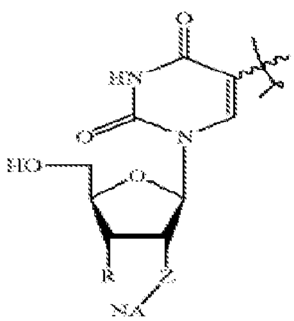 | 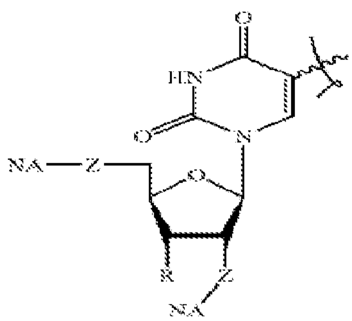 |

-continued

119

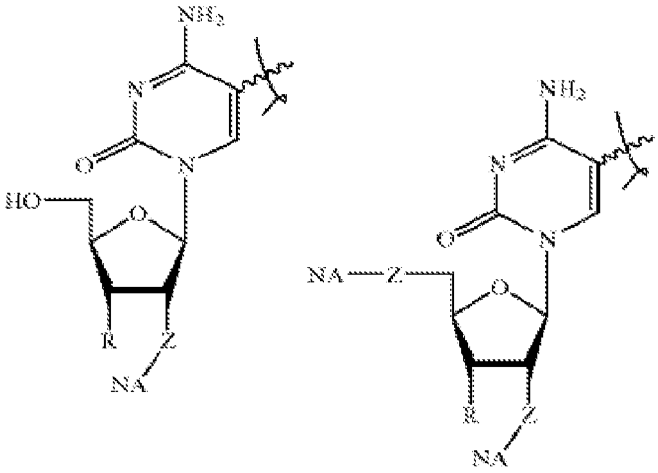

## Oligonucleotide Agent Structure

An oligonucleotide agent that is NAT ("nucleic acid targeting") includes a region of sufficient complementarity to the target gene, and is of sufficient length in terms of nucleotides, such that the oligonucleotide agent forms a duplex with the target nucleic acid. The oligonucleotide agent can modulate the function of the targeted molecule. For example, when the targeted molecule is an mRNA or pre-mRNA, the NAT can inhibit gene expression; when the target is an miRNA, the NAT will inhibit the miRNA function and will thus up-regulate expression of the mRNAs targeted by the particular miRNA; when the target is a region of a pre-mRNA the affects splicing, the NAT can alter the choice of splice site and thus the mRNA sequence; when the NAT functions as an miRNA, expression of the targeted mRNA is inhibited. For ease of exposition the term nucleotide or ribonucleotide is sometimes used herein in reference to one or more monomeric subunits of an oligonucleotide agent. It will be understood herein that the usage of the term "ribonucleotide" or "nucleotide" herein can, in the case of a modified RNA or nucleotide surrogate, also refer to a modified nucleotide, or surrogate replacement moiety at one or more positions.

A NAT oligonucleotide agent is, or includes, a region that is at least partially, and in some embodiments fully, complementary to the target RNA. It is not necessary that there be perfect complementarity between the oligonucleotide agent and the target, but the correspondence must be sufficient to enable the oligonucleotide agent, or a cleavage product thereof, to modulate (e.g., inhibit) target gene expression.

An oligonucleotide agent will in certain embodiments have one or more of the following properties:

(1) it will be of the Formula 1, 2, 3, or 4 described below;

(2) it will have a 5' modification that includes one or more phosphate groups or one or more analogs of a phosphate group;

(3) it will, despite modifications, even to a very large number of bases specifically base pair and form a duplex structure with a homologous target RNA of sufficient thermodynamic stability to allow modulation of the activity of the targeted RNA;

(4) it will, despite modifications, even to a very large number, or all of the nucleosides, still have "RNA-like" properties, i.e., it will possess the overall structural, chemical and physical properties of an RNA molecule, even though not exclusively, or even partly, of ribonucleotide-based content. For example, all of the nucleotide sugars can contain e.g., 2'OMe, 2' fluoro in place of 2' hydroxyl. This deoxyribonucleotide-containing agent can still be expected to exhibit RNA-like properties. While not wishing to be bound by theory, the electronegative fluorine prefers an axial orientation when attached to the C2' position of ribose. This spatial preference of fluorine can, in turn, force the sugars to adopt a C3'-endo pucker. This is the same puckering mode as observed in RNA molecules and gives rise to the RNA-characteristic A-family-type helix. Further, since fluorine is a good hydrogen bond acceptor, it can participate in the same hydrogen bonding interactions with water molecules that are known to stabilize RNA structures. (Generally, it is preferred that a modified moiety at the 2' sugar position will be able to enter into hydrogen-bonding which is more characteristic of the 2'-OH moiety of a ribonucleotide than the 2'-H moiety of a deoxyribonucleotide. A preferred oligonucleotide agent will: exhibit a C3'-endo pucker in all, or at least 50, 75, 80, 85, 90, or 95% of its sugars; exhibit a C3'-endo pucker in a sufficient amount of its sugars that it can give rise to a the RNA-characteristic A-family-type helix; will have no more than 20, 10, 5, 4, 3, 2, or 1 sugar which is not a C3'-endo pucker

structure.

In certain embodiments, 2'-modifications with C3'-endo sugar pucker include:

2'-OH, 2'-O-Me, 2'-O-methoxyethyl, 2'-O-aminopropyl, 2'-F, 2'-O-CH2-CO-NHMe, 2'-O--CH2-CH2-O--CH2-CH2-N(Me)2, LNA

(5) regardless of the nature of the modification, and even though the oligonucleotide agent can contain deoxynucleotides or modified deoxynucleotides, it is advantageous in some embodiments that DNA molecules, or any molecule in which more than 50, 60, or 70% of the nucleotides in the molecule are deoxyribonucleotides, or modified deoxyribonucleotides which are deoxy at the 2' position, are excluded from the definition of oligonucleotide agent.

In certain embodiments, 2'-modifications with a C2'-endo sugar pucker include:
2'-H, 2'-Me, 2'-S-Me, 2'-Ethynyl, 2'-ara-F.

Sugar modifications can also include L-sugars and 2'-5'-linked sugars.

As used herein, "specifically hybridizable" and "complementary" are terms that are used to indicate a sufficient degree of complementarity such that stable and specific binding occurs between a compound of the invention and a target RNA molecule in the case of NAT oligonucleotides agents that bind target RNAs. Specific binding requires a sufficient lack of complementarity to non-target sequences under conditions in which specific binding is desired, i.e., under physiological conditions in the case of in vivo assays or therapeutic treatment, or in the case of in vitro assays, under conditions in which the assays are performed. It has been shown that a single mismatch between targeted and non-targeted sequences are sufficient to provide discrimination for siRNA targeting of an mRNA (Brummelkamp et al., Cancer Cell, 2002, 2:243).

In one embodiment, a NAT oligonucleotide agent is "sufficiently complementary" to a target RNA, such that the oligonucleotide agent inhibits production of protein encoded by the target mRNA. The target RNA can be, e.g., a pre-mRNA, mRNA, or miRNA endogenous to the subject. In another embodiment, the oligonucleotide agent is "exactly complementary" (excluding the SRMS containing subunit(s)) to a target RNA, e.g., the target RNA and the oligonucleotide agent can anneal to form a hybrid made exclusively of Watson-Crick base pairs in the region of exact complementarity. A "sufficiently complementary" target RNA can include a region (e.g., of at least 7 nucleotides) that is exactly complementary to a target RNA. Moreover, in some embodiments, the oligonucleotide agent specifically discriminates a single-nucleotide difference. In this case, the oligonucleotide agent only down-regulates gene expression if exact complementarity is found in the region the single-nucleotide difference.

Oligonucleotide agents discussed herein include otherwise unmodified RNA and DNA as well as RNA and DNA that have been modified, e.g., to improve efficacy, and polymers of nucleoside surrogates. Unmodified RNA refers to a molecule in which the components of the nucleic acid, namely sugars, bases, and phosphate moieties, are the same or essentially the same as that which occur in nature, preferably as occur naturally in the human body. The art has referred to rare or unusual, but naturally occurring, RNAs as modified RNAs, see, e.g., Limbach et al. (Nucleic Acids Res., 1994, 22:2183-2196). Such rare or unusual RNAs, often termed modified RNAs, are typically the result of a post transcriptional modification and are within the term unmodified RNA as used herein. Modified RNA, as used herein, refers to a molecule in which one or more of the components of the nucleic acid, namely sugars, bases, and phosphate moieties, are different from that which occur in nature, preferably different from that which occurs in the human body. While they are referred to as "modified RNAs" they will of course, because of the modification, include molecules that are not, strictly speaking, RNAs. Nucleoside surrogates are molecules in which the ribophosphate backbone is replaced with a non-ribophosphate construct that allows the bases to the presented in the correct spatial relationship such that hybridization is substantially similar to what is seen with a ribophosphate backbone, e.g., non-charged mimics of the ribophosphate backbone. Examples of all of the above are discussed herein.

As nucleic acids are polymers of subunits or monomers, many of the modifications described below occur at a position which is repeated within a nucleic acid, e.g., a modification of a base, or a phosphate moiety, or a non-linking O of a phosphate moiety. In some cases the modification will occur at all of the subject positions in the nucleic acid but in many, and infact in most cases it will not. By way of example, a modification may only occur at a 3' or 5' terminal position, may only occur in a terminal regions, e.g. at a position on a terminal nucleotide or in the last 2, 3, 4, 5, or 10 nucleotides of a strand. The ligand can be at attached at the 3' end, the 5' end, or at an internal position, or at a combination of these positions. For example, the ligand can be at the 3' end and the 5' end; at the 3' end and at one or more internal positions; at the 5' end and at one or more internal positions; or at the 3' end, the 5' end, and at one or more internal positions. E.g., a phosphorothioate modification at a non-linking O position may only occur at one or both termini, or may only occur in a terminal region, e.g., at a position on a terminal nucleotide or in the last 2, 3, 4, 5, or 10 nucleotides of the oligonucleotide. The 5' end can be phosphorylated.

Modifications and nucleotide surrogates are discussed below.

FORMULA 1

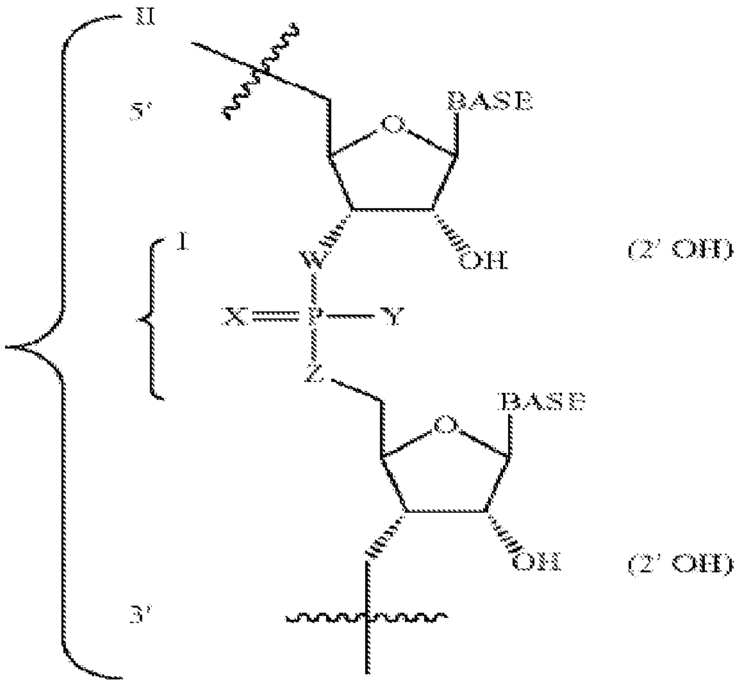

The scaffold presented above in Formula 1 represents a portion of a ribonucleic acid. The basic components are the ribose sugar, the base, the terminal phosphates, and phosphate internucleotide linkers. Where the bases are naturally occurring bases, e.g., adenine, uracil, guanine or cytosine, the sugars are the unmodified 2' hydroxyl ribose sugar (as depicted) and W, X, Y, and Z are all O, Formula 1 represents a naturally occurring unmodified oligoribonucleotide.

Unmodified oligoribonucleotides may be less than optimal in some applications, e.g., unmodified oligoribonucleotides can be prone to degradation by e.g., cellular nucleases. Nucleases can hydrolyze nucleic acid phosphodiester bonds. However, chemical modifications to one or more of the above RNA components can confer improved properties, and, e.g., can render oligoribonucleotides more stable to nucleases. Unmodified oligoribonucleotides may also be less than optimal in terms of offering tethering points for attaching ligands or other moieties to an oligonucleotide agent.

Modified nucleic acids and nucleotide surrogates can include one or more of:

(i) alteration, e.g., replacement, of one or both of the non-linking (X and Y) phosphate oxygens and/or of one or more of the linking (W and Z) phosphate oxygens (When the phosphate is in the terminal position, one of the positions W or Z will not link the phosphate to an additional element in a naturally occurring ribonucleic acid. However, for simplicity of terminology, except where otherwise noted, the W position at the 5' end of a nucleic acid and the terminal Z position at the 3' end of a nucleic acid, are within the term "linking phosphate oxygens" as used herein.);
(ii) alteration, e.g., replacement, of a constituent of the ribose sugar, e.g., of the 2' hydroxyl on the ribose sugar, or wholesale replacement of the ribose sugar with a structure other than ribose, e.g., as described herein;
(iii) wholesale replacement of the phosphate moiety (bracket I) with "dephospho" linkers;
(iv) modification or replacement of a naturally occurring base;
(v) replacement or modification of the ribose-phosphate backbone (bracket II);
(vi) modification of the 3' end or 5' end of the RNA, e.g., removal, modification or replacement of a terminal phosphate group or conjugation of a moiety, e.g. a fluorescently labeled moiety, to either the 3' or 5' end of RNA.

The terms replacement, modification, alteration, and the like, as used in this context, do not imply any process limitation, e.g., modification does not mean that one must start with a reference or naturally occurring ribonucleic acid and modify it to produce a modified ribonucleic acid but rather modified simply indicates a difference from a naturally occurring molecule.

It is understood that the actual electronic structure of some chemical entities cannot be adequately represented by only one canonical form (i.e. Lewis structure). While not wishing to be bound by theory, the actual structure can instead be some hybrid or weighted average of two or more canonical forms, known collectively as resonance forms or structures. Resonance structures are not discrete chemical entities and exist only on paper. They differ from one another only in the placement or "localization" of the bonding and nonbonding electrons for a particular chemical entity. It can

be possible for one resonance structure to contribute to a greater extent to the hybrid than the others. Thus, the written and graphical descriptions of the embodiments of the present invention are made in terms of what the art recognizes as the predominant resonance form for a particular species. For example, any phosphoroamidate (replacement of a nonlinking oxygen with nitrogen) would be represented by X=O and Y=N in the above figure.

Further Exemplary Oligonucleotide Agents

In one aspect, disclosed oligonucleotide agents have the following structure (see Formula 2 below):

FORMULA 2

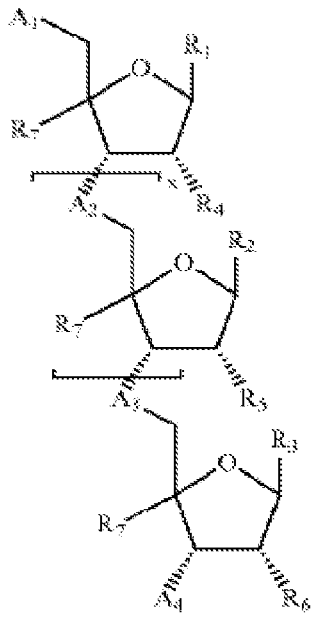

Referring to Formula 2 above, R1, R2, and R3 are each, independently, H, (i.e. abasic nucleotides), adenine, guanine, cytosine and uracil, inosine, thymine, xanthine, hypoxanthine, nubularine, tubercidine, isoguanisine, 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine, 7-deazaadenine, 7-deazaguanine, N6, N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3carboxypropyl)uracil, 3-methylcytosine, 5-methylcytosine, N4-acetyl cytosine, 2-thiocytosine, N6-methyladenine, N6-isopentyladenine, 2-methylthio-N6-isopentenyladenine, N-methylguanines, or O-alkylated bases.

R4, R5, and R6 are each, independently, OR8, O(CH2CH2O)mCH2CH2OR8; O(CH2)nR9; O(CH2)nOR9, H; halo; NH2; NHR8; N(R8)2; NH(CH2CH2NH)mCH2CH2NHR9; NHC(O)R8; cyano; mercapto, SR8; alkyl-thio-alkyl; alkyl, aralkyl, cycloalkyl, aryl, heteroaryl, alkenyl, alkynyl, each of which may be optionally substituted with halo, hydroxy, oxo, nitro, haloalkyl, alkyl, alkaryl, aryl, aralkyl, alkoxy, aryloxy, amino, alkylamino, dialkylamino, heterocyclyl, arylamino, diaryl amino, heteroaryl amino, diheteroaryl amino, acylamino, alkylcarbamoyl, arylcarbamoyl, aminoalkyl, alkoxycarbonyl, carboxy, hydroxyalkyl, alkanesulfonyl, alkanesulfonamido, arenesulfonamido, aralkylsulfonamido, alkylcarbonyl, acyloxy, cyano, or ureido; or R4, R5, or R6 together combine with R7 to form an [-O-CH2-] covalently bound bridge between the sugar 2' and 4' carbons.

$A^1$ is:

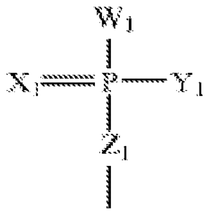 or 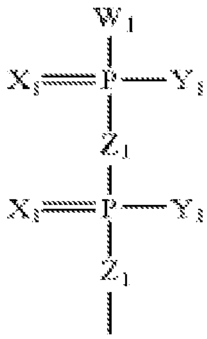 or 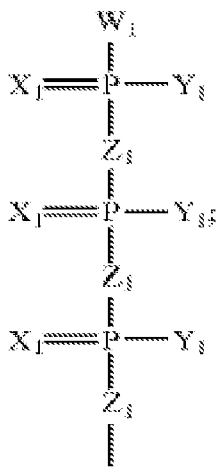

;

H; OH; OCH3; W1; an abasic nucleotide; or absent; (in some embodiments A1, especially with regard to anti-sense strands, is chosen from 5'-monophosphate ((HO)2(O)P--O-5'), 5'-diphosphate ((HO)2(O)P--O-P(HO)(O)-O-5'), 5'-triphosphate ((HO)2(O)P--O--(HO)(O)P--O--P(HO)(O)--O-5'), 5'-guano sine cap (7-methylated or non-methylated) (7m-G-O-5'-(HO)(O)P--O--(HO)(O)P--O-P(HO)(O)-O-5'), 5'-adenosine cap (Appp), and any modified or unmodified nucleotide cap structure (N-O-5'-(HO)(O)P--O--(HO)(O)P--O--P(HO)(O)--O-5'), 5'-monothiophosphate (phosphorothioate; (HO)2(S)P--O-5'), 5'-monodithiophosphate (phosphorodithioate; (HO)(HS)(S)P-0-5'), 5'-phosphorothiolate ((HO)2(O)P--S-5'); any additional combination of oxgen/sulfur replaced monophosphate, diphosphate and triphosphates (e.g. 5'-alpha-thiotriphosphate, 5'-gamma-thiotriphosphate, etc.), 5'-phosphoramidates ((HO)2(O)P--NH-5', (HO)(NH2)(O)P-O-5'), 5'-alkylphosphonates (R=alkyl=methyl, ethyl, isopropyl, propyl, etc., e.g. RP(OH)(O)-O-5'-, (OH)2(O)P-5'-CH2--), 5'-alkyletherphosphonates (R=alkylether=methoxymethyl (MeOCH2-), ethoxymethyl, etc., e.g. RP(OH)(O)--O-5'-)).

$A^2$ is:

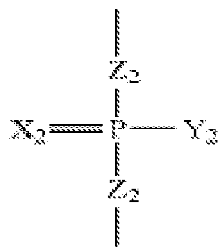

$A^3$ is:

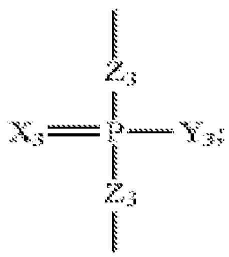

and

$A^4$ is:

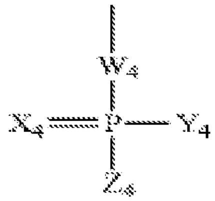 or 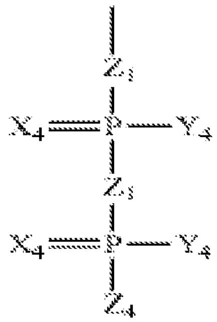 or 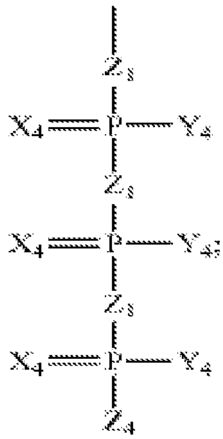 ;

H; Z4; an inverted nucleotide; an abasic nucleotide; or absent.

W1 is OH, (CH2)nR10, (CH2)nNHR10, (CH2)nOR10, (CH2)nSR10; O(CH2)nR10; O(CH2)nOR10, O(CH2)nNR10, O(CH2)nSR10; O(CH2)nSS(CH2)nOR10, O(CH2)nC(O)OR10, NH(CH2)nR10; NH(CH2)nNR10; NH(CH2)nOR10, NH(CH2)nSR10; S(CH2)nR10, S(CH2)nNR10, S(CH2)nOR10, S(CH2)nSR10 O(CH2CH2O)mCH2CH2OR10; O(CH2CH2O)mCH2CH2NHR10, NH(CH2CH2NH)mCH2CH2NHR10; Q-R10, O-Q-R10 N-Q-R10, S-Q-R10 or -O-. W4 is O, CH2, NH, or S.

X1, X2, X3, and X4 are each, independently, O or S.

Y1, Y2, Y3, and Y4 are each, independently, OH, O-, OR8, S, Se, BH3 -, H, NHR9, N(R9)2 alkyl, cycloalkyl, aralkyl, aryl, or heteroaryl, each of which may be optionally substituted.

Z1, Z2, and Z3 are each independently O, CH2, NH, or S. Z4 is OH, (CH2)nR10, (CH2)nNHR10, (CH2)nOR10, (CH2)nSR10; O(CH2)nR10; O(CH2)nOR10, O(CH2)nNR10, O(CH2)nSR10, O(CH2)nSS(CH2)nOR10, O(CH2)nC(O)OR10; NH(CH2)nR10; NH(CH2)nNR10; NH(CH2)nOR10, NH(CH2)nSR10; S(CH2)nR10, S(CH2)nNR10, S(CH2)nOR10, S(CH2)nSR10 O(CH2CH2O)mCH2CH2OR10, O(CH2CH2O)mCH2CH2NHR10, NH(CH2CH2NH)mCH2CH2NHR10; Q-R10, O-Q-R10N-Q-R10, S-Q-R10.

X is 5-100, chosen to comply with a length for an oligonucleotide agent described herein.

R7 is H; or is together combined with R4, R5, or R6 to form an [-O-CH2-] covalently bound bridge between the sugar 2' and 4' carbons.

R8 is alkyl, cycloalkyl, aryl, aralkyl, heterocyclyl, heteroaryl, amino acid, or sugar; R9 is NH2, alkylamino, dialkylamino, heterocyclyl, arylamino, diaryl amino, heteroaryl amino, diheteroaryl amino, or amino acid; and R10 is H; fluorophore (pyrene, TAMRA, fluorescein, Cy3 or Cy5 dyes); sulfur, silicon, boron or ester protecting group; intercalating agents (e.g. acridines), cross-linkers (e.g. psoralene, mitomycin C), porphyrins (TPPC4, texaphyrin, Sapphyrin), polycyclic aromatic hydrocarbons (e.g., phenazine, dihydrophenazine), artificial endonucleases (e.g. EDTA), lipohilic carriers (cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG [MPEG]2, polyamino; alkyl, cycloalkyl, aryl, aralkyl, heteroaryl; radiolabelled markers, enzymes, haptens (e.g. biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles); or an oligonucleotide agent. M is 0-1,000,000, and n is 0-20. Q is a spacer selected from the group consisting of abasic sugar, amide, carboxy, oxyamine, oxyimine, thioether, disulfide, thiourea, sulfonamide, or morpholino, biotin or fluorescein reagents.

Exemplary oligonucleotide agents in which the entire phosphate group has been replaced have the following structure (see Formula 3 below):

FORMULA 3

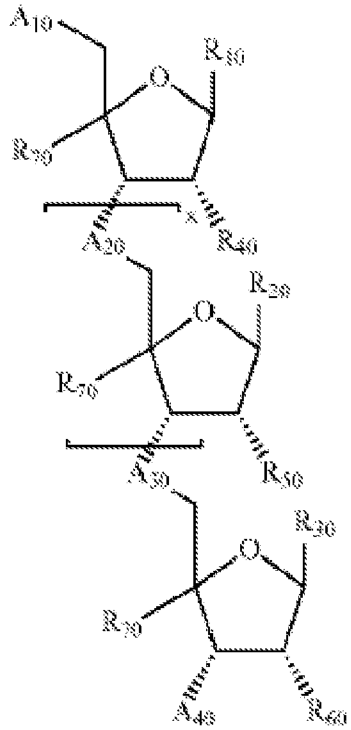

Referring to Formula 3, A10-A40 is L-G-L; A10 and/or A40 may be absent, in which L is a linker, wherein one or both L may be present or absent and is selected from the group consisting of CH2(CH2)g; N(CH2)g; O(CH2)g; S(CH2)g. G is a functional group selected from the group consisting of siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

R10, R20, and R30 are each, independently, H, (i.e. abasic nucleotides), adenine, guanine, cytosine and uracil, inosine, thymine, xanthine, hypoxanthine, nubularine, tubercidine, isoguanisine, 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine, 7-deazaadenine, 7-deazaguanine, N6, N6-dimethyladenine, 2,6-diaminopurine, 5-amino-allyl-uracil, N3-methyluracil substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3carboxypropyl)uracil, 3-methylcytosine, 5-methylcytosine, N4-acetyl cytosine, 2-thiocytosine, N6-methyladenine, N6-isopentyladenine, 2-methylthio-N6-isopentenyladenine, N-methylguanines, or O-alkylated bases.

R40, R50, and R60 are each, independently, OR8, O(CH2CH2O)mCH2CH2OR8; O(CH2)nR9; O(CH2)nOR9, H; halo; NH2; NHR8; N(R8)2; NH(CH2CH2NH)mCH2CH2R9; NHC(O)R8; cyano; mercapto, SR7; alkyl-thio-alkyl; alkyl, aralkyl, cycloalkyl, aryl, heteroaryl, alkenyl, alkynyl, each of which may be optionally substituted with halo, hydroxy, oxo, nitro, haloalkyl, alkyl, alkaryl, aryl, aralkyl, alkoxy, aryloxy, amino, alkylamino, dialkylamino, heterocyclyl, arylamino, diaryl amino, heteroaryl amino, diheteroaryl amino, acylamino, alkylcarbamoyl, arylcarbamoyl, aminoalkyl, alkoxycarbonyl, carboxy, hydroxyalkyl, alkanesulfonyl, alkanesulfonamido, arenesulfonamido, aralkylsulfonamido, alkylcarbonyl, acyloxy, cyano, and ureido groups; or R40, R50, or R60 together combine with R70 to form an [-O-CH2-] covalently bound bridge between the sugar 2' and 4' carbons.

X is 5-100 or chosen to comply with a length for an oligonucleotide agent described herein.

R70 is H; or is together combined with R40, R50, or R60 to form an [-O-CH2-] covalently bound bridge between the sugar 2' and 4' carbons.

R8 is alkyl, cycloalkyl, aryl, aralkyl, heterocyclyl, heteroaryl, amino acid, or sugar; and R9 is NH2, alkylamino, dialkylamino, heterocyclyl, arylamino, diaryl amino, heteroaryl amino, diheteroaryl amino, or amino acid. M is 0-1,000,000,

n is 0-20, and g is 0-2.

In another aspect, certain disclosed nucleoside surrogates have the following structure (see Formula 4 below):

$$SRL^{100}\text{-}(M\text{-}SRL^{200})_x\text{-}M\text{-}SRL^{300} \quad \text{FORMULA 4}$$

S is a nucleoside surrogate selected from the group consisting of mophilino, cyclobutyl, pyrrolidine and peptide nucleic acid. L is a linker and is selected from the group consisting of CH2(CH2)g; N(CH2)g; O(CH2)g; S(CH2)g; -C(O)(CH2)n-or may be absent. M is an amide bond; sulfonamide; sulfinate; phosphate group; modified phosphate group as described herein; or may be absent.

R100, R200, and R300 are each, independently, H (i.e., abasic nucleotides), adenine, guanine, cytosine and uracil, inosine, thymine, xanthine, hypoxanthine, nubularine, tubercidine, isoguanisine, 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine, 7-deazaadenine, 7-deazaguanine, N6, N6-dimethyladenine, 2,6-diaminopurine, 5-amino-allyl-uracil, N3-methyluracil substituted 1,2,4,-triazoles, 2-pyridinones, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3carboxypropyl)uracil, 3-methylcytosine, 5-methylcytosine, N4-acetyl cytosine, 2-thiocytosine, N6-methyladenine, N6-isopentyladenine, 2-methylthio-N6-isopentenyladenine, N-methylguanines, or O-alkylated bases.

X is 5-100, or chosen to comply with a length for an oligonucleotide agent described herein; and g is 0-2.

**Nuclease Resistant Monomers**

The monomers and methods described herein can be used to prepare an oligonucleotide agent, that incorporates a nuclease resistant monomer (NRM).

An oligonucleotide agent can include monomers which have been modifed so as to inhibit degradation, e.g., by nucleases, e.g., endonucleases or exonucleases, found in the body of a subject. These monomers are referred to herein as NRMs, or nuclease resistance promoting monomers or modifications. In many cases these modifications will modulate other properties of the oligonucleotide agent as well, e.g., the ability to interact with a protein, e.g., a transport protein, e.g., serum albumin, or a member of the RISC (RNA-induced Silencing Complex), or the ability of the first and second sequences to form a duplex with one another or to form a duplex with another sequence, e.g., a target molecule.

While not wishing to be bound by theory, it is believed that modifications of the sugar, base, and/or phosphate backbone in an oligonucleotide agent can enhance endonuclease and exonuclease resistance, and can enhance interactions with transporter proteins and one or more of the functional components of the RISC complex. Preferred modifications are those that increase exonuclease and endonuclease resistance and thus prolong the half-life of the oligonucleotide agent prior to interaction with the RISC complex, but at the same time do not render the oligonucleotide agent resistant to endonuclease activity in the RISC complex. Again, while not wishing to be bound by any theory, it is believed that placement of the modifications at or near the 3' and/or 5' end of the oligonucleotide agent can result in agents that meet the preferred nuclease resistance criteria delineated above.

Modifications that can be useful for producing oligonucleotide agents that meet the preferred nuclease resistance criteria delineated above can include one or more of the following chemical and/or stereochemical modifications of the sugar, base, and/or phosphate backbone:

(i) chiral (SP) thioates. Thus, in some embodiments NRMs include nucleotide dimers with an enriched for or having a pure chiral form of a modified phosphate group containing a heteroatom at the nonbridging position, e.g., Sp or Rp, at the position X, where this is the position normally occupied by the oxygen. The atom at X can also be S, Se, $NR_2$, or $BR_3$. When X is S, enriched or chirally pure Sp linkage is preferred. Enriched means at least 70, 80, 90, 95, or 99% of the preferred form. Such NRMs are discussed in more detail below;

(ii) attachment of one or more cationic groups to the sugar, base, and/or the phosphorus atom of a phosphate or modified phosphate backbone moiety. Thus, preferred NRMs include monomers at the terminal position derivatized at a cationic group. As the 5' end of an oligonucleotide agent should have a terminal -OH or phosphate group, this NRM is preferably not used at the 5' end of the agent. The group should be attached at a position on the base which minimizes interference with H bond formation and hybridization, e.g., away from the face which interacts with the complementary base on the other strand, e.g, at the 5' position of a pyrimidine or a 7-position of a purine. These

are discussed in more detail below;

(iii) nonphosphate linkages at the termini. Thus, NRMs of this type include non-phosphate linkages, e.g., a linkage of 4 atoms which confers greater resistance to cleavage than does a phosphate bond. Examples include 3' CH2-NCH3-O-CH2-5' and 3' CH2-NH-(O=)-CH2-5'.;

(iv) 3'-bridging thiophosphates and 5'-bridging thiophosphates. Thus, preferred NRM's can include these structures;

(v) L-RNA, 2'-5' linkages, inverted linkages, a-nucleosides. Thus, other NRMs include: L nucleosides and dimeric nucleotides derived from L-nucleosides; 2'-5' phosphate, non-phosphate and modified phosphate linkages (e.g., thiophosphates, phosphoramidates and boronophosphates); dimers having inverted linkages, e.g., 3'-3' or 5'-5' linkages; monomers having an alpha linkage at the 1' site on the sugar, e.g., the structures described herein having an alpha linkage;

(vi) conjugate groups. Thus, NRMs can include e.g., a targeting moiety or a conjugated ligand described herein, e.g., conjugated with the monomer, e.g., through the sugar, base, or backbone;

(vi) abasic linkages. Thus, NRMs can include an abasic monomer, e.g., an abasic monomer as described herein (e.g., a nucleobaseless monomer); an aromatic or heterocyclic or polyheterocyclic aromatic monomer as described herein; and

(vii) 5'-phosphonates and 5'-phosphate prodrugs. Thus, NRMs include monomers, e.g. at the terminal position, e.g., the 5' position, in which one or more atoms of the phosphate group are derivatized with a protecting group, which protecting group or groups, are removed as a result of the action of a component in the subject's body, e.g, a carboxyesterase or an enzyme present in the subject's body. E.g., a phosphate prodrug in which a carboxy esterase cleaves the protected molecule resulting in the production of a thioate anion which attacks a carbon adjacent to the O of a phosphate and resulting in the production of an unprotected phosphate.

One or more different NRM modifications can be introduced into an oligonucleotide agent or into a sequence of an oligonucleotide agent. An NRM modification can be used more than once in a sequence or in an oligonucleotide agent. As some NRMs interfere with hybridization, the total number incorporated should be such that acceptable levels of oligonucleotide agent/target RNA duplex formation are maintained.

**Chiral SP Thioates**

A modification can include the alteration, e.g., replacement, of one or both of the non-linking (X and Y) phosphate oxygens and/or of one or more of the linking (W and Z) phosphate oxygens. Formula X below depicts a phosphate moiety linking two sugar/sugar surrogate-base moieties, SB1 and SB2.

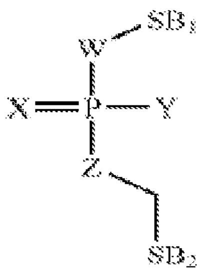

FORMULA X

In certain embodiments, one of the non-linking phosphate oxygens in the phosphate backbone moiety (X and Y) can be replaced by any one of the following: S, Se, BR3 (R is hydrogen, alkyl, aryl, etc.), C (i.e., an alkyl group, an aryl group, etc.), H, NR2 (R is hydrogen, alkyl, aryl, etc.), or OR (R is alkyl or aryl). The phosphorus atom in an unmodified phosphate group is achiral. However, replacement of one of the non-linking oxygens with one of the above atoms or groups of atoms renders the phosphorus atom chiral; in other words a phosphorus atom in a phosphate group modified in this way is a stereogenic center. The stereogenic phosphorus atom can possess either the "R" configuration (herein RP) or the "S" configuration (herein SP). Thus if 60% of a population of stereogenic phosphorus atoms have the RP configuration, then the remaining 40% of the population of stereogenic phosphorus atoms have the SP configuration.

In some embodiments, oligonucleotide agents have phosphate groups in which a phosphate non-linking oxygen has been replaced by another atom or group of atoms, may contain a population of stereogenic phosphorus atoms in which at least about 50% of these atoms (e.g., at least about 60% of these atoms, at least about 70% of these atoms, at least about 80% of these atoms, at least about 90% of these atoms, at least about 95% of these atoms, at least about 98% of these atoms, at least about 99% of these atoms) have the SP configuration. Alternatively, oligonucleotide agents having phosphate groups in which a phosphate non-linking oxygen has been replaced by another atom or group of

atoms may contain a population of stereogenic phosphorus atoms in which at least about 50% of these atoms (e.g., at least about 60% of these atoms, at least about 70% of these atoms, at least about 80% of these atoms, at least about 90% of these atoms, at least about 95% of these atoms, at least about 98% of these atoms, at least about 99% of these atoms) have the RP configuration. In other embodiments, the population of stereogenic phosphorus atoms may have the SP configuration and may be substantially free of stereogenic phosphorus atoms having the RP configuration. In still other embodiments, the population of stereogenic phosphorus atoms may have the RP configuration and may be substantially free of stereogenic phosphorus atoms having the SP configuration. As used herein, the phrase "substantially free of stereogenic phosphorus atoms having the RP configuration" means that moieties containing stereogenic phosphorus atoms having the RP configuration cannot be detected by conventional methods known in the art (chiral HPLC, $^1$H NMR analysis using chiral shift reagents, etc.). As used herein, the phrase "substantially free of stereogenic phosphorus atoms having the SP configuration" means that moieties containing stereogenic phosphorus atoms having the SP configuration cannot be detected by conventional methods known in the art (chiral HPLC, $^1$H NMR analysis using chiral shift reagents, etc.).

In some embodiments, modified oligonucleotide agents contain a phosphorothioate group, i.e., a phosphate groups in which a phosphate non-linking oxygen has been replaced by a sulfur atom. In an embodiment, the population of phosphorothioate stereogenic phosphorus atoms may have the SP configuration and be substantially free of stereogenic phosphorus atoms having the RP configuration.

Phosphorothioates may be incorporated into oligonucleotide agents using dimers e.g., formulas X-1 and X-2. The former can be used to introduce phosphorothioate

X-1

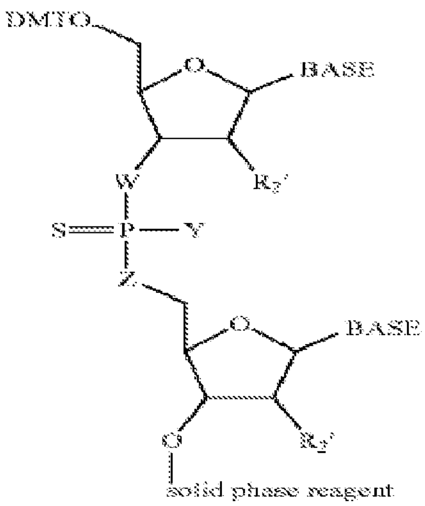

X-2

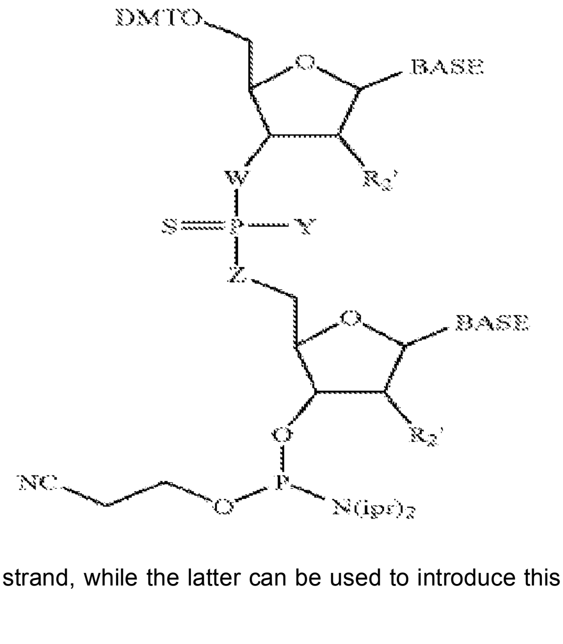

at the 3' end of a strand, while the latter can be used to introduce this modification at the 5' end or at a position that

occurs e.g., 1, 2, 3, 4, 5, or 6 nucleotides from either end of the strand. In the above formulas, Y can be 2-cyanoethoxy, W and Z can be O, R2' can be, e.g., a substituent that can impart the C-3 endo configuration to the sugar (e.g., OH, F, OCH3), DMT is dimethoxytrityl, and "BASE" can be a natural, unusual, or a universal base.

X-1 and X-2 can be prepared using chiral reagents or directing groups that can result in phosphorothioate-containing dimers having a population of stereogenic phosphorus atoms having essentially only the RP configuration (i.e., being substantially free of the SP configuration) or only the SP configuration (i.e., being substantially free of the RP configuration). Alternatively, dimers can be prepared having a population of stereogenic phosphorus atoms in which about 50% of the atoms have the RP configuration and about 50% of the atoms have the SP configuration. Dimers having stereogenic phosphorus atoms with the RP configuration can be identified and separated from dimers having stereogenic phosphorus atoms with the SP configuration using e.g., enzymatic degradation and/or conventional chromatography techniques.

**Cationic Groups**

Modifications can also include attachment of one or more cationic groups to the sugar, base, and/or the phosphorus atom of a phosphate or modified phosphate backbone moiety. A cationic group can be attached to any atom capable of substitution on a natural, unusual or universal base. A preferred position is one that does not interfere with hybridization, i.e., does not interfere with the hydrogen bonding interactions needed for base pairing. A cationic group can be attached e.g., through the C2' position of a sugar or analogous position in a cyclic or acyclic sugar surrogate. Cationic groups can include e.g., protonated amino groups, derived from e.g., O-AMINE (AMINE=NH2; alkylamino, dialkylamino, heterocyclyl, arylamino, diaryl amino, heteroaryl amino, or diheteroaryl amino, ethylene diamine, polyamino); aminoalkoxy, e.g., O(CH2)nAMINE, (e.g., AMINE=NH2; alkylamino, dialkylamino, heterocyclyl, arylamino, diaryl amino, heteroaryl amino, or diheteroaryl amino, ethylene diamine, polyamino); amino (e.g. NH2; alkylamino, dialkylamino, heterocyclyl, arylamino, diaryl amino, heteroaryl amino, diheteroaryl amino, or amino acid); or NH(CH2CH2NH)nCH2CH2-AMINE (AMINE=NH2; alkylamino, dialkylamino, heterocyclyl, arylamino, diaryl amino, heteroaryl amino, or diheteroaryl amino).

**Nonphosphate Linkages**

Modifications can also include the incorporation of nonphosphate linkages at the 5' and/or 3' end of a strand. Examples of nonphosphate linkages which can replace the phosphate group include methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino. In some embodiments, the replacement is selected from the methyl phosphonate and hydroxylamino groups.

3'-bridging thiophosphates and 5'-bridging thiophosphates; locked-RNA, 2'-5' likages, inverted linkages, $\alpha$-nucleosides; conjugate groups; abasic linkages; and 5'-phosphonates and 5'-phosphate prodrugs are also linkages that can be included in oligonucleotide agents.

Referring to formula X above, modifications can include replacement of one of the bridging or linking phosphate oxygens in the phosphate backbone moiety (W and Z). Unlike the situation where only one of X or Y is altered, the phosphorus center in the phosphorodithioates is achiral which precludes the formation of oligonucleotide agents containing a stereogenic phosphorus atom.

Modifications can also include linking two sugars via a phosphate or modified phosphate group through the 2' position of a first sugar and the 5' position of a second sugar. Also contemplated are inverted linkages in which both a first and second sugar are eached linked through the respective3' positions. Modified RNAs can also include "abasic" sugars, which lack a nucleobase at C-1'. The sugar group can also contain one or more carbons that possess the opposite stereochemical configuration than that of the corresponding carbon in ribose. Thus, a modified oligonucleotide agent can include nucleotides containing e.g., arabinose, as the sugar. In another subset of this modification, the natural, unusual, or universal base may have the $\alpha$-configuration. Modifcations can also include L-RNA.

Modifications can also include 5'-phosphonates, e.g., P(O)(O-)2--X--C5'-sugar (X=CH2, CF2, CHF and 5'-phosphate prodrugs, e.g., P(O)[OCH2CH2SC(O)R]2CH2C5'-sugar. In the latter case, the prodrug groups may be decomposed via reaction first with carboxy esterases. The remaining ethyl thiolate group via intramolecular SN2 displacement can depart as episulfide to afford the underivatized phosphate group.

Modification can also include the addition of conjugating groups described elsewhere herein, which are prefereably attached to an oligonucleotide agent through any amino group available for conjugation.

Nuclease resistant modifications include some which can be placed only at the terminus and others which can go at any position. Generally, these modifications can inhibit hybridization so it is preferably to use them only in terminal regions, and preferable to not use them at the cleavage site or in the cleavage region of a sequence.

Modifications which interfere with or inhibit endonuclease cleavage should not be inserted in the region of an oligonucleotide agent which is subject to RISC mediated cleavage, e.g., the cleavage site or the cleavage region. As used herein, "cleavage site" refers to the nucleotide on either side of the cleavage site on the target or on the oligonucleotide agent strand which hybridizes to it. "Cleavage region" means a nucleotide with 1, 2, or 3 nucletides of the cleave site, in either direction.)

Such modifications can be introduced into the terminal regions, e.g., at the terminal position or with 2, 3, 4, or 5 positions of the terminus.

An oligonucleotide agent can have the following:

an NRM modification at or within 1, 2, 3, 4, 5, or 6 positions from the 3' end;
an NRM modification at or within 1, 2, 3, 4, 5, or 6 positions from the 5' end (5' end NRM modifications are preferentially not at the terminus but rather at a position 1, 2, 3, 4, 5, or 6 away from the 5' terminus of the oligonucleotide agent);
an NRM modification at or within 1, 2, 3, 4, 5, or 6 positions from the 3' end and which has a NRM modification at or within 1, 2, 3, 4, 5, or 6 positions from the 5' end;
an NRM modification at the cleavage site or in the cleavage region;
an NRM modification at the cleavage site or in the cleavage region and one or more of an NRM modification at or within 1, 2, 3, 4, 5, or 6 positions from the 3' end, an NRM modification at or within 1, 2, 3, 4, 5, or 6 positions from the 5' end, or NRM modifications at or within 1, 2, 3, 4, 5, or 6 positions from both the 3' and the 5' end (5' end NRM modifications are preferentially not at the terminus but rather at a position 1, 2, 3, 4, 5, or 6 away from the 5' terminus of the oligonucleotide agent).

**Ribose Mimics**

The monomers and methods described herein can be used to prepare an oligonucleotide agent that incorporates a ribose mimic.

Thus, an aspect of the invention features an oligonucleotide agent that includes a secondary hydroxyl group, which can increase efficacy and/or confer nuclease resistance to the agent. Nucleases, e.g., cellular nucleases, can hydrolyze nucleic acid phosphodiester bonds, resulting in partial or complete degradation of the nucleic acid. The secondary hydroxy group confers nuclease resistance to an oligonucleotide agent by rendering the oligonucleotide agent less prone to nuclease degradation relative to an oligonucleotide agent that lacks the modification. While not wishing to be bound by theory, it is believed that the presence of a secondary hydroxyl group on the oligonucleotide agent can act as a structural mimic of a 3' ribose hydroxyl group, thereby causing it to be less susceptible to degradation.

The secondary hydroxyl group refers to an "OH" radical that is attached to a carbon atom substituted by two other carbons and a hydrogen. The secondary hydroxyl group that confers nuclease resistance as described above can be part of any acyclic carbon-containing group. The hydroxyl may also be part of any cyclic carbon-containing group, and preferably one or more of the following conditions is met (1) there is no ribose moiety between the hydroxyl group and the terminal phosphate group or (2) the hydroxyl group is not on a sugar moiety which is coupled to a base. The hydroxyl group is located at least two bonds (e.g., at least three bonds away, at least four bonds away, at least five bonds away, at least six bonds away, at least seven bonds away, at least eight bonds away, at least nine bonds away, at least ten bonds away, etc.) from the terminal phosphate group phosphorus of the oligonucleotide agent. In preferred embodiments, there are five intervening bonds between the terminal phosphate group phosphorus and the secondary hydroxyl group.

Certain exemplary oligonucleotide agent delivery modules with five intervening bonds between the terminal phosphate group phosphorus and the secondary hydroxyl group have the following structure (see formula Y below):

(Y)

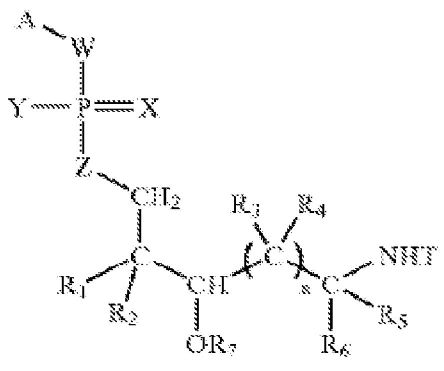

Referring to formula Y, A is an oligonucleotide agent, including any oligonucleotide agent described herein. The oligonucleotide agent may be connected directly or indirectly (e.g., through a spacer or linker) to "W" of the phosphate group. These spacers or linkers can include e.g., -(CH2)n-, -(CH2)nN-, -(CH2)nO-, -(CH2)nS-, O(CH2CH2O)nCH2CH2OH (e.g., n=3 or 6), abasic sugars, amide, carboxy, amine, oxyamine, oxyimine, thioether, disulfide, thiourea, sulfonamide, or morpholino, or biotin and fluorescein reagents.

The oligonucleotide agents can have a terminal phosphate group that is unmodified (e.g., W, X, Y, and Z are O) or modified. In a modified phosphate group, W and Z can be independently NH, O, or S; and X and Y can be independently S, Se, BH3-, C1-C6 alkyl, C6-C10 aryl, H, O, O-, alkoxy or amino (including alkylamino, arylamino, etc.). In some embodiments,, W, X and Z are O and Y is S.

R1 and R3 are each, independently, hydrogen; or C1-C100 alkyl, optionally substituted with hydroxyl, amino, halo, phosphate or sulfate and/or may be optionally inserted with N, O, S, alkenyl or alkynyl.

R2 is hydrogen; C1-C100 alkyl, optionally substituted with hydroxyl, amino, halo, phosphate or sulfate and/or may be optionally inserted with N, O, S, alkenyl or alkynyl; or, when n is 1, R2 may be taken together with R4 or R6 to form a ring of 5-12 atoms.

R4 is hydrogen; C1-C100 alkyl, optionally substituted with hydroxyl, amino, halo, phosphate or sulfate and/or may be optionally inserted with N, O, S, alkenyl or alkynyl; or, when n is 1, R4 may be taken together with R2 or R5 to form a ring of 5-12 atoms.

R5 is hydrogen, C1-C100 alkyl optionally substituted with hydroxyl, amino, halo, phosphate or sulfate and/or may be optionally inserted with N, O, S, alkenyl or alkynyl; or, when n is 1, R5 may be taken together with R4 to form a ring of 5-12 atoms.

R6 is hydrogen, C1-C100 alkyl, optionally substituted with hydroxyl, amino, halo, phosphate or sulfate and/or may be optionally inserted with N, O, S, alkenyl or alkynyl, or, when n is 1, R6 may be taken together with R2 to form a ring of 6-10 atoms;

R7 is hydrogen, C1-C100 alkyl, or C(O)(CH2)qC(O)NHR9; T is hydrogen or a functional group; n and q are each independently 1-100; R8 is C1-C10 alkyl or C6-C10 aryl; and R9 is hydrogen, C1-C10 alkyl, C6-C10 aryl or a solid support agent.

Preferred embodiments may include one of more of the following subsets of oligonucleotide agent delivery modules.

In one subset of oligonucleotide agent delivery modules, A can be connected directly or indirectly through a terminal 3' or 5' ribose sugar carbon of the oligonucleotide agent.

In another subset of oligonucleotide agent delivery modules, X, W, and Z are O and Y is S.

In still yet another subset of oligonucleotide agent delivery modules, n is 1, and R2 and R6 are taken together to form a ring containing six atoms and R4 and R5 are taken together to form a ring containing six atoms. In some embodiments, the ring system is a trans-decalin. For example, the oligonucleotide agent delivery module of this subset can include a compound of Formula (Y-1):

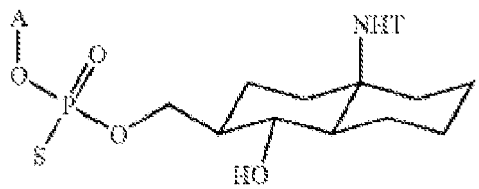

The functional group can be, for example, a targeting group (e.g., a steroid or a carbohydrate), a reporter group (e.g., a fluorophore), or a label (an isotopically labelled moiety). The targeting group can further include protein binding agents, endothelial cell targeting groups (e.g., RGD peptides and mimetics), cancer cell targeting groups (e.g., folate, Vitamin B12, Biotin), bone cell targeting groups (e.g., bisphosphonates, polyglutamates, polyaspartates), multivalent mannose (for e.g., macrophage testing), lactose, galactose, N-acetyl-galactosamine, monoclonal antibodies, glycoproteins, lectins, melanotropin, or thyrotropin.

As can be appreciated by the skilled artisan, methods of synthesizing the compounds of the formulae herein will be evident to those of ordinary skill in the art. The synthesized compounds can be separated from a reaction mixture and further purified by a method such as column chromatography, high pressure liquid chromatography, or recrystallization. Additionally, the various synthetic steps may be performed in an alternate sequence or order to give the desired compounds. Synthetic chemistry transformations and protecting group methodologies (protection and deprotection) useful in synthesizing the compounds described herein are known in the art and include, for example, those such as described in R. Larock, Comprehensive Organic Transformations, VCH Publishers (1989); T. W. Greene and P. G. M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of

Reagents for Organic Synthesis, John Wiley and Sons (1995), and subsequent editions thereof.

In some embodiments, a disclosed therapeutic agent, e.g. iRNA, can be conjugated to a low molecular weight polyethylene glycol (PEG) molecule, or guanidium group, and in another embodiment, the oligonucleotide agent can be conjugated to an RGD peptide, peptide analog, or peptide mimetic or derivative thereof. An oligonucleotide conjugated to an RGD peptide, peptide analog, or peptide mimetic can bind to an αvβ3 integrin.

Synthetic Integrin Inhibitors

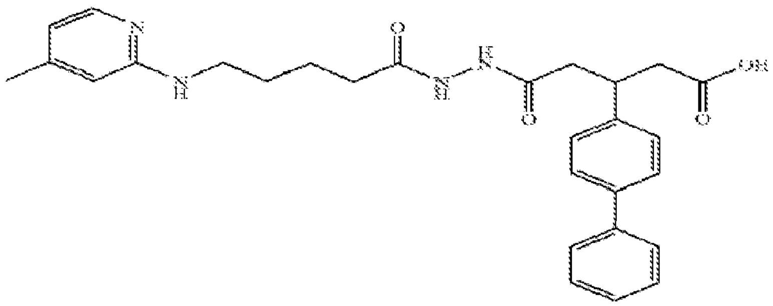

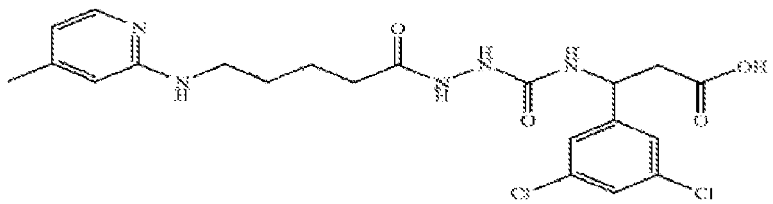

-continued

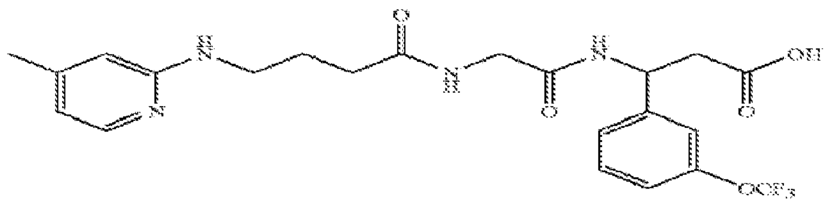

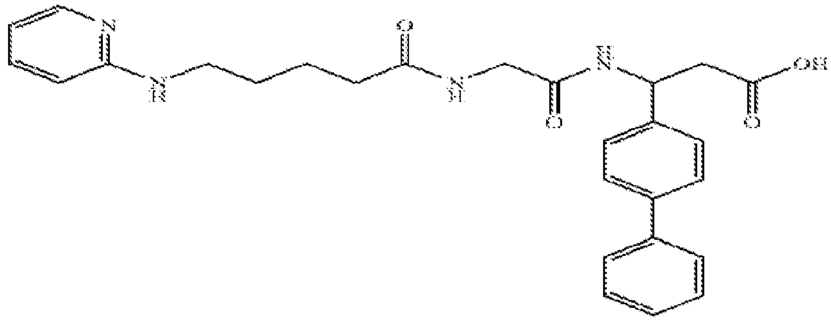

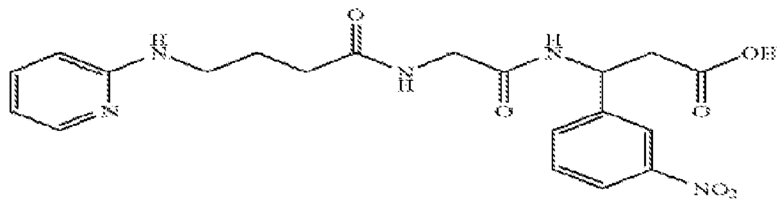

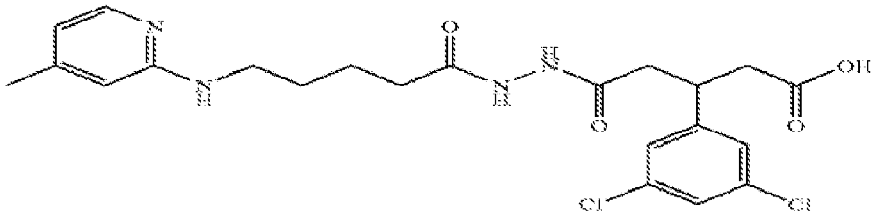

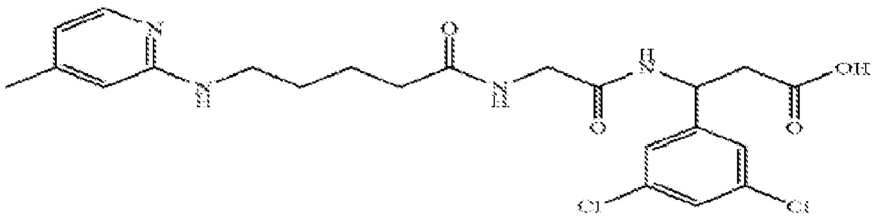

-continued

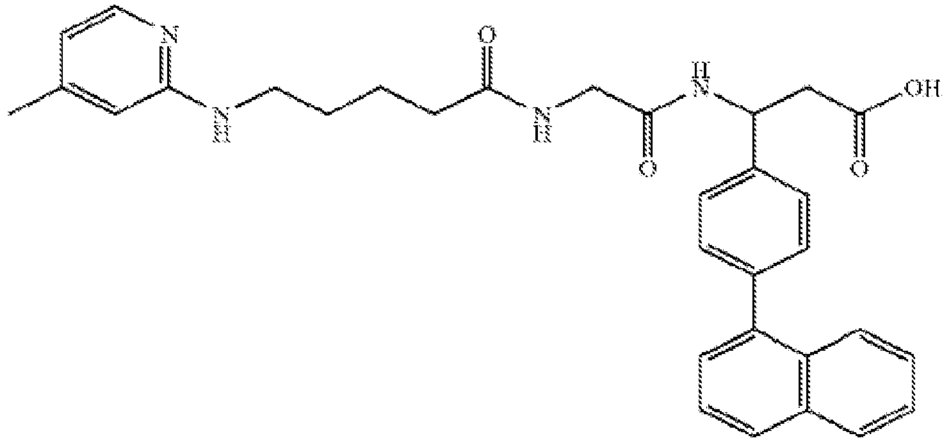

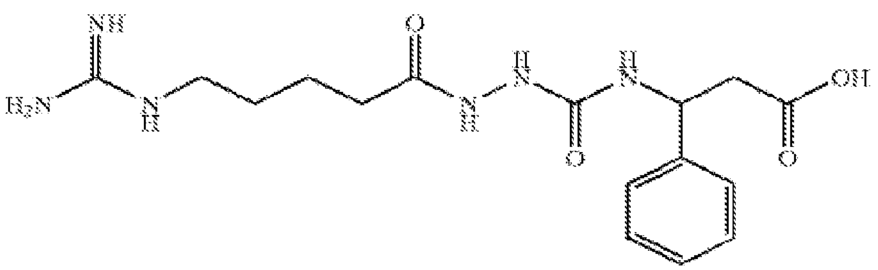

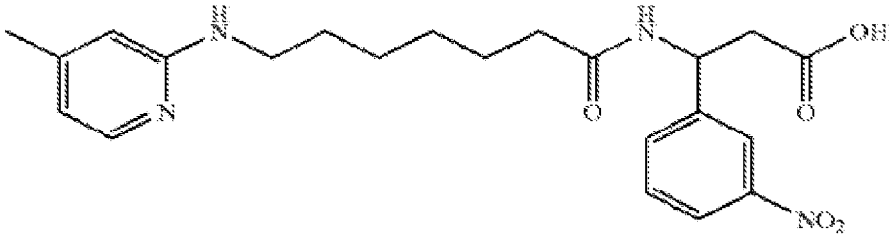

Ref: Goodman, S. L.; Hölzemann, G.; Sulyok, G. A. G.; 35 Kessler, H. J. Med. Chem. 2002, 45, 1045-1051.

## Table F

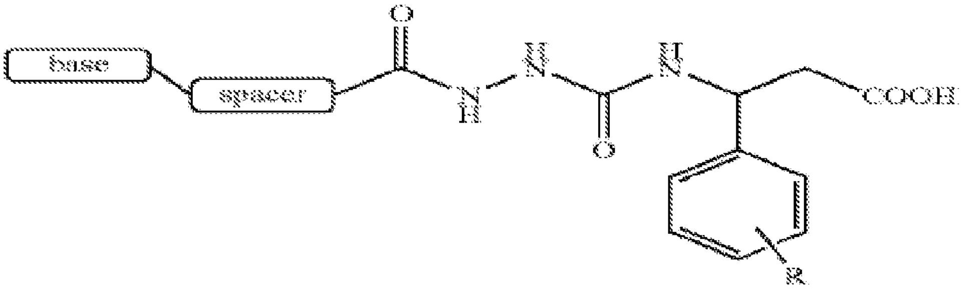

| R | base | spacer |
|---|---|---|
| (Carboxamide)[a] | guanidine | m-$C_6H_4$----- |
| none | guanidine | ω-$C_4H_8$----- |
| none | guanidine | m-$C_6H_4$----- |
| 4-F | guanidine | m-$C_6H_4$----- |
| 4-Cl | guanidine | m-$C_6H_4$----- |
| 4-Br | guanidine | m-$C_6H_4$----- |
| 4-$OCH_3$ | guanidine | m-$C_6H_4$----- |
| 4-$OCF_3$ | guanidine | guanidine |
| (1-naphthyl)[a] | guanidine | m-$C_6H_4$----- |
| 3-Cl,5-Cl | guanidine | m-$C_6H_4$----- |
| (H)[a] | 2-$NH_2$-pyridine | ω-$C_4H_8$----- |
| none | 2-$NH_2$-pyridine | ω-$C_4H_8$----- |
| 4-F | 2-$NH_2$-pyridine | ω-$C_4H_8$----- |
| 4-Cl | 2-$NH_2$-pyridine | ω-$C_4H_8$----- |
| 4-Br | 2-$NH_2$-pyridine | ω-$C_4H_8$----- |
| 4-$OCH_3$ | 2-$NH_2$-pyridine | ω-$C_4H_8$----- |
| 4-$OCF_3$ | 2-$NH_2$-pyridine | ω-$C_4H_8$----- |

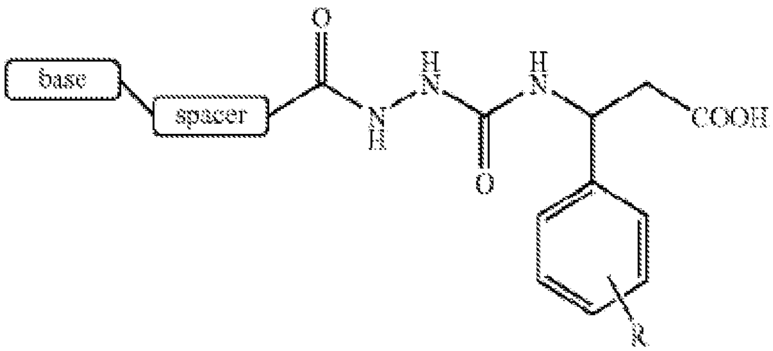

| R | base | spacer |
|---|---|---|
| (1-naphthyl)[a] | 2-$NH_2$-pyridine | ω-$C_4H_8$----- |
| 3-Cl,5-Cl | 2-$NH_2$-pyridine | ω-$C_4H_8$----- |

[a]Instead of substituted phenyl ring

Ref: Sulyok, G. A. G.; Gibson, C.; Goodman, S. L.; Hölzemann, G.; Wiesner, M.; Kessler H. J. Med. Chem. 2001, 44, 1938-1950.

In some embodiments, at least 30%, 40%, 50%, 60%, 70%, 80%, 90% or more of the oligonucleotide agent administered to the subject is successfully targeted to the kidney. In some embodiments, between 30-90%, 40-80% or 50-70% 50-80%, or 50-90% of the oligonucleotide agent administered to the subject is successfully targeted to the kidney.

In any of the embodiments described above, the oligonucleotide agent/conjugate can have additional modifications, such as a stabilizing modification. For example, a linker molecule can tether a protein, PEG or RGD peptide to the oligonucleotide agent. Exemplary linkers are described infra, and can include amino linkers (e.g., aminooxy linkers), thiol linkers, carboxyl linkers, aldehyde linkers, haloacetyl linkers, and the like.

In another aspect, the invention features a conjugate oligonucleotide agent. The conjugate includes an oligonucleotide agent coupled to, e.g., linked to, a ligand or therapeutic agent. The oligonucleotide agent is optionally coupled to the ligand or therapeutic agent by a linker (e.g., a peptide linker or other linker described herein). The ligand can function to, e.g., affect the distribution of the oligonucleotide agent in the body and/or to target the oligonucleotide agent to a particular tissue or cell.

The ligand can be placed at an end of the oligonucleotide agent, preferably at the 3' end of an oligonucleotide agent. The ligand can also be placed at the 5' end, or within the middle of the oligonucleotide agent. In some embodiments, more than one ligand can be coupled to the oligonucleotide agent. For example, a ligand can be coupled to the 3' end of an oligonucleotide agent; a ligand can be coupled to an end, e.g., a 3' end, and to the middle of an oligonucleotide agent; a ligand can be coupled to the 3' end and the 5' of an oligonucleotide agent; a ligand can be coupled to the 3' end, the 5' end, and to one or more internal positions of an oligonucleotide agent.

In some embodiments, the ligand of a conjugated oligonucleotide agent is a lipid or lipid-based molecule. Such a lipid or lipid-based molecule preferably binds a serum protein, e.g., human serum albumin (HSA). An HSA binding ligand allows for distribution of the conjugate to a target tissue, e.g., a non-kidney target tissue of the body. For example, the target tissue can be the liver, including, but not limited to parenchymal cells of the liver. Other molecules that can bind HSA can also be used as ligands. For example, neproxin or aspirin can be used. A lipid or lipid-based ligand can (a) increase resistance to degradation of the conjugate, (b) increase targeting or transport into a target cell or cell membrane, and/or (c) can be used to adjust binding to a serum protein, e.g., HSA.

A lipid based ligand can be used to modulate, e.g., control the binding of the conjugate to a target tissue. For example, a lipid or lipid-based ligand that binds to HSA more strongly will be less likely to be targeted to the kidney and therefore less likely to be cleared from the body. A lipid or lipid-based ligand that binds to HSA less strongly can be used to target the conjugate to the kidney.

In a preferred embodiment, the lipid based ligand binds HSA. Preferably, it binds HSA with a sufficient affinity such that the conjugate will be preferably distributed to a non-kidney tissue. However, it is preferred that the affinity not be so strong that the HSA-ligand binding cannot be reversed.

In another preferred embodiment, the lipid based ligand binds HSA weakly or not at all, such that the conjugate will be preferably distributed to the kidney. Other moieties that target to kidney cells can also be used in place of or in addition to the lipid based ligand.

In a preferred embodiment, the lipid or lipid based ligand is a phosphorothioate. In this embodiment, it is preferred that the number of sulfurs on the phosphorothioate not be so prevalent that they interfere with binding to a serum protein, e.g., HSA.

In another embodiment, the ligand is a peptide or peptoid. Peptoids, in particular amphipathic species, such as Antennapedia or tat, are preferred.

In another embodiment, the ligand is a polyethylene glycol (PEG) or derivatives thereof. A PEG can, e.g., allow the agent to be kept in circulation. A PEG is intrinsically amphipathic, and can promote stability, particularly if coupled at the 3'end of the oligonucleotide agent.

In another embodiment, the ligand is a charged group or moiety, e.g., a polyamine or cationic group or moiety. This type of linker moiety, e.g., because of its charge, e.g., its negative charge, can help overcome the resistance of entry of the oligonucleotide agent into a cell. Preferably, these are conjugated at the 3' end, but they can also be at the 5' end or within the middle of the oligonucleotide molecule. Exemplary polyamines include polyarginine, polylysine, polyhistidine, polypreprozine, or polymorpholinos, polyornithine.

In another embodiment, the ligand is a vitamin or other moiety that is taken up by a target cell, e.g., a proliferating cell. These are particularly useful for treating disorders characterized by unwanted cell proliferation, e.g., of the malignant or non-malignant type, e.g., cancer cells. Exemplary vitamins are B vitamin, e.g., folic acid, B12, riboflavin, biotin, pyridoxal or other vitamins or nutrients taken up by cancer cells. Also included are HSA and low density lipoprotein (LDL).

In another embodiment, the ligand is a cell-permeation agent, preferably a helical cell-permeation agent. Preferably, the agent is amphipathic. An exemplary agent is a peptide such as tat or Antennapodia. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

The ligand can be a targeting agent. The targeting agent can be a sugar, a peptide, e.g., an RGD containing

peptide.

Another useful targeting agent is one that incorporates a sugar, e.g., galactose and/or analogs thereof. These are useful because they target the liver, in particular, the parenchymal cells of the liver. In a preferred embodiment, the targeting agent includes more than one galactose moiety, preferably two or three. Preferably, the targeting agent includes 3 galactose moieties, e.g., spaced about 15 angstroms from each other. The targeting agent can be lactose. Lactose is a glucose coupled to a galactose. Preferably, the targeting agent includes three lactoses. The targeting agent can also be N-Acetyl-Galactosamine, N-Ac-Glucosamine. A mannose, or mannose-6-phosphate targeting agent can be used for macrophage targeting.

Peptides that target markers enriched in proliferating cells can be used. E.g., RGD containing peptides and peptidomimetics can target cancer cells, in particular cells that exhibit an $\alpha v\beta 3$ integrin. Thus, one could use RGD peptides, cyclic peptides containing RGD, RGD peptides that include D-amino acids, as well as synthetic RGD mimics. In additional to RGD, one can use other moieties that target the $\alpha v$-$\beta 3$ integrin ligand. Generally, such ligands can be used to control proliferating cells and angiogenesis. Preferred conjugates of this type include an oligonucleotide agent that targets PECAM-1, VEGF, or other cancer gene, e.g., a cancer gene described herein.

In one embodiment, an oligonucleotide agent is linked, e.g., directly linked, e.g., covalently, or non-covalently linked, to the targeting agent, e.g., a targeting agent described herein. This is referred to as a "conjugation" approach. In another embodiment, the targeting agent (e.g., the same targeting agent) is simply mixed with the oligonucleotide agent. This is referred to as a "complexing" approach. In a complexing approach, the oligonucleotide agent can be mixed with, e.g., a cationic molecule, e.g., a cationic lipid, e.g., with or without a targeting group, e.g., with or without a sugar or an RGD construct described herein. In some embodiments, the oligonucleotide agent is mixed with a polymer-based system, e.g., with or without a targeting group. In other embodiments, the oligonucleotide agent is mixed with a nanoparticle.

Exemplary therapeutic agents for use in the present invention, their functions and examples of clinical uses are provided in Table 1, below.

**Table 1: Exemplary Therapeutic Agents**

| Therapeutic | Trade name | Function | Examples of clinical use |
|---|---|---|---|
| ***Endocrine disorders (hormone deficiencies)*** | | | |
| Insulin | Humulin, Novolin | Regulates blood glucose, shifts potassium into cells | Diabetes mellitus, diabetic ketoacidosis, hyperkalaemia |
| Insulin human inhalation | Exubera | Insulin formulated for inhalation with faster onset of action | Diabetes mellitus |
| Insulin aspart; insulin glulisine; Insulin lispro | Novolog (aspart), Apidra (glulisine); Humalog (lispro) | Insulin analogues with faster onset of action and shorter duration of action | Diabetes mellitus |
| Isophane insulin | NPH | Insulin protamine crystalline formulation with slower onset of action and longer duration of action | Diabetes mellitus |
| Insulin detemir; Insulin glargine | Levemir (detemir), Lantus (glargine) | Insulin analogues with slower onset of action and longer duration of action | Diabetes mellitus |
| Insulin zinc extended | Lente, Ultralente | Insulin zinc hexameric complex with slower onset of action and longer duration of action | Diabetes mellitus |
| Pramlintide acetate | Symlin | Mechanism unknown; recombinant synthetic peptide analogue of human amylin (a naturally occurring neuroendocrine hormone regulating post-prandial glucose control) | Diabetes mellitus, in combination with insulin |

(continued)

| Therapeutic | Trade name | Function | Examples of clinical use |
|---|---|---|---|
| Growth hormone (GH), somatotropin | Genotropin, Humatrope, Norditropin, NorlVitropin, Nutropin, Omnitrope, Protropin, Siazen, Serostim, Valtropin | Anabolic and anticatabolic effector | Growth failure due to GH deficiency or chronic renal insufficiency, Prader-Willi syndrome, Turner syndrome, AIDS wasting or cachexia with antiviral therapy |
| Mecasermin | Increlex | Recombinant insulin-like growth factor 1 (IGF1) induces mitogenesis, chondrocyte growth and organ growth, which combine to restore appropriate statural growth | Growth failure in children with GH gene deletion or severe primary IGF1 deficiency |
| Mecasermin rinfabate | IPlex | Similar to mecasermin; IGF1 bound to IGF binding protein 3 (IGFBP3) is thought to keep the hormone inactive until it reaches its target tissues, thereby decreasing hypoglycaemia-like side effects | Growth failure in children with GH gene deletion or severe primary IGF1 deficiency |
| ***Haemostasis and thrombosis*** | | | |
| Factor VIII | Bioclate, Helixate, Kogenate, Recombinate, ReFacto | Coagulation factor | Haemophilia A |
| Factor IX | Benefix | Coagulation factor | Haemophilia B |
| Antithrombin III (AT-111) | Thrombate III | Purified human AT-III from pooled plasma inactivates thrombin by forming a covalent bond between the catalytic serine residue of thrombin and an arginine reactive site on AT-III; AT-III replacement therapy prevents inappropriate blood-clot formation | Hereditary AT-III deficiency in connection with surgical or obstetrical procedures or for thromboembolism |
| Protein C concentrate | Ceprotin | After activation by the thrombin-thrombomodulin complex, protein C inhibits coagulation factors Va and VIIIa | Treatment and prevention of venous thrombosis and purpura fulminans in patients with severe hereditary protein C deficiency |
| ***Metabolic enzyme deficiencies*** | | | |
| β-Gluco-cerebrosidase | Cerezyme | Hydrolyzes glucocerebroside to glucose and ceramide | Gaucher's disease |
| β-Gluco-cerebrosidase | Ceredase (purified from pooled human placenta) | Hydrolyzes glucocerebroside to glucose and ceramide | Gaucher's disease |

(continued)

| Therapeutic | Trade name | Function | Examples of clinical use |
|---|---|---|---|
| Alglucosidase-α | Myozyme | Degrades glycogen by catalyzing the hydrolysis of α-1,4 and α-1,6 glycosidic linkages of lysosomal glycogen | Pompe disease (glycogen storage disease type II) |
| Laronidase (α-L-iduronidase) | Aldurazyme | Digests endogenous glycosaminoglycans (GAGs) within lysosomes, and thereby prevents an accumulation of GAGs that can cause cellular, tissue, and organ dysfunction | Hurler and Hurler-Scheie forms of mucopolysaccharidosis I |
| Idursulphase (Iduronate-2-sulphatase) | Elaprase | Cleaves the terminal 2-*O*-sulphate moieties from the GAGs dermatan sulphate and heparan sulphate, thereby allowing their digestion and preventing GAG accumulation | Mucopolysaccharidosis II (Hunter syndrome) |
| Galsulphase | Naglazyme | Cleaves the terminal sulphate from the GAG dermatan sulphate, thereby allowing its digestion and preventing GAG accumulation | Mucopolysaccharidosis VI |
| Agalsidase-β (human α- | Fabrazyme | Enzyme that hydrolyzes globotriaosylceramide (GL3) and other | Fabry disease; prevents accumulation of lipids |
| galactosidase A) | | glycosphingolipids, reducing deposition of these lipids in capillary endothelium of the kidney and certain other cell types | that could lead to renal and cardiovascular complications |
| ***Pulmonary and gastrointestinal-tract disorders*** | | | |
| α-1-Proteinase inhibitor | Aralast, Prolastin | Inhibits elastase-mediated destruction of pulmonary tissue; purified from pooled human plasma | Congenital α-1-antitrypsin deficiency |
| Lactase | Lactaid | Digests lactose; purified from fungus *Aspergillus oryzae* | Gas, bloating, cramps and diarrhoea due to inability to digest lactose |
| Pancreatic enzymes (lipase, amylase, protease) | Arco-Lase, Cotazym, Creon, Donnazyme, Pancrease, Viokase, Zymase | Digests food (protein, fat and carbohydrate); purified from hogs and pigs | Cystic fibrosis, chronic pancreatitis, pancreatic insufficiency, post-Billroth II gastric bypass surgery, pancreatic duct obstruction, steatorrhoea, poor digestion, gas, bloating |
| ***Immunodeficiencies*** | | | |
| Adenosine deaminase (pegademase bovine, PEG-ADA) | Adagen | Metabolizes adenosine, prevents accumulation of adenosine; purified from cows | Severe combined immunodeficiency due to adenosine deaminase deficiency |
| Pooled immunoglobulins | Octagam | Intravenous immunoglobulin preparation | Primary immunodefiencies |
| ***Other*** | | | |

(continued)

| Therapeutic | Trade name | Function | Examples of clinical use |
|---|---|---|---|
| Human albumin | Albumarc, Albumin, Albumiar, AlbuRx, Albutein, Flexbumin, Buminate, Plasbumin | Increases circulating plasma osmolarity, thereby restoring and maintaining circulating blood volume | Decreased production of albumin (hypoproteinaemia), increased loss of albumin (nephrotic syndrome), hypovolaemia, hyperbilirubinaemia |
| ***Cancer*** | | | |
| Bevacizumab | Avastin | Humanized mAb that binds all isoforms of VEGFA | Colorectal cancer, non-small-cell lung cancer |
| Cetuximab | Erbitux | Humanized mAb that binds EGFR | Colorectal cancer, head and neck cancer |
| Paniturnumab | Vectibix | Human mAb that binds EGFR | Metastatic colorectal cancer |
| Alemtuzumab | Campath | Humanized mAb directed against CD52 antigen on T and B cells | B-cell chronic lymphocytic leukaemia in patients who have been treated with alkylating agents and who have failed fludabarine therapy |
| Rituximab | Rituxan | Chimeric (human/mouse) mAb that binds CD20, a transmembrane protein found on over 90% of B-cell non-Hodgkin's lymphomas (NHL); synergistic effect with some small-molecule chemotherapeutic agents has been demonstrated in lymphoma cell lines | Relapsed or refractory low-grade or follicular $CD20^+$ B-cell NHL, primary low-grade or follicular $CD20^+$ B-cell NHL in combination with CVP chemotherapy; diffuse large B-cell $CD20^+$ NHL in combination with CHOP or other anthracyline-based chemotherapy; rheumatoid arthritis in combination with methotrexate |
| Trastuzumab | Herceptin | Humanized mAb that binds HER2/Neu cell surface receptor and controls cancer cell growth | Breast cancer |
| ***Immunoregulation*** | | | |
| Abatacept | Orencia | Fusion protein between extracellular domain of human CTLA4 and the modified Fc portion of human immunoglobulin G1; selective co-stimulation modulator; inhibits T-cell activation by binding to CD80 and CD86, thereby blocking interaction with CD28 and inhibiting autoimmune T-cell activation | Rheumatoid arthritis (especially when refractory to TNF$\alpha$ inhibition) |
| Anakinra | Antril, Kineret | Recombinant interleukin 1 (IL1) receptor antagonist | Moderate to severe active rheumatoid arthritis in adults who have failed one or more disease-modifying antirheumatic drug |

(continued)

| Therapeutic | Trade name | Function | Examples of clinical use |
|---|---|---|---|
| Adalimumab | Humira | Human mAb that binds specifically to TNFα and blocks its interaction with p55 and p75 cell surface TNF receptors, resulting in decreased levels of inflammation markers including CRP, ESR, and IL6 | Rheumatoid arthritis, Crohn's disease, ankylosing spondylitis, psoriatic arthritis |
| Etanercept | Enbrel | Dimeric fusion protein between recombinant soluble TNF receptor and Fc portion of human immunoglobulin GI | Rheumatoid arthritis, polyarticular-course juvenile rheumatoid arthritis, psoriatic arthritis, ankylosing spondylitis, plaque psoriasis |
| Infliximab | Remicade | Chimeric mAb that binds and neutralizes TNFα, preventing induction of pro-inflammatory cytokines, changes in endothelial permeability, activation of eosinophils and neutrophils, induction of acute phase reactants, and enzyme elaboration by synoviocytes and/or chondrocytes | Rheumatoid arthritis, Crohn's disease, ankylosing spondylitis, psoriatic arthritis, plaque psoriasis |
| Alefacept | Amevive | Dimeric fusion protein that binds CD2 on the surface of lymphocytes and inhibits interaction with LFA3; this association is important for the activation of T lymphocytes in psoriasis | Adults with moderate to severe chronic plaque psoriasis who are candidates for systemic therapy or phototherapy |
| Efalizumab | Raptiva | Humanized mAb directed against CD11a | Adults with chronic moderate to severe plaque psoriasis who are candidates for systemic therapy or phototherapy |
| Natalizumab | Tysabri | Mechanism unknown; humanized mAb that binds to the α4-subunit of α4β1 and α4β7 integrins, blocking their interactions with VCAM1 and MadCAM1, respectively | Relapsing multiple sclerosis |
| Eculizumab | Soliris | Humanized mAb that binds complement protein C5 and inhibits its cleavage to C5a and C5b, preventing the formation of the terminal complement complex C5b-9 | Paroxysmal nocturnal haemoglobinuria |
| ***Enzymatic degradation of macromolecules*** | | | |
| Botulinum toxin type A | Botox | Cleaves SNAP25 at neuromuscular junctions to disrupt SNARE complex and prevent acetylcholine release, causing flaccid paralysis | Many types of dystonia, particularly cervical; cosmetic uses |
| Botulinum toxin type B | Myoblock | Cleaves synaptobrevin at neuromuscular junctions to disrupt SNARE complex and prevent acetylcholine release, causing flaccid paralysis | Many types of dystonia, particularly cervical; cosmetic uses |

(continued)

| Therapeutic | Trade name | Function | Examples of clinical use |
|---|---|---|---|
| Collagenase | Collagenase, Santyl | Collagenase obtained from fermentation by *Clostridium histolyticum*; digests collagen in necrotic base of wounds | Debridement of chronic dermal ulcers and severely burned areas |
| Human deoxyribonuclease I, dornase-$\alpha$ | Pulmozyme | Degrades DNA in purulent pulmonary secretions | Cystic fibrosis; decreases respiratory tract infections in selected patients with FVC greater than 40% of predicted |
| Hyaluronidase (bovine, ovine) | Amphadase (bovine), Hydase (bovine), Vitrase (ovine) | Catalyses the hydrolysis of hyaluronic acid to increase tissue permeability and allow faster drug absorption | Used as an adjuvant to increase the absorption and dispersion of injected drugs, particularly anaesthetics in ophthalmic surgery and certain imaging agents |
| Hyaluronidase (recombinant human) | Hylenex | Catalyses the hydrolysis of hyaluronic acid to increase tissue permeability and allow faster drug absorption | Used as an adjuvant to increase the absorption and dispersion of injected drugs, particularly anaesthetics in ophthalmic surgery and certain imaging agents |
| Papain | Accuzyme, Panafil | Protease from the Carica papaya fruit | Debridement of necrotic tissue or liquefication of slough in acute and chronic lesions, such as pressure ulcers, varicose and diabetic ulcers, burns, postoperative wounds, pilonidal cyst wounds, carbuncles, and other wounds |
| ***Enzymatic degradation of small-molecule metabolites*** | | | |
| L-Asparaginase | ELSPAR | Provides exogenous asparaginase activity, removing available asparagine from serum; purified from *Escherichia coli* | Acute lymphocytic leukaemia, which requires exogenous asparagine for proliferation |
| Peg-asparaginase | Oncaspar | Provides exogenous asparaginase activity, removing available asparagine from serum; purified from *E. coli* | Acute lymphocytic leukaemia, which requires exogenous asparagine for proliferation |
| Rasburicase | Elitek | Catalyzes enzymatic oxidation of uric acid into an inactive, soluble metabolite (allantoin); originally isolated from *Aspergillus flavus* | Paediatric patients with leukaemia, lymphoma, and solid tumours who are undergoing anticancer therapy that may cause tumour lysis syndrome |
| ***Haemostasis and thrombosis*** | | | |
| Lepirudin | Refludan | Recombinant hirudin, a thrombin inhibitor from the salivary gland of the medicinal leech *Hirudo medicinalis* | Heparin-induced thrombocytopaenia |
| Bivalirudin | Angiomax | Synthetic hirudin analogue; specifically binds both the catalytic site and the anion-binding exosite of circulating and clot-bound thrombin | Reduce blood-clotting risk in coronary angioplasty and heparin-induced thrombocytopaenia |

(continued)

| Therapeutic | Trade name | Function | Examples of clinical use |
|---|---|---|---|
| Streptokinase | Streptase | Converts plasminogen to plasmin; produced by group C β-haemolytic streptococci | Acute evolving transmural myocardial infarction, pulmonary embolism, deep vein thrombosis, arterial thrombosis or embolism, occlusion of arteriovenous cannula |
| Anistreplase (anisoylated plasminogen streptokinase activator complex; APSAC) | Eminase | Converts plasminogen to plasmin; p-anisoyl group protects the catalytic centre of the plasminogen-streptokinase complex and prevents premature deactivation, thereby providing longer duration of action than streptokinase | Thrombolysis in patients with unstable angina |
| ***Haemostasis and thrombosis*** | | | |
| Alteplase (tissue plasminogen activator; tPA) | Activase | Promotes fibrinolysis by binding fibrin and converting plasminogen to plasmin | Pulmonary embolism, myocardial infarction, acute ischaemic stroke, occlusion of central venous access devices |
| Reteplase (deletion mutein of tPA) | Retavase | Contains the non-glycosylated kringle 2 and protease domains of human tPA; functions similarly to tPA | Management of acute myocardial infarction, improvement of ventricular function |
| Tenecteplase | TNKase | tPA with greater specificity for plasminogen conversion; has amino-acid substitutions of Thr103 to Asp, Asp117 to Gln, and Ala for amino-acids 296-299 | Acute myocardial infarction |
| Urokinase | Abbokinase | Nonrecombinant plasminogen activator derived from human neonatal kidney cells | Pulmonary embolism |
| Factor VIIa | NovoSeven | Pro-thrombotic (activated factor VII; initiates the coagulation cascade) | Haemorrhage in patients with haemophilia A or B and inhibitors to factor VIII or factor IX |
| Drotrecogin-α (activated protein C) | Xigris | Antithrombotic (inhibits coagulation factors Va and VIIIa), antiinflammatory | Severe sepsis with a high risk of death |
| ***Endocrine disorders*** | | | |
| Salmon calcitonin | Fortical, Miacalcin | Mechanism unknown; inhibits osteoclast function | Postmenopausal osteoporosis |
| Teriparatide (human parathyroid hormone residues 1-34) | Forteo | Markedly enhances bone formation; administered as a once-daily injection | Severe osteoporosis |

(continued)

| Therapeutic | Trade name | Function | Examples of clinical use |
|---|---|---|---|
| Exenatide | Byetta | Incretin mimetic with actions similar to glucagon-like peptide 1 (GLP1); increases glucose-dependent insulin secretion, suppresses glucagon secretion, slows gastric emptying, decreases appetite (first identified in saliva of the Gila monster *Heloderma suspectum)* | Type 2 diabetes resistant to treatment with metformin and a sulphonylurea |
| ***Growth Regulation*** | | | |
| Octreotide | Sandostatin | Potent somatostatin analogue; inhibits growth hormone, glucagon and insulin | Acromegaly, symptomatic relief of VIP-secreting adenoma and metastatic carcinoid tumours |
| Dibotermin-α (recombinant human bone morphogenic protein 2; rhBMP2) | Infuse | Mechanism unknown | Spinal fusion surgery, bone injury repair |
| Recombinant human bone morphogenic protein 7 (rhBMP7) | Osteogenic protein 1 | Mechanism unknown | Tibial fracture nonunion, lumbar spinal fusion |
| Histrelin acetate (gonadotropin releasing hormone; GnRH) | Supprelin LA, Vantas | Synthetic analogue of human GnRH; acts as a potent inhibitor of gonadotropin secretion when administered continuously by causing a reversible downregulation of GnRH receptors in the pituitary and desensitizing the pituitary gonadotropes | Precocious puberty |
| Palifermin (keratinocyte growth factor; KGF) | Kepivance | Recombinant analogue of KGF; stimulates keratinocyte growth in skin, mouth, stomach and colon | Severe oral mucositis in patients undergoing chemotherapy |
| Becaplermin (platelet -derived growth factor; PDGF) | Regranex | Promotes wound healing by enhancing granulation tissue formation and fibroblast proliferation and differentiation | Debridement adjunct for diabetic ulcers |
| *Other* | | | |
| Trypsin | Granule x | Proteolysis | Decubitus ulcer, varicose ulcer, debridement of eschar, dehiscent wound, sunburn |
| Nesiritide | Natrecor | Recombinant B-type natriuretic peptide | Acute decompensated congestive heart failure |
| ***Transplantation*** | | | |
| Antithymocyte globulin (rabbit) | Thymoglobulin | Selective depletion of T cells; exact mechanism unknown | Acute kidney transplant rejection, aplastic anaemia |

(continued)

| Therapeutic | Trade name | Function | Examples of clinical use |
|---|---|---|---|
| Basiliximab | Simulect | Chimeric (human/mouse) IgG1 that blocks cellular immune response in graft rejection by binding the alpha chain of CD25 (IL2 receptor) and thereby inhibiting the IL2-mediated activation of lymphocytes | Prophylaxis against allograft rejection in renal transplant patients receiving an immunosuppressive regimen including cyclosporine and corticosteroids |
| Daclizumab | Zenapax | Humanized IgG1 mAb that blocks cellular immune response in graft rejection by binding the alpha chain of CD25 (IL2 receptor) and thereby inhibiting the IL2-mediated activation of lymphocytes | Prophylaxis against acute allograft rejection in patients receiving renal transplants |
| Muromonab-CD3 | Orthoclone, OKT3 | Murine mAb that binds CD3 and blocks T-cell function | Acute renal allograft rejection or steroid-resistant cardiac or hepatic allograft rejection |
| ***Pulmonary disorders*** | | | |
| Omalizumab | Xolair | Humanized mAb that inhibits IgE binding to the high-affinity IgE receptor on mast cells and basophils, decreasing activation of these cells and release of inflammatory mediators | Adults and adolescents (at least 12 years old) with moderate to severe persistent asthma who have a positive skin test or in vitro reactivity to a perennial aeroallergen and whose symptoms are inadequately controlled with inhaled corticosteroids |
| Palivizumab | Synagis | Humanized IgG1 mAb that binds the A antigenic site of the F protein of respiratory syncytial virus | Prevention of respiratory syncytial virus infection in high-risk paediatric patients |
| ***Infectious diseases*** | | | |
| Enfuvirtide | Fuzeon | 36 amino-acid peptide that inhibits HIV entry into host cells by binding to the HIV envelope protein gp120/gp41 | Adults and children (at least 6 years old) with advanced HIV infection |
| ***Haemostasis and thrombosis*** | | | |
| Abciximab | ReoPro | Fab fragment of chimeric (human/ mouse) mAb 7E3 that inhibits platelet aggregation by binding to the glycoprotein IIb/IIIa integrin receptor | Adjunct to aspirin and heparin for prevention of cardiac ischaemia in patients undergoing percutaneous coronary intervention or patients about to undergo percutaneous coronary intervention with unstable angina not responding to medical therapy |
| ***Endocrine disorders*** | | | |
| Pegvisomant | Somavert | Recombinant human growth hormone conjugated to PEG; blocks the growth hormone receptor | Acromegaly |
| *Other* | | | |

(continued)

| Therapeutic | Trade name | Function | Examples of clinical use |
|---|---|---|---|
| Crotalidae polyvalent immune Fab (ovine) | Crofab | Mixture of Fab fragments of IgG that bind and neutralize venom toxins of ten clinically important North American Crotalidae snakes | Crotalidae envenomation (Western diamondback, Eastern diamondback and Mojave rattlesnakes, and water moccasins) |
| Digoxin immune serum Fab (ovine) | Digifab | Monovalent Fab immunoglobulin fragment obtained from sheep immunized with a digoxin derivative | Digoxin toxicity |
| Ranibizumab | Lucentis | Humanized mAb fragment that binds isoforms of vascular endothelial growth factor A (VEGFA) | Neovascular age-related macular degeneration |
| ***In vivo infectious disease diagnostics*** | | | |
| Recombinant purified protein derivative (DPPD) | DPPD | Noninfectious protein from *Mycobacterium tuberculosis* | Diagnosis of tuberculosis exposure |
| ***Hormones*** | | | |
| Glucagon | GlucaGen | Pancreatic hormone that increases blood glucose by stimulating the liver to convert glycogen to glucose | Diagnostic aid to slow gastrointestinal motility in radiographic studies; reversal of hypoglycaemia |
| Growth hormone releasing hormone (GHRH) | Geref | Recombinant fragment of GHRH that stimulates growth hormone release by somatotroph cells of the pituitary gland | Diagnosis of defective growth-hormone secretion |
| Secretin | ChiRhoStim (human peptide), SecreFlo (porcine peptide) | Stimulation of pancreatic secretions and gastrin | Aid in the diagnosis of pancreatic exocrine dysfunction or gastrinoma; facilitates identification of the ampulla of Vater and accessory papilla during endoscopic retrograde cholangiopancreato graph y |
| Thyroid stimulating hormone (TSH), thyrotropin | Thyrogen | Stimulates thyroid epithelial cells or well-differentiated thyroid cancer tissue to take up iodine and produce and secrete thyroglobulin, triiodothyronine and thyroxine | Adjunctive diagnostic for serum thyroglobulin testing in the follow-up of patients with well-differentiated thyroid cancer |
| ***Imaging agents, cancer*** | | | |
| Capromab pendetide | ProstaScint | Imaging agent; indium-111-labelled anti-PSA antibody; recognizes intracellular PSA | Prostate cancer detection |
| Indium-111-octreotide | OctreoScan | Imaging agent; indium-111-labelled octreotide | Neuroendocrine tumour and lymphoma detection |
| Satumomab pendetide | OncoScint | Imaging agent; indium-111-labelled mAb specific for tumour-associated glycoprotein (TAG-72) | Colon and ovarian cancer detection |
| Arcitumomab | CEA-scan | Imaging agent; technetium-labelled anti-CEA antibody | Colon and breast cancer detection |

(continued)

| Therapeutic | Trade name | Function | Examples of clinical use |
|---|---|---|---|
| Nofetumomab | Verluma | Imaging agent; technetium-labelled antibody specific for small-cell lung cancer | Small-cell lung cancer detection and staging |
| ***Imaging agents, other*** | | | |
| Apcitide | Acutect | Imaging agent; technetium-labelled synthetic peptide; binds GPIIb/IIIa receptors on activated platelets | Imaging of acute venous thrombosis |
| Imciromab pentetate | Myoscint | Imaging agent; indium-111-labelled antibody specific for human cardiac myosin | Detects presence and location of myocardial injury in patients with suspected myocardial infarction |
| Technetium fanolesomab | NeutroSpec | Imaging agent; technetium-labelled anti-CD 15 antibody; binds neutrophils that infiltrate sites of infection | Diagnostic agent (used in patients with equivocal signs and symptoms of appendicitis) |
| ***Examples of in vitro diagnostics*** | | | |
| HIV antigens | Enzyme immunoassay, OraQuick, Uni-Gold | Detects human antibodies to HIV (enzyme immunoassay, western blot) | Diagnosis of HIV infection |
| Hepatitis C antigens | Recombinant immunoblot assay (RIBA) | Detects human antibodies to hepatitis C virus | Diagnosis of hepatitis C exposure |
| Deninleukin diftitox | Ontak | Directs the cytocidal action of diphtheria toxin to cells expressing the IL2 receptor | Persistent or recurrent cutaneous T-cell lymphoma whose malignant cells express the CD25 component of the IL2 receptor |
| Ibritumomab tiuxetan | Zevalin | A mAb portion that recognizes $CD20^+$ B cells and induces apoptosis while the chelation site allows either imaging (In-111) or cellular damage by beta emission (Y-90) | Relapsed or refractory low-grade, follicular, or transformed B-cell non-Hodgkin's lymphoma (NHL), including rituximab-refractory follicular NHL |
| Gemtuzumab ozogamicin | Mylotarg | Humanized anti-CD33 IgG4κ mAb conjugated to calicheamicin, a small-molecule chemotherapeutic agent | Relapsed $CD33^+$ acute myeloid leukaemia in patients who are more than 60 years old and are not candidates for cytotoxic chemotherapy |
| Tositumomab and $^{131}$I-tositumomab | Bexxar, Bexxar 1-131 | Tositumomab is a mAb that binds CD20 surface antigen and stimulates apoptosis. Tositumomab coupled to radioactive iodine-131 binds CD20 surface antigen and delivers cytotoxic radiation | $CD20^+$ follicular NHL, with and without transformation, in patients whose disease is refractory to rituximab and has relapsed following chemotherapy; tositumomab and then $^{131}$I-tositumomab are used sequentially in the treatment regimen |
| ***Protecting against a deleterious foreign agent (IIIa)*** | | | |

(continued)

| Therapeutic | Trade name | Function | Examples of clinical use |
|---|---|---|---|
| Hepatitis B surface antigen (HBsAg) | Engerix, Recombivax HB | Non-infectious protein on surface of hepatitis B virus | Hepatitis B vaccination |
| HPV vaccine | Gardasil | Quadrivalent HPV recombinant vaccine (strains 6, 11, 16, 18); contains major capsid proteins from four HPV strains | Prevention of HPV infection |
| OspA | LYMErix | Non-infectious lipoprotein on outer surface of *Borrelia burgdorferi* | Lyme disease vaccination |
| ***Treating an autoimmune disease (IIIB)*** | | | |
| Anti-Rhesus (Rh) immunoglobulin G | Rhophylac | Neutralizes Rh antigens that could otherwise elicit anti-Rh antibodies in an Rh-negative individual | Routine antepartum and postpartum prevention of Rh(D) immunization in Rh(D)-negative women; Rh prophylaxis in case of obstetric complications or invasive procedures during pregnancy; suppression of Rh immunization in Rh(D)-negative individuals transfused with Rh(D)-positive red blood cells |

Exemplary therapeutic agents that are useful for encapsulation in therapeutic-loaded exosomes of the present invention are provided in Table 1, above. Accordingly, in certain embodiments, the present invention provides a therapeutic-loaded exosome, wherein the therapeutic agent is selected from any of those set forth in Table 1, above. In some embodiments, the present invention provides a therapeutic-loaded exosome, wherein the therapeutic agent is selected from those described herein, below.

In some embodiments, the therapeutic is an incretin mimetic or derivative of an incretin (e.g. human incretin), such as liraglutide (Victoza®, Saxenda®), semaglutide, exenatide (Byetta®, Bydureon®), or dulaglutide (Trulicity®); or octreotide, calcitonin (including salmon calcitonin), parathyroid hormone (PTH), teriparatide (a recombinant form of PTH) insulin, a peptide agonist of GLP-1 such as exenatide, liraglutide, lixisenatide, albiglutide and/or dulaglutide, a GLP-1/GIP co-agonist, a GLP-2 agonist, or a peptide GPCR agonist.

In one aspect, a therapeutic-loaded exosome according to the present invention is useful as a diagnostic, prognostic, or therapeutic in the context of cancer, autoimmune disorders, liver disorders, gene therapy, immuno-oncology, and other diseases, disorders, and conditions as described in detail herein.

In another aspect, a therapeutic-loaded exosome according to the present invention is useful in treating, preventing, or ameliorating a hyperproliferative disorder, viral or microbial infection, autoimmune disease, allergic condition, inflammatory disease, disorder, or condition, cardiovascular disease, metabolic disease, or neurodegenerative disease.

In some embodiments, the therapeutic agent is an allergen, adjuvant, antigen, or immunogen. In some embodiments, the allergen, antigen, or immunogen elicits a desired immune response to increase allergen tolerance or reduce the likelihood of an allergic or immune response such as anaphylaxis, bronchial inflammation, airway constriction, or asthma. In some embodiments, the allergen, antigen, or immunogen elicits a desired immune response to increase viral or pathogenic resistance or elicit an anticancer immune response. In some embodiments, the allergen or antigen elicits a desired immune response to treat an allergic or autoimmune disease. In some embodiments, the therapeutic agent increases immunological tolerance to treat an autoimmune disease or decreases an autoimmune response to treat an autoimmune disease.

As used herein, the term "adjuvant" refers to any substance which enhances an immune response (e.g. in the vaccine, autoimmune, or cancer context) by a mechanism such as: recruiting of professional antigen-presenting cells (APCs) to the site of antigen exposure; increasing the delivery of antigens by delayed/slow release (depot generation); immunomodulation by cytokine production (selection of Th1 or Th2 response); inducing T-cell response (prolonged exposure of peptide-MHC complexes (signal 1) and stimulation of expression of T-cell-activating co-stimulators (signal 2) on an APC surface) and targeting (e.g. carbohydrate adjuvants which target lectin receptors on APCs), and the like.

In some embodiments, the allergen is selected from a food, animal (e.g. pet such as dog, cat, or rabbit), or

environmental allergen (such as dust, pollen, or mildew). In some embodiments, the allergen is selected from abalone, perlemoen, acerola, alaska pollock, almond, aniseed, apple, apricot, avocado, banana, barley, bell pepper, brazil nut, buckwheat, cabbage, camomile, carp, carrot, casein, cashew, castor bean, celery, celeriac, cherry, chestnut, chickpea, garbanzo, bengal gram, cocoa, coconut, cod, cotton seed, courgette, zucchini, crab, date, egg (e.g. hen's egg), fig, fish, flax seed, linseed, frog, garden plum, garlic, gluten, grape, hazelnut, kiwi fruit (chinese gooseberry), legumes, lentil, lettuce, lobster, lupin or lupine, lychee, mackerel, maize (corn), mango, melon, milk (e.g. cow), molluscs, mustard, oat, oyster, peach, peanut (or other ground nuts or monkey nuts), pear, pecan, persimmon, pistachio, pine nuts, pineapple, pomegranate, poppy seed, potato, pumpkin, rice, rye, salmon, sesame, shellfish (e.g. crustaceans, black tiger shrimp, brown shrimp, greasyback shrimp, Indian prawn, neptune rose shrimp, white shrimp), snail, soy, soybean (soya), squid, strawberry, sulfur dioxide (sulphites), sunflower seed, tomato, tree nuts, tuna, turnip, walnut, or wheat (e.g. breadmaking wheat, pasta wheat, kamut, spelt).

In some embodiments, the allergen is selected from an allergenic protein, peptide, oligo- or polysaccharide, toxin, venom, nucleic acid, or other allergen, such as those listed at http://www.allergenonline.org/databasebrowse.shtml. In some embodiments, the allergen is selected from an airborne fungus, mite or insect allergen, plant allergen, venom or salivary allergen, animal allergen, contact allergen, parasitic allergen, or bacterial airway allergen.

In some embodiments, the therapeutic agent is an autoimmune antigen. In some embodiments, the autoimmune antigen is selected from an antigen against a disease, disorder, or condition listed in Table 2, below. In some embodiments, the antigen is selected from those listed in Table 2, below.

**Table 2: Exemplary Autoimmune Diseases and Exemplary Antigens**

| AAA Disease Name (101) | Antigen |
|---|---|
| Achlorhydria | against parietal cells which normally produce gastric acid |
| Acute disseminated encephalomyelitis | MOG |
| Addison's Disease | antibodies against 21-hydroxylase enzyme |
| Alopecia areata | antibodies against hair follicles |
| Anemia, Pernicious | antibodies to parietal cells and intrinsic factor |
| Ankylosing spondylitis | Anti-neutrophil cytoplasmic antibodies (ANCAs) |
| Anti - Glomerular Basement Membrane Disease | |
| Anti-GBM/Anti-TBM nephritis | |
| Anti-NMDA receptor encephalitis | N-methyl-D-aspartate receptor (NMDA) |
| Antiphospholipid syndrome (APS) | Antiphospholipid antibodies |
| Aplastic Anemia | |
| Autoimmune Atrophic Gastritis | |
| Autoimmune Hearing Loss | |
| Autoimmune hemolytic anemia | |
| Autoimmune Hepatitis | Antinuclear, anti mitochondrial and anti-smooth muscle antibodies, Liver kidney microsomal type 1 antibody, Anti-smooth muscle antibody |
| Autoimmune hypoparathyroidism | |
| Autoimmune hypophysitis | |
| Autoimmune inner ear disease (AIED) | |
| Autoimmune Lymphoproliferative | |
| Autoimmune Myocarditis | |
| Autoimmune oophoritis | |
| Autoimmune orchitis | spermatozoa normally sequestered in the testis (occurs after vasectomy) |

(continued)

| AAA Disease Name (101) | Antigen |
|---|---|
| Autoimmune Polyendocrinopathy - Candidiasis - Ectodermal - Dystrophy | NA |
| Autoimmune Syndrome Type II, Polyglandular | |
| Axonal & neuronal neuropathy (AMAN) | Anti-ganglioside antibodies GD3 |
| Behcet Syndrome | Anti-p62 antibodies, Anti-splOO antibodies, Anti-glycoprotein-210 antibodies |
| Biliary Cirrhosis | Anti-mitochondrial antibody |
| Bullous pemphigoid | |
| Castleman disease (CD) | |
| Celiac disease | Synapsin 1, transglutaminase, gluten |
| Chagas disease | |
| Cholangitis, Sclerosing | |
| Chronic inflammatory demyelinating polyneuropathy (CIDP) | |
| Chronic lymphocytic thyroiditis | |
| Chronic recurrent multifocal osteomyelitis (CRMO) | |
| Churg-Strauss syndrome | |
| Cicatricial pemphigoid/benign mucosal pemphigoid | |
| Cogan’s syndrome | |
| Cold agglutinin disease | |
| Colitis, Ulcerative | |
| Congenital heart block | |
| Coxsackie myocarditis | |
| CREST syndrome | Anti-centromere antibodies |
| Crohn’s disease | |
| Cryoglobulinemia | |
| Cushing Syndrome | |
| Dermatitis herpetiformis | |
| Dermatomyositis | |
| Devic’s disease (neuromyelitis optica) | |
| Diabetes Mellitus, Insulin - Dependent | intracellular islet cell antigens such as glutamic acid decarboxylase |
| Diabetes, Type 1 | islet cell autoantibodies, insulin autoantibodies, autoantibodies targeting the 65-kDa isoform of glutamic acid decarboxylase (GAD), autoantibodies targeting the phosphatase-related IA-2 molecule, and zinc transporter autoantibodies (ZnT8) |
| Diffuse Cerebral Sclerosis of Schilder | |
| Discoid lupus | |
| Dressler’s syndrome | |

(continued)

| AAA Disease Name (101) | Antigen |
|---|---|
| Encephalomyelitis,Autoimmune,Experimental | |
| Endometriosis | |
| Eosinophilic esophagitis (EoE) | |
| Eosinophilic fasciitis | |
| Epidermolysis Bullosa Acquisita | |
| Erythema nodosum | |
| Erythematosis | |
| Essential mixed cryoglobulinemia | |
| Evans syndrome | |
| Felty's Syndrome | |
| Fibromyalgia | |
| Fibrosing alveolitis | |
| Giant cell arteritis (temporal arteritis) | |
| Giant cell myocarditis | |
| Glomerulonephritis, IGA | renal autoantigen |
| Glomerulonephritis, Membranous | |
| Goodpasture Syndrome | collagen in basement membrane of kidneys and lungs |
| Granulomatosis with polyangiitis | Anti-neutrophil cytoplasmic antibody (C-ANCA) |
| Graves' Disease | antibodies against the TSH receptor, thyroid-stimulating immunoglobulin (TSI), thyroglobulin or the thyroid hormones T3 and T4 |
| Guillain - Barre Syndrome | myelin protein |
| HAM/tropical spastic paraparesis | hnRNP A1 |
| Hamman-Rich syndrome | |
| Hashimoto's Thyroidosis | thyroid antigens : (a) Thyroglobulin, (b) Thyroid peroxidase, (c) TSH receptor, and (d) Iodine transporter |
| Hemolytic anemia | |
| Henoch-Schonlein purpura (HSP) | |
| Hepatitis, Chronic Active | |
| Herpes gestationis or pemphigoid gestationis (PG) | |
| Hypogammalglobulinemia | |
| Idiopathic thrombocytopenia | |
| IgA Nephropathy | |
| IgG4-related sclerosing disease | |
| Inclusion body myositis (IBM) | |
| Inflammatory Bowel Diseases | |
| Interstitial cystitis (IC) | |
| Juvenile myositis (JM) | |
| Kawasaki disease | |

(continued)

| AAA Disease Name (101) | Antigen |
|---|---|
| Lambert - Eaton Myasthenic Syndrome | voltage-gated calcium channel (P/Q-type) |
| Lens-induced uveitis | |
| Leukocytoclastic vasculitis | |
| Lichen planus | |
| Lichen sclerosus | |
| Lichen Sclerosus et Atrophicus | |
| Ligneous conjunctivitis | |
| Limbic encephalitis | AMPAR (GluRl, GluR2), Anti-Hu (ANNA-1), Lgil, NMDAR, NR1/NR2B, voltage-gated potassium channel (VGKC) |
| Linear IgA disease (LAD) | |
| Lupus Erythematosus, Discoid | |
| Lupus Erythematosus, Systemic | double stranded DNA and Smith (Sm) antigen, Anti-histone antibodies, Anti-SSA/Ro autoantibodies, anti-thrombin antibodies, NR2A/NR2B, Neuronal surface P antigen |
| Lupus Hepatitis | |
| Lyme disease chronic | |
| Lymphopenia | |
| Meniere’s disease | |
| Microscopic polyangiitis (MPA) | Anti-neutrophil cytoplasmic antibody (P-ANCA) |
| Mixed connective tissue disease (MCTD) | anti-Ribonucleoprotein antibodies |
| Mooren’s ulcer | |
| Mucha-Habermann disease | |
| Mucocutaneous Lymph Node Syndrome | |
| Multifocal motor neuropathy with conduction block (MMN) | Anti-ganglioside antibodies to GM1 |
| Multiple Sclerosis | |
| Myasthenia Gravis | nicotinic acetylcholine receptor of neuromuscular junction, Muscle-specific kinase (MUSK) |
| Myelitis, Transverse | |
| Myocarditis | |
| Myositis | |
| Narcolepsy | |
| Neuritis, Autoimmune, Experimental | |
| Neuromyelitis optica | AQP4, Collapsin response mediator protein 5 |
| Neutropenia | |
| Ocular cicatricial pemphigoid | |
| Oculovestibuloauditory syndrome | |
| Ophthalmia, Sympathetic | |
| Opsoclonus - Myoclonus Syndrome | |

(continued)

| AAA Disease Name (101) | Antigen |
|---|---|
| Optic neuritis | |
| Palindromic rheumatism (PR) | |
| Pancreatitis | |
| PANDAS (Pediatric Autoimmune Neuropsychiatric Disorders Associated with Streptococcus) | |
| Paraneoplastic cerebellar degeneration (PCD) | Anti-Hu (ANNA-1), Anti-Yo, Anti-Tr, anti-amphiphysin |
| Paroxysmal nocturnal hemoglobinuria (PNH) | |
| Parry Romberg syndrome | |
| Pars planitis (peripheral uveitis) | |
| Parsonnage-Turner syndrome | |
| Pemphigoid, Bullous | |
| Pemphigus | |
| Pemphigus foliaceous | |
| Pemphigus Vulgaris | |
| Peripheral neuropathy | |
| Perivenous encephalomyelitis | |
| POEMS syndrome (polyneuropathy, organomegaly, endocrinopathy, monoclonal gammopathy, skin changes) | |
| Polyarteritis nodosa | |
| Polychondritis, Relapsing | |
| Polyendocrinopathies, Autoimmune | |
| Polymyalgia Rheumatica | |
| Polymyositis | anti-signal recognition particle, Anti-PM-Scl |
| Polyradiculo neuropathy | |
| Postmyocardial infarction syndrome | |
| Postpericardiotomy syndrome | |
| Poststreptococcal movement disorders, Sydenham's chorea, and PANDAS | Lysoganglioside dopamine D2 receptor, Tubulin |
| Primary biliary cirrhosis | Antimitochondrial antibodies |
| Primary sclerosing cholangitis | |
| Progesterone dermatitis | |
| Psoriasis | |
| Psoriatic arthritis | |
| Pure red cell aplasia (PRCA) | |
| Pyoderma gangrenosum | |
| Rasmussen encephalitis | GluR3 |
| Raynaud's phenomenon | |
| Reactive Arthritis | |

(continued)

| AAA Disease Name (101) | Antigen |
|---|---|
| Reflex sympathetic dystrophy | |
| Reiter's syndrome | autoimmune response involving cross-reactivity of bacterial antigens with joint tissues or by bacterial antigens that have somehow become deposited in the joints |
| Relapsing polychondritis | |
| Restless legs syndrome (RLS) | |
| Retroperitoneal fibrosis | |
| Rheumatic Fever | M proteins of Streptococcus pyogenes |
| Rheumatoid Arthritis | Fc portion of IgG, anti-cyclic citrullinated peptide (Anti-CCP), Rheumatoid factor |
| Sarcoidosis | |
| Schmidt syndrome | |
| Scleritis | |
| Scleroderma | Anti-topoisomerase antibodies |
| Sjogren's syndrome | Anti-La/SS-Bautoantibodies |
| Sperm & testicular autoimmunity | |
| Stiff-person syndrome | GAD, Gephrin, GABA(B) receptor, amphiphysin |
| Still's Disease, Adult Onset | |
| Subacute bacterial endocarditis (SBE) | |
| Susac's syndrome | |
| Sympathetic ophthalmia (SO) | |
| Takayasu's arteritis | |
| Temporal Arteritis | |
| Temporal arteritis/Giant cell arteritis | |
| Thrombocytopenic purpura (TTP) | |
| Thyrotoxicosis | |
| Tolosa-Hunt syndrome (THS) | |
| Transverse myelitis | |
| Ulcerative colitis (UC) | |
| Undifferentiated connective tissue disease (UCTD) | |
| Uveitis | |
| Uveomeningoencephalitic Syndrome | |
| Vasculitis | |
| Vitiligo | immune system attacking and destroying the melanocytes |

In some embodiments, the autoimmune antigen treats, prevents, or ameliorates an autoimmune disease, such as Rheumatoid Arthritis, Diabetes Mellitus, Insulin-DependentLupus Erythematosus (Systemic), Multiple Sclerosis, Psoriasis, Pancreatitis, Inflammatory Bowel Diseases, Crohn's disease, ulcerative colitis, Sjogren's Syndrome, autoimmune encephalomyelitis, experimental Graves' Disease, Sarcoidosis, Scleroderma, primary biliary cirrhosis, Chronic lymphocytic thyroiditis, Lymphopenia, Celiac Disease, Myocarditis, Chagas Disease, Myasthenia Gravis, Glomerulonephritis, IGA, Aplastic Anemia, Lupus Nephritis, Hamman-Rich syndrome, Hepatitis, Chronic Active Dermatomyositis, Glomerulonephritis, Membranous Mucocutaneous Lymph Node Syndrome, Pemphigoid, Bullous Behcet Syndrome, Spondylitis, Ankylosing Hepatitis, Autoimmune Cushing Syndrome, Guillain-Barre Syndrome, Cholangitis, Sclerosing Antiphospholipid Syndrome, Vitiligo, Thyrotoxicosis, Wegener's Granulomatosis, idiopathic purpura, Raynaud's Thrombocytopenia, Autoimmune hemolytic anemia, Cryoglobulinemia, Mixed Connective Tissue Disease, Temporal Arteritis, Pemphigus Vulgaris, Addison's Disease, Rheumatic Fever, pernicious anemia, Alopecia Areata, Lupus Erythematosus, Discoid Narcolepsy, Takayasu's Arteritis, autoimmune neuritis, Experimental Polyarteritis Nodosa, Polymyalgia Rheumatica, Dermatitis Herpetiformis, Autoimmune Myocarditis, Meniere's Disease, Chronic Inflammatory Demyelinating Polyneuropathy, Lambert-Eaton Myasthenic Syndrome, Lichen Sclerosus et Atrophicus, Churg-Strauss Syndrome, Erythematosis, Reiter Disease, Anti-Glomerular Basement Membrane Disease, autoimmune polyendocrinopathies, Felty's Syndrome, Goodpasture Syndrome, Achlorhydria, Autoimmune Lymphoproliferative Polyradiculoneuropathy, Uveomeningoencephalitic Syndrome, Polychondritis, Relapsing Atopic Allergy, Idiopathic thrombocytopenia, Stiff-Person Syndrome, Autoimmune Polyendocrinopathy-Candidiasis-Ectodermal-Dystrophy, Epidermolysis, Bullosa Acquisita, Autoimmune orchitis, Oculovestibuloauditory syndrome, Ophthalmia, Sympathetic Myelitis, Transverse Diffuse Cerebral Sclerosis of Schilder, Neuromyelitis Optica, Still's Disease, Adult Onset Autoimmune oophoritis, Mooren's ulcer, Autoimmune Syndrome Type II, Polyglandular Autoimmune hypophysitis, Lens-induced uveitis, pemphigus foliaceus, Opsoclonus-Myoclonus Syndrome, Type B Insulin Resistance, Autoimmune Atrophic Gastritis, Lupus Hepatitis, Autoimmune Hearing Loss, Acute hemorrhagic leukencephalitis, autoimmune hypoparathyroidism, or Hashimoto's Thyroidosis. In some embodiments, the autoimmune antigen treats, prevents, or ameliorates an autoimmune disease, such as Addison's disease, Agammaglobulinemia, Alopecia areata, Amyloidosis, Ankylosing spondylitis, Anti-GBM/Anti-TBM nephritis, Antiphospholipid syndrome (APS), Autoimmune hepatitis, Autoimmune inner ear disease (AIED), Axonal & neuronal neuropathy (AMAN), Behcet's disease, Bullous pemphigoid, Castleman disease (CD), Celiac disease, Chagas disease, Chronic inflammatory demyelinating polyneuropathy (CIDP), Chronic recurrent multifocal osteomyelitis (CRMO), Churg-Strauss Cicatricial pemphigoid/benign mucosal pemphigoid, Cogan's syndrome, Cold agglutinin disease, Congenital heart block, Coxsackie myocarditis, CREST syndrome, Crohn's disease, Dermatitis herpetiformis, Dermatomyositis, Devic's disease (neuromyelitis optica), Discoid lupus, Dressier's syndrome, Endometriosis, Eosinophilic esophagitis (EoE), Eosinophilic fasciitis, Erythema nodosum, Essential mixed cryoglobulinemia, Evans syndrome, Fibromyalgia, Fibrosing alveolitis, Giant cell arteritis (temporal arteritis), Giant cell myocarditis, Glomerulonephritis, Goodpasture's syndrome, Granulomatosis with Polyangiitis, Graves' disease, Guillain-Barre syndrome, Hashimoto's thyroiditis, Hemolytic anemia, Henoch-Schonlein purpura (HSP), Herpes gestationis or pemphigoid gestationis (PG), Hypogammalglobulinemia, IgA Nephropathy, IgG4-related sclerosing disease, Inclusion body myositis (IBM), Interstitial cystitis (IC), Juvenile arthritis, Juvenile diabetes (Type 1 diabetes), Juvenile myositis (JM), Kawasaki disease, Lambert-Eaton syndrome, Leukocytoclastic vasculitis, Lichen planus, Lichen sclerosus, Ligneous conjunctivitis, Linear IgA disease (LAD), Lupus, chronic Lyme disease, Meniere's disease, Microscopic polyangiitis (MPA), Mixed connective tissue disease (MCTD), Mooren's ulcer, Mucha-Habermann disease, Multiple sclerosis (MS), Myasthenia gravis, Myositis, Narcolepsy, Neuromyelitis optica, Neutropenia, Ocular cicatricial pemphigoid, Optic neuritis, Palindromic rheumatism (PR), PANDAS (Pediatric Autoimmune Neuropsychiatric Disorders Associated with Streptococcus), Paraneoplastic cerebellar degeneration (PCD), Paroxysmal nocturnal hemoglobinuria (PNH), Parry Romberg syndrome, Pars planitis (peripheral uveitis), Parsonnage-Turner syndrome, Pemphigus, Peripheral neuropathy, Perivenous encephalomyelitis, Pernicious anemia (PA),POEMS syndrome (polyneuropathy, organomegaly, endocrinopathy, monoclonal gammopathy, skin changes), Polyarteritis nodosa, Polymyalgia rheumatica, Polymyositis, Postmyocardial infarction syndrome, Postpericardiotomy syndrome, Primary biliary cirrhosis, Primary sclerosing cholangitis, Progesterone dermatitis, Psoriasis, Psoriatic arthritis, Pure red cell aplasia (PRCA), Pyoderma gangrenosum, Raynaud's phenomenon, Reactive Arthritis, Reflex sympathetic dystrophy, Reiter's syndrome, Relapsing polychondritis, Restless legs syndrome (RLS), Retroperitoneal fibrosis, Rheumatic fever, Rheumatoid arthritis (RA), Sarcoidosis, Schmidt syndrome, Scleritis, Scleroderma, Sjogren's syndrome, Sperm and testicular autoimmunity, Stiff person syndrome (SPS), Subacute bacterial endocarditis (SBE), Susac's syndrome, Sympathetic ophthalmia (SO), Takayasu's arteritis, Temporal arteritis/Giant cell arteritis, Thrombocytopenic purpura (TTP), Tolosa-Hunt syndrome (THS), Transverse myelitis, Type 1 diabetes, Ulcerative colitis (UC), Undifferentiated connective tissue disease (UCTD), Uveitis, Vasculitis, Vitiligo, or Wegener's granulomatosis (now termed Granulomatosis with Polyangiitis (GPA).

In some embodiments, the antigen is a brain reactive antigen. Exemplary brain reactive antigens are set forth in Table 3, below.

**Table 3: Brain Reactive Antigens**

| Diamond et al., 2015: Brain reactive antibodies and disease | | | | | | | | |
|---|---|---|---|---|---|---|---|---|
| **Disorder** | **Defined antigen** | **Ab in CSF** | **Ab useful in diagn osis** | **Clinical respon se** | **Ab relevant to disease mechanis m** | **Mechanis m** | **Subcellu lar site of action** | **Etiolog y** |
| | | | | | | | | |
| HAM/tropica l spastic paraparesis | hnRNP A1 (244) | Yes (245) | ND | ND | ND | Inhibits neuronal activity (246) | Intracellu lar | Molecul ar mimicry (246) |
| | | | | | | | | |
| Neuromyeliti s optica | AQP4(15 0, 151, 171) | Yes (171, 247) | Yes (154) | Yes (248), suppres sion | Yes (152, 249) | Receptor-mediated internaliza tion; compleme nt-mediated toxicity | Extracell ular | Autoim munity |
| | | | | | | | | |
| Acute disseminate d encephalom yelitis | MOG (138) | Yes (138, 139) | Yes (250) | Yes (251-253), modulati on | Yes (254) | Complem ent-mediated demyelina tion | Extracell ular | Autoim munity |
| | | | | | | | | |
| Systemic lupus erythematos us | NR2A/N R2B | Yes (106-108, 255, 256) | Yes (103-108, 255, 256) | Yes (106, 107) | Yes (2, 100, 257) | Receptor modulatio n, apoptosis (50, 100, 101) | Extracell ular | Autoim munity |
| | | | | | | | | |
| | Neuronal surface P antigen (116) | Yes (114) | Yes (258) | ND | Yes (116) | Ca2+ influx, apoptosis (116) | Extracell ular | Autoim munity |
| | | | | | | | | |
| Poststreptoc occal movement disorders, Sydenham's chorea, and PANDAS | Lysogan glioside dopamin e D2 receptor | Yes (199, 215, 216) | Yes | Yes (218) | Yes (213, 214, 217) | Aberrant cell signaling, neurotran smitter release (216, 259) | Extracell ular | Molecul ar mimicry |
| | Tubulin (199, 215, 216, 259) | | | | | | Intracellu lar | |

(continued)

| Disorder | Defined antigen | Ab in CSF | Ab useful in diagn osis | Clinical respon se | Ab relevant to disease mechanis m | Mechanis m | Subcellu lar site of action | Etiolog y |
|---|---|---|---|---|---|---|---|---|
| Diamond et al., 2015: Brain reactive antibodies and disease | | | | | | | | |
| **Disorder** | **Defined antigen** | **Ab in CSF** | **Ab useful in diagn osis** | **Clinical respon se** | **Ab relevant to disease mechanis m** | **Mechanis m** | **Subcellu lar site of action** | **Etiolog y** |
| Celiac disease | Synapsin 1 (260) | Yes | ND | Yes | Yes (260) | ND | Intracellu lar | Autoim munity/ molecul ar mimicry |
| | | | | | | | | |
| | Transglut aminase | ND | Yes (261) | Yes (262) | ND | ND | Intracellu lar | Autoim munity |
| | | | | | | | | |
| Autism | ND (238, 239) | ND | ND | ND (263) | ND (239) | ND | ND | ND |
| | | | | | | | | |
| Limbic encephalitis | AMPAR (GluR1, GluR2) | Yes | Yes | Yes | Yes | Altered receptor location (264) | Extracell ular | Autoim munity |
| | | | | | | | | |
| | NMDAR (265) [NR1/NR 2B (224)] | Yes | Yes | Yes | Yes | Receptor internaliza tion (224) | Extracell ular | Autoim munity |
| | | | | | | | | |
| | Lgi1 (24) | Yes (264) | Yes (24) | Yes (24, 266) | ND | ND (24) | Extracell ular | Autoim munity |
| | | | | | | | | |
| Rasmussen encephalitis | GluR3 (267) | Yes | Yes | Yes (268, 269) | Yes (269) | Complem ent-mediated toxicity (270) | Extracell ular | Autoim munity |

(continued)

| Disorder | Defined antigen | Ab in CSF | Ab useful in diagn osis | Clinical respon se | Ab relevant to disease mechanis m | Mechanis m | Subcellu lar site of action | Etiolog y |
|---|---|---|---|---|---|---|---|---|
| | | | | | | | | |
| Hashimoto's encephalitis | Aldehyde reductas e (271) | Yes (271) | ND | ND | ND | ND | Intracellu lar | Autoim munity |
| | Thyroglo bulin (271, 272) | | | | | | Extracell ular | |
| | | | | | | | | |
| Encephalitis lethargica | ND | Yes (273) | ND | ND | ND | ND | ND | Autoim munity |
| | | | | | | | | |
| Stiff-person | GAD | Yes | Yes | Yes | Yes (233) | ND | Intracellu | Autoim |
| Diamond et al., 2015: Brain reactive antibodies and disease | | | | | | | | |
| **Disorder** | **Defined antigen** | **Ab in CSF** | **Ab useful in diagn osis** | **Clinical respon se** | **Ab relevant to disease mechanis m** | **Mechanis m** | **Subcellu lar site of action** | **Etiolog y** |
| syndrome | (274) | (275, 276) | (233) | (274) | | | lar | munity |
| | | | | | | | | |
| | Gephryin (275) | Yes | Yes | Yes | Yes | ND | Intracellu lar | Autoim munity |
| | GABA(B) receptor (277) | | | | | | Extrace llular | |
| | | | | | | | | |
| | Amphiph ysin (233) | Yes | Yes | Yes | Yes | Synaptic inhibition (233) | Intracellu lar | Autoim munity |
| | | | | | | | | |

In some embodiments, the therapeutic agent or disease is selected from those listed in Table 4, below.

**Table 4: Exemplary Therapeutic Agents**

| Drug ID | Drug Name | CAS Number | Highest Dev. Phase | Indication |
|---|---|---|---|---|
| 800006154 | Fingolimod | 162359-56-0 | Marketed | Multiple sclerosis, Chronic inflammatory demyelinating polyradiculoneuropathy, Amyotrophic lateral sclerosis, Renal transplant rejection, Optic neuritis, Type 1 diabetes mellitus, Rheumatoid arthritis, Graft-versus-host disease, Myocarditis |
| 800031108 | Guselkumab | 1350289-85-8 | Phase III | Plaque psoriasis, Erythrodermic psoriasis, Palmoplantar pustulosis, Rheumatoid arthritis, Psoriatic arthritis |
| 800004275 | Rituximab | 174722-31-7 | Marketed | Non-Hodgkin's lymphoma, Rheumatoid arthritis, Microscopic polyangiitis, Wegener's granulomatosis, Follicular lymphoma, Chronic lymphocytic leukaemia, Nephrotic syndrome, Lymphoproliferative disorders, Diffuse large B cell lymphoma, Pemphigus vulgaris, Transplant rejection, Neuromyelitis optica, Mantle-cell lymphoma, B cell lymphoma, Multiple sclerosis, Ulcerative colitis, Sjogren's syndrome, Ocular inflammation, Scleritis, Primary biliary cirrhosis, Lupus nephritis, Systemic lupus erythematosus, Graft-versus-host disease, Dermatomyositis, Immune thrombocytopenic purpura |
| 800033563 | Ozanimod | 1306760-87-1 | Phase III | Multiple sclerosis, Ulcerative colitis, Crohn's disease |
| 800029879 | Corticotropin gel - Mallinckrodt | 9002-60-2 | Marketed | Membranous glomerulonephritis, Juvenile rheumatoid arthritis, Polymyositis, Infantile spasms, Rheumatoid arthritis, Adrenal cortex disorders, Nephrotic syndrome, Sarcoidosis, Systemic lupus erythematosus, Psoriatic arthritis, Ankylosing spondylitis, Multiple sclerosis, Diabetic nephropathies, Amyotrophic lateral sclerosis |
| 800015868 | Piclidenoson | 152918-18-8 | Phase II/III | Psoriasis, Rheumatoid arthritis, Glaucoma, Uveitis, Osteoarthritis, Dry eyes, Colorectal cancer, Solid tumours |
| 800006080 | Eculizumab | 219685-50-4 | Marketed | Paroxysmal nocturnal haemoglobinuria, Haemolytic uraemic syndrome, Myasthenia gravis, Neuromyelitis optica, Delayed graft function, Renal transplant rejection, Guillain-Barre syndrome, Heart transplant rejection, Antiphospholipid syndrome, Rheumatoid arthritis, Autoimmune haemolytic anaemia, Age-related macular degeneration, |

(continued)

| Drug ID | Drug Name | CAS Number | Highest Dev. Phase | Indication |
|---|---|---|---|---|
| | | | | Membranous glomerulonephritis, Glomerulonephritis, Systemic lupus erythematosus, Allergic asthma, Motor neuron disease, Lupus nephritis, Psoriasis, Dermatomyositis, Bullous pemphigoid, Adult respiratory distress syndrome, Immune thrombocytopenic purpura |
| 800019064 | Ocrelizumab | 637334-45-3 | Prere gistratio n | Multiple sclerosis, Systemic lupus erythematosus, Rheumatoid arthritis, Lupus nephritis, Haematological malignancies, Eye disorders |
| 800002822 | Abatacept | 332348-12-6 | Marketed | Rheumatoid arthritis, Juvenile rheumatoid arthritis, Lupus nephritis, Psoriatic arthritis, Sjogren's syndrome, Diffuse scleroderma, Nephrotic syndrome, Inflammation, Ulcerative colitis, Crohn's disease, Systemic lupus erythematosus, Multiple sclerosis, Psoriasis, Graft-versus-host disease, Transplant rejection, Xenotransplant rejection |
| 800027858 | Sarilumab | 1189541-98-7 | Prere gistratio n | Rheumatoid arthritis, Juvenile rheumatoid arthritis, Uveitis, Ankylosing spondylitis |
| 800026523 | Sirukumab | 1194585-53-9 | Prere gistratio n | Rheumatoid arthritis, Giant cell arteritis, Lupus nephritis, Asthma, Major depressive disorder, Atherosclerosis |
| 800029418 | Ixekizumab | 1143503-69-8 | Marketed | Plaque psoriasis, Psoriatic arthritis, Pustular psoriasis, Erythrodermic psoriasis, Spondylarthritis, Ankylosing spondylitis, Rheumatoid arthritis |
| 800014900 | Belimumab | 356547-88-1 | Marketed | Systemic lupus erythematosus, Antineutrophil cytoplasmic antibody-associated vasculitis, Lupus nephritis, Myositis, Myasthenia gravis, Sjogren's syndrome, Systemic scleroderma, Renal transplant rejection, Membranous glomerulonephritis, Waldenstrom's macroglobulinaemia, Rheumatoid arthritis |
| 800036998 | 122 0551 | | Phase III | Plaque psoriasis |
| 800023920 | Secukinumab | 1229022-83-6 | Marketed | Plaque psoriasis, Psoriatic arthritis, Ankylosing spondylitis, Pustular psoriasis, Rheumatoid arthritis, Psoriasis, Atopic dermatitis, Alopecia areata, Uveitis, Asthma, Multiple sclerosis, Dry eyes, Polymyalgia rheumatica, Type 1 diabetes mellitus, Crohn's disease |
| 800019919 | Apremilast | 608141-41-9 | Marketed | Psoriatic arthritis, Plaque psoriasis, Behcet's syndrome, Ankylosing spondylitis, Atopic dermatitis, Ulcerative colitis, Crohn's disease, Rheumatoid arthritis, Asthma, Cancer |
| 800037410 | ABT 494 | | Phase III | Rheumatoid arthritis, Crohn's disease, Ulcerative colitis, Atopic dermatitis |

(continued)

| Drug ID | Drug Name | CAS Number | Highest Dev. Phase | Indication |
|---|---|---|---|---|
| 800002909 | Daclizumab | 152923-56-3 | Marketed | Renal transplant rejection, Multiple sclerosis, Graft-versus-host disease, Asthma, Type 1 diabetes mellitus, Immune-mediated uveitis, Liver transplant rejection, Ulcerative colitis, Psoriasis, Tropical spastic paraparesis, Haematological malignancies |
| 800035644 | Infliximab biosimilar - Celltrion | | Marketed | Rheumatoid arthritis, Ulcerative colitis, Plaque psoriasis, Crohn's disease, Ankylosing spondylitis, Psoriatic arthritis |
| 800035561 | Adalimumab biosimilar - Boehringer Ingelheim | 331731-18-1 | Phase III | Rheumatoid arthritis, Plaque psoriasis, Crohn's disease |
| 800013731 | Immune globulin - CSL Behring | 9007-83-4 | Marketed | Mucocutaneous lymph node syndrome, Immune thrombocytopenic purpura, Immunodeficiency disorders, Guillain-Barre syndrome, Haemolytic disease of newborn, Rabies, Hepatitis A, Varicella zoster virus infections, Chronic inflammatory demyelinating polyradiculoneuropathy, Tetanus, Hepatitis B, Encephalitis, Renal transplant rejection, Skin and soft tissue infections, Motor neuron disease, Systemic lupus erythematosus |
| 800032143 | Desoximetason e topical - Taro Pharmaceutical s | 382-67-2 | Marketed | Plaque psoriasis, Atopic dermatitis |
| 800029381 | Siponimod | 1220909-40-9 | Phase III | Multiple sclerosis, Polymyositis, Dermatomyositis, Renal failure, Liver failure |
| 800010359 | Tocilizumab | 375823-41-9 | Marketed | Rheumatoid arthritis, Juvenile rheumatoid arthritis, Giant lymph node hyperplasia, Giant cell arteritis, Systemic scleroderma, Vasculitis, Polymyalgia rheumatica, Polymyositis, Amyotrophic lateral sclerosis, Dermatomyositis, Chronic lymphocytic leukaemia, Ankylosing spondylitis, Multiple myeloma, Crohn's disease, Pancreatic cancer, Systemic lupus erythematosus |
| 800018021 | Ofatumumab | 679818-59-8 | Marketed | Chronic lymphocytic leukaemia, Follicular lymphoma, Multiple sclerosis, Diffuse large B cell lymphoma, MALT lymphoma, Neuromyelitis optica, Pemphigus vulgaris, Rheumatoid arthritis, Waldenstrom's macro globulinaemia |
| 800010315 | Mepolizumab | 196078-29-2 | Marketed | Asthma, Chronic obstructive pulmonary disease, Churg-Strauss syndrome, Hypereosinophilic syndrome, Nasal polyps, Eosinophilic oesophagitis |

(continued)

| Drug ID | Drug Name | CAS Number | Highest Dev. Phase | Indication |
|---|---|---|---|---|
| 800035998 | Risankizumab | 1612838-76-2 | Phase III | Plaque psoriasis, Crohn's disease, Ankylosing spondylitis, Asthma, Psoriatic arthritis, Psoriasis |
| 800019706 | Dimethyl fumarate | 624-49-7 | Marketed | Multiple sclerosis, Rheumatoid arthritis, Psoriasis |
| 800008414 | Adalimumab | 331731-18-1 | Marketed | Juvenile rheumatoid arthritis, Ulcerative colitis, Plaque psoriasis, Ankylosing spondylitis, Crohn's disease, Hidradenitis suppurativa, Psoriatic arthritis, Spondylarthritis, Behcet's syndrome, Rheumatoid arthritis, Uveitis, Pustular psoriasis, Unspecified, Interstitial cystitis |
| 800017051 | Calcipotriol/bet amethasone dipropionate | | Marketed | Plaque psoriasis, Psoriasis |
| 800030194 | Tildrakizumab | 1326244-10-3 | Phase III | Plaque psoriasis, Autoimmune disorders |
| 800020727 | Golimumab | 476181-74-5 | Marketed | Psoriatic arthritis, Rheumatoid arthritis, Ankylosing spondylitis, Ulcerative colitis, Juvenile rheumatoid arthritis, Hearing disorders, Type 1 diabetes mellitus, Sarcoidosis, Asthma, Uveitis, Cardiovascular disorders |
| 800028075 | Brodalumab | 1174395-19-7 | Marketed | Psoriatic arthritis, Erythrodermic psoriasis, Pustular psoriasis, Plaque psoriasis, Asthma, Crohn's disease, Rheumatoid arthritis, Psoriasis |
| 800010395 | Certolizumab pegol | 428863-50-7 | Marketed | Rheumatoid arthritis, Ankylosing spondylitis, Crohn's disease, Psoriatic arthritis, Spondylitis, Plaque psoriasis, Juvenile rheumatoid arthritis, Interstitial cystitis, Cognition disorders |
| 800027760 | Forigerimod | 497156-60-2 | Phase III | Systemic lupus erythematosus |
| 800029638 | Masitinib | 790299-79-5 | Prere gistratio n | Amyotrophic lateral sclerosis, Mastocytosis, Prostate cancer, Alzheimer's disease, Colorectal cancer, Malignant melanoma, Pancreatic cancer, Gastrointestinal stromal tumours, Multiple myeloma, Asthma, Peripheral T-cell lymphoma, Multiple sclerosis, Crohn's disease, Ovarian cancer, Progressive supranuclear palsy, Breast cancer, Chronic obstructive pulmonary disease, Non-small cell lung cancer, Mood disorders, Head and neck cancer, Glioblastoma, Hepatocellular carcinoma, Gastric cancer, Oesophageal cancer, Stroke, Psoriasis, Rheumatoid arthritis |

(continued)

| Drug ID | Drug Name | CAS Number | Highest Dev. Phase | Indication |
|---|---|---|---|---|
| 800020410 | Canakinumab | 914613-48-2 | Marketed | Cryopyrin-associated periodic syndromes, Familial Mediterranean fever, Juvenile rheumatoid arthritis, Gouty arthritis, Peroxisomal disorders, Familial autosomal dominant periodic fever, Cardiovascular disorders, Behcet's syndrome, Peripheral arterial occlusive disorders, Mucocutaneous lymph node syndrome, Abdominal aortic aneurysm, Pulmonary sarcoidosis, Atherosclerosis, Osteoarthritis, Diabetic retinopathy, Chronic obstructive pulmonary disease, Type 2 diabetes mellitus, Rheumatoid arthritis, Type 1 diabetes mellitus, Polymyalgia rheumatica, Asthma |
| 800032685 | Filgotinib | 1206161-97-8 | Phase III | Rheumatoid arthritis, Crohn's disease, Ulcerative colitis |
| 800036014 | Etanercept biosimilar - Coherus Biosciences | 185243-69-0 | Phase III | Plaque psoriasis, Rheumatoid arthritis |
| 800001292 | Cladribine | 4291-63-8 | Marketed | Lymphoma, Leukaemia, Chronic lymphocytic leukaemia, Hairy cell leukaemia, Multiple sclerosis, Psoriasis, Transplant rejection |
| 800038738 | Adalimumab biosimilar - Amgen | 331731-18-1 | Registered | Ankylosing spondylitis, Psoriatic arthritis, Ulcerative colitis, Juvenile rheumatoid arthritis, Rheumatoid arthritis, Crohn's disease, Plaque psoriasis |
| 800018418 | U stekinumab | 815610-63-0 | Marketed | Plaque psoriasis, Psoriatic arthritis, Crohn's disease, Spondylarthritis, Ulcerative colitis, Systemic lupus erythematosus, Atopic dermatitis, Inflammation, Palmoplantar pustulosis, Sarcoidosis, Rheumatoid arthritis, Primary biliary cirrhosis, Multiple sclerosis |
| 800024855 | Ponesimod | 854107-55-4 | Phase III | Multiple sclerosis, Graft-versus-host disease, Immunological disorders, Plaque psoriasis |
| 800039480 | Adalimumab biosimilar - Sandoz | | Phase III | Plaque psoriasis, Rheumatoid arthritis |
| 800017661 | Teriflunomide | 108605-62-5 | Marketed | Multiple sclerosis |
| 800038193 | Infliximab biosimilar - Pfizer | | Phase III | Rheumatoid arthritis |
| 800011618 | Laquinimod | 248281-84-7 | Prere gistratio n | Multiple sclerosis, Huntington's disease, Crohn's disease, Lupus nephritis, Systemic lupus erythematosus |

(continued)

| Drug ID | Drug Name | CAS Number | Highest Dev. Phase | Indication |
|---|---|---|---|---|
| 800004155 | Infliximab | 170277-31-3 | Marketed | Crohn's disease, Rheumatoid arthritis, Psoriasis, Ulcerative colitis, Psoriatic arthritis, Ankylosing spondylitis, Plaque psoriasis, Behcet's syndrome, Mucocutaneous lymph node syndrome, Hepatitis C, Pyoderma, Berylliosis |
| 800018131 | Baricitinib | 1187594-09-7 | Prere gistratio n | Rheumatoid arthritis, Systemic lupus erythematosus, Diabetic nephropathies, Atopic dermatitis, Psoriasis |
| 800003804 | Glatiramer acetate | 147245-92-9 | Marketed | Multiple sclerosis, Amyotrophic lateral sclerosis, Huntington's disease, Neurological disorders, Glaucoma |
| 800027190 | Amifampridine | 54-96-6 | Marketed | Lambert-Eaton myasthenic syndrome, Congenital myasthenic syndromes, Myasthenia gravis |
| 800019029 | Tofacitinib | 477600-75-2 | Marketed | Rheumatoid arthritis, Psoriatic arthritis, Juvenile rheumatoid arthritis, Ulcerative colitis, Plaque psoriasis, Atopic dermatitis, Ankylosing spondylitis, Crohn's disease, Dry eyes, Renal transplant rejection, Irritable bowel syndrome, Asthma |
| 800038107 | Etanercept biosimilar -Sandoz | | Registered | Plaque psoriasis, Ankylosing spondylitis, Psoriatic arthritis, Rheumatoid arthritis, Juvenile rheumatoid arthritis |
| 800043035 | Ulobetasol lotion - Valeant Pharmaceutical s | | Phase III | Plaque psoriasis |
| 800037371 | Rituximab biosimilar - Boehringer Ingelheim | 174722-31-7 | Phase III | Rheumatoid arthritis, Follicular lymphoma |
| 800040562 | DFD 06 | | Phase III | Plaque psoriasis |
| 800003273 | Etanercept | 185243-69-0 | Marketed | Juvenile rheumatoid arthritis, Plaque psoriasis, Ankylosing spondylitis, Psoriatic arthritis, Rheumatoid arthritis, Graft-versus-host disease, Discoid lupus erythematosus, Metabolic syndrome, Heart failure, Wegener's granulomatosis, Pulmonary fibrosis, Transplant rejection, Asthma, Adult-onset Still's disease, Myasthenia gravis, Behcet's syndrome, Cachexia, Septic shock |
| 800041067 | Adalimumab biosimilar - Coherus BioSciences | 331731-18-1 | Phase III | Plaque psoriasis |

(continued)

| Drug ID | Drug Name | CAS Number | Highest Dev. Phase | Indication |
|---|---|---|---|---|
| 800042069 | Adalimumab biosimilar - Momenta Pharmaceutical s | | Phase III | Plaque psoriasis, Rheumatoid arthritis, Inflammation, Autoimmune disorders |
| 800043884 | Bee venom - Apimeds | | Marketed | Osteoarthritis, Multiple sclerosis |
| 800035854 | Adalimumab biosimilar - Fujifilm Kyowa Kirin Biologics | 331731-18-1 | Phase III | Rheumatoid arthritis |
| 800021494 | Anifrolumab | 1326232-46-5 | Phase III | Systemic lupus erythematosus, Scleroderma |
| 800033985 | Tazarotene/ulo betasol | | Phase III | Plaque psoriasis |
| 800029302 | Olokizumab | 1007223-17-7 | Phase III | Rheumatoid arthritis |
| 800002472 | Anakinra | 143090-92-0 | Marketed | Rheumatoid arthritis, Cryopyrin-associated periodic syndromes, Gout, Juvenile rheumatoid arthritis, Septic shock, Ankylosing spondylitis, Osteoarthritis, Graft-versus-host disease, Pneumococcal infections |
| 800031049 | Calcipotriol - Stiefel | 112965-21-6 | Marketed | Plaque psoriasis, Psoriasis |
| 800006904 | Fampridine sustained - release | 504-24-5 | Marketed | Multiple sclerosis, Neurological disorders, Stroke, Spinocerebellar degeneration, Spinal cord injuries, Parkinson’s disease, Cerebral palsy |
| 800018689 | Clobetasol propionate topical -Galderma | 25122-41-2 | Marketed | Atopic dermatitis, Psoriasis, Skin disorders |
| 800023488 | Prednisone controlled-release - Horizon Pharma/ Vectura | 53-03-2 | Marketed | Asthma, Rheumatoid arthritis, Chronic obstructive pulmonary disease, Psoriatic arthritis, Ankylosing spondylitis, Polymyalgia rheumatica, Nocturnal asthma |
| 800007752 | Ciclosporin - Novartis | 59865-13-3 | Marketed | Psoriasis, Liver transplant rejection, Transplant rejection, Pancreas transplant rejection, Atopic dermatitis, Rheumatoid arthritis, Heart transplant rejection, Myasthenia gravis, Renal transplant rejection, Ulcerative colitis |
| 800006793 | Natalizumab | 189261-10-7 | Marketed | Multiple sclerosis, Crohn’s disease, Stroke, Graft-versus-host disease, Rheumatoid arthritis, Multiple myeloma |
| 800002523 | Alemtuzumab | 216503-57-0 | Marketed | Multiple sclerosis, Chronic lymphocytic leukaemia, T cell prolymphocytic leukaemia, Graft-versus-host disease, Rheumatoid arthritis |

(continued)

| Drug ID | Drug Name | CAS Number | Highest Dev. Phase | Indication |
|---|---|---|---|---|
| 800016270 | Atacicept | | Phase II/III | Systemic lupus erythematosus, Rheumatoid arthritis, Multiple sclerosis, Lupus nephritis, Chronic lymphocytic leukaemia, Non-Hodgkin's lymphoma, Multiple myeloma |
| 800038364 | Adalimumab biosimilar - Pfizer | 331731-18-1 | Phase III | Rheumatoid arthritis |
| 800038469 | Infliximab biosimilar - Merck & Co/Samsung Bioepis | 170277-31-3 | Registered | Rheumatoid arthritis, Ulcerative colitis, Psoriatic arthritis, Plaque psoriasis, Crohn's disease, Ankylosing spondylitis |
| 800039191 | DFD 01 | 5593-20-4 | Marketed | Plaque psoriasis |
| 800033254 | Pefcalcitol | 381212-03-9 | Phase III | Plaque psoriasis, Palmoplantar keratoderma |
| 800015135 | Immune globulin 10% - Grifols | 9007-83-4 | Marketed | Immune thrombocytopenic purpura, Immunodeficiency disorders, Chronic inflammatory demyelinating polyradiculoneuropathy, Myasthenia gravis, Multiple sclerosis |
| 800040965 | ALKS 8700 | | Phase III | Multiple sclerosis |
| 800016064 | Peginterferon beta-la - Biogen | 1211327-92-2 | Marketed | Multiple sclerosis |
| 800040608 | Fluocinonide cream- Valeant | 356-12-7 | Marketed | Skin disorders, Plaque psoriasis |
| 800006422 | Interferon beta-1a - Biogen | 145258-61-3 | Marketed | Multiple sclerosis, Hepatitis B, Human papillomavirus infections, Hepatitis C, Ulcerative colitis, Glioma, Chronic inflammatory demyelinating polyradiculoneuropathy, Pulmonary fibrosis |
| 800000782 | Interferon beta-1b - Bayer HealthCare Pharmaceuticals/ Novartis | 145155-23-3 | Marketed | Multiple sclerosis, Prostate cancer, Cardiomyopathies, HIV infections, Rhinovirus infections |
| 800001086 | Meloxicam | 71125-38-7 | Marketed | Osteoarthritis, Periarthritis, Rheumatoid arthritis, Neuropathic pain, Gout, Ankylosing spondylitis, Back pain, Juvenile rheumatoid arthritis, Preterm labour |
| 800003883 | Alefacept | 222535-22-0 | Marketed | Psoriasis, Transplant rejection, Psoriatic arthritis |

(continued)

| Drug ID | Drug Name | CAS Number | Highest Dev. Phase | Indication |
|---|---|---|---|---|
| 800006795 | Celecoxib | 169590-42-5 | Marketed | Dysmenorrhoea, Acute pain, Tenosynovitis, Familial adenomatous polyposis, Back pain, Ankylosing spondylitis, Tendinitis, Dental pain, Rheumatoid arthritis, Postoperative pain, Osteoarthritis, Pain, Rheumatic disorders, Juvenile rheumatoid arthritis, Cervicobrachial syndrome, Periarthritis, Non-small cell lung cancer, Stomatitis, Gouty arthritis, Bladder cancer, Alzheimer’s disease, Prostate cancer |
| 800024954 | Esomeprazole/ naproxen | | Marketed | Osteoarthritis, Rheumatoid arthritis, Ankylosing spondylitis |
| 800002515 | Tazarotene topical | 118292-40-3 | Marketed | Acne vulgaris, Psoriasis, Photodamage |
| 800004239 | Calcipotriol | 112965-21-6 | Marketed | Psoriasis |
| 800013806 | Epratuzumab | 205923-57-5 | Phase III | Systemic lupus erythematosus, Acute lymphoblastic leukaemia, Non-Hodgkin’s lymphoma, Cachexia |
| 800007022 | Interferon beta-1a - Merck Serono | 145258-61-3 | Marketed | Multiple sclerosis, Hepatitis C, Human papillomavirus infections, Non-small cell lung cancer, Ulcerative colitis, Crohn’s disease, Rheumatoid arthritis |
| 800045068 | Ulobetasol lotion - Sun Pharmaceutical Industries | 66852-54-8 | Registered | Plaque psoriasis |
| 800031664 | Immune globulin 10% - Octapharma | 308067-58-5 | Marketed | Immunodeficiency disorders, Immune thrombocytopenic purpura, Chronic inflammatory demyelinating polyradiculoneuropathy, Alzheimer’s disease |
| 800034238 | Methotrexate subcutaneous auto-injection - Antares Pharma | 59-05-2 | Marketed | Psoriasis, Rheumatoid arthritis, Juvenile rheumatoid arthritis |
| 800044876 | VAL BRO 03 | | Phase III | Psoriatic arthritis |
| 800004586 | Acitretin | 55079-83-9 | Marketed | Psoriasis, Dermatitis, Cancer |
| 800044389 | Juvenile rheumatoid arhtritis therapeutic - Marathon Pharmaceuticals | | Phase III | Juvenile rheumatoid arthritis |
| 800006246 | Rheumatoid arthritis vaccine (IR 501) - Immune Response BioPharma | | Phase III | Rheumatoid arthritis |

(continued)

| Drug ID | Drug Name | CAS Number | Highest Dev. Phase | Indication |
|---|---|---|---|---|
| 800025490 | Ibuprofen/ famotidine | 1011231-26-7 | Marketed | Musculoskeletal pain, Osteoarthritis, Rheumatoid arthritis, NSAID-induced ulcer, Ankylosing spondylitis |
| 800039732 | Methotrexate subcutaneous auto-injection - Medac Pharma | 59-05-2 | Marketed | Juvenile rheumatoid arthritis, Rheumatoid arthritis, Psoriasis |
| 800009362 | Calcitriol - Galderma | 32222-06-3 | Marketed | Plaque psoriasis |
| 800014212 | Mometasone/sa licylic acid | | Marketed | Psoriasis, Skin disorders |
| 800022272 | Clobetasol propionate foam (Olux-E) - Stiefel Laboratories | 25122-46-7 | Marketed | Atopic dermatitis, Psoriasis |
| 800012485 | Clobetasol propionate foam (Olux) - Stiefel Laboratories | 25122-46-7 | Marketed | Skin disorders, Psoriasis |
| 800009052 | Mitoxantrone | 65271-80-9 | Marketed | Breast cancer, Acute nonlymphocytic leukaemia, Cancer, Acute promyelocytic leukaemia, Cancer pain, Acute myeloid leukaemia, Ovarian cancer, Leukaemia, Liver cancer, Multiple sclerosis, Non-Hodgkin's lymphoma |
| 800012483 | Betamethasone valerate foam - Stiefel Laboratories | 2152-44-5 | Marketed | Atopic dermatitis, Psoriasis, Seborrhoeic dermatitis, Skin disorders |
| 800012233 | Mahonia aquifolium extract | | Marketed | Psoriasis |

In some embodiments, the present invention provides a method of modulating an immune response, comprising administering to a patient in need thereof an effective amount of a therapeutic-loaded exosome. In some embodiments, the patient is suffering from a hyperproliferative disease, disorder, or condition such as cancer. In some embodiments, the patient is suffering from an autoimmune disease, disorder, or condition. In some embodiments, the therapeutic agent's target *in vivo* is one of those listed in Table 5, below. In some embodiments, the therapeutic-loaded exosome is administered in combination with a compound listed in Table 5, or a pharmaceutically acceptable salt thereof. In some embodiments, the therapeutic agent loaded in the exosome and the coadministered compound of Table 5 modulate a target in Table 5.

## Table 5: Immuno-oncology Targets

| Target | Location | Function | Compound (MOA) | Company or institution | Model or indication | Status[†] |
|---|---|---|---|---|---|---|
| *Amino acid catabolism* | | | | | | |
| IDO | Macrophages, DCs, upregulated in tumours | Depletion of tryptophan and metabolites promote $T_{Reg}$ cell differentiation, suppression of immune response and decreased DC function | INCB24360 (inhibitor) | Incyte | Murine syngeneic tumour (PAN02) | Phase II |
| | | | 1-Methyl tryptophan (inhibitor) | NewLink Genetics | Murine syngeneic tumour model (Lewis lung cancer) | Phase I |
| | | | NLG919 (inhibitor) | NewLink Genetics | Murine syngeneic tumour (Pan02) | Phase I |
| TDO | Hepatocytes | Depletion of tryptophan and metabolites promote $T_{Reg}$ cell differentiation, suppression of immune response and decreased DC function | LM10 (inhibitor) | Ludwig Institute for Cancer Research | Murine syngeneic tumour (P815B/TDO) | Research |
| ARG1, ARG2 | MDSCs, TAMs, vascular endothelium | Depletion of the CD3ζ chain of the TCR suppresses T cell responses to antigen | Compound 9 (inhibitor) | The Institutes for Pharmaceutical Discovery | Reperfusion injury from myocardial ischaemia | Research |
| iNOS, ARG1, ARG2 | MDSCs | Supports generation of ROS that modify CCL2 levels, disabling T cell chemotaxis | NCX-4016 (dual inhibitor) | NicOx | Preventing colorectal carcinoma | Phase II, discontinued |
| | | | AT38 (dual inhibitor) | Istituti di Ricovero e Cura a Carattere Scientifico (IRCCS) | MCA-203 fibrosarcoma-bearing mice | Research |
| PDE5 | MDSCs | Decreases functional IL-13 receptors | Tadalafil (inhibitor) | Eli Lilly and Company | Investigational for immuno-oncology | Approved for erectile dysfunction and hypertension |

| | | | | | | |
|---|---|---|---|---|---|---|
| *Signalling of tumour-derived extracellular ATP* | | | | | | |
| P2X7 | Broadly expressed on lymphocytes, often upregulated in tumours | Induction of IL-1β release in DCs, enhances tumour-specific CD8 T cell cytotoxicity | ATP (agonist) | Istituti di Ricovero e Cura a Carattere Scientifico (IRCCS) | Immuno-stimulant | Research |
| | Broadly expressed on lymphocytes, often upregulated in tumours | Increases CCL2, ROS, ARG1 and TGFβ levels; activates MDSCs, tumour growth and angiogenesis | AZ10606120 (antagonist) | University of Ferrara, Italy | Murine B16 F10 melanoma | Research |
| $P2Y_{11}$ | ATP derived from tumour binds receptor on DCs | Inhibits synthesis of IL-1, TNFα, IL-6; increases secretion of TSP1, IL-10 and IDO1, resulting in DC semi-maturation | NF340 (antagonist) | University of Duesseldorf, Germany | Immuno-stimulant | Research |
| *Adenosine signalling* | | | | | | |
| $A_{2A}$ receptor | $T_{Reg}$ cells, DCs, NK cells, NK T cells, tumours | Elevated cAMP blunts TCR-mediated cytotoxicity; inhibits effector T cells; expands $T_{Reg}$ cells; enhances NK cell cytotoxicity | SCH58261 (antagonist) | Peter MacCallum Cancer Centre, Victoria, Australia | B16 melanoma metastasis | Research |
| | $T_{Reg}$ cells, DCs, NK cells, NK T cells, tumours | Elevated cAMP blunts TCR-mediated cytotoxicity; inhibits effector T cells; expands $T_{Reg}$ cells; enhances NK cell cytotoxicity | SCH420814 (antagonist) | Merck | Parkinson disease | Phase III, discontinued |

| Target | Location | Function | Compound (MOA) | Company or institution | Model or indication | Status‡ |
|---|---|---|---|---|---|---|
| $A_{2B}$ receptor | Myeloid cells, expression driven by HIF1α | Elevated cAMP increases IL-10 and CCL2 levels; expansion of MDSCs and TAMs | PSB1115 (antagonist) | University of Salerno, Italy | Murine B16 F10 melanoma | Research |
| *Adenosine production* | | | | | | |
| CD39 | $T_{Reg}$ cells, B cells, MDSCs, NK cells, tumours, endothelium | Contributes to the production of adenosine, which binds to $A_1$, $A_{2A}$, $A_{2B}$ and $A_3$ receptors | ARL 67176 (inhibitor) | OREGA Biotech | Murine B16 F10 melanoma | Research |
| CD73 | $T_{Reg}$ cells, B cells, MDSCs, NK cells, tumours, endothelium | Contributes to the production of adenosine, which binds to $A_1$, $A_{2A}$, $A_{2B}$ and $A_3$ receptors | AMPCP (inhibitor) | Cancer Therapy and Research Center, University of Texas San Antonio, USA | Murine B16 F10 melanoma | Research |
| *Elevation of cyclic AMP* | | | | | | |
| COX2 | MDSCs, TAMs, $T_{Reg}$ cells, tumours | Generates $PGE_2$, which is immunosuppressive (via $EP_2$ and $EP_4$ receptors) | Celecoxib (inhibitor) | Pfizer | Rheumatoid arthritis, osteoarthritis, pain | Approved |
| $EP_2$ receptor | MDSCs, NK cells, $T_{Reg}$ cells, tumours | $T_{Reg}$ cell activation; tumour proliferation and angiogenesis | PF-04418948 (antagonist) | Pfizer | None indicated | Phase I, discontinued |
| $EP_4$ receptor | MDSCs, NK cells, $T_{Reg}$ cells, tumours | Activates suppressor cell function of MDSCs and TAMs | RQ-15986 (antagonist) | RaQualia Pharma | Murine mammary 66.1 tumour metastasis | Preclinical |
| *Chemokines and chemokine receptors* | | | | | | |
| CXCR1, CXCR2 | PMNCs, monocytes, endothelium, mast cells | Migration of CXCR2 expressing MDSCs into the TME; directs effects on tumour proliferation | CXCR2-specific mAb§ (antagonist) | Pediatric Oncology Branch, National Cancer Institute, National Institutes of Health, USA | Murine rhabdomyosarcoma | Research |
| CXCR4 | T cells, B cells, monocytes, PMNCs, immature DCs, tumours | Ligand expression in stroma mediates metastasis by tumour-specific and T cell-based mechanisms | Plerixafor (also known as AMD3100) (antagonist) | Sanofi-Aventis, Cancer Research UK | Pancreactic ductal adenocarcinoma | Approved for stem cell mobilization |

| | | | | | | |
|---|---|---|---|---|---|---|
| CCR2 | Monocytes, PMNCs, immature DCs, T cells, NK cells | Drives TAM and monocytic MDSC infiltration into the TME | PF-4136309 (antagonist) | Pfizer, Washington University School of Medicine, National Cancer Institute, USA | Murine pancreatic model supportive of clinical study | Phase IB |
| CCR5 | $T_H1$ cells, $CD8^+$ T cells, monocytes, macrophages | $T_{Reg}$ cell infiltration and infiltration of precursors to generate TAMs and MDSCs | Maraviroc (antagonist) | National Center for Tumour Diseases, Germany | Blockade of metastatic colorectal cancer | Phase I |
| *Recognition of foreign organisms to activate the immune response* | | | | | | |
| TLR4 | Monocytes, macrophages, DCs | Bacterial host defence; activation results in cytokine burst (IL-1, TNFα and type I IFNs) | OM-174 (agonist) | Centre Hospitalier Universitaire, France | Rat colon cancer, solid tumours | Phase I |
| TLR7, TLR8 | DCs, plasmacytoid DCs, macrophages | Binds to viral ssRNA and bacterial DNA; induces secretion of inflammatory cytokines and type I IFN, which promotes a $T_H1$-directed activation of DCs and NK cells to directly kill tumour cells and suppress $T_{Reg}$ cells | Imiquimod (agonist) | Graceway Pharmaceuticals | Basal cell carcinoma | Approved |

| Target | Location | Function | Compound (MOA) | Company or institution | Model or indication | Status‡ |
|---|---|---|---|---|---|---|
| TLR7 | DCs, plasmacytoid DCs, macrophages | Host defence recognizing viral ssRNA and bacterial DNA; inflammatory cytokines and type I IFN secretion promoting a $T_H1$-directed activation of DCs and NK cells to directly kill tumour cells and suppress $T_{Reg}$ cells | 852A (agonist) | Pfizer | Solid and haematological malignancies | Phase I/II |
| TLR8 | DCs, plasmacytoid DCs, macrophages | Host defence recognizing viral ssRNA and bacterial DNA; inflammatory cytokines and type I IFN secretion promoting a $T_H1$-directed activation of DCs and NK cells to directly kill tumour cells and suppress $T_{Reg}$ cells | VTX-2337 (agonist) | VentiRx Pharmaceuticals | Solid and haematological malignancies | Phase I/II |
| TLR9 | DCs, plasmacytoid DCs, macrophages | Host defence recognizing viral ssRNA and bacterial DNA; inflammatory cytokines and type I IFN secretion promoting a $T_H1$-directed activation of DCs and NK cells to directly kill tumour cells and suppress $T_{Reg}$ cells | IMO-2055 (agonist) | Hybridon, Idera Pharmaceuticals | Advanced solid malignancies | Phase I/II |

| | | | | | | |
|---|---|---|---|---|---|---|
| ***Signal transduction: kinase inhibitors*** | | | | | | |
| ALK5 | Downstream of TGFβ, which is often overexpressed by tumours | Attenuation of TGFβ signalling causes activation of $CD8^+$ cells, generation of CTLs, and stimulation of NK cells | LY2157299 | Eli Lilly and Company | Murine B16 F10 melanoma | Phase I/II |
| | | | EW-7197 | Ewha Womens University, Seoul, Korea | Murine B16 F10 melanoma | Phase I |
| BRAF$^{V600E}$ | Tumours | V600E-driven IL-1 expression promotes immunosuppressive TAF and MDSC function | Vemurafenib<br>Dabrafenib | Plexxikon, Genentech, GlaxoSmithKline, MD Anderson Cancer Center, USA | Patients with melanoma | Approved for metastatic melanoma |
| RON | Expressed on myeloid cells. Tumours secrete its ligand MSP | Decreases IL-12, IFNγ and TNF, and increases IL-10; favours M2 phenotype | BMS-777607 | Bristol-Myers Squibb, Huntsman Cancer Institute, Utah, USA | Inhibits metathesis in MMTV-PyMT transgenic mice | Phase I/II |
| CSF1 | Glioma cells and TAMs express CSF ligand | M1 to M2 polarization, which promotes tumour growth and survival | BLZ945 | Memorial Sloan-Kettering Cancer Center, New York, USA | Murine glioblastoma | Research |
| PI3Kδ | B cells, T cells, myeloid lineage cells | Inhibition preferentially suppresses $T_{Reg}$ cell function, resulting in effector T cell activation | PI-3065 | Piramed Pharma, University College London Cancer Institute, UK | 4T1 breast cancer and other solid tumours | Research |
| PI3Kγ | Haematopoietic cells, primarily myeloid lineage | Required for α4β1-dependent myeloid cell infiltration into tumours | TG100-115 | University of California San Diego, Moores Cancer Center, USA | Lewis lung carcinoma and PyMT spontaneous breast carcinomas | Research |

**Abbreviations used in Table 5:** AMPCP, adenosine 5'-(α,β methylene)diphosphate; ARG, arginase; COX2, cyclooxygenase 2; CSF, colony stimulating factor; CTL, cytotoxic T lymphocyte; DC, dendritic cell; HIF1α, hypoxia-inducible factor 1α; IDO, indoleamine 2,3-dioxygenase; IFN, interferon; IL, interleukin; iNOS, inducible nitric oxide synthase; MDSC, myeloid-derived suppressor cell; MOA, mechanism of action; MSP, macrophage-stimulating protein; NK, natural killer; PDE5, phosphodiesterase type 5; $PGE_2$, prostaglandin E2; PMNC, peripheral mononuclear cell; ROS, reactive oxygen species; TAF, tumour-associated fibroblasts; TAM, tumour-associated macrophage; TCR, T cell receptor; TDO, tryptophan 2,3-dioxygenase; $T_H$, T helper; $TGF_β$, transforming growth factor-β; TLR, Toll-like receptor; TME, tumor microenvironment; TNF, tumour necrosis factor; $T_{Reg}$, regulatory T; TSP1, thrombospondin 1. *Listed are small-molecule drug targets that have been proposed for cancer immunotherapy. ‡For some examples, the clinical development status provided is for a non-immuno-oncology indication. In these cases the literature supports clinical consideration in light of its impact on innate immune function. §While the scientific literature illustrates CXCR2 antagonism using a mAb, several small-molecule CXCR1 and CXCR2 antagonists have reached clinical trials and in principle could show similar efficacy.

*Non-Coding RNA Therapeutic Agents*

**ncRNA and IncRNA**

The broad application of next-generation sequencing technologies in conjunction with improved bioinformatics has helped to illuminate the complexity of the transcriptome, both in terms of quantity and variety. In humans, 70-90% of the genome is transcribed, but only ~2% actually codes for proteins. Hence, the body produces a huge class of non-translated transcripts, called long non-coding RNAs (IncRNAs), which have received much attention in the past decade.

Recent studies have illuminated the fact that lncRNAs are involved in a plethora of cellular signaling pathways and actively regulate gene expression via a broad selection of molecular mechanisms.

Human and other mammalian genomes pervasively transcribe tens of thousands of long non-coding RNAs (lncRNAs). The latest edition of data produced by the public research consortium GenCode (version #27) catalogs just under 16,000 lncRNAs in the human genome, producing nearly 28,000 transcripts; when other databases are included, more than 40,000 lncRNAs are known.

These mRNA-like transcripts have been found to play a controlling role at nearly all levels of gene regulation, and in biological processes like embryonic development. A growing body of evidence also suggests that aberrantly expressed lncRNAs play important roles in normal physiological processes as well as multiple disease states, including cancer. lncRNAs are a group that is commonly defined as transcripts of more than 200 nucleotides (e.g. about 200 to about 1200 nt, about 2500 nt, or more) that lack an extended open reading frame (ORF). The term "non-coding RNA" (ncRNA) includes lncRNA as well as shorter transcripts of, e.g., less than about 200 nt, such as about 30 to 200 nt. Several lncRNAs, e.g. gadd74 and lncRNA-RoR5, modulate cell cycle regulators such as cyclins, cyclin-dependent kinases (CDKs), CDK inhibitors and p53 and thus provide an additional layer of flexibility and robustness to cell cycle progression. In addition, some lncRNAs are linked to mitotic processes such as centromeric satellite RNA, which is essential for kinetochore formation and thus crucial for chromosome segregation during mitosis in humans and flies. Another nuclear lncRNA, MA-lincl, regulates M phase exit by functioning in *cis* to repress the expression of its neighbouring gene Pur$\alpha$, a regulator of cell proliferation. Since deregulation of the cell cycle is closely associated with cancer development and growth, cell cycle regulatory lncRNAs may have oncogenic properties.

Thus, in some embodiments, delivery of a ncRNA, such as to a specific tissue or organ of interest, corrects aberrant RNA expression levels or modulates levels of disease-causing lncRNA. Accordingly, in some embodiments, the present invention provides a therapeutic-loaded exosome, wherein the therapeutic is a non-coding RNA (ncRNA). In some embodiments, the ncRNA is a long non-coding RNA (lncRNA) of about 200 nucleotides (nt) in length or greater. In some embodiments, the therapeutic is a ncRNA of about 25 nt or about 30 nt to about 200 nt in length. In some embodiments, the lncRNA is about 200 nt to about 1,200 nt in length. In some embodiments, the lncRNA is about 200 nt to about 1,100, about 1,000, about 900, about 800, about 700, about 600, about 500, about 400, or about 300 nt in length.

**Micro RNA (miRNA)**

In some embodiments, the thereapeutic is a miRNA. As would be recognized by those skilled in the art, miRNAs are small non-coding RNAs that are about 17 to about 25 nucleotide bases (nt) in length in their biologically active form. In some embodiments, the miRNA is about 17 to about 25, about 17 to about 24, about 17 to about 23, about 17 to about 22, about 17 to about 21, about 17 to about 20, about 17 to about 19, about 18 to about 25, about 18 to about 24, about 18 to about 23, about 18 to about 22, about 18 to about 21, about 18 to about 20, about 19 to about 25, about 19 to about 24, about 19 to about 23, about 19 to about 22, about 19 to about 21, about 20 to about 25, about 20 to about 24, about 20 to about 23, about 20 to about 22, about 21 to about 25, about 21 to about 24, about 21 to about 23, about 22 to about 25, about 22 to about 24, or about 22 nt in length. miRNAs regulate gene expression post-transcriptionally by decreasing target mRNA translation. It is thought that miRNAs function as negative regulators. There are generally three forms of miRNAs: primary miRNAs (pri-miRNAs), premature miRNAs (pre-miRNAs), and mature miRNAs. Primary miRNAs are expressed as stem-loop structured transcripts of about a few hundred bases to over 1 kb. The pri-miRNA transcripts are cleaved in the nucleus by Drosha, an RNase II endonuclease, that cleaves both strands of the stem near the base of the stem loop. Drosha cleaves the RNA duplex with staggered cuts, leaving a 5' phosphate and 2 nt overhang at the 3' end. The cleaved product, the premature miRNA (pre-miRNA) is about 60 to about 110 nt long with a hairpin structure formed in a fold-back manner. Pre-miRNA is transported from the nucleus to the cytoplasm by Ran-GTP and Exportin-5. Pre-miRNAs are processed further in the cytoplasm by another RNase II endonuclease called Dicer. Dicer recognizes the 5' phosphate and 3' overhang, and cleaves the loop off at the stem-loop junction to form miRNA duplexes. The miRNA duplex binds to the RNA-induced silencing complex (RISC), where the antisense strand is preferentially degraded and the sense strand mature miRNA directs RISC to its target site. It is the mature miRNA that is the biologically active form of the miRNA and is about 17 to about 25 nt in length. In some embodiments, the miRNAs encapsulated by the microvesicles of the presently-disclosed subject matter are selected from miR-155, which is known to act as regulator of T- and B-cell maturation and the innate immune response, or miR-223, which is known as a regulator of neutrophil proliferation and activation. Other non-natural miRNAs such as iRNAs (e.g. siRNA) or natural or non-natural oligonucleotides may be present in the milk-derived exosome and represent an encapsulated therapeutic agent, as the term is used herein.

**Short Interfering RNA (siRNA)**

In some embodiments, the therapeutic is a siRNA. Small interfering RNA (siRNA), sometimes known as short

interfering RNA or silencing RNA, is a class of double-stranded RNA molecules, 20-25 base pairs in length (of similar length to miRNA). siRNAs generally exert their biological effects through the RNA interference (RNAi) pathway. siRNAs generally have 2 nucleotide overhangs that are produced through the enzymatic cleavage of longer precursor RNAs by the ribonuclease Dicer. siRNAs can limit the expression of specific genes by targeting their RNA for destruction through the RNA interference (RNAi) pathway. It interferes with the expression of specific genes with complementary nucleotide sequences by degrading mRNA after transcription, preventing translation. siRNA can also act in RNAi-related pathways as an antiviral mechanism or play a role in the shaping of the chromatin structure of a genome.

The therapeutic agent may also be selected from mRNA, antisense RNA, or other nucleic acids and analogs thereof described herein.

In one aspect, the present invention provides a therapeutic-loaded milk exosome, wherein the therapeutic is a nucleic acid and the therapeutic is not naturally-occurring in the milk from which the milk exosome is derived.

In some embodiments, the nucleic acid is an mRNA.

In some embodiments, the nucleic acid is an antisense RNA.

In some embodiments, the nucleic acid is a non-coding RNA (ncRNA) of about 30 to about 200 nucleotides (nt) in length or a long non-coding RNA (IncRNA) of about 200 to about 800 nt in length.

In some embodiments, the IncRNA is a long intergenic non-coding RNA (lincRNA), pretranscript, pre-miRNA, pre-mRNA, competing endogenous RNA (ceRNA), small nuclear RNA (snRNA), small nucleolar RNA (snoRNA), pseudogene, rRNA, or tRNA.

In some embodiments, the ncRNA is selected from a piwi-interacting RNA (piRNA), primary miRNA (pri-miRNA), or premature miRNA (pre-miRNA).

In some embodiments, the nucleic acid is a siRNA or short hairpin RNA (shRNA).

In some embodiments, the therapeutic is a nucleic acid conjugated to a hydrophobic group.

In some embodiments, the nucleic acid is selected from an mRNA, an antisense RNA, an siRNA, an shRNA, a non-coding RNA (ncRNA) of about 30 to about 200 nucleotides (nt) in length, or a long non-coding RNA (IncRNA) of about 200 to about 800 nt in length.

In some embodiments, the milk exosome is derived from cow, sheep, goat, camel, buffalo, yak, or human milk or colostrum.

## 2. ***Definitions***

While the terms used herein are believed to be well understood by one of ordinary skill in the art, definitions are set forth herein to facilitate explanation of the presently-disclosed subject matter.

Unless defined otherwise, all technical and scientific terms used herein have the same meaning as commonly understood by one of ordinary skill in the art to which the presently-disclosed subject matter belongs. Although any methods, devices, and materials similar or equivalent to those described herein can be used in the practice or testing of the presently-disclosed subject matter, representative methods, devices, and materials are now described.

The terms "a," "an," and "the" refer to "one or more" when used in this application, including the claims. Thus, for example, reference to "a cell" includes a plurality of such cells, and so forth.

Unless otherwise indicated, all numbers expressing quantities of ingredients, properties such as reaction conditions, and so forth used in the specification and claims are to be understood as being modified in all instances by the term "about." Accordingly, unless indicated to the contrary, the numerical parameters set forth in this specification and claims are approximations that can vary depending upon the properties sought to be obtained within the scope of the present invention.

As used herein, the term "about," when referring to a value or to an amount of mass, weight, time, volume, concentration or percentage is meant to encompass variations of in some embodiments $\pm$20%, in some embodiments $\pm$10%, in some embodiments $\pm$5%, in some embodiments $\pm$1%, in some embodiments $\pm$0.5%, and in some embodiments $\pm$0.1% from the specified amount, as such variations are appropriate to perform the disclosed method.

As used herein, ranges can be expressed as from "about" one particular value, or "about" one value to "about" another particular value. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if the range of "10-15" is disclosed, then 11, 12, 13, and 14 are also disclosed.

As used herein, the terms "treatment," "treat," and "treating" refer to reversing, alleviating, delaying the onset of, or inhibiting the progress of a disease or disorder, or one or more symptoms thereof, as described herein. In some embodiments, treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence

As used herein, the term "cancer" refers to all types of cancer or neoplasm or malignant tumors found in animals, including leukemias, carcinomas, melanoma, and sarcomas. By "leukemia" is meant broadly progressive, malignant diseases of the blood-forming organs and is generally characterized by a distorted proliferation and development of leukocytes and their precursors in the blood and bone marrow. Leukemia diseases include, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

The term "carcinoma" refers to a malignant new growth made up of epithelial cells tending to infiltrate the surrounding tissues and give rise to metastases. Exemplary carcinomas include, for example, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiennoid carcinoma, carcinoma epitheliale adenoides, exophytic carcinoma, carcinoma ex ulcere, carcinoma fibrosum, gelatiniform carcinoma, gelatinous carcinoma, giant cell carcinoma, glandular carcinoma, granulosa cell carcinoma, hair-matrix carcinoma, hematoid carcinoma, hepatocellular carcinoma, Hurthle cell carcinoma, hyaline carcinoma, hypemephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tuberous carcinoma, verrucous carcinoma, and carcinoma villosum.

The term "sarcoma" generally refers to a tumor which is made up of a substance like the embryonic connective tissue and is generally composed of closely packed cells embedded in a fibrillar or homogeneous substance. Sarcomas include, for example, chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilns' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

The term "melanoma" is taken to mean a tumor arising from the melanocytic system of the skin and other organs. Melanomas include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma subungal melanoma, and superficial spreading melanoma.

Additional cancers include, for example, Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, premalignant skin lesions, testicular cancer, lymphomas, thyroid cancer, neuroblastoma, esophageal cancer, genitourinary tract cancer, malignant hypercalcemia, cervical cancer, endometrial cancer, and adrenal cortical cancer. In some embodiments, the cancer is selected from the group consisting of breast cancer, uterine cancer, lung cancer, prostate cancer, ovarian cancer, cervical cancer, and pancreatic cancer.

## 3. *Uses. Formulation and Administration*

*Pharmaceutically acceptable compositions*

According to another embodiment, the present invention provides a composition comprising a therapeutic-loaded exosome of this invention and a pharmaceutically acceptable carrier, adjuvant, or vehicle. The amount of therapeutic agent encapsulated within a therapeutic-loaded exosome is an amount effective to treat the relevant disease, disorder, or condition in a patient in need thereof. In certain embodiments, a composition of this invention is formulated for administration to a patient in need of such composition. In some embodiments, a composition of this invention is formulated for oral administration to a patient.

The term "patient," as used herein, means an animal, for example a mammal, such as a human.

The term "pharmaceutically acceptable carrier, adjuvant, or vehicle" refers to a nontoxic carrier, adjuvant, or vehicle that does not destroy the pharmacological activity of the therapeutic-loaded exosome with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a nontoxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

In some embodiments, the therapeutic-loaded exosomes or pharmaceutical compositions thereof are administered by an oral, intravenous, subcutaneous, intranasal, inhalation, intramuscular, intraocular, intraperitoneal, intratracheal, transdermal, buccal, sublingual, rectal, topical, local injection, or surgical implantation route. In some embodiments, the administration route is oral.

In some embodiments, the therapeutic, diagnostic, and prognostic attributes of therapeutic-loaded exosomes are achieved via non-oral means. Achieving systemic distribution of the encapsulated therapeutic agent using milk-derived exosomes following delivery would be the major objective of this approach but it is also possible to achieve selective delivery to sites of interest through the use of targeting ligands (e.g., antibodies, peptides, aptamers, or others: *see,* e.g., Friedman, A. D. et al., Curr Pharm Des 2013; 19(35): 6315-6329).

To aid in delivery of the therapeutic-loaded exosomes, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

Pharmaceutically acceptable compositions of this invention may also be administered topically, especially when

the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, provided pharmaceutically acceptable compositions may be formulated in a suitable ointment containing the active component suspended or dissolved in one or more carriers. Carriers for topical administration of a therapeutic-loaded exosome of this invention include, but are not limited to, mineral oil, liquid petrolatum, white petrolatum, propylene glycol, polyoxyethylene, polyoxypropylene compound, emulsifying wax and water. Alternatively, provided pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, provided pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

Pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, pharmaceutically acceptable compositions of this invention are formulated for oral administration. Such formulations may be administered with or without food. In some embodiments, pharmaceutically acceptable compositions of this invention are administered without food. In other embodiments, pharmaceutically acceptable compositions of this invention are administered with food.

The amount of therapeutic-loaded exosomes of the present invention that may be combined with the carrier materials to produce a composition in a single dosage form will vary depending upon the host treated, the particular mode of administration, and other factors known to one of ordinary skill. Preferably, provided compositions should be formulated so that a dosage of between 0.01 - 100 mg/kg body weight/day of the therapeutic agent can be administered to a patient receiving these compositions.

It should also be understood that a specific dosage and treatment regimen for any particular patient will depend upon a variety of factors, including the activity of the specific therapeutic-loaded exosome employed, the age, body weight, general health, sex, diet, time of administration, rate of excretion, drug combination, and the judgment of the treating physician and the severity of the particular disease being treated. The amount of a therapeutic-loaded exosome of the present invention in the composition will also depend upon the particular therapeutic-loaded exosome in the composition.

*Uses of Therapeutic-Loaded Exosomes and Pharmaceutically Acceptable Compositions Thereof*

Pharmaceutically acceptable compositions comprising a therapeutic-loaded exosome, and a pharmaceutically acceptable excipient, diluent, or carrier, are useful for treating a variety of diseases, disorders or conditions. Such diseases, disorders, or conditions include those described herein.

In one aspect, the presently disclosed exosomes are useful as drug delivery vehicles for a biologic therapeutic agent, wherein the biologic therapeutic agent is encapsulated in the exosome, such as a milk-derived exosome.

In some embodiments, the biologic is useful in treating, preventing, or ameliorating a disease or condition such as a pulmonary, ocular, liver, or viral disease or condition. In some embodiments, the biologic is selected from an iRNA or oligonucleotide or analog thereof disclosed in WO 2009/073809, WO 2006/020768, or WO 2006/078278, the disclosure of each of which is hereby incorporated by reference.

In some embodiments, the biologic is useful in treating, preventing, or ameliorating a TTR-mediated disease or condition such as amyloidosis. In some embodiments, the TTR-mediated disease or condition is selected from senile systemic amyloidosis (SSA) (also called senile cardiac amyloidosis (SCA)), TTR amyloidosis (also called ATTR (amyloidosis-transthyretin type)), leptomeningeal/CNS (Central Nervous System) amyloidosis, TTR related ocular amyloidosis, or systemic familial amyloidosis. In some embodiments, the biologic modulates expression of the transthyretin (TTR) gene. In some embodiments, the biologic is selected from an iRNA or oligonucleotide or analog thereof disclosed in WO 2015/042564, WO 2011/056883, WO 2016/033326, WO 2010/048228, WO 2011/123468, or WO 2014/022739, the disclosure of each of which is hereby incorporated by reference.

In some embodiments, the biologic is useful in treating, preventing, or ameliorating hemophilia. In some embodiments, the biologic is selected from an iRNA or oligonucleotide or analog thereof disclosed in WO 2013/163430, WO 2015/175510, or WO 2012/177949, the disclosure of each of which is hereby incorporated by reference.

In some embodiments, the biologic is useful in treating, preventing, or ameliorating complement mediated disease. In some embodiments, the biologic is selected from an iRNA or oligonucleotide or analog thereof disclosed in WO 2014/160129, WO 2004/080406, WO 2009/082607, or WO 2004/091515, the disclosure of each of which is hereby incorporated by reference.

In some embodiments, the biologic is useful in treating, preventing, or ameliorating porphyria. In some embodiments, the biologic is selected from an iRNA or oligonucleotide or analog thereof disclosed in WO 2013/155204, WO 2016/061487, or WO 2008/131419, the disclosure of each of which is hereby incorporated by reference. For example, WO 2008/131419 discloses glyco-conjugates of RNAi agents, the delivery and/or properties of which may be enhanced by encapsulation in a disclosed exosome.

In some embodiments, the biologic is useful in treating, preventing, or ameliorating primary hyperoxaluria. In some embodiments, the biologic is selected from an iRNA or oligonucleotide or analog thereof disclosed in WO 2016/057893, the disclosure of which is hereby incorporated by reference.

In some embodiments, the biologic is useful in treating, preventing, or ameliorating beta thalassemia. In some embodiments, the biologic is selected from an iRNA or oligonucleotide or analog thereof disclosed in WO 2016/085852, WO 2012/135246, or WO 2008/036933, the disclosure of each of which is hereby incorporated by reference.

In some embodiments, the biologic is useful in treating, preventing, or ameliorating alpha-1 antitrypsin deficiency. In some embodiments, the biologic is selected from an iRNA or oligonucleotide or analog thereof disclosed in WO 2013/013017, WO 2013/013019, WO 2012/178033, or WO 2014/190137, the disclosure of each of which is hereby incorporated by reference.

In some embodiments, the biologic is useful in treating, preventing, or ameliorating hypercholesterolemia or hyperlipidemia. In some embodiments, the biologic is selected from an iRNA or oligonucleotide or analog thereof disclosed in WO 2012/058693, WO 2011/038031, WO 2011/028938, WO 2010/148013, WO 2011/053994, WO 2007/134161, WO 2009/134487, WO 2015/123264, WO 2011/029016, WO 2009/129465, or WO 2009/111658, the disclosure of each of which is hereby incorporated by reference.

In some embodiments, the biologic is useful in treating, preventing, or ameliorating chronic liver infection. In some embodiments, the biologic is selected from an iRNA or oligonucleotide or analog thereof disclosed in US 2014/0148497, the disclosure of which is hereby incorporated by reference.

In some embodiments, the biologic is useful as a medicament and in methods for inhibiting the expression of a given gene. In some embodiments, the biologic is selected from an iRNA or oligonucleotide or analog thereof disclosed in WO 2000/044895, the disclosure of which is hereby incorporated by reference.

In some embodiments, the biologic is useful in treating, preventing, or ameliorating hepatitis C virus (HCV) infection. In some embodiments, the biologic is selected from an iRNA or oligonucleotide or analog thereof disclosed in US 8,273,868, the disclosure of which is hereby incorporated by reference.

In some embodiments, the biologic is useful in treating, preventing, or ameliorating hepatitis B virus (HBV), HCV, or hepatitis D virus (HDV) infection. In some embodiments, the biologic is a modified HBV-targeting oligonucleotide or expression construct, e.g. comprising at least two different RNA polymerase III promoters, wherein each promoter is operably linked to a nucleic acid sequence encoding an RNA effector molecule. In some embodiments, the biologic is useful in methods of detecting expression of a gene or reducing hypersensitivity responses in a subject. In some embodiments, the biologic is a partially double-stranded RNA molecule comprising a sequence homologous to a target sequence. In some embodiments, the biologic is fully double-stranded RNA. In some embodiments, the biologic is selected from an iRNA or oligonucleotide or analog thereof disclosed in US 9,352,048, US 2015/0119445, US 8,350,021, EP1833967, EP2316942, US 2012/0028348, US 7,985,581, EP2169072, EP1784492, US 2016/0122759, EP2994167, WO 2014/182661, US 2014/0275211, EP2723865, WO 2012/177906, EP1171586, EP1171586, EP1597351, EP1597351, WO 2016/077321, or WO 2016/077349, the disclosure of each of which is hereby incorporated by reference.

In some embodiments, the biologic is useful in modulating the replication of a single-stranded RNA virus such as HCV. In some embodiments, the biologic is useful in methods and in compositions for modulating viral replication through double-stranded RNA-mediated gene silencing (RNAi), wherein the antiviral methods and compositions preferentially target opposite strand replication intermediates of single-stranded RNA viruses. In some embodiments, the biologic comprises a double-stranded (ds) RNA effector molecule and one or more effector complements. In some embodiments, the biologic is useful in methods for assaying for activity of a gene in a tissue of a subject and methods for evaluating dsRNA-mediated silencing or inhibition of a target nucleotide sequence by a selected dsRNA effector molecule in an RNAi-competent system. In some embodiments, the biologic is selected from an iRNA or oligonucleotide or analog thereof disclosed in US 9,198,927, US 2010/0267805, EP2325314, EP1797185, EP2772541, US 2015/0315593, US 8,987,227, US 8,614,198, US 2014/0141512, US 2010/0324117, EP2173900, US 2012/0028348, US 7,985,581, EP2169072, EP1784492, US 2016/0122759, EP2994167, WO 2014/182661, US 2014/0275211, EP2723865, WO 2012/177906, US 2012/0046478, EP2068886, WO 2008/042973, EP1597351, or EP1597351, the disclosure of each of which is hereby incorporated by reference.

In some embodiments, the biologic is useful in treating, preventing, or ameliorating acromegaly. In some embodiments, the biologic modulates the expression of growth hormone receptor and/or insulin like growth factor-I (IGF-I). In some embodiments, the biologic is selected from an iRNA or oligonucleotide or analog thereof disclosed in EP2492282, EP1664267, or EP3017044, the disclosure of each of which is hereby incorporated by reference.

In some embodiments, the biologic is useful in treating, preventing, or ameliorating Alport syndrome. In certain embodiments, the biologic comprises a translation suppression element inhibitor. In certain embodiments, the translation suppression element inhibitor is a uORF inhibitor. In certain embodiments, the uORF inhibitor is an antisense compound. In some embodiments, the biologic is selected from an iRNA or oligonucleotide or analog thereof disclosed in WO 2016/077837, the disclosure of which is hereby incorporated by reference.

In some embodiments, the biologic is useful in treating, preventing, or ameliorating a neurodegenerative disease such as ALS, Kennedy's Disease, or spinal muscular atrophy. In some embodiments, the biologic is useful in reducing expression of C90RF72 antisense transcript in an animal with C90RF72 antisense transcript specific inhibitors, or altering expression of superoxide dismutase 1. Such methods are useful to treat, prevent, or ameliorate neurodegenerative diseases in an individual in need thereof. In some embodiments, the biologic is selected from an antisense compound, iRNA, oligonucleotide, or analog thereof disclosed in EP3058069, US 2016/0237432, US 2016/0251655, EP3055414, EP2906697, EP2906696, EP2742056, EP2534248, EP2270024, or WO 2016/112132, the disclosure of each of which is hereby incorporated by reference. In some embodiments, the biologic is selected from an antisense compound, iRNA, oligonucleotide, or analog thereof disclosed in EP3058068, US 2016/0230172, EP2527442, EP2021472, EP2458006, EP2363482, EP2363481, EP2951304, EP2943225, EP2906258, EP2906256, EP2906255, EP2742136, EP2742135, WO 2016/077837, WO 2016/044840, or WO 2016/040748, the disclosure of each of which is hereby incorporated by reference.

In some embodiments, the biologic is useful in treating, preventing, or ameliorating androgen receptor-mediated diseases. In certain embodiments, the androgen receptor-mediated disease is Kennedy's Disease, in which a subject carries a mutation in the androgen receptor (AR) gene, such as expansion of a CAG trinucleotide repeat, which is associated with Kennedy's Disease. In some embodiments, the biologic is an antisense compound targeted to AR. In some embodiments, the biologic tagets kinsesin-like 1. In some embodiments, the disease is cancer or a hyperproliferative disorder, such as prostate cancer (such as castrate-resistant prostate cancer), or breast cancer, ovarian cancer, gastric cancer and bladder cancer. In some embodiments, the biologic reduces expression of a nuclear-retained RNA (nrRNA) or pyruvate kinase M transcript in an animal or is useful in treating, ameliorating, delaying or reducing a symptom of a disease or disorder associated with a nuclear-retained RNA or pyruvate kinase M transcript in an animal. In some embodiments, the biologic reduces expression of metastasis-associated-in-lung-adenocarcinoma-transcript-1 (MALAT-1) RNA and/or protein. In some embodiments, reduction of MALAT-1 expression treats a cancer, such as colon cancer, intestinal cancer, lung cancer (e.g. non-small cell lung cancer), liver cancer, and/or prostate cancer. In some embodiments, the biologic is selected from an iRNA, oligonucleotide, or analog thereof disclosed in EP2991661, EP2906225, EP2906226, EP2794880, EP2253706, WO 2016/061263, or EP2595664, the disclosure of each of which is hereby incorporated by reference.

In some embodiments, the biologic is useful in treating, preventing, or ameliorating a cancer such as B-cell lymphoma or hepatocellular carcinoma. In some embodiments, the biologic inhibits expression of signal transducer and activator of transcription 3 (STAT3) mRNA or protein. In some embodiments, the biologic is selected from an iRNA, oligonucleotide, or analog thereof disclosed in EP2920308 or EP2697243, the disclosure of each of which is hereby incorporated by reference.

In some embodiments, the biologic is useful in inhibiting UBE3A-ATS, the endogenous antisense transcript of ubiquitin protein ligase E3A (UBE3A), and thus treating, preventing, or ameliorating a disease or disorder associated with UBE3A-ATS. In some embodiments, the biologic induces expression of paternal UBE3A in cells and animals. In some embodiments, the biologic is selected from an iRNA, oligonucleotide, or analog thereof disclosed in EP2864479, the disclosure of which is hereby incorporated by reference.

In some embodiments, the biologic is useful in treating, preventing, or ameliorating a neurodegenerative disease such as ALS, Kennedy's Disease, Huntington's Disease, or spinal muscular atrophy. In some embodiments, the biologic is an antisense compound that selectively reduces expression of an allelic variant of a gene containing a single nucleotide polymorphism (SNP). In some embodiments, the biologic is useful in treating a disease such as Alzheimer's disease, Parkinson's disease, cardiomyopathy, chronic obstructive pulmonary disease, or liver disease. In some embodiments, the biologic is selected from an iRNA, oligonucleotide, or analog thereof disclosed in EP2751269, EP2991661, EP2951304, EP2906256, EP2906225, EP2906255, EP2906226, EP2812342, EP2742136, EP2742135, EP2742056, EP2595664, EP2534248, or WO 2016/044840, the disclosure of each of which is hereby incorporated by reference.

In some embodiments, the biologic is useful in treating, preventing, or ameliorating thromboembolic complications or other disease conditions. Exemplary thromboembolic complications or other disease conditions include thrombosis, embolism, and thromboembolism, such as deep vein thrombosis, pulmonary embolism, myocardial infarction, stroke, cancer, rheumatoid arthritis, and fibrosis. Exemplary diseases further include clotting disorders. In some embodiments, the biologic is an antisense compound that decreases Factor 11, Factor VII, prekallikrein, or kallikrein. In some

embodiments, the biologic is selected from an iRNA, oligonucleotide, or analog thereof disclosed in EP2379084, the disclosure of which is hereby incorporated by reference. In some embodiments, the biologic is selected from an iRNA, oligonucleotide, or analog thereof disclosed in US 9,322,021, EP2726153, EP3038627, EP3000884, EP2227545, or EP2812433, the disclosure of each of which is hereby incorporated by reference.

In some embodiments, the biologic is useful in treating, preventing, or ameliorating an inflammatory, cardiovascular or metabolic disease, disorder, or condition. Exemplary diseases, disorders, and conditions include Fredrickson Type I dyslipidemia, FCS, and LPLD; and pancreatitis, cardiovascular disease, and metabolic disorders. In some embodiments, the biologic increases HDL levels and/or improves the ratio of TG to HDL and reduces plasma lipids and plasma glucose in a patient with Fredrickson Type I dyslipidemia, FCS, or LPLD. In some embodiments, the biologic decreases apolipoprotein CIII (ApoCIII) to treat, prevent, or ameliorate a disease, disorder or condition related to ApoCIII. In some embodiments, the biologic targets apolipoprotein B (ApoB) or AGPAT5. In some embodiments, biologics targeting Apolipoprotein B (ApoB) include Mipomersen and other antisense compounds targeting ApoB. Exemplary biologics include conjugated oligomeric compounds such as short antisense compounds comprising high-affinity nucleotide modifications, or other iRNA or oligonucleotide or analogs thereof, such as those disclosed in EP2015758, EP2458006, EP2991656, EP2956176, EP2701713, EP2521556, EP2408796, or WO 2016/077704, the disclosure of each of which is hereby incorporated by reference.

In some embodiments, the biologic is useful in treating, preventing, or ameliorating an inflammatory, cardiovascular and/or metabolic disease, disorder, or condition. Exemplary diseases, disorders, and conditions include diabetes, partial lipodystrophy, pancreatitis, cardiovascular disease, metabolic disorder, insulin resistance, atherosclerosis, dyslipidemia, coronary heart disease, non-alcoholic fatty liver disease (NAFLD), or hyperfattyacidemia. In some embodiments, the biologic modulates expression of GCGR, PTP1B, ANGPTL3, AGPAT5, DGAT2, fibroblast growth factor receptor 4 (FGFR4), Apo(A) or Lp(A), or glucocorticoid receptor mRNA and protein. In some embodiments, the biologic is selected from an iRNA or oligonucleotide or analog thereof disclosed in US 9,404,114, EP2758533, US 9,404,113, EP2697244, US 2016/0194349, US 2016/0152974, EP3011026, EP2215102, EP1670896, EP2021472, EP2527442, EP2505649, EP2505648, EP2505647, EP2363482, EP2363481, EP3011028, EP2992097, EP2991661, EP2992009, EP2855500, EP2771463, EP2721156, EP2363480, WO 2016/138355, or WO 2016/077837, the disclosure of each of which is hereby incorporated by reference.

In some embodiments, the biologic is useful in treating, preventing, or ameliorating transthyretin amyloidosis, such as leptomeningeal amyloidosis, familial amyloid polyneuropathy (FAP), and familial amyloid cardiopathy (FAC). In some embodiments, the biologic modulates expression of transthyretin mRNA. In some embodiments, the biologic is selected from an iRNA or oligonucleotide or analog thereof disclosed in US 2015/0252367, US 2014/0256797, US 2011/0237646, EP2323667, or WO 2010/017509, the disclosure of each of which is hereby incorporated by reference. In some embodiments, the biologic is selected from an iRNA or oligonucleotide or analog thereof disclosed in US 2016/0076030, US 9,181,549, US 9,145,558, US 9,127,276, US 2016/0017323, US 2015/0176007, US 2015/0126718, US 2014/0343123, WO 2014/179627, WO 2014/179627, WO 2014/179620, US 2015/0252367, US 9,061,044, US 8,697,860, US 2014/0256797, EP2563920, WO 2011/139917, US 2011/0237646, EP2323667, WO 2010/017509, WO 2015/179693, or WO 2015/188194, the disclosure of each of which is hereby incorporated by reference.

In some embodiments, the biologic is useful in treating, preventing, or ameliorating cardiovascular disease, metabolic disease, Fredrickson Type I dyslipidemia, familial chylomicronemia syndrome, or lipoprotein lipase deficiency. In some embodiments, the biologic modulates expression of an ANGPTL3 or ApoCIII mRNA and protein. In some embodiments, the biologic is selected from an iRNA or oligonucleotide or analog thereof disclosed in US 9,382,540, US 2015/0315594, WO 2015/168589, US 2016/0090595, US 9,163,239, US 2015/0126719, WO 2014/179626, US 2016/0152974, WO 2014/205449, US 2015/0376614, EP2956176, WO 2014/127268, or WO 2015/100394, the disclosure of each of which is hereby incorporated by reference.

In some embodiments, the biologic is useful in treating, preventing, or ameliorating hypercholesterolemia or another disease or condition associated with elevated LDL levels, or in treating, preventing, or managing a major adverse cardiovascular event in a subject with a disease or condition at risk for a major adverse cardiovascular event, e.g., familial hypercholesterolemia. In some embodiments, the biologic decreases expression of ApoB mRNA and protein. In some embodiments, the biologic is selected from an iRNA or oligonucleotide or analog thereof disclosed in EP1799859, US 7,919,472, US 2011/0207797, EP2397563, EP1799859, WO 2006/034348, US 2009/0326040, EP1786472, WO 2006/020676, EP2015758, EP2458006, WO 2016/033424, or WO 2008/118883, the disclosure of each of which is hereby incorporated by reference.

In some embodiments, the biologic is useful in treating, preventing, or ameliorating hypercholesterolemia, such as familial hypercholesterolemia, or modulating HDL or LDL-C levels. In some embodiments, the biologic modulates expression of an mRNA and corresponding protein such as angiopoietin-like 3, ApoCIII, DGAT2, ApoB, PTP1B, GCCR, SGLT2, GCGR, PCSK9, CRP, RBP4, Jun N-terminal kinase 1 (JNK1) protein, microsomal triglyceride transfer protein, tetratricopeptide repeat domain 39 isoform (TTC39), EIF2C1, or CREB. In some embodiments, the biologic is selected from an iRNA or oligonucleotide or analog thereof disclosed in US 9,382,540, US 2015/0315594, WO 2015/168589, US

2016/0017323, US 9,181,549, US 9,127,276, US 2015/0126718, US 2014/0343123, WO 2014/179620, US 2016/0060625, US 9,157,082, US 2014/0128453, EP2701713, WO 2012/149495, US 2015/0344879, US 9,045,754, US 8,969,316, US 8,673,871, US 8,586,554, US 8,372,967, US 8,362,232, US 8,188,059, US 8,143,230, US 2015/0057329, US 2013/0165496, US 2012/0208864, US 2011/0065775, US 2009/0318532, US 2009/0326042, US 2009/0326041, US 2009/0306180, US 2009/0306179, EP2015758, EP2019692, EP2023939, EP2021472, EP2023940, EP2527442, EP2505650, EP2505649, EP2505648, EP2505647, EP2505646, EP2458006, EP2397551, EP2363482, EP2363481, EP2021472, EP2015758, EP2019692, EP2023939, WO 2007/134014, WO 2007/131237, WO 2007/146511, WO 2007/143317, WO 2007/136988, WO 2007/131238, WO 2007/136989, US 2015/0167005, US 8,912,160, US 8,664,190, US 8,093,222, US 8,084,437, US 2014/0194492, US 2012/0077865, US 2010/0144834, EP2455471, EP2453016, EP2102340, WO 2009/148605, WO 2008/066776, US 8,541,388, US 2011/0123521, EP2291200, WO 2009/143390, US 2012/0214736, US 8,101,585, EP2057284, WO 2008/017081, US 7,919,472, US 2011/0207797, EP2397563, EP1799859, WO 2006/034348, US 7,803,930, US 2005/0009088, EP1569695, WO 2004/044181, WO 2003/097662, US 2005/0181376, US 6,767,739, WO 2003/018600, US 2016/0090598, US 2015/0376614, EP2956176, WO 2014/127268, US 2012/0270929, EP2480667, WO 2011/038288, US 2009/0326040, EP1786472, WO 2006/020676, US 2003/0232442, US 2003/0105042, WO 2003/040321, EP2294213, WO 2009/143463, WO 2009/143391, WO 2016/033424, WO 2015/179693, WO 2015/188194, WO 2015/164693, WO 2015/061246, WO 2010/080953, or WO 2008/118883, the disclosure of each of which is hereby incorporated by reference.

In some embodiments, the biologic is useful in treating, preventing, or ameliorating diseases and conditions associated with a heat shock protein. In some embodiments, the biologic is useful for treating, preventing, or ameliorating diabetes, obesity, metabolic syndrome X, hyperglycemia, or hyperlipidemia. In some embodiments, the biologic is useful in (i) decreasing blood glucose levels in an animal, (ii) treating an animal having a disease or condition associated with glucocorticoid receptor, (iii) decreasing blood lipid levels in an animal, or (iv) decreasing body fat mass in an animal. In some embodiments, the biologic modulates expression of the glucocorticoid receptor. In some embodiments, the biologic is selected from an iRNA or oligonucleotide or analog thereof disclosed in EP2363480 or WO 2016/077837, the disclosure of each of which is hereby incorporated by reference. In some embodiments, the disease or condition is associated with a uORF-containing gene, such as those disclosed in Tables 1 and 2 of WO 2016/077837.

In some embodiments, the biologic is useful in treating, preventing, or ameliorating an inflammatory condition, such as hereditary angioedema (HAE) or a prekallikrein-associated condition. In some embodiments, the biologic treats, prevents, or ameliorates a disease or condition such as edema or vascular permeability or leakage. In some embodiments, the biologic modulates kallikrein (KLKB1) or prekallikrein (PKK) expression. In some embodiments, the biologic is selected from an iRNA or oligonucleotide or analog thereof disclosed in US 9,315,811, EP2717923, EP3038627, or WO 2016/077837, the disclosure of each of which is hereby incorporated by reference.

In some embodiments, the biologic is useful for treating, preventing, or ameliorating a neurodegenerative disease such as transthyretin amyloidosis, familial amyloid polyneuropathy (FAP), familial amyloid cardiopathy (FAC), amyotrophic lateral sclerosis (ALS), frontotemporal dementia (FTD), corticalbasal degeneration syndrome (CBD), atypical Parkinsonian syndrome, olivopontocerellar degeneration (OPCD), tauopathy, Alzheimer's Disease, fronto-temporal dementia (FTD), FTDP-17, progressive supranuclear palsy (PSP), chronic traumatic encephalopathy (CTE), corticobasal ganglionic degeneration (CBD), epilepsy, Dravet's Syndrome, spinocerebellar ataxia, dentatorubral-pallidoluysian atrophy, or Huntington's Disease. In some embodiments, the neurodegenerative disease is associated with repeat RNA. In some embodiments, the biologic is useful for treating, preventing, or ameliorating a neurodegenerative disease such as Atrophin 1 (DRPLA), Huntington's Disease, Huntington disease-like 2 (HDL2), spinal and bulbar muscular atrophy, Kennedy disease, spinocerebellar ataxia 1, spinocerebellar ataxia 12, spinocerebellar ataxia 17, Huntington disease-like 4 (HDL4), spinocerebellar ataxia 2, spinocerebellar ataxia 3, Machado-Joseph disease, spinocerebellar ataxia 6, or spinocerebellar ataxia 7; or myotonic dystrophy (DM1) or spinocerebellar ataxia 8; or fragile X syndrome, ataxin 3, or Friedrich's ataxia. In some embodiments, the biologic is useful for treating, preventing, or ameliorating Alzheimer's disease, Creutzfeldt-Jakob disease, fatal familial insomnia, Alexander disease, Parkinson's disease, amyotrophic lateral sclerosis, dentato-rubral and pallido-luysian atrophy DRPA, spinocerebellar ataxia, torsion dystonia, cardiomyopathy, chronic obstructive pulmonary disease (COPD), liver disease, hepatocellular carcinoma, systemic lupus erythematosus, hypercholesterolemia, breast cancer, asthma, type 1 diabetes, rheumatoid arthritis, Graves' disease, SLE, spinal and bulbar muscular atrophy, Kennedy's disease, progressive childhood posterior subcapsular cataracts, cholesterol gallstone disease, atherosclerosis, cardiovascular disease, primary hypercalciuria, alpha-thallasemia, obsessive compulsive disorder, anxiety, comorbid depression, congenital visual defects, hypertension, metabolic syndrome, prostate cancer, congential myasthenic syndrome, peripheral arterial disease, atrial fibrillation, sporadic pheochromocytoma, congenital malformations, Machado-Joseph disease, Duchenne muscular dystrophy, Huntington's Disease, or retinitis pigmentosa (RP) disease, such as autosomal dominant retinitis pigmentosa (AdRP) disease. In some embodiments, the biologic is useful for treating, preventing, or ameliorating AIATD associated liver disease or pulmonary diseases such as AIATD associated pulmonary disease. In some embodiments, the biologic is useful for treating, preventing, or ameliorating a

prion disease or conformational neurodegenerative disorder. In some embodiments, the biologic is useful for treating, preventing, or ameliorating myotonia or reducing spliceopathy or, for example, type 1 myotonic dystrophy or facioscapulohumeral muscular dystrophy. In some embodiments, the biologic is useful for treating, preventing, or ameliorating macular degeneration, age related macular degeneration (AMD), wet AMD, dry AMD, or geographic atrophy. In some embodiments, the biologic modulates expression of transthyretin, apolipoprotein C-III (ApoCIII), alpha-1-antitrypsin (AIAT), complement factor B, tau, ATXN-3 pre-mRNA, ATN-1, a human Prp, SMN2, C90RF72, DMPK, alpha-synuclein, DUX4, or huntingtin mRNA and protein. In some embodiments, the biologic increases DMN1, BDNF, and synapsin 1 expression by decreasing REST expression, thus treating, preventing, or ameliorating Huntington's Disease. In some embodiments, the biologic is selected from an iRNA or oligonucleotide or analog thereof disclosed in US 9,428,750, US 9,409,934, US 9,404,114, EP2758533, US 9,404,113, EP2697244, US 9,403,865, EP2885312, US 9,399,774, EP2563920, US 2016/0237434, US 9,365,848, EP2441449, EP3002007, EP2428227, US 9,353,372, EP2161038, EP2422819, WO 2007/089584, US 9,353,371, US 2016/0186185, US 9,321,799, EP2601204, US 9,340,784, US 9,322,021, EP2726153, US 9,315,811, EP2717923, US 9,273,315, US 8,906,873, EP2475675, WO 2011/032045, US 9,290,534, US 2015/0292015, US 9,006,198, US 2013/0046008, EP2534262, WO 2011/097644, US 8,957,040, US 2013/0046007, EP2534248, WO 2011/097643, US 2016/0244477, EP3058069, US 2016/0237432, EP3058068, US 2016/0230172, US 2016/0251655, US 2016/0222389, EP3043827, US 2016/0194638, US 2016/0194637, US 2016/0186175, US 2016/0194349, US 2016/0186174, US 2016/0145617, EP3022217, US 2016/0159846, EP3027617, US 2016/0152974, EP3011026, EP2951304, US 2015/0376625, WO 2014/121287, EP2906256, US 2015/0275208, WO 2014/059356, US 2013/0059902, EP2536738, WO 2011/097641, US 2011/0269818, WO 2010/019270, EP2625186, EP1937312, EP2606057, EP2751269, WO 2013/033223, EP2580228, EP2215102, EP2492282, EP1664267, EP2125852, EP2673361, EP2742136, EP2742135, EP2742056, WO 2013/022990, WO 2013/022984, EP1730309, EP2331141, EP2173358, EP2462153, EP1984499, EP2548560, EP2644700, EP2365094, EP2246443, EP2957568, EP1560840, EP2361923, EP1670896, EP2410053, EP2092065, EP2410054, EP2015758, EP2021472, EP2527442, EP2505649, EP2505648, EP2505647, EP2458006, EP2363482, EP2363481, EP2332951, EP3055414, EP2951191, EP2906258, EP2906255, EP2943225, EP3038627, EP3030658, EP3031920, EP2595663, EP2595664, WO 2012/012467, EP2906225, EP3017044, EP3011028, EP2906226, EP2920308, EP2906697, EP2906699, EP2906696, EP2864479, EP2852606, EP2992097, EP2991661, EP2992098, EP2992009, EP2991656, EP2877579, EP3000884, EP2227545, EP2956176, EP2971142, EP2855500, EP2850092, EP2831232, EP2839006, EP2812342, EP2794880, EP2771463, EP2812433, EP2802674, EP2776564, EP2751270, EP2697243, EP2699583, EP2701713, EP2721156, EP2640853, EP2582397, EP2521556, EP2442816, EP2447274, EP2358397, EP2399588, EP2360166, EP2408796, EP2379084, EP2363480, EP2327709, EP2334319, EP2282744, EP2272958, EP2270024, EP2253706, EP2222851, EP2219680, EP1957507, EP1827459, EP1427289, EP1159282, WO 2016/138355, WO 2016/138353, WO 2016/138017, WO 2016/137923, WO 2016/112132, WO 2016/115490, WO 2016/077704, WO 2016/077540, WO 2016/086104, WO 2016/077837, WO 2016/061263, WO 2016/044840, WO 2016/044828, WO 2015/168532, WO 2016/040748, WO 2011/097614, WO 2011/031998, US 9,057,066, US 8,952,145, US 8,415,465, US 7,951,934, US 2016/0053256, US 2015/0307877, US 2013/0281684, US 2013/0189782, US 2011/0306652, US 2010/0069472, EP1991677, WO 2007/089611, US 9,321,799, US 2015/0159155, US 2015/0329859, US 8,669,102, US 6,969,763, EP1248791, US 6,303,374, WO 2001/053310, WO 2002/020840, US 6,258,601, US 2016/0138014, WO 2014/059341, WO 2015/017675, US 2015/0051389, US 2014/0323707, US 2014/0309279, US 2014/0303235, WO 2013/022967, WO 2013/022966, US 2015/0018540, WO 2013/033230, US 2014/0316121, US 2013/0225659, EP1083980, US 2005/0239737, WO 2004/043394, US 2004/0092465, WO 2004/048522, US 2004/0102394, US 2004/0096834, EP1436430, US 2003/0087854, WO 2003/023004, WO 2015/168172, or WO 2014/036301, the disclosure of each of which is hereby incorporated by reference.

In some embodiments, the biologic is useful in modulating enhancer RNAs (eRNAs). In some embodiments, the biologic is selected from an iRNA or oligonucleotide or analog thereof disclosed in EP2852606, the disclosure of which is hereby incorporated by reference.

In some embodiments, the biologic is useful in modulating AGT and modulating a RAS pathway related disease, disorder or condition. RAS related diseases such as hypertension or organ damage can be treated, ameliorated or prevented with the administration of antisense compounds targeted to AGT; in some embodiments, these include shortened life expectancy, hypertension, chronic kidney disease, stroke, cardiac disease, aneurysms of the blood vessels, peripheral artery disease, and organ damage. In some embodiments, the biologic is selected from an iRNA or oligonucleotide or analog thereof disclosed in EP2877579, the disclosure of which is hereby incorporated by reference.

In some embodiments, the biologic is useful in treating, preventing, or ameliorating a disease associated with CD40. Examples of disease conditions that can be ameliorated with the administration of antisense compounds targeted to CD40 include hyperproliferative disorders, graft versus host disease (GVHD), graft rejection, asthma, airway hyperresponsiveness, chronic obstructive pulmonary disease (COPD), multiple sclerosis (MS), systemic lupus erythematosus (SLE), and certain forms of arthritis. In some embodiments, the biologic is selected from an iRNA or oligonucleotide or analog thereof disclosed in EP2222851, the disclosure of which is hereby incorporated by reference.

In some embodiments, the biologic is useful in treating, preventing, or ameliorating ulcerative colitis. In some embodiments, the biologic is selected from an iRNA or oligonucleotide or analog thereof disclosed in EP1827459, the disclosure of which is hereby incorporated by reference.

In some embodiments, the biologic is useful in treating, preventing, or ameliorating a disease related to expanded repeat-containing RNA, such as ataxin 3, atrophin 1, fragile X syndrome, Friedrich's ataxia, Huntington's disease, Huntington's disease-like 2, myotonic dystrophy, spinal and bulbar muscular atrophy, and spinocerebellar ataxia. In some embodiments, the disease is myotonic dystrophy, such as type 1 myotonic dystrophy. In some embodiments, the biologic reduces expression of a DMPK mRNA and protein, or nrRNA, or ATXN-3 pre-mRNA or ATN-1 mRNA. In some embodiments, the biologic is selected from an iRNA or oligonucleotide or analog thereof disclosed in EP2751269, EP3030658, EP3031920, EP2595663, EP2595664, US 2016/0159846, EP3027617, EP2906258, EP2906697, EP2906696, EP2751270, or WO 2016/077837, the disclosure of each of which is hereby incorporated by reference.

In some embodiments, the biologic is useful in treating, preventing, or ameliorating spinal muscular atrophy (SMA), such as type I, II, or III SMA. In some embodiments, the biologic is useful for treating, preventing, or ameliorating familial dysautonomia. In some embodiments, the biologic modulates splicing of the SMN2 gene. In some embodiments, the biologic modulates the expression of a Gemin gene. In some embodiments, the biologic modulates EIF2C2 and/or DDX36 expression. In some embodiments, the biologic modulates splicing of the IKBKAP gene. In certain embodiments, the IKBKAP gene includes a mutation that results in defective splicing and a truncated IKAP protein. In some embodiments, the biologic modulates expression of fibrillarin; or modulates expression of phosphodiesterase 4D. In some embodiments, the biologic is selected from an iRNA or oligonucleotide or analog thereof disclosed in US 8,980,853, US 2016/0002627, EP2943225, WO 2014/110291, US 2014/0357558, WO 2012/178146, US 2013/0109091, EP2644700, EP2548560, EP1910395, WO 2007/002390, WO 2005/001031, EP1631659, WO 2015/161170, WO 2010/120820, EP2442816, WO 2010/148249, US 2015/0353929, US 8,946,183, US 8,361,977, US 2010/0216238, US 8,409,856, US 7,759,479, US 2011/0105586, US 7,709,453, US 2015/0284725, EP2906225, WO 2014/059364, US 2015/0275205, EP2831232, EP2831231, WO 2013/148260, WO 2013/148283, US 2015/0025231, EP2802674, WO 2013/106770, US 2004/0102403, US 2003/0220273, or WO 2011/031998, the disclosure of each of which is hereby incorporated by reference.

In some embodiments, the biologic is useful in treating, preventing, or ameliorating pouchitis. In some embodiments, the biologic modulates expression of ICAM-1. In some embodiments, the biologic is selected from an iRNA or oligonucleotide or analog thereof disclosed in US 8,946,178, US 8,084,432, US 2012/0270920, US 2004/0162259, WO 2004/071453, US 2009/0275631, EP1827459, WO 2006/060649, or WO 2015/188194, the disclosure of each of which is hereby incorporated by reference.

In some embodiments, the biologic is useful in treating, preventing, or ameliorating a cancer, such as prostate, colon, or hepatoma. In some embodiments, the biologic is useful in a method of inducing apoptosis in cancer cells by supercharging Alpha 2-HS glycoprotein with zinc and administering said glycoprotein to the cancer cells. In some embodiments, the biologic comprises fetuin or an extract of Melothria indica Lou. In some embodiments, the biologic is selected from an iRNA or oligonucleotide or analog thereof disclosed in US 7,238,662, the disclosure of which is hereby incorporated by reference.

In some embodiments, the biologic is useful in treating, preventing, or ameliorating a hepatitis viral infection, such as hepatitis A, hepatitis B, or hepatitis C. In some embodiments, the biologic modulates expression of a hepatitis viral protein. In some embodiments, the biologic is selected from an iRNA or oligonucleotide or analog thereof disclosed in US 2015/0361432, US 9,139,833, US 2015/0376621, US 9,084,808, EP2726613, US 2013/0005793, WO 2013/003520, EP2651420, or WO 2012/083185, the disclosure of each of which is hereby incorporated by reference.

In some embodiments, the biologic is useful in treating, preventing, or ameliorating adrenoleukodystrophy and/or adrenomyeloneuropathy. In some embodiments, the biologic is useful in treating, preventing, or ameliorating hemoglobinopathy such as thalassemia, sickle cell disease, adrenoleukodystrophy or an adrenomyeloneuropathy. In some embodiments, the biologic is selected from a retroviral vector, iRNA, or oligonucleotide or analog thereof disclosed in US 9,061,031, US 8,858,928, US 2015/0064150, US 2015/0037296, US 2013/0004471, EP2717922, WO 2012/170911, US 2015/0216903, WO 2014/026110, US 2014/0234278, EP2760994, WO 2013/049615, US 2014/0199279, EP2661489, WO 2012/094193, WO 2014/015318, WO 2012/170431, the disclosure of each of which is hereby incorporated by reference.

In some embodiments, the biologic is useful in treating, preventing, or ameliorating EPAS1-related diseases such as cancer, metastases, astrocytoma, bladder cancer, breast cancer, chondrosarcoma, colorectal carcinoma, gastric carcinoma, glioblastoma, head and neck squamous cell carcinoma, hepatocellular carcinoma, lung adenocarcinoma, neuroblastoma, non-small cell lung cancer, melanoma, multiple myeloma, ovarian cancer, rectal cancer, renal cancer, clear cell renal cell carcinoma (and metastases of this and other cancers), gingivitis, psoriasis, Kaposi's sarcoma-associated herpesvirus, preemclampsia, inflammation, chronic inflammation, neovascular diseases, or rheumatoid arthritis. In some embodiments, the biologic modulates expression of EPAS1 (HIF-2alpha). In some embodiments, the biologic is selected from an iRNA or oligonucleotide or analog thereof disclosed in US 2016/0010089 or EP2961843,

the disclosure of each of which is hereby incorporated by reference.

In some embodiments, the biologic is useful in treating, preventing, or ameliorating a B cell related condition or a cancer such as multiple myeloma (MM), chronic lymphocytic leukemia (CLL), or non-Hodgkin's lymphoma (NHL), or systemic lupus erythematosus, rheumatoid arthritis, idiopathic thrombocytopenia purpura, myasthenia gravis, or autoimmune hemolytic anemia. In some embodiments, the biologic is a chimeric antigen receptor (CAR). In some embodiments, the biologic is useful in adoptive T cell therapy. In some embodiments, the biologic is selected from a therapeutic agent such as a T cell composition or CAR disclosed in WO 2016/094304, WO 2016/014789, WO 2015/188119, WO 2015/164739, WO 2015/164759,

EP3027204, US 2015/0266973, WO 2015/017214, WO 2015/164745, or WO 2015/164739, the disclosure of each of which is hereby incorporated by reference.

In some embodiments, the biologic is useful in treating, preventing, or ameliorating hemoglobinopathic conditions such as diseases, disorders, and conditions of the hematopoietic system such as thalassemias and anemias, for example sickle cell anemia. In some embodiments, the biologic is useful in cell therapy or gene therapy. In some embodiments, the biologic is useful in treating therapeutic indications amenable to treatment with hematopoietic stem cell gene therapies. In some embodiments, the biologic is useful in increasing cell transduction efficiency. In some embodiments, the biologic is selected from those disclosed in US 2016/0022839, US 9,068,199, US 7,901,671, US 2012/0009161, US 2015/0203868, WO 2013/043196, US 2015/0216903, WO 2014/026110, US 2014/0234278, EP2760994, WO 2013/049615, US 2014/0199279, EP2661489, WO 2012/094193, WO 2014/015318, or WO 2012/170431, the disclosure of each of which is hereby incorporated by reference.

In some embodiments, the biologic is useful in treating, preventing, or ameliorating hemoglobinopathic conditions such as hemoglobinopathy, including for example hemoglobin sickle cell disease (SCD), sickle cell anemia, and β-thalassemia. In some embodiments, the biologic is useful in cell therapy or gene therapy. In some embodiments, the biologic is useful in treating therapeutic indications amenable to treatment with hematopoietic stem cell gene therapies. In some embodiments, the biologic is useful in increasing cell transduction efficiency. In some embodiments, the biologic modulates expression of a globin gene. In some embodiments, the biologic is selected from a those disclosed in US 2016/0022839, US 9,068,199, US 7,901,671, US 2012/0009161, US 2015/0203868, WO 2013/043196, US 2015/0216903, WO 2014/026110, US 2014/0234278, EP2760994, WO 2013/049615, US 2014/0199279, EP2661489, WO 2012/094193, WO 2014/015318, or WO 2012/170431, the disclosure of each of which is hereby incorporated by reference.

In some embodiments, the biologic is useful in treating, preventing, or ameliorating a cardiovascular indication such as heart failure, acute heart failure, chronic heart failure, congestive heart failure, acute decompensated heart failure, abnormal fluid accumulation in the heart, myocardial edema, or dypsnea. In some embodiments, the biologic is useful in treating, preventing, or ameliorating cardiovascular, renal, pulmonary, or neuronal syndromes while avoiding a rebound. In some embodiments, the biologic is selected from a peptide such as a natriuretic peptide, diuretic peptide, or vasodilatory peptide; or a relaxin; for example, atrial natriuretic peptide (ANP), brain natriuretic peptide (BNP), neseritide, C-type natriuretic peptide (CNP), dendroaspis natriuretic peptide (DNP), and urodilatin, or an analog thereof. In some embodiments, the biologic is ularitide. In some embodiments, the biologic prevents or minimizes nitrosylation of myocardial cells. In some embodiments, the biologic is selected from those disclosed in EP2948165, US 2014/0213520, US 2014/0213519, WO 2014/115033, US 9,358,271, US 9,023,794, US 2015/0224174, US 2014/0287999, EP2510942, EP2510942, EP2948165, US 2014/0213520, US 2014/0213519, WO 2014/115033, or EP2547356, the disclosure of each of which is hereby incorporated by reference.

In some embodiments, the biologic is useful in treating, preventing, or ameliorating cancer diseases, infectious diseases such as HIV, allergies and autoimmune diseases such as rheumatoid arthritis or allergic conditions; or is useful in modulating an immune response or as a vaccine. In some embodiments, the biologic is selected from a polymeric carrier cargo complex, nucleic acid, antigen, or other biologic disclosed in US 9,314,535, US 8,703,906, US 2014/0294877, US 2012/0219573, US 2011/0053829, EP2810661, EP2331138, WO 2011/026641, US 9,226,959, US 2012/0021043, EP2548960, EP2176408, WO 2009/095226, EP3035955, US 2016/0168227, WO 2015/024666, EP3035954, US 2016/0166668, WO 2015/024664, US 2016/0166691, US 2016/0136263, US 2015/0093413, EP2814962, WO 2013/120628, WO 2013/120499, EP2678038, US 2013/0259879, WO 2012/113513, WO 2012/113413, US 2013/0251742, EP2195015, WO 2009/046975, US 2013/0202645, EP2197481, WO 2009/046974, EP2762165, US 2011/0250225, EP2331129, WO 2010/037539, WO 2010/037408, WO 2011/144358, EP2387999, WO 2011/069587, WO 2011/069528, WO 2011/069529, WO 2010/088927, WO 2003/059381, US 9,447,431, US 9,421,255, US 9,439,956, US 9,433,670, US 9,433,669, US 9,155,788, US 8,217,016, US 2016/0095911, US 2016/0089426, US 2016/0095912, US 2016/0089425, US 2016/0089424, US 2016/0082092, US 2015/0030633, US 2011/0311472, EP1458410, EP2769733, EP1925317, EP1905844, US 9,402,887, US 9,352,028, US 2016/0206756, US 9,234,013, EP2603590, EP2796557, WO 2012/019780, WO 2012/019630, US 2016/0250321, EP2955230, US 8,968,746, US 2015/0258214, US 2013/0142818, EP2449113, WO 2012/013326, US 8,383,340, EP2092064, WO 2008/077592, US 2016/0206719, US 2016/0130345, EP2958588, WO 2014/127917, US 2016/0185840, US 2016/0168254, US 2016/0166692, US

2016/0166690, US 2016/0152706, US 2016/0152691, US 2016/0145346, US 2013/0195867, EP2101823, WO 2008/083949, US 2016/0184406, US 2014/0037660, US 2010/0203076, EP2484770, EP2188379, WO 2009/030481, WO 2009/030254, EP3035960, US 2016/0168207, WO 2015/024668, EP3036330, US 2016/0166710, WO 2015/024667, EP3035961, US 2016/0166711, WO 2015/024665, EP3035959, US 2016/0166678, WO 2015/024669, US 2016/0151474, US 2013/0295043, EP2680881, WO 2012/116811, WO 2012/116714, US 2016/0136301, US 2016/0136259, US 2016/0136258, US 2016/0136247, US 2016/0136243, US 2016/0129105, US 2015/0104476, US 2011/0269950, US 2011/0077287, US 2010/0239608, EP2305699, EP1857122, EP1800697, EP1832603, EP1604688, EP1392341, EP2842964, EP1903054, US 2015/0320847, EP2814961, WO 2013/120627, WO 2013/120500, US 2015/0306249, EP2854857, WO 2013/174409, US 2015/0218554, EP2831241, WO 2013/143699, US 2015/0184195, EP2831239, WO 2013/143698, US 2015/0165006, EP2814964, WO 2013/120626, WO 2013/120498, US 2015/0118264, EP2809353, WO 2013/113501, WO 2013/113326, US 2015/0118183, EP2809354, WO 2013/113502, WO 2013/113325, US 2015/0141498, EP2510100, WO 2011/069586, US 2015/0057340, EP2814963, WO 2013/120629, WO 2013/120497, US 2015/0050302, EP2831240, WO 2013/143700, US 2015/0037326, EP2809352, EP2623121, WO 2013113736, EP2680880, US 20130336998, WO 2012/116715, WO 2012/116810, EP2658569, US 2013/0280283, WO 2012/089338, WO 2012/089225, EP2216027, US 2013/0273001, US 2010/0303851, EP1685844, EP1521585, EP2216028, EP1806139, EP1797886, WO 2004/004743, US 2012/0213818, EP1928494, WO 2006/024518, US 2012/0009221, EP2223700, EP2229953, EP1938833, EP1615662, WO 2005/016376, EP2762165, US 2010/0047261, EP2083851, WO 2008/052770, US 2008/0171711, EP1768703, WO 2006/008154, EP1383556, WO 2016/107877, WO 2016/097065, WO 2016/091391, WO 2015/149944, WO 2015/135558, WO 2015/101414, WO 2015/101415, the disclosure of each of which is hereby incorporated by reference.

In some embodiments, the biologic is useful in treating, preventing, or ameliorating a disease caused by or associated with non-coding RNA. In some embodiments, the biologic modulates expression of miR-103 and/or miR-107 or another small non-coding RNA. In some embodiments, the biologic is selected from an iRNA or oligonucleotide or analog thereof disclosed in US 9,267,138, US 2015/0037305, EP1648914, or WO 2016/022753, the disclosure of each of which is hereby incorporated by reference.

In some embodiments, the biologic is useful in treating, preventing, or ameliorating a disease or disorder associated with a miRNA, such as Alport Syndrome, or cancer, diabetic retinopathy, cardiovascular disease, rheumatoid arthritis, or psoriasis. In some embodiments, the biologic modulates expression of an RNA such as miR-21, miR-33, miR-103, miR-107, miR-122, miR-155, miR-214, miR-15, or miR-16. In some embodiments, the biologic is selected from an iRNA or oligonucleotide or analog thereof disclosed in US 9,447,413, US 9,447,412, US 9,359,609, US 9,012,423, US 2015/0299704, US 2014/0100263, EP2906698, WO 2014/058881, US 9,267,138, US 9,139,832, US 8,946,179, US 8,859,521, US 8,809,294, US 8,765,701, US 8,697,663, US 8,546,350, US 8,466,120, US 8,178,506, US 8,133,876, US 8,110,558, US 8,106,025, US 7,759,319, US 7,683,036, US 2016/0017329, US 2015/0337305, US 2015/0337304, US 2015/0247142, US 2015/0094461, US 2014/0336370, US 2014/0329882, US 2014/0121365, US 2014/0121364, US 2014/0057963, US 2012/0283319, US 2012/0157514, US 2012/0122216, US 2012/0035248, US 2011/0224277, US 2010/0267813, US 2010/0249215, US 2009/0317907, US 2009/0298174, US 2009/0291907, US 2009/0291906, US 2009/0286969, US 2009/0203893, EP1931780, EP2530157, EP2338992, EP1931780, EP1648914, US 2016/0244753, US 9,267,137, US 8,969,317, US 2015/0218558, US 2013/0289093, EP2841579, WO 2013/163258, US 2016/0138016, US 9,181,547, US 8,912,161, US 2014/0329887, US 2014/0107183, US 2012/0270928, EP2702155, WO 2012/148952, US 2016/0046941, US 9,150,857, US 8,680,067, US 8,211,867, US 2014/0206854, US 2012/0295962, US 2011/0251150, US 2010/0267814, EP2217248, EP2992096, US 2015/0031130, WO 2014/179445, EP3060664A1, WO 2015/061536, WO 2012/012716, or WO 2011/126842, the disclosure of each of which is hereby incorporated by reference.

In some embodiments, the biologic is useful in treating, preventing, or ameliorating fibrosis, cancer, or Alport Syndrome. In some embodiments, the biologic modulates expression of miR-21. In some embodiments, the biologic is selected from an iRNA or oligonucleotide or analog thereof disclosed in US 9,267,137, US 8,969,317, US 2015/0218558, US 2013/0289093, EP2841579, WO 2013/163258, EP2906698, US 9,012,423, US 2014/0100263, WO 2014/058881, US 8,697,663, US 8,466,120, US 8,110,558, US 2011/0224277, US 2010/0267813, EP1648914, US 2014/0107183, US 2012/0270928, EP2702155, WO 2012/148952, EP3060664, WO 2015/061536, or WO 2011/126842, the disclosure of each of which is hereby incorporated by reference.

In some embodiments, the biologic is useful in treating, preventing, or ameliorating a viral infection such as hepatitis C or a disease or condition such as fibrosis. In some embodiments, the biologic modulates expression of miR-122, miR-214, or miR-21. In some embodiments, the biologic is selected from an iRNA or oligonucleotide or analog thereof disclosed in US 2016/0251657, US 9,309,513, US 9,157,083, US 2015/0105449, US 2014/0350090, EP2992095, WO 2014/179446, US 2016/0244753, US 9,267,137, US 8,969,317, US 2015/0218558, US 2013/0289093, EP2841579, WO 2013/163258, US 2016/0138016, US 9,181,547, US 8,912,161, US 2014/0329887, US 2014/0107183, US 2012/0270928, EP2702155, WO 2012/148952, US 2016/0108397, US 9,181,548, US 8,815,826, US 2015/0080453, US 2013/0184217, WO 2012/012716, EP3060664, or WO 2015/061536, the disclosure of each of which is hereby

incorporated by reference.

In some embodiments, the biologic is useful in treating, preventing, or ameliorating a viral infection such as hepatitis C virus, a liver disease such as non-alcoholic fatty liver disease, or other diseases or conditions such as cardiovascular and metabolic diseases. In some embodiments, the biologic modulates expression of miR-122. In some embodiments, the biologic is selected from an iRNA or oligonucleotide or analog thereof disclosed in US 2016/0251657, US 9,309,513, US 9,157,083, US 2015/0105449, US 2014/0350090, EP2992095, WO 2014/179446, US 8,969,314, US 2015/0232841, EP2338991, or EP1931782, the disclosure of each of which is hereby incorporated by reference.

In some embodiments, the biologic is useful in treating, preventing, or ameliorating a cardiovascular or metabolic disease. In some embodiments, the biologic modulates expression of miR-122a. In some embodiments, the biologic is selected from an iRNA or oligonucleotide or analog thereof disclosed in US 2016/0251657, US 9,309,513, US 2015/0105449, US 2014/0350090, EP2992095, WO 2014/179446, or US 2015/0232841, the disclosure of each of which is hereby incorporated by reference.

In some embodiments, the biologic is useful in treating, preventing, or ameliorating Alport Syndrome, a liver cancer, and/or fibrosis. In some embodiments, the biologic modulates expression of miR-21 and/or miR-214. In some embodiments, the biologic is selected from an iRNA or oligonucleotide or analog thereof disclosed in US 9,359,609, US 9,012,423, US 2015/0299704, US 2014/0100263, EP2906698, WO 2014/058881, US 2016/0244753, US 9,267,137, US 8,969,317, US 2015/0218558, US 2013/0289093, EP2841579, WO 2013/163258, US 2016/0108397, US 9,181,548, US 8,815,826, US 2015/0080453, US 2013/0184217, WO 2012/012716, EP3060664, or WO2015/061536, the disclosure of each of which is hereby incorporated by reference.

In some embodiments, the biologic is useful in treating, preventing, or ameliorating ALS. In some embodiments, the biologic modulates expression of FXN, SMN1 and/or SMN2. In some embodiments, the biologic is selected from an iRNA or oligonucleotide or analog thereof disclosed in EP3052632, US 2016/0222391, WO 2015/051283, EP3033424, US 2015/0247145, US 2015/0247144, US 2015/0232847, US 2015/0232846, US 2015/0232845, US 2015/0232844, US 2015/0225715, US 2015/0050738, WO 2015/023975, US 2015/0252364, EP2850186, WO 2013/173638, US 2015/0232858, WO 2016/130943, WO 2016/130929, WO 2011/116152, WO 2007/092181, or WO 2007/089607, the disclosure of each of which is hereby incorporated by reference.

In some embodiments, the biologic is useful in treating, preventing, or ameliorating ALS. In some embodiments, the biologic modulates expression of SMN1, SMN2, ABCA1. APOA1, FOXP3 and/or BDFN. In some embodiments, the biologic is selected from an iRNA or oligonucleotide or analog thereof disclosed in EP3052632, US 2016/0222391, WO 2015/051283, US 2016/0122760, EP3004354, WO 2014/197826, US 2015/0315585, US 2015/0315586, US 2015/0247141, EP2895200, WO 2014/043544, US 2015/0315587, US 2015/0299695, US 2015/0315588, EP2756080, WO 2013/040429, EP3033424, US 2015/0247145, US 2015/0247144, US 2015/0232847, US 2015/0232846, US 2015/0232845, US 2015/0232844, US 2015/0225715, US 2015/0050738, WO 2015/023975, US 2015/0252364, US 2015/0191722, US 2015/0218560, US 2015/0159161, US 2015/0133362, US 2015/0133529, EP2850189, EP2850186, EP2849801, EP2849800, WO 2013/173652, WO 2013/173647, WO 2013/173638, WO 2013/173601, US 2015/0232836, EP2850183, WO 2013/173635, US 2015/0141320, EP2850184, WO 2013/173637, WO 2016/130963, WO 2016/130943, or WO 2016/130929, the disclosure of each of which is hereby incorporated by reference.

In some embodiments, the biologic is useful in treating, preventing, or ameliorating a liver disease. In some embodiments, the biologic modulates expression of THRB or NR1H4. In some embodiments, the biologic is selected from an iRNA or oligonucleotide or analog thereof disclosed in US 2015/0315585, US 2015/0315586, EP2895200, WO 2014/043544, US 2015/0315587, US 2015/0315588, EP2756080, WO 2013/040429, or WO 2016/130963, the disclosure of each of which is incorporated by reference.

In some embodiments, the biologic is useful in treating, preventing, or ameliorating an inflammatory disease. In some embodiments, the biologic modulates expression of FOXP3. In some embodiments, the biologic is selected from an iRNA or oligonucleotide or analog thereof disclosed in US 2016/0122760, EP3004354, WO 2014/197826, US 2015/0315585, US 2015/0315586, US 2015/0247141, EP2895200, WO 2014/043544, US 2015/0315587, US 2015/0299695, US 2015/0315588, EP2756080, WO 2013/040429, EP3033424, US 2015/0247145, US 2015/0247144, US 2015/0232847, US 2015/0232846, US 2015/0232845, US 2015/0232844, US 2015/0225715, US 2015/0050738, WO 2015/023975, US 2015/0232836, EP2850183, WO 2013/173635, US 2015/0218560, US 2015/0133362, EP2850189, WO 2013/173652, EP3033422, US 2015/0232858, WO 2015/023941, US 2015/0141320, EP2850184, WO 2013/173637, WO 2016/130943, or WO2016/130929, the disclosure of each of which is hereby incorporated by reference.

In some embodiments, the biologic is useful in treating, preventing, or ameliorating Friedrich's ataxia or a disease associated with heterochromatin formation. In some embodiments, the biologic modulates expression of Frataxin (FXN). In some embodiments, the biologic is selected from an iRNA or oligonucleotide or analog thereof disclosed in US 2016/0201064, EP3033425, WO 2015/023939, US 2016/0201063, EP3033423, WO 2015/023938, EP3033424, US 2015/0247145, US 2015/0247144, US 2015/0232847, US 2015/0232846, US 2015/0232845, US 2015/0232844, US 2015/0225715, US 2015/0050738, WO 2015/023975, EP3033114, US 2015/0225722, WO 2015/023937, EP3033422,

US 2015/0232858, WO 2015/023941, WO 2016/130963, WO 2016/130943, or WO 2016/130929, the disclosure of each of which is hereby incorporated by reference.

In some embodiments, the biologic is useful in treating, preventing, or ameliorating microvascular disorders, eye diseases, respiratory conditions, hearing problems, or optic neuropathies. In some embodiments, the biologic modulates expression of RTP801L or ENDO180. In some embodiments, the biologic is selected from an iRNA or oligonucleotide or analog thereof disclosed in US 9,222,087, US 8,017,764, US 7,626,015, US 2013/0303590, US 2012/0108647, US 2010/0168204, EP2026843, WO 2007/141796, US 9,056,903, US 8,067,570, US 2012/0156208, EP2402443, EP1984003, EP2862929, US 8,778,904, EP2510098, EP2510098, WO 2011/072091, US 8,642,571, US 8,309,532, US 8,168,607, US 7,741,299, US 2013/0095117, US 2011/0117102, US 2011/0028532, EP1791568, EP2319925, EP1791568, US 2014/0350068, US 8,614,311, US 2013/0131143, WO 2009/074990, US 8,444,983, US 2015/0359905, US 2014/0072552, EP2411413, WO 2010/111198, US 2013/0190387, US 8,404,654, US 7,825,099, US 2010/0029746, US 8,344,104, US 8,034,575, US 7,723,052, US 2012/0034599, US 2011/0045499, WO 2008/054534, WO 2008/054534, WO 2008/001361, US 8,034,902, EP1885396, US 7,973,156, US 7,524,935, US 2009/0264634, EP1115733, US 2011/0098337, US 7,872,119, EP2137205, WO 2008/106102, US 2015/0267194, EP2895607, WO 2014/043289, US 2014/0323549, EP2776565, WO 2013/070821, EP2268316, WO 2009/116037, EP2152316, WO 2008/132723, EP1933880, EP1357881, WO 2010/080452, or WO 2008/126085, the disclosure of each of which is hereby incorporated by reference.

In some embodiments, the biologic is useful in treating, preventing, or ameliorating microvascular disorders, eye diseases, respiratory conditions, hearing problems, or optic neuropathies; or fibrotic diseases and disorders including liver fibrosis, pulmonary fibrosis, peritoneal fibrosis and kidney fibrosis; or neurodegenerative disorders including Alzheimer's disease and Amyotrophic Lateral Sclerosis, eye diseases including glaucoma and ION, acute renal failure, hearing loss, acute respiratory distress syndrome and in preventing or treating ischemia-reperfusion injury in organ transplant patients. In some embodiments, the biologic modulates expression of RTP801L, ENDO180, RhoA, TP53, HTRA2, KEAP1, SHC1-SHC, ZNHIT1, LGALS3, HI95, hsp47, nrf2, NOX4, NOX1, NOX2 (gp91phox, CYBB), NOX5, DUOX2, NOXO1, NOXA1, NOXA2 (p67phox), tissue inhibitor of metalloproteinase 1, or tissue inhibitor of metalloproteinase 2 (TIMP1 and TIMP2, respectively). In some embodiments, the biologic modulates expression of a gene selected from the group consisting of ABAT; ADRB1; ADRB3; ARHGEF9; ARRB1; ATP1A1; CACNB4; CAMK2A; CAMK2D; CBLN1; CDH22; CDK5R1; CHN1; CTSD; DDN; DRD3; DUSP6; ENPP1; ENPP2; EPHA4; GABRA1; GMFG; GPM6A; GPNMB; GPR23; HAPLN4; IGF2; IGFBP2; KCNA1; KIF5A; MAPK10; MEF2C; NAPB; NOS1; NPTX2; NRGN; NTS; NUCB1; PCP4; PDCD2; PDE4D; PENK; PHCA; PJA2; PLP1; PMCH; PVALB; QDPR; RPN1; SLC17A7; SLC28A2; SLC8A1; SNAP91; SYN2; SYT1; TKT; TPT1; UGT8 and VIP. In some embodiments, the nucleic acid is an oligonucleotide or analog thereof (for example, short interfering nucleic acid (siNA), short interfering RNA (siRNA), double-stranded RNA (dsRNA), micro-RNA (miRNA), or short hairpin RNA (shRNA)). In some embodiments, the biologic is selected from an iRNA or oligonucleotide or analog thereof disclosed in US 9,446,062, US 9,222,087, US 8,017,764, US 7,626,015, US 2013/0303590, US 2012/0108647, US 2010/0168204, EP2026843, WO 2007/141796, EP2350279, US 9,121,020, US 8,765,931, US 2015/0361430, US 2015/0050328, EP2350279, WO 2010/048352, US 2016/0102313, US 9,045,755, US 2013/0137750, EP2585594, WO 2011/163436, US 9,056,903, US 8,067,570, US 2012/0156208, EP2402443, EP1984003, US 2015/0065559, US 8,901,097, WO 2011/057171, US 8,785,408, EP2170403, WO 2009/001359, US 2015/0152412, US 8,796,239, US 2011/0178157, EP2509991, EP2504435, WO 2011/072082, WO 2011/066475, EP2862929, US 8,778,904, EP2510098, EP2510098, WO 2011/072091, US 2014/0350068, US 8,614,311, US 2013/0131143, WO 2009/074990, EP2411413, US 8,444,983, US 2015/0359905, US 2014/0072552, EP2411413, WO 2010/111198, US 8,410,069, US 7,812,002, US 2011/0230543, EP2136847, WO 2008/114262, US 2015/0329866, US 8,404,654, US 7,910,566, US 7,825,099, US 2013/0190387, US 2010/0029746, EP2371958, EP2076526, WO 2008/050329, US 8,278,287, EP2285385, WO 2009/144704, US 8,198,258, US 7,939,652, US 2011/0201670, EP1758998, EP2330111, EP1758998, US 8,034,902, EP1885396, US 2011/0098337, US 7,872,119, EP2137205, WO 2008/106102, US 2015/0259676, WO 2014/043291, US 2015/0018404, EP2739637, WO 2013/020097, WO 2013/020097, EP2649181, US 2013/0324591, EP2649181, WO 2012/078536, US 2013/0267578, US 2012/0136044, WO 2008/152636, US 2013/0030034, US 2012/0142754, WO 2012/044620, US 2009/0202566, EP1624788, EP1933880, WO 2012/170957, WO 2010/080452, WO 2010/046889, or WO 2008/020435, the disclosure of each of which is hereby incorporated by reference.

In some embodiments, the biologic is useful in treating, preventing, or ameliorating a cancerous disease or hyperproliferative disease or disorder, such as a lung cancer. In some embodiments, the biologic modulates expression of Nrf2. In some embodiments, the biologic is selected from an iRNA or oligonucleotide or analog thereof disclosed in US 8,410,069, US 7,812,002, or US 2011/0230543, the disclosure of each of which is hereby incorporated by reference.

In some embodiments, the biologic is useful in treating, preventing, or ameliorating microvascular disorders, eye diseases, hearing impairment, neurodegenerative diseases and disorders, spinal cord injury, respiratory conditions, or a CNS disease. In some embodiments, the biologic modulates expression of RTP801 or a human p53 gene. In some embodiments, the biologic is selected from an iRNA or oligonucleotide or analog thereof disclosed in US 9,446,062,

EP1799269, US 9,334,499, US 9,006,196, US 8,765,699, US 8,148,342, US 7,842,674, US 2015/0141487, US 2012/0184597, US 2008/0287382, EP1799269, US 9,222,087, US 8,017,764, US 7,626,015, US 2013/0303590, US 2012/0108647, US 2010/0168204, EP2026843, WO 2007/141796, EP2350279, US 9,121,020, US 8,765,931, US 2015/0361430, US 2015/0050328, EP2350279, WO 2010/048352, US 9,089,591, US 8,431,692, US 2014/0066493, EP2293800, WO 2009/147684, US 9,056,903, US 8,067,570, US 2012/0156208, EP2402443, EP1984003, US 2015/0065559, US 8,901,097, WO 2011/057171, US 2015/0152412, US 8,796,239, EP2504435, WO 2011/066475, EP2862929, US 8,778,904, EP2510098, WO 2011/072091, US 8,642,571, US 8,309,532, US 8,168,607, US 7,741,299, US 2013/0095117, US 2011/0117102, US 2011/0028532, EP2319925, EP1791568, US 8,614,309, US 2013/0123334, EP2231168, WO 2009/044392, US 2014/0350068, US 8,614,311, US 2013/0131143, WO 2009/074990, US 2015/0329866, US 8,404,654, US 7,910,566, US 7,825,099, US 2013/0190387, US 2011/0251260, US 2010/0029746, EP2371958, EP2076526, WO 2008/050329, US 8,362,229, EP1989307, WO 2007/091269, US 8,344,104, US 8,034,575, US 7,723,052, US 2012/0034599, US 2011/0045499, WO 2008/054534, WO 2008/054534, WO 2008/001361, US 7,973,156, US 2011/0098337, US 7,872,119, EP2137205, WO 2008/106102, US 2015/0259676, WO 2014/043291, US 2015/0267194, EP2895607, WO 2014/043289, US 2014/0323549, EP2776565, WO 2013/070821, EP2649181, US 2013/0324591, WO 2012/078536, EP2268316, WO 2009/116037, EP2152316, or WO 2008/132723, the disclosure of each of which is hereby incorporated by reference.

In some embodiments, the biologic is useful in treating, preventing, or ameliorating microvascular disorders, eye diseases, respiratory conditions and hearing problems, or a disease of the CNS. In some embodiments, the biologic modulates expression of RTP801 or a human p53 gene. In some embodiments, the biologic is selected from an iRNA or oligonucleotide or analog thereof disclosed in US 9,056,903, US 2012/0156208, EP2402443, EP1984003, US 8,642,571, US 8,309,532, US 2013/0095117, US 2011/0117102, EP2319925, EP1791568, US 8,344,104, US 8,034,575, US 7,723,052, US 2012/0034599, US 2011/0045499, WO 2008/054534, WO 2008/001361, US 2015/0259676, WO 2014/043291, US 2014/0323549, EP2776565, WO 2013/070821, EP2268316, WO 2009/116037, EP2152316, WO 2008/132723, EP2137205, WO 2008/106102, or EP1799269, the disclosure of each of which is hereby incorporated by reference.

In some embodiments, the biologic is useful in treating, preventing, or ameliorating lung diseases, disorders and injury in a mammal, including treatment of acute respiratory distress syndrome (ARDS), acute lung injury, pulmonary fibrosis (idiopathic), bleomycin induced pulmonary fibrosis, mechanical ventilator induced lung injury, chronic obstructive pulmonary disease (COPD), chronic bronchitis, emphysema, bronchiolitis obliterans after lung transplantation and lung transplantation-induced acute graft dysfunction, including treatment, prevention or prevention of progression of primary graft failure, ischemia-reperfusion injury, reperfusion injury, reperfusion edema, allograft dysfunction, pulmonary reimplantation response, bronchiolitis obliterans after lung transplantation and/or primary graft dysfunction (PGD). In some embodiments, the biologic modulates expression of TLR2 or TLR4. In some embodiments, the biologic is selected from an iRNA or oligonucleotide or analog thereof disclosed in US 2016/0215284, US 9,205,100, US 2014/0005253, EP2681314, WO 2012/118910, US 2015/0152412, US 8,796,239, EP2504435, WO 2011/066475, US 2015/0259676, WO 2014/043291, EP2649181, US 2013/0324591, EP2649181, WO 2012/078536, EP2681315, WO 2012/118911, or WO 2010/080452, the disclosure of each of which is hereby incorporated by reference.

In some embodiments, the biologic is useful in treating, preventing, or ameliorating a disease, disorder, or condition associated with p53 such as an injury or disorder of the CNS, a hearing disorder, a hearing loss and/or a balance impairment, or chronic kidney disease. In some embodiments, the biologic modulates expression of RTP801, HES1, HES5, HEY1, HEY2, ID1, ID2, ID3, CDKN1B, or NOTCH1. In some embodiments, the biologic is selected from an iRNA or oligonucleotide or analog thereof disclosed in US 9,446,062, US 9,434,946, US 2015/0126586, US 2015/0018404, US 2014/0364484, EP2802657, EP2739637, WO 2013/020097, WO 2013/106494, WO 2013/020097, EP1799269, US 9,334,499, US 9,006,196, US 8,765,699, US 8,148,342, US 7,842,674, US 2015/0141487, US 2012/0184597, US 2008/0287382, EP1799269, US 9,222,087, US 8,017,764, US 7,626,015, US 2013/0303590, US 2012/0108647, US 2010/0168204, EP2026843, WO 2007/141796, EP2350279, US 9,121,020, US 8,765,931, US 2015/0361430, US 2015/0050328, EP2350279, WO 2010/048352, US 9,089,591, US 8,431,692, US 2014/0066493, EP2293800, WO 200/9147684, US 9,056,903, US 8,067,570, US 2012/0156208, EP2402443, EP1984003, US 2015/0065559, US 8,901,097, WO 2011/057171, US 8,785,408, EP2170403, WO 2009/001359, US 2015/0152412, US 8,796,239, EP2504435, WO 2011/066475, EP2862929, US 8,778,904, EP2510098, WO 2011/072091, US 2014/0140922, US 8,637,482, EP2440214, WO 2010/144336, US 8,642,571, US 8,309,532, US 8,168,607, US 7,741,299, US 2013/0095117, US 2011/0117102, US 2011/0028532, EP1791568, EP2319925, US 8,614,309, US 2013/0123334, EP2231168, WO 2009/044392, US 2014/0350068, US 8,614,311, US 2013/0131143, WO 2009/074990, EP2411413, US 8,444,983, US 2015/0359905, US 2014/0072552, US 2015/0329866, US 8,404,654, US 7,910,566, US 7,825,099, US 2013/0190387, US 2011/0251260, US 2010/0029746, EP2371958, EP2076526, WO 2008/050329, US 8,362,229, EP1989307, WO 2007/091269, US 8,344,104, US 8,034,575, US 7,723,052, US 2012/0034599, US 2011/0045499, WO 2008/054534, WO 2008/054534, WO 2008/001361, US 7,973,156, US 7,524,935, US 2009/0264634, EP1115733, US 2011/0098337, US 7,872,119, EP2137205, WO 2008/106102, US 2015/0259676, WO

2014/043291, US 2015/0203845, EP2895608, WO 2014/043292, EP2649181, US 2013/0324591, WO 2012/078536, US 2013/0267578, US 2012/0136044, WO 2008/152636, EP2268316, WO 2009/116037, EP2242854, WO 2009/090639, EP2152316, WO 2008/132723, EP1933880, WO 2015/183842, WO 2012/044620, WO 2010/080452, WO 2010/046889, WO 2008/126085, or WO 2008/020435, the disclosure of each of which is hereby incorporated by reference.

In some embodiments, the biologic is useful in treating, preventing, or ameliorating a disease, disorder, or condition associated with p53 such as an injury or disorder of the CNS, a hearing disorder, a hearing loss, a balance impairment, alopecia or acute renal failure, or chronic kidney disease. In some embodiments, the biologic modulates expression of RTP801, TP53, HTRA2, KEAP1, SHC1-SHC, ZNHIT1, LGALS3, or HI95. In some embodiments, the biologic is selected from an iRNA or oligonucleotide or analog thereof disclosed in EP1799269, US 9,334,499, US 9,006,196, US 8,765,699, US 7,842,674, US 2015/0141487, US 2012/0184597, US 2008/0287382, EP1799269, US 9,089,591, US 2014/0066493, US 8,785,408, US 8,778,904, US 2014/0140922, US 8,637,482, EP2440214, WO 2010/144336, US 2013/0190387, US 8,404,654, US 7,910,566, US 7,825,099, US 2010/0029746, US 2015/0259676, WO 2014/043291, US 2015/0203845, EP2895608, WO 2014/043292, EP2152316, WO 2008/132723, or WO 2015/183842, the disclosure of each of which is hereby incorporated by reference.

In some embodiments, the biologic is useful in treating, preventing, or ameliorating an ear disorder, including hearing loss arising from chemical-induced ototoxicity, acoustic trauma and presbycusis and microbial infections. In some embodiments, the biologic is selected from an iRNA or oligonucleotide or analog thereof disclosed in US 9,089,591, US 8,431,692, US 2014/0066493, EP2293800, or WO 2009/147684, the disclosure of each of which is hereby incorporated by reference.

In some embodiments, the biologic is useful in treating, preventing, or ameliorating of ocular disease, disorder, or injury. In some embodiments, the biologic modulates expression of CASP2 or DDIT4. In some embodiments, the biologic is selected from an iRNA or oligonucleotide or analog thereof disclosed in US 9,382,542, US 2014/0371439, EP2800812, WO 2013/103632, EP2862929, US 8,778,904, EP2510098, WO 2011/072091, US 2015/0267194, EP2895607, or WO 2014/043289, the disclosure of each of which is hereby incorporated by reference.

In some embodiments, the biologic is useful in treating, preventing, or ameliorating of ocular disease, disorder, or injury such as non-arteritic anterior ischemic optic neuropathy (NAION). In some embodiments, the biologic modulates expression of CASP2. In some embodiments, the biologic is selected from an iRNA or oligonucleotide or analog thereof disclosed in US 9,382,542, US 2014/0371439, EP2800812, WO 2013/103632, or US 8,778,904, the disclosure of each of which is hereby incorporated by reference.

In some embodiments, the biologic is useful in treating, preventing, or ameliorating a disease, disorder, or condition such as ischemia-reperfusion injury, alopecia, renal failure, glaucoma, ischemic optic neuropathy (ION), or Meniere's disease. In some embodiments, the biologic modulates expression of ABAT, ADRB1, ADRB3, ARHGEF9, ARRB1, ATP1A1, CACNB4, CAMK2A, CAMK2D, CBLN1, CDH22, CDK5R1, CHN1, CTSD, DDN, DRD3, DUSP6, ENPP1, ENPP2, EPHA4, GABRA1, GMFG, GPM6A, GPNMB, GPR23, HAPLN4, IGF2, IGFBP2, KCNA1, KIF5A, MAPK10, MEF2C, NAPB, NOS1, NPTX2, NRGN, NTS, NUCB1, PCP4, PDCD2, PDE4D, PENK, PHCA, PJA2, PLP1, PMCH, PVALB, QDPR, RPN1, SLC17A7, SLC28A2, SLC8A1, SNAP91, SYN2, SYT1, TKT, TPT1, UGT8, VIP, NOX4, NOX1, NOX2 (gp91phox, CYBB), NOX5, DUOX2, NOXO1, NOXA1, NOXA2 (p67phox), SOX9, ASPP1, CTSD, CAPNS1, FAS, or FAS ligand. In some embodiments, the biologic is selected from an iRNA or oligonucleotide or analog thereof disclosed in US 9,446,062, EP1799269, US 9,334,499, US 9,006,196, US 8,765,699, US 8,148,342, US 7,842,674, US 2015/0141487, US 2012/0184597, US 2008/0287382, EP1799269, US 9,222,087, US 8,017,764, US 7,626,015, US 2013/0303590, US 2012/0108647, US 2010/0168204, EP2026843, WO 2007/141796, US 2016/0102313, US 9,045,755, US 2013/0137750, EP2585594, WO 2011/163436, US 9,056,903, US 8,067,570, US 2012/0156208, EP2402443, EP1984003, US 8,785,408, EP2170403, WO 2009/001359, US 8,642,571, US 8,309,532, US 8,168,607, US 7,741,299, US 2013/0095117, US 2011/0117102, US 2011/0028532, EP1791568, EP2319925, EP1791568, US 2014/0350068, US 8,614,311, US 2013/0131143, WO 2009/074990, EP2411413, US 8,444,983, US 2015/0359905, US 2014/0072552, EP2411413, WO 2010/111198, US 7,973,156, US 7,524,935, US 2009/0264634, EP1115733, US 2015/0329866, US 7,910,566, EP2371958, EP2076526, WO 2008/050329, US 2011/0098337, US 7,872,119, EP2137205, WO 2008/106102, US 2015/0259676, WO 2014/043291, US 2015/0203845, EP2895608, WO 2014/043292, US 2013/0267578, US 2012/0136044, WO 2008/152636, US 2013/0030034, US 2012/0142754, WO 2012/044620, EP2509991, US 2011/0178157, WO 2011/072082, EP2268316, WO 2009/116037, EP2242854, WO 2009/090639, EP1933880, EP1753464, WO 2012/170957, WO 2010/080452, or WO 2008/020435, the disclosure of each of which is hereby incorporated by reference.

In some embodiments, the biologic is useful in treating, preventing, or ameliorating a disease, disorder, or condition associated with hearing loss, or a microvascular disorder, eye disease, or respiratory condition, such as tinnitus and Meniere's disease. In some embodiments, the biologic modulates expression of RTP801, RhoA, HES1, HES5, HEY2, CDKN1B, or NOTCH1. In some embodiments, the biologic is selected from an iRNA or oligonucleotide or analog thereof disclosed in US 9,434,946, US 2015/0126586, US 2015/0018404, US 2014/0364484, EP2802657, EP2739637,

WO 2013/020097, WO 2013/106494, WO 2013/020097, US 9,422,560, US 2015/0031746, EP2773758, WO 2013/067076, US 9,222,087, US 8,017,764, US 7,626,015, US 2013/0303590, US 2012/0108647, US 2010/0168204, EP2026843, WO 2007/141796, US 2016/0102313, US 9,045,755, US 2013/0137750, EP2585594, WO 2011/163436, US 9,056,903, US 8,067,570, US 2012/0156208, EP2402443, EP1984003, US 8,785,408, EP2170403, WO 2009/001359, US 2014/0350068, US 8,614,311, US 2013/0131143, WO 2009/074990, US 2015/0329866, US 8,404,654, US 7,910,566, US 7,825,099, US 2013/0190387, US 2010/0029746, EP2371958, EP2076526, WO 2008/050329, US 2011/0098337, US 7,872,119, EP2137205, WO 2008/106102, EP2649181, US 2013/0324591, EP2649181, WO 2012/078536, US 2013/0267578, US 2012/0136044, WO 2008/152636, EP2242854, WO 2009/090639, or WO 2010/080452, the disclosure of each of which is hereby incorporated by reference.

In some embodiments, the biologic is useful in treating, preventing, or ameliorating an inner ear disease or disease, disorder, or condition such as hearing loss, acute renal failure (ARF), Delayed Graft Function (DGF) after kidney transplantation, glaucoma, ocular ischemic conditions, including non-arteric ischemic optic neuropathy (NAION), anterior ischemic optic neuropathy, age-related macular degeneration (AMD), Ischemic Optic Neuropathy (ION) and dry eye syndrome, acute respiratory distress syndrome (ARDS) and other acute lung and respiratory injuries, chronic obstructive pulmonary disease (COPD), primary graft failure, ischemia-reperfusion injury, reperfusion injury, reperfusion edema, allograft dysfunction, pulmonary reimplantation response and/or primary graft dysfunction (PGD) after organ transplantation, in particular in lung transplantation, organ transplantation including lung, liver, heart, pancreas, and kidney transplantation, nephro- and neurotoxicity, spinal cord injury, brain injury, neurodegenerative disease or condition, pressure sores, oral mucositis, fibrotic disorders, cancer, or Meniere's disease. In some embodiments, the biologic modulates expression of RhoA or Caspase 2, Apoptosis-Related Cysteine Peptidase (CASP2) gene. In some embodiments, the biologic is selected from an iRNA or oligonucleotide or analog thereof disclosed in US 9,422,560, US 2015/0031746, EP2773758, WO 2013/067076, US 8,404,654, US 2016/0102313, US 2013/0137750, EP2585594, WO 2011/163436, EP2649181, US 2013/0324591, EP2649181, or WO 2012/078536, the disclosure of each of which is hereby incorporated by reference.

In some embodiments, the biologic is useful in treating, preventing, or ameliorating microvascular disorders, eye diseases and respiratory conditions, or transplant rejection conditions. In some embodiments, the biologic modulates expression of RTP801. In some embodiments, the biologic is selected from an iRNA or oligonucleotide or analog thereof disclosed in EP1791568, US 8,168,607, US 7,741,299, US 2011/0117102, US 2011/0028532, EP2319925, EP1791568, US 2012/0156208, US 8,067,570, EP2402443, or EP1984003, the disclosure of each of which is hereby incorporated by reference.

In some embodiments, the biologic is useful in treating, preventing, or ameliorating an ear disorder such as hearing loss, balance impairment, or promoting the replacement, regeneration, or protection of otic (sensory) hair cells of the inner ear, or effecting hearing restoration or regeneration. In some embodiments, the biologic modulates expression of a gene associated with hearing loss, for example p53, HES1, HES5, HEY2, CDKN1B, or NOTCH1. In some embodiments, the biologic is selected from an iRNA or oligonucleotide or analog thereof disclosed in US 9,434,946, US 2015/0126586, US 2015/0018404, US 2014/0364484, EP2802657, EP2739637, WO 2013/020097, WO 2013/106494, US 9,089,591, US 8,431,692, US 2014/0066493, EP2293800, WO 2009/147684, US 2015/0152412, US 8,796,239, EP2504435, WO 2011/066475, US 2015/0203845, EP2895608, or WO 2014/043292, the disclosure of each of which is hereby incorporated by reference.

In some embodiments, the biologic is useful in treating, preventing, or ameliorating microvascular disorders, eye diseases and respiratory conditions such as diabetic macular edema. In some embodiments, the biologic modulates expression of RTP801. In some embodiments, the biologic is selected from an iRNA or oligonucleotide or analog thereof disclosed in EP1791568, US 8,168,607, US 2011/0117102, US 2011/0028532, EP2319925, or EP2402443, the disclosure of each of which is hereby incorporated by reference, optionally in combination with a VEGF inhibitor or VEGF-Receptorl inhibitor.

In some embodiments, the biologic is useful in treating, preventing, or ameliorating microvascular disorders, eye diseases and respiratory conditions such as diabetic macular degeneration. In some embodiments, the biologic modulates expression of RTP801. In some embodiments, the biologic is selected from an iRNA or oligonucleotide or analog thereof disclosed in EP1791568, US 8,168,607, US 7,741,299, US 2011/0117102, US 2011/0028532, EP2319925, EP1791568, US 2012/0156208, US 8,067,570, EP2402443, or EP1984003, the disclosure of each of which is hereby incorporated by reference, optionally in combination with a VEGF inhibitor or VEGF-Receptorl inhibitor.

In some embodiments, the biologic is useful in treating, preventing, or ameliorating a disease, disorder, or condition such as delayed graft function (DGF) or ischemia reperfusion injury (IRI) in organs. In some embodiments, the biologic modulates expression of p53, RTP801, TP53, HTRA2, KEAP1, SHC1-SHC, ZNHIT1, LGALS3, or HI95. In some embodiments, the biologic is selected from an iRNA or oligonucleotide or analog thereof disclosed in US 9,446,062, US 9,249,414, US 8,859,751, US 2015/0159154, EP2521783, WO 2011/084193, WO 2011/085056, US 8,785,408, EP2170403, EP2170403, WO 2009/001359, US 2015/0152412, US 8,796,239, EP2504435, WO 2011/066475, US 8,614,309, US 2013/0123334, EP2231168, WO 2009/044392, US 2011/0098337, US 7,872,119, EP2137205, WO

2008/106102, US 2015/0329866, US 2011/0251260, EP2371958, EP2371958, EP2076526, WO 2008/050329, US 2015/0259676, WO 2014/043291, US 2015/0203845, EP2895608, WO 2014/043292, EP2649181, US 2013/0324591, EP2649181, WO 2012/078536, EP2268316, WO 2009/116037, WO 2015/183842, or WO 2010/080452, the disclosure of each of which is hereby incorporated by reference.

In some embodiments, the biologic is useful in treating, preventing, or ameliorating a disease, disorder, or condition such as delayed graft function (DGF) or ischemia reperfusion injury (IRI) in organs. In some embodiments, the biologic modulates expression of p53, RTP801, TP53, HTRA2, KEAP1, SHC1-SHC, ZNHIT1, LGALS3, or HI95. In some embodiments, the biologic is selected from an iRNA or oligonucleotide or analog thereof disclosed in US 8,785,408, EP2170403, EP2170403, WO 2009/001359, US 2015/0152412, US 8,796,239, EP2504435, WO 2011/066475, US 2015/0203845, EP2895608, WO 2014/043292, EP2649181, US 2013/0324591, EP2649181, WO 2012/078536, or WO 2015/183842, the disclosure of each of which is hereby incorporated by reference.

In some embodiments, the biologic is useful in treating, preventing, or ameliorating lung diseases, disorders and injury in a mammal, including treatment of acute respiratory distress syndrome (ARDS), acute lung injury, pulmonary fibrosis (idiopathic), bleomycin induced pulmonary fibrosis, mechanical ventilator induced lung injury, chronic obstructive pulmonary disease (COPD), chronic bronchitis, emphysema, bronchiolitis obliterans after lung transplantation and lung transplantation-induced acute graft dysfunction, or DGF. In some embodiments, the biologic modulates expression of RTP801, TLR2, TP53, HTRA2, KEAP1, SHC1-SHC, ZNHIT1, LGALS3, or HI95. In some embodiments, the biologic is selected from an iRNA or oligonucleotide or analog thereof disclosed in US 9,446,062, US 9,249,414, US 8,859,751, US 2015/0159154, EP2521783, WO 2011/084193, WO 2011/085056, US 2016/0215284, US 9,205,100, US 2014/0005253, EP2681314, WO 2012/118910, US 9,222,087, US 8,017,764, US 7,626,015, US 2013/0303590, US 2012/0108647, US 2010/0168204, EP2026843, WO 2007/141796, US 9,056,903, US 8,067,570, US 2012/0156208, EP2402443, EP1984003, US 8,785,408, EP2170403, EP2170403, WO 2009/001359, US 2015/0152412, US 8,796,239, EP2504435, WO 2011/066475, US 8,642,571, US 8,309,532, US 8,168,607, US 7,741,299, US 2013/0095117, US 2011/0117102, US 2011/0028532, EP1791568, EP2319925, EP1791568, US 8,614,309, US 2013/0123334, EP2231168, WO 2009/044392, US 2014/0350068, US 8,614,311, US 2013/0131143, WO 2009/074990, US 8,362,229, EP1989307, EP1989307, WO 2007/091269, US 8,344,104, US 8,034,575, US 7,723,052, US 2012/0034599, US 2011/0045499, WO 2008/054534, WO 2008/054534, US 2011/0098337, US 7,872,119, EP2137205, WO 2008/106102, US 2015/0329866, US 2011/0251260, EP2371958, EP2371958, EP2076526, WO 2008/050329, US 2015/0259676, WO 2014/043291, US 2015/0267194, EP2895607, WO 2014/043289, EP2649181, US 2013/0324591, EP2649181, WO 2012/078536, US 2013/0267578, US 2012/0136044, WO 2008/152636, EP2681315, WO 2012/118911, EP2268316, WO 2009/116037, EP2242854, WO 2009/090639, EP2152316, WO 2008/132723, WO 2010/080452, WO 2008/104978, or WO 2008/020435, the disclosure of each of which is hereby incorporated by reference.

In some embodiments, the biologic is useful in treating, preventing, or ameliorating lung diseases, disorders or injury in a mammal, including treatment of acute respiratory distress syndrome (ARDS), acute lung injury, pulmonary fibrosis (idiopathic), bleomycin induced pulmonary fibrosis, mechanical ventilator induced lung injury, chronic obstructive pulmonary disease (COPD), chronic bronchitis, emphysema, bronchiolitis obliterans after lung transplantation and lung transplantation-induced acute graft dysfunction, or DGF. In some embodiments, the biologic modulates expression of RTP801, TLR2, TP53, HTRA2, KEAP1, SHC1-SHC, ZNHIT1, LGALS3, or HI95. In some embodiments, the biologic is selected from an iRNA or oligonucleotide or analog thereof disclosed in US 2015/0152412, US 8,796,239, EP2504435, WO 2011/066475, US 8,642,571, EP1791568, EP2319925, US 2015/0259676, WO 2014/043291, EP2681314, US 2014/0005253, WO 2012/118910, EP2649181, US 2013/0324591, EP2649181, WO 2012/078536, US 2013/0267578, US 2012/0136044, WO 2008/152636, US 2011/0098337, EP2402443, EP2371958, EP2076526, WO 2008/050329, EP2231168, WO 2009/044392, EP2242854, or WO 2009/090639, the disclosure of each of which is hereby incorporated by reference.

In some embodiments, the biologic is useful in treating, preventing, or ameliorating inner ear diseases and disorders, including tinnitus, non-arteritic anterior ischemic optic neuropathy (NAION), and Meniere's disease, and increasing neuroprotection to neurons in the inner ear. In some embodiments, the biologic modulates expression of p53, RTP801, or CASP2. In some embodiments, the biologic is selected from an iRNA or oligonucleotide or analog thereof disclosed in US 9,446,062, US 9,422,560, US 2015/0031746, EP2773758, WO 2013/067076, US 9,382,542, US 2014/0371439, EP2800812, WO 2013/103632, US 9,334,499, US 9,006,196, US 8,765,699, US 8,148,342, US 7,842,674, US 2015/0141487, US 2012/0184597, US 2008/0287382, EP1799269, EP2350279, US 9,121,020, US 8,765,931, US 2015/0361430, US 2015/0050328, EP2350279, WO 2010/048352, US 9,089,591, US 2014/0066493, US 9,056,903, US 8,067,570, US 2012/0156208, EP2402443, EP1984003, US 2015/0065559, US 8,901,097, WO 2011/057171, US 2015/0152412, US 8,796,239, EP2504435, WO 2011/066475, EP2862929, US 8,778,904, EP2510098, EP2510098, WO 2011/072091, US 2014/0140922, US 8,637,482, EP2440214, WO 2010/144336, US 8,614,309, US 2013/0123334, EP2231168, WO 2009/044392, US 2015/0329866, US 8,404,654, US 7,910,566, US 7,825,099, US 2013/0190387, US 2011/0251260, US 2010/0029746, EP2371958, EP2371958, EP2076526, WO 2008/050329, US 2011/0098337, US 7,872,119, EP2137205, WO 2008/106102, US 2015/0259676, WO 2014/043291,

US 2014/0323549, EP2776565, WO 2013/070821, EP2649181, US 2013/0324591, EP2649181, WO 2012/078536, EP2152316, or WO 2008/132723, the disclosure of each of which is hereby incorporated by reference.

In some embodiments, the biologic is useful in treating, preventing, or ameliorating a disease, disorder, or condition such as alopecia or renal failure. In some embodiments, the biologic modulates expression of a human p53 gene. In some embodiments, the biologic is selected from an iRNA or oligonucleotide or analog thereof disclosed in EP1799269, US 9,334,499, US 9,006,196, US 8,765,699, US 8,148,342, US 7,842,674, US 2015/0141487, US 2012/0184597, US 2008/0287382, EP1799269, US 2013/0190387, US 8,404,654, US 7,910,566, US 7,825,099, US 2010/0029746, US 2015/0203845, EP2895608, or WO 2014/043292, the disclosure of each of which is hereby incorporated by reference.

In some embodiments, the biologic is useful in treating, preventing, or ameliorating neuropathic pain, inflammation, primary graft dysfunction (PGD) after lung transplantation, spinal cord injury, or allodynia. In some embodiments, the biologic modulates expression of RhoA or the toll-like receptor pathway. In some embodiments, the biologic is selected from an iRNA or oligonucleotide or analog thereof disclosed in US 2016/0102313, US 9,045,755, US 2013/0137750, EP2585594, WO 2011/163436, US 2015/0065559, US 8,901,097, WO 2011/057171, EP2681315, WO 2012/118911, or WO 2008/020435, the disclosure of each of which is hereby incorporated by reference.

In some embodiments, the biologic is useful in treating, preventing, or ameliorating a disease associated with smad7. In some embodiments, the biologic modulates expression of smad7. In some embodiments, the biologic is selected from an iRNA or oligonucleotide or analog thereof disclosed in US 2015/0211006, the disclosure of which is hereby incorporated by reference.

In some embodiments, the biologic is useful in treating, preventing, or ameliorating a disease, disorder, or condition such as myotonic dystrophy, Huntington's disease, or HTT. In some embodiments, the biologic modulates expression of a dystrophin gene such as DMPK. In some embodiments, the biologic is selected from an iRNA or oligonucleotide or analog thereof disclosed in US 2015/0211006, EP2872147, or WO 2015/107425, the disclosure of each of which is hereby incorporated by reference.

In some embodiments, the biologic is useful in treating, preventing, or ameliorating of a disease, disorder, or condition associated with a dystrophin gene such as DMPK. In some embodiments, the biologic modulates expression of a dystrophin gene such as DMPK. In some embodiments, the biologic is selected from an iRNA or oligonucleotide or analog thereof disclosed in US 2015/0166999, EP2873674, or WO 2015/107425, the disclosure of each of which is hereby incorporated by reference.

In some embodiments, the biologic is useful in treating, preventing, or ameliorating a disease, disorder, or condition such as cancer, malignant blood disease (leukemia), inflammatory diseases or conditions, allergic diseases or conditions, or proliferative diseases or conditions, psoriasis, eczema, dermatitis, Crohn's disease, asthma, COPD, allergic rhinitis, and inflammatory bowel disease, cancer, proliferative diseases or conditions, inflammatory diseases or conditions, allergic diseases or conditions, infectious diseases or conditions, autoimmune diseases or conditions, or transplantation/allograft rejection, hearing loss, deafness, tinnitus, motion and balance disorders, or AMD. In some embodiments, the biologic modulates expression of XIAP, checkpoint kinase (e.g., checkpoint kinase-1 or CHK-1), HIF-1, vascular adhesion molecule (e.g. VCAM-1), matrix metalloproteinase (e.g., MMP13), GRB2 associated binding protein (GAB2), intercellular adhesion molecule (ICAM), STAT3, stromal cell-derived factor-1 (SDF-1), a cyclin kinase, an interleukin, BCL2, ADAM33, BCR-ABL, ERG, EWS-ERG, TEL-AML1, EWS-FLI1, TLS-FUS, PAX3-FKHR, and/or AML1-ETO, wingless (WNT), c-Fos, stromal cell-derived factor- 1 (SDF-I), retinoblastoma (RBI), HDAC, B7-H1, VEGF, early growth response (Egr-1), placental growth factor (e.g., PGF-1 or PIGF-1, PGF-2 or PIGF-2, and/or PGF-3 or PIGF-3), polycomb group protein EZH2, Angiopoietin, c-Fos, TGF-beta and/or TGF-betaR, mitogen activated protein kinase (MAP kinase), retinoblastoma (RB1), tumor necrosis factor and/or tumor necrosis factor receptor, RAS, myostatin, platelet derived growth factor (PDGF) and/or platelet derived growth factor receptor (PDGFr), platelet-derived endothelial cell growth factor, or receptor (ECGF1 and/or ECGF1r) genes. In some embodiments, the biologic is selected from an iRNA or oligonucleotide or analog thereof or other biologic disclosed in US 9,260,471, EP2632472, EP1931781, US 7,910,725, US 7,897,755, US 7,893,302, US 7,910,724, US 7,897,753, US 7,897,752, US 7,855,284, US 7,795,422, US 7,700,760, US 7,678,897, US 7,691,405, US 7,683,165, US 7,667,030, US 7,667,029, US 7,662,952, US 7,683,166, US 7,641,915, US 7,517,864, US 7,514,099, US 7,404,969, US 2016/0152973, US 2011/0160281, US 2010/0184824, US 2010/0144851, US 2010/0099744, US 2010/0130592, US 2010/0099743, US 2009/0306182, US 2009/0299045, US 2009/0247613, US 2009/0253773, US 2009/0253774, US 20090192105, US 2009/0156533, US 2009/0192104, US 2009/0176725, US 2009/0149408, US 2009/0137512, US 2009/0137511, US 2009/0137509, US 2009/0137508, US 2009/0105178, US 2009/0143324, US 2009/0137510, US 2009/0137507, US 2009/0143325, US 2009/0093438, US 2009/0099119, US 2009/0099116, US2009/0093439, US 2009/0093437, US 2009/0093436, US 2009/0093435, US 2009/0048197, US 2008/0249294, US 2008/0188430, US 2008/0188675, US 2008/0161256, US 2008/0020058, US 2007/0270579, US 2007/0185049, US 2007/0160980, US 2007/0093437, US 2007/0042983, US 2007/0032441, US 2006/0240554, US 2006/0217332, US 2006/0217331, US 2006/0216747, US 2006/0142225, US 2005/0282188, US 2006/0019917, US 2005/0288242, US 2005/0287128, US 2005/0239731, US 2005/0233998, US 2005/0261219, US

2005/0266422, US 2005/0267058, US2005/0260620, US 2005/0233997, US 2005/0233344, US 2005/0227935, US 2005/0196767, US 2005/0196781, US 2005/0222066, US 2005/0227936, US 2005/0203040, US 2005/0196765, US 2005/0187174, US 2005/0143333, US 2005/0148530, US 2005/0182007, US 2005/0153916, US 2005/0153915, US 2005/0182009, US 2005/0182006, US 2005/0176663, US 2005/0176025, US 2005/0176024, US 2005/0171039, US 2005/0164968, US 2005/0164967, US 2005/0164966, US 2005/0164224, US 2005/0159382, US 2005/0159381, US 2005/0159380, US 2005/0158735, US 2005/0153914, US 2005/0130181, US 2005/0079610, US 2005/0048529, US 2005/0032733, EP1627061, EP1664299, EP2104740, EP1931781, EP1922300, EP1891217, EP1844147, EP1817415, EP1682661, EP1675953, EP1664299, EP1627061, EP1423406, WO 2008/147438, WO 2008/030239, WO 2007/086883, WO 2007/084865, WO 2007/067981, WO 2007/086881, WO 2007/022369, WO 2006/128141, WO 2006/078798, WO 2006/060598, WO 2005/035759, WO 2005/028649, WO 2005/044981, WO 2005/045039, WO 2005/040379, WO 2005/045032, WO 2005/019453, WO 2005/007855, WO 2005/014811, WO 2004/111237, WO 2004/097020, WO 2003/074654, US 2016/0272975, US 2016/0264964, US 2015/0299696, EP2844261, or US 2005/0042632, the disclosure of each of which is hereby incorporated by reference.

In some embodiments, the biologic is useful in treating, preventing, or ameliorating an ocular disease or condition, including age related macular degeneration (AMD) and diabetic retinopathy. In some embodiments, the biologic modulates expression of VEGF or VEGFR. In some embodiments, the biologic is selected from an iRNA or oligonucleotide or analog thereof disclosed in US 7,517,864, US 2006/0217332, or WO 2007/0676981, the disclosure of each of which is hereby incorporated by reference.

In some embodiments, the biologic is useful in treating, preventing, or ameliorating a disease, disorder, or condition such as cancer, malignant blood disease (leukemia), inflammatory diseases or conditions, allergic diseases or conditions, or proliferative diseases or conditions, psoriasis, eczema, dermatitis, Crohn's disease, asthma, COPD, allergic rhinitis, and inflammatory bowel disease, cancer, proliferative diseases or conditions, inflammatory diseases or conditions, allergic diseases or conditions, infectious diseases or conditions, autoimmune diseases or conditions, or transplantation/allograft rejection, hearing loss, deafness, tinnitus, motion and balance disorders, or AMD. In some embodiments, the biologic modulates expression of XIAP, checkpoint kinase (e.g., checkpoint kinase-1 or CHK-1), HIF-1, vascular adhesion molecule (e.g. VCAM-1), matrix metalloproteinase (e.g., MMP13), GRB2 associated binding protein (GAB2), intercellular adhesion molecule (ICAM), STAT3, stromal cell-derived factor-1 (SDF-1), a cyclin kinase, an interleukin, BCL2, ADAM33, BCR-ABL, ERG, EWS-ERG, TEL-AML1, EWS-FLI1, TLS-FUS, PAX3-FKHR, and/or AML1-ETO, wingless (WNT), c-Fos, stromal cell-derived factor- 1 (SDF-I), retinoblastoma (RBI), HDAC, B7-H1, VEGF, early growth response (Egr-1), placental growth factor (e.g., PGF-1 or PIGF-1, PGF-2 or PIGF-2, and/or PGF-3 or PIGF-3), polycomb group protein EZH2, Angiopoietin, c-Fos, TGF-beta and/or TGF-betaR, mitogen activated protein kinase (MAP kinase), retinoblastoma (RB1), tumor necrosis factor and/or tumor necrosis factor receptor, RAS, myostatin, platelet derived growth factor (PDGF) and/or platelet derived growth factor receptor (PDGFr), platelet-derived endothelial cell growth factor, or receptor (ECGF1 and/or ECGF1r) genes. In some embodiments, the biologic is selected from an iRNA or oligonucleotide or analog thereof or other biologic disclosed in US 9,260,471, EP2632472, US 2016/0222381, US 9,243,246, EP2609198, US 2016/0244760, US 9,181,551, US 7,897,755, US 7,893,302, US 7,897,753, US 7,858,769, US 7,795,422, US 7,678,897, US 7,691,405, US 7,683,165, US 7,641,915, US 7,517,864, US 7,514,099, US 7,404,969, US 7,034,009, US 2016/0152973, US 2016/0053269, US 2015/0267200, US 2015/0148530, US 2014/0288148, US 2010/0184824, US 2010/0144851, US 2009/0306182, US 2009/0192105, US 2009/0156533, US 2009/0192104, US 2009/0176725, US 2009/0170197, US 2009/0137511, US 2009/0137500, US 2009/0105178, US 2009/0143324, US 2009/0137507, US 2009/0093438, US 2009/0048197, US 2008/0249294, US 2008/0188430, US 2008/0188675, US 2008/0161256, US 2008/0020058, US 2008/0039412, US 2008/0039414, US 2007/0270579, US 2007/0185049, US 2007/0160980, US 2007/0093437, US 2007/0042983, US 2007/0042029, US 2007/0032441, US 2006/0240554, US 2006/0217332, US 2006/0217331, US 2005/0282188, US 2006/0019917, US 2005/0239731, US 2005/0233998, US 2005/0266422, US 2005/0267058, US 2005/0233329, US 2005/0222066, US 2005/0187174, US 2005/0143333, US 2005/0153915, US 2005/0171039, US 2005/0164967, US 2005/0159380, US 2005/0048529, US 2005/0032733, US 2005/0020525, US 2004/0220128, EP1931781, EP1627061, EP1713915, EP1664299, EP2104740, EP1931781, EP1922300, EP1891217, EP1767632, EP1713915, EP1682661, EP1664299, EP1627061, EP1522583, EP1522583, EP1390385, WO 2008/147438, WO 2008/030239, WO 2007/086883, WO 2007/067981, WO 2007/086881, WO 2007/022369, WO 2006/128141, WO 2005/078097, WO 2005/035759, WO 2005/028649, WO 2005/044981, WO 2005/045039, WO 2005/045032, WO 2005/019453, WO 2005/014811, US 2015/0376613, US 9,096,850, US 2016/0272975, US 2016/0264964, EP3068407, US 2016/0256570, WO 2015/070158, US 2015/0299696, or EP2844261, the disclosure of each of which is hereby incorporated by reference.

In some embodiments, the biologic is useful in treating, preventing, or ameliorating cancer or ocular diseases, including age related macular degeneration (AMD) and diabetic retinopathy. In some embodiments, the biologic modulates expression of VEGF and/or VEGFR. In some embodiments, the biologic is selected from an iRNA or oligonucleotide or analog thereof disclosed in US 7,517,864, US 2007/0042029, US 2006/0217332, or WO 2007/067981, the disclosure of each of which is hereby incorporated by reference.

In some embodiments, the biologic is useful in treating, preventing, or ameliorating pancreatic cancer, glioblastoma, prostate cancer, breast cancer, lung cancer, liver cancer, colon cancer, pancreatic cancer and leukemia, diabetes, obesity, cardiovascular diseases, and metabolic diseases. In some embodiments, the biologic modulates expression of PKN3 or VEGF. In some embodiments, the biologic is selected from an iRNA or oligonucleotide or analog thereof disclosed in US 2016/0130587, US 9,222,092, US 8,933,215, US 8,324,370, US 7,893,245, US 7,452,987, US 2015/0105545, US 2013/0102769, US 2011/0118456, US 2009/0186845, EP1389637, EP1527176, EP2258847, EP1857547, EP1389637, US 2015/0359906, US 9,125,820, US 8,735,453, US 8,017,804, US 2014/0329885, US 2013/0165381, US 2012/0065138, US 2011/0294871, US 2008/0274116, EP1771206, US 2015/0368650, US 9,133,515, US 2014/0179755, EP2849771, WO 2013/170960, US 2014/0249207, US 8,722,875, US 2011/0217367, EP2350278, WO 2010/034487, EP2546337, US 8,232,256, EP2049658, WO 2008/009477, US 2009/0252783, EP2007890, EP2992875, US 2009/0074852, EP2007356, EP1325955, or WO 2010/091878, the disclosure of each of which is hereby incorporated by reference.

In some embodiments, the biologic is useful in treating, preventing, or ameliorating malignancies such as carcinomas, sarcomas, hematopoietic malignancies, and germ cell tumors, viral infections, microvascular disorders, eye diseases and respiratory conditions. In some embodiments, the biologic modulates expression of RTP801, VEGF, or PKN3. In some embodiments, the biologic is selected from an iRNA or oligonucleotide or analog thereof disclosed in EP1791568, US 8,168,607, US 7,741,299, US 2011/0117102, US 2011/0028532, EP2319925, US 2012/0156208, US 8,067,570, EP2402443, or EP1984003, the disclosure of each of which is hereby incorporated by reference.

In some embodiments, the biologic is useful in treating, preventing, or ameliorating pre-eclampsia, malignancies such as carcinomas, sarcomas, hematopoietic malignancies, and germ cell tumors, viral infections, microvascular disorders, eye diseases and respiratory conditions. In some embodiments, the biologic modulates expression of RTP801, VEGF, or PKN3. In some embodiments, the biologic is selected from an iRNA or oligonucleotide or analog thereof disclosed in US 2015/0359906, US 9,125,820, US 8,735,453, US 8,017,804, US 2014/0329885, US 2013/0165381, US 2012/0065138, US 2011/0294871, US 2008/0274116, EP1771206, US 2015/0368650, US 9,133,515, US 2014/0179755, EP2849771, WO 2013/170960, US 2014/0249207, US 8,722,875, US 2011/0217367, EP2350278, WO 2010/034487, EP2546337, US 8,232,256, EP2049658, WO 2008/009477, US 2009/0252783, EP2007890, EP2992875, US 2009/0074852, EP2007356, EP2007356, WO 2016/083623, or WO 2015/082080, the disclosure of each of which is hereby incorporated by reference.

In some embodiments, the biologic is useful in treating, preventing, or ameliorating a cancer or pre-eclampsia. In some embodiments, the biologic modulates expression of VEGF or PKN3. In some embodiments, the biologic is selected from an iRNA or oligonucleotide or analog thereof disclosed in US 2015/0368650, US 9,133,515, US 2014/0179755, EP2849771, WO 2013/170960, EP2546337, US 8,232,256, EP2049658, or WO 2008/009477, the disclosure of each of which is hereby incorporated by reference.

In some embodiments, the biologic is useful in treating, preventing, or ameliorating a cancer such as pancreatic cancer, or pre-eclampsia, or a cardiovascular-related disease. In some embodiments, the biologic is selected from an iRNA or oligonucleotide or analog thereof disclosed in US 8,735,453, US 2013/0165381, US 2012/0065138, US 2008/0274116, EP1771206, US 8,232,256, EP2049658, WO 2008/009477, US 2014/0249207, US 2011/0217367, EP2350278, WO 2010/034487, US 2009/0252783, EP2007890, EP2992875, US 2009/0074852, EP2007356, EP1389637, EP1527176, EP2258847, EP1857547, EP1389637, EP1325955, or WO 2010/091878, the disclosure of each of which is hereby incorporated by reference.

In some embodiments, the biologic is useful in treating, preventing, or ameliorating proliferative or DNA virus viral disease, wherein said proliferative disease is selected from carcinomas, sarcomas, hematopoietic malignancies, germ cell tumors, bladder cancer, melanoma, breast cancer, non-Hodgkin lymphoma, colon cancer, rectal cancer, pancreatic cancer, endometrial cancer, prostate cancer, kidney cancer, renal cell cancer, non-melanoma skin cancer, leukemia, thyroid cancer, lung cancer, neurofibromatosis or vascular proliferative diseases and wherein the viral disease is selected from Bell palsy, Burkitt lymphoma, chickenpox, cytomegalovirus infections, ecthyma, contagious, encephalitis, herpes simplex, Epstein-Barr virus infections, erythema infectiosum, exanthema subitum, herpes labialis, herpes simplex, herpes zoster, herpes zoster oticus, infectious mononucleosis, molluscum contagiosum, polyomavirus infections, smallpox, warts, infectious mononucleosis or EBV-associated malignancies. In some embodiments, the biologic modulates expression of ORC-1. In some embodiments, the biologic is selected from an iRNA or oligonucleotide or analog thereof disclosed in US 2014/0249207, US 8,722,875, US 2011/0217367, EP2350278, or WO 2010/034487, the disclosure of each of which is hereby incorporated by reference.

In some embodiments, the biologic is useful in treating, preventing, or ameliorating a hyperproliferative disease such as psoriasis, contact dermatitis, a retinopathy, endometriosis, uterine fibroids, dysfunctional vascular proliferation, endometrial microvascular growth, inflammation, arthritis, rheumatoid arthritis, acute lung injury (ALI), acute respiratory distress syndrome (ARDS), atherosclerosis, hereditary hemorrhagic telangiectasia, cavernous hemangioma, angiogenesis induced obesity, transplant arteriopathy, diabetic retinopathy, inflammatory bowel disease, periodontal disease, ascites, menorrhagia, pulmonary hypertension, pneumonia, pre-eclampsia, pulmonary fibrosis, emphysema, asthma,

chronic obstructive pulmonary disease (COPD), pancreatitis, sepsis, thrombosis, ischemic heart disease, multiple sclerosis, stroke, macular degeneration, liver cirrhosis, malaria, or systemic lupus erythematosus (SLE). In some embodiments, the biologic modulates expression of ANG2. In some embodiments, the biologic is selected from an iRNA or oligonucleotide or analog thereof disclosed in US 2014/0328903, US 8,829,179, US 2012/0022138, EP2398903, WO 2010/094491, or WO 2015/082080, the disclosure of each of which is hereby incorporated by reference.

In some embodiments, the biologic is useful in treating, preventing, or ameliorating non-alcoholic steatohepatitis. In some embodiments, the biologic modulates expression of miR-21. In some embodiments, the biologic is selected from an iRNA or oligonucleotide or analog thereof disclosed in US 2016/0244753, US 9,267,137, US 8,969,317, US 2015/0218558, US 2013/0289093, EP2841579, WO 2013/163258, US 2016/0138016, US 9,181,547, US 8,912,161, US 2014/0329887, US 2014/0107183, US 2012/0270928, EP2702155, WO 2012/148952, US 9,181,548, US 8,815,826, EP1931782, US 8,969,314, US 2015/0232841, EP1931782, EP2338991, EP1931782, US 2016/0046940, WO 2016/022753, EP3060664, or WO 2015/061536, the disclosure of each of which is hereby incorporated by reference.

In some embodiments, the biologic is useful in treating, preventing, or ameliorating fibrosis and fibroproliferative conditions, cardiovascular or metabolic diseases characterized by elevated serum total cholesterol, elevated serum LDL-cholesterol, or elevated serum triglycerides. In some embodiments, the biologic modulates expression of miR-214, miR-103, miR-107, or miR-122. In some embodiments, the biologic is selected from an iRNA or oligonucleotide or analog thereof disclosed in US 2016/0108397, US 9,181,548, US 8,815,826, US 2015/0232841, US 8,969,314, EP2338991, EP1931782, US 2016/0046940, WO 2016/022753, EP2841579, US 2013/0289093, WO 2013/163258, EP3060664, WO 2015/061536, EP2702155, or WO 2012/148952, the disclosure of each of which is hereby incorporated by reference.

In some embodiments, the biologic is useful in treating, preventing, or ameliorating hepatitis C virus and related conditions. In some embodiments, the biologic is useful in treating, preventing, or ameliorating fibrosis and fibroproliferative conditions, cardiovascular or metabolic diseases characterized by elevated serum total cholesterol, elevated serum LDL-cholesterol, or elevated serum triglycerides, or liver cancer. In some embodiments, the biologic modulates expression of miR-34, pri-miR-15, pri-miR-16, miR-214, miR-103, miR-107, or miR-122. In some embodiments, the biologic is selected from an iRNA or oligonucleotide or analog thereof disclosed in US 2016/0251657, US 9,309,513, US 9,157,083, US 2015/0105449, US 2014/0350090, EP2992095, WO 2014/179446, US 2016/0108397, US 9,181,548, US 8,815,826, US 2015/0080453, US 2013/0184217, WO 2012/012716, US 2016/0046941, US 9,150,857, US 8,680,067, US 8,211,867, US 2014/0206854, US 2012/0295962, US 2011/0251150, US 2010/0267814, EP2217248, US 2015/0232841, US 8,969,314, US 8,466,120, US 7,759,319, US 2010/0249215, EP2338991, EP1931782, US 2015/0087607, US 8,846,631, WO 2011/088309, US 7,998,677, US 2009/0236225, US 2016/0046940, WO 2016/022753, EP2992096, US 2015/0031130, or WO 2014/179445, the disclosure of each of which is hereby incorporated by reference.

In some embodiments, the biologic is useful in treating, preventing, or ameliorating hepatitis C, or cardiovascular or metabolic diseases characterized by elevated serum total cholesterol, or elevated serum triglycerides. In some embodiments, the biologic modulates expression of miR-122 or miR-122a. In some embodiments, the biologic is selected from an iRNA or oligonucleotide or analog thereof disclosed in EP1931782, US 7,683,036, EP1931782, US 2016/0251657, US 2015/0105449, US 2014/0350090, EP2992095, or WO 2014/179446, the disclosure of each of which is hereby incorporated by reference.

In some embodiments, the biologic is useful in treating, preventing, or ameliorating a disease, disorder, or condition associated with small non-coding RNAs. In some embodiments, the biologic modulates expression of miR-103 or miR-107. In some embodiments, the biologic is selected from an iRNA or oligonucleotide or analog thereof disclosed in US 9,447,413, US 9,447,412, US 9,267,138, US 9,139,832, US 8,946,179, US 8,859,521, US 8,809,294, US 8,765,701, US 8,697,663, US 8,546,350, US 8,178,506, US 8,133,876, US 8,110,558, US 8,106,025, US 7,683,036, US 2016/0017329, US 2015/0337305, US 20150337304, US 2015/0094461, US 2014/0336370, US 20140329882, US 2014/0121365, US 2014/0121364, US 2014/0057963, US 2012/0283319, US 2012/0157514, US 2012/0122216, US 2012/0035248, US 2011/0224277, US 2010/0267813, US 2009/0317907, US 2009/0298174, US 2009/0291907, US 2009/0291906, US 2009/0286969, EP2530157, US 2016/0046940, or WO 2016/022753, the disclosure of each of which is hereby incorporated by reference.

In some embodiments, the biologic is useful in treating, preventing, or ameliorating a disease, disorder, or condition associated with small non-coding RNAs. In some embodiments, the biologic modulates expression of miR-103 or miR-107. In some embodiments, the biologic is selected from an iRNA or oligonucleotide or analog thereof disclosed in US 9,267,138, US 2015/0337305, EP1648914, or WO 2016/022753, the disclosure of each of which is hereby incorporated by reference.

In some embodiments, the biologic is useful in treating, preventing, or ameliorating a disease, disorder, or condition associated with small non-coding RNAs. In some embodiments, the biologic modulates expression of miR-33, miR-103, or miR-107. In some embodiments, the biologic is selected from an iRNA or oligonucleotide or analog thereof disclosed in US 9,447,413, US 9,447,412, US 9,267,138, US 9,139,832, US 8,946,179, US 8,859,521, US 8,809,294, US 8,765,701, US 8,697,663, US 8,546,350, US 8,178,506, US 8,133,876, US 8,110,558, US 8,106,025,

US 7,683,036, US 2016/0017329, US 2015/0337305, US 2015/0337304, US 2015/0094461, US 2014/0336370, US 2014/0329882, US 2014/0121365, US 2014/0121364 US 2014/0057963, US 2012/0283319, US 2012/0157514, US 2012/0122216, US 2012/0035248, US 2011/0224277, US 2010/0267813, US 2009/0317907, US 2009/0298174, US 2009/0291907, US 2009/0291906, US 2009/0286969, EP2530157, EP1648914, US 2016/0046940, or WO 2016/022753, the disclosure of each of which is hereby incorporated by reference.

In some embodiments, the biologic is useful in treating, preventing, or ameliorating B-cell lymphoma or hepatocellular carcinoma. In some embodiments, the biologic modulates expression of STAT3. In some embodiments, the biologic is selected from an iRNA or oligonucleotide or analog thereof disclosed in EP2991661, EP2920308, or EP2697243, the disclosure of each of which is hereby incorporated by reference.

In some embodiments, the biologic is useful in treating, preventing, or ameliorating metabolic disease, for example diabetes, or a symptom thereof. In some embodiments, the biologic modulates expression of PTP1B. In some embodiments, the biologic is selected from an iRNA or oligonucleotide or analog thereof disclosed in US 9,404,113, EP2697244, or EP2992097, the disclosure of each of which is hereby incorporated by reference.

In some embodiments, the biologic is useful in treating, preventing, or ameliorating metabolic disease, for example diabetes, or a symptom thereof. In some embodiments, the biologic modulates expression of PTP1B or DGAT1. In some embodiments, the biologic is selected from an iRNA or oligonucleotide or analog thereof disclosed in US 9,404,113, EP2697244, EP2365094, EP2246443, EP1670896, EP2527442, EP2021472, EP2505649, EP2505648, EP2505647, EP2458006, EP2363482, EP2363481, EP2991661, or EP2992097, the disclosure of each of which is hereby incorporated by reference.

In some embodiments, the biologic is useful in treating, preventing, or ameliorating diseases, disorders, or conditions associated with small non-coding RNA. In some embodiments, the biologic modulates expression of miR-122. In some embodiments, the biologic is selected from an iRNA or oligonucleotide or analog thereof disclosed in EP1984499, or EP2447274, the disclosure of each of which is hereby incorporated by reference.

In some embodiments, the biologic is useful in treating, preventing, or ameliorating cancer or macular degeneration, age related macular degeneration (AMD), wet AMD, dry AMD, Geographic Atrophy, or a neurodegenerative disease. In some embodiments, the biologic modulates expression of Complement Factor B (CFB), GHR, apo(a), or apoplipoprotein C-III (ApoCIII), C90RF72, or androgen receptor (AR). In some embodiments, the biologic is selected from an iRNA or oligonucleotide or analog thereof disclosed in US 9,428,750, EP2282744, US 9,409,934, US 9,403,865, EP2885312, EP2601204, US 9,321,799, US 2016/0186185, EP2601204, US 9,340,784, US 2016/0222389, EP3043827, US 2016/0194638, US 2016/0194637, US 2016/0194349, US 2016/0186175, US 2016/0186174, US 2016/0159846, EP3027617, EP2625186, EP2625186, EP2606057, EP2606057, EP2751269, EP2751269, EP2580228, EP2580228, EP3067421, EP2673361, EP2742136, EP2742135, EP2742056, EP2673361, EP2462153, EP1984499, EP3066219, EP3011028, EP3017044, EP2991656, EP2991661, EP2992097, EP2992098, EP2992009, EP2951304, EP2956176, EP2906258, EP2906256, EP2906225, EP2906255, EP2906226, EP2906697, EP2906699, EP2906696, EP2864479, EP2850092, EP2831232, EP2852606, EP2839006, EP2794880, EP2812342, EP2751270, EP2582397, EP2447274, EP2358397, WO 2016/138353, WO 2016/138017, WO 2016/138355, WO 2016/115490, WO 2016/077704, WO 2016/077540, WO 2016/086104, WO 2016/077837, WO 2016/044840, or WO 2016/044828, the disclosure of each of which is hereby incorporated by reference.

In some embodiments, the biologic is useful in treating, preventing, or ameliorating a disease, disorder, or condition associated with microRNA (miRNA). In some embodiments, the biologic modulates expression of a target miRNA. In some embodiments, the biologic is selected from an iRNA or oligonucleotide or analog thereof disclosed in EP2582397, or WO 2016/138017, the disclosure of each of which is hereby incorporated by reference.

In some embodiments, the biologic is useful in treating, preventing, or ameliorating an HBV-related disease, disorder or condition. In some embodiments, the biologic modulates expression of HBV or transthyretin (TTR). In some embodiments, the biologic is selected from an iRNA or oligonucleotide or analog thereof disclosed in EP2991661, EP2992098, EP2699583, or WO 2016/077837, the disclosure of each of which is hereby incorporated by reference.

In some embodiments, the biologic is useful in treating, preventing, or ameliorating metabolic disease, for example, diabetes or a symptom thereof. In some embodiments, the biologic modulates expression of DGAT2, ApoB, or GCGR. In some embodiments, the biologic is selected from an iRNA or oligonucleotide or analog thereof disclosed in US 9,404,114, EP2758533, EP1670896, EP2015758, EP2021472, EP2527442, EP2505649, EP2505648, EP2505647, EP2458006, EP2363482, EP2363481, EP2991661, EP2992097, EP2812342, or EP2327709, the disclosure of each of which is hereby incorporated by reference.

In some embodiments, the biologic is useful in treating, preventing, or ameliorating a metabolic disease or a symptom thereof, or a disease associated with fibroblast growth factor receptor 4 (FGFR4). In some embodiments, the biologic modulates expression of fibroblast growth factor receptor 4 (FGFR4). In some embodiments, the biologic is selected from an iRNA or oligonucleotide or analog thereof disclosed in EP2215102, EP2991661, EP2992097, or EP2721156, the disclosure of each of which is hereby incorporated by reference.

In some embodiments, the biologic is useful in treating, preventing, or ameliorating a metabolic disorder. In

some embodiments, the biologic modulates expression of SOD1, ApoB, SGLT2, PCSK9, CRP, GCCR, GCGR, DGAT2, PTP1B or PTEN. In some embodiments, the biologic is selected from an iRNA or oligonucleotide or analog thereof disclosed in EP1670896, EP2527442, EP2021472, EP2505649, EP2505648, EP2505647, EP2458006, EP2363482, or EP2363481, the disclosure of each of which is hereby incorporated by reference.

In some embodiments, the biologic is useful in treating, preventing, or ameliorating a microbial infection. In some embodiments, the biologic is selected from an analog of an aminoglycoside compound disclosed in EP1957507, the disclosure of which is hereby incorporated by reference.

In some embodiments, the biologic is useful in treating, preventing, or ameliorating diseases involving unwanted neovascularization such as a cancer, an ocular disease, arthritis, or an inflammatory disease. In some embodiments, the biologic modulates expression of VEGF, VEGFR1, VEGFR2, VEGFR3, PDGF, PDGFR-$\alpha$, PDGFR-$\beta$, EGF, EGFR, RAF-a, RAF-c, AKT, RAS, NFkB, HIF, bFGF, bFGFR, Her-2, c-Met, c-Myc, HGF, EGFR-RP, TRA1, MFGE8, TNFSF13, ZFP236, ILK, HIF-1, or ICTE 030. In some embodiments, the biologic is selected from an iRNA or oligonucleotide or analog thereof disclosed in US 2011/0124710, US 7,893,244, US 7,893,243, US 7,786,092, US 7,723,316, US 7,534,878, US 2009/0227657, EP1877065, WO 2006/110813, US 7,781,414, US 2010/0203036, EP1615670, WO 2004/089284, US 2011/0046067, EP2170404, US 2011/0038849, EP2069498, WO 2008/076127, US 2011/0015249, EP2170351, US 2010/0280097, WO 2009/039300, US 2010/0279919, WO 2009/032930, US 2010/0210710, EP2209895, WO 2009/051659, US 2010/0028848, EP1963508, WO 2007/064846, US 2009/0247604, EP1711510, WO 2005/076998, US 2007/0219118, EP1448586, WO 2003/040399, US 2006/0211637, EP1546173, WO 2004/013310, EP1713819, WO 2005/076999, EP1451572, or WO 2003/063765, the disclosure of each of which is hereby incorporated by reference.

In some embodiments, the biologic is useful in treating, preventing, or ameliorating diseases involving unwanted neovascularization such as a cancer, an ocular disease, arthritis, or an inflammatory disease. In some embodiments, the biologic modulates expression of VEGF, VEGFR1, VEGFR2, VEGFR3, PDGF, PDGFR-$\alpha$, PDGFR-$\beta$, EGF, EGFR, RAF-a, RAF-c, AKT, RAS, NFkB, HIF, bFGF, bFGFR, Her-2, c-Met, c-Myc, HGF, EGFR-RP, TRA1, MFGE8, TNFSF13, ZFP236, ILK, HIF-1, or ICTE 030. In some embodiments, the biologic is selected from an iRNA or oligonucleotide or analog thereof disclosed in US 2011/0124710, US 7,893,244, US 7,893,243, US 7,786,092, US 7,723,316, US 7,534 878, US 2009/0227657, EP1877065, WO 2006/110813, US 2009/0247604, EP1711510, WO 2005/076998, US 2007/0219118, EP1448586, WO 2003/040399, US 2006/0211637, EP1546173, WO 2004/013310, EP1713819, or WO 2005/076999, the disclosure of each of which is hereby incorporated by reference.

In some embodiments, the biologic is useful in treating, preventing, or ameliorating EMT (Epithelial to Mesenchymal Transition), squamous cell carcinoma, a disease associated with neovascularization, melanoma, or head and neck cancer. In some embodiments, the biologic modulates expression of miRNA-96, miRNA-203, miRNA-lOb, miRNA-18b, miRNA-129, miRNA-128, miRNA-184, miRNA-190b, miRNA-3157, miRNA-133a, miRNA-200c, miRNA- 610, miRNA-182, miRNA-16, miRNA-95, miRNA-193a, miRNA-497, miRNA-509, miRNA-7, miRNA-3157-5p, miRNA-10b-3p, miRNA-129-5p, miRNA-96-5p, miRNA-200c-5p, miRNA-182-3p, miRNA-16-5p, miRNA-497-5p, miRNA-518b, miRNA-7-5p, miRNA-323, miRNA-342, miRNA-326, miRNA-371, miRNA-3157, or miRNA-345. In some embodiments, the biologic is selected from an iRNA or oligonucleotide or analog thereof disclosed in US 9,441,222, US 2014/0005251, EP2663641, EP2474617, WO 2012/096573, US 2016/0017338, US 9,161,947, US 2013/0072545, EP2542678, WO 2011/108930, US 2015/0297626, EP2917348, WO 2014/072357, US 2015/0225716, EP2794881, WO 2013/095132, US 2015/0152499, EP2870263, WO 2014/007623, EP2591106, US 2013/0109741, WO 2012/005572, EP2607483, or WO 2015/194956, the disclosure of each of which is hereby incorporated by reference, optionally in combination with a B-raf and/or MEK inhibitor, such as vemurafenib and/or dabrafenib and/or trametinib and/or selumetinib.

In some embodiments, the biologic is useful in treating, preventing, or ameliorating inflammatory conditions, autoimmune diseases, infectious diseases, neurodegenerative diseases or cancer. In some embodiments, the biologic modulates expression of miR-122a. In some embodiments, the biologic is selected from an iRNA or oligonucleotide or analog thereof disclosed in US 2016/0024508, US 9,157,919, US 2013/0281519, US 8,895,522, EP1940472, WO 2007/050034, US 2015/0099799, US 8,877,724, US 2011/0301225, EP2342341, EP2806028, EP2342341, WO 2010/053430, EP2269622, US 8,592,390, US 8,258,107, EP2380584, EP1901759, EP2380584, EP1901759, WO 2007/004977, EP2220489, US 8,574,834, US 2014/0030723, EP2220489, WO 2009/078793, US 8,569,257, US 8,148,341, EP2179737, EP1904077, EP2179737, EP1904077, WO 2007/004979, US 2015/0004187, EP2782602, EP2596806, WO 2013/076262, EP2350282, WO 2010/053433, EP2288702, or WO 2009/154565, the disclosure of each of which is hereby incorporated by reference.

In some embodiments, the biologic is useful in treating, preventing, or ameliorating of inflammatory conditions. In some embodiments, the biologic modulates expression of TLR9, IL-10, or an inflammation biomarker. In some embodiments, the biologic is selected from an iRNA or oligonucleotide or analog thereof disclosed in US 2016/0024508, US 9,157,919, US 2013/0281519, EP2655622, EP2468866, EP2468867, WO 2012/084996, WO 2012/084993, WO 2012/084991, US 8,895,522, EP1940472, WO 2007/050034, US 2015/0099799, US 8,877,724, US 2011/0301225, EP2342341, EP2806028, EP2342341, WO 2010/053430, US 8,569,257, US 8,148,341, EP2179737, EP1904077, EP2179737, EP1904077, WO 2007/004979, EP2350282, WO 2010/053433, EP2288702, or WO 2009/154565, the

disclosure of each of which is hereby incorporated by reference.

In some embodiments, the biologic is useful in treating, preventing, or ameliorating inflammation. In some embodiments, the biologic modulates expression of properties and behavior of polymorphonuclear cells, e.g. suppressing endothelial adhesion and transmigration of said cells, and through this mechanism reduce the recruitment and/or migration of polymorphonuclear cells to a site of inflammation. In some embodiments, the biologic is selected from an iRNA or oligonucleotide or analog thereof disclosed in US 2015/0099799, US 8,877,724, US 2011/0301225, EP2342341, EP2806028, EP2342341, or WO 2010/053430, the disclosure of each of which is hereby incorporated by reference.

In some embodiments, the biologic is useful in treating, preventing, or ameliorating surfactant protein B deficiency. In some embodiments, the biologic modulates expression of surfactant protein B or erythropoietin. In some embodiments, the biologic is selected from an iRNA or oligonucleotide or analog thereof or other therapeutic disclosed in US 8,567,410, US 2016/0177295, US 2015/0291678, US 2015/0290288, US 2015/0258174, or US 2012/0195936, the disclosure of each of which is hereby incorporated by reference.

In some embodiments, the biologic is useful in treating, preventing, or ameliorating surfactant protein B deficiency. In some embodiments, the biologic modulates expression of surfactant protein B. In some embodiments, the biologic is selected from an aerosol containing magnetic particles, wherein the aerosols comprise magnetic particles and a pharmaceutical active agent, or other therapeutic agent disclosed in US 8,567,410, the disclosure of which is hereby incorporated by reference.

In some embodiments, the biologic is useful in treating, preventing, or ameliorating diseases associated with mRNA encoded protein such as a bone or lung disease, disorder, or condition. In some embodiments, the biologic modulates expression of miR-122a or surfactant protein B or erythropoietin. In some embodiments, the biologic is selected from an iRNA or oligonucleotide or analog thereof or other therapeutic agent disclosed in US 8,567,410, US 2016/0177295, US 2015/0291678, US 2015/0290288, US 2015/0258174, US 2012/0195936, EP2459231, EP2459231, US 2015/0157565, EP2858679, WO 2013/185069, US 2015/0126589, EP2858677, WO 2013/182683, EP3013964, WO 2014/207231, WO 2016/075154, WO 2016/009000, or WO 2015/128030, the disclosure of each of which is hereby incorporated by reference.

In some embodiments, the biologic is useful in treating, preventing, or ameliorating of a disease, disorder, or condition such as surfactant protein B (SPB) deficiency, ATP-binding cassette sub-family A member 3 (ABCA3) deficiency, cystic fibrosis, alpha-1 antitrypsin (A1AT) deficiency, lung cancer, surfactant protein C (SPC) deficiency, alveolar proteinosis, sarcoidosis, acute or chronic bronchitis, emphysema, McLeod-Syndrom, chronic obstructive pulmonary disease (COPD), asthma bronchiale, bronchiectasis, pneumoconiosis, asbestosis, Acute Respiratory Distress Syndrome (ARDS), Infant respiratory distress syndrome (IRDS), pulmonary oedema, pulmonary eosinophilia, Loffler's pneumonia, Hamman-Rich syndrome, idiopathic pulmonary fibrosis, interstitial pulmonary diseases, primary ciliary dyskinesia, pulmonary arterial hypertension (PAH) and STAT5b deficiency, a clotting defect, hemophilia A and B, a complement defect, protein C deficiency, thrombotic thrombocytopenic purpura or congenital hemochromatosis, Hepcidin deficiency, a pulmonary infectious disease, respiratory syncytial virus (RSV) infection, parainfluenza virus (PIV) infection, influenza virus infection, rhinoviruses infection, severe acute respiratory syndrome (corona virus (SARS-CoV) infection, tuberculosis, Pseudomonas aeruginosa infection, Burkholderia cepacia infection, Methicillin-Resistant Staphylococcus aureus (MRSA) infection, or Haemophilus influenzae infection. In some embodiments, the biologic is selected from an iRNA or oligonucleotide or analog thereof disclosed in EP2459231, US 2015/0290288, US 2015/0258174, US 2015/0157565, EP2858679, WO 2013/185069, US 2015/0126589, EP2858677, or WO 2013/182683, the disclosure of each of which is hereby incorporated by reference.

In some embodiments, the biologic is useful in treating, preventing, or ameliorating a lactate dehydrogenase knockdown-treatable disease or disorder such as PHI, PH2, PH3 and idiopathic hyperoxaluria. In some embodiments, the biologic modulates expression of Glycolate Oxidase (HAO1) or lactate dehydrogenase. In some embodiments, the biologic is selected from an iRNA or oligonucleotide or analog thereof disclosed in US 2015/0184160, WO 2015/100436, or WO 2016/057932, the disclosure of each of which is hereby incorporated by reference.

In some embodiments, the biologic is useful in treating, preventing, or ameliorating a lactate dehydrogenase knockdown-treatable disease or disorder such as PHI, PH2, PH3 and idiopathic hyperoxaluria. In some embodiments, the biologic modulates expression of lactate dehydrogenase. In some embodiments, the biologic is selected from an iRNA or oligonucleotide or analog thereof disclosed in WO 2016/057932, the disclosure of which is hereby incorporated by reference.

In some embodiments, the biologic is useful in treating, preventing, or ameliorating a lactate dehydrogenase knockdown-treatable disease or disorder such as PHI, PH2, PH3 and idiopathic hyperoxaluria. In some embodiments, the biologic modulates expression of Glycolate Oxidase (HAO1) or lactate dehydrogenase. In some embodiments, the biologic is selected from an iRNA or oligonucleotide or analog thereof disclosed in US 2015/0184160, WO 2015100436, or WO 2016/057932, the disclosure of each of which is hereby incorporated by reference.

In some embodiments, the biologic is useful in treating, preventing, or ameliorating hyperoxaluria or a lactate dehydrogenase knockdown-treatable disease or disorder such as PHI, PH2, PH3 and idiopathic hyperoxaluria. In some

embodiments, the biologic modulates expression of Glycolate Oxidase (HAO1) or lactate dehydrogenase. In some embodiments, the biologic is selected from an iRNA or oligonucleotide or analog thereof disclosed in US 2015/0184160 or WO 2015/100436, the disclosure of each of which is hereby incorporated by reference.

In some embodiments, the biologic is useful in treating, preventing, or ameliorating a disease, disorder, or condition associated with β-catenin, EGFR, CKAP5, MCL1, MYC, or HIF-1α. In some embodiments, the biologic modulates expression of β-catenin, EGFR, CKAP5, MCL1, MYC, or HIF-1α. In some embodiments, the biologic is selected from an iRNA or oligonucleotide or analog thereof disclosed in US 9,428,752, US 9,243,244, US 8,815,825, US 2016/0186176, US 2016/0053263, US 2014/0288292, US 2013/0109740, EP3037538, EP2591105, WO 2012/006243, US 9,365,850, US 2015/0240234, US 2014/0107178, EP2895609, WO 2014/043311, US 2016/0177303, US 9,206,420, US 2013/0303593, WO 2012/100172, US 2016/0083729, US 9,217,146, US 2014/0179765, WO 2012/173994, US 2015/0197756, US 8,927,515, US 2013/0131149, EP2591104, WO 2012/006241, US 2015/0038555, US 8,927,705, US 8,513,207, US 8,349,809, US 2015/0038554, US 2014/0350074, US 2014/0221454, US 2013/0096290, US 2013/0041010, US 2012/0263738, US 2012/0095200, US 2011/0111056, US 2011/0059187, US 2011/0003881, US 2010/0249214, US 2010/0173974, US 2010/0173973, US 2010/0184841, EP2513334, EP2437752, EP2437751, EP2379083, EP2341943, WO 2011/075188, WO 2011/072292, WO 2010/141726, WO 2010/141724, WO 2010/141933, WO 2010/080129, WO 2010/093788, WO 2010/033225, EP2968149, US 2015/0374842, WO 2014/153163, US 2015/0315583, EP2931746, WO 2014/093746, US 2015/0065555, WO 2013/138668, US 2014/0371293, EP2768958, WO 2013/059496, US 2014/0315983, WO 2013/066721, US 2014/0155462, WO 2012/145582, or WO 2016/100401, the disclosure of each of which is hereby incorporated by reference.

In some embodiments, the biologic is useful in treating, preventing, or ameliorating a disease, disorder, or condition associated with KRAS, MYC, or androgen receptor (AR) such as cancer. In some embodiments, the biologic modulates expression of KRAS, MYC, or AR In some embodiments, the biologic is selected from an iRNA or oligonucleotide or analog thereof disclosed in US 9,365,850, US 2015/0240234, US 2014/0107178, EP2895609, WO 2014/043311, US 2014/0371293, EP2768958, or WO 2013/059496, the disclosure of each of which is hereby incorporated by reference.

In some embodiments, the biologic is useful in treating, preventing, or ameliorating a disease, disorder, or condition associated with KRAS, EGFR, or androgen receptor (AR). In some embodiments, the biologic modulates expression of KRAS, EGFR, or AR In some embodiments, the biologic is selected from an iRNA or oligonucleotide or analog thereof disclosed in US 2016/0083729, US 9,217,146, US 2014/0179765, WO 2012/173994, US 9,200,284, US 8,927,705, US 8,513,207, US 8,372,816, US 8,349,809, US 2015/0057337, US 2015/0038555, US 2015/0038554, US 2014/0350074, US 2014/0221454, US 2013/0096290, US 2013/0123342, US 2013/0041010, US 2012/0095200, US 2011/0111056, US 2011/0059187, US 2011/0021604, US 2011/0003881, US 2010/0173974, US 2010/0173973, US 2010/0184841, EP2756845, EP2513334, EP2437752, EP2437751, EP2414374, EP2379083, EP2341943, WO 2011/075188, WO 2011/072292, WO 2010/141726, WO 2010/141724, WO 2010/141933, WO 2010/115206, WO 2010/115202, WO 2010/080129, WO 2010/033225, US 2015/0197756, US 8,927,515, US 2013/0131149, EP2591104, WO 2012/006241, EP2968149, US 2015/0374842, WO 2014/153163, US 2014/0371293, EP2768958, WO 2013/059496, US 2014/0155462, WO 2012/145582, EP2873732, US 2014/0044755, WO 2016/100401, or WO 2013/032643, the disclosure of each of which is hereby incorporated by reference.

In some embodiments, the biologic is useful in treating, preventing, or ameliorating a disease, disorder, or condition associated with KRAS or EGFR. In some embodiments, the biologic modulates expression of KRAS or EGFR. In some embodiments, the biologic is selected from an iRNA or oligonucleotide or analog thereof disclosed in US 9,200,284, US 8,927,705, US 8,513,207, US 8,372,816, US 8,349,809, US 2015/0057337, US 2014/0221454, US 2013/0096290, US 2013/0123342, US 2011/0021604, US 2011/0003881, US 2010/0173974, US 2010/0173973, US 2010/0184841, EP2756845, EP2513334, EP2414374, EP2379083, WO 2011/075188, WO 2010/115206, WO 2010/115202, WO 2010/080129, US 2014/0371293, EP2768958, WO 2013/059496, US 2014/0155462, WO 2012/145582, EP2873732, or US 2014/0044755, the disclosure of each of which is hereby incorporated by reference.

In some embodiments, the biologic is useful in treating, preventing, or ameliorating a disease, disorder, or condition associated with β-catenin, TTR, lactate dehydrogenase, MET, α-1 antitrypsin, MCL1, MYC, or CKAP5 such as hepatocellular carcinoma. In some embodiments, the biologic modulates expression of β-catenin, TTR, lactate dehydrogenase, MET, α-1 antitrypsin, MCL1, MYC, or CKAP5. In some embodiments, the biologic is selected from an iRNA or oligonucleotide or analog thereof disclosed in US 9,428,752, US 9,243,244, US 8,815,825, US 2016/0186176, US 2016/0053263, US 2014/0288292, US 2013/0109740, EP3037538, EP2591105, WO 2012/006243, US 9,365,850, US 2015/0240234, US 2014/0107178, EP2895609, WO 2014/043311, EP2968149, US 2015/0374842, WO 2014/153163, US 2015/0315583, EP2931746, WO 2014/093746, US 2015/0065555, WO 2013/138668, EP3017047, US 2015/0011607, WO 2015/003113, US 2014/0371293, EP2768958, WO 2013/059496, US 2014/0315983, WO 2013/066721, WO 2016/057932, WO 2015/085158, or WO 2009/131661, the disclosure of each of which is hereby incorporated by reference.

In some embodiments, the biologic is useful in treating, preventing, or ameliorating a disease, disorder, or

condition associated with MYC or α-1 antitrypsin. In some embodiments, the biologic modulates expression of MYC or α-1 antitrypsin. In some embodiments, the biologic is selected from an iRNA or oligonucleotide or analog thereof disclosed in US 9,365,850, US 2015/0240234, US 2014/0107178, EP2895609, WO 2014/043311, EP3017047, US 2015/0011607, WO 2015/003113, US 2014/0371293, EP2768958, or WO 2013/059496, the disclosure of each of which is hereby incorporated by reference.

In some embodiments, the biologic is useful in treating, preventing, or ameliorating a disease, disorder, or condition associated with β-catenin or lactate dehydrogenase. In some embodiments, the biologic modulates expression of β-catenin or lactate dehydrogenase. In some embodiments, the biologic is selected from an iRNA or oligonucleotide or analog thereof disclosed in US 9,428,752, US 9,243,244, US 8,815,825, US 2016/0186176, US 2016/0053263, US 2014/0288292, US 2013/0109740, EP3037538, EP2591105, WO 2012/006243, WO 2016/057932, or WO 2009/131661, the disclosure of each of which is hereby incorporated by reference.

In some embodiments, the biologic is useful in treating, preventing, or ameliorating a disease, disorder, or condition associated with Glycolate Oxidase (HAO1) or a lactate dehydrogenase knockdown-treatable disease or disorder. In some embodiments, the biologic modulates expression of HAO1 or lactase dehydrogenase. In some embodiments, the biologic is selected from an iRNA or oligonucleotide or analog thereof disclosed in US 2015/0184160, WO 2015/100436, or WO 2016/057932, the disclosure of each of which is hereby incorporated by reference.

In some embodiments, the biologic is useful in treating, preventing, or ameliorating cancer, such as liver cancer. In some embodiments, the biologic modulates expression of, or is selected from, miR-34, miR-124, or miR-215. In some embodiments, the biologic is selected from an iRNA or oligonucleotide or analog thereof disclosed in US 8,071,562, US 2016/0136181, WO 2015/153757, US 2015/0344881, US 2014/0314833, US 2015/0246070, WO 2015/131115, EP2968567, US 2015/0272981, US 2014/0308274, US 2014/0309278, WO 2014/143855, US 2010/0179213, WO 2010/056737, EP3013975, or WO 2014/209970, the disclosure of each of which is hereby incorporated by reference; optionally in combination with an additional therapeutic agent such as an EGFR-TKI agent; or an additional therapeutic agent such as sorafenib.

In some embodiments, the biologic is useful in treating, preventing, or ameliorating cancer, such as liver cancer. In some embodiments, the biologic modulates expression of, or is selected from, miR-34, miR-124, miR-126, miR-147, or miR-215. In some embodiments, the biologic is selected from an iRNA or oligonucleotide or analog thereof disclosed in EP2968567, US 2015/0272981, US 2014/0308274, US 2014/0309278, WO 2014/143855, EP3013975, or WO 2014/209970, the disclosure of each of which is hereby incorporated by reference; optionally in combination with an additional therapeutic agent such as an EGFR-TKI agent; or an additional therapeutic agent such as sorafenib.

In some embodiments, the biologic is useful in treating, preventing, or ameliorating a cancer such as liver cancer. In some embodiments, the biologic modulates expression of miR-101, miR-34, or miR-215. In some embodiments, the biologic is selected from an iRNA or oligonucleotide or analog thereof disclosed in US 2015/0344881, US 2014/0314833, US 2015/0246070, WO 2015/131115, US 2015/0272981, US 2014/0308274, US 2014/0309278, US 2010/0179213, or WO 2010/056737, the disclosure of each of which is hereby incorporated by reference; optionally in combination with an additional therapeutic agent such as an EGFR-TKI agent; or an additional therapeutic agent such as sorafenib.

In some embodiments, the biologic is useful in treating, preventing, or ameliorating a cancer such as liver cancer. In some embodiments, the biologic modulates expression of miR-101, miR-34, or miR-215. In some embodiments, the biologic is selected from an iRNA or oligonucleotide or analog thereof disclosed in US 2015/0344881, US 2010/0179213, WO 2010/056737, the disclosure of each of which is hereby incorporated by reference; optionally in combination with an additional therapeutic agent such as an EGFR-TKI agent; or an additional therapeutic agent such as sorafenib.

In some embodiments, the biologic is useful in treating, preventing, or ameliorating a cancer. In some embodiments, the biologic modulates expression of miR-34 or is a miR-34 mimic, including a miR-34a or miR-34c mimic. In some embodiments, the biologic is selected from an iRNA or oligonucleotide or analog thereof disclosed in US 9,371,526, US 8,586,727, US 2016/0053264, US 2014/0107182, EP2670850, WO 2012/106591, US 2016/0151406, WO 2016/081773, US 2016/0136181, WO 2015/153757, US 2015/0246070, WO 2015/131115, EP2968567, US 2015/0272981, US 2014/0308274, US 2014/0309278, WO 2014/143855, US 2010/0179213, WO 2010/056737, EP3013975, WO 2014/209970, or WO 2016/161196, the disclosure of each of which is hereby incorporated by reference; optionally in combination with an additional therapeutic agent such as an EGFR-TKI agent; or an additional therapeutic agent such as sorafenib; or a c-Met inhibitor (e.g., tivantinib).

In some embodiments, the biologic is useful in treating, preventing, or ameliorating cancer. In some embodiments, the biologic modulates expression of miR-34 or is a miR-34 mimic, including a miR-34a or miR-34c mimic. In some embodiments, the biologic is selected from an iRNA or oligonucleotide or analog thereof disclosed in EP2968567, US 2015/0272981, US 2014/0308274, US 2014/0309278, WO 2014/143855, EP3013975, WO 2014/209970, WO 2016/161196, or WO 2016/081773, the disclosure of each of which is hereby incorporated by reference; optionally in combination with an additional therapeutic agent such as an EGFR-TKI agent; or an additional therapeutic agent such

as sorafenib; or a c-Met inhibitor (e.g., tivantinib).

In some embodiments, the biologic is useful in treating, preventing, or ameliorating a vascular disease, including cancer, cardiac diseases, vascular diseases of the eye, and inflammatory diseases. In some embodiments, the biologic modulates expression of miR-7, miR-16, miR-21, or miR-124; or is a mimic of miR-7, miR-16, miR-21, or miR-124. In some embodiments, the biologic is selected from an iRNA or oligonucleotide or analog thereof disclosed in US 2016/0222385, US 9,365,852, US 8,258,111, US 2015/0344880, US 8,071,562, US 2015/0344881, US 2014/0314833, US 2014/0308274, US 2010/0179213, or WO 2010/056737, the disclosure of each of which is hereby incorporated by reference; optionally in combination with an additional therapeutic agent such as an EGFR-TKI agent; or an additional therapeutic agent such as sorafenib.

In some embodiments, the biologic is useful in treating, preventing, or ameliorating of vascular diseases including cancer, cardiac diseases, vascular diseases of the eye, and inflammatory diseases. In some embodiments, the biologic modulates expression of miR-7, miR-16, miR-21, or miR-124; or is a mimic of miR-7, miR-16, miR-21, or miR-124. In some embodiments, the biologic is selected from an iRNA or oligonucleotide or analog thereof disclosed in US 2016/0222385, US 2010/0179213, or WO 2010/056737, the disclosure of each of which is hereby incorporated by reference; optionally in combination with an additional therapeutic agent such as an EGFR-TKI agent; or an additional therapeutic agent such as sorafenib.

In some embodiments, the biologic is useful in treating, preventing, or ameliorating cancer, for example by targeting cancer stem cells. In some embodiments, the biologic modulates expression of miR-34, miR-124, miR-126, miR-147, miR-215, or is a mimic thereof. In some embodiments, the biologic is selected from an iRNA or oligonucleotide or analog thereof disclosed in US 8,071,562, US 2015/0344881, US 2014/0314833, EP2968567, US 2015/0272981, US 2014/0308274, US 2014/0309278, WO 2014/143855, US 2010/0179213, or WO 2010/056737, the disclosure of each of which is hereby incorporated by reference; optionally in combination with an additional therapeutic agent such as an EGFR-TKI agent; or an additional therapeutic agent such as sorafenib.

In some embodiments, the biologic is useful in treating, preventing, or ameliorating of vascular diseases including cancer, cardiac diseases, vascular diseases of the eye, and inflammatory diseases. In some embodiments, the biologic modulates expression of miR-34, miR-124, miR-126, miR-147, miR-215, or is a mimic thereof. In some embodiments, the biologic is selected from an iRNA or oligonucleotide or analog thereof disclosed in US 2016/0222385, US 9,365,852, US 8,258,111, US 2015/0344880, US 9,371,526, US 8,586,727, US 2016/0053264, US 2014/0107182, EP2670850, WO 2012/106591, US 2016/0060629, US 9,222,085, EP2670849, WO 2012/106586, US 8,900,627, EP2306978, US 8,071,562, US 2016/0151406, WO 2016/081773, US 2016/0136181, WO 2015/153757, US 2015/0344881, US 2014/0314833, US 2015/0246070, WO 2015/131115, EP2968567, US 2015/0272981, US 2014/0308274, US 2014/0309278, WO 2014/143855, US 2010/0179213, WO 2010/056737, EP3013975, WO 2014209970, or WO 2016/161196, the disclosure of each of which is hereby incorporated by reference; optionally in combination with an additional therapeutic agent such as an EGFR-TKI agent; or an additional therapeutic agent such as sorafenib; a c-Met inhibitor (e.g., tivantinib).

In some embodiments, the biologic is useful in treating, preventing, or ameliorating cancer. In some embodiments, the biologic modulates expression of miR-34 or miR-124, miR-126, miR-147, miR-215, or is a mimic thereof. In some embodiments, the biologic is selected from an iRNA or oligonucleotide or analog thereof disclosed in US 9,371,526, US 2016/0053264, US 2014/0107182, EP2670850, WO 2012/106591, US 2016/0222385, US 8,258,111, US 8,071,562, US 2016/0151406, WO 2016/081773, US 2016/0136181, WO 2015/153757, US 2016/0060629, EP2670849, WO 2012/106586, US 2015/0344881, US 2014/0314833, US 2015/0246070, WO 2015/131115, EP2968567, US 2015/0272981, US 2014/0308274, US 2014/0309278, WO 2014/143855, US 2010/0179213, WO 2010/056737, EP3013975, WO 2014/209970, or WO 2016/161196, the disclosure of each of which is hereby incorporated by reference optionally in combination with an additional therapeutic agent such as a c-Met inhibitor (e.g., tivantinib).

In some embodiments, the biologic is useful in treating, preventing, or ameliorating of essential hypertension, secondary hypertension, renovascular hypertension, resistant hypertension, peripheral arterial disease, coronary artery disease, atherosclerosis, arteriosclerosis, aneurysm, angina, hypertensive heart disease, heart failure, ischemia, cor pulmonale, pulmonary hypertension, pulmonary arterial hypertension, diabetic nephropathy, diabetic retinopathy, optic neuropathy, cerebrovascular disease, stroke, hypertensive encephalopathy, myocardial infarction, vascular calcification, hypertensive retinopathy, hypertensive nephropathy, hypertensive nephrosclerosis, restenosis, or thrombosis. In some embodiments, the biologic modulates expression of miR-92, miR-137, or miR-138, or is a mimic thereof. In some embodiments, the biologic is selected from an iRNA or oligonucleotide or analog thereof disclosed in US 9,388,408, US 2013/0344135, EP2864482, WO 2013/192576, WO 2016/118612, US 2016/0208258, or WO 2016/069717, the disclosure of each of which is hereby incorporated by reference.

In some embodiments, the biologic is useful in treating, preventing, or ameliorating of cardiac disorders. In some embodiments, the biologic modulates expression of miR-155. In some embodiments, the biologic is selected from an iRNA or oligonucleotide or analog thereof disclosed in US 2014/0024700, EP2652146, or WO 2012/083004, the disclosure of each of which is hereby incorporated by reference.

In some embodiments, the biologic is useful in treating, preventing, or ameliorating a cardiac disease, disorder, or condition. In some embodiments, the biologic modulates expression of miR-92 or miR-92a. In some embodiments, the biologic is selected from an iRNA or oligonucleotide or analog thereof disclosed in US 9,388,408, US 2013/0344135, EP2864482, WO 2013/192576, WO 2016/118612, US 2016/0208258, US 2014/0024700, EP2652146, or WO 2012/083004, the disclosure of each of which is hereby incorporated by reference.

In some embodiments, the biologic is useful in treating, preventing, or ameliorating of age-related cardiomyopathy or a tissue fibrotic condition. In some embodiments, the biologic modulates expression of miR-29. In some embodiments, the biologic is selected from an iRNA or oligonucleotide or analog thereof disclosed in US 9,388,408, US 2013/0344135, EP2864482, WO 2013/192576, US 9,376,681, US 2016/0068842, WO 2016/040373, US 2014/0187603, US 8,642,751, US 2012/0184596, EP2652151, WO 2012/083005, EP2970968, US 2016/0010090, WO 2014/145356, US 2012/0238619, or WO 2015/142735, the disclosure of each of which is hereby incorporated by reference.

In some embodiments, the biologic is useful in treating, preventing, or ameliorating a disease, disorder, or condition associated with a miR-15 family RNA. In some embodiments, the biologic modulates expression of a miR-15 family RNA, such as miR-15a, miR-15b, miR-16, miR-195, miR-424, or miR-497. In some embodiments, the biologic is selected from an iRNA or oligonucleotide or analog thereof disclosed in US 9,388,408, US 2013/0344135, EP2864482, WO 2013/192576, US 9,163,235, US 2013/0345288, EP2863956, WO 2013/192486, EP2970968, US 2016/0010090, WO 2014/145356, US 2014/0066491, US 2012/0148664, EP2440566, or WO 2010/144485, the disclosure of each of which is hereby incorporated by reference.

In some embodiments, the biologic is useful in treating, preventing, or ameliorating hypertrophic cardiomyopathy or heart failure. In some embodiments, the biologic modulates expression of MYH7B or a miR-208 family miRNA, including miR-208a, miR-208b, and/or miR-499. In some embodiments, the biologic is selected from an iRNA or oligonucleotide or analog thereof disclosed in US 2014/0187603, US 8,642,751, US 2012/0184596, EP2652151, WO 2012/083005, WO 2016/022536, or US 2016/0032286, the disclosure of each of which is hereby incorporated by reference.

In some embodiments, the biologic is useful in treating, preventing, or ameliorating dysregulation of extracellular matrix genes, such as tissue fibrotic conditions, e.g. cutaneous or pulmonary fibrosis. In some embodiments, the biologic modulates expression of miR-29. In some embodiments, the biologic is selected from an iRNA or oligonucleotide or analog thereof disclosed in US 9,376,681, US 2016/0068842, or WO 2016/040373, the disclosure of each of which is hereby incorporated by reference.

In some embodiments, the biologic is useful in treating, preventing, or ameliorating a disease, disorder, or condition associated with regulating eukaryotic promoter-driven gene expression in prokaryotes. In some embodiments, the biologic modulates expression of miR-29 or regulates eukaryotic promoter-driven gene expression in prokaryotes. In some embodiments, the biologic is selected from a therapeutic agent disclosed in US 2015/0118734, US 2015/0064771, US 2013/0210120, EP2742127, or WO 2013/025248, the disclosure of each of which is hereby incorporated by reference.

In some embodiments, the biologic is useful in treating, preventing, or ameliorating familial adenomatous polyposis. In some embodiments, the biologic is selected from a vector or other therapeutic agent disclosed in EP2356235, the disclosure of which is hereby incorporated by reference.

In some embodiments, the biologic upon delivery to a cell, such as a cancer cell, modulates expression of RAS, β-catenin, one or more HPV oncogenes, APC, HER-2, MDR-1, MRP-2, FATP4, SGLUT-1, GLUT-2, GLUT-5, APOBEC-1, MTP, IL-6, IL-6R, IL-7, IL-12, IL-13, IL-13 Ra-1, IL-18, p38/JNK MAP Kinase, p65/NF-κB, CCL20 (or MIP-3α), Claudin-2, Chitinase 3-like 1, APOA-IV, MHC class I, or MHC class II. In some embodiments, the biologic is selected from a plasmid, vector, or an iRNA or oligonucleotide or analog thereof disclosed in US 2015/0184167, US 9,012,213, or EP2356235, the disclosure of each of which is hereby incorporated by reference.

In some embodiments, the biologic is useful in treating, preventing, or ameliorating a disease, disorder, or condition associated with STAT3, such as cancer, e.g. B-cell lymphoma or hepatocellular carcinoma. In some embodiments, the biologic modulates expression of STAT3. In some embodiments, the biologic is selected from an iRNA or oligonucleotide or analog thereof disclosed in EP2991661, EP2920308, EP2697243, EP2595664, or WO 2016/077837, the disclosure of each of which is hereby incorporated by reference.

In some embodiments, the biologic is useful in treating, preventing, or ameliorating a viral infection, such as HBV or HCV infection. In some embodiments, the biologic modulates expression of a viral gene such as an HBV or HCV gene. In some embodiments, the biologic is selected from an iRNA or oligonucleotide or analog thereof disclosed in US 2015/0361432, US 9,139,833, US 2015/0376621, US 9,084,808, EP2726613, US 2013/0005793, or WO 2013/003520, the disclosure of each of which is hereby incorporated by reference.

In some embodiments, the biologic is useful in treating, preventing, or ameliorating a viral infection, such as HBV or HCV infection. In some embodiments, the biologic modulates expression of a viral gene such as an HBV or HCV gene. In some embodiments, the biologic is selected from an iRNA or oligonucleotide or analog thereof disclosed in US 9,139,833, US 2015/0376621, or US 9,084,808, the disclosure of each of which is hereby incorporated by reference.

In some embodiments, the biologic is useful in treating, preventing, or ameliorating of a disease, disorder, or condition associated with MLL or ribonucleotide reductase M2 (RRM2). In some embodiments, the biologic modulates expression of an RRM2 or MLL gene. In some embodiments, the biologic is selected from an iRNA or oligonucleotide or analog thereof disclosed in US 2015/0113670, US 8,946,176, EP2851426, EP2630240, WO 2012/052258, or WO 2012/082894, the disclosure of each of which is hereby incorporated by reference.

In some embodiments, the biologic is useful in treating, preventing, or ameliorating a disease, disorder, or condition such as cancer, metastases, astrocytoma, bladder cancer, breast cancer, chondrosarcoma, colorectal carcinoma, gastric carcinoma, glioblastoma, head and neck squamous cell carcinoma, hepatocellular carcinoma, lung adenocarcinoma, neuroblastoma, non-small cell lung cancer, melanoma, multiple myeloma, ovarian cancer, rectal cancer, renal cancer, clear cell renal cell carcinoma (and metastases of this and other cancers), gingivitis, psoriasis, Kaposi's sarcoma-associated herpesvirus, preemclampsia, inflammation, chronic inflammation, neovascular diseases, or rheumatoid arthritis. In some embodiments, the biologic modulates expression of EPAS1. In some embodiments, the biologic is selected from an iRNA or oligonucleotide or analog thereof disclosed in US 2016/0010089 or EP2961843, the disclosure of each of which is hereby incorporated by reference.

In some embodiments, the biologic is useful in treating, preventing, or ameliorating of hypertriglyceridemia (e.g., Type V Hypertriglyceridemia), abnormal lipid metabolism, abnormal cholesterol metabolism, atherosclerosis, hyperlipidemia, diabetes, including Type 2 diabetes, obesity, cardiovascular disease, and coronary artery disease, among other disorders relating to abnormal metabolism or otherwise. In some embodiments, the biologic modulates expression of APOC3. In some embodiments, the biologic is selected from an iRNA or oligonucleotide or analog thereof disclosed in WO 2016/011123, the disclosure of which is hereby incorporated by reference.

In some embodiments, the biologic is useful in treating, preventing, or ameliorating alpha 1-antitrypsin deficiency and associated diseases such as chronic hepatitis, cirrhosis, hepatocellular carcinoma, and fulminant hepatic failure. In some embodiments, the biologic modulates expression of alpha 1-antitrypsin. In some embodiments, the biologic is selected from an iRNA or oligonucleotide or analog thereof disclosed in WO 2015/195628, the disclosure of which is hereby incorporated by reference.

In some embodiments, the biologic is useful in treating, preventing, or ameliorating sepsis. In some embodiments, the biologic modulates expression of TNF$\alpha$. In some embodiments, the biologic is selected from a therapeutic agent disclosed in US 2002/0187208, US 6,352,729, or WO 2002/036737, the disclosure of each of which is hereby incorporated by reference.

In some embodiments, the biologic is useful in treating, preventing, or ameliorating cancer such as colon cancer, sepsis, oxidative stress, or a metabolic disease. In some embodiments, the biologic selectively modulates apoptosis. In some embodiments, the biologic is selected from a therapeutic agent such as a zinc-charged protein disclosed in US 8,247,380, US 7,445,784, US 7,238,662, EP1119367, US 7,528,108, EP1874796, WO 2006/116410, US 2002/0187208, US 6,352,729, WO 2002/036737, WO 2001/084938, US 6,312,737, EP2323682, US 2010/0022442, EP2323682, WO 2010/011533, or WO 2001/082871, the disclosure of each of which is hereby incorporated by reference.

In some embodiments, the biologic is useful in treating, preventing, or ameliorating cancer such as colon cancer. In some embodiments, the biologic selectively modulates apoptosis. In some embodiments, the biologic is selected from a therapeutic agent such as a zinc-charged protein disclosed in US 8,247,380, US 7,445,784, US 7,238,662, EP1119367, WO 2001/084938, US 6,312,737, EP2323682, or WO 2001/082871, the disclosure of each of which is hereby incorporated by reference.

In some embodiments, the biologic is useful in treating, preventing, or ameliorating disease, disorder, or condition such as an infectious disease, allergic condition, inflammatory disease, autoimmune disease, or cancer, such as respiratory syncytial virus (RSV) or influenza. In some embodiments, the biologic is useful as an adjuvant or vaccine. In some embodiments, the biologic modulates an adaptive immune response in a patient in need thereof. In some embodiments, the biologic is selected from an iRNA or oligonucleotide or analog thereof, or antibody, antigen, or other therapeutic agent, disclosed in US 9,421,255, US 2013/0259879, EP2678038, WO 2012/113513, WO 2012/113413, US 9,402,887, US 2013/0251742, EP2195015, WO 2009/046975, WO 2009/046739, US 9,352,028, US 2013/0202645, EP2197481, WO 2009/046974, WO 2009/046738, US 2016/0250321, US 9,226,959, US 2012/0021043, EP2176408, EP2176408, EP2548960, EP2176408, WO 2009/095226, US 2016/0206719, US 2016/0130345 EP2958588, WO 2014/127917, US 2016/0185840, US 2016/0168254, US 2016/0166692, US 2016/0166691, US 2016/0166690, US 2016/0152706, US 2016/0152691, US 2016/0145346, US 2013/0195867, EP3035955, US 2016/0168227, WO 2015/024666, EP3035960, US 2016/0168207, WO 2015/024668, EP3036330, US 2016/0166710, WO 2015/024667, EP3035954, US 2016/0166668, WO 2015/024664, EP3035961, US 2016/0166711, WO 2015/024665, EP3035959, US 2016/0166678, WO 2015/024669, US 2016/0151474, US 2013/0295043, EP2680881, WO 2012/116811, WO 2012/116714, US 2015/0118264, EP2809353, WO 2013/113501, WO 2013/113326, US 2015/0118183, EP2809354, WO 2013/113502, WO 2013/113325, US 2015/0141498, EP2510100, WO 2011/069586, WO 2011/069529, US 2015/0093413, EP2814962, WO 2013/120628, WO 2013/120499, EP2680880, US 2013/0336998, WO 2012/116715, WO 2012/116810, EP2658569, US 2013/0280283, WO 2012/089338, WO 2012/089225, EP2650368, US 2013/0121988, US

2009/0324584, EP2046954, WO 2008/014979, EP2762165, US 2011/0250225, EP2331129, EP2331129, WO 2010/037539, WO 2010/037408, US 2011/0053829, EP1083232, WO 2016/107877, WO 2016/097065, WO 2016/091391, WO 2015/149944, WO 2015/135558, WO 2015/101414, WO 2015/101415, or WO 2010/088927, the disclosure of each of which is hereby incorporated by reference.

In some embodiments, the biologic is useful in treating, preventing, or ameliorating disease, disorder, or condition such as an infectious disease, allergic condition, inflammatory disease, autoimmune disease, or cancer, such as respiratory syncytial virus (RSV) or influenza. In some embodiments, the biologic is useful as an adjuvant or vaccine. In some embodiments, the biologic modulates an adaptive immune response in a patient in need thereof. In some embodiments, the biologic is selected from an iRNA or oligonucleotide or analog thereof, or antibody, antigen, or other therapeutic agent, disclosed in US 9,226,959, US 2012/0021043, EP2176408, EP2176408, EP2548960, EP2176408, WO 2009/095226, US 2016/0185840, EP3035960, US 2016/0168207, WO 2015/024668, EP3035959, US 2016/0166678, WO 2015/024669, US 2015/0118264, EP2809353, WO 2013/113501, WO 2013/113326, US 2015/0118183, EP2809354, WO 2013/113502, WO 2013/113325, US 2015/0093413, EP2814962, WO 2013/120628, WO 2013/120499, EP2650368, US 2009/0324584, EP2046954, WO 2008/014979, WO 2015/149944, WO 2009/046739, or WO 2009/046738, the disclosure of each of which is hereby incorporated by reference.

In some embodiments, the biologic is useful in treating, preventing, or ameliorating an infectious disease (such as RSV or rabies) or cancer, a cardiovascular disease, an infectious disease, an autoimmune disease or genetic disease, or in gene therapy, or as an adjuvant or immunostimulating agent. In some embodiments, the biologic modulates expression of a viral RNA or protein. In some embodiments, the biologic is selected from an iRNA or oligonucleotide or analog thereof, or antibody, antigen, or other therapeutic agent, disclosed in US 9,447,431, US 9,421,255, US 2013/0259879, EP2678038, WO 2012/113513, WO 2012/113413, US 2016/0206756, US 9,234,013, EP2603590, EP2796557, EP2603590, WO 2012/019780, WO 2012/019630, US 2016/0250321, US 9,226,959, US 2012/0021043, EP2176408, EP2176408, EP2548960, EP2176408, WO 2009/095226, EP2955230, US 8,968,746, US 2015/0258214, US 2013/0142818, EP2449113, EP2449113, EP2449113, WO 2012/013326, US 2016/0206719, US 2016/0130345, EP2958588, WO 2014/127917, US 2016/0185840, US 2016/0168254, US 2016/0166692, US 2016/0166691, US 2016/0166690, US 2016/0152706, US 2016/0152691, US 2016/0145346, US 2013/0195867, EP2101823, WO 2008/083949, US 2016/0184406, US 2014/0037660, US 2010/0203076, EP2484770, EP2188379, EP2484770, EP2188379, WO 2009/030481, WO 2009/030254, EP3035960, US 2016/0168207, WO 2015/024668, EP3036330, US 2016/0166710, WO 2015/024667, EP3035961, US 2016/0166711, WO 2015/024665, US 2016/0151474, US 2013/0295043, EP2680881, WO 2012/116811, WO 2012/116714, US 2015/0118264, EP2809353, WO 2013/113501, WO 2013/113326, US 2015/0118183, EP2809354, WO 2013/113502, WO 2013/113325, US 2015/0141498, EP2510100, WO 2011/069586, WO 2011/069529, US 2015/0057340, EP2814963, WO 2013/120629, WO 2013/120497, US 2015/0093413, EP2814962, WO 2013/120628, WO 2013/120499, EP2680880, US 2013/0336998, WO 2012/116715, WO 2012/116810, EP2650368, US 2013/0121988, US 2009/0324584, EP2046954, WO 2008/014979, US 2012/0213818, EP2762165, US 2011/0250225, EP2331129, EP2331129, WO 2010/037539, WO 2010/037408, US 2011/0053829, US 2010/0047261, EP2083851, WO 2008/052770, US 2008/0171711, US 2007/0280929, WO 2016/107877, WO 2016/091391, WO 2015/149944, WO 2015/135558, WO 2015/101414, WO 2015/101415, WO 2011/069587, WO 2011/069528, WO 2010/088927, or WO 2009/127230, the disclosure of each of which is hereby incorporated by reference.

In some embodiments, the biologic is useful in treating, preventing, or ameliorating an infectious disease (such as RSV or rabies) or cancer, a cardiovascular disease, an infectious disease, an autoimmune disease or genetic disease, or in gene therapy, or as an adjuvant or immunostimulating agent. In some embodiments, the biologic modulates expression of a viral RNA or protein. In some embodiments, the biologic is selected from an iRNA or oligonucleotide or analog thereof, or antibody, antigen, or other therapeutic agent, disclosed in US 2016/0166692, US 2013/0195867, EP2101823, WO 2008/083949, EP3035961, US 2016/0166711, WO 2015/024665, US 2015/0118264, EP2809353, WO 2013/113501, WO 2013/113326, US 2015/0118183, EP2809354, WO 2013/113502, WO 2013/113325, US 2015/0093413, EP2814962, WO 2013/120628, WO 2013/120499, EP2176408, US 2012/0021043, EP2176408, EP2548960, EP2176408, WO 2009/095226, US 2010/0047261, EP2083851, WO 2008/052770, EP2650368, US 2009/0324584, EP2046954, WO 2008/014979, US 2007/0280929, WO 2015/149944, or WO 2009/127230, the disclosure of each of which is hereby incorporated by reference.

In some embodiments, the biologic is useful in treating, preventing, or ameliorating an infectious disease (such as RSV or rabies) or cancer (such as prostate cancer), a cardiovascular disease, an infectious disease, an autoimmune disease or genetic disease, or is useful in gene therapy, or as an adjuvant or immunostimulating agent. In some embodiments, the biologic modulates expression of a viral RNA or protein. In some embodiments, the biologic is selected from an iRNA or oligonucleotide or analog thereof, or antibody, antigen, or other therapeutic agent, disclosed in US 9,447,431, US 9,421,255, US 2013/0259879, EP2678038, WO 2012/113513, WO 2012/113413, US 9,439,956, US 9,433,670, US 9,433,669, US 9,155,788, US 8,217,016, US 2016/0095911, US 2016/0089426, US 2016/0095912, US 2016/0089425, US 2016/0089424, US 2016/0082092, US 2015/0030633, US 2011/0311472, EP1458410, EP2769733,

EP1925317, EP1905844, EP1458410, WO 2003/051401, US 9,402,887, US 2013/0251742, EP2195015, WO 2009/046975, WO 2009/046739, US 9,352,028, US 2013/0202645, EP2197481, WO 2009/046974, WO 2009/046738, US 2016/0206756, US 9,234,013, EP2603590, EP2796557, EP2603590, WO 2012/019780, WO 2012/019630, US 2016/0250321, US 9,226,959, US 2012/0021043, EP2176408, EP2176408, EP2548960, EP2176408, WO 2009/095226, EP2955230, US 8,968,746, US 2015/0258214, US 2013/0142818, EP2449113, EP2449113, EP2449113, WO 2012/013326, US 2016/0206719, US 2016/0130345, EP2958588, WO 2014/127917, US 2016/0185840, US 2016/0168254, US 2016/0166692, US 2016/0166691, US 2016/0166690, US 2016/0152706, US 2016/0152691, US 2016/0145346, US 2013/0195867, EP2101823, WO 2008/083949, US 2016/0184406, US 2014/0037660, US 2010/0203076, EP2484770, EP2188379, EP2484770, EP2188379, WO 2009/030481, WO 2009/030254, EP3035955, US 2016/0168227, WO 2015/024666, EP3036330, US 2016/0166710, WO 2015/024667, EP3035954, US 2016/0166668, WO 2015/024664, US 2016/0151474, US 2013/0295043, EP2680881, WO 2012/116811, WO 2012/116714, US 2015/0320847, EP2814961, WO 2013/120627, WO 2013/120500, US 2015/0118264, EP2809353, WO 2013/113501, WO 2013/113326, US 2015/0118183, EP2809354, WO 2013/113502, WO 2013/113325, US 2015/0141498, EP2510100, WO 2011/069586, WO 2011/069529, US 2015/0057340, EP2814963, WO 2013/120629, WO 2013/120497, EP2680880, US 2013/0336998, WO 2012/116715, WO 2012/116810, EP2658569, US 2013/0280283, WO 2012/089338, WO 2012/089225, EP2650368, US 2013/0121988, US 2009/0324584, EP2046954, WO 2008/014979, EP2762165, US 2011/0250225, EP2331129, EP2331129, WO 2010/037539, WO 2010/037408, US 2011/0053829, US 2010/0047261, EP2083851, WO 2008/052770, US 2007/0280929, WO 2016/107877, WO 2016/091391, WO 2015/149944, WO 2015/135558, WO 2015/101414, WO 2015/101415, WO 2011/069587, WO 2011/069528, WO 2010/088927, or WO 2009/127230, the disclosure of each of which is hereby incorporated by reference.

In some embodiments, the biologic is useful in treating, preventing, or ameliorating an infectious disease (such as RSV or rabies) or cancer (such as prostate cancer), a cardiovascular disease, an infectious disease, an autoimmune disease or genetic disease, or in gene therapy, or as an adjuvant or immunostimulating agent. In some embodiments, the biologic modulates expression of a viral RNA or protein. In some embodiments, the biologic is selected from an iRNA or oligonucleotide or analog thereof, or antibody, antigen, or other therapeutic agent, disclosed in US 9,433,669, US 2016/0095911, US 2016/0089424, US 9,402,887, US 2013/0251742, EP2195015, WO 2009/046975, WO 2009/046739, EP3036330, US 2016/0166710, WO 2015/024667, EP3035954, US 2016/0166668, WO 2015/024664, US 2016/0151474, US 2013/0195867, EP2101823, WO 2008/083949, EP2650368, US 2013/0121988, US 2009/0324584, EP2046954, WO 2008/014979, EP2176408, US 2012/0021043, EP2176408, EP2548960, EP2176408, WO 2009/095226, US 2010/0047261, EP2083851, WO 2008/052770, US 2007/0280929, EP1881847, or WO 2009/127230, the disclosure of each of which is hereby incorporated by reference.

In some embodiments, the biologic is useful in treating, preventing, or ameliorating a cancer (such as prostate cancer or non-small cell lung cancer (NSCLC)), a cardiovascular disease, an autoimmune disease or genetic disease, or in gene therapy, or as an adjuvant or immunostimulating agent. In some embodiments, the biologic modulates expression of a viral RNA or protein. In some embodiments, the biologic is selected from an iRNA or oligonucleotide or analog thereof, or antibody, antigen, or other therapeutic agent, disclosed in US 9,447,431, US 9,352,028, US 2013/0202645, EP2197481, WO 2009/046974, WO 2009/046738, US 2016/0206719, US 2016/0130345, EP2958588, WO 2014/127917, US 2016/0185840, US 2016/0168254, US 2016/0166692, US 2016/0166691, US 2016/0166690, US 2016/0152706, US 2016/0152691, US 2016/0145346, US 2013/0195867, EP3035955, US 2016/0168227, WO 2015/024666, EP3036330, US 2016/0166710, WO 2015/024667, US 2016/0151474, US 2013/0295043, EP2680881, WO 2012/116811, WO 2012/116714, US 2015/0320847, EP2814961, WO 2013/120627, WO 2013/120500, US 2015/0141498, EP2510100, WO 2011/069586, WO 2011/069529, US 2015/0057340, EP2814963, WO 2013/120629, WO 2013/120497, EP2680880, US 2013/0336998, WO 2012/116715, WO 2012/116810, EP2762165, US 2011/0250225, EP2331129, EP2331129, WO 2010/037539, US 2011/0053829, WO 2016/107877, WO 2016/091391, WO 2015/135558, WO 2015/101414, WO 2015/101415, WO 2011/069587, or WO 2011/069528, the disclosure of each of which is hereby incorporated by reference.

In some embodiments, the biologic is useful in treating, preventing, or ameliorating a cancer (such as prostate cancer or non-small cell lung cancer (NSCLC)), a cardiovascular disease, an autoimmune disease or genetic disease, or in gene therapy, or as an adjuvant or immunostimulating agent. In some embodiments, the biologic modulates expression of a viral RNA or protein. In some embodiments, the biologic is selected from an iRNA or oligonucleotide or analog thereof, or antibody, antigen, or other therapeutic agent, disclosed in US 9,352,028, US 2013/0202645, EP2197481, WO 2009/046974, WO 2009/046738, EP3035955, US 2016/0168227, or WO 2015/024666, the disclosure of each of which is hereby incorporated by reference.

In some embodiments, the biologic is useful in treating, preventing, or ameliorating an infectious disease (such as RSV or rabies) or cancer (such as prostate cancer or non-small cell lung cancer (NSCLC)), a cardiovascular disease, an autoimmune disease or genetic disease, or in gene therapy, or as an adjuvant or immunostimulating agent. In some embodiments, the biologic modulates expression of a viral RNA or protein. In some embodiments, the biologic is selected

from an iRNA or oligonucleotide or analog thereof, or antibody, antigen, or other therapeutic agent, disclosed in US 9,447,431, US 9,421,255, US 2013/0259879, EP2678038, WO 2012/113513, WO 2012/113413, US 9,439,956, US 9,433,670, US 9,433,669, US 9,155,788, US 8,217,016, US 2016/0095911, US 2016/0089426, US 2016/0095912, US 2016/0089425, US 2016/0089424, US 2016/0082092, US 2015/0030633, US 2011/0311472, EP1458410, EP2769733, EP1925317, EP1905844, EP1458410, WO 2003/051401, US 2016/0206756, US 9,234,013, EP2603590, EP2796557, EP2603590, WO 2012/019780, WO 2012/019630, US 2016/0250321, US 9,226,959, US 2012/0021043, EP2176408, EP2176408, EP2548960, EP2176408, WO 2009/095226, EP2955230, US 8,968,746, US 2015/0258214, US 2013/0142818, EP2449113, EP2449113, EP2449113, WO 2012/013326, EP3062798, US 2016/0235864, WO 2015/062738, US 2016/0206719, US 2016/0130345, EP2958588, WO 2014/127917, US 2016/0185840, US 2016/0168254, US 2016/0166692, US 2016/0166691, US 2016/0166690, US 2016/0152706, US 2016/0152691, US 2016/0145346, US 2013/0195867, EP2101823, WO 2008/083949, US 2016/0184406, US 2014/0037660, US 2010/0203076, EP2484770, EP2188379, EP2484770, EP2188379, WO 2009/030481, WO 2009/030254, EP3035955, US 2016/0168227, WO 2015/024666, EP3035960, US 2016/0168207, WO 2015/024668, EP3036330, US 2016/0166710, WO 2015/024667, EP3035954, US 2016/0166668, WO 2015/024664, EP3035961, US 2016/0166711, WO 2015/024665, EP3035959, US 2016/0166678, WO 2015/024669, US 2016/0151474, US 2013/0295043, EP2680881, WO 2012/116811, WO 2012/116714, US 2016/0136301, US 2016/0136263, US 2016/0136259, US 2016/0136258, US 2016/0136247, US 2016/0136243, US 2016/0129105, US 2015/0104476, US 2011/0269950, US 2011/0077287, US 2010/0239608, EP2305699, EP1857122, EP1800697, EP1832603, EP1604688, EP1392341, EP2842964, EP2305699, EP1903054, EP1857122, EP1832603, EP1800697, EP1604688, EP1392341, US 2015/0218554, EP2831241, WO 2013/143699, US 2015/0118264, EP2809353, WO 2013/113501, WO 2013/113326, US 2015/0118183, EP2809354, WO 2013/113502, WO 2013/113325, US 2015/0141498, EP2510100, WO 2011/069586, WO 2011/069529, US 2015/0057340, EP2814963, WO 2013/120629, WO 2013/120497, US 2015/0093413, EP2814962, WO 2013/120628, WO 2013/120499, US 2015/0050302, EP2831240, WO 2013/143700, EP2680880, US 2013/0336998, WO 2012/116715, WO 2012/116810, EP2216027, US 2013/0273001, US 2010/0303851, EP1685844, EP1685844, EP1521585, EP2216028, P2216027, EP1806139, EP1797886, EP1685844, EP1685844, EP1521585, WO 2004/004743, EP2650368, US 2013/0121988, US 2009/0324584, EP2046954, WO 2008/014979, US 2012/0213818, EP1928494, WO 2006/024518, US 2012/0009221, EP1615662, EP2223700, EP2229953, EP1938833, EP1615662, WO 2005/016376, EP2762165, US 2011/0250225, EP2331129, EP2331129, WO 2010/037539, WO 2010/037408, US 2011/0053829, US 2010/0047261, EP2083851, WO 2008/052770, EP1881847, US 2008/0267873, EP1881847, WO 2006/122828, US 2008/0171711, EP1768703, WO 2006/008154, US 2007/0280929, WO 2007/095976, EP1619254, EP1083232, EP1818409, EP1619254, EP1541690, EP1541690, EP1083232, WO 2016/107877, WO 2016/097065, WO 2016/091391, WO 2015/188933, WO 2015/149944, WO 2015/135558, WO 2015/101414, WO 2015/101415, WO 2011/069587, WO 2011/069528, WO 2010/088927, or WO 2009/127230, the disclosure of each of which is hereby incorporated by reference.

In some embodiments, the biologic is useful in treating, preventing, or ameliorating an infectious disease (such as RSV or rabies) or cancer (such as prostate cancer or non-small cell lung cancer (NSCLC)), a cardiovascular disease, an infectious disease, an autoimmune disease or genetic disease, or in gene therapy, or as an adjuvant or immunostimulating agent. In some embodiments, the biologic modulates expression of a viral RNA or protein. In some embodiments, the biologic is selected from an iRNA or oligonucleotide or analog thereof, or antibody, antigen, or other therapeutic agent, disclosed in US 2016/0152691, US 2013/0195867, EP2101823, WO 2008/083949, EP3035959, US 2016/0166678, WO 2015/024669, US 2016/0129105, US 2010/0239608, EP1857122, EP1392341, US 2015/0118264, EP2809353, WO 2013/113501, WO 2013/113326, US 2015/0118183, EP2809354, WO 2013/113502, WO 2013/113325, US 2015/0093413, EP2814962, WO 2013/120628, WO 2013/120499, US 2012/0213818, EP1928494, WO 2006/024518, EP2176408, US 2012/0021043, EP2176408, EP2548960, EP2176408, WO 2009/095226, US 2010/0047261, EP2083851, WO 2008/052770, EP2650368, US 2009/0324584, EP2046954, WO 2008/014979, US 2007/0280929, WO 2007/095976, EP1768703, WO 2006/008154, WO 2015/149944, or WO 2009/127230, the disclosure of each of which is hereby incorporated by reference.

*Combination Therapies*

A provided therapeutic-loaded exosome, or pharmaceutically acceptable composition thereof, may be administered to a patient in need thereof in combination with one or more additional therapeutic agents and/or therapeutic processes.

A therapeutic-loaded exosome of the current invention can be administered alone or in combination with one or more other therapeutic compounds, possible combination therapy taking the form of fixed combinations or the administration of a therapeutic-loaded exosome of the invention and one or more other therapeutic compounds being staggered or given independently of one another, or the combined administration of fixed combinations and one or more other therapeutic compounds. A therapeutic-loaded exosome of the current invention can besides or in addition be

administered especially for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

Such additional agents may be administered separately from a provided therapeutic-loaded exosome-containing composition, as part of a multiple dosage regimen. Alternatively, those agents may be part of a single dosage form, mixed together with a therapeutic-loaded exosome of this invention in a single composition. If administered as part of a multiple dosage regime, the two active agents may be submitted simultaneously, sequentially or within a period of time from one another.

As used herein, the term "combination," "combined," and related terms refers to the simultaneous or sequential administration of therapeutic agents in accordance with this invention. For example, a therapeutic-loaded exosome of the present invention may be administered with another therapeutic agent simultaneously or sequentially in separate unit dosage forms or together in a single unit dosage form. Accordingly, the present invention provides a single unit dosage form comprising a therapeutic-loaded exosome of the current invention, an additional therapeutic agent, and a pharmaceutically acceptable carrier, adjuvant, or vehicle. In some embodiments, the additional agent is encapsulated in the same exosome as the first therapeutic agent. In some embodiments, the additional agent is encapsulated in a different exosome than the first therapeutic agent. In some embodiments, the additional agent is not encapsulated in an exosome. In some embodiments, the additional agent is formulated in a separate composition from the therapeutic-loaded exosome.

The amount of both a disclosed therapeutic-loaded exosome and additional therapeutic agent (in those compositions which comprise an additional therapeutic agent as described above) that may be combined with the carrier materials to produce a single dosage form will vary depending upon the patient treated and the particular mode of administration. In certain embodiments, compositions of this invention should be formulated so that a dosage of between 0.01-100 mg/kg body weight/day of a disclosed therapeutic-loaded exosome can be administered.

In those compositions which comprise an additional therapeutic agent, that additional therapeutic agent and the therapeutic-loaded exosome of this invention may act synergistically. Therefore, the amount of additional therapeutic agent in such compositions will be less than that required in a monotherapy utilizing only that therapeutic agent. In such compositions a dosage of between 0.01-1,000 μg/kg body weight/day of the additional therapeutic agent can be administered.

The amount of additional therapeutic agent present in the compositions of this invention will be no more than the amount that would normally be administered in a composition comprising that therapeutic agent as the only active agent. Preferably the amount of additional therapeutic agent in the presently disclosed compositions will range from about 50% to 100% of the amount normally present in a composition comprising that agent as the only therapeutically active agent.

Examples of agents with which the therapeutic-loaded exosomes of this invention may be combined include, without limitation: treatments for Alzheimer's Disease such as Aricept® and Excelon®; treatments for HIV such as ritonavir; treatments for Parkinson's Disease such as L-DOPA/carbidopa, entacapone, ropinrole, pramipexole, bromocriptine, pergolide, trihexephendyl, and amantadine; agents for treating Multiple Sclerosis (MS) such as beta interferon (e.g., Avonex® and Rebif®), Copaxone®, and mitoxantrone; treatments for asthma such as albuterol and Singulair®; agents for treating schizophrenia such as zyprexa, risperdal, seroquel, and haloperidol; anti-inflammatory agents such as corticosteroids, TNF blockers, IL-1 RA, azathioprine, cyclophosphamide, and sulfasalazine; immunomodulatory and immunosuppressive agents such as cyclosporin, tacrolimus, rapamycin, mycophenolate mofetil, interferons, corticosteroids, cyclophophamide, azathioprine, and sulfasalazine; neurotrophic factors such as acetylcholinesterase inhibitors, MAO inhibitors, interferons, anti-convulsants, ion channel blockers, riluzole, and anti-Parkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders such as corticosteroids, anti-leukemic agents, and growth factors; agents that prolong or improve pharmacokinetics such as cytochrome P450 inhibitors (i.e., inhibitors of metabolic breakdown) and CYP3A4 inhibitors (e.g., ketokenozole and ritonavir), and agents for treating immunodeficiency disorders such as gamma globulin.

In certain embodiments, combination therapies of the present invention, or a pharmaceutically acceptable composition thereof, include a monoclonal antibody or a siRNA therapeutic, which may or may not be encapsulated in a disclosed exosome.

In another embodiment, the present invention provides a method of treating an inflammatory disease, disorder or condition by administering to a patient in need thereof a therapeutic-loaded exosome and one or more additional therapeutic agents. Such additional therapeutic agents may be small molecules or a biologic and include, for example, acetaminophen, non-steroidal anti-inflammatory drugs (NSAIDS) such as aspirin, ibuprofen, naproxen, etodolac (Lodine®) and celecoxib, colchicine (Colcrys®), corticosteroids such as prednisone, prednisolone, methylprednisolone, hydrocortisone, and the like, probenecid, allopurinol, febuxostat (Uloric®), sulfasalazine (Azulfidine®), antimalarials such

as hydroxychloroquine (Plaquenil®) and chloroquine (Aralen®), methotrexate (Rheumatrex®), gold salts such as gold thioglucose (Solganal®), gold thiomalate (Myochrysine®) and auranofin (Ridaura®), D-penicillamine (Depen® or Cuprimine®), azathioprine (Imuran®), cyclophosphamide (Cytoxan®), chlorambucil (Leukeran®), cyclosporine (Sandimmune®), leflunomide (Arava®) and "anti-TNF" agents such as etanercept (Enbrel®), infliximab (Remicade®), golimumab (Simponi®), certolizumab pegol (Cimzia®) and adalimumab (Humira®), "anti-IL-1" agents such as anakinra (Kineret®) and rilonacept (Arcalyst®), canakinumab (Ilaris®), anti-Jak inhibitors such as tofacitinib, antibodies such as rituximab (Rituxan®), "anti-T-cell" agents such as abatacept (Orencia®), "anti-IL-6" agents such as tocilizumab (Actemra®), diclofenac, cortisone, hyaluronic acid (Synvisc® or Hyalgan®), monoclonal antibodies such as tanezumab, anticoagulants such as heparin (Calcinparine® or Liquaemin®) and warfarin (Coumadin®), antidiarrheals such as diphenoxylate (Lomotil®) and loperamide (Imodium®), bile acid binding agents such as cholestyramine, alosetron (Lotronex®), lubiprostone (Amitiza®), laxatives such as Milk of Magnesia, polyethylene glycol (MiraLax®), Dulcolax®, Correctol® and Senokot®, anticholinergics or antispasmodics such as dicyclomine (Bentyl®), Singulair®, beta-2 agonists such as albuterol (Ventolin® HFA, Proventil® HFA), levalbuterol (Xopenex®), metaproterenol (Alupent®), pirbuterol acetate (Maxair®), terbutaline sulfate (Brethaire®), salmeterol xinafoate (Serevent®) and formoterol (Foradil®), anticholinergic agents such as ipratropium bromide (Atrovent®) and tiotropium (Spiriva®), inhaled corticosteroids such as beclomethasone dipropionate (Beclovent®, Qvar®, and Vanceril®), triamcinolone acetonide (Azmacort®), mometasone (Asthmanex®), budesonide (Pulmocort®), and flunisolide (Aerobid®), Afviar®, Symbicort®, Dulera®, cromolyn sodium (Intal®), methylxanthines such as theophylline (Theo-Dur®, Theolair®, Slo-bid®, Uniphyl®, Theo-24®) and aminophylline, IgE antibodies such as omalizumab (Xolair®), nucleoside reverse transcriptase inhibitors such as zidovudine (Retrovir®), abacavir (Ziagen®), abacavir/lamivudine (Epzicom®), abacavir/lamivudine/zidovudine (Trizivir®), didanosine (Videx®), emtricitabine (Emtriva®), lamivudine (Epivir®), lamivudine/zidovudine (Combivir®), stavudine (Zerit®), and zalcitabine (Hivid®), non-nucleoside reverse transcriptase inhibitors such as delavirdine (Rescriptor®), efavirenz (Sustiva®), nevairapine (Viramune®) and etravirine (Intelence®), nucleotide reverse transcriptase inhibitors such as tenofovir (Viread®), protease inhibitors such as amprenavir (Agenerase®), atazanavir (Reyataz®), darunavir (Prezista®), fosamprenavir (Lexiva®), indinavir (Crixivan®), lopinavir and ritonavir (Kaletra®), nelfinavir (Viracept®), ritonavir (Norvir®), saquinavir (Fortovase® or Invirase®), and tipranavir (Aptivus®), entry inhibitors such as enfuvirtide (Fuzeon®) and maraviroc (Selzentry®), integrase inhibitors such as raltegravir (Isentress®), doxorubicin (Hydrodaunorubicin®), vincristine (Oncovin®), bortezomib (Velcade®), and dexamethasone (Decadron®) in combination with lenalidomide (Revlimid®), or any combination(s) thereof.

In another embodiment, the present invention provides a method of treating gout comprising administering to a patient in need thereof a therapeutic-loaded exosome and one or more additional therapeutic agents selected from non-steroidal anti-inflammatory drugs (NSAIDS) such as aspirin, ibuprofen, naproxen, etodolac (Lodine®) and celecoxib, colchicine (Colcrys®), corticosteroids such as prednisone, prednisolone, methylprednisolone, hydrocortisone, and the like, probenecid, allopurinol and febuxostat (Uloric®).

In another embodiment, the present invention provides a method of treating rheumatoid arthritis comprising administering to a patient in need thereof a therapeutic-loaded exosome and one or more additional therapeutic agents selected from non-steroidal anti-inflammatory drugs (NSAIDS) such as aspirin, ibuprofen, naproxen, etodolac (Lodine®) and celecoxib, corticosteroids such as prednisone, prednisolone, methylprednisolone, hydrocortisone, and the like, sulfasalazine (Azulfidine®), antimalarials such as hydroxychloroquine (Plaquenil®) and chloroquine (Aralen®), methotrexate (Rheumatrex®), gold salts such as gold thioglucose (Solganal®), gold thiomalate (Myochrysine®) and auranofin (Ridaura®), D-penicillamine (Depen® or Cuprimine®), azathioprine (Imuran®), cyclophosphamide (Cytoxan®), chlorambucil (Leukeran®), cyclosporine (Sandimmune®), leflunomide (Arava®) and "anti-TNF" agents such as etanercept (Enbrel®), infliximab (Remicade®), golimumab (Simponi®), certolizumab pegol (Cimzia®) and adalimumab (Humira®), "anti-IL-1" agents such as anakinra (Kineret®) and rilonacept (Arcalyst®), antibodies such as rituximab (Rituxan®), "anti-T-cell" agents such as abatacept (Orencia®) and "anti-IL-6" agents such as tocilizumab (Actemra®).

In some embodiments, the present invention provides a method of treating osteoarthritis comprising administering to a patient in need thereof a therapeutic-loaded exosome and one or more additional therapeutic agents selected from acetaminophen, non-steroidal anti-inflammatory drugs (NSAIDS) such as aspirin, ibuprofen, naproxen, etodolac (Lodine®) and celecoxib, diclofenac, cortisone, hyaluronic acid (Synvisc® or Hyalgan®) and monoclonal antibodies such as tanezumab.

In some embodiments, the present invention provides a method of treating lupus comprising administering to a patient in need thereof a therapeutic-loaded exosome and one or more additional therapeutic agents selected from acetaminophen, non-steroidal anti-inflammatory drugs (NSAIDS) such as aspirin, ibuprofen, naproxen, etodolac (Lodine®) and celecoxib, corticosteroids such as prednisone, prednisolone, methylprednisolone, hydrocortisone, and the like, antimalarials such as hydroxychloroquine (Plaquenil®) and chloroquine (Aralen®), cyclophosphamide (Cytoxan®), methotrexate (Rheumatrex®), azathioprine (Imuran®) and anticoagulants such as heparin (Calcinparine® or Liquaemin®) and warfarin (Coumadin®).

In some embodiments, the present invention provides a method of treating inflammatory bowel disease comprising administering to a patient in need thereof a therapeutic-loaded exosome and one or more additional therapeutic

agents selected from mesalamine (Asacol®) sulfasalazine (Azulfidine®), antidiarrheals such as diphenoxylate (Lomotil®) and loperamide (Imodium®), bile acid binding agents such as cholestyramine, alosetron (Lotronex®), lubiprostone (Amitiza®), laxatives such as Milk of Magnesia, polyethylene glycol (MiraLax®), Dulcolax®, Correctol® and Senokot® and anticholinergics or antispasmodics such as dicyclomine (Bentyl®), anti-TNF therapies, steroids, and antibiotics such as Flagyl or ciprofloxacin.

In some embodiments, the present invention provides a method of treating asthma comprising administering to a patient in need thereof a therapeutic-loaded exosome and one or more additional therapeutic agents selected from Singulair®, beta-2 agonists such as albuterol (Ventolin® HFA, Proventil® HFA), levalbuterol (Xopenex®), metaproterenol (Alupent®), pirbuterol acetate (Maxair®), terbutaline sulfate (Brethaire®), salmeterol xinafoate (Serevent®) and formoterol (Foradil®), anticholinergic agents such as ipratropium bromide (Atrovent®) and tiotropium (Spiriva®), inhaled corticosteroids such as prednisone, prednisolone, beclomethasone dipropionate (Beclovent®, Qvar®, and Vanceril®), triamcinolone acetonide (Azmacort®), mometasone (Asthmanex®), budesonide (Pulmocort®), flunisolide (Aerobid®), Afviar®, Symbicort®, and Dulera®, cromolyn sodium (Intal®), methylxanthines such as theophylline (Theo-Dur®, Theolair®, Slo-bid®, Uniphyl®, Theo-24®) and aminophylline, and IgE antibodies such as omalizumab (Xolair®).

In some embodiments, the present invention provides a method of treating COPD comprising administering to a patient in need thereof a therapeutic-loaded exosomes and one or more additional therapeutic agents selected from beta-2 agonists such as albuterol (Ventolin® HFA, Proventil® HFA), levalbuterol (Xopenex®), metaproterenol (Alupent®), pirbuterol acetate (Maxair®), terbutaline sulfate (Brethaire®), salmeterol xinafoate (Serevent®) and formoterol (Foradil®), anticholinergic agents such as ipratropium bromide (Atrovent®) and tiotropium (Spiriva®), methylxanthines such as theophylline (Theo-Dur®, Theolair®, Slo-bid®, Uniphyl®, Theo-24®) and aminophylline, inhaled corticosteroids such as prednisone, prednisolone, beclomethasone dipropionate (Beclovent®, Qvar®, and Vanceril®), triamcinolone acetonide (Azmacort®), mometasone (Asthmanex®), budesonide (Pulmocort®), flunisolide (Aerobid®), Afviar®, Symbicort®, and Dulera®, and combinations thereof.

In some embodiments, the present invention provides a method of treating HIV comprising administering to a patient in need thereof a therapeutic-loaded exosome and one or more additional therapeutic agents selected from nucleoside reverse transcriptase inhibitors such as zidovudine (Retrovir®), abacavir (Ziagen®), abacavir/lamivudine (Epzicom®), abacavir/lamivudine/zidovudine (Trizivir®), didanosine (Videx®), emtricitabine (Emtriva®), lamivudine (Epivir®), lamivudine/zidovudine (Combivir®), stavudine (Zerit®), and zalcitabine (Hivid®), non-nucleoside reverse transcriptase inhibitors such as delavirdine (Rescriptor®), efavirenz (Sustiva®), nevairapine (Viramune®) and etravirine (Intelence®), nucleotide reverse transcriptase inhibitors such as tenofovir (Viread®), protease inhibitors such as amprenavir (Agenerase®), atazanavir (Reyataz®), darunavir (Prezista®), fosamprenavir (Lexiva®), indinavir (Crixivan®), lopinavir and ritonavir (Kaletra®), nelfinavir (Viracept®), ritonavir (Norvir®), saquinavir (Fortovase® or Invirase®), and tipranavir (Aptivus®), entry inhibitors such as enfuvirtide (Fuzeon®) and maraviroc (Selzentry®), integrase inhibitors such as raltegravir (Isentress®), and combinations thereof.

In another embodiment, the present invention provides a method of treating a hematological malignancy comprising administering to a patient in need thereof a therapeutic-loaded exosome and one or more additional therapeutic agents selected from rituximab (Rituxan®), cyclophosphamide (Cytoxan®), doxorubicin (Hydrodaunorubicin®), vincristine (Oncovin®), prednisone, a hedgehog signaling inhibitor, a Bcl-2 inhibitor, a BTK inhibitor, a JAK/pan-JAK inhibitor, a TYK2 inhibitor, a PI3K inhibitor, a SYK inhibitor, and combinations thereof.

In another embodiment, the present invention provides a method of treating a solid tumor comprising administering to a patient in need thereof a therapeutic-loaded exosome and one or more additional therapeutic agents selected from rituximab (Rituxan®), cyclophosphamide (Cytoxan®), doxorubicin (Hydrodaunorubicin®), vincristine (Oncovin®), prednisone, a hedgehog signaling inhibitor, a Bcl-2 inhibitor, a BTK inhibitor, a JAK/pan-JAK inhibitor, a TYK2 inhibitor, a PI3K inhibitor, a SYK inhibitor, and combinations thereof.

In another embodiment, the present invention provides a method of treating a hematological malignancy comprising administering to a patient in need thereof a therapeutic-loaded exosome and a Hedgehog (Hh) signaling pathway inhibitor. In some embodiments, the hematological malignancy is DLBCL.

In another embodiment, the present invention provides a method of treating diffuse large B-cell lymphoma (DLBCL) comprising administering to a patient in need thereof a therapeutic-loaded exosome and one or more additional therapeutic agents selected from rituximab (Rituxan®), cyclophosphamide (Cytoxan®), doxorubicin (Hydrodaunorubicin®), vincristine (Oncovin®), prednisone, a hedgehog signaling inhibitor, and combinations thereof.

In another embodiment, the present invention provides a method of treating multiple myeloma comprising administering to a patient in need thereof a therapeutic-loaded exosome and one or more additional therapeutic agents selected from bortezomib (Velcade®), and dexamethasone (Decadron®), a hedgehog signaling inhibitor, a Bcl-2 inhibitor, a BTK inhibitor, a JAK/pan-JAK inhibitor, a TYK2 inhibitor, a PI3K inhibitor, a SYK inhibitor in combination with lenalidomide (Revlimid®).

In another embodiment, the present invention provides a method of treating Waldenstrom's macroglobulinemia comprising administering to a patient in need thereof a therapeutic-loaded exosome and one or more additional therapeutic agents selected from chlorambucil (Leukeran®), cyclophosphamide (Cytoxan®, Neosar®), fludarabine (Fludara®), cladribine (Leustatin®), rituximab (Rituxan®), a hedgehog signaling inhibitor, a Bcl-2 inhibitor, a BTK inhibitor, a JAK/pan-JAK inhibitor, a TYK2 inhibitor, a PI3K inhibitor, and a SYK inhibitor.

In some embodiments, the present invention provides a method of treating Alzheimer's disease comprising administering to a patient in need thereof a therapeutic-loaded exosome and one or more additional therapeutic agents selected from donepezil (Aricept®), rivastigmine (Excelon®), galantamine (Razadyne®), tacrine (Cognex®), and memantine (Namenda®).

In another embodiment, the present invention provides a method of treating organ transplant rejection or graft vs. host disease comprising administering to a patient in need thereof a therapeutic-loaded exosome and one or more additional therapeutic agents selected from a steroid, cyclosporin, FK506, rapamycin, a hedgehog signaling inhibitor, a Bcl-2 inhibitor, a BTK inhibitor, a JAK/pan-JAK inhibitor, a TYK2 inhibitor, a PI3K inhibitor, and a SYK inhibitor.

In another embodiment, the present invention provides a method of treating or lessening the severity of a disease comprising administering to a patient in need thereof a therapeutic-loaded exosome and a BTK inhibitor, wherein the disease is selected from inflammatory bowel disease, arthritis, systemic lupus erythematosus (SLE), vasculitis, idiopathic thrombocytopenic purpura (ITP), rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, autoimmune thyroiditis, Sjogren's syndrome, multiple sclerosis, systemic sclerosis, Lyme neuroborreliosis, Guillain-Barre syndrome, acute disseminated encephalomyelitis, Addison's disease, opsoclonus-myoclonus syndrome, ankylosing spondylosis, antiphospholipid antibody syndrome, aplastic anemia, autoimmune hepatitis, autoimmune gastritis, pernicious anemia, celiac disease, Goodpasture's syndrome, idiopathic thrombocytopenic purpura, optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behcet's disease, chronic fatigue, dysautonomia, membranous glomerulonephropathy, endometriosis, interstitial cystitis, pemphigus vulgaris, bullous pemphigoid, neuromyotonia, scleroderma, vulvodynia, a hyperproliferative disease, rejection of transplanted organs or tissues, Acquired Immunodeficiency Syndrome (AIDS, caused by HIV), type 1 diabetes, graft versus host disease, transplantation, transfusion, anaphylaxis, allergies (e.g., allergies to plant pollens, latex, drugs, foods, insect poisons, animal hair, animal dander, dust mites, or cockroach calyx), type I hypersensitivity, allergic conjunctivitis, allergic rhinitis, and atopic dermatitis, asthma, appendicitis, atopic dermatitis, asthma, allergy, blepharitis, bronchiolitis, bronchitis, bursitis, cervicitis, cholangitis, cholecystitis, chronic graft rejection, colitis, conjunctivitis, Crohn's disease, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, Henoch-Schonlein purpura, hepatitis, hidradenitis suppurativa, immunoglobulin A nephropathy, interstitial lung disease, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, polymyositis, proctitis, prostatitis, pyelonephritis, rhinitis, salpingitis, sinusitis, stomatitis, synovitis, tendonitis, tonsillitis, ulcerative colitis, uveitis, vaginitis, vasculitis, or vulvitis, B-cell proliferative disorder, e.g., diffuse large B cell lymphoma, follicular lymphoma, chronic lymphocytic lymphoma, chronic lymphocytic leukemia, acute lymphocytic leukemia, B-cell prolymphocytic leukemia, lymphoplasmacytic lymphoma/Waldenstrom macroglobulinemia, splenic marginal zone lymphoma, multiple myeloma (also known as plasma cell myeloma), non-Hodgkin's lymphoma, Hodgkin's lymphoma, plasmacytoma, extranodal marginal zone B cell lymphoma, nodal marginal zone B cell lymphoma, mantle cell lymphoma, mediastinal (thymic) large B cell lymphoma, intravascular large B cell lymphoma, primary effusion lymphoma, Burkitt lymphoma/leukemia, or lymphomatoid granulomatosis, breast cancer, prostate cancer, or cancer of the mast cells (e.g., mastocytoma, mast cell leukemia, mast cell sarcoma, systemic mastocytosis), bone cancer, colorectal cancer, pancreatic cancer, diseases of the bone and joints including, without limitation, rheumatoid arthritis, seronegative spondyloarthropathies (including ankylosing spondylitis, psoriatic arthritis and Reiter's disease), Behcet's disease, Sjogren's syndrome, systemic sclerosis, osteoporosis, bone cancer, bone metastasis, a thromboembolic disorder, (e.g., myocardial infarct, angina pectoris, reocclusion after angioplasty, restenosis after angioplasty, reocclusion after aortocoronary bypass, restenosis after aortocoronary bypass, stroke, transitory ischemia, a peripheral arterial occlusive disorder, pulmonary embolism, deep venous thrombosis), inflammatory pelvic disease, urethritis, skin sunburn, sinusitis, pneumonitis, encephalitis, meningitis, myocarditis, nephritis, osteomyelitis, myositis, hepatitis, gastritis, enteritis, dermatitis, gingivitis, appendicitis, pancreatitis, cholocystitus, agammaglobulinemia, psoriasis, allergy, Crohn's disease, irritable bowel syndrome, ulcerative colitis, Sjogren's disease, tissue graft rejection, hyperacute rejection of transplanted organs, asthma, allergic rhinitis, chronic obstructive pulmonary disease (COPD), autoimmune polyglandular disease (also known as autoimmune polyglandular syndrome), autoimmune alopecia, pernicious anemia, glomerulonephritis, dermatomyositis, multiple sclerosis, scleroderma, vasculitis, autoimmune hemolytic and thrombocytopenic states, Goodpasture's syndrome, atherosclerosis, Addison's disease, Parkinson's disease, Alzheimer's disease, diabetes, septic shock, systemic lupus erythematosus (SLE), rheumatoid arthritis, psoriatic arthritis, juvenile arthritis, osteoarthritis, chronic idiopathic thrombocytopenic purpura, Waldenstrom macroglobulinemia, myasthenia gravis, Hashimoto's thyroiditis, atopic dermatitis, degenerative joint disease, vitiligo, autoimmune hypopituitarism, Guillain-Barre syndrome, Behcet's disease, scleraderma, mycosis fungoides, acute

inflammatory responses (such as acute respiratory distress syndrome and ischemia/reperfusion injury), and Graves' disease.

In some embodiments the present invention provides a method of treating or lessening the severity of a disease comprising administering to a patient in need thereof a therapeutic-loaded exosome and a Bcl-2 inhibitor, wherein the disease is an inflammatory disorder, an autoimmune disorder, a proliferative disorder, an endocrine disorder, a neurological disorder, or a disorder associated with transplantation. In some embodiments, the disorder is a proliferative disorder, lupus, or lupus nephritis. In some embodiments, the proliferative disorder is chronic lymphocytic leukemia, diffuse large B-cell lymphoma, Hodgkin's disease, small-cell lung cancer, non-small-cell lung cancer, myelodysplastic syndrome, lymphoma, a hematological neoplasm, or a solid tumor.

In another embodiment, the present invention provides a method of treating or lessening the severity of a disease comprising administering to a patient in need thereof a therapeutic-loaded exosome and a PI3K inhibitor, wherein the disease is selected from a cancer, a neurodegenerative disorder, an angiogenic disorder, a viral disease, an autoimmune disease, an inflammatory disorder, a hormone-related disease, conditions associated with organ transplantation, immunodeficiency disorders, a destructive bone disorder, a proliferative disorder, an infectious disease, a condition associated with cell death, thrombin-induced platelet aggregation, chronic myelogenous leukemia (CML), chronic lymphocytic leukemia (CLL), liver disease, pathologic immune conditions involving T cell activation, a cardiovascular disorder, and a CNS disorder.

In another embodiment, the present invention provides a method of treating or lessening the severity of a disease comprising administering to a patient in need thereof a therapeutic-loaded exosome and a PI3K inhibitor, wherein the disease is selected from benign or malignant tumor, carcinoma or solid tumor of the brain, kidney (e.g., renal cell carcinoma (RCC)), liver, adrenal gland, bladder, breast, stomach, gastric tumors, ovaries, colon, rectum, prostate, pancreas, lung, vagina, endometrium, cervix, testis, genitourinary tract, esophagus, larynx, skin, bone or thyroid, sarcoma, glioblastomas, neuroblastomas, multiple myeloma or gastrointestinal cancer, especially colon carcinoma or colorectal adenoma or a tumor of the neck and head, an epidermal hyperproliferation, psoriasis, prostate hyperplasia, a neoplasia, a neoplasia of epithelial character, adenoma, adenocarcinoma, keratoacanthoma, epidermoid carcinoma, large cell carcinoma, non-small-cell lung carcinoma, lymphomas, (including, for example, non-Hodgkin's Lymphoma (NHL) and Hodgkin's lymphoma (also termed Hodgkin's or Hodgkin's disease)), a mammary carcinoma, follicular carcinoma, undifferentiated carcinoma, papillary carcinoma, seminoma, melanoma, or a leukemia, diseases include Cowden syndrome, Lhermitte-Dudos disease and Bannayan-Zonana syndrome, or diseases in which the PI3K/PKB pathway is aberrantly activated, asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection, acute lung injury (ALI), adult/acute respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy, bronchitis of whatever type or genesis including, but not limited to, acute, arachidic, catarrhal, croupus, chronic or phthinoid bronchitis, pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis, Loffler's syndrome, eosinophilic, pneumonia, parasitic (in particular metazoan) infestation (including tropical eosinophilia), bronchopulmonary aspergillosis, polyarteritis nodosa (including Churg-Strauss syndrome), eosinophilic granuloma and eosinophil-related disorders affecting the airways occasioned by drug-reaction, psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphisus, epidermolysis bullosa acquisita, conjunctivitis, keratoconjunctivitis sicca, and vernal conjunctivitis, diseases affecting the nose including allergic rhinitis, and inflammatory disease in which autoimmune reactions are implicated or having an autoimmune component or etiology, including autoimmune hematological disorders (e.g. hemolytic anemia, aplastic anemia, pure red cell anemia and idiopathic thrombocytopenia), systemic lupus erythematosus, rheumatoid arthritis, polychondritis, sclerodoma, Wegener granulamatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), endocrine opthalmopathy, Grave's disease, sarcoidosis, alveolitis, chronic hypersensitivity pneumonitis, multiple sclerosis, primary biliary cirrhosis, uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minal change nephropathy, restenosis, cardiomegaly, atherosclerosis, myocardial infarction, ischemic stroke and congestive heart failure, Alzheimer's disease, Parkinson's disease, amyotrophic lateral sclerosis, Huntington's disease, and cerebral ischemia, and neurodegenerative disease caused by traumatic injury, glutamate neurotoxicity and hypoxia.

A therapeutic-loaded exosome of the current invention may also be used to advantage in combination with an antiproliferative compound. Such antiproliferative compounds include, but are not limited to, aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; matrix metalloproteinase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (Temodal®); kinesin spindle protein inhibitors, such as SB715992 or SB743921 from GlaxoSmithKline, or pentamidine/chlorpromazine from CombinatoRx; MEK inhibitors such as ARRY142886 from Array BioPharma, AZD6244 from AstraZeneca, PD181461 from Pfizer and leucovorin. The term "aromatase inhibitor" as used herein relates to a compound which inhibits estrogen production, for instance, the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole. Exemestane is marketed under the trade name Aromasin™. Formestane is marketed under the trade name Lentaron™. Fadrozole is marketed under the trade name Afema™. Anastrozole is marketed under the trade name Arimidex™. Letrozole is marketed under the trade names Femara™ or Femar™. Aminoglutethimide is marketed under the trade name Orimeten™. A combination of the invention comprising a chemotherapeutic agent which is an aromatase inhibitor is particularly useful for the treatment of hormone receptor positive tumors, such as breast tumors.

The term "antiestrogen" as used herein relates to a compound which antagonizes the effect of estrogens at the estrogen receptor level. The term includes, but is not limited to tamoxifen, fulvestrant, raloxifene and raloxifene hydrochloride. Tamoxifen is marketed under the trade name Nolvadex™. Raloxifene hydrochloride is marketed under the trade name Evista™. Fulvestrant can be administered under the trade name Faslodex™. A combination of the invention comprising a chemotherapeutic agent which is an antiestrogen is particularly useful for the treatment of estrogen receptor positive tumors, such as breast tumors.

The term "anti-androgen" as used herein relates to any substance which is capable of inhibiting the biological effects of androgenic hormones and includes, but is not limited to, bicalutamide (Casodex™). The term "gonadorelin agonist" as used herein includes, but is not limited to abarelix, goserelin and goserelin acetate. Goserelin can be administered under the trade name Zoladex™.

The term "topoisomerase I inhibitor" as used herein includes, but is not limited to topotecan, gimatecan, irinotecan, camptothecian and its analogues, 9-nitrocamptothecin and the macromolecular camptothecin conjugate PNU-166148. Irinotecan can be administered, e.g. in the form as it is marketed, e.g. under the trademark Camptosar™. Topotecan is marketed under the trade name Hycamptin™.

The term "topoisomerase II inhibitor" as used herein includes, but is not limited to the anthracyclines such as doxorubicin (including liposomal formulation, such as Caelyx™), daunorubicin, epirubicin, idarubicin and nemorubicin, the anthraquinones mitoxantrone and losoxantrone, and the podophillotoxines etoposide and teniposide. Etoposide is marketed under the trade name Etopophos™. Teniposide is marketed under the trade name VM 26-Bristol Doxorubicin is marketed under the trade name Acriblastin™ or Adriamycin™. Epirubicin is marketed under the trade name Farmorubicin™. Idarubicin is marketed under the trade name Zavedos™. Mitoxantrone is marketed under the trade name Novantron.

The term "microtubule active agent" relates to microtubule stabilizing, microtubule destabilizing compounds and microtublin polymerization inhibitors including, but not limited to taxanes, such as paclitaxel and docetaxel; vinca alkaloids, such as vinblastine or vinblastine sulfate, vincristine or vincristine sulfate, and vinorelbine; discodermolides; cochicine and epothilones and derivatives thereof. Paclitaxel is marketed under the trade name Taxol™. Docetaxel is marketed under the trade name Taxotere™. Vinblastine sulfate is marketed under the trade name Vinblastin R.P™. Vincristine sulfate is marketed under the trade name Farmistin™.

The term "alkylating agent" as used herein includes, but is not limited to, cyclophosphamide, ifosfamide, melphalan or nitrosourea (BCNU or Gliadel). Cyclophosphamide is marketed under the trade name Cyclostin™. Ifosfamide is marketed under the trade name Holoxan™.

The term "histone deacetylase inhibitors" or "HDAC inhibitors" relates to compounds which inhibit the histone deacetylase and which possess antiproliferative activity. This includes, but is not limited to, suberoylanilide hydroxamic acid (SAHA).

The term "antineoplastic antimetabolite" includes, but is not limited to, 5-fluorouracil or 5-FU, capecitabine, gemcitabine, DNA demethylating compounds, such as 5-azacytidine and decitabine, methotrexate and edatrexate, and folic acid antagonists such as pemetrexed. Capecitabine is marketed under the trade name Xeloda™. Gemcitabine is

marketed under the trade name Gemzar™.

The term "platin compound" as used herein includes, but is not limited to, carboplatin, cis-platin, cisplatinum and oxaliplatin. Carboplatin can be administered, e.g., in the form as it is marketed, e.g. under the trademark Carboplat™. Oxaliplatin can be administered, e.g., in the form as it is marketed, e.g. under the trademark Eloxatin™.

The term "compounds targeting/decreasing a protein or lipid kinase activity; or a protein or lipid phosphatase activity; or further anti-angiogenic compounds" as used herein includes, but is not limited to, protein tyrosine kinase and/or serine and/or threonine kinase inhibitors or lipid kinase inhibitors, such as a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, such as an N-phenyl-2-pyrimidine-amine derivative, such as imatinib, SU101, SU6668 and GFB-111; b) compounds targeting, decreasing or inhibiting the activity of the fibroblast growth factor-receptors (FGFR); c) compounds targeting, decreasing or inhibiting the activity of the insulin-like growth factor receptor I (IGF-IR), such as compounds which target, decrease or inhibit the activity of IGF-IR, especially compounds which inhibit the kinase activity of IGF-I receptor, or antibodies that target the extracellular domain of IGF-I receptor or its growth factors; d) compounds targeting, decreasing or inhibiting the activity of the Trk receptor tyrosine kinase family, or ephrin B4 inhibitors; e) compounds targeting, decreasing or inhibiting the activity of the Axl receptor tyrosine kinase family; f) compounds targeting, decreasing or inhibiting the activity of the Ret receptor tyrosine kinase; g) compounds targeting, decreasing or inhibiting the activity of the Kit/SCFR receptor tyrosine kinase, such as imatinib; h) compounds targeting, decreasing or inhibiting the activity of the C-kit receptor tyrosine kinases, which are part of the PDGFR family, such as compounds which target, decrease or inhibit the activity of the c-Kit receptor tyrosine kinase family, especially compounds which inhibit the c-Kit receptor, such as imatinib; i) compounds targeting, decreasing or inhibiting the activity of members of the c-Abl family, their gene-fusion products (e.g. BCR-Abl kinase) and mutants, such as compounds which target decrease or inhibit the activity of c-Abl family members and their gene fusion products, such as an N-phenyl-2-pyrimidine-amine derivative, such as imatinib or nilotinib (AMN107); PD180970; AG957; NSC 680410; PD173955 from ParkeDavis; or dasatinib (BMS-354825); j) compounds targeting, decreasing or inhibiting the activity of members of the protein kinase C (PKC) and Raf family of serine/threonine kinases, members of the MEK, SRC, JAK/pan-JAK, FAK, PDK1, PKB/Akt, Ras/MAPK, PI3K, SYK, TYK2, BTK and TEC family, and/or members of the cyclin-dependent kinase family (CDK) including staurosporine derivatives, such as midostaurin; examples of further compounds include UCN-01, safingol, BAY 43-9006, Bryostatin 1, Perifosine; Ilmofosine; RO 318220 and RO 320432; GO 6976; Isis 3521; LY333531/LY379196; isochinoline compounds; FTIs; PD184352 or QAN697 (a PI3K inhibitor) or AT7519 (CDK inhibitor); k) compounds targeting, decreasing or inhibiting the activity of protein-tyrosine kinase inhibitors, such as compounds which target, decrease or inhibit the activity of protein-tyrosine kinase inhibitors include imatinib mesylate (Gleevec™) or tyrphostin such as Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556, AG957 and adaphostin (4-{[(2,5-dihydroxyphenyl)methyl]amino}-benzoic acid adamantyl ester; NSC 680410, adaphostin); 1) compounds targeting, decreasing or inhibiting the activity of the epidermal growth factor family of receptor tyrosine kinases ($EGFR_1$ ErbB2, ErbB3, ErbB4 as homo- or heterodimers) and their mutants, such as compounds which target, decrease or inhibit the activity of the epidermal growth factor receptor family are especially compounds, proteins or antibodies which inhibit members of the EGF receptor tyrosine kinase family, such as EGF receptor, ErbB2, ErbB3 and ErbB4 or bind to EGF or EGF related ligands, CP 358774, ZD 1839, ZM 105180; trastuzumab (Herceptin™), cetuximab (Erbitux™), Iressa, Tarceva, OSI-774, CI-1033, EKB-569, GW-2016, E1.1, E2.4, E2.5, E6.2, E6.4, E2.11, E6.3 or E7.6.3, and 7H-pyrrolo-[2,3-d]pyrimidine derivatives; m) compounds targeting, decreasing or inhibiting the activity of the c-Met receptor, such as compounds which target, decrease or inhibit the activity of c-Met, especially compounds which inhibit the kinase activity of c-Met receptor, or antibodies that target the extracellular domain of c-Met or bind to HGF, n) compounds targeting, decreasing or inhibiting the kinase activity of one or more JAK family members (JAK1/JAK2/JAK3/TYK2 and/or pan-JAK), including but not limited to PRT-062070, SB-1578, baricitinib, pacritinib, momelotinib, VX-509, AZD-1480, TG-101348, tofacitinib, and ruxolitinib; o) compounds targeting, decreasing or inhibiting the kinase activity of PI3 kinase (PI3K) including but not limited to ATU-027, SF-1126, DS-7423, PBI-05204, GSK-2126458, ZSTK-474, buparlisib, pictrelisib, PF-4691502, BYL-719, dactolisib, XL-147, XL-765, and idelalisib; and q) compounds targeting, decreasing or inhibiting the signaling effects of hedgehog protein (Hh) or smoothened receptor (SMO) pathways, including but not limited to cyclopamine, vismodegib, itraconazole, erismodegib, and IPI-926 (saridegib).

The term "PI3K inhibitor" as used herein includes, but is not limited to compounds having inhibitory activity against one or more enzymes in the phosphatidylinositol-3-kinase family, including, but not limited to PI3Kα, PI3Kγ, PI3Kδ, PI3Kβ, PI3K-C2α, PI3K-C2β, PI3K-C2γ, Vps34, p110-α, p110-β, p110-γ, p110-δ, p85-α, p85-β, p55-γ, p150, p101, and p87. Examples of PI3K inhibitors useful in this invention include but are not limited to ATU-027, SF-1126, DS-7423, PBI-05204, GSK-2126458, ZSTK-474, buparlisib, pictrelisib, PF-4691502, BYL-719, dactolisib, XL-147, XL-765, and idelalisib.

The term "Bcl-2 inhibitor" as used herein includes, but is not limited to compounds having inhibitory activity

against B-cell lymphoma 2 protein (Bcl-2), including but not limited to ABT-199, ABT-731, ABT-737, apogossypol, Ascenta's pan-Bcl-2 inhibitors, curcumin (and analogs thereof), dual Bcl-2/Bcl-xL inhibitors (Infinity Pharmaceuticals/Novartis Pharmaceuticals), Genasense (G3139), HA14-1 (and analogs thereof; see WO2008118802), navitoclax (and analogs thereof, see US7390799), NH-1 (Shenayng Pharmaceutical University), obatoclax (and analogs thereof, see WO 2004/106328, hereby incorporated by reference), S-001 (Gloria Pharmaceuticals), TW series compounds (Univ. of Michigan), and venetoclax. In some embodiments the Bcl-2 inhibitor is a small molecule therapeutic. In some embodiments the Bcl-2 inhibitor is a peptidomimetic.

The term "BTK inhibitor" as used herein includes, but is not limited to compounds having inhibitory activity against Bruton's Tyrosine Kinase (BTK), including, but not limited to AVL-292 and ibrutinib.

The term "SYK inhibitor" as used herein includes, but is not limited to compounds having inhibitory activity against spleen tyrosine kinase (SYK), including but not limited to PRT-062070, R-343, R-333, Excellair, PRT-062607, and fostamatinib.

Further examples of BTK inhibitory compounds, and conditions treatable by such compounds in combination with compounds of this invention can be found in WO 2008/039218 and WO 2011/090760, the entirety of which are incorporated herein by reference.

Further examples of SYK inhibitory compounds, and conditions treatable by such compounds in combination with compounds of this invention can be found in WO 2003/063794, WO 2005/007623, and WO 2006/078846, the entirety of which are incorporated herein by reference.

Further examples of PI3K inhibitory compounds, and conditions treatable by such compounds in combination with compounds of this invention can be found in WO 2004/019973, WO 2004/089925, WO 2007/016176, US 8,138,347, WO 2002/088112, WO 2007/084786, WO 2007/129161, WO 2006/122806, WO 2005/113554, and WO 2007/044729 the entirety of which are incorporated herein by reference.

Further examples of JAK inhibitory compounds, and conditions treatable by such compounds in combination with compounds of this invention can be found in WO 2009/114512, WO 2008/109943, WO 2007/053452, WO 2000/142246, and WO 2007/070514, the entirety of which are incorporated herein by reference.

Further anti-angiogenic compounds include compounds having another mechanism for their activity, e.g. unrelated to protein or lipid kinase inhibition e.g. thalidomide (Thalomid™) and TNP-470.

Examples of proteasome inhibitors useful for use in combination with therapeutic-loaded exosomes of the invention include, but are not limited to bortezomib, disulfiram, epigallocatechin-3-gallate (EGCG), salinosporamide A, carfilzomib, ONX-0912, CEP-18770, and MLN9708.

Compounds which target, decrease or inhibit the activity of a protein or lipid phosphatase are e.g. inhibitors of phosphatase 1, phosphatase 2A, or CDC25, such as okadaic acid or a derivative thereof.

Compounds which induce cell differentiation processes include, but are not limited to, retinoic acid, α- γ- or δ-tocopherol or α- γ- or δ-tocotrienol.

The term "cyclooxygenase inhibitor" as used herein includes, but is not limited to, Cox-2 inhibitors, 5-alkyl substituted 2-arylaminophenylacetic acid and derivatives, such as celecoxib (Celebrex™), etoricoxib, valdecoxib or a 5-alkyl-2- arylaminophenylacetic acid, such as 5-methyl-2-(2'-chloro-6'-fluoroanilino)phenyl acetic acid, lumiracoxib.

The term "bisphosphonates" as used herein includes, but is not limited to, etridonic, clodronic, tiludronic, pamidronic, alendronic, ibandronic, risedronic and zoledronic acid. Etridonic acid is marketed under the trade name Didronel™. Clodronic acid is marketed under the trade name Bonefos™. Tiludronic acid is marketed under the trade name Skelid™. Pamidronic acid is marketed under the trade name Aredia™. Alendronic acid is marketed under the trade name Fosamax™. Ibandronic acid is marketed under the trade name Bondranat™. Risedronic acid is marketed under the trade name Actonel™. Zoledronic acid is marketed under the trade name Zometa™. The term "mTOR inhibitors" relates to compounds which inhibit the mammalian target of rapamycin (mTOR) and which possess antiproliferative activity such as sirolimus (Rapamune®), everolimus (Certican™), CCI-779 and ABT578.

The term "heparanase inhibitor" as used herein refers to compounds which target, decrease or inhibit heparin sulfate degradation. The term includes, but is not limited to, PI-88. The term "biological response modifier" as used herein refers to a lymphokine or interferons.

The term "inhibitor of Ras oncogenic isoforms", such as H-Ras, K-Ras, or N-Ras, as used herein refers to compounds which target, decrease or inhibit the oncogenic activity of Ras; for example, a "farnesyl transferase inhibitor" such as L-744832, DK8G557 or R115777 (Zarnestra™). The term "telomerase inhibitor" as used herein refers to compounds which target, decrease or inhibit the activity of telomerase. Compounds which target, decrease or inhibit the activity of telomerase are especially compounds which inhibit the telomerase receptor, such as telomestatin.

The term "methionine aminopeptidase inhibitor" as used herein refers to compounds which target, decrease or inhibit the activity of methionine aminopeptidase. Compounds which target, decrease or inhibit the activity of methionine aminopeptidase include, but are not limited to, bengamide or a derivative thereof.

The term "proteasome inhibitor" as used herein refers to compounds which target, decrease or inhibit the activity of the proteasome. Compounds which target, decrease or inhibit the activity of the proteasome include, but are not

limited to, Bortezomib (Velcade™) and MLN 341.

The term "matrix metalloproteinase inhibitor" or ("MMP" inhibitor) as used herein includes, but is not limited to, collagen peptidomimetic and nonpeptidomimetic inhibitors, tetracycline derivatives, e.g. hydroxamate peptidomimetic inhibitor batimastat and its orally bioavailable analogue marimastat (BB-2516), prinomastat (AG3340), metastat (NSC 683551) BMS-279251 , BAY 12-9566, TAA211 , MMI270B or AAJ996.

The term "compounds used in the treatment of hematologic malignancies" as used herein includes, but is not limited to, FMS-like tyrosine kinase inhibitors, which are compounds targeting, decreasing or inhibiting the activity of FMS-like tyrosine kinase receptors (Flt-3R); interferon, 1-β-D-arabinofuransylcytosine (ara-c) and bisulfan; and ALK inhibitors, which are compounds which target, decrease or inhibit anaplastic lymphoma kinase.

Compounds which target, decrease or inhibit the activity of FMS-like tyrosine kinase receptors (Flt-3R) are especially compounds, proteins or antibodies which inhibit members of the Flt-3R receptor kinase family, such as PKC412, midostaurin, a staurosporine derivative, SU11248 and MLN518.

The term "HSP90 inhibitors" as used herein includes, but is not limited to, compounds targeting, decreasing or inhibiting the intrinsic ATPase activity of HSP90; degrading, targeting, decreasing or inhibiting the HSP90 client proteins via the ubiquitin proteosome pathway. Compounds targeting, decreasing or inhibiting the intrinsic ATPase activity of HSP90 are especially compounds, proteins or antibodies which inhibit the ATPase activity of HSP90, such as 17-allylamino, 17-demethoxygeldanamycin (17AAG), a geldanamycin derivative; other geldanamycin related compounds; radicicol and HDAC inhibitors.

The term "antiproliferative antibodies" as used herein includes, but is not limited to, trastuzumab (Herceptin™), Trastuzumab-DM1, erbitux, bevacizumab (Avastin™), rituximab (Rituxan®), PRO64553 (anti-CD40) and 2C4 Antibody. By antibodies is meant intact monoclonal antibodies, polyclonal antibodies, multispecific antibodies formed from at least 2 intact antibodies, and antibodies fragments so long as they exhibit the desired biological activity.

For the treatment of acute myeloid leukemia (AML), therapeutic-loaded exosomes of the current invention can be used in combination with standard leukemia therapies, especially in combination with therapies used for the treatment of AML. In particular, therapeutic-loaded exosomes of the current invention can be administered in combination with, for example, farnesyl transferase inhibitors and/or other drugs useful for the treatment of AML, such as Daunorubicin, Adriamycin, Ara-C, VP-16, Teniposide, Mitoxantrone, Idarubicin, Carboplatinum and PKC412.

Other anti-leukemic compounds include, for example, Ara-C, a pyrimidine analog, which is the 2'-alpha-hydroxy ribose (arabinoside) derivative of deoxycytidine. Also included is the purine analog of hypoxanthine, 6-mercaptopurine (6-MP) and fludarabine phosphate. Compounds which target, decrease or inhibit activity of histone deacetylase (HDAC) inhibitors such as sodium butyrate and suberoylanilide hydroxamic acid (SAHA) inhibit the activity of the enzymes known as histone deacetylases. Specific HDAC inhibitors include MS275, SAHA, FK228 (formerly FR901228), Trichostatin A and compounds disclosed in US 6,552,065 including, but not limited to, N-hydroxy-3-[4-[[[2-(2-methyl-1H-indol-3-yl)-ethyl]-amino]methyl]phenyl]-2E-2-propenamide, or a pharmaceutically acceptable salt thereof and N-hydroxy-3-[4-[(2-hydroxyethyl){2-(1H-indol-3-yl)ethyl]-amino]methyl]phenyl]-2E-2-propenamide, or a pharmaceutically acceptable salt thereof, especially the lactate salt. Somatostatin receptor antagonists as used herein refer to compounds which target, treat or inhibit the somatostatin receptor such as octreotide, and SOM230. Tumor cell damaging approaches refer to approaches such as ionizing radiation. The term "ionizing radiation" referred to above and hereinafter means ionizing radiation that occurs as either electromagnetic rays (such as X-rays and gamma rays) or particles (such as alpha and beta particles). Ionizing radiation is provided in, but not limited to, radiation therapy and is known in the art. See Hellman, Principles of Radiation Therapy, Cancer, in Principles and Practice of Oncology, Devita et al., Eds., 4th Edition, Vol. 1, pp. 248-275 (1993).

Also included are EDG binders and ribonucleotide reductase inhibitors. The term "EDG binders" as used herein refers to a class of immunosuppressants that modulates lymphocyte recirculation, such as FTY720. The term "ribonucleotide reductase inhibitors" refers to pyrimidine or purine nucleoside analogs including, but not limited to, fludarabine and/or cytosine arabinoside (ara-C), 6-thioguanine, 5-fluorouracil, cladribine, 6-mercaptopurine (especially in combination with ara-C against ALL) and/or pentostatin. Ribonucleotide reductase inhibitors are especially hydroxyurea or 2-hydroxy-1H-isoindole-1,3-dione derivatives.

Also included are in particular those compounds, proteins or monoclonal antibodies of VEGF such as 1-(4-chloroanilino)-4-(4-pyridylmethyl)phthalazine or a pharmaceutically acceptable salt thereof, 1-(4-chloroanilino)-4-(4-pyridylmethyl)phthalazine succinate; Angiostatin™; Endostatin™; anthranilic acid amides; ZD4190; ZD6474; SU5416; SU6668; bevacizumab; or anti-VEGF antibodies or anti-VEGF receptor antibodies, such as rhuMAb and RHUFab, VEGF aptamer such as Macugon; FLT-4 inhibitors, FLT-3 inhibitors, VEGFR-2 IgGI antibody, Angiozyme (RPI 4610) and Bevacizumab (Avastin™).

Photodynamic therapy as used herein refers to therapy which uses certain chemicals known as photosensitizing compounds to treat or prevent cancers. Examples of photodynamic therapy include treatment with compounds, such as Visudyne™ and porfimer sodium.

Angiostatic steroids as used herein refers to compounds which block or inhibit angiogenesis, such as, e.g.,

anecortave, triamcinolone, hydrocortisone, 11-α-epihydrocotisol, cortexolone, 17α-hydroxyprogesterone, corticosterone, desoxycorticosterone, testosterone, estrone and dexamethasone.

Other chemotherapeutic compounds include, but are not limited to, plant alkaloids, hormonal compounds and antagonists; biological response modifiers, preferably lymphokines or interferons; antisense oligonucleotides or oligonucleotide derivatives; shRNA or siRNA; or miscellaneous compounds or compounds with other or unknown mechanism of action.

The therapeutic-loaded exosomes of the invention are also useful as co-therapeutic compounds for use in combination with other drug substances such as anti-inflammatory, bronchodilatory or antihistamine drug substances, particularly in the treatment of obstructive or inflammatory airways diseases such as those mentioned hereinbefore, for example as potentiators of therapeutic activity of such drugs or as a means of reducing required dosaging or potential side effects of such drugs. A therapeutic-loaded exosome of the invention may be mixed with the other drug substance in a fixed pharmaceutical composition or it may be administered separately, before, simultaneously with or after the other drug substance. Accordingly the invention includes a combination of a therapeutic-loaded exosome of the invention as hereinbefore described with an anti-inflammatory, bronchodilatory, antihistamine or anti-tussive drug substance, said therapeutic-loaded exosome of the invention and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate; non-steroidal glucocorticoid receptor agonists; LTB4 antagonists such LY293111, CGS025019C, CP-195543, SC-53228, BIIL 284, ONO 4057, SB 209247; LTD4 antagonists such as montelukast and zafirlukast; PDE4 inhibitors such cilomilast (Ariflo® GlaxoSmithKline), Roflumilast (Byk Gulden),V-11294A (Napp), BAY19-8004 (Bayer), SCH-351591 (Schering-Plough), Arofylline (Almirall Prodesfarma), PD189659 / PD168787 (ParkeDavis), AWD-12- 281 (Asta Medica), CDC-801 (Celgene), SelCID(TM) CC-10004 (Celgene), VM554/UM565 (Vernalis), T-440 (Tanabe), KW-4490 (Kyowa Hakko Kogyo); A2a agonists; A2b antagonists; and beta-2 adrenoceptor agonists such as albuterol (salbutamol), metaproterenol, terbutaline, salmeterol fenoterol, procaterol, and especially, formoterol and pharmaceutically acceptable salts thereof. Suitable bronchodilatory drugs include anticholinergic or antimuscarinic compounds, in particular ipratropium bromide, oxitropium bromide, tiotropium salts and CHF 4226 (Chiesi), and glycopyrrolate.

Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine.

Other useful combinations of therapeutic-loaded exosomes of the invention with anti-inflammatory drugs are those with antagonists of chemokine receptors, e.g. CCR-1 , CCR-2, CCR-3, CCR-4, CCR-5, CCR-6, CCR-7, CCR-8, CCR-9 and CCR10, CXCR1 , CXCR2, CXCR3, CXCR4, CXCR5, particularly CCR-5 antagonists such as Schering-Plough antagonists SC-351125, SCH- 55700 and SCH-D, and Takeda antagonists such as N-[[4-[[[6,7-dihydro-2-(4-methylphenyl)-5H-benzo-cyclohepten-8-yl]carbonyl]amino]phenyl]-methyl]tetrahydro-N,N-dimethyl-2H-pyran-4-aminium chloride (TAK-770).

In some embodiments, the additional therapeutic agent is selected from Abacavir, Abiraterone, Acetylcysteine, acyclovir, adefovir dipivoxil, Alatrofloxacin, Albendazole, albuterol, Alendronic acid, Altropane, Amifostine, Aminolevulinic acid, amiodarone (e.g. cosolvent-free), Amisulpride, amitriptyline, amprenavir, anastrozole, Apomorphine, apremilast, Arbutamine, Argatroban, Arsenic trioxide, aspirin, Atazanavir/cobicistat, Atorvastatin, Avibactam/ceftazidime, Azacitidine, azathioprine, Azithromycin, Belinostat, bendamustine, Bexarotene, Biapenem, Bicalutamide, Bortezomib, Bosentan, bosutinib, Bromfenac, Buprenorphine, Bupropion, Busulfan, C1 esterase inhibitor, Caffeine, calcium levofolinate, Cangrelor, capecitabine, capsaicin, Carfilzomib, Carvedilol, Cefepime, Ceftaroline fosamil, Ceftazidime, Ceftibuten, Ceftolozane/tazobactam, celecoxib, Celgosivir, chlorambucil, Cidofovir, Ciprofloxacin, Cladribine, Clazosentan, Clofarabine, Clopidogrel, cyclophosphamide, cytarabine, danazol, Dantrolene, dasatinib, Daunorubicin, Decitabine, Deferiprone, delavirdine, Deoxycholic acid, deoxythymidine, Dexamethasone, Dexmedetomidine, Dexrazoxane, Diclofenac, Didanosine, diethylcarbamazine, Docetaxel, Dolasetron, Doripenem, Doxapram, Doxercalciferol, Doxorubicin, doxycycline, Efavirenz, Eflapegrastim, elvitegravir, emtricitabine, Entacapone, Epacadostat, epinephrine, epitiostanol, Epoprostenol, ergotamine, Eribulin, Esomeprazole, estradiol, estrogen, etonogestrel, Ezetimibe, Ezetimibe/simvastatin, Fasudil, Fenoldopam, Fentanyl, Ferric carboxymaltose, Finasteride, Fingolimod, Florbenazine F18, Florbetaben F 18, florbetapir F 18, Fludarabine, Fluorine 18 AV 1451, fluorouracil, Fluoxymesterone, Flurpiridaz F-18, Flutafuranol F 18, Flutemetamol F 18, Fomepizole, Fosaprepitant, Fosphenytoin, Fospropofol, fulvestrant, Furosemide, Gadobenic acid, Gadobutrol, Gadoversetamide, Gadoxetate disodium, gemcitabine, Glimepiride, Granisetron, Guadecitabine, hydroxychloroquine, Ibandronic acid, ibuprofen, imatinib, Imiquimod, Iobenguane I-123, Ioflupane 123I, Ioxilan, Irinotecan, Isavuconazonium, isosorbidedinitrate, ivermectin, ixabepilone, labelalol, Lacosamide, lamivudine, Lamotrigine, Lansoprazole, Lapatinib, L-dopa, leflunomide, Letermovir, Letrozole, Levetiracetam, Levofloxacin, Levothyroxine, Lidocaine, lidocaine, Linezolid, Lobaplatin, Lomitapide, lopinavir, maraviroc, Meloxicam, melphalan, mercaptopurine, Meropenem, Mesna, methotrexate, Methylnaltrexone, Methylphenidate, metoprolol, midazolam, Minocycline IV, Mitoxantrone, Moxifloxacin, Mycophenolate mofetil, naloxone, naltrexone, naproxen, Nefazodone, nelarabine, nelfinavir, Nevirapine, nilotinib, Nilutamide, nitrosoureas, nortriptyline, Omacetaxine mepesuccinate, Omadacycline, Omeprazole, an opioid such as codeine, meperidine, fentanyl, morphine, oxycodone, hydrocodone, hydromorphone, or methadone, Oxaliplatin, oxprenolol, Oxybutynin, Oxymetholone, paclitaxel (Taxol®), Palonosetron, Pantoprazole, Paracetamol, Pemetrexed, pentazocine, Pentostatin, Phenylephrine, Pirmenol, platinum, Plazomicin, Plerixafor, ponatinib, pralatrexate, predisone, prednisolone, Propofol, propranolol, Quinapril, Radium-223 chloride, Raloxifene, raltegravir, Raltitrexed, Ramatroban, Regadenoson, Remifentanil, Remimazolam besylate, rilpivirine, rinotecan, Risperidone, Ritonavir, Rivastigmine, rofecoxib, Romidepsin, Ropeginterferon alfa-2b, Rotigotine, salbutamol, Salmeterol, Samarium 153 lexidronam, saquinavir, Selegiline, Sertraline, Sildenafil, Simvastatin, Sorivudine, Stavudine, sulfasalazine, Sulfur hexafluoride, Sumatriptan, Sunitinib, Tacrine, tamoxifen, Technetium Tc 99m trofolastat, Tedizolid, Temozolomide, tenofovir, Terbinafine, Testosterone propionate, thiotepa, Tianeptine, Tigecycline, Tizanidine, Topiramate, Topotecan, toremifene, Treprostinil, Tretinoin, Triciribine, verapamil, Verteporfin, Vinorelbine, Vismodegib, Voglibose, zalcitabine, zidovudine, Zileuton, or Zoledronic acid; or a pharmaceutically acceptable salt thereof.

The structure of the active compounds identified by code numbers, generic or trade names may be taken from the actual edition of the standard compendium "The Merck Index" or from databases, e.g. Patents International (e.g. IMS World Publications).

A therapeutic-loaded exosome of the current invention may also be used in combination with known therapeutic processes, for example, the administration of hormones or radiation. In certain embodiments, a provided therapeutic-loaded exosome is used as a radiosensitizer, especially for the treatment of tumors which exhibit poor sensitivity to radiotherapy.

The therapeutic-loaded exosomes and compositions, according to the method of the present invention, may be administered using any amount and any route of administration effective for treating or lessening the severity of a disease, disorder, or condition such as cancer, an autoimmune disorder, a proliferative disorder, an inflammatory disorder, a neurodegenerative or neurological disorder, schizophrenia, a bone-related disorder, liver disease, or a cardiac disorder. The exact amount required will vary from subject to subject, depending on the species, age, and general condition of the subject, the severity of the infection, the particular agent, its mode of administration, and the like. Therapeutic-loaded exosomes of the invention are preferably formulated in dosage unit form for ease of administration and uniformity of dosage. The expression "dosage unit form" as used herein refers to a physically discrete unit of agent appropriate for the patient to be treated. It will be understood, however, that the total daily usage of the therapeutic-loaded exosomes and compositions of the present invention will be decided by the attending physician within the scope of sound medical judgment. The specific effective dose level for any particular patient or organism will depend upon a variety of factors including the disorder being treated and the severity of the disorder; the activity of the specific therapeutic-loaded exosome employed; the specific composition employed; the age, body weight, general health, sex and diet of the patient; the time of administration, route of administration, and rate of excretion of the specific therapeutic-loaded exosome or compound employed; the duration of the treatment; drugs used in combination or coincidental with the specific therapeutic-loaded exosome or compound employed, and like factors well known in the medical arts. The term "patient," as used herein, means an animal, preferably a mammal, and most preferably a human.

### *4. **Methods of Making Exosomes and Loading with Therapeutic Agents***

#### *Production Methods of Making Exosomes, Including Milk-Derived Exosomes*

In one aspect, milk or colostrum-derived exosomes may be harvested from primary sources of a milk-producing animal. In some embodiments, the exosome is derived (e.g. isolated or manipulated) from milk or colostrum from a cow, human, buffalo, goat, sheep, camel, donkey, horse, reindeer, moose, or yak. In some embodiments, the milk is from a cow. In some embodiments, the milk or colostrum is in powder form. In some embodiments, the exosomes are produced and subsequently isolated from mammary epithelial cells lines adapted to recapitulate the exosome architecture of that naturally occurring in milk. In another aspect, suitable exosomes are isolated from milk produced by a transgenic cow or other milk-producing mammal whose characteristics are optimized for producing exosomes with desirable properties for drug delivery, e.g. oral drug delivery.

Exosomes, while produced naturally, still need to be harvested from body fluids or cell culture. This has important consequences from the perspective of scalable production. For example, usually the non-exosome content must be removed from the medium or mixture that contains the exosomes.

In one aspect, the exosomes are provided usuing a cell line one in a batch-like process, wherein the exosomes may be harvested periodically from the cell line media. The challenge with cell line based production methods is the potential for contamination from exosomes present in fetal bovine serum (media used to grow cells). In another aspect, this challenge can be overcome with the use of suitable serum free media conditions so that exosomes purely from the cell line of interest are harvested from the culture medium.

In one aspect, the exosomes are isolated or derived from a milk or colostrum solution. Separation of exosomes from the bulk solution must be performed with care. In some embodiments, a filter such as a 0.2 micron filter is used to remove larger debris from solution. In some embodiments, the method for separation of milk exosomes (for example, in the 80-120 nanometer range) includes separation based on specific exosome properties such as size, charge, density, morphology, protein content, lipid content, or epitopes recognized by antibodies on an immobilized surface (immuno-isolation).

In some embodiments, the separation method comprises a centrifugation step. In some embodiments, the separation method comprises PEG based volume excluding polymers.

In some embodiments, the separation method comprises ultra-centrifugation to separate the desired milk exosomes from bulk solution. In some embodiments, sequential steps involving initial spins at 20,000 $\times$ g for up to 30 minutes followed by multiple spins at ranges of about 100,000 $\times$ g to about 120,000 $\times$ g for about 1 to about 2 hours provides a pellet or isolate rich in milk-derived exosomes.

In some embodiments, ultracentrifugation provides milk-derived exosomes that can be resuspended, for example, in phosphate buffered saline or a solution of choice. In some embodiments, the exosomes are further assessed for desired properties by assessing their attributes when exposed to a sucrose density gradient and picking the fraction in 1.13-1.19 g/mL range.

In other embodiments, isolation of exosomes of the present invention includes using combinations of filters that exclude different sizes of particles, for example 0.45 $\mu$M or 0.22 $\mu$M filters can be used to eliminate vesicles or particles bigger than those of interest. Exosomes or microvesicles may be purified by several means, including antibodies, lectins, or other molecules that specifically bind microvesicles of interest, eventually in combination with beads (e.g. agarose/sepharose beads, magnetic beads, or other beads that facilitate purification) to enrich for the desired microvesicles. A marker derived from the microvesicle type of interest may also be used for purifying microvesicles. For example, microvesicles expressing a given biomarker such as a surface-bound protein may be purified from cell-free fluids to distinguish the desired microvesicle from other types. Other techniques to purify microvesicles include density gradient centrifugation (e.g. sucrose or optiprep gradients), and electric charge separation. All these enrichment and purification techniques may be combined with other methods or used by themselves. A further way to purify microvesicles is by selective precipitation using commercially available reagents such as ExoQuick™ (System Biosciences, Inc.) or Total Exosome Isolation kit (Invitrogen™ Life Technologies Corporation).

Suitable exosomes may also be derived by artificial production means, such as from exosome-secreting cells and/or engineered as is known in the art.

In some embodiments, exosomes can be further characterized by one or more of nanoparticle tracking analysis to assess particle size, transmission electron microscopy to assess size and architecture, immunogold labeling of exosomes or their contents prior to electron microscopy to track species of interest associated with exosomes, immunoblotting, or protein content assessment using the Bradford Assay.

Various methods are known in the art to encapsulate a therapeutic agent in a microvesicle that are compatible with the present invention. Accordingly, the present invention provides a method of encapsulating a disclosed therapeutic agent in a microvesicle such as a milk-derived exosome. In some embodiments, the method comprises the step of exposing the microvesicle to electroporation, sonication, saponification, extrusion, freeze/thaw cycles, or partitioning of the therapeutic agent and the microvesicle in a mixture of two or more solvents, to effect encapsulation of the therapeutic agent in the microvesicle.

In some embodiments, isolation of the microvesicle is achieved by centrifuging raw (i.e., unpasteurized and/or unhomogenized milk or colostrum) at high speeds to isolate the microvesicle. In some embodiments, a milk-derived microvesicle is isolated in a manner that provides amounts greater than about 50 mg (e.g., greater than about 300 mg) of microvesicles per 100 mL of milk. In some embodiments, the present invention provides a method of isolating a milk-derived microvesicle comprising the steps of: providing a quantity of milk (e.g., raw milk or colostrum); and performing a centrifugation, e.g. sequential centrifugations, on the milk to yield greater than about 50 mg of milk-dervied exosomes per 100 mL of milk. In some embodiments, the sequential centrifugations yield greater than 300 mg of milk-derived exosomes per 100 mL of milk. In some embodiments, the series of sequential centrifugations comprises a first centrifugation at 20,000 $\times$ g at 4 °C for 30 min, a second centrifugation at 100,000 $\times$ g at 4 °C for 60 min, and a third centrifugation at 120,000 $\times$ g at 4 °C for 90 min. In some embodiments, the isolated exosomes can then be stored at a concentration of about 5 mg/mL to about 10 mg/mL to prevent coagulation and allow the isolated exosomes to effectively be used for the encapsulation of one or more therapeutic agents. In some embodiments, the isolated exosomes are passed through a 0.22 $\mu$m filter to remove any coagulated particles as well as microorganisms, such as bacteria.

In some embodiments, a microvesicle composition described herein further includes one or more microRNAs (miRNAs) loaded into the microvesicle, either by virtue of being present in the microvesicles upon their isolation or by virtue of loading a miRNA for use as a therapeutic agent into the microvesicles subsequent to their initial isolation. In some embodiments, the miRNA loaded into the microvesicle is not naturally occurring in the source of the microvesicles. For example, mammalian milk exosomes sometimes include loaded miRNAs in their natural state, and such miRNAs

remain loaded in the exosomes upon their isolation. Such naturally-occurring miRNAs are distinguished from any miRNA therapeutic agent (or other iRNA, oligonucleotide, or other biologic) that is artificially loaded into the microvesicles.

Loading into the microvesicles, e.g. encapsulation, can be verified by disrupting the membrane of the therapeutic-loaded milk-derived microvesicles, e.g., with a detergent to release its contents. The contents level can be evaluated, for example, via protein/RNA/DNA quantification assays.

In some embodiments, the presently disclosed milk-derived exosomes are able to deliver their cargo while withstanding stressed conditions or conditions under which the therapeutic agent would become deactivated, metabolized, or decomposed, e.g. saliva, digestive enzymes, acidic conditions in the stomach, peristaltic motions, and/or exposure to the various proteases, lipases, amylases, and nucleases that break down ingested components in the gastrointestinal tract.

## EXEMPLIFICATION

### Example 1: Isolation of Exosomes from Milk

#### PURPOSE

The purpose of this protocol is to establish a procedure for the isolation of exosomes from milk through use of several centrifugation and ultracentrifugation steps.

#### DEFINITIONS

Whey - fluidic part of milk remaining after separation of visible fat
RCF - Relative Centrifugal Force
RPM - Rotations Per Minute
Kf - K factor

#### REFERENCES

https://www.researchgate.net/publication/284234296_Bovine_milk-derived_exosomes_for_drug_delivery

#### Materials

Milk
Phosphate-Buffered Saline without calcium & magnesium IX (Corning Cellgro #21-040-CV)
Distilled water

#### Supplies and equipment

1L bottle with screw cap
500mL bottle with screw cap
29×104mm polycarbonate centrifuge tubes (Beckman Coulter #375002)
Eppendorf Easypet 3 serological pipet
25mL serological pipet tips (VWR #414004-268)
1L beaker
Whatman paper, size 1 (Whatman #1001-125)
Aerobie Aeropress with funnel (Aerobie)
25×89mm ultra-clear ultracentrifuge tubes (Beckman Coulter #344058)
SW-32 Ti rotor (Beckman Coulter)
Optima XE90 ultracentrifuge (Beckman Coulter)
Eppendorf Research 1000uL pipet
1000uL pipet tips (TipOne #1111-2821)

Ocelot orbital rocker (VWR)
4 °C refrigerator

**PROCEDURE**

Remove cellular debris

Using Easypet 3 serological pipet (VWR) fitted with 25mL pipet tip (VWR #414004-268), place 40mL milk sample into fill 25x89mm ultra-clear ultracentrifuge tubes (Beckman Coulter #344058). N = 6 tubes.

*Note: Tubes must be filled to within 3mm of top of tube and all tubes must be balanced to within 0.5g.*

Place tubes in Optima XE90 ultracentrifuge (Beckman Coulter) fitted with SW-32 Ti rotor (Beckman Coulter) according to manufacturer instructions.

Centrifuge sample at the following settings:

| RCF | RPM | Kf | Run Time | Temperature |
|---|---|---|---|---|
| 13000 | 8700 | 2762.5 | 30 mins | 4 °C |

Collect whey fluid

Remove samples from centrifuge and gently pour all samples into a 1L beaker, taking care not to disturb the pellet.

*Note: It may be necessary to poke through the fat layer settled at the top of the tube in order to pour the fluid out of the tube.*

Cut a piece of Whatman paper into a 64mm diameter disk.

Place disk into filter cap of Aerobie Aeropress and secure filter cap in place.

Place Aeropress funnel in 1L beaker and place Aeropress body in funnel.

Pour collected fluid from [00920] into Aeropress body. Add Aeropress plunger and gently press down on plunger until all fluid has been filtered and collected in 1L beaker.

Remove large particles

Using Easypet 3 serological pipet (VWR) fitted with 25mL pipet tip (VWR #414004-268), fill 25×89mm ultra-clear ultracentrifuge tubes (Beckman Coulter #344058) with 30mL strained whey fluid. Add distilled water to each tube as needed (approximately 10mL) in order to satisfy manufacturer instructions for properly filled/balanced tubes. N = 6.

*Note: Tubes must be filled to within 3mm of top of tube and all tubes must be balanced to within 0.5g.*

Place tubes in Optima XE90 ultracentrifuge (Beckman Coulter) fitted with SW-32 Ti rotor (Beckman Coulter) according to manufacturer instructions.

Centrifuge samples at the following settings:

| RCF | RPM | Kf | Run Time | Temperature |
|---|---|---|---|---|
| 100,000 | 24,200 | 357.1 | 69 mins | 4C |

Collect exosome fraction

Remove samples from ultracentrifuge. Using Easypet 3 serological pipet (VWR) fitted with 25mL pipet tip (VWR #414004-268), carefully remove 30mL supernatant from top of samples and place in 25×89mm ultra-clear ultracentrifuge tubes (Beckman Coulter #344058). Add distilled water to each tube as needed in order to satisfy manufacturer instructions for properly filled/balanced tubes. N = 6.

Place tubes in Optima XE90 ultracentrifuge (Beckman Coulter) fitted with SW-32 Ti rotor (Beckman Coulter) according to manufacturer instructions.

Centrifuge samples at the following settings:

| RCF | RPM | Kf | Run Time | Temperature |
|---|---|---|---|---|
| 135,000 | 28,100 | 264.5 | 103 mins | 4C |

Wash exosomes

Remove samples from ultracentrifuge.

Using Easypet 3 serological pipet (VWR) fitted with 25mL pipet tip (VWR #414004-268), carefully remove 35mL supernatant from top of samples taking care not to disturb the pellet. The supernatant may be placed in a 500mL screw-top bottle and placed in a 4C fridge for up to one week, if required for comparative analysis.

Using Eppendorf Research 1000ul pipet fitted with 1000ul pipet tips (TipOne #1111-2821), vigorously pipet remaining fluid in centrifuge up and down 20 times in order to resuspend the pellet.

*Note: It may not be possible to completely resuspend the pellet during this step, depending on the integrity of the pellet.*

Using Easypet 3 serological pipet (VWR) fitted with 25mL pipet tip (VWR #414004-268), add approximately 35mL PBS (Corning Cellgro #21-040-CV) on top of resuspended exosome pellet (or however much is required to fill the tube). Ensure that enough PBS is added to satisfy manufacturer instructions for properly filled/balanced tubes. N = 6.

Place tubes in Optima XE90 ultracentrifuge (Beckman Coulter) fitted with SW-32 Ti rotor (Beckman Coulter) according to manufacturer instructions.

Centrifuge samples at the following settings:

| RCF | RPM | Kf | Run Time | Temperature |
|---|---|---|---|---|
| 135,000 | 28,100 | 264.5 | 103 mins | 4C |

Remove samples from ultracentrifuge and repeat steps [00937] through [00942] for a total of three washings.

Resuspend exosomes

Upon completion of final washing, remove samples from ultracentrifuge.

Carefully pour off the supernatant from top of samples, taking care not to disturb the pellet. The supernatant may be placed in a 1L screw-top bottle and placed in a 4C fridge for up to one week, if required for comparative analysis.

Using Eppendorf Research 1000ul pipet fitted with 1000ul pipet tips (TipOne #1111-2821), add 1mL of PBS to sample to cover pellet and resuspend pellet by vigorously pipetting PBS up and down 20 times.

Pool final resuspended exosome pellets in 15mL conical centrifuge tube (VWR #TC1500).

*Note: The final pooled volume of resuspended exosomes should be between 6 and 8mL.*

Place centrifuge tube on Ocelot orbital rocker (VWR) in 4C fridge overnight or until pellets completely dissolved.

Sterilization

Affix 0.8/0.2um Supor Membrane filter (Pall Life Sciences #4658) to 10mL luer lock syringe (McMaster Carr #7510A653) and remove piston from syringe.

Pour resuspended exosomes into open end of syringe.

Re-affix piston to syringe and filter pooled exosome suspension into 15mL conical centrifuge tube (VWR #TC1500).

Place tube in 4C fridge for storage for up to one week for short-term storage or a - 20C fridge for long-term storage.

Results:

**Table 6: Summary of Exosome Isolation Results**

| Isolation Procedure | Yield (μg exo/L input) | Size (nm) DLS | Size (nm) NTA | # exosomes/mg protein - NTA | Total RNA (μg/mg protein) | CD81 (Y/N) |
|---|---|---|---|---|---|---|
| **Colostrum Powder** | **410** | **126** | **100** | **$7.9 \times 10^{11}$** | **0.35** | **Y** |
| **Raw Milk** | **60** | **200** | **135** | **$8.5 \times 10^{11}$** | **0.21** | **Y** |

## Example 2: Micro-BCA Assay Protocol

### Purpose:

To analyze protein-containing solutions and quantify the total protein content contained within. This assay is accurate at concentrations of protein from 3.125 to 200 μg/mL.

### Materials:

1X PBS buffer (Corning, Catalog #: 21-040-CV, Lot #: 32516005)

10 - 20 μL pipette tips, RNAse-free (USA Scientific, Catalog #: 1110-3800)

20 - 200 μL pipette tips, RNAse-free (USA Scientific, Catalog #: 1111-0706)

100 - 1000 μL pipette tips, RNAse-free (USA Scientific, Catalog #: 1111-2821)

Micro-BCA analysis kit (ThermoFisher, Catalog #: 23235, Lot #: PJ203823B) Reagent BCA-A

Reagent BCA-B

Reagent BCA-C

Bovine serum albumin standard solution (2 mg/mL)

Polystyrene reagent reservoir (2) (VWR, Catalog #: 89094-666)

Clear-bottomed 96-well plate (ThermoFisher, Catalog #: 9205)

96- well plate sealing tape (ThermoFisher, Catalog #: 15036)

RNAse-free 1.5 mL centrifuge tubes (Ambion, Catalog #: AM12400, lot #: 02470003)

50 mL centrifuge tube (VWR, Catalog #: 89039-656)

### Equipment:

Plate reader w/ 540-590 nm filter (Tecan Infinite M200)
Incubating Microplate Shaker (VWR)
Pipettes (Various)

### Working Reagent:

Calculate the amount of working reagent needed by multiplying the total number of wells to be used (standard and sample wells) by 110. This is the amount of working reagent needed, in μL.

Using the 100-1000 μL pipette, mix reagent BCA-A, BCA-B and BCA-C in a ratio of 25:24:1 to obtain the desired amount or working reagent. For example, if 5 mL of working reagent is required, add 2.5 mL BCA-A, 2.4 mL BCA-B, and 0.1 mL BCA-C to the 50mL centrifuge tube.

Vortex the mixture for 3-5 seconds in the centrifuge tube and use within 5 hours of mixing.

### Method:

Using a marker, mark a 96-well plate for your desired experiment, including wells for a standard curve and for sample analysis. See sample layout below for an example well-plate.

*Standard Curve Preparation*

Perform a 2x serial dilution down the plate to produce a standard curve:
Using a 20-200 μL pipette, add 20 μL of 2 μg/mL bovine serum albumin (BSA) standard solution to wells A1, A2, and A3. Using the 20-200 μL multichannel pipette, add 180 μL of 1x PBS to these three wells and pipette up and down five times to mix. Using the 20-200 μL multichannel pipette, add 100 μL of 1x PBS to the remainder of the wells running down the plate (B1 - B3 to H1 - H3). Using the 20-200 μL multichannel pipette, transfer 100 μL of solution from A1-A3 to B1-B3 and pipette up and down five times to mix. Continue down well-plate, leaving wells H1-H3 as blanks (PBS only).
Note: Don't forget to remove excess fluid from final standard wells (G1-G3) to ensure only 100μL remains.

*Sample Preparation*

Dilute samples to analyze as needed:
Using the correct pipette for the volume required, add the necessary sample amount to a 1.5 mL centrifuge tube.
Using the 100-1000 μL pipette, add 1x PBS in the correct dilution amount to the centrifuge tube.
Cap the centrifuge tube and vortex for 3-5 seconds.

| | Sample Volume | 1x PBS Volume |
|---|---|---|
| **1:1 Dilution** | 500 μL | 500 μL |
| **1:5 Dilution** | 200 μL | 800 μL |
| **1:10 Dilution** | 100 μL | 900 μL |
| **1:50 Dilution** | 20 μL | 980 μL |
| **1:100 Dilution** | 10 μL | 990 μL |

*Running the Assay*

Using the 20-200 μL pipette with 20-200 μL tips, add 100 μL of each sample in triplicate to the marked-out sample wells.
Using the 20-200 μL multichannel pipette, add 100 μL of working reagent from a reagent reservoir to all standard curve and sample wells.
Cover the plate with 96-well plate sealing tape.
Incubate the plate in the microplate shaker at 37°C, 100 rpm for two hours.
Remove the plate from the microplate shaker and read the plate at 562 nm using the plate reader:
Remove sealing tape.
Use the i-control software located on the lab computer to control plate reader and measure absorbance.
Note: If using a substitute plate reader, any available filters from 540 to 590 nm are acceptable, but may result in a decreased absorbance signal

*Data Analysis*

Import the data generated by the plate reader into GraphPad Prism.
Using the polynomial regression function and the standard curve data, create a quadratic standard curve for the experiment.
Using the data interpolation function, fit sample data to the standard curve.
The results of a 14-day stability study are shown in **FIG. 4**. Protein concentration was measured each day for a sample stored at 4 °C (upper graph). Protein concentrations were also measured at day 14 for samples stored at room temperature, 4 °C, -20 °C, and -80 °C, respectively (lower graph). The results show that milk exosomes from both raw milk ("PT Raw" data) and colostrum ("PT Colostrum" data) are stable for at least 14 days at all temperatures tested.

**Example 3: Particle Concentration and Size Measurements**

**Goal**: Check agreement between multiple methods of measuring particle concentration. NTA (Nanoparticle Tracking Analysis) - Image-based detection based on Brownian motion. IZON/TRPS (Tunable Resistive Pulse Sensing) - Resistance measurement based on water displacement as particle passes through small pores. Dynamic Light Scattering (DLS) is also used for measuring particle size.

**Nanosight Tracking Analysis:**

**Samples:**

UL_13APR17_01
UL_20APR17_01
EXO1-25APR17_01
EXO1-26APR17_01
EXO1-09MAY17_01
EXO1-15MAY17_01

**Preparation of samples for shipping:**

Using a 100-1000 uL pipette, add 450 uL of DI water to four different 1.5 mL Eppendorf tubes with a cap.
Using a 5-50 uL pipette take 50 ul of UL_13APR17_01 and place into a 1.5 mL Eppendorf tube that was filled with 450 uL of DI water. Invert three times and wrap parafilm around the top of the 1.5 mL Eppendorf tube.
Using a 5-50 uL pipette take 50 ul of UL_20APR17_01 and place into a 1.5 mL Eppendorf tube that was filled with 450 uL of DI water. Invert three times and wrap parafilm around the top of the 1.5 mL Eppendorf tube.
Using a 5-50 uL pipette take 50 ul of EXO1_25APR17_01 and place into a 1.5 mL Eppendorf tube that was filled with 450 uL of DI water. Invert three times and wrap parafilm around the top of the 1.5 mL Eppendorf tube.
Using a 5-50 uL pipette take 50 ul of EXO1_26APR17_01 and place into a 1.5 mL Eppendorf tube that was filled with 450 uL of DI water. Invert three times and wrap parafilm around the top of the 1.5 mL Eppendorf tube.
Using a 100-1000 uL pipette, add 450 uL of 1X PBS to six different 1.5 mL Eppendorf tubes with a cap
Using a 5-50 uL pipette take 50 uL of UL_13APR17_01 and place into a 1.5 mL Eppendorf tube that was filled with 450 uL 1X PBS. Invert three times and wrap parafilm around the top of the 1.5 mL Eppendorf tube.
Using a 5-50 uL pipette take 50 uL of UL_20APR17_01 and place into a 1.5 mL Eppendorf tube that was filled with 450 uL 1X PBS. Invert three times and wrap parafilm around the top of the 1.5 mL Eppendorf tube.
Using a 5-50 uL pipette take 50 uL of EXO1_25APR17_01 and place into a 1.5 mL Eppendorf tube that was filled with 450 uL 1X PBS. Invert three times and wrap parafilm around the top of the 1.5 mL Eppendorf tube.
Using a 5-50 uL pipette take 50 uL of EXO1_26APR17_01 and place into a 1.5 mL Eppendorf tube that was filled with 450 uL 1X PBS. Invert three times and wrap parafilm around the top of the 1.5 mL Eppendorf tube.
Using a 5-50 uL pipette take 50 uL of EXO1_09MAY17_01 and place into a 1.5 mL Eppendorf tube that was filled with 450 uL 1X PBS. Invert three times and wrap parafilm around the top of the 1.5 mL Eppendorf tube.
Using a 5-50 uL pipette take 50 uL of EXO1_15MAY17_01 and place into a 1.5 mL Eppendorf tube that was filled with 450 uL 1X PBS. Invert three times and wrap parafilm around the top of the 1.5 mL Eppendorf tube.
Each sample is packed in dry ice for shipping.
Nanoparticle tracking analysis (NTA) is a technique for visualizing and analysis of dilute aqueous suspensions containing particles. Particles are visualized as they scatter light of the laser beam passing through the sample cell. Particles with size under 1000 nm freely move in solution under Brownian motion. Visualized particles tracks are recorded by camera. Track length traveled by particles per unit of time is analyzed by software and allows determination of a size, size distribution profile and concentration of particles with a diameter of approximately 10-1000 nm. Particle size is calculated to a sphere equivalent hydrodynamic radius through the Stokes-Einstein equation.
Nanosight LM10 instrument is equipped with CCD camera and 638nm laser.

**Materials:**

a. Syringe filter 0.22 μm, Millex-GV, lot R6MA09809, Millipore
b. Powder-free exam gloves, Purple Nitrile, lot SY355ZZZ04AX, Halyard
c. PBS, 10x, USP sterile, lot 0866C325, Amresco
d. Micro tube, 1.5 mL, lot 60U4411, Sarstedt
e. Vortex Maxi-Mix 1, type 16700, Thermolyne
f. Cell Culture Grade Water, lot 30816005, Corning

**Instrument Qualification:**

Instrument qualification was performed by analyzing 100 nm polystyrene bead standard in 1x PBS solution. Mode size meet acceptance criteria and was measured to be 102.0 nm.

**Samples:**

Samples (total of 10) were submitted for analysis.

**Sample preparation:**

Standard laboratory protection equipment (gloves, coat, goggles, and mask) was used on all steps of sample preparation and analysis to prevent sample contamination with dust particles. PBS solution was filtered on the day of analysis through 0.22 μm syringe filter and its purity confirmed by Nanosight analysis prior to the study. PBS purity evaluation is reported as Sample Blank.

Samples were placed into a -80 °C freezer. At the time of analysis, each sample was unfrozen by incubation at room temperature and prepared immediately prior to analysis. Sample solution was homogenized by shaking on vortex for 30 seconds. Samples of the batch appeared to have different concentration and were diluted accordingly to fit in 108 particles/mL range.

Dilution 1000x was performed by adding 10 μL of the original sample after shaking it on vortex for 30 sec to 990 μL of PBS 1x and shaking on vortex for 30 sec. After that, 100 μL of diluted sample was added to 900 μL of PBS 1x to achieve final dilution of 100x and mixed on vortex shaker for 30 sec.

Dilution 250x was performed by adding 10 μL of the original sample after shaking it on vortex for 30 sec to 990 μL of PBS 1x and shaking on vortex for 30 sec. After that, 400 μL of diluted sample was added to 600 μL of PBS 1x to achieve final dilution of 100x and mixed on vortex shaker for 30 sec.

Dilution 100x was performed by adding 10 μL of the original sample after shaking it on vortex for 30 sec to 990 μL of PBS 1x and shaking on vortex for 30 sec.

Ten replicates of analysis by 15 seconds was performed for each sample. After analysis, each sample was returned to -80° C freezer.

**Analysis sequence and notes:**

a. Sample Blank - 1x PBS used for dilutions was analyzed prior to the study. No particles observed in 60 seconds, solution was found appropriately clean to be used for samples preparation.
b. Exo1-Lot25Apr2017-01 was analyzed at 1000x dilution.
c. Exo1_Lot26Apr17_01 was analyzed at 1000x dilution.
d. Exo1-Lot26Apr2017-01_PBS was analyzed at 1000x dilution first. Sample concentration was found to be lower desired 108 concentration range and additional dilution of 250x was prepared and analyzed.
e. Exo1-Lot15May17_01 was prepared and analyzed at 1000x dilution. Mode peak measured at 115nm, presence of 200nm particles observed in all replicates. Particles with size 300nm were found in 3 replicates out of 10.
f. Exo1-Lot9May17-01 was analyzed at 1000x dilution.
g. Exo1_Lot25Apr17_01PBS was prepared and analyzed at 1000x dilution first. As concentration was found to be under 108 , additional dilution of 250x was prepared and analyzed.

Results are shown in Table 7 below. A size distribution graph for sample EXO1-LOT26APR2017 is shown in **FIG. 1**.

**Table 7: Data Summary**

| Dilution | Sample ID | Mean size (nm) | Mode Size (nm) | SD (nm) | Concentration (particles/mL) | +/- | D10 (nm) | D50 (nm) | D90 (nm) |
|---|---|---|---|---|---|---|---|---|---|
| | | | | | | | | | |
| **1000x** | **Exol-Lot25Apr2017-01** | 119.8 +/- 1.7 | 123.0 +/- 3.9 | 31,2 | 4.95e+0.08 | 1.48e+007 | 76.0 | 118.5 | 158.4 |
| **1000x** | **Exol Lot26Apr17 01** | 156.3 +/- 8.2 | 114.8 +/- 13.2 | 58.7 | 2.62e+008 | 1.49a+007 | 67.0 | 162.4 | 223.7 |
| **1000x** | **Exol-Lot26Apr2017-01 PBS** | 153.7 +/- 6.6 | 162.4 +/- 13.1 | 58.1 | 8.30e+007 | 8.87e+006 | 72.2 | 152.4 | 234.1 |
| **250x** | **Exol-Lot26Apr2017-01 PBS** | 184.7 +/- 7.8 | 172.1 +/- 18.3 | 70.2 | 3.48e+008 | 2.90e+007 | 94.6 | 178.3 | 268.3 |
| **1000x** | **Exol-Lot15May17 01** | 162.6 +/- 14.6 | 172.8 +/- 14.4 | 43.7 | 1.35e+008 | 1.80e+007 | 100.2 | 164.4 | 210.8 |
| **1000x** | **Exol.Lot9May17-01** | 120.1 +/- 2.8 | 126.6 +/- 4.1 | 37.8 | 1.84e+008 | 1.05e+007 | 62.5 | 123.6 | 168.8 |
| **1000x** | **Exol_Lot25Apr17 01PBS** | 99.1 +/- 5.3 | 109.6 +/- 15.2 | 38.4 | 9.53e+007 | 6.88e+006 | 45.7 | 98.8 | 147.5 |
| **250x** | **Exol_Lot25Apr17_01PBS** | 139.3 +/- 5.6 | 115.3 +/- 5.9 | 64.9 | 4.37e+008 | 1.03e+007 | 88.4 | 121.4 | 200.2 |

*Note:* Concentrations reported here and in the instrument reports do not account for dilution factor. Reported concentrations must be multiplied by dilution factor to achieve particles concentration in original sample.

**EXOIZON:**

**Sample Used:**

| Sample ID | Volume (ul) | Dilution |
|---|---|---|
| EXO1-LOT26APR2017 | 500 | 1:10 |

**Preparation of sample:**

1. An exosome sample (EXO1-LOT26APR2017) previously diluted 1:10 in 1X PBS was used in the IZON run.

**Overview of qNano (Izon):**

The qNano instrument uses TRPS (Tunable Resistive Pulse Sensing), a technique for analysis of dilute aqueous particles solutions. Particles concentration and size are measured during particles migration induced by pressure through nano-membrane with single pore of a known size. A voltage is applied across a pore that is filled with electrolyte, resulting in an ionic current. As particles cross the pore they briefly increase electrical resistance, creating a resistive pulse, which is precisely proportional to particle volume. The actual measurement of each particle crossing the pore is achieved using calibration particles that have been accurately calibrated for size and concentration. Particle concentration, being the number of particles/ml for a specified size range. Particle size and accurate number based size distribution, derived on a real particle by particle basis. Particle charge and number based charge distribution, also derived on a real particle by particle basis. The rate of flow of particles is proportional to particle concentration, so particle number can accurately obtained at the same time as individual particle sizes.

**Materials:**

a. Syringe filter 0.22 μm, Millex-GV, lot R6MA09809, Millipore
b. Powder-free exam gloves, Purple Nitrile, lot SY355ZZZ_04AX, Halyard
c. DPBS, 10x, Lot DPBS, 10x, USP sterile, Thermo Fischer
d. Micro tube, 1.5 mL, lot 60U4411, Sarstedt
e. Vortex Maxi-Mix 1, type 16700, Thermolyne
f. Cell Culture Grade Water, lot 30816005, Corning
g. 150nm qNano pore, lot NP150, IZON

**Sample preparation:**

Standard laboratory protection equipment (gloves, coat, goggles, and mask) was used on all steps of sample preparation and analysis to prevent samples contamination with dust particles. DPBS solution was filtered on the day of analysis through 0.22 μm syringe filter and its purity confirmed by Nanosight analysis prior to the study. Upon delivery sample were placed to -80 °C freezer. At the time of analysis, sample was unfrozen by incubation at room temperature and 10 μL of the solution was used for Nanosight Analysis. 15 μL of the sample was mixed 1485 μL of the filtered DPBS solution, vortexed for 3 minutes and analyzed by qNano.

**Table 8: Data Summary**

| Sample | Method | Dilution | Measured Particle Concentration (particles/mL) | Calculated Particle Concentration (particles/mL) |
|---|---|---|---|---|
| **EXO1-LOT26APR2017** | **IZON** | **1:1000** | **8.0E08** | **8.0E11** |
| **EXO1-LOT26APR2017** | **NTA** | **1:10,000** | **8.3E07** | **8.3E11** |
| **EXO1-LOT26APR2017** | **NTA** | **1:2500** | **3.48E08** | **8.7E11** |

| Sample ID | | Dilution | Mean Size (nm) | Mode Size (nm) | D10 (nm) | D50 (nm) | D90 (nm) | Measured Concentration (particles/mL) | Stock Concentration (particles/mL) |
|---|---|---|---|---|---|---|---|---|---|
| | EXO1-LOT26APR2017-01-PBS | 1000 | 125.1 | 107.3 | 104.1 | 119.9 | 150.8 | 8.0E+008 | 8.0E+011 |

In the table above, D10 refers to the size (104.1 nm, as calculated) at which the cumulative mass of all particles less than that size represents 10% of the population. D50 and D90 refer to corresponding calculated sizes for which the cumulative mass of all particles less than the sizes shown above represents less than 50% or 90% of the population, respectively.

The results of a 14-day stability study are shown in **FIG. 5**. Particle size was measured each day for a sample stored at 4 °C (upper graph). Particle size was also measured at day 14 for samples stored at room temperature, 4 °C, -20 °C, and -80 °C, respectively (lower graph). The results show that milk exosomes from both raw milk ("PT Raw" data) and colostrum ("PT Colostrum" data) are stable for at least 14 days at all temperatures tested.

**Conclusions:**

IZON and NTA produce a similar particle concentration measurement. NTA could be used as a reliable tool for reporting particle concentration pending a dilution linearity experiment. Exosomes were stable for at least 14 days under a variety of temperature conditions.

**DLS Protocol:**

**Purpose:**

To analyze and quantify the sizes of particulates found in experimental samples. The piece of equipment used to produce this data is accurate up to a size of 1 μm.

**Materials:**

384-well, glass-bottom plate w/ cover (Greiner BioOne, Catalog #: 82051-546)
1x PBS buffer (Corning, Catalog #: 21-040-CV, Lot #: 32516005)
20 - 200 μL pipette tips (USA Scientific, Catalog #: 1111-0706)
100 - 1000 μL pipette tips (USA Scientific, Catalog #: 1111-2821)
1.5 mL plastic centrifuge tubes w/ caps (Ambion, Catalog #: AM12400, lot #: 02470003)
Centrifuge tube rack
DLS capable plate reader (Wyatt DynaPro Plate Reader) and DLS software.

**Method:**

Sample Prep:
Make sure samples to be tested are free of dust or other contaminants, as this will interfere with the measurements.

Dilute samples to be tested as needed. 1:10 dilution in PBS is usually adequate.

Using the 20-200 μL pipette, load 40 μL of each sample into the 384-well plate. Start at the left side of the plate (well 1) and move towards the right side (well 24). Make note of which well contains which sample for later analysis.

**Example 4: Shelf-Life and Gut Stability Short-Term Study**

Results of a shelf-life and gut stability study (14 days, 4 °C) are shown in **FIG. 6**. Each of the two samples tested maintained their particle size during the study as shown in the upper bar graph. Results of a gut stability study (pH 2.5 SGF, simulated gastric fluid and pH 7 SIF, simulated intestinal fluid) are shown in the lower bar graph.

**Example 5: Loading of Exosomes With siRNA via Sonication**

**Purpose:**

To load exosomes with siRNA and/or cholesterol-siRNA via a sonication cycle procedure. The siRNA used is GFP siRNA, a published, validated siRNA control that targets and silences green fluorescent protein expression. Functional testing shows effective knockdown at mRNA and protein levels.

**Materials:**

10X PBS buffer (Gibco #70011-044)
10 - 200 μL pipette tips, RNAse-free (USA Scientific, Catalog #: 1110-3800)
20 - 200 μL pipette tips, RNAse-free (USA Scientific, Catalog #: 1111-0706)
100 - 1000 μL pipette tips, RNAse-free (USA Scientific, Catalog #: 1111-2821)
GFP siRNA (MW 13,925.3 g/mol) (Dharmacon): "siRNA"
GFP siRNA, Accell (MW 14,192.7 g/mol) (Dharmacon): "Cholesterol-siRNA"
RNAse-free 1.5 mL centrifuge tubes (Ambion, Catalog #AM12400)
Exosomes from Colostrum Milk

**Equipment:**

4 °C refrigerator
Qsonica Q700 sonicator
Fisher Scientific Hot plate
Pipettes (various)
Beakers (various)
Spectrum Labs Micro Float-a-Lyzer, 100kD, 250-500 uL (Spectrum Labs #F235071)
1mL syringes

**Buffers:**

1x PBS in nuclease-free water:
10x PBS was diluted 1:10 using nuclease-free water and used for initial sample preparation

**Methods:**

**Sample Preparation and Sonication:**

A 0.25mL batch of both "siRNA Exosomes" and "Cholesterol-siRNA Exosomes" were prepared by adding the correct amounts of reagents to a 1.5 mL centrifuge tube using a pipette, as shown below. Nuclease-free PBS was used. A siRNA/exosome ratio of 500:1 was used for both groups.

**Table 9: Sample siRNA/exosome ratio calculations**

| **Exosome batch** | Final number of exosomes (particles/mL) | Final volume of sample (mL) | **Concentration of exosomes (mol/mL)** | siRNA/ exosome | **Concentration of siRNA (mol/mL)** | Concentration of exosome stock (particle/mL) | | **Volume of siRNA stock (0.1 nmol/uL)** | **Volume of exosomes (mL)** | **Volume of PBS (mL)** |
|---|---|---|---|---|---|---|---|---|---|---|
| Exo1_11JUL2017_02 | 5.00E+12 | 0.25 | 8.31E-12 | 500 | 1.04E-09 | 1.59E+13 | | 0.010 | 0.079 | 0.161 |

**Table 10: Sample batch preparations**

| | **Amount (uL)** | |
|---|---|---|
| | **siRNA Exosomes** | **Chol.-siRNA Exosomes** |
| Exo1_11JUL2017_02 Exosomes from Colostrum Milk | 10 | 10 |
| siRNA | 79 | 79 |
| PBS | 411 | 411 |

*Note:* Samples were prepared according to a 0.25 mL final volume calculation base. However, an additional 250 uL of PBS was added to each sample to ensure that the volume of the samples was sufficient to be sonicated.

Samples were vortexed to ensure complete mixing. A 50 uL aliquot of each sample group was removed ("Pre-sonication") and placed in a 4 °C fridge for later analysis.

Samples were sonicated on Qsonica Q700 at 20% power for 6 cycles of 4 seconds on/2 seconds off followed by 2 minutes on ice and then another 6 cycles.

A 50 uL aliquot of each sample group was removed ("Post-sonication, pre-dialysis) and placed in a 4 °C fridge for later analysis.

**Dialysis to remove free siRNA from sonicated samples:**

Dialysis devices (Spectrum Labs Micro Float-a-Lyzer, 100kD, 250-500 uL (Spectrum Labs #F235071)) were prepared according to manufacturer instructions.

The remaining 400 uL of each of the samples were loaded into the dialysis devices using a 1mL syringe.

The devices were placed in separate 200mL beakers which were filled with roomtemperature PBS.

The beakers were placed on stir plates to gently agitate the fluid and they were covered with aluminum foil.

The samples were left to perform dialysis overnight.

Upon completion of the dialysis, the samples were removed from the devices using a 1mL syringe and placed in separate 1.5mL centrifuge tubes ("Post-sonication, dialysis").

Other methods of loading will also be explored, as shown in Table 11 below.

**Table 11: Exosome Loading Methods**

| **Method** | **Key Parameters** | **Success Criteria** |
|---|---|---|
| Direct Mix | • Cargo:Exosome Ratio<br>• Cargo concentration | Initial Screen<br>• DLS +/- 30% of initial<br>• > 5% cargo associated (by UC) |
| Freeze-Thaw | • # of cycles | |
| Sonication | • # of cycles<br>• Power per cycle<br>• Time per cycle<br>• Ice before/after cycle | Complete Analysis<br>• NTA +/- 30% initial<br>• cryoTEM diameter +/- 30% initial<br>• CD81 Western (+/-30% vs. initial) |
| Saponification | • Concentration of saponin | |

**Example 6: Investigation of Optimal Loading Ratio of Exosome to siRNA**

**Purpose:** To determine the minimal ratio of exosome to siRNA needed for drug loading association.

**Equipment:**

Eppendorf Centrifuge 5430
BioRad Mini Protean Tetra Cell
Biorad Power Pac Basic
Fisher Scientific Hot plate

ChemiDoc 2.0 MP

**Materials:**

10x Tris/Boric Acid/EDTA (TBE) Nucleic Acid Electrophoresis Buffer pH 8.3 (Biorad catalog no. 161-0733)
10% Mini-PROTEAN TBE Gel, 10 well, 30 µl (Biorad catalog no. 4565033)
Gel Loading Buffer II (Thermofisher catalog no. AM8546G)
TipOne, 0.1-10 ul pipette tips (USA scientific catalog no. 1111-3700)
TipOne, 1-200 ul pipette tips (USA scientific catalog no. 1111-0736)
Tip One, 100-1000 ul pipette tips (USA scientific catalog no. 1111-3700)
RNAse free Microfuge tubes 1.5 mL (Ambion catalog no. AM12400 lot no. 02417003)
SYBR Gold Nucleic Acid Gel Stain (Thermofisher catalog no. S11494)
GFP siRNA (MW 13,925.3 g/mol) (Dharmacon)
GFP siRNA, Accell (MW 14,192.7 g/mol) (Dharmacon)
10X PBS buffer (Gibco catalog no. 70011-044 lot no. 1694280)
Nuclease free water, Autoclaved, 0.2 um filtered (Ambion catalog no. AM9939 lot no. 1702082)
**EXO1-LOT26APR2017**

**Buffers/Solutions:**

**1X PBS buffer in Nuclease free water**

NOTE: To prepare a 1X PBS buffer in nuclease free water, dilute the 10X PBS buffer 1:10 using nuclease free water.

**0.1 nmol/ul of siRNA in 1X PBS buffer in Nuclease free water**

NOTE: To prepare 0.1 nmol/ul of siRNA in 1X PBS buffer in Nuclease free water, 300 ul of 1X PBS buffer in Nuclease free water is added to 30 nmol of GFP siRNA (MW 13,925.3 g/mol) (Dharmacon).

**0.1 nmol/ul of chol siRNA in 1X PBS buffer in Nuclease free water**

NOTE: To prepare 0.1 nmol/ul of chol siRNA in 1X PBS buffer in Nuclease free water, 320 ul of 1X PBS buffer in Nuclease free water is added to 32 nmol of GFP chol siRNA (MW 14,192.7 g/mol) (Dharmacon).

**Running buffer: 1X Tris/Boric Acid/EDTA (TBE) Nucleic Acid**

**Electrophoresis Buffer**

130 mM Tris, 45 mM Boric acid, and 2.5 mM EDTA, pH 8.3
NOTE: To prepare a 1X TBE running buffer from 10X stock TBE buffer, mix 100 mL stock TBE buffer with 900 mL of deionized water.

**Loading buffer: Gel loading buffer II**

95% formamide, 18 mM EDTA, 0.025% SDS, 0.025% Xylene cyanol, 0.025% Bromophenol blue

**Procedure:**

**Sample Preparation:**

Take 7 RNase free Microfuge tubes and label them 1-7.
Add 1 ul of 0.1 nmol/ul of chol siRNA to tubes 1-4.
Add 1 ul of 0.1 nmol/ul of siRNA to tubes 6 and 7.
Add the following amounts of EXO1-26APR2017 to the tubes:

Tube 2- 17 ul
Tube 3- 6 ul
Tube 4- 1 ul
Tube 5- 17 ul
Tube 7- 17 ul

Add the following amounts of 1X PBS in nuclease free water to the tubes:

Tube 1- 19 ul
Tube 2- 2 ul
Tube 3- 13 ul
Tube 4- 18 ul
Tube 5- 3 ul
Tube 6- 19 ul
Tube 7- 2 ul

Vortex samples briefly and cover the samples with aluminum foil, then allow them to incubate at room temperature for 90 minutes.

**Preparing samples for Gel Electrophoresis:**

Add 20 ul of Gel loading buffer II to each tube.
Briefly, vortex and spin down all samples w/ gel loading buffer II before placing all tubes into a 95 °C water bath for 5 minutes.
After heat denaturing, spin down the tubes in the centrifuge prior to loading the samples onto the gel. *NOTE: The samples must be loaded to the gel immediately to avoid the formation of secondary structures.*

**PAGE:**

*Set up for the Biorad Mini Protean Tetra Cell Electrophoresis Module*

Take the electrode assembly and set the clamping frame to the open position on a clean flat surface. Remove the tape from the bottom of the gel cassette and place one of the gel cassette (with the short plate facing inward) onto the gel supports; gel supports are molded into the bottom of the clamping frame assembly; there are two supports in each side of the assembly.
*NOTE: The gel will now rest at a 30° angle, tilting away from the center of the clamping frame. Also, use caution when placing the first gel, making sure that the clamping frame remains balanced and does not tip over.* Place the buffer dam on the other side of the clamping frame (with the side wording facing inward) onto the gel supports. *NOTE: At this point both gel cassette and buffer dam are at an angle on the clamping frame.* Using one hand, gently pull both the gel and buffer dam towards each other, making sure that they rest firmly and squarely against the green gaskets that are built into the clamping frame. *NOTE: Make certain that the short plates sit just below the notch at the top of the green gasket.* While gently squeezing the gel cassette and buffer against the green gaskets with one hand (keeping constant pressure on both the gels to keep them in place), slide the green arms of the clamping frame over the gels, locking them into place. Place the electrode assembly in the back position of the cell, making sure that the red (-) and black (+) electrode jack matches the red and black marking on the top right inside edge of the tank. Fill the inner chamber of the electrode assembly with running buffer (1X TBE) to the top of the gel cassette's short plate. Allow the running buffer to over flow the wells in the gel, slightly.

*Sample loading onto Gel*

Using a 0.5-10 ul pipette, add 10 ul of each sample and place them into the wells.

*Gel electrophoresis*

Add enough running buffer (1X TBE) to fill the tank to the line marking 2 gels on the tank.
Place the lid on the Mini-PROTEAN Tetra tank. Make sure to align the color-coded banana plugs and jacks then press down on the lid with your thumbs using even pressure, till the lid is securely and tightly positioned on the tank. *NOTE: The correct orientation is made by matching the jacks on the lid with the banana plugs on the electrode*

*assembly.*

Insert the electrical leads into the Biorad Power Pac Basic supply to the proper polarity. Run the gel at 35 V for 90 minutes.

*Gel Removal*

After electrophoresis is complete, turn off the power supply and disconnect the electrical leads. Remove the tank lid and carefully lift out the electrode assembly. Pour off and discard the running buffer. *NOTE: Always pour off the buffer before opening the arms of the assembly, to avoid spilling the buffer.* Open the arms of the assembly and remove the gel cassettes. To remove the gel from the gel cassette, gently separate the two plates of the gel cassette by cracking the plastic seals on each side of the gel cassette. This can be done by wedging tweezers or scissors between the two plates of the gel cassette from the sides. *NOTE: Do not disrupt the gel while breaking the plastic seal between the two plates of the gel cassette.*

**Fluorescence Imaging of siRNA Polyacrylamide Gel:**

Open the door to the ChemiDoc 2.0 MP and pull out the imaging platform.

Place the Chemi/UV/Stain Free tray on imaging platform and make sure the it is aligned with the white knob on the imaging platform.

Place the small gel guide onto the Chemi/UV/Stain Free tray then place your gel onto the center of the small gel guide.

Slide the imaging platform back into the ChemiDoc and close the door. Once the door is closed, select the camera icon on the top left of the screen.

On the touch screen select camera

Select MULTI.

Select the size of the gel (small).

Select application and set the application to nucleic acid gels and Alex 647 (700/50)

Select exposure and select auto rapid.

On the bottom, left hand corner select the camera to take the image.

**SYBR Gold Nucleic Acid Gel Stain of the Polyacrylamide Gel:**

Remove the gel from the ChemiDoc 2.0 MP and place the gel in a container with enough volume of 1X SYBR Gold Nucleic Acid Gel Stain in 1X TBE buffer to cover the gel then allow the gel to incubate in the stain for 40 minutes under agitation. *NOTE: Cover the gel container with either aluminum foil or a box because SYBR Gold Nucleic Acid Gel Stain is light sensitive.*

**Imaging of Polyacrylamide Gel Stained with SYBR Gold Nucleic Acid Stain:**

Open the door to the ChemiDoc 2.0 MP and pull out the imaging platform.

Place the Chemi/UV/Stain Free tray on imaging platform and make sure the it is aligned with the white knob on the imaging platform.

Place the small gel guide onto the Chemi/UV/Stain Free tray then place gel onto the center of the small gel guide.

Slide the imaging platform back into the ChemiDoc and close the door. Once the door is closed, select the camera icon on the top left of the screen.

On the touch screen select camera, select Single, select the size of the gel (small).

Select application and set the application to nucleic acid gels and SYBR Gold.

Select exposure and select auto rapid.

On the bottom, left hand corner select the camera to take the image.

**Results:**

**FIG. 7** shows results of experiments to determine optimal siRNA to exosomes ratios for loading. The top portion of the figure shows a PAGE gel of RNA stained with SYBR Gold Nucleic Acid stain. The bottom portion of the figure shows PAGE of RNA fluorophore. In the PAGE of RNA fluorophore gel, free chol siRNA was seen in wells 1, 3, and 4 as well as free siRNA in wells 6 and 7. No RNA was detected in lane 5, which is the exosome lane.

In the PAGE of RNA fluorophore gel, chol siRNA was detected close to the beginning of the well in wells 2 and 3. It is also reinforced by the less dense bands of the free chol siRNA in lanes 2 and 3 as well as the distinct bands at

the exosome area of the loaded exosomes on the PAGE of RNA stained with SYBR gold nucleic acid stain (top image) as compared to its control.

Interestingly, in the PAGE of RNA stained with SYBR gold nucleic acid stain, the chol siRNA appears as 4 distinct bands and 2 bands for siRNA as compared 2 and 1 band distinct bands in the PAGE of RNA fluorophore. This could be due to the chol siRNA and siRNA's fluorophore being located on one side of the double stranded structure. During denaturation, the strands are separated and hence, the chol siRNA and siRNA containing the fluorophore showed up on the PAGE of RNA fluorophore and all strands of the chol siRNA and siRNA showed up on the PAGE of RNA staining with SYBR gold nucleic acid stain.

In well 6 and 7, the siRNA band intensity was relatively the same in the control and drug loaded samples. This means the siRNA did not become associated with the exosomes.

**Conclusions:**

Optimal drug loading ratio for chol siRNA and exosomes is above 500 siRNA molecules to 1 exosome particle, e.g. 500 to 1400 chol siRNA molecules to 1 exosome particle. It appears that more particles of exosome (>1 $\times$ $10^{11}$ particles) needed to load siRNA.

Additional ratios were explored and the results are shown in **FIG. 8** and **9**. The gels demonstrate that the amount of siRNA loaded increases with the number of exosomes.

**Example 7: Free-thaw Cycles**

Two 120 uL samples of 500/1 siRNA/exosome were prepared in 1.5 mL Eppendorf tubes. The samples contained:

1) 55 uL exosomes (Exo1_11JUL2017_01; 1.09E13 particles/mL stock), 5 uL siRNA-DY677 (stock 0.1 nmol/uL, 0.1 uM, 4.98E-10 mol/uL), and 60 uL PBS;
2) 55 uL exosomes (Exo1_11JUL2017_01; 1.09E13 particles/mL stock), 7 uL Cholesterol-siRNA-DY677 (stock 0.1 nmol/uL, 0.1 uM), and 58 uL PBS.

Absorbance spectra of siRNA-DY677 and Chol-siRNA-DY677 showed that the cholesterol construct had 1.4-fold higher absorbance at 666 nm (excitation wavelength for DY677) which corresponds to 1.4-fold higher dye concentration. Thus, the volumes of the stock solutions were adjusted to account for this.

The two Eppendorf tubes contacting the two samples of siRNA/exosomes were subjected to 12 freeze-thaw cycles. The tubes were kept on dry ice till they were completely frozen (about 3 min). Subsequently, the tubes were submerged into a water bath at 37 °C and kept till the solution turned liquid (about 1 min). After the $11^{th}$ cycle the samples were placed in -80 °C freezer and kept for 2 days before using.

The samples were used in Stern-Volmer quenching experiments.

**Example 8: Stern-Volmer Quenching of siRNA**

**Constructs Encapsulated by Freeze-thawing**

Methyl viologen (paraquat) is a good electron acceptor and therefore can participate in electron transfer quenching of fluorescence of many fluorophores. $MV^{2+}$ is also water soluble and lipid membrane impermeable. Therefore, it would quench the emission of dyes that are water soluble and will not interact with dyes that are encapsulated in lipid membranes (liposomes, exosomes).

Thus, an encapsulated dye fraction can be calculated using the Stern-Volmer equation:

$$\frac{I_0}{I} = 1 + K_{SV}[Q]$$

If the fluorophore is fully exposed to the quencher, emission of the fluorophore will decrease linearly with increasing quencher concentration. If there is an unquenchable fraction (encapsulated dye), the emission will reach a plateau. Full quenching will be observed with about 1 M concentration of the quencher.

**Materials:**

10X PBS buffer (Gibco #70011-044)
10 - 200 μL pipette tips, RNAse-free (USA Scientific, Catalog #: 1110-3800)
20 - 200 μL pipette tips, RNAse-free (USA Scientific, Catalog #: 1111-0706)
100 - 1000 μL pipette tips, RNAse-free (USA Scientific, Catalog #: 1111-2821)
GFP siRNA (MW 13,925.3 g/mol) (Dharmacon)
GFP siRNA, Accell (MW 14,192.7 g/mol) (Dharmacon): "Cholesterol-siRNA"
RNAse-free 1.5 mL centrifuge tubes (Ambion, Catalog #AM12400)
5% Mini-PROTEAN TBE Gel (BioRad #4565013)
TBE gel running buffer
Gel loading buffer II
Dry Ice
Exo1_7AUG2017_01 Exosomes from Colostrum Milk
Colostrum powder
Methyl viologen

**Equipment:**

Tecan wellplate reader
Qsonica Q700 sonicator
BioRad ChemiDoc MP Imaging System
BioRad Mini Protean Tetra Cell
Biorad Power Pac Basic
Fisher Scientific Hot plate
Pipettes (various)
Beakers (various)

**Buffers:**

1x PBS in nuclease-free water

**Methods:**

***Sample Preparation***

The following samples were prepared (total volume 120 uL). The ratio of siRNA/exosomes was kept constant at 500/1:

**siRNA/PBS:** 5 uL siRNA-DY677 (0.1 nmol) in 115 uL PBS (0.004 nmol);
**Ch-siRNA/PBS:** 7 uL Chol-siRNA-DY677 in 113 uL PBS (0.004 nmol);
**siRNA/exo-0cycles:** 55 uL exosomes (Exo1_11JUL2017_01; 1.09E13 particles/mL stock), 5 uL siRNA-DY677 (stock 0.1 nmol/uL, 0.1 uM, 4.98E-10 mol/uL), and 60 uL PBS. The exosomes, siRNA, and PBS were left incubating for 90 min at rt in the dark.
**Ch-siRNA/exo-0cycles:** 55 uL exosomes (Exo1_11JUL2017_01; 1.09E13 particles/mL stock), 7 uL Cholesterol-siRNA-DY677 (stock 0.1 nmol/uL, 0.1 uM), and 58 uL PBS. The exosomes, siRNA, and PBS were left incubating for 90 min at rt in the dark.
**siRNA/exo-12cycles:** 55 uL exosomes (Exo1_11JUL2017_01; 1.09E13 particles/mL stock), 5 uL siRNA-DY677 (stock 0.1 nmol/uL, 0.1 uM, 4.98E-10 mol/uL), and 60 uL PBS. The sample was subjected to 12 freeze-thaw cycles.
**Ch-siRNA/exo-12cycles:** 55 uL exosomes (Exo1_11JUL2017_01; 1.09E13 particles/mL stock), 7 uL Cholesterol-siRNA-DY677 (stock 0.1 nmol/uL, 0.1 uM), and 58 uL PBS. The sample was subjected to 12 freeze-thaw cycles.
**siRNA/Colostrum:** take 1.7 mg colostrum powder and hydrate with 5 uL siRNA-DY677 (stock 0.1 nmol/uL) and 115 uL PBS. Subject the suspension to 4 x Is pulses at 1 % amplitude (total energy 2 J). Centrifuge the sample at 1500 cfm for 1 min at 4 °C.

**Ch-siRNA/Colostrum:** 1.7 mg colostrum powder was hydrated with 7 uL Ch-siRNA-DY677 (stock 0.1 nmol/uL) and 113 uL PBS. Subject the suspension to 2 x ls pulses at 1 % amplitude (total energy 2 J). Centrifuge the sample at 1500 cfm for 1 min at 4 °C.

**Freeze-thaw cycles of exosomes**

Exosomes, siRNA-Dy677 or chsiRNA-DY677, and PBS were mixed in the amounts noted above for a total sample volume of 120 uL. Samples were then vortexed for 5 seconds to ensure complete mixing. The samples were placed on dry ice for 4 minutes or until the samples were completely frozen. The samples were then removed from ice and placed in a room-temperature water bath for 3 minutes or until the samples were completely thawed. The process was repeated 11 times. The $12^{th}$ cycle was done as the samples were placed in a -80 °C freezer.

**Quenching experiment**

80 uL were taken from each sample and diluted to 600 uL. The solutions were split in two. To one set of 300 uL solutions 18 mg of Methyl Viologen ($MV^{2+}$) were added for a final concentration of 0.233 M. Each sample was placed in a clear 96-wellplate in the following amounts/well: 50, 45, 40, 35, 30, 25, 20, 15, 10, 0 uL. To the wells the corresponding amounts of MV stock solution were added for a total volume of 50 uL/well: 0, 5, 10, 15, 20, 25, 30, 25, 40, 50 uL. The emission was recorded using a wellplate reader at 700 nm (20 nm bandwidth) upon excitation at 666 nm (9 nm bandwidth) with 35 flashes, 120 gain, 20 us integration time, and multiple reads per well (4x4). The data were analyzed according to the Stern-Volmer equation:

$$\frac{I_0}{I} = 1 + K_{SV}[Q]$$

**Results:**

The data were plotted as $I_0/I$ vs $[MV^{2+}]$ . The results are shown in FIG. 13. Linear decrease in fluorescence was observed in samples containing siRNA. This points to the availability of the dye to the quencher and therefore lack of encapsulation and protection from the exosomes. Linear decrease in fluorescence was observed in samples of Colostrum/siRNA. However, the slope was lower compared to that of siRNA in PBS or in exosomes. The lack of plateau suggests that the siRNA is not encapsulated but is interacting with the colostrum and is less available for the quencher.

ChsiRNA is fully quenched in PBS. Unquenchable fraction is noticed in samples of chsiRNA mixed with exosomes 500/1, chsiRNA-exosomes subjected to 12 freeze-thaw cycles, and chsiRNA mixed with colostrum and sonicated for 4x1 s cycles.

**Table 12: Mixing vs. Free-Thaw Cycles vs. Sonication and Resulting Loading**

| Freeze-Thaw | siRNA/Exo (500/1) | Freeze-Thaw | Ch-siRNA/Exo (500/1) |
|---|---|---|---|
| **PBS** | 5.9 | **PBS** | 4.6 |
| **0 cycles** | 8.6 | **0 cycles** | 26.0 |
| **12 cycles** | 7.5 | **12 cycles** | 32.1 |
| **Colostrum (sonicate)** | 17.2 | **Colostrum (sonicate)** | 31.1 |

**Gel electrophoresis:**

5 % polyacrylamide TBE gel was used in Mini-PROTEAN® Electrophoresis Cell. The gel was run at 50 V for 1 h. The siRNA was stained with SYBR Gold (10000:1 dilution) for 40 min and imaged using BioRad ChemiDoc MP Imaging System.

***Sample Preparation for Gel Electrophoresis*:**

Transfer 7.5 ul of each sample to a new RNase Free Microfuge tube, 1mL. The samples are as follows:

siRNA Exosomes, post-sonication, pre-dialysis

Chol.-siRNA Exosomes, post-sonication, pre-dialysis
siRNA Exosomes, post-sonication, dialysis
Chol.-siRNA Exosomes, post-sonication, dialysis
siRNA Exosomes, post-sonication, pre-dialysis
Chol.-siRNA Exosomes, post-sonication, pre-dialysis
siRNA Exosomes, post-sonication, dialysis
Chol.-siRNA Exosomes, post-sonication, dialysis.

Add 7.5 ul of Gel loading buffer II to each RNase Free Microfuge tube. Briefly, vortex and spin down samples 1-4 w/ gel loading buffer II before placing all tubes into a 95C water bath for 5 minutes. After heat denaturing, spin down samples in the centrifuge at 1000 g for 1 min prior to loading the samples on the gel.

**PAGE Analysis**

*Set up for the Biorad Mini Protean Tetra Cell Electrophoresis Module*

Take the electrode assembly and set the clamping frame to the open position on a clean flat surface. Remove the tape from the bottom of the gel cassette and place both of the gel cassette (with the short plate facing inward) onto the gel supports; gel supports are molded into the bottom of the clamping frame assembly; there are two supports in each side of the assembly.

*NOTE: The gel will now rest at a 30° angle, tilting away from the center of the clamping frame. Also, use caution when placing the first gel, making sure that the clamping frame remains balanced and does not tip over.* Using one hand, gently pull both the gels toward each other, making sure that they rest firmly and squarely against the green gaskets that are built into the clamping frame. *NOTE: Make certain that the short plates sit just below the notch at the top of the green gasket.*

While gently squeezing the gel cassette and buffer against the green gaskets with one hand (keeping constant pressure on both the gels to keep them in place), slide the green arms of the clamping frame over the gels, locking them into place.

Place the electrode assembly in the back position of the cell, making sure that the red (-) and black (+) electrode jack matches the red and black marking on the top right inside edge of the tank.

Fill the inner chamber of the electrode assembly with running buffer (IX TBE) to the top of the gel cassette's short plate. Allow the running buffer to over flow the wells in the gel, slightly.

*Sample loading onto Gel*

Using a 0.5-10 ul pipette, add 10 ul of each sample and place them into the wells.

*Gel electrophoresis*

Add enough running buffer (IX TBE) to fill the tank to the line marking 2 gels on the tank. Place the lid on the Mini-PROTEAN Tetra tank. Make sure to align the color-coded banana plugs and jacks then press down on the lid with your thumbs using even pressure, till the lid is securely and tightly positioned on the tank. *NOTE: The correct orientation is made by matching the jacks on the lid with the banana plugs on the electrode assembly.*

Insert the electrical leads into the Biorad Power Pac Basic supply to the proper polarity. Run the gel at 50 V for 75 minutes.

*Gel Removal*

After electrophoresis is complete, turn off the power supply and disconnect the electrical leads. Remove the tank lid and carefully lift out the electrode assembly. Pour off and discard the running buffer. *NOTE: Always pour off the buffer before opening the arms of the assembly, to avoid spilling the buffer.* Open the arms of the assembly and remove the gel cassettes.

To remove the gel from the gel cassette, gently separate the two plates of the gel cassette by cracking the plastic seals on each side of the gel cassette. This can be done by wedging tweezers or scissors between the two plates of the gel cassette from the sides. *NOTE: Do not disrupt the gel while breaking the plastic seal between the two plates of the gel cassette.*

Rinse the Mini-PROTEAN Tetra cell electrode assembly, clamping frame, and mini tank with distilled water after use.

**SYBR Gold Nucleic Acid Gel Stain of the Polyacrylamide Gel**

To stain the gel, place the gel in a container with enough volume of IX SYBR Gold Nucleic Acid Gel Stain in 1x TBE buffer to cover the gel and allow the gel to incubate in the stain for 40 minutes in the dark, under agitation.

*Imaging*

After staining, gels were imaged on BioRad ChemiDoc MP Imaging System for SYBR Gold and Alexa 647. Results are shown in **FIG. 15** and **FIG. 16.**

**Stern-Volmer quenching of sonicated exosomes with chsiRNA:**

The following samples were prepared (total volume 150 uL). The amount of chsiRNA was kept constant and the ratios of chsiRNA/exosomes were varied. The samples were either sonicated for 5×1s cycles at 1% amplitude or not subjected to sonication:

**Ch-siRNA/exo-250/1-0cycles:** 110 uL exosomes (Exo1_7AUG2017_01; 7.36E12 particles/mL stock), 5 uL Cholesterol-siRNA-DY677 (stock 0.1 nmol/uL, 0.1 uM), and 35 uL PBS. The mixture was left incubating for 90 min on ice in the dark.
**Ch-siRNA/exo-500/1-0cycles:** 55 uL exosomes (Exo1_7AUG2017_01; 7.36E12 particles/mL stock), 5 uL Cholesterol-siRNA-DY677 (stock 0.1 nmol/uL, 0.1 uM), and 90 uL PBS. The mixture was left incubating for 90 min on ice in the dark.
**Ch-siRNA/exo-1000/1-0cycles:** 27.6 uL exosomes (Exo1_7AUG2017_01; 7.36E12 particles/mL stock), 5 uL Cholesterol-siRNA-DY677 (stock 0.1 nmol/uL, 0.1 uM), and 117 uL PBS. The mixture was left incubating for 90 min on ice in the dark.
**Ch-siRNA/exo-250/1-5cycles:** 110 uL exosomes (Exo1_7AUG2017_01; 7.36E12 particles/mL stock), 5 uL Cholesterol-siRNA-DY677 (stock 0.1 nmol/uL, 0.1 uM), and 35 uL PBS. The mixture was sonicated for 5xls pulses with 2 s time off between pulses at 1% amplitude for a total energy of 18 J. The mixture was left incubating for 90 min on ice in the dark.
**Ch-siRNA/exo-500/1-5cycles**: 55 uL exosomes (Exo1_7AUG2017_01; 7.36E12 particles/mL stock), 5 uL Cholesterol-siRNA-DY677 (stock 0.1 nmol/uL, 0.1 uM), and 90 uL PBS. The mixture was sonicated for 5xls pulses with 2 s time off between pulses at 1% amplitude for a total energy of 18 J. The mixture was left incubating for 90 min on ice in the dark.
**Ch-siRNA/exo-1000/1-5cycles:** 27.6 uL exosomes (Exo1_7AUG2017_01; 7.36E12 particles/mL stock), 5 uL Cholesterol-siRNA-DY677 (stock 0.1 nmol/uL, 0.1 uM), and 117 uL PBS. The mixture was sonicated for 5xls pulses with 2 s time off between pulses at 1% amplitude for a total energy of 18 J. The mixture was left incubating for 90 min on ice in the dark.
**Ch-siRNA/Colostrum-10** cycles: 2 mg colostrum powder were hydrated with 5 uL Ch-siRNA-DY677 (stock 0.1 nmol/uL) and 145 uL PBS. The suspension was subjected to 10×1s sonication pulses with 2 s time off between pulses at 1% amplitude for a total energy of 30 J. Centrifuge the sample at 1500 cfm for 1 min at 4 C.

100 uL were taken from each sample and diluted to 700 uL with PBS. The solutions were split in two. To 320 uL solutions 20 mg of Methyl Viologen ($MV^{2+}$) were added for a final concentration of 0.243 M. Each sample was placed in a clear 96-wellplate in the following amounts/well: 70, 60, 50, 40, 30, 20, 10, 0 uL. To the wells the corresponding amounts of MV stock solution were added for a total volume of 70 uL/well: 0, 10, 20, 30, 40, 50, 60, 70 uL. The emission was recorded at 700 nm (20 nm bandwidth) upon excitation at 666 nm (9 nm bandwidth) with 35 flashes, 120 gain, 20 us integration time, and multiple reads per well (4x4). The data was analyzed according to the Stern-Volmer equation:

$$\frac{I_0}{I} = 1 + K_{SV}[Q]$$

The data was plotted $I_0/I$ vs $[MV^{2+}]$ . As shown in **FIG. 17** and **FIG. 18,** encapsulation is affected by both sonication and the ratio of siRNA to exosomes employed.

**Table 13: Results of Encapsulation with Mixing or Sonication**

| Ch-siRNA/Exo | No Sonication | Ch-siRNA/Exo | Sonication |
|---|---|---|---|
| **250/1** | 39.2 | **250/1** | 41.6 |
| **500/1** | 34.7 | **500/1** | 36.5 |
| **1000/1** | 28.0 | **1000/1** | 27.0 |
| **PBS** | 5.3 | **Colostrum** | 46.4 |

**Gel electrophoresis:**

5 % polyacrylamide TBE gel was used in Mini-PROTEAN® Electrophoresis Cell. The gel was run at 120 V for 20 min in an ice bath. The siRNA was stained with SYBR Gold (10000:1 dilution) for 40 min and imaged using BioRad ChemiDoc MP Imaging System for SYBR Gold and Alexa 647. The results are shown in **FIG. 19** and **20.**

**Purification of chol siRNA loaded exosomes via Ultracentrifugation:**

800 uL chsiRNA/exosome sample was prepared at 500/1 loading ratio. 21 uL chsiRNA (0.1 nmol/ul) was mixed with 340 uL exosomes (7AUG17_1) and 639 uL PBS. The Exosome concentration was $2.5 \times 10^{12}$ particles/mL.
Transfer 800 ul of sample into individual 4.4 mL Beckman Coulter centrifuge tube.
Add 1.2 mL of IX PBS buffer in Nuclease free water to each centrifuge tube. Place the centrifuge tubes into the rotor (SW 60 Ti). *NOTE: Rotor balanced with additional centrifuge tubes with the appropriate volumes.*
Place the rotor into the Beckman Counter Optima XE90 Ultracentrifuge. Centrifuge the samples at 135,000 RCF for 100 minutes at 4 °C.
After centrifugation, remove the centrifuge tubes from the rotor then remove the supernatant from each centrifuge tube.
Resuspend the pellet in 800 ul of IX PBS buffer in Nuclease free water.

**Example 9: Cholesterol solubilization by 3.8 mM**

**Methyl beta cyclodextrin and 1% Triton X**

Methyl beta cyclodextrin (MBCD) is a water soluble macromolecule with hydrophobic center that can fit in its cavity cholesterol, thus rendering cholesterol water soluble. It is used to deplete lipid membranes in cells from cholesterol. See **FIG. 10** for an illustration.
Triton X is a molecule that acts as a surfactant and can create holes in lipid membranes.

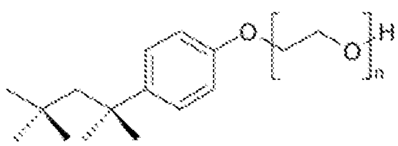

Therefore, MBCD should pull out (solubilize) chsiRNA from exosomes and Triton X should burst the exosomes without pulling out chsiRNA from the lipid membrane.

**Sample preparation:**

chsiRNA/exosomes (500/1) stock solution before ultracentrifugation (800 uL chsiRNA/exosome sample was prepared at 500/1 loading ratio. 21 uL chsiRNA (0.1 nmol/ul) was mixed with 340 uL exosomes (7AUG17_1) and 639 uL PBS. The Exosome concentration was 2.5 x $10^{12}$ particles/mL)

chsiRNA/exosomes (500/1) resuspended pellet after ultracentrifugation
supernatant from ultracentrifugation
chsiRNA/exosomes (500/1) resuspended pellet after ultracentrifugation containing 1 % Triton X (see sample preparation below)
chsiRNA/exosomes (500/1) resuspended pellet after ultracentrifugation containing 3.8 mM MBCD (see sample

preparation below)

chsiRNA/exosomes (500/1) stock solution sonicated with 5 pulses before ultracentrifugation (340 uL exosomes (Exo1_7AUG2017_01; 7.36 x $10^{12}$ particles/mL stock), 21 uL Cholesterol-siRNA-DY677 (stock 0.1 nmol/uL, 0.1 uM), and 639 uL PBS. The mixture was sonicated for 5xls pulses with 2 s time off between pulses at 1% amplitude for a total energy of 18 J. The mixture was left incubating for 90 min on ice in the dark)

chsiRNA/exosomes (500/1) resuspended pellet after ultracentrifugation (from sonicated stock) supernatant from ultracentrifugation (from sonicated stock)

chsiRNA/colostrum stock (13 mg colostrum powder were hydrated with 21 uL Ch-siRNA-DY677 (stock 0.1 nmol/uL) and 979 uL PBS. The suspension was subjected to 10×1s sonication pulses with 2 s time off between pulses at 1% amplitude for a total energy of 30 J. Centrifuge the sample at 1500 cfm for 1 min at 4 C).

90 ul sample from ultracentrifuged chsiRNA/exo (500/1) pellet were mixed with either 10 ul of 38.2 mM solution of methyl beta cyclodextrin in PBS or 10 uL of 10 % Triton X in PBS. The final concentration was 3.8 mM MBCD and 1% Triton X.

60 ul of each sample were placed in a black plastic well plate. The emission was recorded at 700 nm (20 nm bandwidth) upon excitation at 666 nm (9 nm bandwidth) with 35 flashes, 120 gain, 20 μs integration time, and multiple reads per well (4x4). Absorbance spectra for each sample were recorded from 550 to 750 nm with 2 nm steps. The results are shown in **FIG. 21-22.**

**Gel electrophoresis:**

5% polyacrylamide TBE gel was used in Mini-PROTEAN® Electrophoresis Cell. The gel was run at 35 V for 90 min. The chsiRNA was stained with SYBR Gold (10000:1 dilution) for 40 min and imaged using BioRad ChemiDoc MP Imaging System for SYBR Gold and Alexa 647. The results are shown in **FIG. 23-24.**

## NUMBERED EMBODIMENTS

The present disclosure is also represented by the following numbered embodiments:

1. A therapeutic-loaded milk exosome, wherein the therapeutic is a biologic therapeutic agent and the therapeutic is not naturally occurring in a milk exosome.

2. The therapeutic-loaded milk exosome of embodiment 1, wherein the biologic therapeutic agent is selected from an antibody, a hormone, a factor, a cofactor, a metabolic enzyme, an immunoregulatory enzyme, an interferon, an interleukin, a gastrointestinal enzyme, an enzyme or factor implicated in hemostasis, a growth regulatory enzyme, a vaccine, an antithrombolytic, a toxin, or an antitoxin.

3. The therapeutic-loaded milk exosome of embodiment 1, wherein the biologic therapeutic agent is a peptide.

4. The therapeutic-loaded milk exosome of embodiment 3, wherein the biologic therapeutic agent is a protein.

5. The therapeutic-loaded milk exosome of embodiment 1, wherein the biologic therapeutic agent is a nucleic acid.

6. The therapeutic-loaded milk exosome of embodiment 5, wherein the nucleic acid is selected from a single-stranded or double-stranded DNA, an iRNA, a siRNA, a shRNA, a mRNA, a non-coding RNA (ncRNA), an antisense RNA, a LNA, a morpholino oligonucleotide, or an analog or conjugate thereof.

7. The therapeutic-loaded milk exosome of embodiment 5, wherein the nucleic acid is a ncRNA of about 30 to about 200 nucleotides (nt) in length or a long non-coding RNA (IncRNA) of about 200 to about 800 nt in length.

8. The therapeutic-loaded milk exosome of embodiment 7, wherein the IncRNA is a long intergenic non-coding RNA (lincRNA), pretranscript, pre-miRNA, pre-mRNA, competing endogenous RNA (ceRNA), small nuclear RNA (sn-RNA), small nucleolar RNA (snoRNA), pseudo-gene, rRNA, or tRNA.

9. The therapeutic-loaded milk exosome of embodiment 7, wherein the ncRNA is selected from a piwi-interacting RNA (piRNA), primary miRNA (pri-miRNA), or premature miRNA (pre miRNA).

10. The therapeutic-loaded milk exosome of embodiment 1, wherein the biologic therapeutic agent is selected from any of those set forth in any of Table 1, Table 2, Table 3, or Table 4.

11. The therapeutic-loaded milk exosome of any one of embodiments 1-10, wherein the milk exosome is derived from cow, sheep, goat, camel, buffalo, yak, or human milk or colostrum.

12. A therapeutic-loaded milk exosome, wherein the therapeutic is a biologic therapeutic agent conjugated to a hydrophobic group.

13. The therapeutic-loaded milk exosome of embodiment 12, wherein the biologic therapeutic agent is selected from an antibody, a hormone, a factor, a cofactor, a metabolic enzyme, an immunoregulatory enzyme, an interferon, an interleukin, a gastrointestinal enzyme, an enzyme or factor implicated in hemostasis, a growth regulatory enzyme, a vaccine, an antithrombolytic, a toxin, or an antitoxin.

14. The therapeutic-loaded milk exosome of embodiment 12, wherein the biologic therapeutic agent is a peptide.

15. The therapeutic-loaded milk exosome of embodiment 14, wherein the biologic therapeutic agent is a protein.

16. The therapeutic-loaded milk exosome of embodiment 12, wherein the biologic therapeutic agent is a nucleic acid.

17. The therapeutic-loaded milk exosome of embodiment 16, wherein the nucleic acid is selected from a single-stranded or double-stranded DNA, an iRNA, a siRNA, a shRNA, a mRNA, a ncRNA, an antisense RNA, a LNA, a morpholino oligonucleotide, or an analog or conjugate thereof.

18. The therapeutic-loaded milk exosome of embodiment 16, wherein the nucleic acid is a non-coding RNA (ncRNA) of about 30 to about 200 nucleotides (nt) in length or a long non-coding RNA (lncRNA) of about 200 to about 800 nt in length.

19. The therapeutic-loaded milk exosome of embodiment 18, wherein the IncRNA is a long intergenic non-coding RNA (lincRNA), pretranscript, pre-miRNA, pre-mRNA, competing endogenous RNA (ceRNA), small nuclear RNA (snRNA), small nucleolar RNA (snoRNA), pseudo-gene, rRNA, or tRNA.

20. The therapeutic-loaded milk exosome of embodiment 19, wherein the ncRNA is selected from a piwi-interacting RNA (piRNA), primary miRNA (pri-miRNA), or premature miRNA (pre miRNA).

21. The therapeutic-loaded milk exosome of embodiment 12, wherein the biologic therapeutic agent is selected from any of those set forth in any of Table 1, Table 2, Table 3, or Table 4.

22. The therapeutic-loaded milk exosome of any one of embodiments 12-21, wherein the hydrophobic group is selected from a lipid, a sterol, a steroid, a terpene, cholic acid, adamantane acetic acid, 1-pyrene butyric acid, 1,3-bis-0(hexadecyl)glycerol, a geranyloxyhexyl group, hexadecylglycerol, borneol, 1,3-propanediol, heptadecyl group, 03-(oleoyl)lithocholic acid, 03- (oleoyl)cholenic acid, dimethoxytrityl, or phenoxazine.

23. The therapeutic-loaded milk exosome of any one of embodiments 12-22, wherein the milk exosome is derived from cow, sheep, goat, camel, buffalo, yak, or human milk or colostrum.

24. A pharmaceutical composition comprising the therapeutic-loaded milk exosome according to any one of embodiments 1-23, and a pharmaceutically acceptable adjuvant, vehicle, or carrier.

25. A method of treating a disease, disorder, or condition in a patient in need thereof, comprising administering to the patient the therapeutic-loaded milk exosome according to any one of embodiments 1-23, or a pharmaceutically acceptable composition thereof.

26. The method according to embodiment 25, wherein the disease, disorder, or condition is selected from a hyperproliferative disorder, viral or microbial infection, autoimmune disease, allergic condition, inflammatory disease, cardiovascular disease, metabolic disease, or neurodegenerative disease.

27. The method according to embodiment 25 or 26, wherein the disease, disorder, or condition is selected from those set forth in Table 1, 2, 3, 4, or 5.

28. The method according to any one of embodiments 25-27, wherein the therapeutic-loaded milk exosome is

administered orally.

29. The method according to any one of embodiments 25-28, further comprising administering to the patient an additional therapeutic agent.

## Claims

1. A microvesicle comprising a biologic therapeutic agent encapsulated by the microvesicle, wherein the biologic therapeutic agent is conjugated to a hydrophobic group; and wherein the biologic therapeutic agent is not naturally-occurring in the microvesicle.

2. The microvesicle of claim 1, wherein the biologic therapeutic agent is a nucleic acid.

3. The microvesicle of claim 2, wherein the nucleic acid is selected from the group consisting of a single-stranded or double-stranded DNA, an iRNA, a siRNA, a shRNA, a mRNA, a non-coding RNA (ncRNA), an antisense RNA, a LNA, a morpholino oligonucleotide, or an analog or conjugate thereof.

4. The microvesicle of claim 3, wherein the nucleic acid is a siRNA modified to comprise a lipid or steroid, which optionally is cholesterol.

5. The microvesicle of any one of claims 1 to 4, wherein the hydrophobic group is selected from a lipid, a sterol, a steroid, a terpene, cholic acid, adamantane acetic acid, 1-pyrene butyric acid, 1,3-bis-O(hexadecyl)glycerol, a geranyloxyhexyl group, hexadecylglycerol, borneol, 1,3-propanediol, heptadecyl group, O3-(oleoyl)lithocholic acid, O3-(oleoyl)cholenic acid, dimethoxytrityl, or phenoxazine.

6. The microvesicle of claim 5, wherein the hydrophobic group is a sterol or a steroid.

7. The microvesicle of claim 6, wherein the hydrophobic group is cholesterol.

8. The microvesicle of any one of claims 1 to 7, wherein the microvesicle is about 30 to 1000 nm in size.

9. A pharmaceutical composition comprising the microvesicle of one of claims 1 to 8, and a pharmaceutically acceptable adjuvant, vehicle, or carrier.

10. The microvesicle of any one of claims 1 to 9, or a pharmaceutical composition thereof, for use in treating a disease, disorder, or condition in a patient in need thereof.

11. The microvesicle, or the pharmaceutical composition thereof, for use according to claim 10, wherein the disease, disorder, or condition is selected from a hyperproliferative disorder, viral or microbial infection, autoimmune disease, allergic condition, inflammatory disease, cardiovascular disease, metabolic disease, or neurodegenerative disease.

12. The microvesicle, or the pharmaceutical composition thereof, for use according to claim 10 or claim 11, wherein the microvesicle is administered orally.

FIG. 1

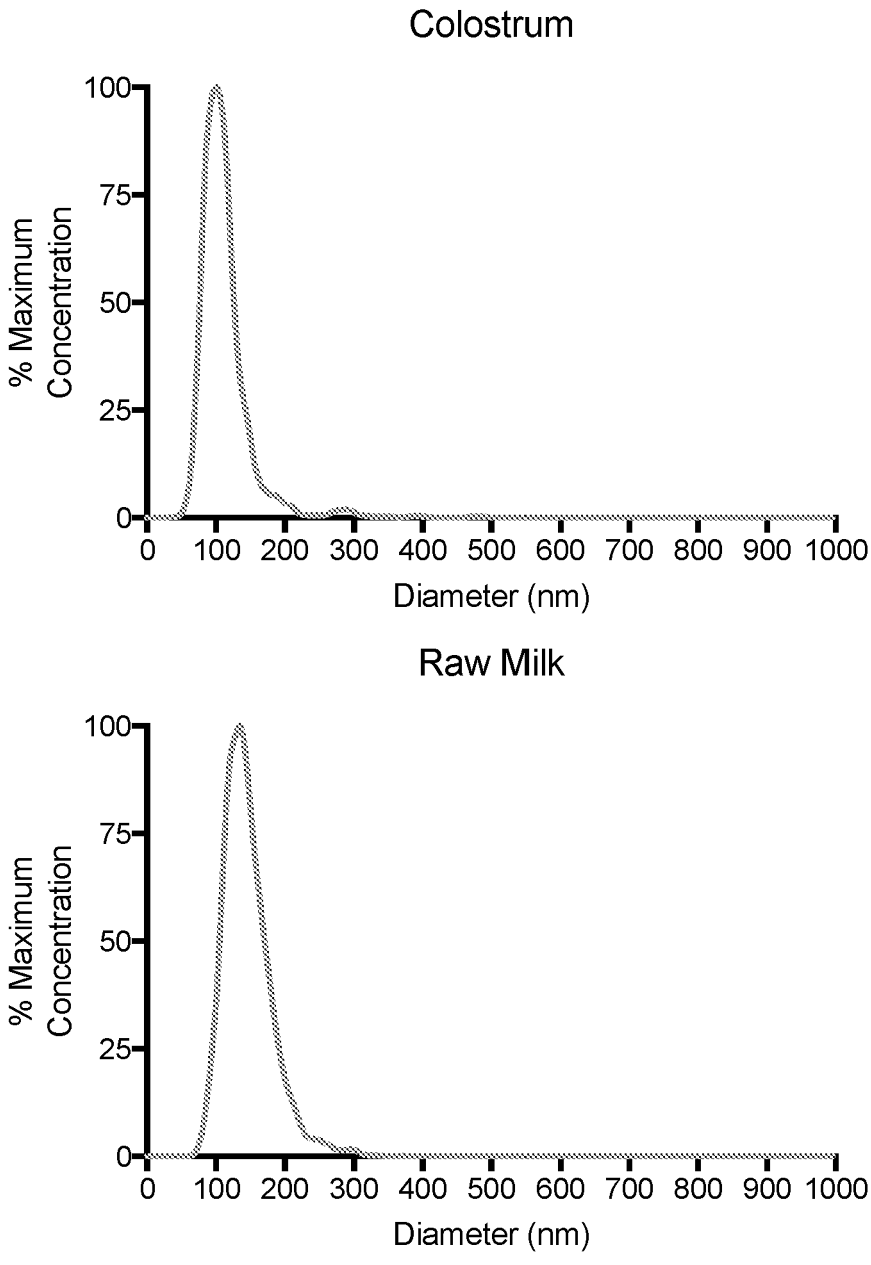
Colostrum
100
75
50
25
0
% Maximum Concentration
0 100 200 300 400 500 600 700 800 900 1000
Diameter (nm)
Raw Milk
100
75
50
25
0
% Maximum Concentration
0 100 200 300 400 500 600 700 800 900 1000
Diameter (nm)

FIG. 2

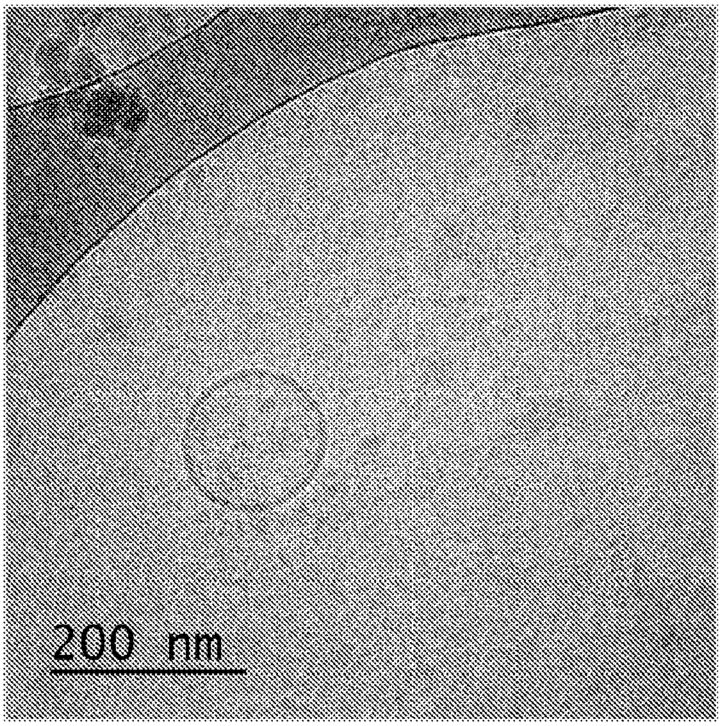


Colostrum Powder Cryo-TEM

FIG. 3

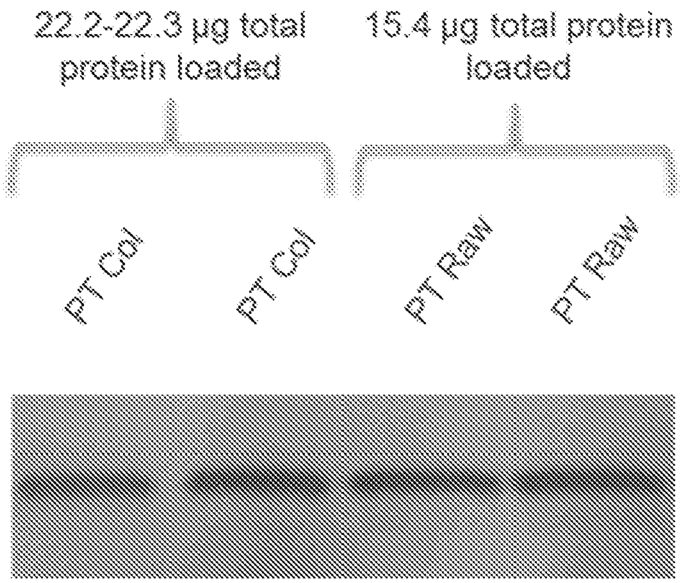
22.2-22.3 µg total protein loaded
15.4 µg total protein loaded
PT Col
PT Col
PT Raw
PT Raw

FIG. 4

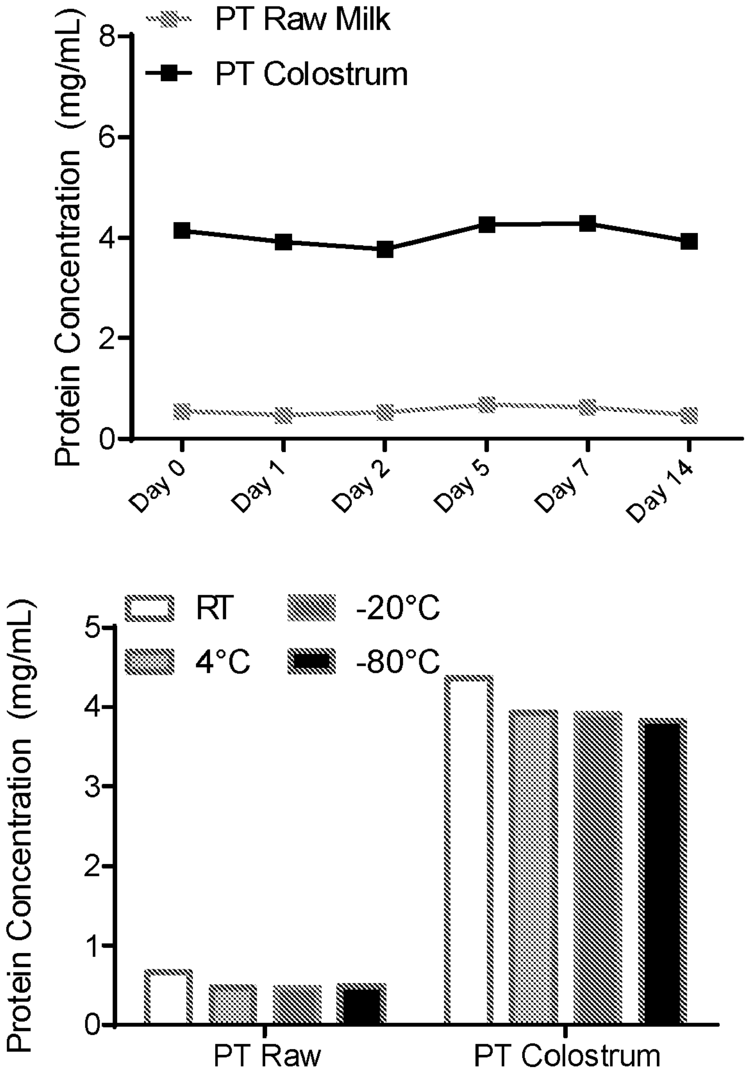
PT Raw Milk
PT Colostrum
Protein Concentration (mg/mL)
8
6
4
2
0
Day 0
Day 1
Day 2
Day 5
Day 7
Day 14
RT
-20°C
4°C
-80°C
5
3
1
PT Raw
PT Colostrum

FIG. 5

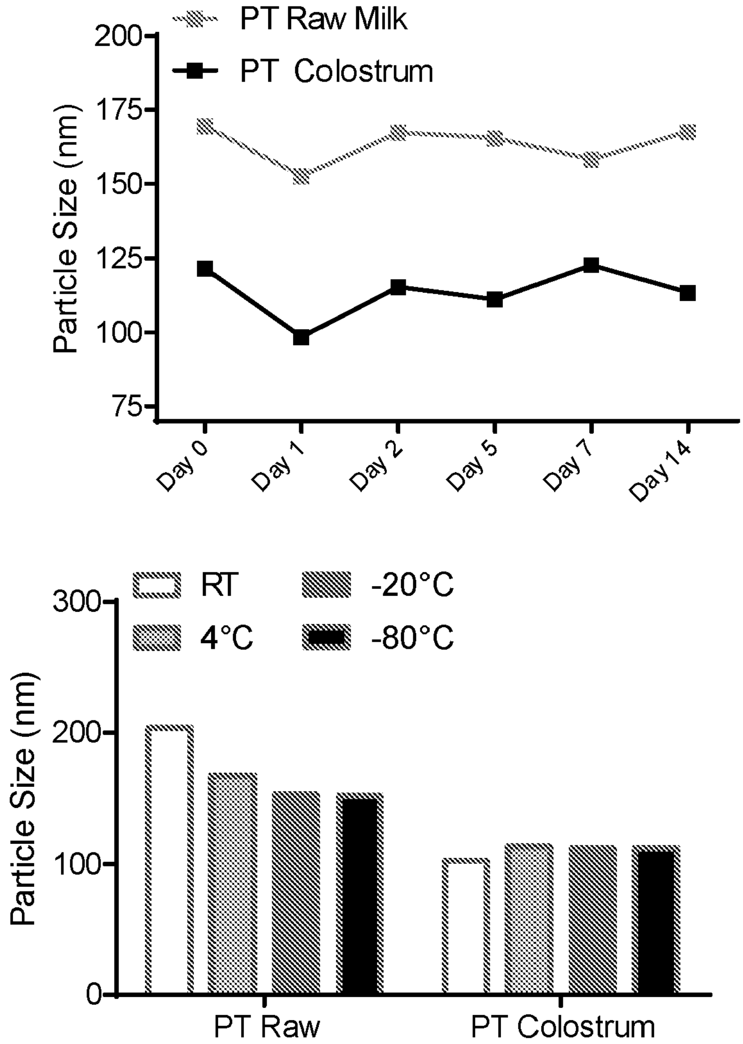
PT Raw Milk
PT Colostrum
Particle Size (nm)
200
175
150
125
100
75
Day 0
Day 1
Day 2
Day 5
Day 7
Day 14
RT
-20°C
4°C
-80°C
Particle Size (nm)
300
200
100
0
PT Raw
PT Colostrum

FIG. 6

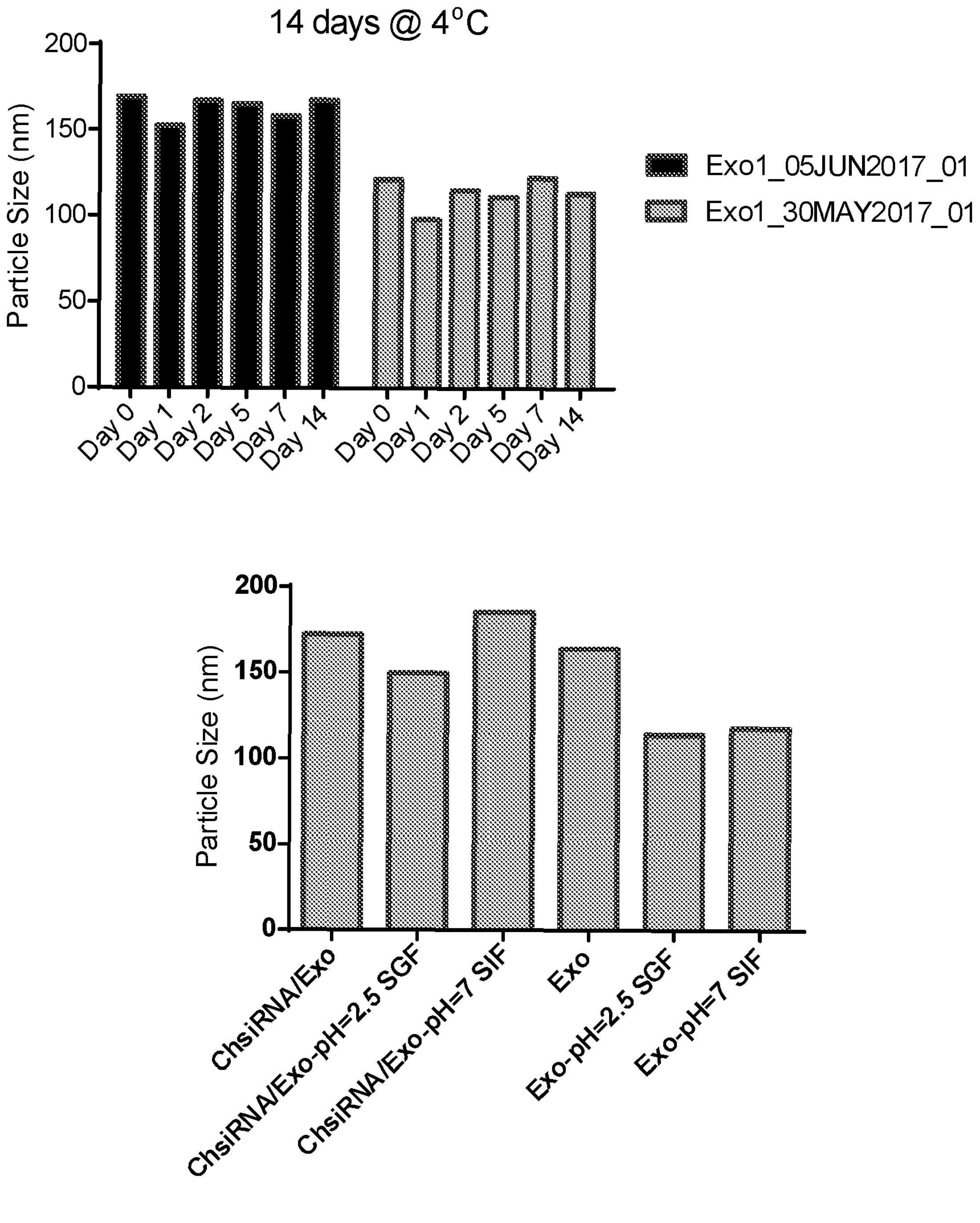
14 days @ 4°C
Particle Size (nm)
200
150
100
50
0
Day 0
Day 1
Day 2
Day 5
Day 7
Day 14
Day 0
Day 1
Day 2
Day 5
Day 7
Day 14
Exo1_05JUN2017_01
Exo1_30MAY2017_01
Particle Size (nm)
200
150
100
50
0
ChsiRNA/Exo
ChsiRNA/Exo-pH=2.5 SGF
ChsiRNA/Exo-pH=7 SIF
Exo
Exo-pH=2.5 SGF
Exo-pH=7 SIF

# FIG. 7

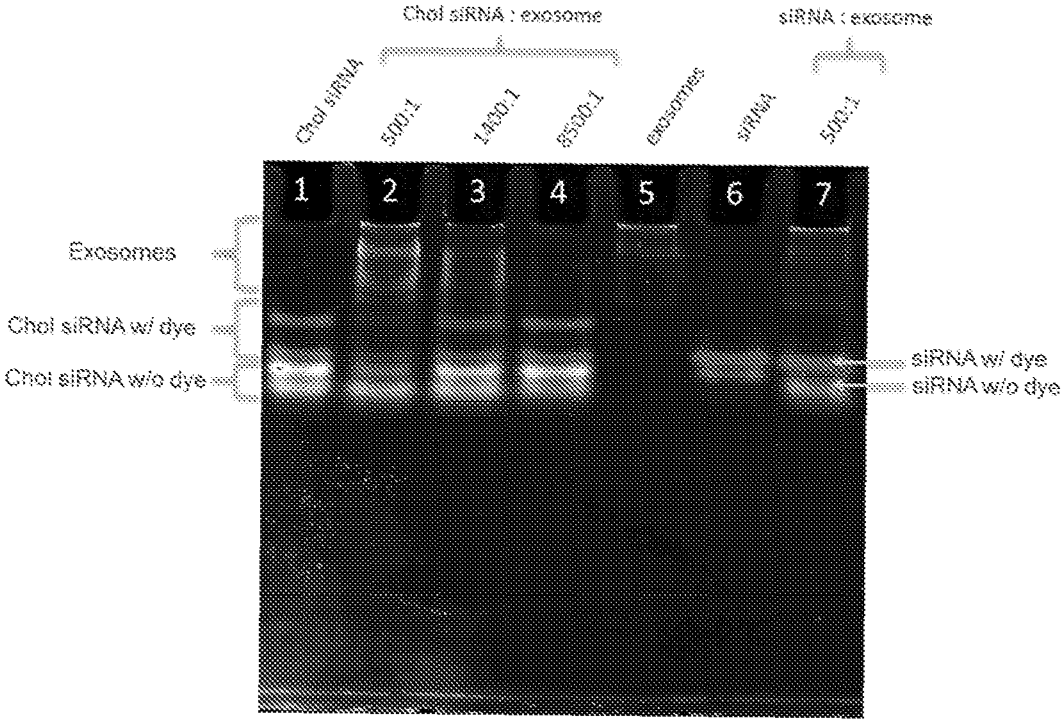


PAGE of RNA stained with SYBR Gold Nucleic Acid Stain.

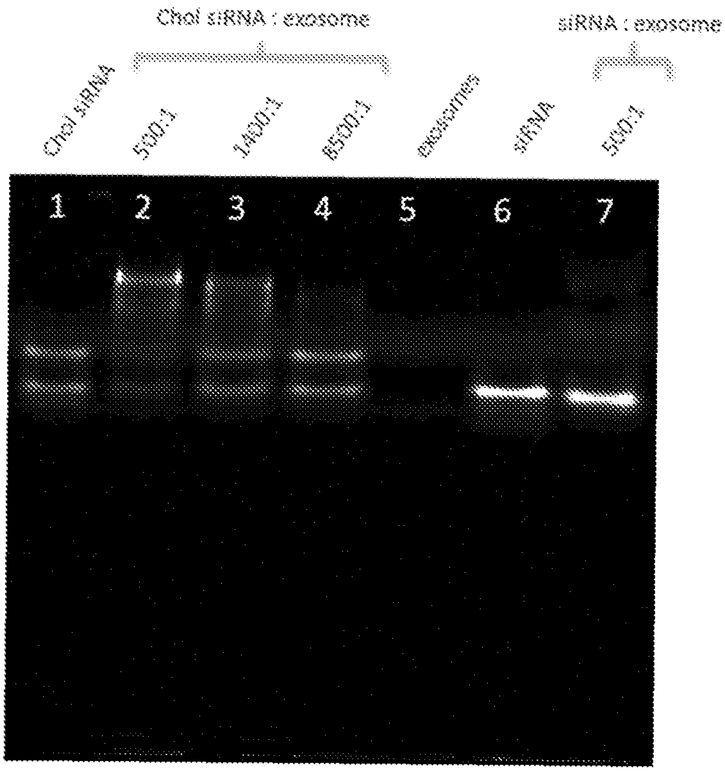


PAGE of RNA fluorophore.

FIG. 8

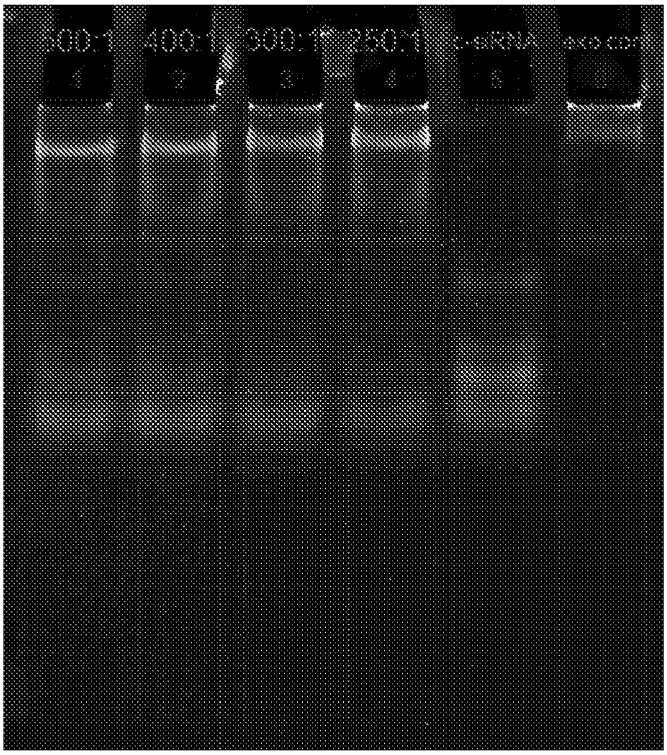


SYBR Gold Stain

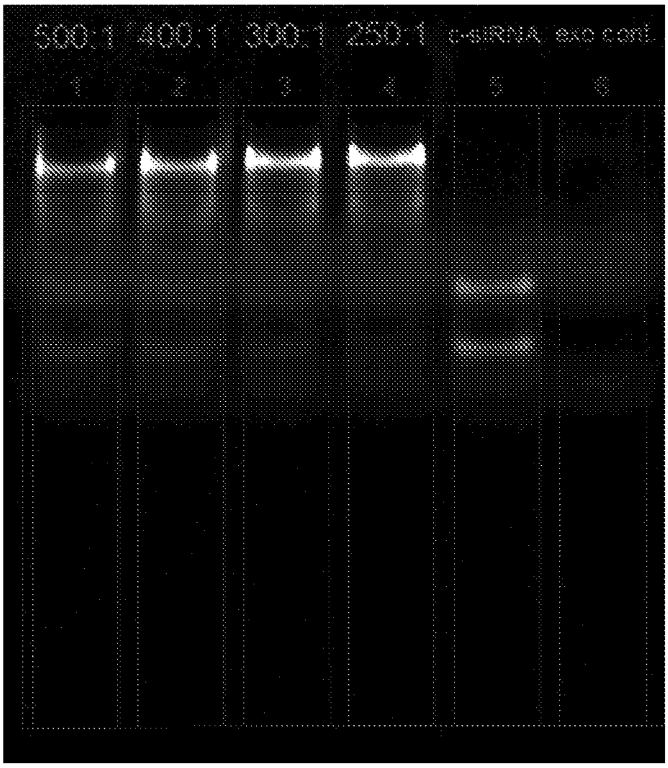


Fluorophore

FIG. 9

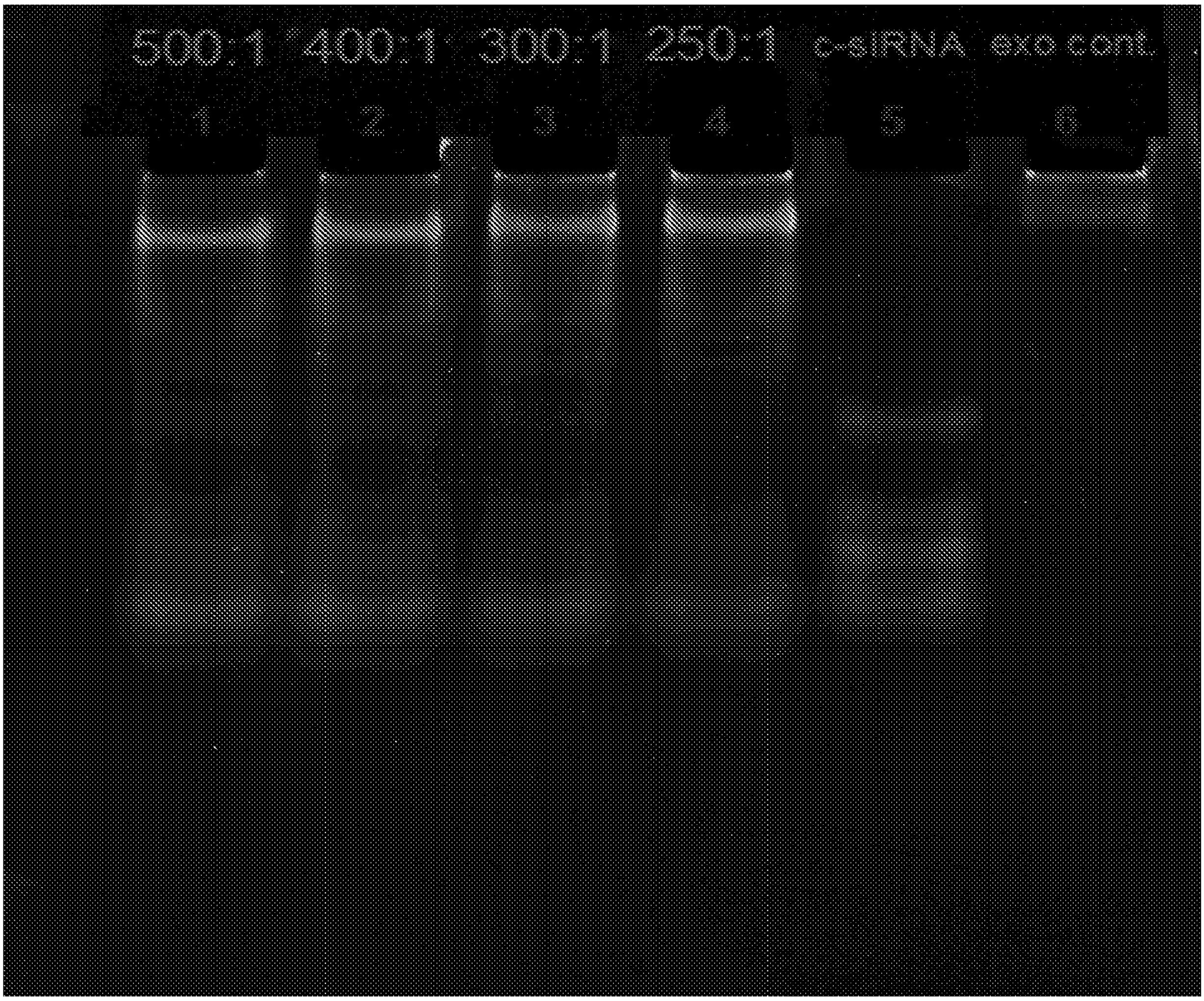

**FIG. 10**

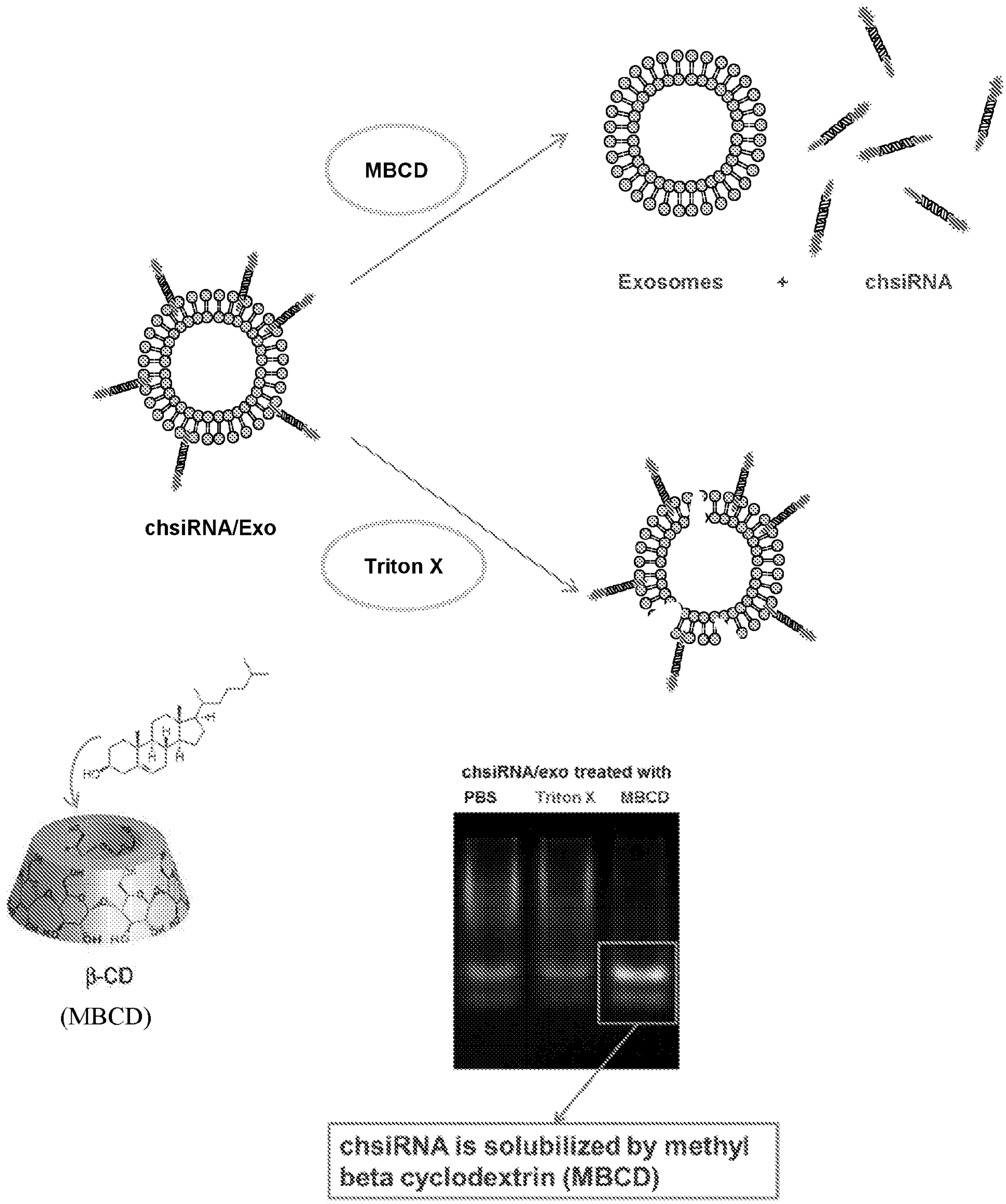
MBCD
Exosomes
+
chsiRNA
chsiRNA/Exo
Triton X
β-CD
(MBCD)
chsiRNA/exo treated with
PBS
Triton X
MBCD
chsiRNA is solubilized by methyl beta cyclodextrin (MBCD)

FIG. 11

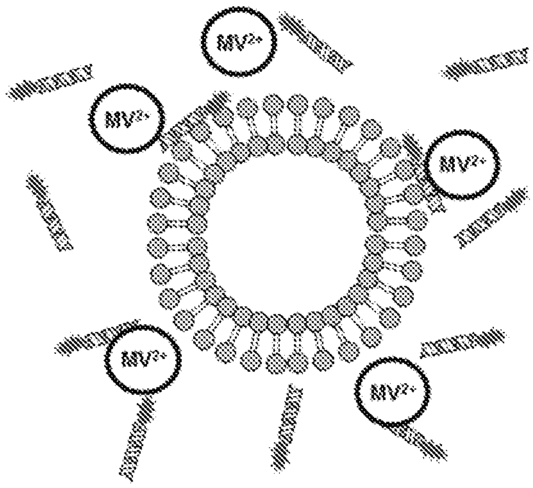


siRNA-DY677 and exosome interaction – outside

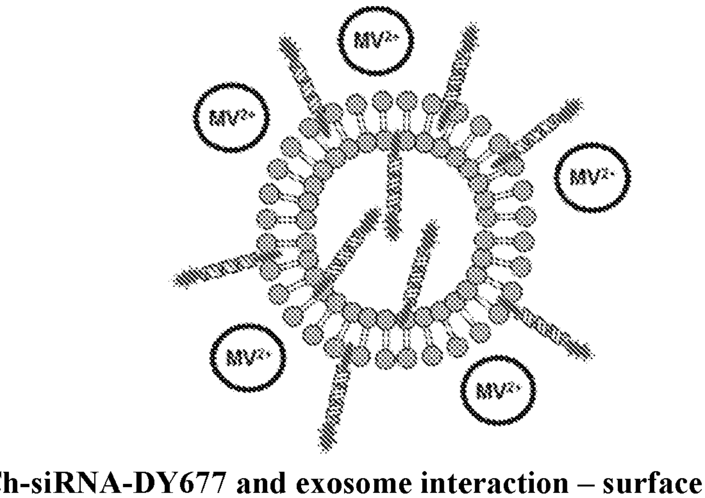


Ch-siRNA-DY677 and exosome interaction – surface + inside

FIG. 12

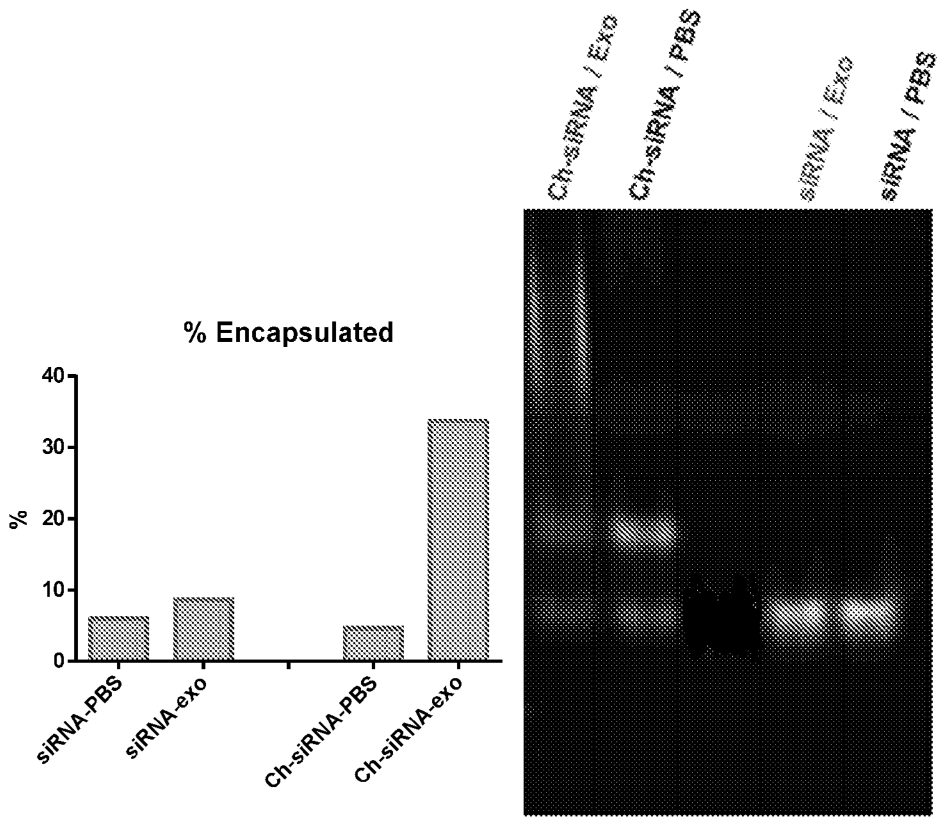


| siRNA | Encapsulation % | Ch-siRNA | Encapsulation % |
|---|---|---|---|
| PBS | 5.9 | PBS | 4.6 |
| Exosomes | 8.6 | Exosomes | 33.6 |

FIG. 13

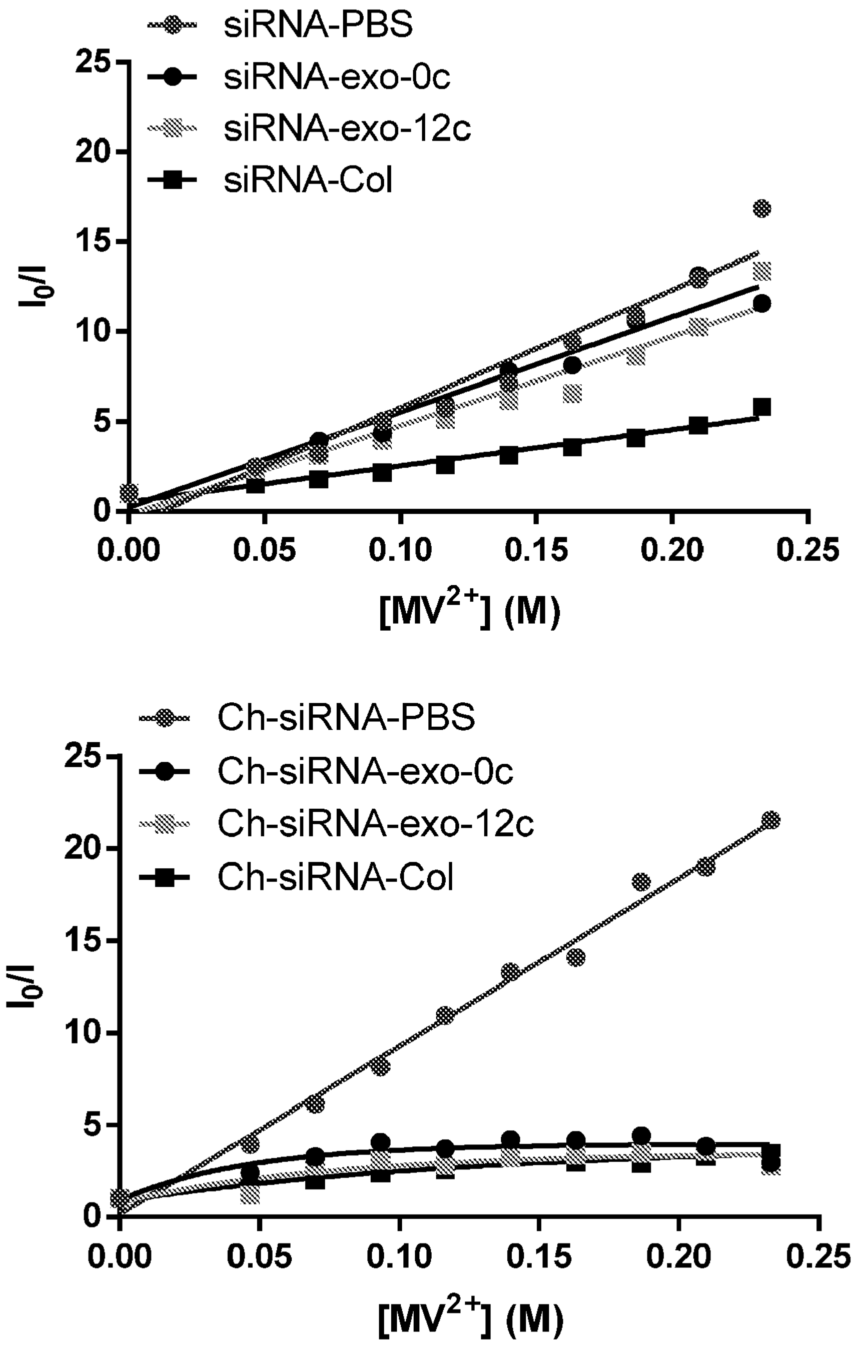
siRNA-PBS
siRNA-exo-0c
siRNA-exo-12c
siRNA-Col
$I_0/I$
$[MV^{2+}]$ (M)
0.00 0.05 0.10 0.15 0.20 0.25
0 5 10 15 20 25
Ch-siRNA-PBS
Ch-siRNA-exo-0c
Ch-siRNA-exo-12c
Ch-siRNA-Col
$I_0/I$
$[MV^{2+}]$ (M)
0.00 0.05 0.10 0.15 0.20 0.25
0 5 10 15 20 25

FIG. 14

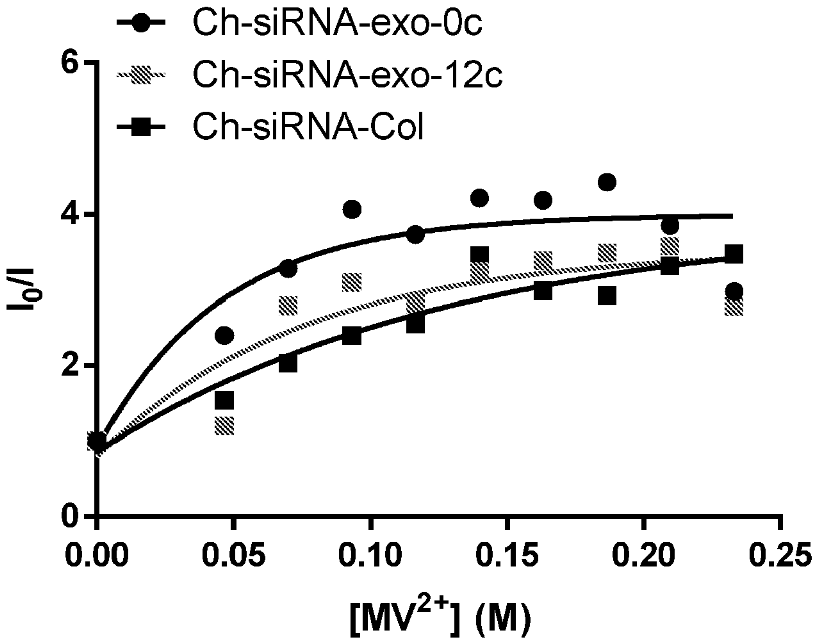
Ch-siRNA-exo-0c
Ch-siRNA-exo-12c
Ch-siRNA-Col
6
4
2
0
I0/I
0.00
0.05
0.10
0.15
0.20
0.25
[MV2+] (M)

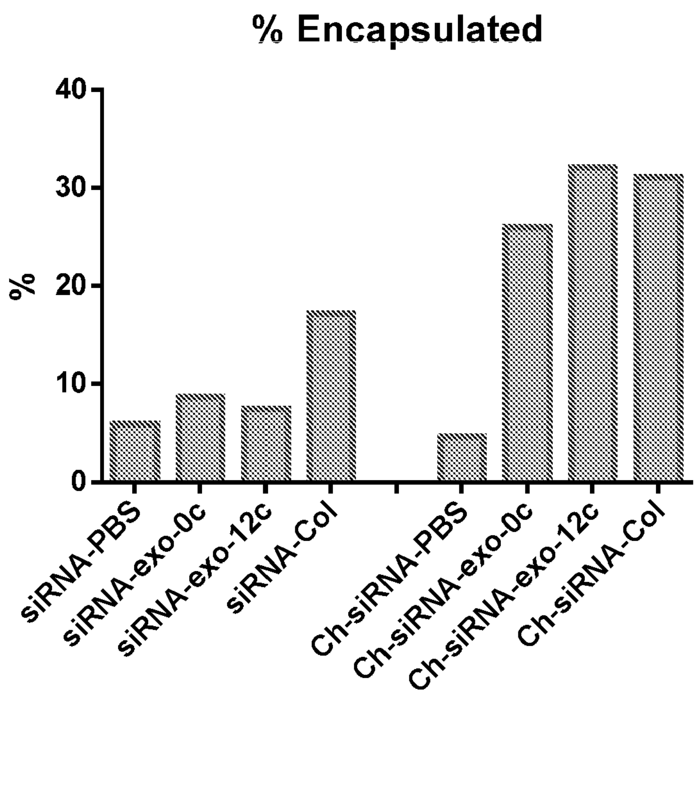
% Encapsulated
40
30
20
10
0
%
siRNA-PBS
siRNA-exo-0c
siRNA-exo-12c
siRNA-Col
Ch-siRNA-PBS
Ch-siRNA-exo-0c
Ch-siRNA-exo-12c
Ch-siRNA-Col

FIG. 15

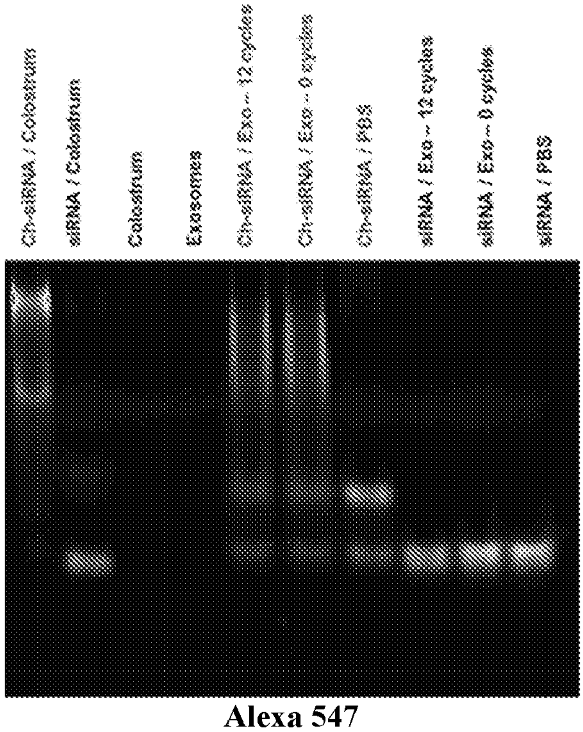


Alexa 547

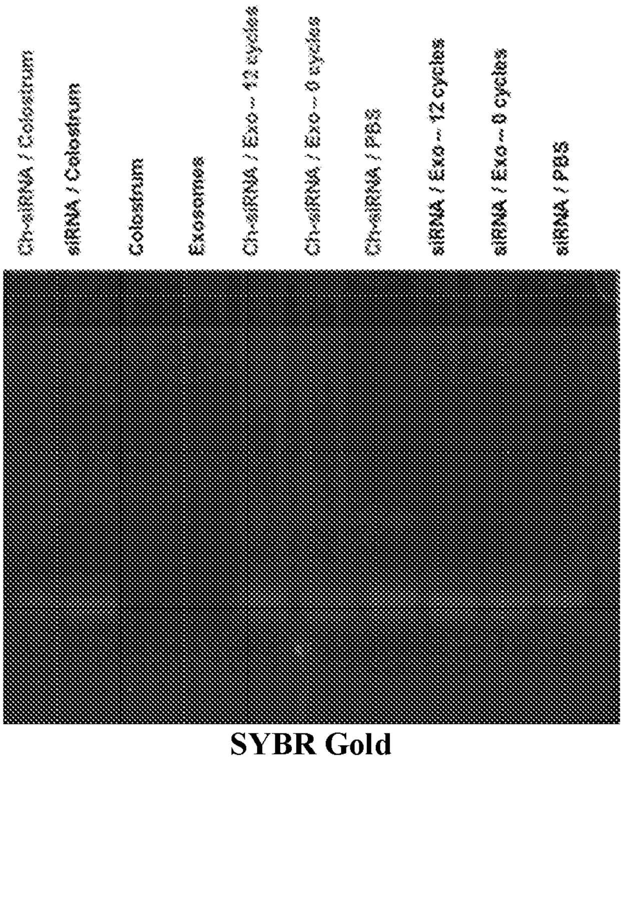


SYBR Gold

FIG. 16

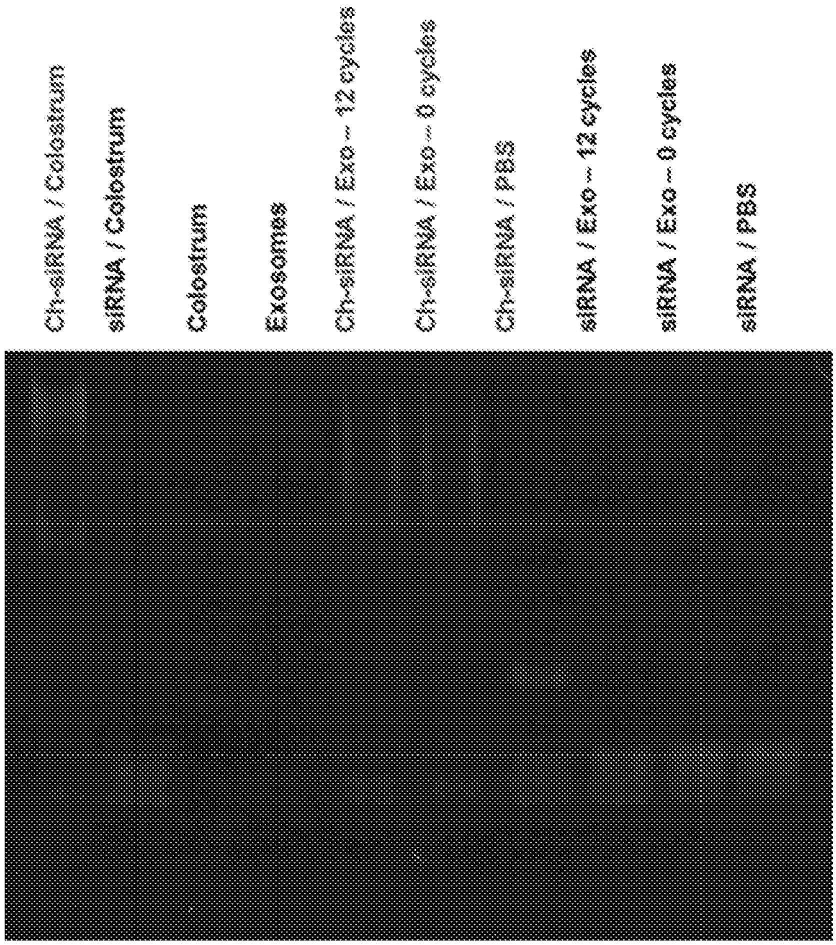


Merge of Alex 547 and SYBR Gold

FIG. 17

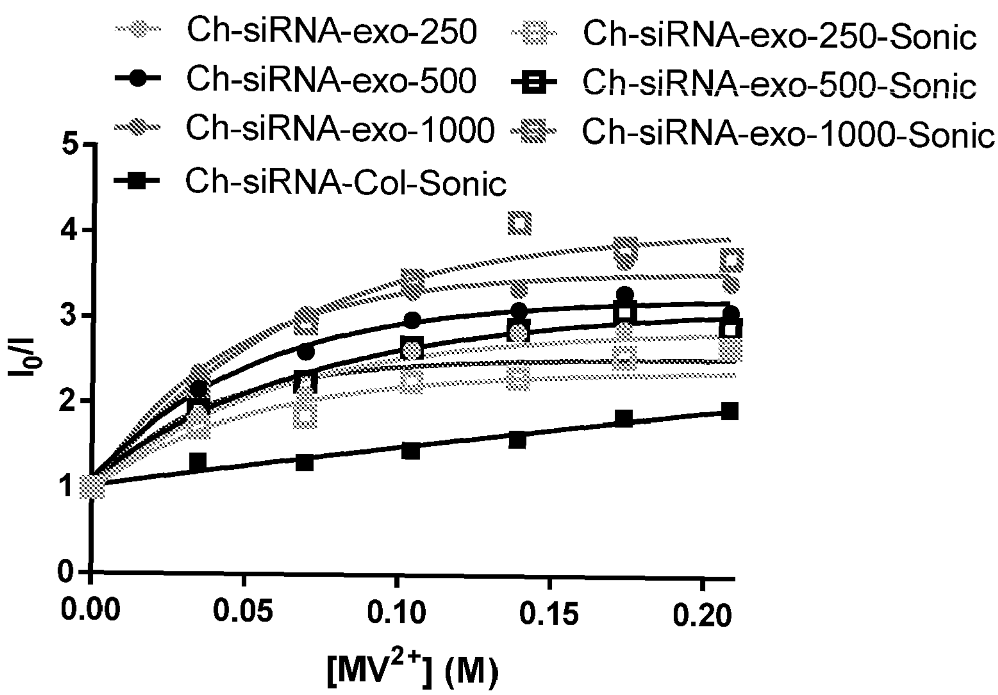
Ch-siRNA-exo-250
Ch-siRNA-exo-500
Ch-siRNA-exo-1000
Ch-siRNA-Col-Sonic
Ch-siRNA-exo-250-Sonic
Ch-siRNA-exo-500-Sonic
Ch-siRNA-exo-1000-Sonic
I0/I
0
1
2
3
4
5
0.00
0.05
0.10
0.15
0.20
[MV2+] (M)

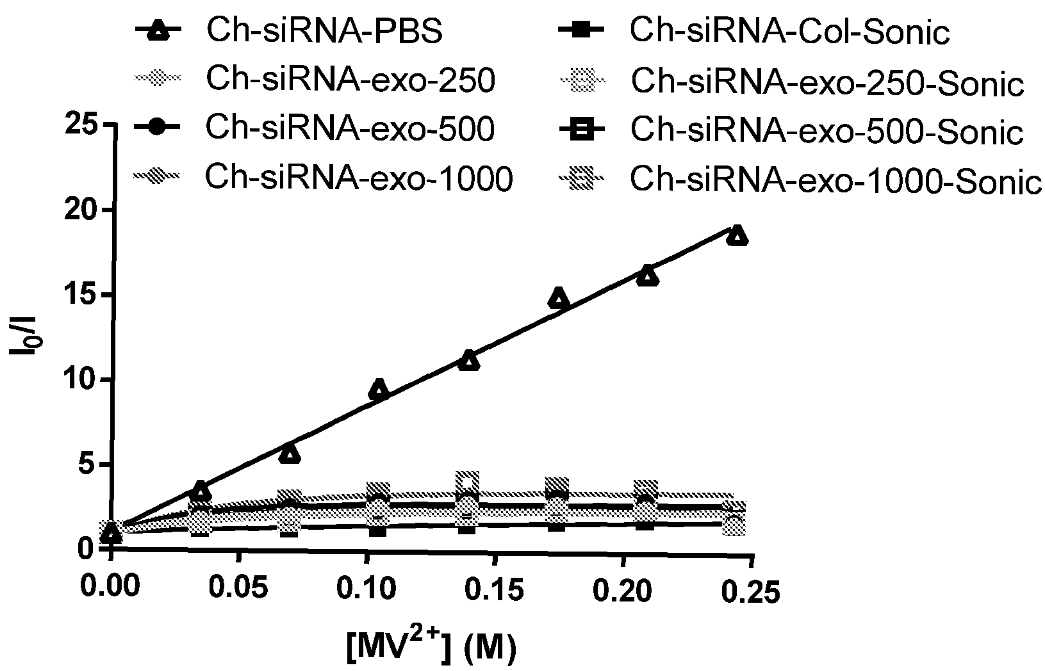
Ch-siRNA-PBS
Ch-siRNA-exo-250
Ch-siRNA-exo-500
Ch-siRNA-exo-1000
Ch-siRNA-Col-Sonic
Ch-siRNA-exo-250-Sonic
Ch-siRNA-exo-500-Sonic
Ch-siRNA-exo-1000-Sonic
I0/I
0
5
10
15
20
25
0.00
0.05
0.10
0.15
0.20
0.25
[MV2+] (M)

FIG. 18

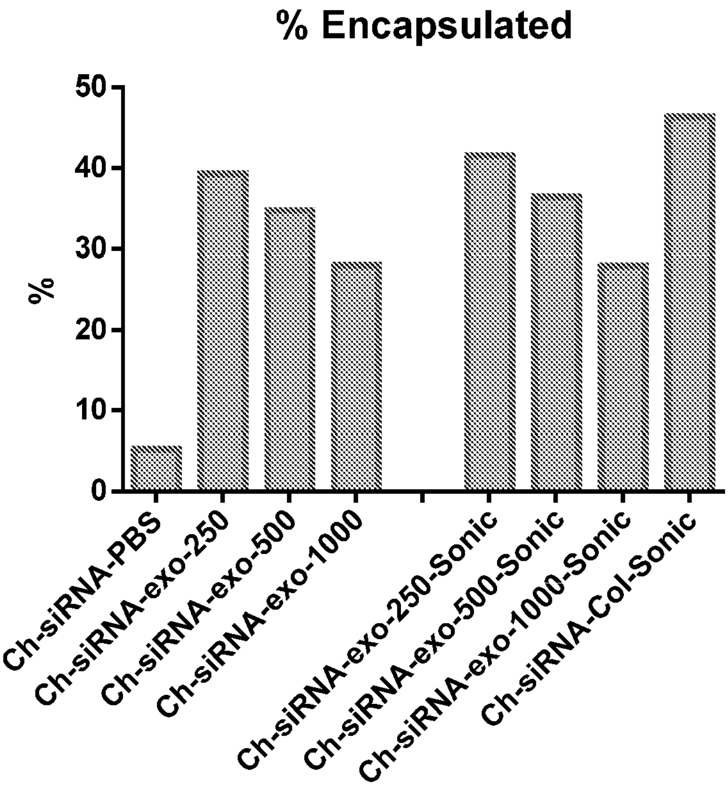
% Encapsulated
%
50
40
30
20
10
0
Ch-siRNA-PBS
Ch-siRNA-exo-250
Ch-siRNA-exo-500
Ch-siRNA-exo-1000
Ch-siRNA-exo-250-Sonic
Ch-siRNA-exo-500-Sonic
Ch-siRNA-exo-1000-Sonic
Ch-siRNA-Col-Sonic

**FIG. 19**

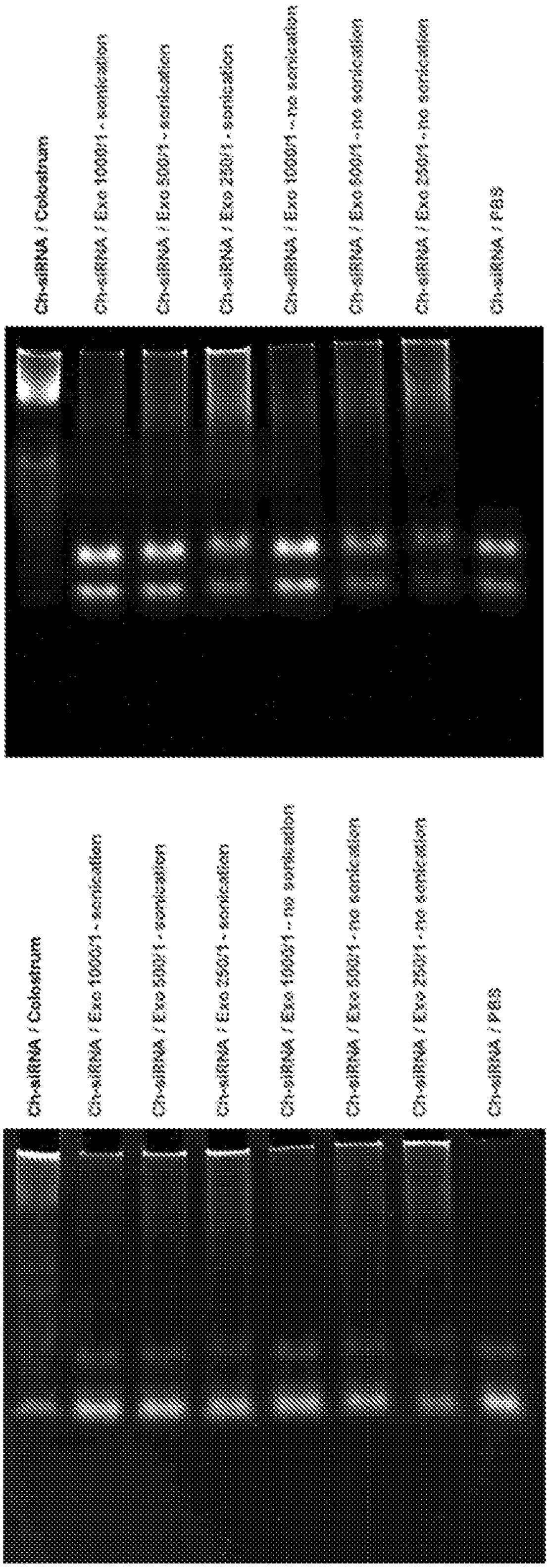
Ch-siRNA / Colostrum
Ch-siRNA / Exo 1000/1 - sonication
Ch-siRNA / Exo 500/1 - sonication
Ch-siRNA / Exo 250/1 - sonication
Ch-siRNA / Exo 1000/1 - no sonication
Ch-siRNA / Exo 500/1 - no sonication
Ch-siRNA / Exo 250/1 - no sonication
Ch-siRNA / PBS

FIG. 20

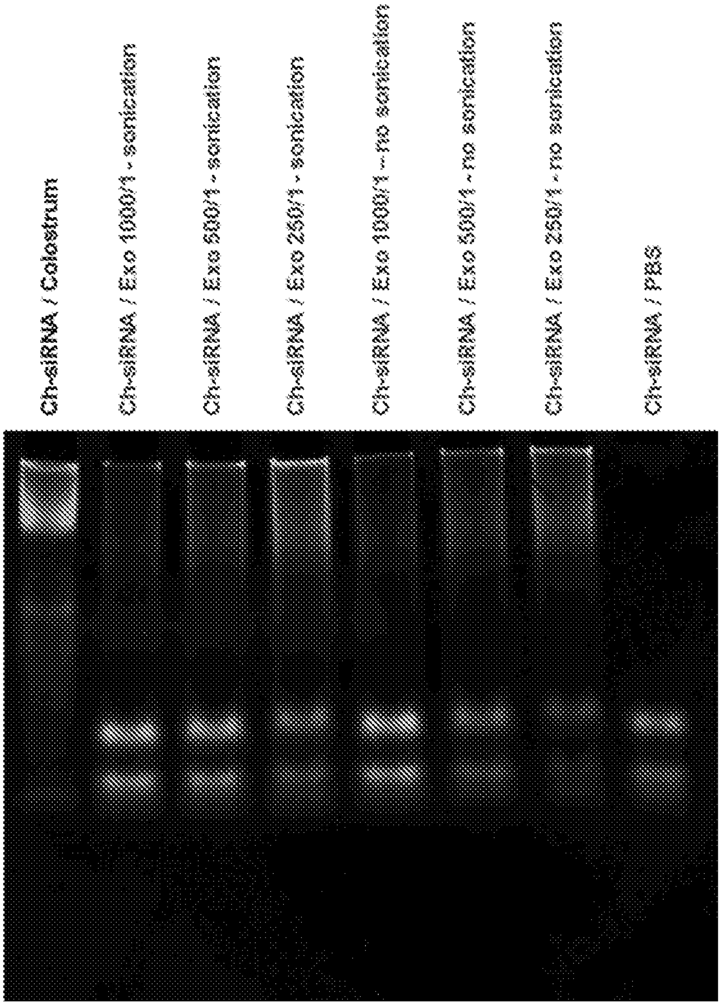
Ch-siRNA / Colostrum
Ch-siRNA / Exo 1000/1 - sonication
Ch-siRNA / Exo 500/1 - sonication
Ch-siRNA / Exo 250/1 - sonication
Ch-siRNA / Exo 1000/1 - no sonication
Ch-siRNA / Exo 500/1 - no sonication
Ch-siRNA / Exo 250/1 - no sonication
Ch-siRNA / PBS

FIG. 21

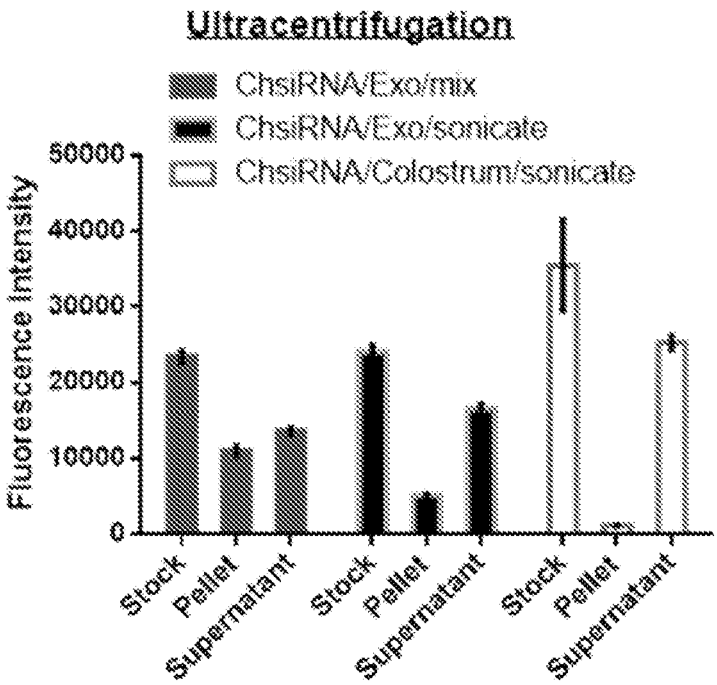
Ultracentrifugation
ChsiRNA/Exo/mix
ChsiRNA/Exo/sonicate
ChsiRNA/Colostrum/sonicate
Fluorescence Intensity
50000
40000
30000
20000
10000
0
Stock
Pellet
Supernatant
Stock
Pellet
Supernatant
Stock
Pellet
Supernatant

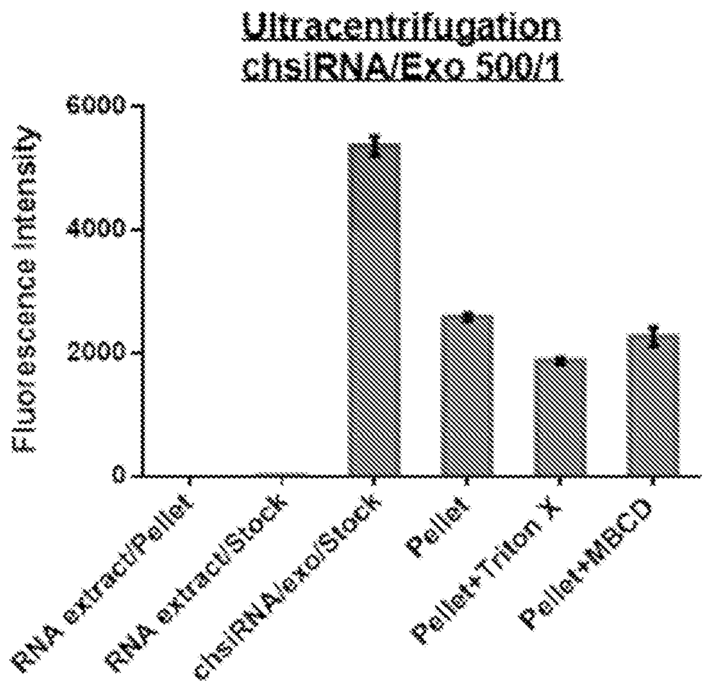
Ultracentrifugation
chsiRNA/Exo 500/1
Fluorescence Intensity
6000
4000
2000
0
RNA extract/Pellet
RNA extract/Stock
chsiRNA/exo/Stock
Pellet
Pellet+Triton X
Pellet+MBCD

FIG. 22

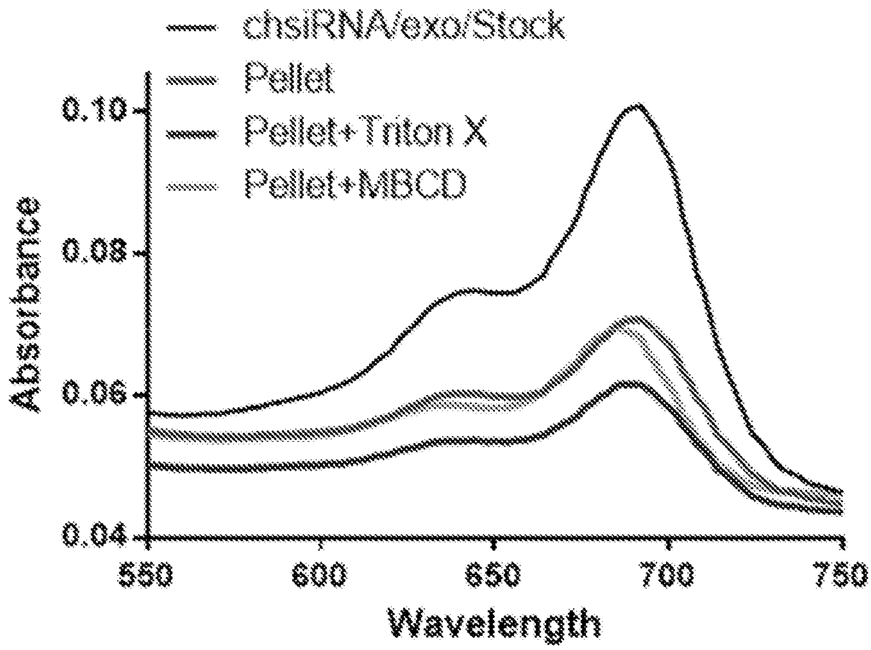
chsiRNA/exo/Stock
Pellet
Pellet+Triton X
Pellet+MBCD
0.10
0.08
0.06
0.04
Absorbance
550
600
650
700
750
Wavelength

FIG. 23

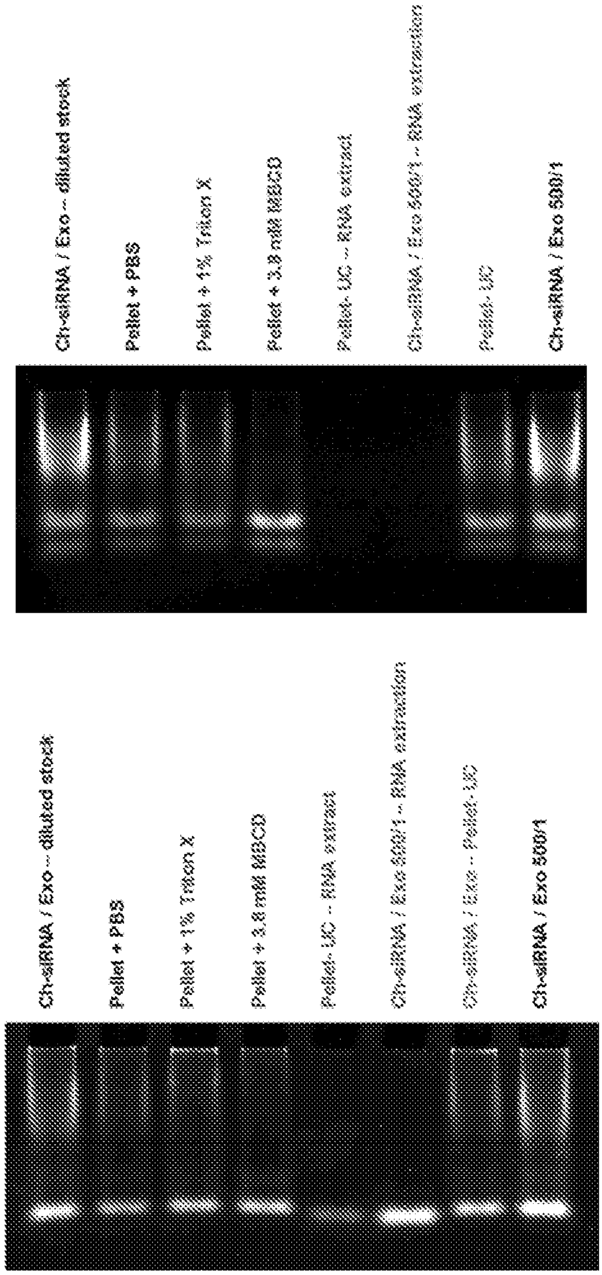
Ch-siRNA / Exo – diluted stock
Pellet + PBS
Pellet + 1% Triton X
Pellet + 3.8 mM MBCD
Pellet- UC – RNA extract
Ch-siRNA / Exo 500/1 – RNA extraction
Pellet- UC
Ch-siRNA / Exo 500/1
Ch-siRNA / Exo – diluted stock
Pellet + PBS
Pellet + 1% Triton X
Pellet + 3.8 mM MBCD
Pellet- UC – RNA extract
Ch-siRNA / Exo 500/1 – RNA extraction
Ch-siRNA / Exo – Pellet- UC
Ch-siRNA / Exo 500/1

FIG. 24

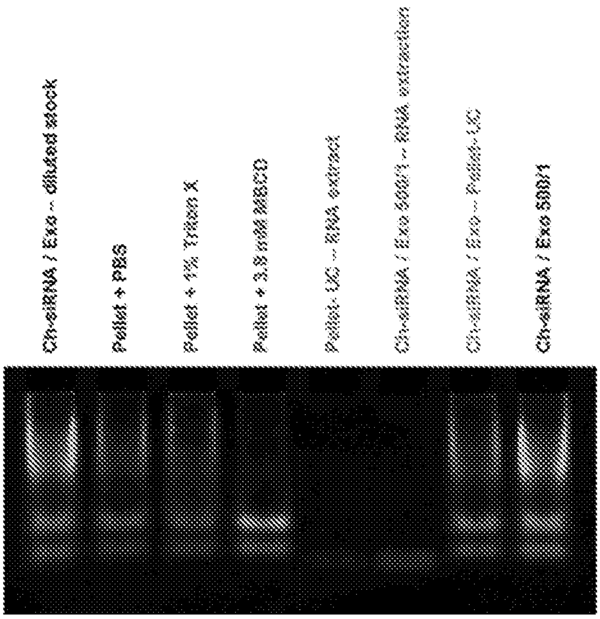
Ch-siRNA / Exo – diluted stock
Pellet + PBS
Pellet + 1% Triton X
Pellet + 3.8 mM MBCD
Pellet- UC – RNA extract
Ch-siRNA / Exo 50B/1 – RNA extraction
Ch-siRNA / Exo – Pellet- UC
Ch-siRNA / Exo 50B/1

**FIG. 25**

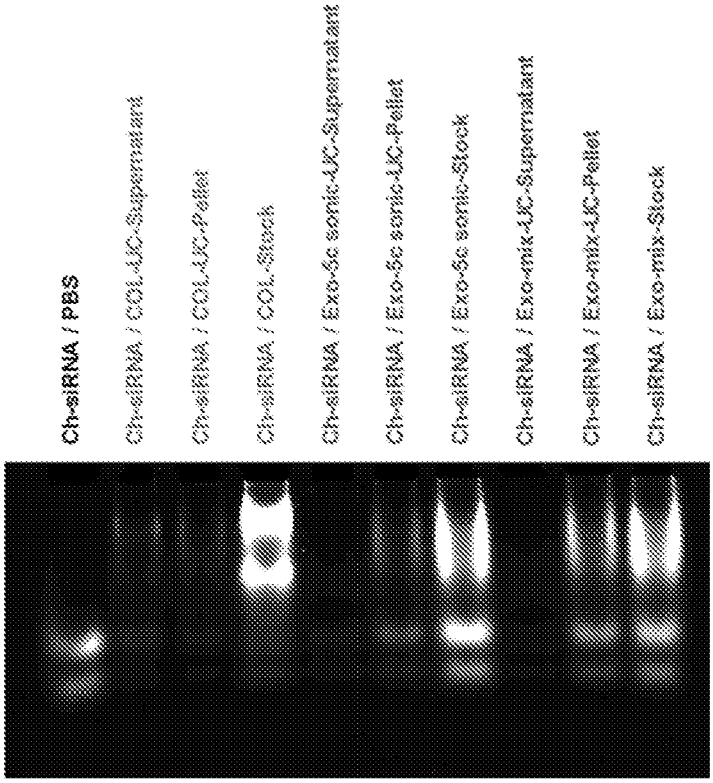
Ch-siRNA / PBS
Ch-siRNA / COL-UC-Supernatant
Ch-siRNA / COL-UC-Pellet
Ch-siRNA / COL-Stock
Ch-siRNA / Exo-5c sonic-UC-Supernatant
Ch-siRNA / Exo-5c sonic-UC-Pellet
Ch-siRNA / Exo-5c sonic-Stock
Ch-siRNA / Exo-mix-UC-Supernatant
Ch-siRNA / Exo-mix-UC-Pellet
Ch-siRNA / Exo-mix-Stock

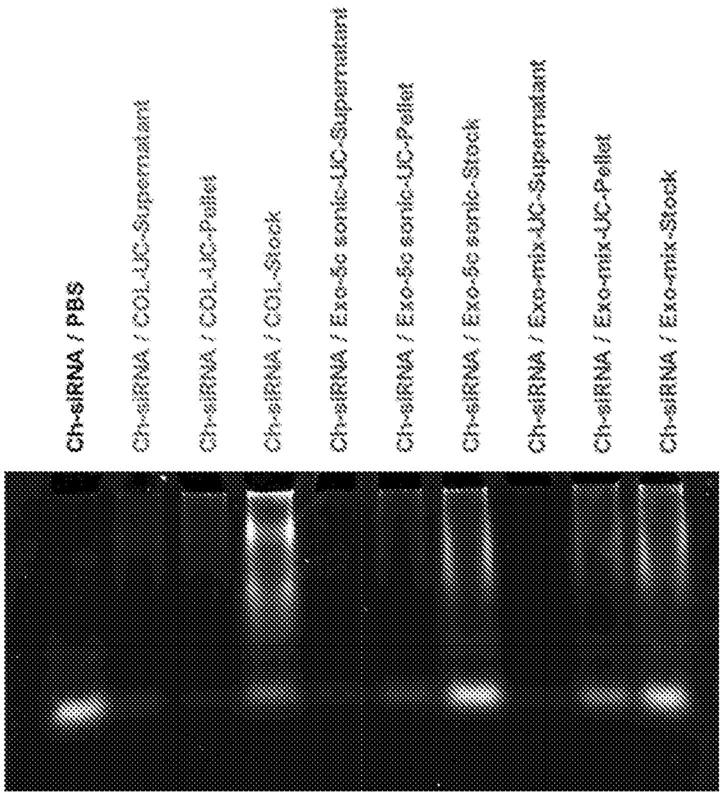
Ch-siRNA / PBS
Ch-siRNA / COL-UC-Supernatant
Ch-siRNA / COL-UC-Pellet
Ch-siRNA / COL-Stock
Ch-siRNA / Exo-5c sonic-UC-Supernatant
Ch-siRNA / Exo-5c sonic-UC-Pellet
Ch-siRNA / Exo-5c sonic-Stock
Ch-siRNA / Exo-mix-UC-Supernatant
Ch-siRNA / Exo-mix-UC-Pellet
Ch-siRNA / Exo-mix-Stock

**FIG. 26**

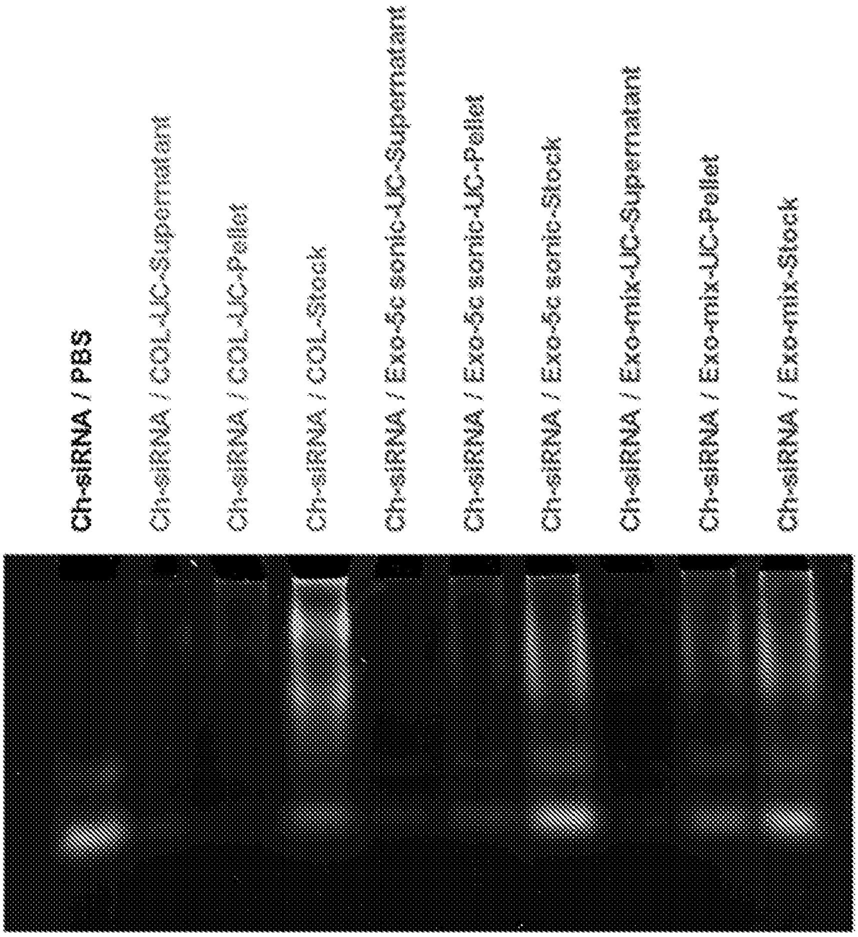
Ch-siRNA / PBS
Ch-siRNA / COL-UC-Supernatant
Ch-siRNA / COL-UC-Pellet
Ch-siRNA / COL-Stock
Ch-siRNA / Exo-5c sonic-UC-Supernatant
Ch-siRNA / Exo-5c sonic-UC-Pellet
Ch-siRNA / Exo-5c sonic-Stock
Ch-siRNA / Exo-mix-UC-Supernatant
Ch-siRNA / Exo-mix-UC-Pellet
Ch-siRNA / Exo-mix-Stock

FIG. 27A

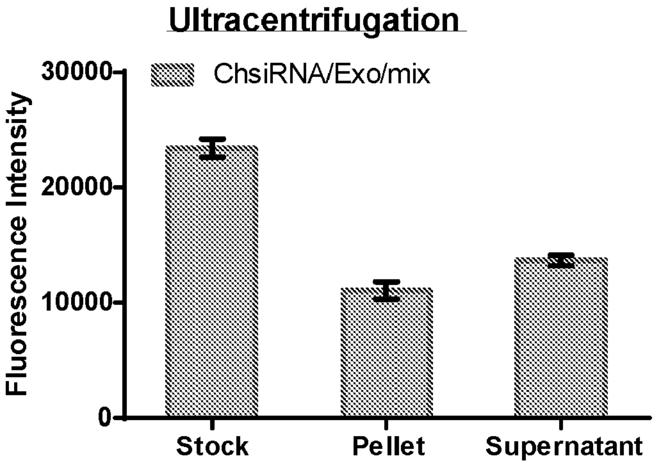


FIG. 27B

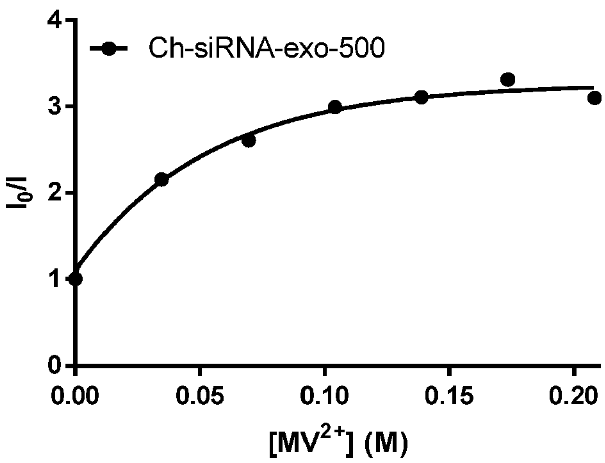


| | chsiRNA/exo 500/1 |
|---|---|
| Pellet (UC) (total loaded) | 48.1 |
| Encapsulation (inside exosome) | 34.7 |

FIG. 28A

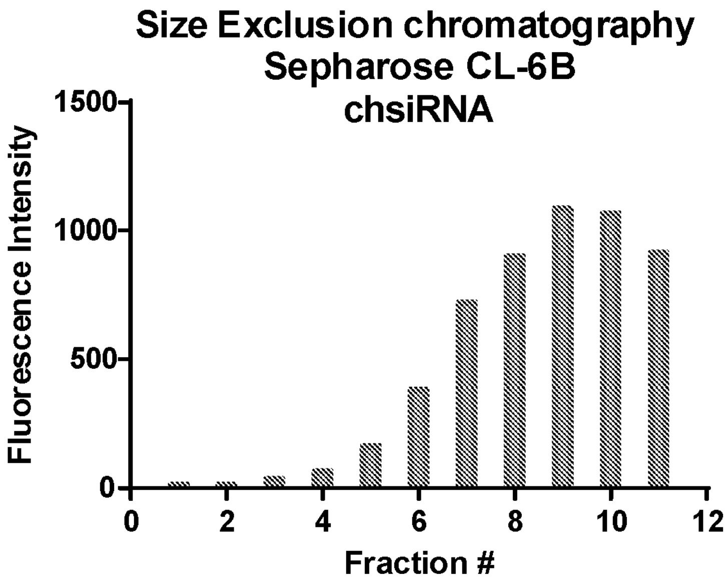


FIG. 28B

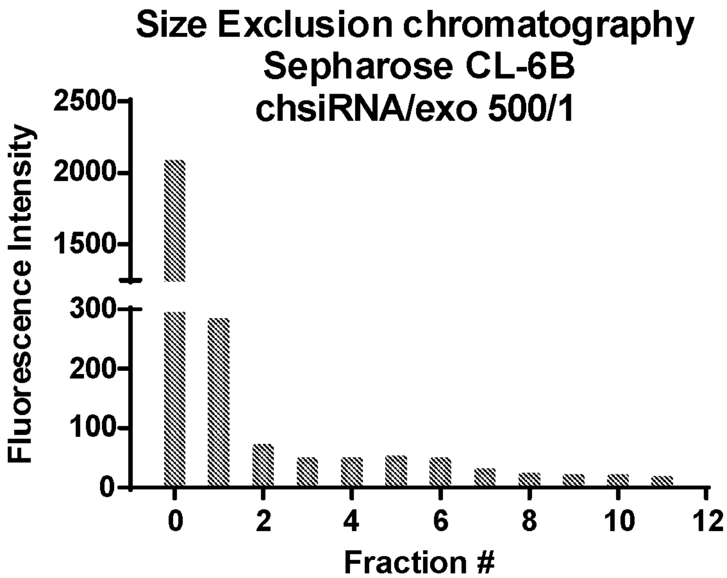

Europäisches Patentamt
European Patent Office
Office européen des brevets

# EUROPEAN SEARCH REPORT

Application Number
EP 22 15 1613

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AWWAD A. RADWAN ET AL: "Targeting cancer using cholesterol conjugates", SAUDI PHARMACEUTICAL JOURNAL, vol. 22, no. 1, 1 January 2014 (2014-01-01), pages 3-16, XP055362089, AMSTERDAM, NL ISSN: 1319-0164, DOI: 10.1016/j.jsps.2013.01.003 * page 10, right column, point 9, figure 10; page 13, right column, point 12 * | 1-12 | INV. A61K9/133 A61K9/127 A61K9/10 A61K9/00 A61K48/00 |
| X | MARIE-CÉCILE DIDIOT ET AL: "Exosome-mediated Delivery of Hydrophobically Modified siRNA for Huntingtin mRNA Silencing", MOLECULAR THERAPY, vol. 24, no. 10, 1 October 2016 (2016-10-01), pages 1836-1847, XP055395881, US ISSN: 1525-0016, DOI: 10.1038/mt.2016.126 * abstract; page 1836, right column paragraph 3; figure 1; page 1838, left column last sentence * | 1-12 | TECHNICAL FIELDS SEARCHED (IPC) A61K |
| X,P | DANIELLE IRBY ET AL: "Lipid–Drug Conjugate for Enhancing Drug Delivery", MOLECULAR PHARMACEUTICS, vol. 14, no. 5, 12 January 2017 (2017-01-12), pages 1325-1338, XP055582321, US ISSN: 1543-8384, DOI: 10.1021/acs.molpharmaceut.6b01027 * abstract * | 1-12 | |

-/--

The present search report has been drawn up for all claims

1

| Place of search | Date of completion of the search | Examiner |
|---|---|---|
| The Hague | 24 May 2022 | Konter, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 1613

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SOCHEATA LY ET AL: "Visualization of self-delivering hydrophobically modified siRNA cellular internalization", NUCLEIC ACIDS RESEARCH, vol. 45, no. 1, 28 November 2016 (2016-11-28), pages 15-25, XP055752164, GB ISSN: 0305-1048, DOI: 10.1093/nar/gkw1005 * abstract * ----- | 1-12 | |

TECHNICAL FIELDS SEARCHED (IPC)

The present search report has been drawn up for all claims

1

| Place of search | Date of completion of the search | Examiner |
|---|---|---|
| The Hague | 24 May 2022 | Konter, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62427531
- US 62559921 B
- US 62559967 B
- CA 2581651
- US 8138161 B
- EP 2264167 A
- US 9040492 B
- US 9320814 B
- US 20100298411 A
- US 7582744 B
- US 9453222 B
- US 8957223 B
- US 8017763 B
- US 8404862 B
- WO 2009073809 A
- WO 2006020768 A
- WO 2006078278 A
- WO 2015042564 A
- WO 2011056883 A
- WO 2016033326 A
- WO 2010048228 A
- WO 2011123468 A
- WO 2014022739 A
- WO 2013163430 A
- WO 2015175510 A
- WO 2012177949 A
- WO 2014160129 A
- WO 2004080406 A
- WO 2009082607 A
- WO 2004091515 A
- WO 2013155204 A
- WO 2016061487 A
- WO 2008131419 A
- WO 2016057893 A
- WO 2016085852 A
- WO 2012135246 A
- WO 2008036933 A
- WO 2013013017 A
- WO 2013013019 A
- WO 2012178033 A
- WO 2014190137 A
- WO 2012058693 A
- WO 2011038031 A
- WO 2011028938 A
- WO 2010148013 A
- WO 2011053994 A
- WO 2007134161 A
- WO 2009134487 A
- WO 2015123264 A
- WO 2011029016 A
- WO 2009129465 A
- WO 2009111658 A
- US 20140148497 A
- WO 2000044895 A
- US 8273868 B
- US 9352048 B
- US 20150119445 A
- US 8350021 B
- EP 1833967 A
- EP 2316942 A
- US 20120028348 A
- US 7985581 B
- EP 2169072 A
- EP 1784492 A
- US 20160122759 A
- EP 2994167 A
- WO 2014182661 A
- US 20140275211 A
- EP 2723865 A
- WO 2012177906 A
- EP 1171586 A
- EP 1597351 A
- WO 2016077321 A
- WO 2016077349 A
- US 9198927 B
- US 20100267805 A
- EP 2325314 A
- EP 1797185 A
- EP 2772541 A
- US 20150315593 A
- US 8987227 B
- US 8614198 B
- US 20140141512 A
- US 20100324117 A
- EP 2173900 A
- US 20120046478 A
- EP 2068886 A
- WO 2008042973 A
- EP 2492282 A
- EP 1664267 A
- EP 3017044 A
- WO 2016077837 A
- EP 3058069 A
- US 20160237432 A
- US 20160251655 A
- EP 3055414 A

- EP 2906697 A
- EP 2906696 A
- EP 2742056 A
- EP 2534248 A
- EP 2270024 A
- WO 2016112132 A
- EP 3058068 A
- US 20160230172 A
- EP 2527442 A
- EP 2021472 A
- EP 2458006 A
- EP 2363482 A
- EP 2363481 A
- EP 2951304 A
- EP 2943225 A
- EP 2906258 A
- EP 2906256 A
- EP 2906255 A
- EP 2742136 A
- EP 2742135 A
- WO 2016044840 A
- WO 2016040748 A
- EP 2991661 A
- EP 2906225 A
- EP 2906226 A
- EP 2794880 A
- EP 2253706 A
- WO 2016061263 A
- EP 2595664 A
- EP 2920308 A
- EP 2697243 A
- EP 2864479 A
- EP 2751269 A
- EP 2812342 A
- EP 2379084 A
- US 9322021 B
- EP 2726153 A
- EP 3038627 A
- EP 3000884 A
- EP 2227545 A
- EP 2812433 A
- EP 2015758 A
- EP 2991656 A
- EP 2956176 A
- EP 2701713 A
- EP 2521556 A
- EP 2408796 A
- WO 2016077704 A
- US 9404114 B
- EP 2758533 A
- US 9404113 B
- EP 2697244 A
- US 20160194349 A
- US 20160152974 A
- EP 3011026 A
- EP 2215102 A
- EP 1670896 A
- EP 2505649 A
- EP 2505648 A
- EP 2505647 A
- EP 3011028 A
- EP 2992097 A
- EP 2992009 A
- EP 2855500 A
- EP 2771463 A
- EP 2721156 A
- EP 2363480 A
- WO 2016138355 A
- US 20150252367 A
- US 20140256797 A
- US 20110237646 A
- EP 2323667 A
- WO 2010017509 A
- US 20160076030 A
- US 9181549 B
- US 9145558 B
- US 9127276 B
- US 20160017323 A
- US 20150176007 A
- US 20150126718 A
- US 20140343123 A
- WO 2014179627 A
- WO 2014179620 A
- US 9061044 B
- US 8697860 B
- EP 2563920 A
- WO 2011139917 A
- WO 2015179693 A
- WO 2015188194 A
- US 9382540 B
- US 20150315594 A
- WO 2015168589 A
- US 20160090595 A
- US 9163239 B
- US 20150126719 A
- WO 2014179626 A
- WO 2014205449 A
- US 20150376614 A
- WO 2014127268 A
- WO 2015100394 A
- EP 1799859 A
- US 7919472 B
- US 20110207797 A
- EP 2397563 A
- WO 2006034348 A
- US 20090326040 A

- EP 1786472 A
- WO 2006020676 A
- WO 2016033424 A
- WO 2008118883 A
- US 20160060625 A
- US 9157082 B
- US 20140128453 A
- WO 2012149495 A
- US 20150344879 A
- US 9045754 B
- US 8969316 B
- US 8673871 B
- US 8586554 B
- US 8372967 B
- US 8362232 B
- US 8188059 B
- US 8143230 B
- US 20150057329 A
- US 20130165496 A
- US 20120208864 A
- US 20110065775 A
- US 20090318532 A
- US 20090326042 A
- US 20090326041 A
- US 20090306180 A
- US 20090306179 A
- EP 2019692 A
- EP 2023939 A
- EP 2023940 A
- EP 2505650 A
- EP 2505646 A
- EP 2397551 A
- WO 2007134014 A
- WO 2007131237 A
- WO 2007146511 A
- WO 2007143317 A
- WO 2007136988 A
- WO 2007131238 A
- WO 2007136989 A
- US 20150167005 A
- US 8912160 B
- US 8664190 B
- US 8093222 B
- US 8084437 B
- US 20140194492 A
- US 20120077865 A
- US 20100144834 A
- EP 2455471 A
- EP 2453016 A
- EP 2102340 A
- WO 2009148605 A
- WO 2008066776 A
- US 8541388 B
- US 20110123521 A
- EP 2291200 A
- WO 2009143390 A
- US 20120214736 A
- US 8101585 B
- EP 2057284 A
- WO 2008017081 A
- US 7803930 B
- US 20050009088 A
- EP 1569695 A
- WO 2004044181 A
- WO 2003097662 A
- US 20050181376 A
- US 6767739 B
- WO 2003018600 A
- US 20160090598 A
- US 20120270929 A
- EP 2480667 A
- WO 2011038288 A
- US 20030232442 A
- US 20030105042 A
- WO 2003040321 A
- EP 2294213 A
- WO 2009143463 A
- WO 2009143391 A
- WO 2015164693 A
- WO 2015061246 A
- WO 2010080953 A
- US 9315811 B
- EP 2717923 A
- US 9428750 B
- US 9409934 B
- US 9403865 B
- EP 2885312 A
- US 9399774 B
- US 20160237434 A
- US 9365848 B
- EP 2441449 A
- EP 3002007 A
- EP 2428227 A
- US 9353372 B
- EP 2161038 A
- EP 2422819 A
- WO 2007089584 A
- US 9353371 B
- US 20160186185 A
- US 9321799 B
- EP 2601204 A
- US 9340784 B
- US 9273315 B
- US 8906873 B
- EP 2475675 A
- WO 2011032045 A
- US 9290534 B
- US 20150292015 A
- US 9006198 B
- US 20130046008 A
- EP 2534262 A
- WO 2011097644 A
- US 8957040 B
- US 20130046007 A
- WO 2011097643 A
- US 20160244477 A

- US 20160222389 A
- EP 3043827 A
- US 20160194638 A
- US 20160194637 A
- US 20160186175 A
- US 20160186174 A
- US 20160145617 A
- EP 3022217 A
- US 20160159846 A
- EP 3027617 A
- US 20150376625 A
- WO 2014121287 A
- US 20150275208 A
- WO 2014059356 A
- US 20130059902 A
- EP 2536738 A
- WO 2011097641 A
- US 20110269818 A
- WO 2010019270 A
- EP 2625186 A
- EP 1937312 A
- EP 2606057 A
- WO 2013033223 A
- EP 2580228 A
- EP 2125852 A
- EP 2673361 A
- WO 2013022990 A
- WO 2013022984 A
- EP 1730309 A
- EP 2331141 A
- EP 2173358 A
- EP 2462153 A
- EP 1984499 A
- EP 2548560 A
- EP 2644700 A
- EP 2365094 A
- EP 2246443 A
- EP 2957568 A
- EP 1560840 A
- EP 2361923 A
- EP 2410053 A
- EP 2092065 A
- EP 2410054 A
- EP 2332951 A
- EP 2951191 A
- EP 3030658 A
- EP 3031920 A
- EP 2595663 A
- WO 2012012467 A
- EP 2906699 A
- EP 2852606 A
- EP 2992098 A
- EP 2877579 A
- EP 2971142 A
- EP 2850092 A
- EP 2831232 A
- EP 2839006 A
- EP 2802674 A
- EP 2776564 A
- EP 2751270 A
- EP 2699583 A
- EP 2640853 A
- EP 2582397 A
- EP 2442816 A
- EP 2447274 A
- EP 2358397 A
- EP 2399588 A
- EP 2360166 A
- EP 2327709 A
- EP 2334319 A
- EP 2282744 A
- EP 2272958 A
- EP 2222851 A
- EP 2219680 A
- EP 1957507 A
- EP 1827459 A
- EP 1427289 A
- EP 1159282 A
- WO 2016138353 A
- WO 2016138017 A
- WO 2016137923 A
- WO 2016115490 A
- WO 2016077540 A
- WO 2016086104 A
- WO 2016044828 A
- WO 2015168532 A
- WO 2011097614 A
- WO 2011031998 A
- US 9057066 B
- US 8952145 B
- US 8415465 B
- US 7951934 B
- US 20160053256 A
- US 20150307877 A
- US 20130281684 A
- US 20130189782 A
- US 20110306652 A
- US 20100069472 A
- EP 1991677 A
- WO 2007089611 A
- US 20150159155 A
- US 20150329859 A
- US 8669102 B
- US 6969763 B
- EP 1248791 A
- US 6303374 B
- WO 2001053310 A
- WO 2002020840 A
- US 6258601 B
- US 20160138014 A
- WO 2014059341 A
- WO 2015017675 A
- US 20150051389 A
- US 20140323707 A
- US 20140309279 A
- US 20140303235 A

- WO 2013022967 A
- WO 2013022966 A
- US 20150018540 A
- WO 2013033230 A
- US 20140316121 A
- US 20130225659 A
- EP 1083980 A
- US 20050239737 A
- WO 2004043394 A
- US 20040092465 A
- WO 2004048522 A
- US 20040102394 A
- US 20040096834 A
- EP 1436430 A
- US 20030087854 A
- WO 2003023004 A
- WO 2015168172 A
- WO 2014036301 A
- US 8980853 B
- US 20160002627 A
- WO 2014110291 A
- US 20140357558 A
- WO 2012178146 A
- US 20130109091 A
- EP 1910395 A
- WO 2007002390 A
- WO 2005001031 A
- EP 1631659 A
- WO 2015161170 A
- WO 2010120820 A
- WO 2010148249 A
- US 20150353929 A
- US 8946183 B
- US 8361977 B
- US 20100216238 A
- US 8409856 B
- US 7759479 B
- US 20110105586 A
- US 7709453 B
- US 20150284725 A
- WO 2014059364 A
- US 20150275205 A
- EP 2831231 A
- WO 2013148260 A
- WO 2013148283 A
- US 20150025231 A
- WO 2013106770 A
- US 20040102403 A
- US 20030220273 A
- US 8946178 B
- US 8084432 B
- US 20120270920 A
- US 20040162259 A
- WO 2004071453 A
- US 20090275631 A
- WO 2006060649 A
- US 7238662 B
- US 20150361432 A
- US 9139833 B
- US 20150376621 A
- US 9084808 B
- EP 2726613 A
- US 20130005793 A
- WO 2013003520 A
- EP 2651420 A
- WO 2012083185 A
- US 9061031 B
- US 8858928 B
- US 20150064150 A
- US 20150037296 A
- US 20130004471 A
- EP 2717922 A
- WO 2012170911 A
- US 20150216903 A
- WO 2014026110 A
- US 20140234278 A
- EP 2760994 A
- WO 2013049615 A
- US 20140199279 A
- EP 2661489 A
- WO 2012094193 A
- WO 2014015318 A
- WO 2012170431 A
- US 20160010089 A
- EP 2961843 A
- WO 2016094304 A
- WO 2016014789 A
- WO 2015188119 A
- WO 2015164739 A
- WO 2015164759 A
- EP 3027204 A
- US 20150266973 A
- WO 2015017214 A
- WO 2015164745 A
- US 20160022839 A
- US 9068199 B
- US 7901671 B
- US 20120009161 A
- US 20150203868 A
- WO 2013043196 A
- EP 2948165 A
- US 20140213520 A
- US 20140213519 A
- WO 2014115033 A
- US 9358271 B
- US 9023794 B
- US 20150224174 A
- US 20140287999 A
- EP 2510942 A
- EP 2547356 A
- US 9314535 B
- US 8703906 B
- US 20140294877 A
- US 20120219573 A
- US 20110053829 A

- EP 2810661 A
- EP 2331138 A
- WO 2011026641 A
- US 9226959 B
- US 20120021043 A
- EP 2548960 A
- EP 2176408 A
- WO 2009095226 A
- EP 3035955 A
- US 20160168227 A
- WO 2015024666 A
- EP 3035954 A
- US 20160166668 A
- WO 2015024664 A
- US 20160166691 A
- US 20160136263 A
- US 20150093413 A
- EP 2814962 A
- WO 2013120628 A
- WO 2013120499 A
- EP 2678038 A
- US 20130259879 A
- WO 2012113513 A
- WO 2012113413 A
- US 20130251742 A
- EP 2195015 A
- WO 2009046975 A
- US 20130202645 A
- EP 2197481 A
- WO 2009046974 A
- EP 2762165 A
- US 20110250225 A
- EP 2331129 A
- WO 2010037539 A
- WO 2010037408 A
- WO 2011144358 A
- EP 2387999 A
- WO 2011069587 A
- WO 2011069528 A
- WO 2011069529 A
- WO 2010088927 A
- WO 2003059381 A
- US 9447431 B
- US 9421255 B
- US 9439956 B
- US 9433670 B
- US 9433669 B
- US 9155788 B
- US 8217016 B
- US 20160095911 A
- US 20160089426 A
- US 20160095912 A
- US 20160089425 A
- US 20160089424 A
- US 20160082092 A
- US 20150030633 A
- US 20110311472 A
- EP 1458410 A
- EP 2769733 A
- EP 1925317 A
- EP 1905844 A
- US 9402887 B
- US 9352028 B
- US 20160206756 A
- US 9234013 B
- EP 2603590 A
- EP 2796557 A
- WO 2012019780 A
- WO 2012019630 A
- US 20160250321 A
- EP 2955230 A
- US 8968746 B
- US 20150258214 A
- US 20130142818 A
- EP 2449113 A
- WO 2012013326 A
- US 8383340 B
- EP 2092064 A
- WO 2008077592 A
- US 20160206719 A
- US 20160130345 A
- EP 2958588 A

- WO 2014127917 A
- US 20160185840 A
- US 20160168254 A
- US 20160166692 A
- US 20160166690 A
- US 20160152706 A
- US 20160152691 A
- US 20160145346 A
- US 20130195867 A
- EP 2101823 A
- WO 2008083949 A
- US 20160184406 A
- US 20140037660 A
- US 20100203076 A
- EP 2484770 A
- EP 2188379 A
- WO 2009030481 A
- WO 2009030254 A
- EP 3035960 A
- US 20160168207 A
- WO 2015024668 A
- EP 3036330 A
- US 20160166710 A
- WO 2015024667 A
- EP 3035961 A
- US 20160166711 A
- WO 2015024665 A
- EP 3035959 A
- US 20160166678 A
- WO 2015024669 A
- US 20160151474 A
- US 20130295043 A
- EP 2680881 A
- WO 2012116811 A
- WO 2012116714 A
- US 20160136301 A
- US 20160136259 A
- US 20160136258 A
- US 20160136247 A
- US 20160136243 A
- US 20160129105 A
- US 20150104476 A
- US 20110269950 A
- US 20110077287 A
- US 20100239608 A
- EP 2305699 A
- EP 1857122 A
- EP 1800697 A
- EP 1832603 A
- EP 1604688 A
- EP 1392341 A
- EP 2842964 A
- EP 1903054 A
- US 20150320847 A
- EP 2814961 A
- WO 2013120627 A
- WO 2013120500 A
- US 20150306249 A
- EP 2854857 A
- WO 2013174409 A
- US 20150218554 A
- EP 2831241 A
- WO 2013143699 A
- US 20150184195 A
- EP 2831239 A
- WO 2013143698 A
- US 20150165006 A
- EP 2814964 A
- WO 2013120626 A
- WO 2013120498 A
- US 20150118264 A
- EP 2809353 A
- WO 2013113501 A
- WO 2013113326 A
- US 20150118183 A
- EP 2809354 A
- WO 2013113502 A
- WO 2013113325 A
- US 20150141498 A
- EP 2510100 A

- WO 2011069586 A
- US 20150057340 A
- EP 2814963 A
- WO 2013120629 A
- WO 2013120497 A
- US 20150050302 A
- EP 2831240 A
- WO 2013143700 A
- US 20150037326 A
- EP 2809352 A
- EP 2623121 A
- WO 2013113736 A
- EP 2680880 A
- US 20130336998 A
- WO 2012116715 A
- WO 2012116810 A
- EP 2658569 A
- US 20130280283 A
- WO 2012089338 A
- WO 2012089225 A
- EP 2216027 A
- US 20130273001 A
- US 20100303851 A
- EP 1685844 A
- EP 1521585 A
- EP 2216028 A
- EP 1806139 A
- EP 1797886 A
- WO 2004004743 A
- US 20120213818 A
- EP 1928494 A
- WO 2006024518 A
- US 20120009221 A
- EP 2223700 A
- EP 2229953 A
- EP 1938833 A
- EP 1615662 A
- WO 2005016376 A
- US 20100047261 A
- EP 2083851 A
- WO 2008052770 A
- US 20080171711 A
- EP 1768703 A
- WO 2006008154 A
- EP 1383556 A
- WO 2016107877 A
- WO 2016097065 A
- WO 2016091391 A
- WO 2015149944 A
- WO 2015135558 A
- WO 2015101414 A
- WO 2015101415 A
- US 9267138 B
- US 20150037305 A
- EP 1648914 A
- WO 2016022753 A
- US 9447413 B
- US 9447412 B
- US 9359609 B
- US 9012423 B
- US 20150299704 A
- US 20140100263 A
- EP 2906698 A
- WO 2014058881 A
- US 9139832 B
- US 8946179 B
- US 8859521 B
- US 8809294 B
- US 8765701 B
- US 8697663 B
- US 8546350 B
- US 8466120 B
- US 8178506 B
- US 8133876 B
- US 8110558 B
- US 8106025 B
- US 7759319 B
- US 7683036 B
- US 20160017329 A
- US 20150337305 A
- US 20150337304 A
- US 20150247142 A
- US 20150094461 A
- US 20140336370 A
- US 20140329882 A
- US 20140121365 A
- US 20140121364 A
- US 20140057963 A
- US 20120283319 A
- US 20120157514 A
- US 20120122216 A
- US 20120035248 A
- US 20110224277 A
- US 20100267813 A
- US 20100249215 A
- US 20090317907 A
- US 20090298174 A
- US 20090291907 A

- US 20090291906 A
- US 20090286969 A
- US 20090203893 A
- EP 1931780 A
- EP 2530157 A
- EP 2338992 A
- US 20160244753 A
- US 9267137 B
- US 8969317 B
- US 20150218558 A
- US 20130289093 A
- EP 2841579 A
- WO 2013163258 A
- US 20160138016 A
- US 9181547 B
- US 8912161 B
- US 20140329887 A
- US 20140107183 A
- US 20120270928 A
- EP 2702155 A
- WO 2012148952 A
- US 20160046941 A
- US 9150857 B
- US 8680067 B
- US 8211867 B
- US 20140206854 A
- US 20120295962 A
- US 20110251150 A
- US 20100267814 A
- EP 2217248 A
- EP 2992096 A
- US 20150031130 A
- WO 2014179445 A
- EP 3060664 A1
- WO 2015061536 A
- WO 2012012716 A
- WO 2011126842 A
- EP 3060664 A
- US 20160251657 A
- US 9309513 B
- US 9157083 B
- US 20150105449 A
- US 20140350090 A
- EP 2992095 A
- WO 2014179446 A
- US 20160108397 A
- US 9181548 B
- US 8815826 B
- US 20150080453 A
- US 20130184217 A
- US 8969314 B
- US 20150232841 A
- EP 2338991 A
- EP 1931782 A
- EP 3052632 A
- US 20160222391 A
- WO 2015051283 A
- EP 3033424 A
- US 20150247145 A
- US 20150247144 A
- US 20150232847 A
- US 20150232846 A
- US 20150232845 A
- US 20150232844 A
- US 20150225715 A
- US 20150050738 A
- WO 2015023975 A
- US 20150252364 A
- EP 2850186 A
- WO 2013173638 A
- US 20150232858 A
- WO 2016130943 A
- WO 2016130929 A
- WO 2011116152 A
- WO 2007092181 A
- WO 2007089607 A
- US 20160122760 A
- EP 3004354 A
- WO 2014197826 A
- US 20150315585 A
- US 20150315586 A
- US 20150247141 A
- EP 2895200 A
- WO 2014043544 A
- US 20150315587 A
- US 20150299695 A
- US 20150315588 A
- EP 2756080 A
- WO 2013040429 A
- US 20150191722 A
- US 20150218560 A
- US 20150159161 A
- US 20150133362 A
- US 20150133529 A
- EP 2850189 A
- EP 2849801 A
- EP 2849800 A
- WO 2013173652 A
- WO 2013173647 A
- WO 2013173601 A
- US 20150232836 A
- EP 2850183 A
- WO 2013173635 A
- US 20150141320 A
- EP 2850184 A

- WO 2013173637 A
- WO 2016130963 A
- EP 3033422 A
- WO 2015023941 A
- US 20160201064 A
- EP 3033425 A
- WO 2015023939 A
- US 20160201063 A
- EP 3033423 A
- WO 2015023938 A
- EP 3033114 A
- US 20150225722 A
- WO 2015023937 A
- US 9222087 B
- US 8017764 B
- US 7626015 B
- US 20130303590 A
- US 20120108647 A
- US 20100168204 A
- EP 2026843 A
- WO 2007141796 A
- US 9056903 B
- US 8067570 B
- US 20120156208 A
- EP 2402443 A
- EP 1984003 A
- EP 2862929 A
- US 8778904 B
- EP 2510098 A
- WO 2011072091 A
- US 8642571 B
- US 8309532 B
- US 8168607 B
- US 7741299 B
- US 20130095117 A
- US 20110117102 A
- US 20110028532 A
- EP 1791568 A
- EP 2319925 A
- US 20140350068 A
- US 8614311 B
- US 20130131143 A
- WO 2009074990 A
- US 8444983 B
- US 20150359905 A
- US 20140072552 A
- EP 2411413 A
- WO 2010111198 A
- US 20130190387 A
- US 8404654 B
- US 7825099 B
- US 20100029746 A
- US 8344104 B
- US 8034575 B
- US 7723052 B
- US 20120034599 A
- US 20110045499 A
- WO 2008054534 A
- WO 2008001361 A
- US 8034902 B
- EP 1885396 A
- US 7973156 B
- US 7524935 B
- US 20090264634 A
- EP 1115733 A
- US 20110098337 A
- US 7872119 B
- EP 2137205 A
- WO 2008106102 A
- US 20150267194 A
- EP 2895607 A
- WO 2014043289 A

- US 20140323549 A
- EP 2776565 A
- WO 2013070821 A
- EP 2268316 A
- WO 2009116037 A
- EP 2152316 A
- WO 2008132723 A
- EP 1933880 A
- EP 1357881 A
- WO 2010080452 A
- WO 2008126085 A
- US 9446062 B
- EP 2350279 A
- US 9121020 B
- US 8765931 B
- US 20150361430 A
- US 20150050328 A
- WO 2010048352 A
- US 20160102313 A
- US 9045755 B
- US 20130137750 A
- EP 2585594 A
- WO 2011163436 A
- US 20150065559 A
- US 8901097 B
- WO 2011057171 A
- US 8785408 B
- EP 2170403 A
- WO 2009001359 A
- US 20150152412 A
- US 8796239 B
- US 20110178157 A
- EP 2509991 A
- EP 2504435 A
- WO 2011072082 A
- WO 2011066475 A
- US 8410069 B
- US 7812002 B
- US 20110230543 A
- EP 2136847 A
- WO 2008114262 A
- US 20150329866 A
- US 7910566 B
- EP 2371958 A
- EP 2076526 A
- WO 2008050329 A
- US 8278287 B
- EP 2285385 A
- WO 2009144704 A
- US 8198258 B
- US 7939652 B
- US 20110201670 A
- EP 1758998 A
- EP 2330111 A
- US 20150259676 A
- WO 2014043291 A
- US 20150018404 A
- EP 2739637 A
- WO 2013020097 A
- EP 2649181 A
- US 20130324591 A
- WO 2012078536 A
- US 20130267578 A
- US 20120136044 A
- WO 2008152636 A
- US 20130030034 A
- US 20120142754 A
- WO 2012044620 A
- US 20090202566 A
- EP 1624788 A
- WO 2012170957 A
- WO 2010046889 A
- WO 2008020435 A
- EP 1799269 A
- US 9334499 B
- US 9006196 B
- US 8765699 B
- US 8148342 B
- US 7842674 B

- US 20150141487 A
- US 20120184597 A
- US 20080287382 A
- US 9089591 B
- US 8431692 B
- US 20140066493 A
- EP 2293800 A
- WO 2009147684 A
- US 8614309 B
- US 20130123334 A
- EP 2231168 A
- WO 2009044392 A
- US 20110251260 A
- US 8362229 B
- EP 1989307 A
- WO 2007091269 A
- US 20160215284 A
- US 9205100 B
- US 20140005253 A
- EP 2681314 A
- WO 2012118910 A
- EP 2681315 A
- WO 2012118911 A
- US 9434946 B
- US 20150126586 A
- US 20140364484 A
- EP 2802657 A
- WO 2013106494 A
- US 20140140922 A
- US 8637482 B
- EP 2440214 A
- WO 2010144336 A
- US 20150203845 A
- EP 2895608 A
- WO 2014043292 A
- EP 2242854 A
- WO 2009090639 A
- WO 2015183842 A
- US 9382542 B
- US 20140371439 A
- EP 2800812 A
- WO 2013103632 A
- EP 1753464 A
- US 9422560 B
- US 20150031746 A
- EP 2773758 A
- WO 2013067076 A
- US 9249414 B
- US 8859751 B
- US 20150159154 A
- EP 2521783 A
- WO 2011084193 A
- WO 2011085056 A
- WO 2008104978 A
- US 20150211006 A
- EP 2872147 A
- WO 2015107425 A
- US 20150166999 A
- EP 2873674 A
- US 9260471 B
- EP 2632472 A
- EP 1931781 A
- US 7910725 B
- US 7897755 B
- US 7893302 B
- US 7910724 B
- US 7897753 B
- US 7897752 B
- US 7855284 B
- US 7795422 B
- US 7700760 B
- US 7678897 B
- US 7691405 B
- US 7683165 B
- US 7667030 B
- US 7667029 B
- US 7662952 B
- US 7683166 B
- US 7641915 B
- US 7517864 B
- US 7514099 B
- US 7404969 B
- US 20160152973 A
- US 20110160281 A
- US 20100184824 A
- US 20100144851 A
- US 20100099744 A
- US 20100130592 A
- US 20100099743 A
- US 20090306182 A
- US 20090299045 A
- US 20090247613 A
- US 20090253773 A
- US 20090253774 A
- US 20090192105 A
- US 20090156533 A
- US 20090192104 A
- US 20090176725 A
- US 20090149408 A
- US 20090137512 A
- US 20090137511 A
- US 20090137509 A
- US 20090137508 A

- US 20090105178 A
- US 20090143324 A
- US 20090137510 A
- US 20090137507 A
- US 20090143325 A
- US 20090093438 A
- US 20090099119 A
- US 20090099116 A
- US 20090093439 A
- US 20090093437 A
- US 20090093436 A
- US 20090093435 A
- US 20090048197 A
- US 20080249294 A
- US 20080188430 A
- US 20080188675 A
- US 20080161256 A
- US 20080020058 A
- US 20070270579 A
- US 20070185049 A
- US 20070160980 A
- US 20070093437 A
- US 20070042983 A
- US 20070032441 A
- US 20060240554 A
- US 20060217332 A
- US 20060217331 A
- US 20060216747 A
- US 20060142225 A
- US 20050282188 A
- US 20060019917 A
- US 20050288242 A
- US 20050287128 A
- US 20050239731 A
- US 20050233998 A
- US 20050261219 A
- US 20050266422 A
- US 20050267058 A
- US 20050260620 A
- US 20050233997 A
- US 20050233344 A
- US 20050227935 A
- US 20050196767 A
- US 20050196781 A
- US 20050222066 A
- US 20050227936 A
- US 20050203040 A
- US 20050196765 A
- US 20050187174 A
- US 20050143333 A
- US 20050148530 A
- US 20050182007 A
- US 20050153916 A
- US 20050153915 A
- US 20050182009 A
- US 20050182006 A
- US 20050176663 A
- US 20050176025 A
- US 20050176024 A
- US 20050171039 A
- US 20050164968 A
- US 20050164967 A
- US 20050164966 A
- US 20050164224 A
- US 20050159382 A
- US 20050159381 A
- US 20050159380 A
- US 20050158735 A
- US 20050153914 A
- US 20050130181 A
- US 20050079610 A
- US 20050048529 A
- US 20050032733 A
- EP 1627061 A
- EP 1664299 A
- EP 2104740 A
- EP 1922300 A
- EP 1891217 A
- EP 1844147 A
- EP 1817415 A
- EP 1682661 A
- EP 1675953 A
- EP 1423406 A
- WO 2008147438 A
- WO 2008030239 A
- WO 2007086883 A
- WO 2007084865 A
- WO 2007067981 A
- WO 2007086881 A
- WO 2007022369 A
- WO 2006128141 A
- WO 2006078798 A
- WO 2006060598 A
- WO 2005035759 A
- WO 2005028649 A
- WO 2005044981 A
- WO 2005045039 A
- WO 2005040379 A
- WO 2005045032 A
- WO 2005019453 A
- WO 2005007855 A
- WO 2005014811 A
- WO 2004111237 A
- WO 2004097020 A
- WO 2003074654 A
- US 20160272975 A
- US 20160264964 A
- US 20150299696 A
- EP 2844261 A
- US 20050042632 A
- WO 20070676981 A
- US 20160222381 A
- US 9243246 B
- EP 2609198 A
- US 20160244760 A
- US 9181551 B

- US 7858769 B
- US 7034009 B
- US 20160053269 A
- US 20150267200 A
- US 20150148530 A
- US 20140288148 A
- US 20090170197 A
- US 20090137500 A
- US 20080039412 A
- US 20080039414 A
- US 20070042029 A
- US 20050233329 A
- US 20050020525 A
- US 20040220128 A
- EP 1713915 A
- EP 1767632 A
- EP 1522583 A
- EP 1390385 A
- WO 2005078097 A
- US 20150376613 A
- US 9096850 B
- EP 3068407 A
- US 20160256570 A
- WO 2015070158 A
- US 20160130587 A
- US 9222092 B
- US 8933215 B
- US 8324370 B
- US 7893245 B
- US 7452987 B
- US 20150105545 A
- US 20130102769 A
- US 20110118456 A
- US 20090186845 A
- EP 1389637 A
- EP 1527176 A
- EP 2258847 A
- EP 1857547 A
- US 20150359906 A
- US 9125820 B
- US 8735453 B
- US 8017804 B
- US 20140329885 A
- US 20130165381 A
- US 20120065138 A
- US 20110294871 A
- US 20080274116 A
- EP 1771206 A
- US 20150368650 A
- US 9133515 B
- US 20140179755 A
- EP 2849771 A
- WO 2013170960 A
- US 20140249207 A
- US 8722875 B
- US 20110217367 A
- EP 2350278 A
- WO 2010034487 A
- EP 2546337 A
- US 8232256 B
- EP 2049658 A
- WO 2008009477 A
- US 20090252783 A
- EP 2007890 A
- EP 2992875 A
- US 20090074852 A
- EP 2007356 A
- EP 1325955 A
- WO 2010091878 A
- WO 2016083623 A
- WO 2015082080 A
- US 20140328903 A
- US 8829179 B
- US 20120022138 A
- EP 2398903 A
- WO 2010094491 A
- US 20160046940 A
- US 20150087607 A
- US 8846631 B
- WO 2011088309 A
- US 7998677 B
- US 20090236225 A
- EP 3067421 A
- EP 3066219 A
- US 20110124710 A
- US 7893244 B
- US 7893243 B
- US 7786092 B
- US 7723316 B
- US 7534878 B
- US 20090227657 A
- EP 1877065 A
- WO 2006110813 A
- US 7781414 B
- US 20100203036 A
- EP 1615670 A
- WO 2004089284 A
- US 20110046067 A
- EP 2170404 A
- US 20110038849 A
- EP 2069498 A
- WO 2008076127 A
- US 20110015249 A
- EP 2170351 A
- US 20100280097 A
- WO 2009039300 A
- US 20100279919 A
- WO 2009032930 A
- US 20100210710 A
- EP 2209895 A
- WO 2009051659 A
- US 20100028848 A
- EP 1963508 A
- WO 2007064846 A
- US 20090247604 A

- EP 1711510 A
- WO 2005076998 A
- US 20070219118 A
- EP 1448586 A
- WO 2003040399 A
- US 20060211637 A
- EP 1546173 A
- WO 2004013310 A
- EP 1713819 A
- WO 2005076999 A
- EP 1451572 A
- WO 2003063765 A
- US 9441222 B
- US 20140005251 A
- EP 2663641 A
- EP 2474617 A
- WO 2012096573 A
- US 20160017338 A
- US 9161947 B
- US 20130072545 A
- EP 2542678 A
- WO 2011108930 A
- US 20150297626 A
- EP 2917348 A
- WO 2014072357 A
- US 20150225716 A
- EP 2794881 A
- WO 2013095132 A
- US 20150152499 A
- EP 2870263 A
- WO 2014007623 A
- EP 2591106 A
- US 20130109741 A
- WO 2012005572 A
- EP 2607483 A
- WO 2015194956 A
- US 20160024508 A
- US 9157919 B
- US 20130281519 A
- US 8895522 B
- EP 1940472 A
- WO 2007050034 A
- US 20150099799 A
- US 8877724 B
- US 20110301225 A
- EP 2342341 A
- EP 2806028 A
- WO 2010053430 A
- EP 2269622 A
- US 8592390 B
- US 8258107 B
- EP 2380584 A
- EP 1901759 A
- WO 2007004977 A
- EP 2220489 A
- US 8574834 B
- US 20140030723 A
- WO 2009078793 A
- US 8569257 B
- US 8148341 B
- EP 2179737 A
- EP 1904077 A
- WO 2007004979 A
- US 20150004187 A
- EP 2782602 A
- EP 2596806 A
- WO 2013076262 A
- EP 2350282 A
- WO 2010053433 A
- EP 2288702 A
- WO 2009154565 A
- EP 2655622 A
- EP 2468866 A
- EP 2468867 A
- WO 2012084996 A
- WO 2012084993 A
- WO 2012084991 A
- US 8567410 B
- US 20160177295 A
- US 20150291678 A
- US 20150290288 A
- US 20150258174 A
- US 20120195936 A
- EP 2459231 A
- US 20150157565 A
- EP 2858679 A
- WO 2013185069 A
- US 20150126589 A
- EP 2858677 A
- WO 2013182683 A
- EP 3013964 A
- WO 2014207231 A
- WO 2016075154 A
- WO 2016009000 A
- WO 2015128030 A
- US 20150184160 A
- WO 2015100436 A
- WO 2016057932 A
- US 9428752 B
- US 9243244 B
- US 8815825 B
- US 20160186176 A
- US 20160053263 A
- US 20140288292 A
- US 20130109740 A
- EP 3037538 A
- EP 2591105 A
- WO 2012006243 A
- US 9365850 B
- US 20150240234 A
- US 20140107178 A
- EP 2895609 A
- WO 2014043311 A
- US 20160177303 A
- US 9206420 B

- US 20130303593 A
- WO 2012100172 A
- US 20160083729 A
- US 9217146 B
- US 20140179765 A
- WO 2012173994 A
- US 20150197756 A
- US 8927515 B
- US 20130131149 A
- EP 2591104 A
- WO 2012006241 A
- US 20150038555 A
- US 8927705 B
- US 8513207 B
- US 8349809 B
- US 20150038554 A
- US 20140350074 A
- US 20140221454 A
- US 20130096290 A
- US 20130041010 A
- US 20120263738 A
- US 20120095200 A
- US 20110111056 A
- US 20110059187 A
- US 20110003881 A
- US 20100249214 A
- US 20100173974 A
- US 20100173973 A
- US 20100184841 A
- EP 2513334 A
- EP 2437752 A
- EP 2437751 A
- EP 2379083 A
- EP 2341943 A
- WO 2011075188 A
- WO 2011072292 A
- WO 2010141726 A
- WO 2010141724 A
- WO 2010141933 A
- WO 2010080129 A
- WO 2010093788 A
- WO 2010033225 A
- EP 2968149 A
- US 20150374842 A
- WO 2014153163 A
- US 20150315583 A
- EP 2931746 A
- WO 2014093746 A
- US 20150065555 A
- WO 2013138668 A
- US 20140371293 A
- EP 2768958 A
- WO 2013059496 A
- US 20140315983 A
- WO 2013066721 A
- US 20140155462 A
- WO 2012145582 A
- WO 2016100401 A
- US 9200284 B
- US 8372816 B
- US 20150057337 A
- US 20130123342 A
- US 20110021604 A
- EP 2756845 A
- EP 2414374 A
- WO 2010115206 A
- WO 2010115202 A
- EP 2873732 A
- US 20140044755 A
- WO 2013032643 A
- EP 3017047 A
- US 20150011607 A
- WO 2015003113 A
- WO 2015085158 A
- WO 2009131661 A
- US 8071562 B
- US 20160136181 A
- WO 2015153757 A
- US 20150344881 A
- US 20140314833 A
- US 20150246070 A
- WO 2015131115 A
- EP 2968567 A
- US 20150272981 A
- US 20140308274 A
- US 20140309278 A
- WO 2014143855 A
- US 20100179213 A
- WO 2010056737 A
- EP 3013975 A
- WO 2014209970 A
- US 9371526 B
- US 8586727 B
- US 20160053264 A
- US 20140107182 A
- EP 2670850 A
- WO 2012106591 A
- US 20160151406 A
- WO 2016081773 A
- WO 2016161196 A

- US 20160222385 A
- US 9365852 B
- US 8258111 B
- US 20150344880 A
- US 20160060629 A
- US 9222085 B
- EP 2670849 A
- WO 2012106586 A
- US 8900627 B
- EP 2306978 A
- US 9388408 B
- US 20130344135 A
- EP 2864482 A
- WO 2013192576 A
- WO 2016118612 A
- US 20160208258 A
- WO 2016069717 A
- US 20140024700 A
- EP 2652146 A
- WO 2012083004 A
- US 9376681 B
- US 20160068842 A
- WO 2016040373 A
- US 20140187603 A
- US 8642751 B
- US 20120184596 A
- EP 2652151 A
- WO 2012083005 A
- EP 2970968 A
- US 20160010090 A
- WO 2014145356 A
- US 20120238619 A
- WO 2015142735 A
- US 9163235 B
- US 20130345288 A
- EP 2863956 A
- WO 2013192486 A
- US 20140066491 A
- US 20120148664 A
- EP 2440566 A
- WO 2010144485 A
- WO 2016022536 A
- US 20160032286 A
- US 20150118734 A
- US 20150064771 A
- US 20130210120 A
- EP 2742127 A
- WO 2013025248 A
- EP 2356235 A
- US 20150184167 A
- US 9012213 B
- US 20150113670 A
- US 8946176 B
- EP 2851426 A
- EP 2630240 A
- WO 2012052258 A
- WO 2012082894 A
- WO 2016011123 A
- WO 2015195628 A
- US 20020187208 A
- US 6352729 B
- WO 2002036737 A
- US 8247380 B
- US 7445784 B
- EP 1119367 A
- US 7528108 B
- EP 1874796 A
- WO 2006116410 A
- WO 2001084938 A
- US 6312737 B
- EP 2323682 A
- US 20100022442 A
- WO 2010011533 A
- WO 2001082871 A
- WO 2009046739 A
- WO 2009046738 A
- EP 2650368 A
- US 20130121988 A
- US 20090324584 A
- EP 2046954 A
- WO 2008014979 A
- EP 1083232 A
- US 20070280929 A
- WO 2009127230 A
- WO 2003051401 A
- EP 1881847 A
- EP 3062798 A
- US 20160235864 A
- WO 2015062738 A
- WO P2216027 A
- US 20080267873 A
- WO 2006122828 A
- WO 2007095976 A
- EP 1619254 A
- EP 1818409 A
- EP 1541690 A
- WO 2015188933 A
- WO 2008118802 A
- US 7390799 B
- WO 2004106328 A
- WO 2008039218 A
- WO 2011090760 A
- WO 2003063794 A
- WO 2005007623 A
- WO 2006078846 A
- WO 2004019973 A
- WO 2004089925 A
- WO 2007016176 A

- US 8138347 B
- WO 2002088112 A
- WO 2007084786 A
- WO 2007129161 A
- WO 2006122806 A
- WO 2005113554 A
- WO 2007044729 A
- WO 2009114512 A
- WO 2008109943 A
- WO 2007053452 A
- WO 2000142246 A
- WO 2007070514 A

**Non-patent literature cited in the description**


- *Int J Biol Sci.,* 29 November 2011, vol. 8 (1), 118-23
- *PLoS One,* 2015, vol. 10 (3), e0121123
- *J Nutr.,* October 2014, vol. 144 (10), 1495-500
- *Cancer Lett,* 01 February 2016, vol. 371 (1), 48-61
- **KOHLI et al.** *J. Control Rel.,* 2007, vol. 121, 19-27
- **VINOGRADOV et al.** *Bioconjug. Chem.,* 1998, vol. 9, 805-12
- **JOHN et al.** *PLoS Biology,* 2004, vol. 2, 1862-1879
- *PLoS,* 2005, vol. 3, 1328
- **GRIFFITHS-JONES S.** *NAR,* 2004, vol. 32, D109-D111
- **T. W. GREENE ; P. G. M. WUTS.** Protective Groups in Organic Synthesis. John Wiley and Sons, 1991
- **BRUMMELKAMP et al.** *Cancer Cell,* 2002, vol. 2, 243
- **LIMBACH et al.** *Nucleic Acids Res.,* 1994, vol. 22, 2183-2196
- **R. LAROCK.** Comprehensive Organic Transformations. VCH Publishers, 1989
- **L. FIESER ; M. FIESER.** Fieser and Fieser's Reagents for Organic Synthesis. John Wiley and Sons, 1994
- Encyclopedia of Reagents for Organic Synthesis. John Wiley and Sons, 1995
- **GOODMAN, S. L. ; HÖLZEMANN, G. ; SULYOK, G. A. G. ; KESSLER, H.** *J. Med. Chem.,* 2002, vol. 45, 1045-1051
- **SULYOK, G. A. G. ; GIBSON, C. ; GOODMAN, S. L. ; HÖLZEMANN, G. ; WIESNER, M. ; KESSLER H.** *J. Med. Chem.,* 2001, vol. 44, 1938-1950
- **DIAMOND et al.** *Brain reactive antibodies and disease,* 2015
- **FRIEDMAN, A. D. et al.** *Curr Pharm Des,* 2013, vol. 19 (35), 6315-6329
- **HELLMAN et al.** Principles of Radiation Therapy, Cancer, in Principles and Practice of Oncology. 1993, vol. 1, 248-275